# EUROPEAN PATENT APPLICATION

(11) **EP 4 700 135 A2**
(43) Date of publication of application: **25.02.2026**
(21) Application number: 24811467.0
(22) Date of filing: 17.05.2024
(51) Int. Cl.: C12Q 1/70, C12Q 1/689

(54) **CAPTURE PROBE SET FOR SIMULTANEOUS DETECTION OF VIRUS AND BACTERIA BASED ON MURINE TYPHUS SYNDROME AND USE THEREOF**

(30) Priority: 19.05.2023 KR 20230064832
(71) Applicant: Korea Disease Control and Prevention Agency, Chungcheongbuk-go 28159 (KR)
(72) Inventor: KIM, Eun Jin, Cheongju-si, Chungcheongbuk-do 28159 (KR); KIM, Il Hwan, Cheongju-si, Chungcheongbuk-do 28159 (KR); RHEE, Jee Eun, Cheongju-si, Chungcheongbuk-do 28159 (KR); KIM, Jeong Min, Cheongju-si, Chungcheongbuk-do 28159 (KR); PARK, Ae Kyung, Cheongju-si, Chungcheongbuk-do 28159 (KR); KIM, Jeong Ah, Cheongju-si, Chungcheongbuk-do 28159 (KR)
(74) Representative: Gassner, Birgitta
(86) International application number: PCT/KR2024/095792
(87) International publication number: WO 2024/242531

(57) **Abstract**

The present invention relates to a capture probe set for simultaneous detection of viruses and bacteria based on rash syndrome and use thereof

## Description

### BACKGROUND

### [Technical Field]

The present invention relates to capture probe set for simultaneous detection of rash syndrome based viruses and bacteria and their use.

### [Background Art]

Rash syndrome is an acute febrile infectious disease that is spread by infected rat fleas. This is why outbreaks are most common in urban or rural grain warehouses and port areas where rats are common. Although it occurs particularly frequently in the fall, the frequency of disease occurrence and the number of infected people are increasing regardless of location or season due to environmental development and climate change caused by the development of transportation and global warming. Therefore, it is very important to continuously monitor the status of outbreaks of rash syndrome in Korea and collect data on the entire rash syndrome, including the causative pathogen, host, and vector.

Rash syndrome is classified as endemic not only in Korea but also in coastal and port areas of many countries. Countries including the United States, Australia, China, Greece, Israel, Kuwait, and Thailand are actively responding to the outbreak of rash syndrome. As the spread of new infectious diseases, including the recent COVID-19 outbreak, continues to spread globally, there is a need for rapid response measures to address the potential risk of infectious disease symptoms and rash syndrome that may occur after traveling abroad.

The incubation period for rash syndrome is approximately 8-10 days, with rapid onset and a high fever of 39-40° C lasting for about a week. Initially, many patients present with headache, chills, muscle pain, and a dry cough without phlegm, which can be confused with cold symptoms. The representative clinical symptoms are headache, fever, muscle pain, and vomiting, and although it may be a rash, it is difficult to clinically differentiate it from other diseases that cause rashes, including typhus (including Brill-Zinsser disease), typhoid fever, and paratyphoid fever.

Current diagnostic methods involve isolating and identifying the organism from a patient's specimen. A definitive diagnosis can be made if Rickettsia typhi genes or antigens are detected in the specimen. Serological testing is also performed to confirm the diagnosis and differentiate it from other rickettsial diseases.

For early detection and early response, quickly identifying the pathogen causing the disease is paramount. However, technological limitations make simultaneous screening and diagnosis of multiple pathogens challenging. To overcome these limitations, a multi-test panel utilizing next-generation sequencing (NGS) methods, differentiated from conventional methods, is being developed, enabling rapid and accurate simultaneous testing of multiple pathogens.

Currently, there is no panel in Korea that can accurately diagnose and distinguish rash syndrome using NGS technology. Therefore, by developing and providing a diagnostic kit and analysis pipeline for the purpose of rash syndrome testing, we can proactively prepare for and respond to the continued emergence of new infectious diseases.

### DETAILED DESCRIPTION OF THE INVENTION

### [Technical Problem]

The present invention was derived from the above-mentioned needs, and the inventors of the present invention completed the present invention by identifying a capture probe set capable of simultaneously detecting a virus and bacteria based on rash syndrome, a composition for diagnosing rash syndrome, and a method for simultaneously detecting a virus and bacteria based on rash syndrome using the same.

### [Technical Solution]

To solve the above problem, the present invention provides a capture probe set for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, comprising SEQ ID NO: 1 to SEQ ID NO: 3828.

The concentration of the probes can be selected and used in various ways by a person skilled in the art depending on the experimental conditions, and is preferably 1 to 1000 nM each, and more preferably 100 to 500 nM each, but is not limited thereto.

The present invention provides a composition for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, comprising the capture probe set for simultaneous detection.

Also, the present invention provides a composition for diagnosing rash syndrome comprising the capture probe set for simultaneous detection.

Also, the present invention provides a kit for diagnosing rash syndrome, comprising a composition for diagnosing rash syndrome and instructions for use.

In one example of the present invention, a sample for detection or diagnosis may be isolated from feces, blood, serum, urine or biological tissue.

In another example of the present invention, the rash syndrome may be at least one selected from the group consisting of anaplasmosis, anthrax, Lyme disease, relapsing fever, chikungunya fever, Q fever, dengue fever, ehrlichiosis, chickenpox, measles, monkeypox, tsutsugamushi disease, rickettsial infection, typhus, rash, rubella, vaccinia, plague, and Zika virus infection.

The present invention provides a method for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, comprising the steps of preparing an isolated DNA or RNA sample; reacting the isolated DNA or RNA sample using the capture probe set; and obtaining a detection result.

### [Effects of the invention]

The capture probe set for simultaneous detection of viruses and bacteria based on the rash syndrome of the present invention enables simultaneous detection of multiple pathogens through NGS, and secures work efficiency by developing a kit with improved sensitivity, specificity, and accuracy, and can be utilized as a multiple detection system for pathogens causing rash syndrome.

Further, it can be actively utilized as a preemptive response before a rash syndrome pandemic, and can be expanded to respond to other new infectious diseases that may occur in the future.

### [Brief description of the drawings]

Figure 1 illustrates a schematic diagram of a multi-pathogen panel analysis pipeline according to one embodiment of the present invention.
Figure 2 illustrates the results of an in silico analysis of a rash syndrome panel according to one embodiment of the present invention.

### [Detailed Description]

Hereinafter, preferred embodiments of the present invention will be described in detail. Furthermore, the following description includes numerous specific details, such as specific components. These details are provided solely to facilitate a more comprehensive understanding of the present invention. It will be apparent to those skilled in the art that the present invention can be practiced without these specific details. Further, when describing the present invention, if it is determined that a detailed description of a related known function or configuration may unnecessarily obscure the gist of the present invention, the detailed description is omitted.

### <Example 1> Kit component design - Development of a multi-pathogen capture panel

Among the pathogens that can cause rash syndrome, 37 pathogens were selected based on their frequency of occurrence and risk.

Using existing testing methods as a guide, specific genetic regions capable of distinguishing each pathogen species were selected to determine the target for gene capture. To ensure sufficient efficiency of the gene capture panel, the entire genome was targeted for viruses with small genomes. For viruses or bacteria with large genomes, the target area was expanded to encompass the region surrounding specific genes, ensuring capture of at least 0.5% of the entire genome.

The pathogen types, reference genome sequence information, and target region information included in the developed rash syndrome multi-pathogen panel are as shown in Table 1.

**[Table 1]**

| Pathogen information | | Panel target area | | | |
|---|---|---|---|---|---|
| Pathogen name | Infectious disease name | Ref ID | Start | End | Target information |
| Human parvovirus | erythema infectiosum | MH201455.1 | 0 | 5596 | Whole genome |
| Rubella virus | Rubella | JN635287.1 | 0 | 9762 | Whole genome |
| Dengue virus 1 | Dengue fever | ON123661.1 | 0 | 10735 | Whole genome |
| Dengue virus 2 | Dengue fever | MH781014.1 | 0 | 10617 | Whole genome |
| Dengue virus 3 | Dengue fever | ON123670.1 | 0 | 10707 | Whole genome |
| Dengue virus 4 | Dengue fever | MN018393.1 | 0 | 10652 | Whole genome |
| Zika virus | Zika virus infection | MG758786.1 | 0 | 10806 | Whole genome |
| West Nile virus | West Nile fever | NC_001563.2 | 0 | 10962 | Whole genome |
| Chikungunya virus | Chikungunya fever | ON009843.1 | 0 | 11768 | Whole genome |
| Measles virus | Measles | KX838946.2 | 0 | 15894 | Whole genome |
| Human coxsackievirus A6 | Enterovirus infections | AY421764 | 0 | 7435 | Whole genome |
| Echovirus 9 | Enterovirus infections | X92886 | 0 | 6612 | Whole genome |
| Human gammaherpersvirus 4 | infectious mononucleosis | NC_007605.1 | 27901 | 37947 | BWRF1-EBNA-2 |
| Human herpesvirus 6A | Sudden rash (rose rash) | KP257584.1 | 98594 | 108291 | U63-U72 |
| Human herpesvirus 6B | Sudden rash (rose rash) | NC_000898.1 | 99921 | 109627 | U63-U72 |
| Human herpesvirus 3 | varicella | NC_001348.1 | 66074 | 71369 | ORF37+ORF38+ ORF39 |
| Cowpox virus | vaccinia | KY569022.1 | 132 | 7643 | CPXV011-CPXV016 |
| Monkeypox virus CA | monkeypox | AF380138.1 | 44275 | 49449 | F1L-F3L-F4L |
| Monkeypox virus WA | monkeypox | MT903344.1 | 42258 | 47437 | F1L-F3L-F4L |
| Vaccinia virus | vaccinia | MN974381.1 | 173031 | 178089 | VACCP-B7R-B10R~B15R |
| Variola virus | pock | BK010317.1 | 134193 | 139829 | VARV-VD21-U Vic-145-155 |
| Anaplasma phagocytophilum | Anaplasmosis | CP006617.1 | 106164 0 | 106914 9 | 16S rRNA+ peripheral area |
| Borrelia burgdorferi | Lyme disease | CP001205.1 | 439163 | 443763 | 16S rRNA+ 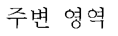 |
| Borrelia hermsii | recurrent fever | CP073148.1 | 445226 | 449962 | 16S rRNA+ peripheral area |
| Coxiella burnet i i | Queue | CP013667.1 | 855200 | 865721 | AUR58_05050~ AUR58_05080 |
| Ehrlichia chaffeensis | Ehrlichiosis | CP000236.1 | 32584 | 38474 | fdxA+120kDa protein gene +virD4 |
| Francisella tularensis | tularemia | AJ749949.2 | 906959 | 916437 | prlC+lpnA+ lpnB+topA |
| Orientia tsutsugamushi | Tsutsugamush i disease | AM494475.1 | 575357 | 586068 | murC+putativ e Orientia tsutsugamush i type-specific antigen+ glyQ+glyS |
| Rickettsia prowazeki i | Epidemic typhus (typhus group, TG) | CP004889. 1 | 812735 | 819080 | gltA+ peripheral area |
| Rickettsia typhi | murine typhus (typhus group, TG) | AE017197.1 | 106037 9 | 106593 9 | RT0831+gltA +RT0833 |
| Rickettsia japonica | Rickettsia infections (spotted fever group, SFG) | AP017602.1 | 118961 6 | 119663 6 | rOmpA+ peripheral area |
| Rickettsia akari | Rickettsia infections (spotted fever group, SFG) | CP000847.1 | 113984 8 | 114600 7 | rOmpA+ peripheral area |
| Rickettsia conorii | Rickettsia infections (spotted fever group, SFG) | AE006914.1 | 117519 1 | 118153 6 | rOmpA+ peripheral area |
| Rickettsia sibirica | Rickettsia infections (spotted fever group, SFG) | AABW01000001. 1 | 755482 | 761758 | rOmpA+ peripheral area |
| Rickettsia rickettsii | Rickettsia infections (spotted fever group, SFG) | CP003307.1 | 117591 1 | 118225 5 | rOmpA+ peripheral area |
| Bacillus anthracis | Anthrax | AE017334.2 | 505920 | 527082 | pf1A~sspE ~hemL1 |
| | | AE017336.2 | 143779 | 147295 | pagA-pagR |
| | | AE017335.3 | 55599 | 56993 | capB |
| Yersinia pestis | pest | NC_003143.1 | 189574 5 | 191553 3 | cheA~yapJ ~cheR |
| | | NC_003134.1 | 81334 | 83343 | caf1R+ caf1M |
| | | NC_003132.1 | 6665 | 8089 | pla+ YPO_RS00040 |
| | | | | | |

Hybridization probes were designed and manufactured so that each selected capture area could be captured by at least two probes (Table 2).

**[Table 2]**

| Sequence number | Pathogen name | Target area information |
|---|---|---|
| 1-125 | Anaplasma phagocytophilum | 16S rRNA |
| 126-477 | Bacillus anthracis | sspE |
| 478-535 | | pagA |
| 536-558 | | capB |
| 559-634 | Borrelia burgdorferi | 16S rRNA |
| 635-712 | Borrelia hermsii | 16S rRNA |
| 713-908 | Chikungunya virus | Whole genome |
| 909-1083 | Coxiella burnetii | IS1111 |
| 1084-1261 | Dengue virus 1 | Whole genome |
| 1262-1437 | Dengue virus 2 | Whole genome |
| 1438-1615 | Dengue virus 3 | Whole genome |
| 1616-1792 | Dengue virus 4 | Whole genome |
| 1793-1883 | Ehrlichia chaffeensis | 120 kDa protein gene |
| 1884-1971 | Human herpesvirus 3 | ORF38 |
| 1972-2235 | Measles virus | Whole genome |
| 2236-2321 | Monkeypox virus WA | F3L |
| 2322-2499 | Orientia tsutsugamushi | tsa56 |
| 2500-2601 | Rickettsia akari | rOmpA |
| 2602-2706 | Rickettsia conorii | rOmpA |
| 2707-2820 | Rickettsia japonica | rOmpA |
| 2821-2925 | Rickettsia prowazekii | gltA |
| 2926-3017 | Rickettsia typhi | gltA |
| 3018-3179 | Rubella virus | Whole genome |
| 3180-3263 | Vaccinia virus | B10R |
| 3264-3592 | Yersinia pestis | Chromosomal hypothetical gene |
| 3593-3625 | | F1 operon |
| 3626-3648 | | pla |

### - Probe sequence

Sequence number 1: Anaplasma phagocytophilum 16S rRNA #1 
ACCGAGCGTATATCCGTAATGAAAGATACTGAAACGGCAGAAAGAATAAAATTCTTCTCTTTACACTA
ACAGCATAGTACTTGAGATCTGGATACTACACATGCCGTGTTATCAGCTTTC
Sequence number 2: Anaplasma phagocytophilum 16S rRNA #2 
TTACACTAACAGCATAGTACTTGAGATCTGGATACTACACATGCCGTGTTATCAGCTTTCTTTGCAGA
ATTCTTGAGCCAACCATAAGAATAATTTATCAGCTTATAAAGCTTCACGGCC
Sequence number 3: Anaplasma phagocytophilum 16S rRNA #3 TTTGCAGAATTCTTGAGCCAACCATAAGAATAATTTATCAGCTTATAAAGCTTCACGGCCACACAACG GGAGATATAACTTTGAGGTCAAAGAATCAAGCATGCAATTGCCTTAAACAAC
Sequence number 4: Anaplasma phagocytophilum 16S rRNA #4 
ACACAACGGGAGATATAACTTTGAGGTCAAAGAATCAAGCATGCAATTGCCTTAAACAACTCGAATAA
TCTTCAGTTCATGGCCCAAAGCATTGCAAAATGCACTACGCTATCTAGCATA
Sequence number 5: Anaplasma phagocytophilum 16S rRNA #5 
TCGAATAATCTTCAGTTCATGGCCCAAAGCATTGCAAAATGCACTACGCTATCTAGCATATTGGATAT
ACGGAGCTAATAAGGCCGTTGACGAAAATGCATGTGTGCGTAACACACATGC
Sequence number 6: Anaplasma phagocytophilum 16S rRNA #6 
TTGGATATACGGAGCTAATAAGGCCGTTGACGAAAATGCATGTGTGCGTAACACACATGCAAGAAACT
TGCCAATCCCCTCCCCAGAATTTTGACGATTGCTGACAGAACCCGTATAGAA
Sequence number 7: Anaplasma phagocytophilum 16S rRNA #7 
AAGAAACTTGCCAATCCCCTCCCCAGAATTTTGACGATTGCTGACAGAACCCGTATAGAAAGAAGCAT
ACAAGAAACCAATGATATCACCAGCTATGACCCTTACCCAACAAATATCGCT
Sequence number 8: Anaplasma phagocytophilum 16S rRNA #8 
AGAAGCATACAAGAAACCAATGATATCACCAGCTATGACCCTTACCCAACAAATATCGCTCATAGTAA
CCTGTGAAAGCGATATAAAAGAAAATAAGTCACCTATACCCGGAAGGATTGT
Sequence number 9: Anaplasma phagocytophilum 16S rRNA #9 
CATAGTAACCTGTGAAAGCGATATAAAAGAAAATAAGTCACCTATACCCGGAAGGATTGTCATATCTC
AAAAAACAGTCCTCTTTGATGTGATGTAAGGTTAGCTTATAGCTTAGCGGGA
Sequence number 10: Anaplasma phagocytophilum 16S rRNA #10 
CATATCTCAAAAAACAGTCCTCTTTGATGTGATGTAAGGTTAGCTTATAGCTTAGCGGGATTGGCAAT
TTTCTCTAAATTGTCGTTATTTACGCACTCCAGCTTCTTTTTCCAGCCTACC
Sequence number 11: Anaplasma phagocytophilum 16S rRNA #11 
TTGGCAATTTTCTCTAAATTGTCGTTATTTACGCACTCCAGCTTCTTTTTCCAGCCTACCGCCATTCA
TGAAATTAAATAGCACACTATCAGCAGGTCCATATCCCACAACCACACCTAA
Sequence number 12: Anaplasma phagocytophilum 16S rRNA #12 
GCCATTCATGAAATTAAATAGCACACTATCAGCAGGTCCATATCCCACAACCACACCTAATTCAGTAA
GATCTTTCCCTACAGCTCGTATCTTTTTCAAATCATCTTCTCCCATAGCAGC
Sequence number 13: Anaplasma phagocytophilum 16S rRNA #13 
TTCAGTAAGATCTTTCCCTACAGCTCGTATCTTTTTCAAATCATCTTCTCCCATAGCAGCAGATACAG
AATCTCTCTTATTATCCAAGTTCTGAGGACCACGCTGCACTTCCCGACTCTT
Sequence number 14: Anaplasma phagocytophilum 16S rRNA #14 
AGATACAGAATCTCTCTTATTATCCAAGTTCTGAGGACCACGCTGCACTTCCCGACTCTTGGCATTAG
CATGGATATCCTCCTTGTCTGCCCCTGTATCAACAGTGTGTTCTGCATGAGA
Sequence number 15: Anaplasma phagocytophilum 16S rRNA #15 
GGCATTAGCATGGATATCCTCCTTGTCTGCCCCTGTATCAACAGTGTGTTCTGCATGAGATTCTGATA
TATCTGAAGCTTTCCCCTTGATTAAGCTTCCATAAAGCCTCGCTGATGCCGC
Sequence number 16: Anaplasma phagocytophilum 16S rRNA #16 
TTCTGATATATCTGAAGCTTTCCCCTTGATTAAGCTTCCATAAAGCCTCGCTGATGCCGCAGTTTCTG
CACGAAACTTCGAAGCCTTCTCTGAACACTCGGAAAAAGCGCTCATTACGTA
Sequence number 17: Anaplasma phagocytophilum 16S rRNA #17 
AGTTTCTGCACGAAACTTCGAAGCCTTCTCTGAACACTCGGAAAAAGCGCTCATTACGTAGTGCTTTA
AAATCGTACTTTGACCGCTACTTGGGTTTAACATTGCGTTTACCGCCAGCGG
Sequence number 18: Anaplasma phagocytophilum 16S rRNA #18 
GTGCTTTAAAATCGTACTTTGACCGCTACTTGGGTTTAACATTGCGTTTACCGCCAGCGGCACGGCCT
TCAACGATGTTCTTACAACCGTTCTTACACAACCTAAGAAACACGATAATGC
Sequence number 19: Anaplasma phagocytophilum 16S rRNA #19 
CACGGCCTTCAACGATGTTCTTACAACCGTTCTTACACAACCTAAGAAACACGATAATGCTGATGCTA
ATCTCTCAAATCCCCGTAACACACTTCCTAAAAATACACAAAATAGCGCAGA
Sequence number 20: Anaplasma phagocytophilum 16S rRNA #20 
TGATGCTAATCTCTCAAATCCCCGTAACACACTTCCTAAAAATACACAAAATAGCGCAGATACTCTTG
AAGCACCCGTTACAGTCCCCCTATCCCTTGCAATAGATAAATGCCTAGCCTT
Sequence number 21: Anaplasma phagocytophilum 16S rRNA #21 
TACTCTTGAAGCACCCGTTACAGTCCCCCTATCCCTTGCAATAGATAAATGCCTAGCCTTCTCTTCAA
AATACGACGCTCCCTTATTTAAACCCTTCACAACAACATTTAGCGAGCTGTG
Sequence number 22: Anaplasma phagocytophilum 16S rRNA #22 
CTCTTCAAAATACGACGCTCCCTTATTTAAACCCTTCACAACAACATTTAGCGAGCTGTGAACAATCA
TAGCAGCCACTTCTAACAGCCCAGCACAGAGGTTAAAAGGAAGCGCAATAAG
Sequence number 23: Anaplasma phagocytophilum 16S rRNA #23 
AACAATCATAGCAGCCACTTCTAACAGCCCAGCACAGAGGTTAAAAGGAAGCGCAATAAGCAATGTAG
GTAACCACATGGTATCACCAGTGACCTCATTCTTAATACGCCTTGTCTCTGG
Sequence number 24: Anaplasma phagocytophilum 16S rRNA #24 
CAATGTAGGTAACCACATGGTATCACCAGTGACCTCATTCTTAATACGCCTTGTCTCTGGATTTACTT
CAGGATTACATACCACGCGCATCAATCTAGGGTCCTTATCACTGCTGCCTAT
Sequence number 25: Anaplasma phagocytophilum 16S rRNA #25 
ATTTACTTCAGGATTACATACCACGCGCATCAATCTAGGGTCCTTATCACTGCTGCCTATAGGAACAA
CTCTATAACCCTCGCATGCGATTACTAATCTTTTGTTCTTATAACGACTAGG
Sequence number 26: Anaplasma phagocytophilum 16S rRNA #26 
AGGAACAACTCTATAACCCTCGCATGCGATTACTAATCTTTTGTTCTTATAACGACTAGGATTTAAAC
CCAAGATCTTGAATGCGTCTTCTGTTTGATTCGTATTGATCGCATAGAGCTG
Sequence number 27: Anaplasma phagocytophilum 16S rRNA #27 
ATTTAAACCCAAGATCTTGAATGCGTCTTCTGTTTGATTCGTATTGATCGCATAGAGCTGCTTGTTAT
CATGAGTAGTGACACGCTCCACGCCCGTGTCAGCAATTGGCATTATCGGAAA
Sequence number 28: Anaplasma phagocytophilum 16S rRNA #28 
CTTGTTATCATGAGTAGTGACACGCTCCACGCCCGTGTCAGCAATTGGCATTATCGGAAAAGTATGCC
CGTCTACAACAAGCAAACCCTTGTTAACCCACATACCTTCCTGCTTGATAGA
Sequence number 29: Anaplasma phagocytophilum 16S rRNA #29 
AGTATGCCCGTCTACAACAAGCAAACCCTTGTTAACCCACATACCTTCCTGCTTGATAGAGCTAGTAG
GCAAAAACACACAAGGCTGTTGCCTGTCAACAACCTGCGAAACAACCATCGG
Sequence number 30: Anaplasma phagocytophilum 16S rRNA #30 
GCTAGTAGGCAAAAACACACAAGGCTGTTGCCTGTCAACAACCTGCGAAACAACCATCGGTACCTTTA
TTAACCTAGGGCGCCCAAAGCCTTCAGGATCCAATTGCGCAAACTTCTTCAA
Sequence number 31: Anaplasma phagocytophilum 16S rRNA #31 TACCTTTATTAACCTAGGGCGCCCAAAGCCTTCAGGATCCAATTGCGCAAACTTCTTCAACGGATTCG CTATAAAACCAAACGTGCTCTTAGCACCATCCACCAACCTACTATAATTCCT
Sequence number 32: Anaplasma phagocytophilum 16S rRNA #32 
CGGATTCGCTATAAAACCAAACGTGCTCTTAGCACCATCCACCAACCTACTATAATTCCTGCCCCTTA
TGCGATCTCCGATCCCAGAAAAAAAACCAGCTAATCTACCTCTTTTACTCTC
Sequence number 33: Anaplasma phagocytophilum 16S rRNA #33 
GCCCCTTATGCGATCTCCGATCCCAGAAAAAAAACCAGCTAATCTACCTCTTTTACTCTCATCCCCGT
ACATTGATTCTCACCATTTATAATTTTGATATGCTATAGGAAAAATATTAAT
Sequence number 34: Anaplasma phagocytophilum 16S rRNA #34 
ATCCCCGTACATTGATTCTCACCATTTATAATTTTGATATGCTATAGGAAAAATATTAATTCCTTGCT
AGAGAGAAAATAATACATGCAGCTATAGCGCAACACAAGCTCATAACACCCC
Sequence number 35: Anaplasma phagocytophilum 16S rRNA #35 
TCCTTGCTAGAGAGAAAATAATACATGCAGCTATAGCGCAACACAAGCTCATAACACCCCAAGAGCCA
CTATGGCGAAGGAAAGATCCAACTATCTTTTTTGATATAACATACCAAAGGC
Sequence number 36: Anaplasma phagocytophilum 16S rRNA #36 
AAGAGCCACTATGGCGAAGGAAAGATCCAACTATCTTTTTTGATATAACATACCAAAGGCGCCTTACG
CCATAATAAAGGCAGCTTGACCTACATACCTAGCGTATGCAGCAACACCACC
Sequence number 37: Anaplasma phagocytophilum 16S rRNA #37 
GCCTTACGCCATAATAAAGGCAGCTTGACCTACATACCTAGCGTATGCAGCAACACCACCAAAGAAAA
TACACAAACTGTTAGTACAGCAAATAATTTTATGAAAGTAATCAGAAAAGCT
Sequence number 38: Anaplasma phagocytophilum 16S rRNA #38 
AAAGAAAATACACAAACTGTTAGTACAGCAAATAATTTTATGAAAGTAATCAGAAAAGCTCGTAGCGC
ATCAGAGCGCACATAATCCTCCAAGACAACCTGCACACCCAAAACACCATGA
Sequence number 39: Anaplasma phagocytophilum 16S rRNA #39 
CGTAGCGCATCAGAGCGCACATAATCCTCCAAGACAACCTGCACACCCAAAACACCATGATAAAAAGC
CGGCATCAAAAATAAAGCAGTCAATGCCCCCTTGTACCCATGCGTTAAATCT
Sequence number 40: Anaplasma phagocytophilum 16S rRNA #40 
TAAAAAGCCGGCATCAAAAATAAAGCAGTCAATGCCCCCTTGTACCCATGCGTTAAATCTAGAAGCCC
CGTAGCAAAATCAGAAACTAATAACGCCTTCACAAGGAATATTGGAACCGGC
Sequence number 41: Anaplasma phagocytophilum 16S rRNA #41 
AGAAGCCCCGTAGCAAAATCAGAAACTAATAACGCCTTCACAAGGAATATTGGAACCGGCAGCATTAC
CACCGCAGTCATCCGCTGCATCCACCAGTGTAAAATACTCTTTCCTGACATA
Sequence number 42: Anaplasma phagocytophilum 16S rRNA #42 
AGCATTACCACCGCAGTCATCCGCTGCATCCACCAGTGTAAAATACTCTTTCCTGACATATATTAACT
GAAGAAACCTATAAAACCCAGCCCGATCAACGAGGTCACCGCAAAACCTAAC
Sequence number 43: Anaplasma phagocytophilum 16S rRNA #43 
TATTAACTGAAGAAACCTATAAAACCCAGCCCGATCAACGAGGTCACCGCAAAACCTAACATCAACCA
ACCACTGAAAGTAGCCTCAGATTTACCAAAGCCAATTCCGCAATCCCAGAAT
Sequence number 44: Anaplasma phagocytophilum 16S rRNA #44 
ATCAACCAACCACTGAAAGTAGCCTCAGATTTACCAAAGCCAATTCCGCAATCCCAGAATAAATGCCG
AATCCCGTTATAATAGTGGTAACACAGCGCAGCAATACAGCCCCCTATTATC
Sequence number 45: Anaplasma phagocytophilum 16S rRNA #45 
AAATGCCGAATCCCGTTATAATAGTGGTAACACAGCGCAGCAATACAGCCCCCTATTATCACCTTTAC
CAACAGTACACCTATCCAGGAGTCTAACAAACCCCACAGCTGACCAGATGTC
Sequence number 46: Anaplasma phagocytophilum 16S rRNA #46 
ACCTTTACCAACAGTACACCTATCCAGGAGTCTAACAAACCCCACAGCTGACCAGATGTCCAATGTAA
AGAAATCAATAACCATGTAAAGAGAGTCAGCCCCATCAATAGCAGGACACCA
Sequence number 47: Anaplasma phagocytophilum 16S rRNA #47 
CAATGTAAAGAAATCAATAACCATGTAAAGAGAGTCAGCCCCATCAATAGCAGGACACCAGTGATTCT
GTGCGTAATCGATAGCCACGCCGTTATCGGCAGCTTATAGACACTAAGATGC
Sequence number 48: Anaplasma phagocytophilum 16S rRNA #48 
GTGATTCTGTGCGTAATCGATAGCCACGCCGTTATCGGCAGCTTATAGACACTAAGATGCGGAGACAG
AGGCCTAGACCTCGACACTATAGTAGCCTCCGGTTAATTCACAATAGAAATA
Sequence number 49: Anaplasma phagocytophilum 16S rRNA #49 
GGAGACAGAGGCCTAGACCTCGACACTATAGTAGCCTCCGGTTAATTCACAATAGAAATAAAGACGGC
GGCACATTTACCGACAAACTACCATAAGATAGAATACTCAGTAAAAAGCCTA
Sequence number 50: Anaplasma phagocytophilum 16S rRNA #50 
AAGACGGCGGCACATTTACCGACAAACTACCATAAGATAGAATACTCAGTAAAAAGCCTAAAGGAGGT
AATCCAGCCGCAGGTTCACCTACAGCTACCTTGTTACGACTTCACCCTAGTC
Sequence number 51: Anaplasma phagocytophilum 16S rRNA #51 
AAGGAGGTAATCCAGCCGCAGGTTCACCTACAGCTACCTTGTTACGACTTCACCCTAGTCACTGACCC
AACCTTAAATGGCTGCCTCCTTTCGGTTGGCGCACCAGCTTCGAGTTAAGCC
Sequence number 52: Anaplasma phagocytophilum 16S rRNA #52 
ACTGACCCAACCTTAAATGGCTGCCTCCTTTCGGTTGGCGCACCAGCTTCGAGTTAAGCCAATTCCCA
TGGCGTGACGGGCAGTGTGTACAAGACCCGAGAACGTATTCACCGTGGCATG
Sequence number 53: Anaplasma phagocytophilum 16S rRNA #53 
AATTCCCATGGCGTGACGGGCAGTGTGTACAAGACCCGAGAACGTATTCACCGTGGCATGCTGATCCA
CGATTACTAGCGATTCCGACTTCATGCCCTCGAGTTGCAGAGGACAATCCGA
Sequence number 54: Anaplasma phagocytophilum 16S rRNA #54 
CTGATCCACGATTACTAGCGATTCCGACTTCATGCCCTCGAGTTGCAGAGGACAATCCGAACTGAGAT
GACTTTTACGGATTAGCTCAGCCTTGCGACATTGCAACCTATTGTAGTCACC
Sequence number 55: Anaplasma phagocytophilum 16S rRNA #55 
ACTGAGATGACTTTTACGGATTAGCTCAGCCTTGCGACATTGCAACCTATTGTAGTCACCATTGTAGC
ACGTGTGTAGCCCACCCCATAAGGGCCGTGCTGACTTGACATCATCCCCACC
Sequence number 56: Anaplasma phagocytophilum 16S rRNA #56 
ATTGTAGCACGTGTGTAGCCCACCCCATAAGGGCCGTGCTGACTTGACATCATCCCCACCTTCCTCCA
GTTTACCACTGGCAGTTTCCTTAAAGTGCCCGGCTTAACCCGCTGGCAACTA
Sequence number 57: Anaplasma phagocytophilum 16S rRNA #57 
TTCCTCCAGTTTACCACTGGCAGTTTCCTTAAAGTGCCCGGCTTAACCCGCTGGCAACTAAGGATGAG
GGTTACGCTCGTTGCGGGACTTAACCCAACATCTCACGACACGAGCTGACGA
Sequence number 58: Anaplasma phagocytophilum 16S rRNA #58 
AGGATGAGGGTTACGCTCGTTGCGGGACTTAACCCAACATCTCACGACACGAGCTGACGACAGCCATG
CAGCACCTGTGCGAGATCCAGCCGAACTGCGCCCTTCCGTTAAGAAGGATCT
Sequence number 59: Anaplasma phagocytophilum 16S rRNA #59 CAGCCATGCAGCACCTGTGCGAGATCCAGCCGAACTGCGCCCTTCCGTTAAGAAGGATCTAATCTCCA TGTCAAGGAGTGGTAAGGTTTTTCGCGTTGCATCGAATTAAACCACATGCTC
Sequence number 60: Anaplasma phagocytophilum 16S rRNA #60 
AATCTCCATGTCAAGGAGTGGTAAGGTTTTTCGCGTTGCATCGAATTAAACCACATGCTCCACCGCTT
GTGCGGGTCCCCGTCAATTCCTTTGAGTTTTAGTCTTGCGACCGTAGTCCCC
Sequence number 61: Anaplasma phagocytophilum 16S rRNA #61 
CACCGCTTGTGCGGGTCCCCGTCAATTCCTTTGAGTTTTAGTCTTGCGACCGTAGTCCCCAGGCGGAG
TGCTTAACGCGTTAGCTACAACACAGAGATAAAAAATCCCCACATTCAGCAC
Sequence number 62: Anaplasma phagocytophilum 16S rRNA #62 
AGGCGGAGTGCTTAACGCGTTAGCTACAACACAGAGATAAAAAATCCCCACATTCAGCACTCATCGTT
TACAGCGTGGACTACCAGGGTATCTAATCCTGTTTGCTCCCCACGCTTTCGC
Sequence number 63: Anaplasma phagocytophilum 16S rRNA #63 
TCATCGTTTACAGCGTGGACTACCAGGGTATCTAATCCTGTTTGCTCCCCACGCTTTCGCACCTCAGC
GTCAGTACCGGACCAGATAGCCGCCTTCGCCACTGGTGTTCCTCCTAATATC
Sequence number 64: Anaplasma phagocytophilum 16S rRNA #64 
ACCTCAGCGTCAGTACCGGACCAGATAGCCGCCTTCGCCACTGGTGTTCCTCCTAATATCTACGAATT
TCACCTCTACACTAGGAATTCCGCTATCCTCTCCCGGACTCTAGTCTGGCAG
Sequence number 65: Anaplasma phagocytophilum 16S rRNA #65 
TACGAATTTCACCTCTACACTAGGAATTCCGCTATCCTCTCCCGGACTCTAGTCTGGCAGTATTAAAA
GCAGCTCCAGGGTTAAGCCCTGGCATTTCACCTTTAACTTACCGAACCGCCT
Sequence number 66: Anaplasma phagocytophilum 16S rRNA #66 
TATTAAAAGCAGCTCCAGGGTTAAGCCCTGGCATTTCACCTTTAACTTACCGAACCGCCTACATGCCC
TTTACGCCCAATAATTCCGAACAACGCTTGCCCCCTCCGTATTACCGCGGCT
Sequence number 67: Anaplasma phagocytophilum 16S rRNA #67 
ACATGCCCTTTACGCCCAATAATTCCGAACAACGCTTGCCCCCTCCGTATTACCGCGGCTGCTGGCAC
GGAGTTTGCCGGGACTTCTTCTGTAGGTACCGTCATTATCTTCCCTACTGAA
Sequence number 68: Anaplasma phagocytophilum 16S rRNA #68 
GCTGGCACGGAGTTTGCCGGGACTTCTTCTGTAGGTACCGTCATTATCTTCCCTACTGAAAGAGTTTT
ACAACCCTAAGGCCTTCCTCACTCACGCGGCATAGCTGGATCAGGCTTGCGC
Sequence number 69: Anaplasma phagocytophilum 16S rRNA #69 
AGAGTTTTACAACCCTAAGGCCTTCCTCACTCACGCGGCATAGCTGGATCAGGCTTGCGCCCATTGTC
CAATATTCCCCACTGCTGCCTCCCGTAGGAGTCTGGACCGTATCTCAGTTCC
Sequence number 70: Anaplasma phagocytophilum 16S rRNA #70 
CCATTGTCCAATATTCCCCACTGCTGCCTCCCGTAGGAGTCTGGACCGTATCTCAGTTCCAGTGTGGC
TGATCATCCTCTCAGACCAGCTATAGATCATCGCCTTGGTAGGCCTTTACCC
Sequence number 71: Anaplasma phagocytophilum 16S rRNA #71 
AGTGTGGCTGATCATCCTCTCAGACCAGCTATAGATCATCGCCTTGGTAGGCCTTTACCCTACCAACT
AGCTAATCTAACATAGGCTCATCTAATAGCGATAAATCTTTCCCCCGCAGGG
Sequence number 72: Anaplasma phagocytophilum 16S rRNA #72 
TACCAACTAGCTAATCTAACATAGGCTCATCTAATAGCGATAAATCTTTCCCCCGCAGGGATTATACA
GTATTACCCACCATTTCTAGTGGCTATCCCATACTACTAGGTAGATTCCTAT
Sequence number 73: Anaplasma phagocytophilum 16S rRNA #73 
ATTATACAGTATTACCCACCATTTCTAGTGGCTATCCCATACTACTAGGTAGATTCCTATGCATTACT
CACCCGTCTGCCACTAACTATTCTTTATAGCAAGCTATAAAGAATAATCCGT
Sequence number 74: Anaplasma phagocytophilum 16S rRNA #74 
GCATTACTCACCCGTCTGCCACTAACTATTCTTTATAGCAAGCTATAAAGAATAATCCGTTCGACTTG
CATGTGTTAAGCTTGCCGCCAGCGTTCGTTCTGAGCCAGGATCAAACTCTCA
Sequence number 75: Anaplasma phagocytophilum 16S rRNA #75 
TCGACTTGCATGTGTTAAGCTTGCCGCCAGCGTTCGTTCTGAGCCAGGATCAAACTCTCAAGTTTGAT
TTACAATCACAAAAGCAAATTATGATCTGGACTCATGCAAAAACCAAAACAA
Sequence number 76: Anaplasma phagocytophilum 16S rRNA #76 
AGTTTGATTTACAATCACAAAAGCAAATTATGATCTGGACTCATGCAAAAACCAAAACAAAATTTAAG
CTTAAGTGCATTAAGAAAAAAATATATTAGCTATATGCACCGCCGCCTTTAT
Sequence number 77: Anaplasma phagocytophilum 16S rRNA #77 
AATTTAAGCTTAAGTGCATTAAGAAAAAAATATATTAGCTATATGCACCGCCGCCTTTATTTCTACTC
TAAATTCTCATCACCAAACTAATCAAGCACCAGAGCCATCAAGCTCTGGGCA
Sequence number 78: Anaplasma phagocytophilum 16S rRNA #78 
TTCTACTCTAAATTCTCATCACCAAACTAATCAAGCACCAGAGCCATCAAGCTCTGGGCAGGAGTATC
AGCTATAGCGCCTGTTATGTCAATAGTTTTTCATATATTTTTTTTAAACTCC
Sequence number 79: Anaplasma phagocytophilum 16S rRNA #79 
GGAGTATCAGCTATAGCGCCTGTTATGTCAATAGTTTTTCATATATTTTTTTTAAACTCCATCACACT
ACATCAATACAAACATTGTCTTTTGCCGCTAACTTACTGATAATCTCCAAGA
Sequence number 80: Anaplasma phagocytophilum 16S rRNA #80 
ATCACACTACATCAATACAAACATTGTCTTTTGCCGCTAACTTACTGATAATCTCCAAGAAATTTCTC
GCTTGTTCATCTATCATTCCCTTGATAAATACTTCTTCTCTAGAAGCAATGC
Sequence number 81: Anaplasma phagocytophilum 16S rRNA #81 
AATTTCTCGCTTGTTCATCTATCATTCCCTTGATAAATACTTCTTCTCTAGAAGCAATGCCCAATATT
AAAAAAGGAATAACTGATTTATGCTCCTCGCGAACTTCTATAATGCAACCCT
Sequence number 82: Anaplasma phagocytophilum 16S rRNA #82 
CCAATATTAAAAAAGGAATAACTGATTTATGCTCCTCGCGAACTTCTATAATGCAACCCTCAGAAAGC
GTATCTCTCCTACCATGAATTTCTAAATGATCTGCGGTAACTTTTGAAATGA
Sequence number 83: Anaplasma phagocytophilum 16S rRNA #83 
CAGAAAGCGTATCTCTCCTACCATGAATTTCTAAATGATCTGCGGTAACTTTTGAAATGATCCCAAAC
TTATCAAGTGCAATTAACAATGCATTGAGTATTTTTCTCTTAGATTCACACA
Sequence number 84: Anaplasma phagocytophilum 16S rRNA #84 
TCCCAAACTTATCAAGTGCAATTAACAATGCATTGAGTATTTTTCTCTTAGATTCACACAATTTTGAC
AGGCCAAAGAATTTTGTGACTCCTGCAGCAAATACACTCACAACACATATAA
Sequence number 85: Anaplasma phagocytophilum 16S rRNA #85 
ATTTTGACAGGCCAAAGAATTTTGTGACTCCTGCAGCAAATACACTCACAACACATATAAGTGGAAGG
TACGAAAAAATACGCTGTATCTCCTGGCTTTGAATTTCAATACCCAGCATAT
Sequence number 86: Anaplasma phagocytophilum 16S rRNA #86 
GTGGAAGGTACGAAAAAATACGCTGTATCTCCTGGCTTTGAATTTCAATACCCAGCATATTGCTAAAT
CCCACATCGATTTTGTATAAATGGCTAGATTGTGCTGATAGACGGATTCTCC
Sequence number 87: Anaplasma phagocytophilum 16S rRNA #87 TGCTAAATCCCACATCGATTTTGTATAAATGGCTAGATTGTGCTGATAGACGGATTCTCCCTCCCATT CTGATAAGAATATCCAGAAAGCTCTTAGTGGTTTTATCGAGCAAAACCCCCG
Sequence number 88: Anaplasma phagocytophilum 16S rRNA #88 
CTCCCATTCTGATAAGAATATCCAGAAAGCTCTTAGTGGTTTTATCGAGCAAAACCCCCGTAATAGTT
ATGGCTGAACCCCGTAAAACCAATGCCATTGCTACAAGATGTAGCACTCCTA
Sequence number 89: Anaplasma phagocytophilum 16S rRNA #89 
TAATAGTTATGGCTGAACCCCGTAAAACCAATGCCATTGCTACAAGATGTAGCACTCCTACTGACGAG
GCGGGTATTACCACAGTTTTTGCATAAAGCTCTTTCTGTCCCATTATTGTTA
Sequence number 90: Anaplasma phagocytophilum 16S rRNA #90 
CTGACGAGGCGGGTATTACCACAGTTTTTGCATAAAGCTCTTTCTGTCCCATTATTGTTATTTCATCA
ATATTTGGCCTCGTAACCACTGATATATCTGCACCAAAGTGCTTTAATAGTG
Sequence number 91: Anaplasma phagocytophilum 16S rRNA #91 
TTTCATCAATATTTGGCCTCGTAACCACTGATATATCTGCACCAAAGTGCTTTAATAGTGTTTCTCCA
GTGGGGGATAAAGAACTGTCAGAATATATACAATTTTTGCCTGCTATATTCA
Sequence number 92: Anaplasma phagocytophilum 16S rRNA #92 
TTTCTCCAGTGGGGGATAAAGAACTGTCAGAATATATACAATTTTTGCCTGCTATATTCATGCTACTC
AGCATCAAAGCCGTTTTGATAACATTGGAGGAAATTACTTCTTTCTCTTCTA
Sequence number 93: Anaplasma phagocytophilum 16S rRNA #93 
TGCTACTCAGCATCAAAGCCGTTTTGATAACATTGGAGGAAATTACTTCTTTCTCTTCTATAGGTAAT
GCGCAATCACTACCCTCAACCATCACTGGAAAGGCTTCACTACGTATGAAAC
Sequence number 94: Anaplasma phagocytophilum 16S rRNA #94 
TAGGTAATGCGCAATCACTACCCTCAACCATCACTGGAAAGGCTTCACTACGTATGAAACTGCATCCT
GAAGCCTTCAATATCCTAATCGCTTCGTATATCTCAGCAAAATCTAAGCCTG
Sequence number 95: Anaplasma phagocytophilum 16S rRNA #95 
TGCATCCTGAAGCCTTCAATATCCTAATCGCTTCGTATATCTCAGCAAAATCTAAGCCTGATACCTCC
ACGCTTGCATCATGGGCATTACTATTAAAAAAAAGCGAAGAAAATGCATGAG
Sequence number 96: Anaplasma phagocytophilum 16S rRNA #96 
ATACCTCCACGCTTGCATCATGGGCATTACTATTAAAAAAAAGCGAAGAAAATGCATGAGTTGATAAA
ATGCCCAGCATAGTAACGGCTATGATCGTATAGTGTCCAACGTCTATACCTC
Sequence number 97: Anaplasma phagocytophilum 16S rRNA #97 
TTGATAAAATGCCCAGCATAGTAACGGCTATGATCGTATAGTGTCCAACGTCTATACCTCCCTCGGGA
TTCGAAAAACCACCAATACCTACTCCTTCAACCGAAACACAGCCTTCCTCTA
Sequence number 98: Anaplasma phagocytophilum 16S rRNA #98 
CCTCGGGATTCGAAAAACCACCAATACCTACTCCTTCAACCGAAACACAGCCTTCCTCTACGAAAACC
CGAACACCCAAAGATCTCAAAATATCAATGACACGTGAAATCATCCTATTTT
Sequence number 99: Anaplasma phagocytophilum 16S rRNA #99 
CGAAAACCCGAACACCCAAAGATCTCAAAATATCAATGACACGTGAAATCATCCTATTTTTGCTCACA
CCGTAAATTTTTGATGTACCTACGGCTTGAGCTGCAATAATTAATGCTGTAC
Sequence number 100: Anaplasma phagocytophilum 16S rRNA #100 
TGCTCACACCGTAAATTTTTGATGTACCTACGGCTTGAGCTGCAATAATTAATGCTGTACAAGATGCA
GTTTCACACCTAGCAACATTTGCTATACCCCTTAACACAAAGCCATTATCGA
Sequence number 101: Anaplasma phagocytophilum 16S rRNA #101 
AAGATGCAGTTTCACACCTAGCAACATTTGCTATACCCCTTAACACAAAGCCATTATCGAGCATACTA
TGAGTAGAGGGACTCAGCTTATACGTGCAATATTTCACCTCTGTACACCCCA
Sequence number 102: Anaplasma phagocytophilum 16S rRNA #102 
GCATACTATGAGTAGAGGGACTCAGCTTATACGTGCAATATTTCACCTCTGTACACCCCATTTTTCAT
CACACCCCACAGTTCCTTAACGTCGTATCTATTCGATTCATGTACACCTTCT
Sequence number 103: Anaplasma phagocytophilum 16S rRNA #103 
TTTTTCATCACACCCCACAGTTCCTTAACGTCGTATCTATTCGATTCATGTACACCTTCTCGTGCCAT
AGCATACGTATAGACACCTCAGTGATGTAAATTAACTACTATTATCTAAGTA
Sequence number 104: Anaplasma phagocytophilum 16S rRNA #104 
CGTGCCATAGCATACGTATAGACACCTCAGTGATGTAAATTAACTACTATTATCTAAGTATACCCACT
AACCATTACAACCGTAACTCAGATCTTGTTCAGGATTCGCAAATGCTGAACA
Sequence number 105: Anaplasma phagocytophilum 16S rRNA #105 
TACCCACTAACCATTACAACCGTAACTCAGATCTTGTTCAGGATTCGCAAATGCTGAACACTCTAGGG
TTTCGTACTATAGATATCCACGGTGATTGATGGTAATGTCATCGACCCAGCT
Sequence number 106: Anaplasma phagocytophilum 16S rRNA #106 
CTCTAGGGTTTCGTACTATAGATATCCACGGTGATTGATGGTAATGTCATCGACCCAGCTGATATTGA
AAATCTGGGCCAATATTTCCGGTGTCACCAAAAAAGCCCCCGGAAGTTTTAT
Sequence number 107: Anaplasma phagocytophilum 16S rRNA #107 
GATATTGAAAATCTGGGCCAATATTTCCGGTGTCACCAAAAAAGCCCCCGGAAGTTTTATCAGAACAA
GATGCCCCTTGGACAAAAGCTCTATATACACTTCCGCTCCCATTGCATCCCG
Sequence number 108: Anaplasma phagocytophilum 16S rRNA #108 
CAGAACAAGATGCCCCTTGGACAAAAGCTCTATATACACTTCCGCTCCCATTGCATCCCGTTTTAACA
CTTCCTGTAACATATGTGCTATCAGAGTGTTTTGCCCTGTATGTATCGCAAT
Sequence number 109: Anaplasma phagocytophilum 16S rRNA #109 
TTTTAACACTTCCTGTAACATATGTGCTATCAGAGTGTTTTGCCCTGTATGTATCGCAATGCTTCTTA
CCCTGCGCATCATCAAGGAGATGCTCTCATGGAAATTACATATGTTTCTGCC
Sequence number 110: Anaplasma phagocytophilum 16S rRNA #110 
GCTTCTTACCCTGCGCATCATCAAGGAGATGCTCTCATGGAAATTACATATGTTTCTGCCCACTAACC
TACCCTTAGACGCATTGTAATCCATGTCTATCATGACAGGGACACCACTGTT
Sequence number 111: Anaplasma phagocytophilum 16S rRNA #111 
CACTAACCTACCCTTAGACGCATTGTAATCCATGTCTATCATGACAGGGACACCACTGTTAAATAAAT
CTCTGTTTTCTTCGATAGTCTTACTATTATAAAATGCTACGTCATTAACCCC
Sequence number 112: Anaplasma phagocytophilum 16S rRNA #112 
AAATAAATCTCTGTTTTCTTCGATAGTCTTACTATTATAAAATGCTACGTCATTAACCCCGTGTGGAT
CCGATACCCTTACAATTGCAAATCTCTCCTTATTTGCAGAGCGCATTTTAAC
Sequence number 113: Anaplasma phagocytophilum 16S rRNA #113 
GTGTGGATCCGATACCCTTACAATTGCAAATCTCTCCTTATTTGCAGAGCGCATTTTAACACTCGACA
CTACCCCAGCTCGTTTTGTCACACCAGATTCTTTCTCTTCTTGCGTGCTACT
Sequence number 114: Anaplasma phagocytophilum 16S rRNA #114 
ACTCGACACTACCCCAGCTCGTTTTGTCACACCAGATTCTTTCTCTTCTTGCGTGCTACTCACAAACC
TTACTCCGAATTTGCCTAACACCTGCTCGTACTTACTCAAAGGATGGTACCT
Sequence number 115: Anaplasma phagocytophilum 16S rRNA #115 CACAAACCTTACTCCGAATTTGCCTAACACCTGCTCGTACTTACTCAAAGGATGGTACCTTAGGTAAA AGCCTATGGTCGCAAATTCATGCTCTATTTTCTCATCTTCCGTCCAGTCATC
Sequence number 116: Anaplasma phagocytophilum 16S rRNA #116 
TAGGTAAAAGCCTATGGTCGCAAATTCATGCTCTATTTTCTCATCTTCCGTCCAGTCATCTACTTCTT
CCAGTTGCCCTTCTTCTTTTTCACTAAAAAGACTGAACTGGTGCGCATCCAT
Sequence number 117: Anaplasma phagocytophilum 16S rRNA #117 
TACTTCTTCCAGTTGCCCTTCTTCTTTTTCACTAAAAAGACTGAACTGGTGCGCATCCATGCTCACCT
TTTTGTTTTCAATAATGTTCAGTATTATGCCCAATGACGCAAAAAGCTGCCT
Sequence number 118: Anaplasma phagocytophilum 16S rRNA #118 
GCTCACCTTTTTGTTTTCAATAATGTTCAGTATTATGCCCAATGACGCAAAAAGCTGCCTACGATTAG
GATGTATGCCGTCTAGCGATCCGGATTTAATTAAACTTTCTAGAGTCCTCTT
Sequence number 119: Anaplasma phagocytophilum 16S rRNA #119 
ACGATTAGGATGTATGCCGTCTAGCGATCCGGATTTAATTAAACTTTCTAGAGTCCTCTTTAAAGGAG
ATCGTATCCTGACGTTACCCGCGAAATCCCAGATATCACTATATCGACCACC
Sequence number 120: Anaplasma phagocytophilum 16S rRNA #120 
TAAAGGAGATCGTATCCTGACGTTACCCGCGAAATCCCAGATATCACTATATCGACCACCTTCAACAG
GTAGTATCTCATGCGCCGTTAATTGACCTACATTTTTTAAGGCTCCCAATCC
Sequence number 121: Anaplasma phagocytophilum 16S rRNA #121 
TTCAACAGGTAGTATCTCATGCGCCGTTAATTGACCTACATTTTTTAAGGCTCCCAATCCATATCTAA
TCGAATTCCCTGATACGGTAAATTCTGCACTAGATAAATTAATATCAGGCGG
Sequence number 122: Anaplasma phagocytophilum 16S rRNA #122 
ATATCTAATCGAATTCCCTGATACGGTAAATTCTGCACTAGATAAATTAATATCAGGCGGCATTACAT
CTATGCCACACATTTTTGCTTGTTGGCAAAGCAAACCCAGTTTGTCTTTATC
Sequence number 123: Anaplasma phagocytophilum 16S rRNA #123 
CATTACATCTATGCCACACATTTTTGCTTGTTGGCAAAGCAAACCCAGTTTGTCTTTATCATCGATAT
CTAAGTTCATGGAGGCAGTAAAGAACTCCTTTGTGTAATTTGCGCGCAAGTA
Sequence number 124: Anaplasma phagocytophilum 16S rRNA #124 
ATCGATATCTAAGTTCATGGAGGCAGTAAAGAACTCCTTTGTGTAATTTGCGCGCAAGTACGCTGTCT
GAAAGCTTATTAACGCGTACGCAGCAGCATGCGATTTATTAAAACCATACCC
Sequence number 125: Anaplasma phagocytophilum 16S rRNA #125 
TAAGTTCATGGAGGCAGTAAAGAACTCCTTTGTGTAATTTGCGCGCAAGTACGCTGTCTGAAAGCTTA
TTAACGCGTACGCAGCAGCATGCGATTTATTAAAACCATACCCTGCAAATTT
Sequence number 126: Bacillus anthracis sspE #1 
TGGTAAAAGGAAGAATTCATTCTGTAGAGTCTTGTGGTACTGTTGATGGCCCAGGAATTCGTTATGTC
ATATTTACACAAGGGTGTTTATTACGTTGTCAATATTGTCATAATGCTGATA
Sequence number 127: Bacillus anthracis sspE #2 
GTTATGTCATATTTACACAAGGGTGTTTATTACGTTGTCAATATTGTCATAATGCTGATACGTGGGAG
ATCGGTAAAGGAAAAGAAATAACAGTCGAAGAAGTAATGCAGGATGTGACAT
Sequence number 128: Bacillus anthracis sspE #3 
CGTGGGAGATCGGTAAAGGAAAAGAAATAACAGTCGAAGAAGTAATGCAGGATGTGACATGTTACCTT
CCTTTTATTGAAGCTTCCGGAGGCGGTATAACAGTTAGTGGTGGGGAGCCAT
Sequence number 129: Bacillus anthracis sspE #4 
GTTACCTTCCTTTTATTGAAGCTTCCGGAGGCGGTATAACAGTTAGTGGTGGGGAGCCATTATTACAG
CTAGATTTCTTAATTGAATTATTTAAGAAATGTAAGGAAATTGGAATTCATA
Sequence number 130: Bacillus anthracis sspE #5 
TATTACAGCTAGATTTCTTAATTGAATTATTTAAGAAATGTAAGGAAATTGGAATTCATACAACAATT
GATTCTTCGGGTGGGTGTTATTCTGAAGAAACAGAATTCCAAAATAAGCTAG
Sequence number 131: Bacillus anthracis sspE #6 
CAACAATTGATTCTTCGGGTGGGTGTTATTCTGAAGAAACAGAATTCCAAAATAAGCTAGACATTTTA
ATGGAGTACACAGATTTAGTTTTATTGGATTTGAAACATATTGATTCAAAGA
Sequence number 132: Bacillus anthracis sspE #7 
ACATTTTAATGGAGTACACAGATTTAGTTTTATTGGATTTGAAACATATTGATTCAAAGAAACATCGT
AAATTAACAGGAAAACCAAATGAACATATTTTACAATTTGCTCGTTATTTAT
Sequence number 133: Bacillus anthracis sspE #8 
AACATCGTAAATTAACAGGAAAACCAAATGAACATATTTTACAATTTGCTCGTTATTTATCGGATAAA
AATAAACCGATTTGGGTACGACATGTATTAGTTCCTGGTGTTACTGATAATG
Sequence number 134: Bacillus anthracis sspE #9 
CGGATAAAAATAAACCGATTTGGGTACGACATGTATTAGTTCCTGGTGTTACTGATAATGAAGAGGAT
CTACAAAAGTTATCTAGCTTTATTCAAAGTCTGTCTAATGTTCAAAAAATTG
Sequence number 135: Bacillus anthracis sspE #10 
AAGAGGATCTACAAAAGTTATCTAGCTTTATTCAAAGTCTGTCTAATGTTCAAAAAATTGAAGTGTTA
CCATATCATAAGCTTGGTGTATATAAATGGGAGGCACTTGGACATAAGTATC
Sequence number 136: Bacillus anthracis sspE #11 
AAGTGTTACCATATCATAAGCTTGGTGTATATAAATGGGAGGCACTTGGACATAAGTATCCACTTGCG
AATGTAGAACCACCTACTGAAAAAAATGTAGAACAAGCAAGACATATTTTAC
Sequence number 137: Bacillus anthracis sspE #12 
CACTTGCGAATGTAGAACCACCTACTGAAAAAAATGTAGAACAAGCAAGACATATTTTACAAGCAGTC
TAATCCAAATATTGGATTAGACTTTTTGCTTTCTTTACAATAAAACCTAGAG
Sequence number 138: Bacillus anthracis sspE #13 
AAGCAGTCTAATCCAAATATTGGATTAGACTTTTTGCTTTCTTTACAATAAAACCTAGAGTATATATA
GAGAAAAGAGTATACTAAAAATAGAATATCCATATTTTTGTAAGGAATAAGT
Sequence number 139: Bacillus anthracis sspE #14 
TATATATAGAGAAAAGAGTATACTAAAAATAGAATATCCATATTTTTGTAAGGAATAAGTGGAGAAAT
TTACATAATTGTAAAGGGAAATCAAAATAGTAAAAAGAATTAAGAAGTAAAA
Sequence number 140: Bacillus anthracis sspE #15 
GGAGAAATTTACATAATTGTAAAGGGAAATCAAAATAGTAAAAAGAATTAAGAAGTAAAAGAGTGAGT
ATCATGGAATAGAGAACATTTGTAAGTTATTGCGAAAGCTCTTATAGGAAAG
Sequence number 141: Bacillus anthracis sspE #16 
GAGTGAGTATCATGGAATAGAGAACATTTGTAAGTTATTGCGAAAGCTCTTATAGGAAAGTATAATGT
AAAGATATGAGCACAGATAGGATTAAGGGGTCAATCTTATATGCTTTGCATA
Sequence number 142: Bacillus anthracis sspE #17 
TATAATGTAAAGATATGAGCACAGATAGGATTAAGGGGTCAATCTTATATGCTTTGCATATCCACGAC
AGGTACATAGCTGAAAGAGAAATATTAGAGTCTGTTTATGTAATTTTTAAGG
Sequence number 143: Bacillus anthracis sspE #18 TCCACGACAGGTACATAGCTGAAAGAGAAATATTAGAGTCTGTTTATGTAATTTTTAAGGAAAATGTT TTTCCTATTGTCGTATATGCAATATGGAGAAACAATAAGCTAGGAGTGGATT
Sequence number 144: Bacillus anthracis sspE #19 
AAAATGTTTTTCCTATTGTCGTATATGCAATATGGAGAAACAATAAGCTAGGAGTGGATTTGTTTGAT
AAAAAACCCCAAGGTTTTAATATTAACTGCACATTACGGTAACGGTCATGTG
Sequence number 145: Bacillus anthracis sspE #20 
TGTTTGATAAAAAACCCCAAGGTTTTAATATTAACTGCACATTACGGTAACGGTCATGTGCAAGTAGC
GAAAACATTAGAACAAACATTTCGCCAAAAAGGAATTAAAGATGTAATTGTA
Sequence number 146: Bacillus anthracis sspE #21 
CAAGTAGCGAAAACATTAGAACAAACATTTCGCCAAAAAGGAATTAAAGATGTAATTGTATGCGATTT
GTTCGGAGAATCACATCCGGTTATAACCGATATTACAAAATATTTATATTTA
Sequence number 147: Bacillus anthracis sspE #22 
TGCGATTTGTTCGGAGAATCACATCCGGTTATAACCGATATTACAAAATATTTATATTTAAAAAGTTA
TACAATAGGAAAAGAATTATATCGCTTGTTTTATTATGGCGTAGAAAAAATT
Sequence number 148: Bacillus anthracis sspE #23 
AAAAGTTATACAATAGGAAAAGAATTATATCGCTTGTTTTATTATGGCGTAGAAAAAATTTATGATAA
AAAAATAGCATCTTGGTATGCGAATTTTGGAAGAAAACGCTTGAAATTACTT
Sequence number 149: Bacillus anthracis sspE #24 
TATGATAAAAAAATAGCATCTTGGTATGCGAATTTTGGAAGAAAACGCTTGAAATTACTTTTACAAGC
AGAGAAACCGGATATTGTTATTAATACCTTTCCGATTATAGCTGTACCAGAA
Sequence number 150: Bacillus anthracis sspE #25 
TTACAAGCAGAGAAACCGGATATTGTTATTAATACCTTTCCGATTATAGCTGTACCAGAATTAAAAAA
GCAAACAGGTATTTCAATTCCTGTGTATAACGTATTAACGGATTTTTGTGTG
Sequence number 151: Bacillus anthracis sspE #26 
TTAAAAAAGCAAACAGGTATTTCAATTCCTGTGTATAACGTATTAACGGATTTTTGTGTGCACAAAAT
ATGGATTCATCGGGAAGTAGATCGTTATTTTGTAGCAACTGATCACGTAAAA
Sequence number 152: Bacillus anthracis sspE #27 
CACAAAATATGGATTCATCGGGAAGTAGATCGTTATTTTGTAGCAACTGATCACGTAAAAAAAGTTAT
GGTTGATATCGGTGTGCCTGCGGAGCAAATTGTTGAAACCGGAATTCCGATT
Sequence number 153: Bacillus anthracis sspE #28 
AAAGTTATGGTTGATATCGGTGTGCCTGCGGAGCAAATTGTTGAAACCGGAATTCCGATTCGTAGTAG
TTTTGAGTTAAAGATCAATCCAGACATTATATATAATAAATATCAGTTATGT
Sequence number 154: Bacillus anthracis sspE #29 
CGTAGTAGTTTTGAGTTAAAGATCAATCCAGACATTATATATAATAAATATCAGTTATGTAAGAATAA
AAAGATTTTACTAATTGTAGCAGGTGCTCATGGGGTATTAGGAAGTGTAAAA
Sequence number 155: Bacillus anthracis sspE #30 
AAGAATAAAAAGATTTTACTAATTGTAGCAGGTGCTCATGGGGTATTAGGAAGTGTAAAAGAACTATG
CCAATCATTTATGTCAGTACCTGATTTACAAGTAGTTGTCGTTTGTGGGAAA
Sequence number 156: Bacillus anthracis sspE #31 
GAACTATGCCAATCATTTATGTCAGTACCTGATTTACAAGTAGTTGTCGTTTGTGGGAAAAATGAAGC
ATTAAAACAGGATTTGGTAGGGGTACAGGAAACAAATCCTGATGCATTAAAA
Sequence number 157: Bacillus anthracis sspE #32 
AATGAAGCATTAAAACAGGATTTGGTAGGGGTACAGGAAACAAATCCTGATGCATTAAAAGTATTTGG
TTATGTTGAAAATATTGATGAACTATTCCGCGTTACTTCTTGTATGATTACA
Sequence number 158: Bacillus anthracis sspE #33 
GTATTTGGTTATGTTGAAAATATTGATGAACTATTCCGCGTTACTTCTTGTATGATTACAAAGCCAGG
CGGTATTACGTTAAGTGAGGCGGCAGCATTGCAAGTACCTGTCATTTTATAT
Sequence number 159: Bacillus anthracis sspE #34 
AAGCCAGGCGGTATTACGTTAAGTGAGGCGGCAGCATTGCAAGTACCTGTCATTTTATATAAGCCTGT
TCCAGGACAAGAAAATGAAAATGCGATGTACTTTGAAAGAAAAGGAGCTGCA
Sequence number 160: Bacillus anthracis sspE #35 
AAGCCTGTTCCAGGACAAGAAAATGAAAATGCGATGTACTTTGAAAGAAAAGGAGCTGCAGTTGTCAT
TCGTGATGATAGTGAGGTTTTTGCAAAAACAGAGGCGTTATTACAAGACGAT
Sequence number 161: Bacillus anthracis sspE #36 
GTTGTCATTCGTGATGATAGTGAGGTTTTTGCAAAAACAGAGGCGTTATTACAAGACGATATGAAGCT
TCTTCAAATGAAAGAAGCGATGAAAAGCATTTATCGTCCGGAGCCAGCTGAT
Sequence number 162: Bacillus anthracis sspE #37 
ATGAAGCTTCTTCAAATGAAAGAAGCGATGAAAAGCATTTATCGTCCGGAGCCAGCTGATCATATTGT
GGATACAATTTTGGCAGAAAATCATGTAGAACCGAATCATATACCTATTAAA
Sequence number 163: Bacillus anthracis sspE #38 
CATATTGTGGATACAATTTTGGCAGAAAATCATGTAGAACCGAATCATATACCTATTAAATCACCTGC
ACTTGCACAATCATTTACTTAATATGAAACCCTCTTTTGTATGTTTAAAGAG
Sequence number 164: Bacillus anthracis sspE #39 
TCACCTGCACTTGCACAATCATTTACTTAATATGAAACCCTCTTTTGTATGTTTAAAGAGGGTTCTTT
ATTGTTCTCTAAGATAAAGACTTATCCACATGAAGATTGCGTTTTCATTAAA
Sequence number 165: Bacillus anthracis sspE #40 
GGTTCTTTATTGTTCTCTAAGATAAAGACTTATCCACATGAAGATTGCGTTTTCATTAAACAGTAAAG
TCGGAATATTAACAACTATATCCACATTATCCACAATTTTTAGAAAAATATC
Sequence number 166: Bacillus anthracis sspE #41 
CAGTAAAGTCGGAATATTAACAACTATATCCACATTATCCACAATTTTTAGAAAAATATCCACAATTC
TAACATTTAATGTACGTTGCAATATATAAACTTGTGAAAGGTTTGGTGAATT
Sequence number 167: Bacillus anthracis sspE #42 
CACAATTCTAACATTTAATGTACGTTGCAATATATAAACTTGTGAAAGGTTTGGTGAATTTATGCACA
ATTCCATAAATTATGTGGATAACGTTATCCACATAAAAAAAGGTTCCTTATT
Sequence number 168: Bacillus anthracis sspE #43 
TATGCACAATTCCATAAATTATGTGGATAACGTTATCCACATAAAAAAAGGTTCCTTATTTATAAGGA
ACCTTTTTTCTACTCTATCACGTAGAAAAAATATGTAATGAGAATGAATAAT
Sequence number 169: Bacillus anthracis sspE #44 
TATAAGGAACCTTTTTTCTACTCTATCACGTAGAAAAAATATGTAATGAGAATGAATAATTAGGGGGG
AGAACTTCTAATTATTCATTCTCACTTTAAATACTTTTCGAGCGATAACCCG
Sequence number 170: Bacillus anthracis sspE #45 
TAGGGGGGAGAACTTCTAATTATTCATTCTCACTTTAAATACTTTTCGAGCGATAACCCGCTGCGCGA
TCTGCTTTACGAAATTCTCGTTGGTAGAGAACTTCTTGTGTACTAGATAACG
Sequence number 171: Bacillus anthracis sspE #46 CTGCGCGATCTGCTTTACGAAATTCTCGTTGGTAGAGAACTTCTTGTGTACTAGATAACGCGTTTTTT GTTTTGTCACGTTTCTTTTTATCCATTACAAATCACCTCGTGTCCGAAAGTT
Sequence number 172: Bacillus anthracis sspE #47 
CGTTTTTTGTTTTGTCACGTTTCTTTTTATCCATTACAAATCACCTCGTGTCCGAAAGTTACTATCTA
TCGTTTCCAAAGACATGGGGTGATATACGTATATATCAAAATGTTAATGGTT
Sequence number 173: Bacillus anthracis sspE #48 
ACTATCTATCGTTTCCAAAGACATGGGGTGATATACGTATATATCAAAATGTTAATGGTTAATTATTA
TTTCTGAAAATTTAGATTATGCTTCTTTTAAGTGATGTGCATCTTGGAACGT
Sequence number 174: Bacillus anthracis sspE #49 
AATTATTATTTCTGAAAATTTAGATTATGCTTCTTTTAAGTGATGTGCATCTTGGAACGTTGTATCGC
GCATTGTAGCAAGTGTATATTGATCTTTTGGAATTTCATATAAGTTATATAC
Sequence number 175: Bacillus anthracis sspE #50 
TGTATCGCGCATTGTAGCAAGTGTATATTGATCTTTTGGAATTTCATATAAGTTATATACTTCTTCAT
TCGCATTAATTTCATCATAAACAGCTTTTCTTGTTTCATTTGCTAGATTTAC
Sequence number 176: Bacillus anthracis sspE #51 
TTCTTCATTCGCATTAATTTCATCATAAACAGCTTTTCTTGTTTCATTTGCTAGATTTACATATTGTA
ATTTCTCTGCAGCTTTAATAGAACGAATATTTATTTTGCGAATACGCATAAA
Sequence number 177: Bacillus anthracis sspE #52 
ATATTGTAATTTCTCTGCAGCTTTAATAGAACGAATATTTATTTTGCGAATACGCATAAAGATTGTTT
CAATATCTAGCTCGTAGAAAAGTTCGCTAAAAAATGAATCTTTTGCCAATTT
Sequence number 178: Bacillus anthracis sspE #53 
GATTGTTTCAATATCTAGCTCGTAGAAAAGTTCGCTAAAAAATGAATCTTTTGCCAATTTATTGTAGC
CTTTTCCATGATAAGGTTTGCCAAGCCATGTTCCAAGGAATCCAGCTTTTTC
Sequence number 179: Bacillus anthracis sspE #54 
ATTGTAGCCTTTTCCATGATAAGGTTTGCCAAGCCATGTTCCAAGGAATCCAGCTTTTTCTTGCACAT
CAAATAAAGTAATTGTTCCGATAGGGTTACCCCATTCATCTAAAATTGTGCG
Sequence number 180: Bacillus anthracis sspE #55 
TTGCACATCAAATAAAGTAATTGTTCCGATAGGGTTACCCCATTCATCTAAAATTGTGCGTGAAATTA
ATTCCCCACGCTCTTCAGCTTCAATCGTTTGTTTCGTTAAAAATAAATATTC
Sequence number 181: Bacillus anthracis sspE #56 
TGAAATTAATTCCCCACGCTCTTCAGCTTCAATCGTTTGTTTCGTTAAAAATAAATATTCCTCATAAG
AATAAGCCTTTTGACGCACAAAAGGGAAGACAGCTGGATCCACCATTAACTC
Sequence number 182: Bacillus anthracis sspE #57 
CTCATAAGAATAAGCCTTTTGACGCACAAAAGGGAAGACAGCTGGATCCACCATTAACTCGTATAGAA
CGTGGCTGTCGTGTAAGTCGCGTTTTTTTAACATACTAACTCCTCCTCACAC
Sequence number 183: Bacillus anthracis sspE #58 
GTATAGAACGTGGCTGTCGTGTAAGTCGCGTTTTTTTAACATACTAACTCCTCCTCACACGAGGGTAG
CATGACGAAAAAACACCCCACCCTCGAAATTTTTATATCAATCTTTAAAAAA
Sequence number 184: Bacillus anthracis sspE #59 
GAGGGTAGCATGACGAAAAAACACCCCACCCTCGAAATTTTTATATCAATCTTTAAAAAATTTCGGGG
TGGGAATCGAACCCACTAGAACCAGTTTCTAACGCTGGTGGCGCACCATTTG
Sequence number 185: Bacillus anthracis sspE #60 
TTTCGGGGTGGGAATCGAACCCACTAGAACCAGTTTCTAACGCTGGTGGCGCACCATTTGCCTTCCCC
ATTCATCAATTTGAAAATGATAATCACGACACAAATTGATTATGGTTTCATT
Sequence number 186: Bacillus anthracis sspE #61 
CCTTCCCCATTCATCAATTTGAAAATGATAATCACGACACAAATTGATTATGGTTTCATTCAGTTTAT
AATCGTATAATACTTTATCTAGTCAAAAAAATAAACTAGTTTTTTTTATTTT
Sequence number 187: Bacillus anthracis sspE #62 
CAGTTTATAATCGTATAATACTTTATCTAGTCAAAAAAATAAACTAGTTTTTTTTATTTTTTTTGTAA
ATTATTTTTCCATCAATCATAGTTAGTACAGGCTTGGCTAAATAATGGAAAG
Sequence number 188: Bacillus anthracis sspE #63 
TTTTGTAAATTATTTTTCCATCAATCATAGTTAGTACAGGCTTGGCTAAATAATGGAAAGGATGATGG
GTCCACAGCACAAGATCAGCGTCTTTTCCGACCTCAATGCTTCCAATTCTAT
Sequence number 189: Bacillus anthracis sspE #64 
GATGATGGGTCCACAGCACAAGATCAGCGTCTTTTCCGACCTCAATGCTTCCAATTCTATCCTCTAAA
CGTAAATTTCGTGCTGGAAATATAGTGATTCCTTCTAGTGCAGTTTTTTCGT
Sequence number 190: Bacillus anthracis sspE #65 
CCTCTAAACGTAAATTTCGTGCTGGAAATATAGTGATTCCTTCTAGTGCAGTTTTTTCGTCTAATCCT
TCCCTTACAGCAACAGCAGCACAAAGATTTAAATATTGAATGGGTGTATAGG
Sequence number 191: Bacillus anthracis sspE #66 
CTAATCCTTCCCTTACAGCAACAGCAGCACAAAGATTTAAATATTGAATGGGTGTATAGGGGTGATCT
GTTGTGATGGAAACTTCCACTCCATTTTTCGATAATATATGGTAAGTATCCC
Sequence number 192: Bacillus anthracis sspE #67 
GGTGATCTGTTGTGATGGAAACTTCCACTCCATTTTTCGATAATATATGGTAAGTATCCCATGTTTTG
TTTTTAAGTTCAATTTTAGAACGGCGTGTAAGCGTGGGGCCAACTGAAACTT
Sequence number 193: Bacillus anthracis sspE #68 
ATGTTTTGTTTTTAAGTTCAATTTTAGAACGGCGTGTAAGCGTGGGGCCAACTGAAACTTTCAAATTG
TGCTTCGAAAGTTCCTCAATAATAAAGTGTCCTTCTGTACAATGTTCAATAC
Sequence number 194: Bacillus anthracis sspE #69 
TCAAATTGTGCTTCGAAAGTTCCTCAATAATAAAGTGTCCTTCTGTACAATGTTCAATACGTAAATCG
AGATTGAACTCTGTTGCAAAACGTAGAGCAGAACTAATATCATCTGCTCGGT
Sequence number 195: Bacillus anthracis sspE #70 
GTAAATCGAGATTGAACTCTGTTGCAAAACGTAGAGCAGAACTAATATCATCTGCTCGGTGAGCGTGG
ATACGTACGGGTATTTCGCGGCGTAATGCTTTTAAAATAGGAAGCATTCGAA
Sequence number 196: Bacillus anthracis sspE #71 
GAGCGTGGATACGTACGGGTATTTCGCGGCGTAATGCTTTTAAAATAGGAAGCATTCGAAAATCAGCT
TCATGCCCGTAGTGTTGTGCTTCATAAAATGATTCCCGAAGTAATCCCATAA
Sequence number 197: Bacillus anthracis sspE #72 
AATCAGCTTCATGCCCGTAGTGTTGTGCTTCATAAAATGATTCCCGAAGTAATCCCATAATTCCCATA
CGCGTAATGGACTCTTTTGTTCCATTACTATGGACTTTTTTAGGATTTTCGC
Sequence number 198: Bacillus anthracis sspE #73 
TTCCCATACGCGTAATGGACTCTTTTGTTCCATTACTATGGACTTTTTTAGGATTTTCGCCAAAGGCA
ATCTTTAAGCCAGCAGGTTCTTGAATAATCATATGATCAATACAAGTTCCGG
Sequence number 199: Bacillus anthracis sspE #74 CAAAGGCAATCTTTAAGCCAGCAGGTTCTTGAATAATCATATGATCAATACAAGTTCCGGCTGTTTTT ATTACACAAGTCGTACCACCAATAATGTTTTGACTTCCTGGCATAACGTGAA
Sequence number 200: Bacillus anthracis sspE #75 
CTGTTTTTATTACACAAGTCGTACCACCAATAATGTTTTGACTTCCTGGCATAACGTGAACAGTTGTA
ATTCCATTTTGTACAGCATCTTGAAATGCAATATCCAAAGGGTGGATTCCGT
Sequence number 201: Bacillus anthracis sspE #76 
CAGTTGTAATTCCATTTTGTACAGCATCTTGAAATGCAATATCCAAAGGGTGGATTCCGTCTAAAGAA
CGGATATGTGGTGTTGAAACTTCAGATGTTTCATTTGCATCATTGCCAGCCC
Sequence number 202: Bacillus anthracis sspE #77 
CTAAAGAACGGATATGTGGTGTTGAAACTTCAGATGTTTCATTTGCATCATTGCCAGCCCAACCAGTA
CCTTCATCGTAGAGACCAAGATGAGTATGGACATCAATAAATCCAGGTAAAA
Sequence number 203: Bacillus anthracis sspE #78 
AACCAGTACCTTCATCGTAGAGACCAAGATGAGTATGGACATCAATAAATCCAGGTAAAAGATGAAGA
GCACGTGCATCTATAACTGTCATATCTTGAGTAGGTTGAATGAAAGGCTTGA
Sequence number 204: Bacillus anthracis sspE #79 
GATGAAGAGCACGTGCATCTATAACTGTCATATCTTGAGTAGGTTGAATGAAAGGCTTGACCTCAGCA
ATTTTTTCTCCTATAACGAGTACATCCCCTTGAAATTTTTGGGATGTAATAG
Sequence number 205: Bacillus anthracis sspE #80 
CCTCAGCAATTTTTTCTCCTATAACGAGTACATCCCCTTGAAATTTTTGGGATGTAATAGGATAGACC
ATGGCTTGTTTGAGCAATATTTTCATAGGTACCTCATTTCTGTTACTTTATA
Sequence number 206: Bacillus anthracis sspE #81 
GATAGACCATGGCTTGTTTGAGCAATATTTTCATAGGTACCTCATTTCTGTTACTTTATAATGTTAAT
GGAATGTAAGGAAAAGTAACTGAACGATTATTTGTGTAATAATGCTCCGGAT
Sequence number 207: Bacillus anthracis sspE #82 
ATGTTAATGGAATGTAAGGAAAAGTAACTGAACGATTATTTGTGTAATAATGCTCCGGATTCTAGGAA
ATGAAAATTTTGGAAGTAACCCCTGTAAAACTATGTTTTAGAAGTACTACAT
Sequence number 208: Bacillus anthracis sspE #83 
TCTAGGAAATGAAAATTTTGGAAGTAACCCCTGTAAAACTATGTTTTAGAAGTACTACATTGTATGCG
AAAGTATATTTTGTAATGCATGGTCTATTGTTGGAAATGTGTATTGATACCC
Sequence number 209: Bacillus anthracis sspE #84 
TGTATGCGAAAGTATATTTTGTAATGCATGGTCTATTGTTGGAAATGTGTATTGATACCCATGCTCAA
TTGCTTTACTAGGTAATACATGTTGTCCTTCTAGTACAAGTATGCTCATCTC
Sequence number 210: Bacillus anthracis sspE #85 
ATGCTCAATTGCTTTACTAGGTAATACATGTTGTCCTTCTAGTACAAGTATGCTCATCTCACCTAATA
AAGCATGAAGCATAAATGAAGGGACAGGTAACCAATGAGGTTTTTTCATTAT
Sequence number 211: Bacillus anthracis sspE #86 
ACCTAATAAAGCATGAAGCATAAATGAAGGGACAGGTAACCAATGAGGTTTTTTCATTATAGTTGCAA
TGGTTTCTCCAAATCCCTTCATTCTTATAGGCTCTGGAGCTGTAATGTTGAG
Sequence number 212: Bacillus anthracis sspE #87 
AGTTGCAATGGTTTCTCCAAATCCCTTCATTCTTATAGGCTCTGGAGCTGTAATGTTGAGCGGGCCAT
CAATTTCTTTTTTGTGTATGATGAAATCAATCATGGAAGCGACATCGTCTAT
Sequence number 213: Bacillus anthracis sspE #88 
CGGGCCATCAATTTCTTTTTTGTGTATGATGAAATCAATCATGGAAGCGACATCGTCTATATGAATCC
ATGATAACCATTGCTTTCCAGATCCAACTGTACCTCCGATATAGAATTGATA
Sequence number 214: Bacillus anthracis sspE #89 
ATGAATCCATGATAACCATTGCTTTCCAGATCCAACTGTACCTCCGATATAGAATTGATAGGGAAGTA
GCATTTTGGGAAGGGCTCCTCCGTCTGGACCTAATATGACTCCGAATCGTGC
Sequence number 215: Bacillus anthracis sspE #90 
GGGAAGTAGCATTTTGGGAAGGGCTCCTCCGTCTGGACCTAATATGACTCCGAATCGTGCATAGATTG
TTCGTATTCCGAGAGAGCGTGCTTTAGATGCTTCTTGTTCCCATGAATATAC
Sequence number 216: Bacillus anthracis sspE #91 
ATAGATTGTTCGTATTCCGAGAGAGCGTGCTTTAGATGCTTCTTGTTCCCATGAATATACTGTATTTG
CTAAAAAGTCATTTCCGGGAGTCACATGCTGTTCTGTAAAAGACTCGGTTTC
Sequence number 217: Bacillus anthracis sspE #92 
TGTATTTGCTAAAAAGTCATTTCCGGGAGTCACATGCTGTTCTGTAAAAGACTCGGTTTCAGACGTTC
CATAGTATCCAATTGCACTCGCGTTAATAAATGTATGTGGTTTTGCAGGGAG
Sequence number 218: Bacillus anthracis sspE #93 
AGACGTTCCATAGTATCCAATTGCACTCGCGTTAATAAATGTATGTGGTTTTGCAGGGAGTGCCTGCA
ATTGTTTAATGAGTCCTTTTGTTGTTTGAATTCTACTGTTTAAAATGATTTT
Sequence number 219: Bacillus anthracis sspE #94 
TGCCTGCAATTGTTTAATGAGTCCTTTTGTTGTTTGAATTCTACTGTTTAAAATGATTTTCTTTTGTT
TCTTTGTCCATCTACTATTAATAGACTCTCCAGCTAGATTAATAACTACATC
Sequence number 220: Bacillus anthracis sspE #95 
CTTTTGTTTCTTTGTCCATCTACTATTAATAGACTCTCCAGCTAGATTAATAACTACATCAATAGAAG
AGAGAGGGAAGGTTTGTAAATCTGGTGTCCATTGCACATACTGAAGGTTAGG
Sequence number 221: Bacillus anthracis sspE #96 
AATAGAAGAGAGAGGGAAGGTTTGTAAATCTGGTGTCCATTGCACATACTGAAGGTTAGGGTGAGAAG
TTTCAGTAGTTTTTTTTCTTGTGAGAATGTAAACCGTATATCCTTTTTGAAT
Sequence number 222: Bacillus anthracis sspE #97 
GTGAGAAGTTTCAGTAGTTTTTTTTCTTGTGAGAATGTAAACCGTATATCCTTTTTGAATAAAAAAAG
TAGAAAGGTATGTGCCGATAAAGCCGGTACCACCAGAAATTGCGATTTTCAC
Sequence number 223: Bacillus anthracis sspE #98 
AAAAAAAGTAGAAAGGTATGTGCCGATAAAGCCGGTACCACCAGAAATTGCGATTTTCACAATTATTT
CCTCCTCATTATATATATTCTTGAAAAGCAAAAACGTTCCTCGACTATGTTA
Sequence number 224: Bacillus anthracis sspE #99 
AATTATTTCCTCCTCATTATATATATTCTTGAAAAGCAAAAACGTTCCTCGACTATGTTAAAATAATA
GAGAGGTGAGAGTATGGCTGTCATTACAAAAATAGAAGTGCAAAAACGATCG
Sequence number 225: Bacillus anthracis sspE #100 
AAATAATAGAGAGGTGAGAGTATGGCTGTCATTACAAAAATAGAAGTGCAAAAACGATCGAAAGAACG
GTTTAATATTTATATCGATAAAGGTCAAGGTGAAGAATACGGATTTAGTGTG
Sequence number 226: Bacillus anthracis sspE #101 
AAAGAACGGTTTAATATTTATATCGATAAAGGTCAAGGTGAAGAATACGGATTTAGTGTGAACGAAGT
AATCTTAATAAAGCACGGATTACAAAAGGGCTTAGAAATTGATGAAATAGCG
Sequence number 227: Bacillus anthracis sspE #102 AACGAAGTAATCTTAATAAAGCACGGATTACAAAAGGGCTTAGAAATTGATGAAATAGCGTTAGGAAA TATTTTGTACAATGAAGAGGTACAAAAAGCATATTTACAAGCAATCTCCTAT
Sequence number 228: Bacillus anthracis sspE #103 
TTAGGAAATATTTTGTACAATGAAGAGGTACAAAAAGCATATTTACAAGCAATCTCCTATTTATCCTA
TCAAATGAGAACGAAACTAGAAATAGAAGATTTCTTACGAAAAAAAGAAGTG
Sequence number 229: Bacillus anthracis sspE #104 
TTATCCTATCAAATGAGAACGAAACTAGAAATAGAAGATTTCTTACGAAAAAAAGAAGTGGGACAAGC
CATCATCTCTGAAGTCGTTTCGAAATTATTACATGACCGATATATTAATGAT
Sequence number 230: Bacillus anthracis sspE #105 
GGACAAGCCATCATCTCTGAAGTCGTTTCGAAATTATTACATGACCGATATATTAATGATAAAGAGTA
TGCTATTTTATATACGAGAACGCAAAGTAATGTGAATCGAAAAGGTCCAACG
Sequence number 231: Bacillus anthracis sspE #106 
AAAGAGTATGCTATTTTATATACGAGAACGCAAAGTAATGTGAATCGAAAAGGTCCAACGGTTATTAA
AAGAGAGTTGTTAAATAAAGGTGTTCAGGATCTAATTATTATGCATAGTTTA
Sequence number 232: Bacillus anthracis sspE #107 
GTTATTAAAAGAGAGTTGTTAAATAAAGGTGTTCAGGATCTAATTATTATGCATAGTTTACAAGAATA
TACGAAGGAAAAGCAAATTGAGAATGCTTTAATTCTTATAGAAAAGAAGAAA
Sequence number 233: Bacillus anthracis sspE #108 
CAAGAATATACGAAGGAAAAGCAAATTGAGAATGCTTTAATTCTTATAGAAAAGAAGAAAAAGTCTTA
TCAAAAGCATTCCTTTTTACAAATGAAACTAAAGTTAGATGAAATGCTTGTT
Sequence number 234: Bacillus anthracis sspE #109 
AAGTCTTATCAAAAGCATTCCTTTTTACAAATGAAACTAAAGTTAGATGAAATGCTTGTTCGTAAAGG
ATATTCTAGAGATGTGATTCAAATTTGTTTGGAAGAATTGAAAGACGAAAAA
Sequence number 235: Bacillus anthracis sspE #110 
CGTAAAGGATATTCTAGAGATGTGATTCAAATTTGTTTGGAAGAATTGAAAGACGAAAAAGATGACGA
AAAGCAACAAGAAGCGTTACACTATCATGGGAACAAATACTATGAGAAATAT
Sequence number 236: Bacillus anthracis sspE #111 
GATGACGAAAAGCAACAAGAAGCGTTACACTATCATGGGAACAAATACTATGAGAAATATAAGAAGTA
TGATGGATGGACGTTTGAAAATAAGATGAAACAAGCGTTATATCGTAAAGGA
Sequence number 237: Bacillus anthracis sspE #112 
AAGAAGTATGATGGATGGACGTTTGAAAATAAGATGAAACAAGCGTTATATCGTAAAGGATTCTCTAT
TGATGAGATAGAGATATTTTTGCAAATGAAACGTGAAGAGGGATGAGGGAAT
Sequence number 238: Bacillus anthracis sspE #113 
TTCTCTATTGATGAGATAGAGATATTTTTGCAAATGAAACGTGAAGAGGGATGAGGGAATAAAATGAA
TATGCCAAAACGCTACAGTGAAATGACACCATATGAGCTAAGGGAAGAAATT
Sequence number 239: Bacillus anthracis sspE #114 
AAAATGAATATGCCAAAACGCTACAGTGAAATGACACCATATGAGCTAAGGGAAGAAATTGGGGTTTT
GAAAGAGCAAGCAATAAAAGCTGAGCAACTTGGAATTGTAAATGAGTTCGAT
Sequence number 240: Bacillus anthracis sspE #115 
GGGGTTTTGAAAGAGCAAGCAATAAAAGCTGAGCAACTTGGAATTGTAAATGAGTTCGATGTATTAAT
GCGAAAGATAGCAATGGCTCGTGCTTATATGACTGACATAAATAAATTCCAT
Sequence number 241: Bacillus anthracis sspE #116 
GTATTAATGCGAAAGATAGCAATGGCTCGTGCTTATATGACTGACATAAATAAATTCCATATTGGTGA
GACGTATGAATTAGTAGAAGAACCTGGTATATTATTTGAAATTACGTATTTC
Sequence number 242: Bacillus anthracis sspE #117 
ATTGGTGAGACGTATGAATTAGTAGAAGAACCTGGTATATTATTTGAAATTACGTATTTCAATGGGGT
ATTTGCTTGGGGACATAAGCAAAATGATAATGAAGAGATTGGAATACCGATT
Sequence number 243: Bacillus anthracis sspE #118 
AATGGGGTATTTGCTTGGGGACATAAGCAAAATGATAATGAAGAGATTGGAATACCGATTTCTTTATT
ACAAGAAAAATAAAAACCAGAGAGCGAATATCTCTCTGGTTTTCATCTTTGG
Sequence number 244: Bacillus anthracis sspE #119 
TCTTTATTACAAGAAAAATAAAAACCAGAGAGCGAATATCTCTCTGGTTTTCATCTTTGGTAGGCGTT
ACAGGAGATAGGAGATTCTAAAATTGGCGTTTTTTACATTCATTCGCCAATA
Sequence number 245: Bacillus anthracis sspE #120 
TAGGCGTTACAGGAGATAGGAGATTCTAAAATTGGCGTTTTTTACATTCATTCGCCAATATAATGAGA
GACTGGGTTTGTTGCGTCTGTCCGTTAACTTGCGCTTTTGCATGTTTCGGAC
Sequence number 246: Bacillus anthracis sspE #121 
TAATGAGAGACTGGGTTTGTTGCGTCTGTCCGTTAACTTGCGCTTTTGCATGTTTCGGACGAGTCCAC
GTATGGTTAAATAATTCAGAACGACTATCTAAGCGATCACGGTAGCTCATCT
Sequence number 247: Bacillus anthracis sspE #122 
GAGTCCACGTATGGTTAAATAATTCAGAACGACTATCTAAGCGATCACGGTAGCTCATCTTTATCACC
TCGTGTAGGAAGTTGAAGTTAAATTACTTTGTACTACTCTTCCTGCTGATTT
Sequence number 248: Bacillus anthracis sspE #123 
TTATCACCTCGTGTAGGAAGTTGAAGTTAAATTACTTTGTACTACTCTTCCTGCTGATTTGCAGCACG
CATACGTTCTTGTGGGTGCGTGTTAATTGTGCCGTTAGGTCGCTTCGAAGCG
Sequence number 249: Bacillus anthracis sspE #124 
GCAGCACGCATACGTTCTTGTGGGTGCGTGTTAATTGTGCCGTTAGGTCGCTTCGAAGCGAAGCGTGA
TTTCGCTTTCGGTTGACCGTCGATTTTGCTGTTGTTTTTTGACTCAGACCAA
Sequence number 250: Bacillus anthracis sspE #125 
AAGCGTGATTTCGCTTTCGGTTGACCGTCGATTTTGCTGTTGTTTTTTGACTCAGACCAAAATTCGGC
TTGTTTACCCATTGCGAACGCCCTCCTCATCATCAGTTGATACCTCTTGAAG
Sequence number 251: Bacillus anthracis sspE #126 
AATTCGGCTTGTTTACCCATTGCGAACGCCCTCCTCATCATCAGTTGATACCTCTTGAAGAAGTATCA
ATTATAGAATGTGCAAAATAGAAGAAACTATCCTTTAGAAATGAAGGGATAA
Sequence number 252: Bacillus anthracis sspE #127 
AAGTATCAATTATAGAATGTGCAAAATAGAAGAAACTATCCTTTAGAAATGAAGGGATAATTAGGTAA
AGGAGATTAGAACATGAATGATATTTATGAAGCATTAACGAAAGAACTATTA
Sequence number 253: Bacillus anthracis sspE #128 
TTAGGTAAAGGAGATTAGAACATGAATGATATTTATGAAGCATTAACGAAAGAACTATTAGAGAAAAA
TGACAAACTTTCTTATGCACAAGCTCGTGCGTGGGTTGAATTACTGTGGGAA
Sequence number 254: Bacillus anthracis sspE #129 
GAGAAAAATGACAAACTTTCTTATGCACAAGCTCGTGCGTGGGTTGAATTACTGTGGGAAGATTTTCA
AACAACTTATGCGAAATCAGGTCGTTATCAAGGAGAAGAAATGACTGAACAA
Sequence number 255: Bacillus anthracis sspE #130 GATTTTCAAACAACTTATGCGAAATCAGGTCGTTATCAAGGAGAAGAAATGACTGAACAAGTGGTGCG ATCATGGATTAATAATCATGGAGGACGCCTTCATGAAATGCGTACAAATAAT
Sequence number 256: Bacillus anthracis sspE #131 
GTGGTGCGATCATGGATTAATAATCATGGAGGACGCCTTCATGAAATGCGTACAAATAATCCGAAATA
TAGCCATCTAATCAATCAAGAAGATCATTTGAAACATTAAAAAAAGCGACTT
Sequence number 257: Bacillus anthracis sspE #132 
CCGAAATATAGCCATCTAATCAATCAAGAAGATCATTTGAAACATTAAAAAAAGCGACTTAAGTCGCT
TTTTTTAATGTGGAAGTAACTCCAGTTTTTTTCGAAGTTTTTCTTCACTAAA
Sequence number 258: Bacillus anthracis sspE #133 
AAGTCGCTTTTTTTAATGTGGAAGTAACTCCAGTTTTTTTCGAAGTTTTTCTTCACTAAAAATCCATC
CTGTATAGGAACTAATAATATTTAAATTATGATCGAGTTGAACGATTGCAAC
Sequence number 259: Bacillus anthracis sspE #134 
AATCCATCCTGTATAGGAACTAATAATATTTAAATTATGATCGAGTTGAACGATTGCAACGAATGGAT
AATAATCTTTACTGCGATATCGCAAATCAATGAATCGAACTTCATAATAATG
Sequence number 260: Bacillus anthracis sspE #135 
GAATGGATAATAATCTTTACTGCGATATCGCAAATCAATGAATCGAACTTCATAATAATGATCATAAT
CGAAAATATCCCAGCGATACACAGGAGAGAAAGATAAAAACGCTGAGATGTT
Sequence number 261: Bacillus anthracis sspE #136 
ATCATAATCGAAAATATCCCAGCGATACACAGGAGAGAAAGATAAAAACGCTGAGATGTTTTCATCTT
GTTTAGCTGCGCGCATAATAGCATTATCTGGGAACGGTACCCGATCGAACTT
Sequence number 262: Bacillus anthracis sspE #137 
TTCATCTTGTTTAGCTGCGCGCATAATAGCATTATCTGGGAACGGTACCCGATCGAACTTGTCATATA
TCATGATATTACCACGGTGCCATCTCGCAACGTAGTAATAATCAGCCGTAAC
Sequence number 263: Bacillus anthracis sspE #138 
GTCATATATCATGATATTACCACGGTGCCATCTCGCAACGTAGTAATAATCAGCCGTAACGACTGCTA
AATGGTAATGATAAAATCGGTAAGAAGGAGAAATGATAATCTTTTCGACATT
Sequence number 264: Bacillus anthracis sspE #139 
GACTGCTAAATGGTAATGATAAAATCGGTAAGAAGGAGAAATGATAATCTTTTCGACATTTTTGAAAC
GTTTGTGTACAACGCTTCTTATATTTCTGTGCATAATAATCCGTCCGATGTA
Sequence number 265: Bacillus anthracis sspE #140 
TTTGAAACGTTTGTGTACAACGCTTCTTATATTTCTGTGCATAATAATCCGTCCGATGTAGTAAATAA
TCATTAATATATACGCTGCTAAGGCAGTATACCCTTTATGGGAACCTACGAG
Sequence number 266: Bacillus anthracis sspE #141 
GTAAATAATCATTAATATATACGCTGCTAAGGCAGTATACCCTTTATGGGAACCTACGAGCATACAAG
CAATTGCGAGTATATGGATAAAGAAAATGACGGTATCAAATGTATTAATGAT
Sequence number 267: Bacillus anthracis sspE #142 
CATACAAGCAATTGCGAGTATATGGATAAAGAAAATGACGGTATCAAATGTATTAATGATGCCGAGTG
CTACCCATTTTTTTGTGAAGGGCCGTAATGCTTGTGTACCGTAGGCATTGAA
Sequence number 268: Bacillus anthracis sspE #143 
GCCGAGTGCTACCCATTTTTTTGTGAAGGGCCGTAATGCTTGTGTACCGTAGGCATTGAAAATATCTA
CAAAGACGTGAAGAAAGACCGCAATAAATGACCAAAGTAGTAGATGAAGATA
Sequence number 269: Bacillus anthracis sspE #144 
AATATCTACAAAGACGTGAAGAAAGACCGCAATAAATGACCAAAGTAGTAGATGAAGATATGGAGCGT
CTGAAAAGAAGGCGAAAGAGATGCCACTTATAAGGAACGACCAAAGAATTAC
Sequence number 270: Bacillus anthracis sspE #145 
TGGAGCGTCTGAAAAGAAGGCGAAAGAGATGCCACTTATAAGGAACGACCAAAGAATTACTGCAGGAA
TGGAATGAGTAATCCCGCGATGATTTCTTATATATTTAGCGTTATTACGCAA
Sequence number 271: Bacillus anthracis sspE #146 
TGCAGGAATGGAATGAGTAATCCCGCGATGATTTCTTATATATTTAGCGTTATTACGCAATTTTAAAA
CTGTATCGATGTCAGGAATATTGGAACCAGCAATTGTAGCAAGCATAACAGC
Sequence number 272: Bacillus anthracis sspE #147 
TTTTAAAACTGTATCGATGTCAGGAATATTGGAACCAGCAATTGTAGCAAGCATAACAGCTTGAGGAC
CAATATCACTTTGTGCTATGGCTGGATCTAACGTGGCCAAACTGCCTAGAGT
Sequence number 273: Bacillus anthracis sspE #148 
TTGAGGACCAATATCACTTTGTGCTATGGCTGGATCTAACGTGGCCAAACTGCCTAGAGTAACGCCCA
TAACAAGGTGAGTGGCTGTGTCCATAGAAATCCACCTCCGTTTTTTATTAAT
Sequence number 274: Bacillus anthracis sspE #149 
AACGCCCATAACAAGGTGAGTGGCTGTGTCCATAGAAATCCACCTCCGTTTTTTATTAATGTAACTCT
TTTTATTCGATACCTCAAGTCCTTTGCCTTCTATTTCCTATGTCATCTTACT
Sequence number 275: Bacillus anthracis sspE #150 
GTAACTCTTTTTATTCGATACCTCAAGTCCTTTGCCTTCTATTTCCTATGTCATCTTACTTAAATTTT
TTCCTAAAATCTTTATAATAGTACTTATATGCAAAAAAATGAGGGGGATCAA
Sequence number 276: Bacillus anthracis sspE #151 
TAAATTTTTTCCTAAAATCTTTATAATAGTACTTATATGCAAAAAAATGAGGGGGATCAAGTTTGACA
CTTGAAATATTAAATAACTTTAACATAGAGCAGTTTCAAAATGATTTAATTG
Sequence number 277: Bacillus anthracis sspE #152 
GTTTGACACTTGAAATATTAAATAACTTTAACATAGAGCAGTTTCAAAATGATTTAATTGGTTGGTTT
GAAAAAGAGCAACGTGACTTACCGTGGCGTAAAAATAAAGATCCATACCGCG
Sequence number 278: Bacillus anthracis sspE #153 
GTTGGTTTGAAAAAGAGCAACGTGACTTACCGTGGCGTAAAAATAAAGATCCATACCGCGTTTGGGTT
TCGGAAATTATGTTGCAGCAAACGAGGGTAGAAGCTGTAAAACCATATTACG
Sequence number 279: Bacillus anthracis sspE #154 
TTTGGGTTTCGGAAATTATGTTGCAGCAAACGAGGGTAGAAGCTGTAAAACCATATTACGCGAATTTT
ATGGGGAAGTTTCCTACTCTAGAAGCTTTGGCCAATGCTGATGATGAAGAAG
Sequence number 280: Bacillus anthracis sspE #155 
CGAATTTTATGGGGAAGTTTCCTACTCTAGAAGCTTTGGCCAATGCTGATGATGAAGAAGTGTTAAAA
GCATGGGAAGGTTTAGGGTATTATTCTAGAGCGCGAAATTTACATGCGGCTG
Sequence number 281: Bacillus anthracis sspE #156 
TGTTAAAAGCATGGGAAGGTTTAGGGTATTATTCTAGAGCGCGAAATTTACATGCGGCTGTAAAAGAA
GTAAAAGAAGTATATGGCGGGATTGTGCCAAGTGATGTAAAGAAGATTGAGA
Sequence number 282: Bacillus anthracis sspE #157 
TAAAAGAAGTAAAAGAAGTATATGGCGGGATTGTGCCAAGTGATGTAAAGAAGATTGAGAAATTAAAA
GGAGTCGGCCCCTATACAAAAGGTGCTATTTTAAGTATCGCATATGGCATAC
Sequence number 283: Bacillus anthracis sspE #158 AATTAAAAGGAGTCGGCCCCTATACAAAAGGTGCTATTTTAAGTATCGCATATGGCATACCAGAGCCG GCAGTTGACGGAAATGTTGTGCGTGTATTGTCTCGTATTTTATCAGTATGGG
Sequence number 284: Bacillus anthracis sspE #159 
CAGAGCCGGCAGTTGACGGAAATGTTGTGCGTGTATTGTCTCGTATTTTATCAGTATGGGATGACATT
GCAAAACCGAAAACTAGAAAAGTGTTTGAAGAGATTGTGCGTGAAATTATTT
Sequence number 285: Bacillus anthracis sspE #160 
ATGACATTGCAAAACCGAAAACTAGAAAAGTGTTTGAAGAGATTGTGCGTGAAATTATTTCGGCTGAA
AATCCATCTTATTTTAATCAAGGTTTGATGGAATTAGGAGCACTGATTTGTA
Sequence number 286: Bacillus anthracis sspE #161 
CGGCTGAAAATCCATCTTATTTTAATCAAGGTTTGATGGAATTAGGAGCACTGATTTGTATTCCTAAA
AATCCAGCATGCTTACTTTGTCCTGTTCGTGAGCATTGCAGGGGATATGCTG
Sequence number 287: Bacillus anthracis sspE #162 
TTCCTAAAAATCCAGCATGCTTACTTTGTCCTGTTCGTGAGCATTGCAGGGGATATGCTGAAGGTGTT
CAAAAGGAATTACCAGTAAAAAGTAAAGCGAAAGCTCCTACGATGGTACCGA
Sequence number 288: Bacillus anthracis sspE #163 
AAGGTGTTCAAAAGGAATTACCAGTAAAAAGTAAAGCGAAAGCTCCTACGATGGTACCGATTGTTGCA
GGAGTACTTCAAACGGAAGATGGTCGTTACGTCATTAATAAACGTCCAAGTA
Sequence number 289: Bacillus anthracis sspE #164 
TTGTTGCAGGAGTACTTCAAACGGAAGATGGTCGTTACGTCATTAATAAACGTCCAAGTACCGGTTTA
TTAGCTAATATGTGGGAGTTTCCGAATGTTGAACTTGGCGAAGGGATTCGTA
Sequence number 290: Bacillus anthracis sspE #165 
CCGGTTTATTAGCTAATATGTGGGAGTTTCCGAATGTTGAACTTGGCGAAGGGATTCGTAATCAGAAG
GAACAGCTTATAGATTATATGAAGGAAAAATTTGAGCTTTCGATTTCTATTG
Sequence number 291: Bacillus anthracis sspE #166 
ATCAGAAGGAACAGCTTATAGATTATATGAAGGAAAAATTTGAGCTTTCGATTTCTATTGAAGAATAC
GCAATGAATGTACAACATACGTTTACACATCGTACTTGGGATATATTCGTAT
Sequence number 292: Bacillus anthracis sspE #167 
AAGAATACGCAATGAATGTACAACATACGTTTACACATCGTACTTGGGATATATTCGTATTTTATGGA
AAAGTAACTGGTGATATTGTTGAAACAGATACATTGAAATTTGTATCGAAAG
Sequence number 293: Bacillus anthracis sspE #168 
TTTATGGAAAAGTAACTGGTGATATTGTTGAAACAGATACATTGAAATTTGTATCGAAAGAAGCATTT
GAGCAGTTACCTTTCTCTAAATCGCATCGTACGATTTATGAGAATTGTGTTG
Sequence number 294: Bacillus anthracis sspE #169 
AAGCATTTGAGCAGTTACCTTTCTCTAAATCGCATCGTACGATTTATGAGAATTGTGTTGAGAAAATT
ACAATGCAATAAACAAAAAAAGTGTTCCCAGGTTGGGAACACTTTTTTAATC
Sequence number 295: Bacillus anthracis sspE #170 
AGAAAATTACAATGCAATAAACAAAAAAAGTGTTCCCAGGTTGGGAACACTTTTTTAATCGTAAGAAA
TTTTTGTCCCATGAGTCCAACTAGCTTCACCTTCTTGTAAGCCACCTCTTGA
Sequence number 296: Bacillus anthracis sspE #171 
GTAAGAAATTTTTGTCCCATGAGTCCAACTAGCTTCACCTTCTTGTAAGCCACCTCTTGATTCAATTT
CTATTACAATTTCTTTGTAAATACTTTGACCTTCCGCGTTTAAATAAGGCAA
Sequence number 297: Bacillus anthracis sspE #172 
TTCAATTTCTATTACAATTTCTTTGTAAATACTTTGACCTTCCGCGTTTAAATAAGGCAAAATCTGTT
GTAATGAATGATGAAAATAAGCTAATTCATCTTCTTTCCATTCGTTTTTTGA
Sequence number 298: Bacillus anthracis sspE #173 
AATCTGTTGTAATGAATGATGAAAATAAGCTAATTCATCTTCTTTCCATTCGTTTTTTGAAACCATTG
TTAGTTCAGTCATATCGCGTCCTATATACATATTCCCACCTCCATTTTCTAT
Sequence number 299: Bacillus anthracis sspE #174 
AACCATTGTTAGTTCAGTCATATCGCGTCCTATATACATATTCCCACCTCCATTTTCTATAGTTTGAA
TGAGTAACAAGAGATATATGCGGAATTTTTTCAAAAGAAAATTACGGATAGC
Sequence number 300: Bacillus anthracis sspE #175 
AGTTTGAATGAGTAACAAGAGATATATGCGGAATTTTTTCAAAAGAAAATTACGGATAGCTTCTAGTT
GAATTGGTTACATTATTGATTGTGGAGGTGAGAAAGATGAGTAAAAAACAAC
Sequence number 301: Bacillus anthracis sspE #176 
TTCTAGTTGAATTGGTTACATTATTGATTGTGGAGGTGAGAAAGATGAGTAAAAAACAACAAGGTTAT
AACAAGGCAACTTCTGGTGCTAGCATTCAAAGCACAAATGCTAGTTATGGTA
Sequence number 302: Bacillus anthracis sspE #177 
AAGGTTATAACAAGGCAACTTCTGGTGCTAGCATTCAAAGCACAAATGCTAGTTATGGTACAGAGTTT
GCGACTGAAACAAATGTACAAGCAGTAAAACAAGCAAACGCACAATCAGAAG
Sequence number 303: Bacillus anthracis sspE #178 
CAGAGTTTGCGACTGAAACAAATGTACAAGCAGTAAAACAAGCAAACGCACAATCAGAAGCTAAGAAA
GCGCAAGCTTCTGGTGCTAGCATTCAAAGCACAAATGCTAGTTATGGTACAG
Sequence number 304: Bacillus anthracis sspE #179 
CTAAGAAAGCGCAAGCTTCTGGTGCTAGCATTCAAAGCACAAATGCTAGTTATGGTACAGAATTTGCA
ACTGAAACAGACGTGCATGCTGTGAAAAAACAAAATGCACAATCAGCTGCAA
Sequence number 305: Bacillus anthracis sspE #180 
AATTTGCAACTGAAACAGACGTGCATGCTGTGAAAAAACAAAATGCACAATCAGCTGCAAAACAATCA
CAATCTTCTAGTTCAAATCAGTAATGAAAGAAAAACACTTCTCTAACTCGTG
Sequence number 306: Bacillus anthracis sspE #181 
AACAATCACAATCTTCTAGTTCAAATCAGTAATGAAAGAAAAACACTTCTCTAACTCGTGAGAAGTGT
TTTCTTTGTGCTTTGGCCATGGTAATGAAAATAAATCGAACGACATAACTTC
Sequence number 307: Bacillus anthracis sspE #182 
AGAAGTGTTTTCTTTGTGCTTTGGCCATGGTAATGAAAATAAATCGAACGACATAACTTCTAATAAGT
CAACAAATGTAGTCAAAGGAGAAGGAAGTTACAGGTTAGAAATATGTAATGA
Sequence number 308: Bacillus anthracis sspE #183 
TAATAAGTCAACAAATGTAGTCAAAGGAGAAGGAAGTTACAGGTTAGAAATATGTAATGAGGAATAAT
AACTGATAAATAAGTTCAAAAGGGGGTAAGTAATGAACGATTTTGATAAGTT
Sequence number 309: Bacillus anthracis sspE #184 
GGAATAATAACTGATAAATAAGTTCAAAAGGGGGTAAGTAATGAACGATTTTGATAAGTTGGTAGGAG
AGCAATTGGAAACGATGGATGAGTTGTTGAAATTACAAGCTCATCTAGAAAA
Sequence number 310: Bacillus anthracis sspE #185 
GGTAGGAGAGCAATTGGAAACGATGGATGAGTTGTTGAAATTACAAGCTCATCTAGAAAAGTATCAGC
AAATTGAAATGAGCGAAAAGGATACGTGCGATAAAAAGGAATTACATTTTAT
Sequence number 311: Bacillus anthracis sspE #186 GTATCAGCAAATTGAAATGAGCGAAAAGGATACGTGCGATAAAAAGGAATTACATTTTATCCGCCAGG AAATATATAGAACAGAATTAGCACTCAAGCTATTGCATGAGAAATTTGAAGA
Sequence number 312: Bacillus anthracis sspE #187 
CCGCCAGGAAATATATAGAACAGAATTAGCACTCAAGCTATTGCATGAGAAATTTGAAGAGCAAACAA
ATAGTGTGATTCAATCTTTTGAAACTGAAAAAATGATTTCAAATTTAGGATA
Sequence number 313: Bacillus anthracis sspE #188 
GCAAACAAATAGTGTGATTCAATCTTTTGAAACTGAAAAAATGATTTCAAATTTAGGATAAGATATAC
CGCTATCTTCAGATAAAAGGTCCTTCTTTACATGGAAATAAAGAAGGGCTTT
Sequence number 314: Bacillus anthracis sspE #189 
AGATATACCGCTATCTTCAGATAAAAGGTCCTTCTTTACATGGAAATAAAGAAGGGCTTTTTTTATTC
TCATTTCCTCGTTTTGCCCGTAACCTAAGAAGATTAGGAGTATGGTATTCAG
Sequence number 315: Bacillus anthracis sspE #190 
TTTTATTCTCATTTCCTCGTTTTGCCCGTAACCTAAGAAGATTAGGAGTATGGTATTCAGTGTGAAAA
ATGGAGCCTTAGCGCTTTAGTTTTAAAATTTCGACAAAAAAGTTATAATAGA
Sequence number 316: Bacillus anthracis sspE #191 
TGTGAAAAATGGAGCCTTAGCGCTTTAGTTTTAAAATTTCGACAAAAAAGTTATAATAGAAAAAAAGT
GAATGCAGCTCAAAGGGCATTTGCAGTTGAAAGAAGGGAGCATATATGGGAT
Sequence number 317: Bacillus anthracis sspE #192 
AAAAAAGTGAATGCAGCTCAAAGGGCATTTGCAGTTGAAAGAAGGGAGCATATATGGGATTTCCCAAA
GAAGGAGAAAAAGTACAAATACATAGTTATAAACATAATGGCTCCATTCATA
Sequence number 318: Bacillus anthracis sspE #193 
TTCCCAAAGAAGGAGAAAAAGTACAAATACATAGTTATAAACATAATGGCTCCATTCATAGAATGTGG
AAAGAGACAACAATTTTAAAAGGGACACAGAGTCTTGTAATCGGAGCGAATG
Sequence number 319: Bacillus anthracis sspE #194 
GAATGTGGAAAGAGACAACAATTTTAAAAGGGACACAGAGTCTTGTAATCGGAGCGAATGATCGTACA
GTAGTGACGGAATCAGATGGCCGAACATGGATTACTCGTGAACCAGCAATTT
Sequence number 320: Bacillus anthracis sspE #195 
ATCGTACAGTAGTGACGGAATCAGATGGCCGAACATGGATTACTCGTGAACCAGCAATTTGTTATTTT
CATGAAAACTATTGGTTTAATGTAATTGGAATGTTAAGGGAAGAGGGAGTAT
Sequence number 321: Bacillus anthracis sspE #196 
GTTATTTTCATGAAAACTATTGGTTTAATGTAATTGGAATGTTAAGGGAAGAGGGAGTATACTATTAT
TGTAATTTAAGCTCACCTTTTGCATATGATTCTGAAGCGTTAAAGTATATTG
Sequence number 322: Bacillus anthracis sspE #197 
ACTATTATTGTAATTTAAGCTCACCTTTTGCATATGATTCTGAAGCGTTAAAGTATATTGATTATGAT
TTAGATATTAAAGTGTATCCAGACATGACATATACGCTTTTAGATGAAGATG
Sequence number 323: Bacillus anthracis sspE #198 
ATTATGATTTAGATATTAAAGTGTATCCAGACATGACATATACGCTTTTAGATGAAGATGAGTATGAA
AAACATAGTCAAATTATGCAATATCCACCTGTTATTGATACGATTTTAAAAC
Sequence number 324: Bacillus anthracis sspE #199 
AGTATGAAAAACATAGTCAAATTATGCAATATCCACCTGTTATTGATACGATTTTAAAACGAAATGTA
GCACAATTAACGCAATGGATTCATCAAAGAAAAGGACCATTCGCGCCAGATT
Sequence number 325: Bacillus anthracis sspE #200 
GAAATGTAGCACAATTAACGCAATGGATTCATCAAAGAAAAGGACCATTCGCGCCAGATTTTGTAGAT
ATGTGGTATGAGAGATATTTAATGTACAGAAATTAACACAAACCTGCAGGGG
Sequence number 326: Bacillus anthracis sspE #201 
TTGTAGATATGTGGTATGAGAGATATTTAATGTACAGAAATTAACACAAACCTGCAGGGGATTTCCCG
GCAGGTTTGTCCTATTAGAGGGGGGATTATGTGCAAGGTATAAAAAGATATT
Sequence number 327: Bacillus anthracis sspE #202 
ATTTCCCGGCAGGTTTGTCCTATTAGAGGGGGGATTATGTGCAAGGTATAAAAAGATATTTGCAATTT
GTTAAACCATATCGATGGCTTATTGCTATTACGATTATGATTGGTCTAGTTA
Sequence number 328: Bacillus anthracis sspE #203 
TGCAATTTGTTAAACCATATCGATGGCTTATTGCTATTACGATTATGATTGGTCTAGTTAAATTCGGC
ATCCCGCTTATTATGCCGTGGTTATTAAAATATATTATTGATGATGTAATTC
Sequence number 329: Bacillus anthracis sspE #204 
AATTCGGCATCCCGCTTATTATGCCGTGGTTATTAAAATATATTATTGATGATGTAATTCAGGGCGCA
GGGTCACTTCAAGATAAAACGTCCCAACTAGTAACAGCGATTGGGATTGCTT
Sequence number 330: Bacillus anthracis sspE #205 
AGGGCGCAGGGTCACTTCAAGATAAAACGTCCCAACTAGTAACAGCGATTGGGATTGCTTTTTTTATT
TTTGCAGTATTAAGACCGCCAATTGAATATTATCGTCAATATTTCGCGCAAC
Sequence number 331: Bacillus anthracis sspE #206 
TTTTTATTTTTGCAGTATTAAGACCGCCAATTGAATATTATCGTCAATATTTCGCGCAACGTATTGCA
AATACAATACTATATGATTTGCGAAAGCATATTTTTGGTCATTTACAAAAGT
Sequence number 332: Bacillus anthracis sspE #207 
GTATTGCAAATACAATACTATATGATTTGCGAAAGCATATTTTTGGTCATTTACAAAAGTTGAGTTTA
CGCTATTATTCGAATACAAAAACAGGGGAGATTATTTCACGTGTCATTCATG
Sequence number 333: Bacillus anthracis sspE #208 
TGAGTTTACGCTATTATTCGAATACAAAAACAGGGGAGATTATTTCACGTGTCATTCATGATGTAGAA
CAAACGAAGGACTTTGTAATTACTGGTTTGATGAATGTTTGGTTAGATACGG
Sequence number 334: Bacillus anthracis sspE #209 
ATGTAGAACAAACGAAGGACTTTGTAATTACTGGTTTGATGAATGTTTGGTTAGATACGGCAACAATT
GCTATCGCTATTATTGTTATGTTTTCTATGAATATAAAATTGACATTTGTTG
Sequence number 335: Bacillus anthracis sspE #210 
CAACAATTGCTATCGCTATTATTGTTATGTTTTCTATGAATATAAAATTGACATTTGTTGCGCTATTA
ATTTTACCTATTTATGTAGTAGCAGTGAAATATTTTTTCGGGCGTCTTCGTA
Sequence number 336: Bacillus anthracis sspE #211 
CGCTATTAATTTTACCTATTTATGTAGTAGCAGTGAAATATTTTTTCGGGCGTCTTCGTAAATTGACG
AAGGAGCGTTCACAAGCGTTAGCAACGATGCAAGGATATTTACATGAACGTA
Sequence number 337: Bacillus anthracis sspE #212 
AATTGACGAAGGAGCGTTCACAAGCGTTAGCAACGATGCAAGGATATTTACATGAACGTATTCAAGGG
ATGCAAGTGACACGTAGCTTTGCATTAGAAGAGTATGAGGGGAAGCAGTTTG
Sequence number 338: Bacillus anthracis sspE #213 
TTCAAGGGATGCAAGTGACACGTAGCTTTGCATTAGAAGAGTATGAGGGGAAGCAGTTTGAAAAAAGA
AATAATGAATTTTTGACGAAAGCGTTAACGCATACGAGTTGGACGGCGAGAA
Sequence number 339: Bacillus anthracis sspE #214 AAAAAAGAAATAATGAATTTTTGACGAAAGCGTTAACGCATACGAGTTGGACGGCGAGAACATTTTCG GCAGTAAATACGTTAACAGATTTAGGGCCGTTACTTGTAATTGGTTTTGCAG
Sequence number 340: Bacillus anthracis sspE #215 
CATTTTCGGCAGTAAATACGTTAACAGATTTAGGGCCGTTACTTGTAATTGGTTTTGCAGCATATGAA
GTGATTCAGGGGCAGTTAACATTAGGGACTATGGTTGCCTTTGTCGGATATA
Sequence number 341: Bacillus anthracis sspE #216 
CATATGAAGTGATTCAGGGGCAGTTAACATTAGGGACTATGGTTGCCTTTGTCGGATATATGGATAGT
TTGTATAGTCCGCTTCGCCGCCTTGTTAATTCATCTACGACATTAACGCAGT
Sequence number 342: Bacillus anthracis sspE #217 
TGGATAGTTTGTATAGTCCGCTTCGCCGCCTTGTTAATTCATCTACGACATTAACGCAGTCTTTCGCA
TCGATGGACCGAGTGTTTGAACTGTTAGATGAAAAATACGATATTGTCAATG
Sequence number 343: Bacillus anthracis sspE #218 
CTTTCGCATCGATGGACCGAGTGTTTGAACTGTTAGATGAAAAATACGATATTGTCAATGTGCCAAGT
GCAGTACAAACGAAAAAATTAAATGGTGAAGTGATATTTGATAATGTATCTT
Sequence number 344: Bacillus anthracis sspE #219 
TGCCAAGTGCAGTACAAACGAAAAAATTAAATGGTGAAGTGATATTTGATAATGTATCTTTTCGTTAT
AATGCGGATGAAAAAGAGATATTACATAACCTTTCGTTAACTATGTTGCCAG
Sequence number 345: Bacillus anthracis sspE #220 
TTCGTTATAATGCGGATGAAAAAGAGATATTACATAACCTTTCGTTAACTATGTTGCCAGGAGAGAAG
GTAGCACTTGTAGGAGCGAGTGGGGGAGGGAAGTCATCTCTTGCTAGTTTAA
Sequence number 346: Bacillus anthracis sspE #221 
GAGAGAAGGTAGCACTTGTAGGAGCGAGTGGGGGAGGGAAGTCATCTCTTGCTAGTTTAATTCCTCGT
TTCTATGATGTTTCCTCAGGGGCAGTTTATGTAGATGGAATAGATGTCAGAA
Sequence number 347: Bacillus anthracis sspE #222 
TTCCTCGTTTCTATGATGTTTCCTCAGGGGCAGTTTATGTAGATGGAATAGATGTCAGAAAGTATGAT
ATGAGAAATTTACGTAGCCATATTGGAATTGTACTGCAAGATAATTTGTTAT
Sequence number 348: Bacillus anthracis sspE #223 
AGTATGATATGAGAAATTTACGTAGCCATATTGGAATTGTACTGCAAGATAATTTGTTATTTAGCGAT
ACAATCGGGGCAAACATTTTATATGGAAATCCGAAAGCTACAGAGGAAGAAG
Sequence number 349: Bacillus anthracis sspE #224 
TTAGCGATACAATCGGGGCAAACATTTTATATGGAAATCCGAAAGCTACAGAGGAAGAAGTAATTGCA
GCTGCTAAAGCTGCGCAAATTCATGATTTTATTATAGAGTTACCTGATGGTT
Sequence number 350: Bacillus anthracis sspE #225 
TAATTGCAGCTGCTAAAGCTGCGCAAATTCATGATTTTATTATAGAGTTACCTGATGGTTATGATACT
GTCGTTGGGGAACGTGGTGTGAAGTTATCTGGTGGTCAAAGGCAACGTGTAG
Sequence number 351: Bacillus anthracis sspE #226 
ATGATACTGTCGTTGGGGAACGTGGTGTGAAGTTATCTGGTGGTCAAAGGCAACGTGTAGCGATTGCA
CGAGTGTTTTTAAAGAATCCATCATTACTTATATTAGATGAAGCGACTCCCG
Sequence number 352: Bacillus anthracis sspE #227 
CGATTGCACGAGTGTTTTTAAAGAATCCATCATTACTTATATTAGATGAAGCGACTCCCGCTTTAGAT
TTAGAAAATGAACGATATATTCAAGAGGCGCTTCAGACGTTAGCTGCTGATC
Sequence number 353: Bacillus anthracis sspE #228 
CTTTAGATTTAGAAAATGAACGATATATTCAAGAGGCGCTTCAGACGTTAGCTGCTGATCGAACGACA
ATCATAATTGCACATCGATTGGCGACGATTACGCATGTGGATACGATTATTT
Sequence number 354: Bacillus anthracis sspE #229 
GAACGACAATCATAATTGCACATCGATTGGCGACGATTACGCATGTGGATACGATTATTTACATTGAA
GATGGTGAAATTAAAGAAACAGGTTCTCATGAAGAATTAATGAAAAAAAGAG
Sequence number 355: Bacillus anthracis sspE #230 
ACATTGAAGATGGTGAAATTAAAGAAACAGGTTCTCATGAAGAATTAATGAAAAAAAGAGGATATTAT
TACAACTTATATCAACTTCAACATATAACAGAAACAGCTCCTTTAGCGTAAA
Sequence number 356: Bacillus anthracis sspE #231 
GATATTATTACAACTTATATCAACTTCAACATATAACAGAAACAGCTCCTTTAGCGTAAAAGGAGCTG
TTTTTTATTCGTCTTCTTTTTTATCATCGATTTTAAGTTCTGCTTCTGGTTT
Sequence number 357: Bacillus anthracis sspE #232 
AGGAGCTGTTTTTTATTCGTCTTCTTTTTTATCATCGATTTTAAGTTCTGCTTCTGGTTTATGGTATG
TGTAGAAGCTATCCACAAGTAAATCTAAATGCTCTACTTCTTCACTATAGTT
Sequence number 358: Bacillus anthracis sspE #233 
ATGGTATGTGTAGAAGCTATCCACAAGTAAATCTAAATGCTCTACTTCTTCACTATAGTTAATAATGG
CAGAAATAAGAGGGAAGAGATGTAGCCATGTTTTATATGCTTCTTCATCGTC
Sequence number 359: Bacillus anthracis sspE #234 
AATAATGGCAGAAATAAGAGGGAAGAGATGTAGCCATGTTTTATATGCTTCTTCATCGTCCTTGTTTT
GATATTCCATAAAGATATCAATTAATTCTTGTTTACCTGTTTCAACTTCATC
Sequence number 360: Bacillus anthracis sspE #235 
CTTGTTTTGATATTCCATAAAGATATCAATTAATTCTTGTTTACCTGTTTCAACTTCATCAAGCATTT
CAACGGATTGTTGTTTTTTTGCTTTACCAATAAATTTTAATAAAACTTGTTC
Sequence number 361: Bacillus anthracis sspE #236 
AAGCATTTCAACGGATTGTTGTTTTTTTGCTTTACCAATAAATTTTAATAAAACTTGTTCATGGTAAT
GCGTTAATGAATCAAGTTCGTTTTGGATAGATTCTTGAAATTCTTCTGGCAT
Sequence number 362: Bacillus anthracis sspE #237 
ATGGTAATGCGTTAATGAATCAAGTTCGTTTTGGATAGATTCTTGAAATTCTTCTGGCATATAGCGCA
ATTCATTTTCAGTACGATGTAATGATTTTAACGTACTTAAAGCACGGCTTGT
Sequence number 363: Bacillus anthracis sspE #238 
ATAGCGCAATTCATTTTCAGTACGATGTAATGATTTTAACGTACTTAAAGCACGGCTTGTCGTCGCTA
ACATTTGTCTGAATAAAACGAGCTTACGAATTTTTGCGAACTGTACTTTTTT
Sequence number 364: Bacillus anthracis sspE #239 
CGTCGCTAACATTTGTCTGAATAAAACGAGCTTACGAATTTTTGCGAACTGTACTTTTTTCGTGTAAC
TTCTTTCTTCTTTATACAGCAAATAGTAATGGTTTAATTTGATCATTTTTTC
Sequence number 365: Bacillus anthracis sspE #240 
CGTGTAACTTCTTTCTTCTTTATACAGCAAATAGTAATGGTTTAATTTGATCATTTTTTCTTTCATAC
GATCAATATCAGTTTTTAACGTTGTAAAATCAGAAGCTTGCCTGCTATTCAT
Sequence number 366: Bacillus anthracis sspE #241 
TTTCATACGATCAATATCAGTTTTTAACGTTGTAAAATCAGAAGCTTGCCTGCTATTCATTCGAATCC
ATTTTACAATTTCTTCTGTGTTATCAACGATGCGATGATATAGCTTTGTTTC
Sequence number 367: Bacillus anthracis sspE #242 TCGAATCCATTTTACAATTTCTTCTGTGTTATCAACGATGCGATGATATAGCTTTGTTTCGTATTTTG GCGGCATAAATACTAAATTTACAAGTGAAGCGGCAATAATACCAACCATAAT
Sequence number 368: Bacillus anthracis sspE #243 
GTATTTTGGCGGCATAAATACTAAATTTACAAGTGAAGCGGCAATAATACCAACCATAATCGTCGCAA
ATCGAAGTAAGGCAAAACTAAAGAAATCTTCGCCCTGATATTCCATAATAGC
Sequence number 369: Bacillus anthracis sspE #244 
CGTCGCAAATCGAAGTAAGGCAAAACTAAAGAAATCTTCGCCCTGATATTCCATAATAGCGATAACAG
TTACTAAAGCGATTGATATTGTGTTTTCTAATCGTAATTGCAATGTAAGAGC
Sequence number 370: Bacillus anthracis sspE #245 
GATAACAGTTACTAAAGCGATTGATATTGTGTTTTCTAATCGTAATTGCAATGTAAGAGCAATTACTA
ATATACATGTTAATCCAATAATAAAAGGATTATGTCCAAAAGCAGTAGCGAA
Sequence number 371: Bacillus anthracis sspE #246 
AATTACTAATATACATGTTAATCCAATAATAAAAGGATTATGTCCAAAAGCAGTAGCGAATGCGATAG
CGAATACCGCACCAATGACGTTTGCTTGAATTTGTTCAAGTGCAGTTAAATA
Sequence number 372: Bacillus anthracis sspE #247 
TGCGATAGCGAATACCGCACCAATGACGTTTGCTTGAATTTGTTCAAGTGCAGTTAAATACGAACGGT
ATACAGACGGTTGAACAGCGAAGATAGCTGAGATTCCCGCAAATACCGGACT
Sequence number 373: Bacillus anthracis sspE #248 
CGAACGGTATACAGACGGTTGAACAGCGAAGATAGCTGAGATTCCCGCAAATACCGGACTCGGTAAGT
TAAGTAAAATACAAGCAAATAAAGCTAAAGTGATGGCAATACCCGTTTTTAA
Sequence number 374: Bacillus anthracis sspE #249 
CGGTAAGTTAAGTAAAATACAAGCAAATAAAGCTAAAGTGATGGCAATACCCGTTTTTAAAATGCGAG
CACCAAGTTTCATACCCTAAATAAGATCCTTTCTCTTGTCATAATGAATGTA
Sequence number 375: Bacillus anthracis sspE #250 
AATGCGAGCACCAAGTTTCATACCCTAAATAAGATCCTTTCTCTTGTCATAATGAATGTACAACATGA
TACTATACATTCATTACAATATGTTAGCAAGTGATATTTTAGTGACGTTAAT
Sequence number 376: Bacillus anthracis sspE #251 
CAACATGATACTATACATTCATTACAATATGTTAGCAAGTGATATTTTAGTGACGTTAATAAGAAATT
AAGATTCATTTTATACTTTTTCCTAATTCTTTTGAAACATCAATTTTTAGTA
Sequence number 377: Bacillus anthracis sspE #252 
AAGAAATTAAGATTCATTTTATACTTTTTCCTAATTCTTTTGAAACATCAATTTTTAGTAAAAAAACC
TGCTGAATTCCAGCAGGTTTCAAATTGTTACTATAGATTACTATGAGAAGTG
Sequence number 378: Bacillus anthracis sspE #253 
AAAAAACCTGCTGAATTCCAGCAGGTTTCAAATTGTTACTATAGATTACTATGAGAAGTGTACTTCGT
CAATTTTTTTCTTGAAGGTGAAAAATAGGTATATCACTCTGTTGAGATAGAT
Sequence number 379: Bacillus anthracis sspE #254 
TACTTCGTCAATTTTTTTCTTGAAGGTGAAAAATAGGTATATCACTCTGTTGAGATAGATGGATCAGC
CTGCTCTTTCTTCGGGATGATTTCAAAAAACTGTTGCTGCATATCATCGAGT
Sequence number 380: Bacillus anthracis sspE #255 
GGATCAGCCTGCTCTTTCTTCGGGATGATTTCAAAAAACTGTTGCTGCATATCATCGAGTAACGGTGT
TAACAAAGCAACTTCTTCATATTGTTCATGTTCATTTAATACACGGATATAA
Sequence number 381: Bacillus anthracis sspE #256 
AACGGTGTTAACAAAGCAACTTCTTCATATTGTTCATGTTCATTTAATACACGGATATAATCTAGTAA
TAACTCGTTTACATCTCGCACACCATTTTTACTAATTGGTTGCAATGTAACA
Sequence number 382: Bacillus anthracis sspE #257 
TCTAGTAATAACTCGTTTACATCTCGCACACCATTTTTACTAATTGGTTGCAATGTAACATTTGCTCC
TTTTGCGATTCTTCTTTTTAAGGCCTGTTCTGTTAAACCTAAGCTTGAATCA
Sequence number 383: Bacillus anthracis sspE #258 
TTTGCTCCTTTTGCGATTCTTCTTTTTAAGGCCTGTTCTGTTAAACCTAAGCTTGAATCATGACGTAA
ATAAACAACGCCGCCAGTCATACCAGCACAGATCCATGGACCAGGATCCCCT
Sequence number 384: Bacillus anthracis sspE #259 
TGACGTAAATAAACAACGCCGCCAGTCATACCAGCACAGATCCATGGACCAGGATCCCCTAAAACAAG
CGCTCGCCCATTTGTCATATATTCAAAAGCAAAACCTTTAATATTAGAGTAT
Sequence number 385: Bacillus anthracis sspE #260 
AAAACAAGCGCTCGCCCATTTGTCATATATTCAAAAGCAAAACCTTTAATATTAGAGTATGCTCCTAT
ATTCCCTTGCTCTTTTTCACGGAGTGGCTTTGTCATGCGCCCTCCAATTATC
Sequence number 386: Bacillus anthracis sspE #261 
GCTCCTATATTCCCTTGCTCTTTTTCACGGAGTGGCTTTGTCATGCGCCCTCCAATTATCATGTCTGC
TCCAGAGAGACGAATGCCAGCGCGAGCATCAGCGTTACCTTGAACGTATAAC
Sequence number 387: Bacillus anthracis sspE #262 
ATGTCTGCTCCAGAGAGACGAATGCCAGCGCGAGCATCAGCGTTACCTTGAACGTATAACGCTCCTTT
TTGTGCACCGTATCCAAAACCTTTTCCGACAGAACCGTTATAATATAGGCCA
Sequence number 388: Bacillus anthracis sspE #263 
GCTCCTTTTTGTGCACCGTATCCAAAACCTTTTCCGACAGAACCGTTATAATATAGGCCATCTTTTCC
TTTTGCTTTTGTTATATAAATACCACCACCAAAGGAGGTTTTACCAATACCA
Sequence number 389: Bacillus anthracis sspE #264 
TCTTTTCCTTTTGCTTTTGTTATATAAATACCACCACCAAAGGAGGTTTTACCAATACCATCTTGTGC
ACCGCCATTTACATGTATAAATAAATTTTCACTATTGTAAGCACCTAATCCA
Sequence number 390: Bacillus anthracis sspE #265 
TCTTGTGCACCGCCATTTACATGTATAAATAAATTTTCACTATTGTAAGCACCTAATCCATTTCCAGG
AATCGAGCCATTCGTATACTTTAATGTCAGTGGTTCAAGTTTTGTATCTTCA
Sequence number 391: Bacillus anthracis sspE #266 
TTTCCAGGAATCGAGCCATTCGTATACTTTAATGTCAGTGGTTCAAGTTTTGTATCTTCATATAATTT
ACTACGCACGCGATAGCAAGATTCTAGACCGCCCATAACGCGTTGCTCTACA
Sequence number 392: Bacillus anthracis sspE #267 
TATAATTTACTACGCACGCGATAGCAAGATTCTAGACCGCCCATAACGCGTTGCTCTACACCAACGCC
TACTAATTCTTTTGAATGAGGGGAATGAATGGGTTCAAATTGTTTTTCTAGT
Sequence number 393: Bacillus anthracis sspE #268 
CCAACGCCTACTAATTCTTTTGAATGAGGGGAATGAATGGGTTCAAATTGTTTTTCTAGTACGTGTTC
TGTTTTTATAACAGATGGATGTTCTATCGTTTGTAGTAAATTACATAAGTTT
Sequence number 394: Bacillus anthracis sspE #269 
ACGTGTTCTGTTTTTATAACAGATGGATGTTCTATCGTTTGTAGTAAATTACATAAGTTTAATAAATT
TTTGCCTCGAACTTGTTCTAATAAATCAGACCGCCCGACAATTTCTTGTAAG
Sequence number 395: Bacillus anthracis sspE #270 AATAAATTTTTGCCTCGAACTTGTTCTAATAAATCAGACCGCCCGACAATTTCTTGTAAGTTTGTATA GCCAAGATTTCCTGTTAATCGTTTTAGTTCTTCACCAAAAGTAGTGAATAAA
Sequence number 396: Bacillus anthracis sspE #271 
TTTGTATAGCCAAGATTTCCTGTTAATCGTTTTAGTTCTTCACCAAAAGTAGTGAATAAATGTAATAA
TCCAGCCACGGCACTTTCTAGTTCGCGTGGGACGAAGCGGCGTAATCCGTGT
Sequence number 397: Bacillus anthracis sspE #272 
TGTAATAATCCAGCCACGGCACTTTCTAGTTCGCGTGGGACGAAGCGGCGTAATCCGTGTTCTTTTGC
CTGAGCTTCAGATTCAATTTGTGTTGCAATTCCAACGTGACAAGTATCTAGA
Sequence number 398: Bacillus anthracis sspE #273 
TCTTTTGCCTGAGCTTCAGATTCAATTTGTGTTGCAATTCCAACGTGACAAGTATCTAGATGACATCC
GCGGCAAGTTGTACAGCCGATTGCAATCATGGATAATGTACCAAATCCAATG
Sequence number 399: Bacillus anthracis sspE #274 
TGACATCCGCGGCAAGTTGTACAGCCGATTGCAATCATGGATAATGTACCAAATCCAATGCGATTTGC
TCCGAGGAGCATGATTTTCAAGGCATCATTAACACTTCGAATACCACCATCA
Sequence number 400: Bacillus anthracis sspE #275 
CGATTTGCTCCGAGGAGCATGATTTTCAAGGCATCATTAACACTTCGAATACCACCATCAGCCCAAAT
TTCTACGTTATGTCTCATATTTGCTTCTAATAAAGCATTGTGAGCGGCTTTC
Sequence number 401: Bacillus anthracis sspE #276 
GCCCAAATTTCTACGTTATGTCTCATATTTGCTTCTAATAAAGCATTGTGAGCGGCTTTCACACCAAT
TTCTACAGGAAGGCCTACATGTTGCAATGCGTGAATACGTGCAGCCCCTGTA
Sequence number 402: Bacillus anthracis sspE #277 
ACACCAATTTCTACAGGAAGGCCTACATGTTGCAATGCGTGAATACGTGCAGCCCCTGTACCACCATC
AAATCCGCTAATATTAATAAAATCAGCACCAGCTTTTGCGATGCCAACAGCA
Sequence number 403: Bacillus anthracis sspE #278 
CCACCATCAAATCCGCTAATATTAATAAAATCAGCACCAGCTTTTGCGATGCCAACAGCAATTGTTCC
AATATTAGGGACAACAGGAACCTTTACAGCTACTTTCGCAAGTTGATTAGCC
Sequence number 404: Bacillus anthracis sspE #279 
ATTGTTCCAATATTAGGGACAACAGGAACCTTTACAGCTACTTTCGCAAGTTGATTAGCCGTTTTAAT
TTCTGTAATCATTTGAGCTAAATCTTCAATGGAATAAATATCATGGTTGTTA
Sequence number 405: Bacillus anthracis sspE #280 
GTTTTAATTTCTGTAATCATTTGAGCTAAATCTTCAATGGAATAAATATCATGGTTGTTAGAAGGTGA
AATTAAATCTGAGCCAATTGTTGCATTGCGTGCTTCAGCGATTTTGGCTGTT
Sequence number 406: Bacillus anthracis sspE #281 
GAAGGTGAAATTAAATCTGAGCCAATTGTTGCATTGCGTGCTTCAGCGATTTTGGCTGTTACTTTTGA
ACCTGGCAAATGTCCACCTTCACCAGGCTTTGCGCCTTGCCCAATTTTTATT
Sequence number 407: Bacillus anthracis sspE #282 
ACTTTTGAACCTGGCAAATGTCCACCTTCACCAGGCTTTGCGCCTTGCCCAATTTTTATTTCAATTAA
GTTTGATGAATTTAGTAGTTCGGCGTTTACGCCAAATCGCCCAGATGCAACT
Sequence number 408: Bacillus anthracis sspE #283 
TCAATTAAGTTTGATGAATTTAGTAGTTCGGCGTTTACGCCAAATCGCCCAGATGCAACTTGTTGTCC
ACGTGTATGTGGATATTTTCCAATCATATCTTTAATTTCGCCGCCTTCACCG
Sequence number 409: Bacillus anthracis sspE #284 
TGTTGTCCACGTGTATGTGGATATTTTCCAATCATATCTTTAATTTCGCCGCCTTCACCGTTCAAGCT
AATCATATTTAGCTGGTCGGCCGCTTCTGCATATGCACGAAAGGCAATTTCA
Sequence number 410: Bacillus anthracis sspE #285 
TTCAAGCTAATCATATTTAGCTGGTCGGCCGCTTCTGCATATGCACGAAAGGCAATTTCATTTTGAGA
GCCAAATGACATGGAACTAATAATAAATGGTAAATCATGATTTTGAATACTG
Sequence number 411: Bacillus anthracis sspE #286 
TTTTGAGAGCCAAATGACATGGAACTAATAATAAATGGTAAATCATGATTTTGAATACTGATAGATAC
CTCTTCAGTTGGAATGACATGTTTTGTTTCTTTCGTTTGAATAAGGTGTCTA
Sequence number 412: Bacillus anthracis sspE #287 
ATAGATACCTCTTCAGTTGGAATGACATGTTTTGTTTCTTTCGTTTGAATAAGGTGTCTAATCGCAAT
TGGATTATTTTCTTCTAATTCACTTAACTTGTCACGGTAATCATCATAAGAG
Sequence number 413: Bacillus anthracis sspE #288 
ATCGCAATTGGATTATTTTCTTCTAATTCACTTAACTTGTCACGGTAATCATCATAAGAGTTTCCTTT
TGCGACATCACCGATTGCTTTCCAAATTCGCGGGAATATATGAAAAGATTTT
Sequence number 414: Bacillus anthracis sspE #289 
TTTCCTTTTGCGACATCACCGATTGCTTTCCAAATTCGCGGGAATATATGAAAAGATTTTCGTATTCT
AACTTTCGGATCGTTATAATCATCCGCTCGCATTTGTGCATCTTCAGCAAGT
Sequence number 415: Bacillus anthracis sspE #290 
CGTATTCTAACTTTCGGATCGTTATAATCATCCGCTCGCATTTGTGCATCTTCAGCAAGTGATTCAAG
AGAAAAGGCTAAATCATTTGAACCTAAGAAATTTGTAATTTGCAATATATTT
Sequence number 416: Bacillus anthracis sspE #291 
GATTCAAGAGAAAAGGCTAAATCATTTGAACCTAAGAAATTTGTAATTTGCAATATATTTGCGATTTC
CTCATGTAATCCAATAGAAGAATAGAGGCGACCGTAACCACGCAATTCATGA
Sequence number 417: Bacillus anthracis sspE #292 
GCGATTTCCTCATGTAATCCAATAGAAGAATAGAGGCGACCGTAACCACGCAATTCATGAATTCCAAT
TGTTGAAATGACTTTCTCCAGTCCTTTATTAAGTGCATTGTATAAATTCGTA
Sequence number 418: Bacillus anthracis sspE #293 
ATTCCAATTGTTGAAATGACTTTCTCCAGTCCTTTATTAAGTGCATTGTATAAATTCGTAATTGGTGT
TTTTGTCCCATCATTTACGGTTGCAAATAATAAATAGGGATTAATACTATCT
Sequence number 419: Bacillus anthracis sspE #294 
ATTGGTGTTTTTGTCCCATCATTTACGGTTGCAAATAATAAATAGGGATTAATACTATCTGCTCCTAA
TCCATGCAGTGTGACGATATCGTGTAAGGAGCGAAGGGCACCCGAGCGTATA
Sequence number 420: Bacillus anthracis sspE #295 
GCTCCTAATCCATGCAGTGTGACGATATCGTGTAAGGAGCGAAGGGCACCCGAGCGTATAACGAGAGA
GCAGTTTCGACGTAATTTATTTTCGCTTAATACTTGATGTACTTTAGCAGTA
Sequence number 421: Bacillus anthracis sspE #296 
ACGAGAGAGCAGTTTCGACGTAATTTATTTTCGCTTAATACTTGATGTACTTTAGCAGTAACTAAATG
TGGATCAATCCATAACCGTTCATTTAGATGAGCATCGGCATCGTCTATTAAT
Sequence number 422: Bacillus anthracis sspE #297 
ACTAAATGTGGATCAATCCATAACCGTTCATTTAGATGAGCATCGGCATCGTCTATTAATAGTAGGGA
AGCTCCATCGTTCACGGCTGTTATCGCTTCAGAACTAATTCGACTTAAGGCG
Sequence number 423: Bacillus anthracis sspE #298 AGTAGGGAAGCTCCATCGTTCACGGCTGTTATCGCTTCAGAACTAATTCGACTTAAGGCGCATTGTAA AGTTTCTGCCTGACTGAATGTAGCAGAAATGGTAGCGATAGAGCTATGAGTG
Sequence number 424: Bacillus anthracis sspE #299 
CATTGTAAAGTTTCTGCCTGACTGAATGTAGCAGAAATGGTAGCGATAGAGCTATGAGTGTTGAATAG
TTCTATAAGTTGTTCATACGTAATTGTGTGTAATTCCTGTGCAATAGATTGT
Sequence number 425: Bacillus anthracis sspE #300 
TTGAATAGTTCTATAAGTTGTTCATACGTAATTGTGTGTAATTCCTGTGCAATAGATTGTGCCACGCT
TCCTTCTAAAATAATGGGGGAAAGCATCTCAACTACAAATGGTTTTTTCGCA
Sequence number 426: Bacillus anthracis sspE #301 
GCCACGCTTCCTTCTAAAATAATGGGGGAAAGCATCTCAACTACAAATGGTTTTTTCGCATCGCTTAA
TAAACTTGGTCGCTCTCCAAGTATCGTTCGTGTGGAGAAGTGTTCGACCTCT
Sequence number 427: Bacillus anthracis sspE #302 
TCGCTTAATAAACTTGGTCGCTCTCCAAGTATCGTTCGTGTGGAGAAGTGTTCGACCTCTCGATCGCG
ATCGATTGCTGGGTTTGTAACAACAGCTACACTTTCCTTAATGAAGTCAGCG
Sequence number 428: Bacillus anthracis sspE #303 
CGATCGCGATCGATTGCTGGGTTTGTAACAACAGCTACACTTTCCTTAATGAAGTCAGCGATATTTCG
TCTATCAGTATCGAGTGCGGCAAGTGGTGAGTCATGCCCAAGTGAGCGGATT
Sequence number 429: Bacillus anthracis sspE #304 
ATATTTCGTCTATCAGTATCGAGTGCGGCAAGTGGTGAGTCATGCCCAAGTGAGCGGATTGGCTCAAC
GCCTTTTGTTGCCATCTGTTCAAGAAGCTGAATATGTTCTCGGTCCCATCCG
Sequence number 430: Bacillus anthracis sspE #305 
GGCTCAACGCCTTTTGTTGCCATCTGTTCAAGAAGCTGAATATGTTCTCGGTCCCATCCGAAAGCAAC
ATATTGTTTCGTTTCAACTTGCATAGAATCACATGCATCTGGAATTTCTTTC
Sequence number 431: Bacillus anthracis sspE #306 
AAAGCAACATATTGTTTCGTTTCAACTTGCATAGAATCACATGCATCTGGAATTTCTTTCGTTTTAGG
GATAGATAAATGTAAATGATAATTGGTAACATCCACTTTCTTTTTAAAACGG
Sequence number 432: Bacillus anthracis sspE #307 
GTTTTAGGGATAGATAAATGTAAATGATAATTGGTAACATCCACTTTCTTTTTAAAACGGTTATATAC
TTGAGTTTGGTAGTCGTCATGTTCGTAGAGTACAAGTTCATCTTGTTCATTC
Sequence number 433: Bacillus anthracis sspE #308 
TTATATACTTGAGTTTGGTAGTCGTCATGTTCGTAGAGTACAAGTTCATCTTGTTCATTCCACTTTAG
GCCGACTTTTTCTCCTGGAGCGAGTGGTTTTGGTTCTGCTACATATTCAGTT
Sequence number 434: Bacillus anthracis sspE #309 
CACTTTAGGCCGACTTTTTCTCCTGGAGCGAGTGGTTTTGGTTCTGCTACATATTCAGTTGGTGATAC
AACTCCAGGTTCAGAAGAGAAAATATAAGAGCTTTCTATTTCAACCTTCCAT
Sequence number 435: Bacillus anthracis sspE #310 
GGTGATACAACTCCAGGTTCAGAAGAGAAAATATAAGAGCTTTCTATTTCAACCTTCCATACTGGTCG
AAGTCCAAGGGAATCCACGCTGAAAACAGCTTCATCTTTATAACGTGAAATA
Sequence number 436: Bacillus anthracis sspE #311 
ACTGGTCGAAGTCCAAGGGAATCCACGCTGAAAACAGCTTCATCTTTATAACGTGAAATAATGCCAGC
TGGACCTTGCGCAAAATGGCCCCATGCTTCACGTATATACGTATATAAATCT
Sequence number 437: Bacillus anthracis sspE #312 
ATGCCAGCTGGACCTTGCGCAAAATGGCCCCATGCTTCACGTATATACGTATATAAATCTTGTAAATG
TGTTTCATAAAGTTTAATTTCATTAATAATTGGTGGGAATAGTATATCCATT
Sequence number 438: Bacillus anthracis sspE #313 
TGTAAATGTGTTTCATAAAGTTTAATTTCATTAATAATTGGTGGGAATAGTATATCCATTGCTTCAAA
TAAACTGTAGCCGTATTGAACAAGTAGAGTTTCTAAGGTGCGGTTTAAATCT
Sequence number 439: Bacillus anthracis sspE #314 
GCTTCAAATAAACTGTAGCCGTATTGAACAAGTAGAGTTTCTAAGGTGCGGTTTAAATCTTGGGAGTC
ACTGCCCCCAGCAGTAAGGGGAACGTGAATCATTTCAGCTTGATCGCGTAAT
Sequence number 440: Bacillus anthracis sspE #315 
TGGGAGTCACTGCCCCCAGCAGTAAGGGGAACGTGAATCATTTCAGCTTGATCGCGTAATTTTGCAAT
TGTGTTAATTTCACCATTATGTCCGAGAATACTAAATGGTTGTACACGAAAA
Sequence number 441: Bacillus anthracis sspE #316 
TTTGCAATTGTGTTAATTTCACCATTATGTCCGAGAATACTAAATGGTTGTACACGAAAAAAATTAGA
TAATGTATTAGTAGAATAGCGATTGTGTCCTAGTGTCATAGTTGAAGCGATA
Sequence number 442: Bacillus anthracis sspE #317 
AAATTAGATAATGTATTAGTAGAATAGCGATTGTGTCCTAGTGTCATAGTTGAAGCGATAAGTGGGTG
TTGTAAATCATTGTAATAGGCAACAAGTGTATCCCCAGCACCGAGAACTTTA
Sequence number 443: Bacillus anthracis sspE #318 
AGTGGGTGTTGTAAATCATTGTAATAGGCAACAAGTGTATCCCCAGCACCGAGAACTTTATATACAAC
GTGATCACGACTTAAGGAAGCAACATGAATCGCTTCGTTTTCCTCTATTTTT
Sequence number 444: Bacillus anthracis sspE #319 
TATACAACGTGATCACGACTTAAGGAAGCAACATGAATCGCTTCGTTTTCCTCTATTTTTATCGTTAC
TGAAAATAAGGTTTGCTCAATATTTACAACTTCATTTTCTGCGATAAGGGCA
Sequence number 445: Bacillus anthracis sspE #320 
ATCGTTACTGAAAATAAGGTTTGCTCAATATTTACAACTTCATTTTCTGCGATAAGGGCAACTTGCCA
AAATAAAGGTTCTTCTTGTTTACCAAGTGGACCGAGTGCATCGGAGTTTATT
Sequence number 446: Bacillus anthracis sspE #321 
ACTTGCCAAAATAAAGGTTCTTCTTGTTTACCAAGTGGACCGAGTGCATCGGAGTTTATTACATTGTC
ACTTTCGAAAAGAATTGCAAAGTTTTCGTTGTTTAGTTGTGTACGAATATGA
Sequence number 447: Bacillus anthracis sspE #322 
ACATTGTCACTTTCGAAAAGAATTGCAAAGTTTTCGTTGTTTAGTTGTGTACGAATATGATTTTTTAA
ATCAACTATATTCTCGTTTTTGTTTAGAAAAAAATGTCCAACGATAAAGTGG
Sequence number 448: Bacillus anthracis sspE #323 
TTTTTTAAATCAACTATATTCTCGTTTTTGTTTAGAAAAAAATGTCCAACGATAAAGTGGGGATGATC
CACGATCGTTGGATTATATCCGCTACTTTTTAATTTTTCATTCCAAAGAGCT
Sequence number 449: Bacillus anthracis sspE #324 
GGATGATCCACGATCGTTGGATTATATCCGCTACTTTTTAATTTTTCATTCCAAAGAGCTTTTGGTAC
ATCAATGTGAATGCCGATTCCATCACCTTCGCCATTAATGAAGCCAGCTCTA
Sequence number 450: Bacillus anthracis sspE #325 
TTTGGTACATCAATGTGAATGCCGATTCCATCACCTTCGCCATTAATGAAGCCAGCTCTATGATTCAT
TTTGACGAGCGATTGTATGCAGAGGTCAATGTTTTCTTTTGTAGGAATTTTT
Sequence number 451: Bacillus anthracis sspE #326 TGATTCATTTTGACGAGCGATTGTATGCAGAGGTCAATGTTTTCTTTTGTAGGAATTTTTCGTTTCTC CATAACGGAAACGATTCCACACGCATCATGTTCTGCATTTTTATAATCTCGA
Sequence number 452: Bacillus anthracis sspE #327 
CGTTTCTCCATAACGGAAACGATTCCACACGCATCATGTTCTGCATTTTTATAATCTCGAAATAAAGA
CGGTGTCCATGTATTTGTTTTATTGAGCATCCGTCAATCCCCCCTCTTGTAA
Sequence number 453: Bacillus anthracis sspE #328 
AATAAAGACGGTGTCCATGTATTTGTTTTATTGAGCATCCGTCAATCCCCCCTCTTGTAAAAGTTAGA
ATAATCTTACTATTTAAGGTGTAGATCACCTTGTACACAGAGATAGTAGTGA
Sequence number 454: Bacillus anthracis sspE #329 
AAGTTAGAATAATCTTACTATTTAAGGTGTAGATCACCTTGTACACAGAGATAGTAGTGATGTAAAAG
AATATAAAACAGATAATTATGAATAATTATACTATTAATACGGGATTTATGA
Sequence number 455: Bacillus anthracis sspE #330 
TGTAAAAGAATATAAAACAGATAATTATGAATAATTATACTATTAATACGGGATTTATGAAATAGAAA
AAAGGAAAAAAGAAAGATGTGCTTTCTTTTTTTCCTTTTAATGATGAGATTA
Sequence number 456: Bacillus anthracis sspE #331 
AATAGAAAAAAGGAAAAAAGAAAGATGTGCTTTCTTTTTTTCCTTTTAATGATGAGATTATTTATTGT
CTGCTAAAGCAGCAAATGCTCTGCCTACAGCTTCAATTGTATATTCAATATC
Sequence number 457: Bacillus anthracis sspE #332 
TTTATTGTCTGCTAAAGCAGCAAATGCTCTGCCTACAGCTTCAATTGTATATTCAATATCTTCTTTTG
TATGCTCTGTTGTTAAGAACCATGCCTCGTATTTAGAAGGTGCTAAGTTAAC
Sequence number 458: Bacillus anthracis sspE #333 
TTCTTTTGTATGCTCTGTTGTTAAGAACCATGCCTCGTATTTAGAAGGTGCTAAGTTAACGCCTTCTT
GAAGCATTAGCTTGAAGAATTTGCCGAACATTTCCCCATCTGTATCTTGTGC
Sequence number 459: Bacillus anthracis sspE #334 
GCCTTCTTGAAGCATTAGCTTGAAGAATTTGCCGAACATTTCCCCATCTGTATCTTGTGCTGCATCGT
AATCTTCAATTGTATTTGTTGTGAAGTATACAGTTAAAGCACCTTTTAGACG
Sequence number 460: Bacillus anthracis sspE #335 
TGCATCGTAATCTTCAATTGTATTTGTTGTGAAGTATACAGTTAAAGCACCTTTTAGACGGTTTAATG
TAATATCGATATTATGTTTTGCAGCTTGCTCTAAAATTCCTTTTTCAAGCAT
Sequence number 461: Bacillus anthracis sspE #336 
GTTTAATGTAATATCGATATTATGTTTTGCAGCTTGCTCTAAAATTCCTTTTTCAAGCATTGCACCAA
GCTCATCTAATTTCTCATAAAGCCCTTCTTGTTGAAGAACTTCTAGACAAGC
Sequence number 462: Bacillus anthracis sspE #337 
TGCACCAAGCTCATCTAATTTCTCATAAAGCCCTTCTTGTTGAAGAACTTCTAGACAAGCAATACCAG
ATGCCATAGAAGCAGGATTTCCTGCCATTGTACCAGCTTGATATGCAGGTCC
Sequence number 463: Bacillus anthracis sspE #338 
AATACCAGATGCCATAGAAGCAGGATTTCCTGCCATTGTACCAGCTTGATATGCAGGTCCGAGCGGGG
CAACTTGTTCCATAATTTCTTTCTTACCACCGTAAGCACCGATTGGTAGTCC
Sequence number 464: Bacillus anthracis sspE #339 
GAGCGGGGCAACTTGTTCCATAATTTCTTTCTTACCACCGTAAGCACCGATTGGTAGTCCGCCGCCGA
TAACTTTACCAAGCGCTGTTAAGTCTGGTGTTACGCCAAGTAAGTCTTGAGC
Sequence number 465: Bacillus anthracis sspE #340 
GCCGCCGATAACTTTACCAAGCGCTGTTAAGTCTGGTGTTACGCCAAGTAAGTCTTGAGCGCCACCGT
ACATGAAGCGGAAAGCGGTAATAACTTCATCATAAATTACTAATGCACCAGC
Sequence number 466: Bacillus anthracis sspE #341 
GCCACCGTACATGAAGCGGAAAGCGGTAATAACTTCATCATAAATTACTAATGCACCAGCTTCATGAA
CAAGTTCATTCACTTTCTCAAGGAAGCCAGGTTTTGGCTCTACAATACCGAA
Sequence number 467: Bacillus anthracis sspE #342 
TTCATGAACAAGTTCATTCACTTTCTCAAGGAAGCCAGGTTTTGGCTCTACAATACCGAAGTTTCCAA
CAATCGGTTCTACAAGAATAGCTGCTACCTCATGTCCCCATTTATCTAATGC
Sequence number 468: Bacillus anthracis sspE #343 
GTTTCCAACAATCGGTTCTACAAGAATAGCTGCTACCTCATGTCCCCATTTATCTAATGCTTCTTTTA
AAGTTTCTACATTATTAAATGGAACAGTAATAACTTCTTGAGCGATACTTTG
Sequence number 469: Bacillus anthracis sspE #344 
TTCTTTTAAAGTTTCTACATTATTAAATGGAACAGTAATAACTTCTTGAGCGATACTTTGTGGTACAC
CGGCTGAGTCAGGGGTTCCTAGTGTAGAAGGGCCAGATCCAGCTGCTACAAG
Sequence number 470: Bacillus anthracis sspE #345 
TGGTACACCGGCTGAGTCAGGGGTTCCTAGTGTAGAAGGGCCAGATCCAGCTGCTACAAGTACTAAAT
CAGAATGACCATGGTAACAACCAGCAAATTTCATAATTTTTGTGCGGCCTGT
Sequence number 471: Bacillus anthracis sspE #346 
TACTAAATCAGAATGACCATGGTAACAACCAGCAAATTTCATAATTTTTGTGCGGCCTGTGTAAGCAC
GAGCGACACGAATTGTTGTCATTACTGCTTCAGTACCAGAGTTTACGAAGCG
Sequence number 472: Bacillus anthracis sspE #347 
GTAAGCACGAGCGACACGAATTGTTGTCATTACTGCTTCAGTACCAGAGTTTACGAAGCGGACTTTAT
CTAATGCAGGCATTGCTTCTTTTAACATTTTTGCAAATTTTACTTCTAGTGC
Sequence number 473: Bacillus anthracis sspE #348 
GACTTTATCTAATGCAGGCATTGCTTCTTTTAACATTTTTGCAAATTTTACTTCTAGTGCTGTTGGTG
TTCCGTAAAGTACACCATTTTCAGCAGCAGTTGTAATTGCTTTTGTGATGTG
Sequence number 474: Bacillus anthracis sspE #349 
TGTTGGTGTTCCGTAAAGTACACCATTTTCAGCAGCAGTTGTAATTGCTTTTGTGATGTGTGGGTGAG
CGTGTCCAGTAATAATAGGACCGTATGCTGCTAAATAATCGATATATTTATT
Sequence number 475: Bacillus anthracis sspE #350 
TGGGTGAGCGTGTCCAGTAATAATAGGACCGTATGCTGCTAAATAATCGATATATTTATTTCCGTCTA
CATCCCAGAAATAAGCACCTTTTCCGCGTTCCATAGCAATAGGAGCGCCGCC
Sequence number 476: Bacillus anthracis sspE #351 
TCCGTCTACATCCCAGAAATAAGCACCTTTTCCGCGTTCCATAGCAATAGGAGCGCCGCCGCCAACTG
CTTTAAAAGAACGAGAAGGACTATTAACGCCGCCAACGATATGTTCTAAAGC
Sequence number 477: Bacillus anthracis sspE #352 
AGCAATAGGAGCGCCGCCGCCAACTGCTTTAAAAGAACGAGAAGGACTATTAACGCCGCCAACGATAT
GTTCTAAAGCTTCTTTATGTAATGCTTCTGATTTTGTGAATTTCACTACCAT
Sequence number 478: Bacillus anthracis pagA #1 
TGAAAAAACGAAAAGTGTTAATACCATTAATGGCATTGTCTACGATATTAGTTTCAAGCACAGGTAAT
TTAGAGGTGATTCAGGCAGAAGTTAAACAGGAGAACCGGTTATTAAATGAAT
Sequence number 479: Bacillus anthracis pagA #2 CAGGTAATTTAGAGGTGATTCAGGCAGAAGTTAAACAGGAGAACCGGTTATTAAATGAATCAGAATCA AGTTCCCAGGGGTTACTAGGATACTATTTTAGTGATTTGAATTTTCAAGCAC
Sequence number 480: Bacillus anthracis pagA #3 
CAGAATCAAGTTCCCAGGGGTTACTAGGATACTATTTTAGTGATTTGAATTTTCAAGCACCCATGGTG
GTTACCTCTTCTACTACAGGGGATTTATCTATTCCTAGTTCTGAGTTAGAAA
Sequence number 481: Bacillus anthracis pagA #4 
CCATGGTGGTTACCTCTTCTACTACAGGGGATTTATCTATTCCTAGTTCTGAGTTAGAAAATATTCCA
TCGGAAAACCAATATTTTCAATCTGCTATTTGGTCAGGATTTATCAAAGTTA
Sequence number 482: Bacillus anthracis pagA #5 
ATATTCCATCGGAAAACCAATATTTTCAATCTGCTATTTGGTCAGGATTTATCAAAGTTAAGAAGAGT
GATGAATATACATTTGCTACTTCCGCTGATAATCATGTAACAATGTGGGTAG
Sequence number 483: Bacillus anthracis pagA #6 
AGAAGAGTGATGAATATACATTTGCTACTTCCGCTGATAATCATGTAACAATGTGGGTAGATGACCAA
GAAGTGATTAATAAAGCTTCTAATTCTAACAAAATCAGATTAGAAAAAGGAA
Sequence number 484: Bacillus anthracis pagA #7 
ATGACCAAGAAGTGATTAATAAAGCTTCTAATTCTAACAAAATCAGATTAGAAAAAGGAAGATTATAT
CAAATAAAAATTCAATATCAACGAGAAAATCCTACTGAAAAAGGATTGGATT
Sequence number 485: Bacillus anthracis pagA #8 
GATTATATCAAATAAAAATTCAATATCAACGAGAAAATCCTACTGAAAAAGGATTGGATTTCAAGTTG
TACTGGACCGATTCTCAAAATAAAAAAGAAGTGATTTCTAGTGATAACTTAC
Sequence number 486: Bacillus anthracis pagA #9 
TCAAGTTGTACTGGACCGATTCTCAAAATAAAAAAGAAGTGATTTCTAGTGATAACTTACAATTGCCA
GAATTAAAACAAAAATCTTCGAACTCAAGAAAAAAGCGAAGTACAAGTGCTG
Sequence number 487: Bacillus anthracis pagA #10 
AATTGCCAGAATTAAAACAAAAATCTTCGAACTCAAGAAAAAAGCGAAGTACAAGTGCTGGACCTACG
GTTCCAGACCGTGACAATGATGGAATCCCTGATTCATTAGAGGTAGAAGGAT
Sequence number 488: Bacillus anthracis pagA #11 
GACCTACGGTTCCAGACCGTGACAATGATGGAATCCCTGATTCATTAGAGGTAGAAGGATATACGGTT
GATGTCAAAAATAAAAGAACTTTTCTTTCACCATGGATTTCTAATATTCATG
Sequence number 489: Bacillus anthracis pagA #12 
ATACGGTTGATGTCAAAAATAAAAGAACTTTTCTTTCACCATGGATTTCTAATATTCATGAAAAGAAA
GGATTAACCAAATATAAATCATCTCCTGAAAAATGGAGCACGGCTTCTGATC
Sequence number 490: Bacillus anthracis pagA #13 
AAAAGAAAGGATTAACCAAATATAAATCATCTCCTGAAAAATGGAGCACGGCTTCTGATCCGTACAGT
GATTTCGAAAAGGTTACAGGACGGATTGATAAGAATGTATCACCAGAGGCAA
Sequence number 491: Bacillus anthracis pagA #14 
CGTACAGTGATTTCGAAAAGGTTACAGGACGGATTGATAAGAATGTATCACCAGAGGCAAGACACCCC
CTTGTGGCAGCTTATCCGATTGTACATGTAGATATGGAGAATATTATTCTCT
Sequence number 492: Bacillus anthracis pagA #15 
GACACCCCCTTGTGGCAGCTTATCCGATTGTACATGTAGATATGGAGAATATTATTCTCTCAAAAAAT
GAGGATCAATCCACACAGAATACTGATAGTCAAACGAGAACAATAAGTAAAA
Sequence number 493: Bacillus anthracis pagA #16 
CAAAAAATGAGGATCAATCCACACAGAATACTGATAGTCAAACGAGAACAATAAGTAAAAATACTTCT
ACAAGTAGGACACATACTAGTGAAGTACATGGAAATGCAGAAGTGCATGCGT
Sequence number 494: Bacillus anthracis pagA #17 
ATACTTCTACAAGTAGGACACATACTAGTGAAGTACATGGAAATGCAGAAGTGCATGCGTCGTTCTTT
GATATTGGTGGGAGTGTATCTGCAGGATTTAGTAATTCGAATTCAAGTACGG
Sequence number 495: Bacillus anthracis pagA #18 
CGTTCTTTGATATTGGTGGGAGTGTATCTGCAGGATTTAGTAATTCGAATTCAAGTACGGTCGCAATT
GATCATTCACTATCTCTAGCAGGGGAAAGAACTTGGGCTGAAACAATGGGTT
Sequence number 496: Bacillus anthracis pagA #19 
TCGCAATTGATCATTCACTATCTCTAGCAGGGGAAAGAACTTGGGCTGAAACAATGGGTTTAAATACC
GCTGATACAGCAAGATTAAATGCCAATATTAGATATGTAAATACTGGGACGG
Sequence number 497: Bacillus anthracis pagA #20 
TAAATACCGCTGATACAGCAAGATTAAATGCCAATATTAGATATGTAAATACTGGGACGGCTCCAATC
TACAACGTGTTACCAACGACTTCGTTAGTGTTAGGAAAAAATCAAACACTCG
Sequence number 498: Bacillus anthracis pagA #21 
CTCCAATCTACAACGTGTTACCAACGACTTCGTTAGTGTTAGGAAAAAATCAAACACTCGCGACAATT
AAAGCTAAGGAAAACCAATTAAGTCAAATACTTGCACCTAATAATTATTATC
Sequence number 499: Bacillus anthracis pagA #22 
CGACAATTAAAGCTAAGGAAAACCAATTAAGTCAAATACTTGCACCTAATAATTATTATCCTTCTAAA
AACTTGGCGCCAATCGCATTAAATGCACAAGACGATTTCAGTTCTACTCCAA
Sequence number 500: Bacillus anthracis pagA #23 
CTTCTAAAAACTTGGCGCCAATCGCATTAAATGCACAAGACGATTTCAGTTCTACTCCAATTACAATG
AATTACAATCAATTTCTTGAGTTAGAAAAAACGAAACAATTAAGATTAGATA
Sequence number 501: Bacillus anthracis pagA #24 
TTACAATGAATTACAATCAATTTCTTGAGTTAGAAAAAACGAAACAATTAAGATTAGATACGGATCAA
GTATATGGGAATATAGCAACATACAATTTTGAAAATGGAAGAGTGAGGGTGG
Sequence number 502: Bacillus anthracis pagA #25 
CGGATCAAGTATATGGGAATATAGCAACATACAATTTTGAAAATGGAAGAGTGAGGGTGGATACAGGC
TCGAACTGGAGTGAAGTGTTACCGCAAATTCAAGAAACAACTGCACGTATCA
Sequence number 503: Bacillus anthracis pagA #26 
ATACAGGCTCGAACTGGAGTGAAGTGTTACCGCAAATTCAAGAAACAACTGCACGTATCATTTTTAAT
GGAAAAGATTTAAATCTGGTAGAAAGGCGGATAGCGGCGGTTAATCCTAGTG
Sequence number 504: Bacillus anthracis pagA #27 
TTTTTAATGGAAAAGATTTAAATCTGGTAGAAAGGCGGATAGCGGCGGTTAATCCTAGTGATCCATTA
GAAACGACTAAACCGGATATGACATTAAAAGAAGCCCTTAAAATAGCATTTG
Sequence number 505: Bacillus anthracis pagA #28 
ATCCATTAGAAACGACTAAACCGGATATGACATTAAAAGAAGCCCTTAAAATAGCATTTGGATTTAAC
GAACCGAATGGAAACTTACAATATCAAGGGAAAGACATAACCGAATTTGATT
Sequence number 506: Bacillus anthracis pagA #29 
GATTTAACGAACCGAATGGAAACTTACAATATCAAGGGAAAGACATAACCGAATTTGATTTTAATTTC
GATCAACAAACATCTCAAAATATCAAGAATCAGTTAGCGGAATTAAACGCAA
Sequence number 507: Bacillus anthracis pagA #30 TTAATTTCGATCAACAAACATCTCAAAATATCAAGAATCAGTTAGCGGAATTAAACGCAACTAACATA TATACTGTATTAGATAAAATCAAATTAAATGCAAAAATGAATATTTTAATAA
Sequence number 508: Bacillus anthracis pagA #31 
CTAACATATATACTGTATTAGATAAAATCAAATTAAATGCAAAAATGAATATTTTAATAAGAGATAAA
CGTTTTCATTATGATAGAAATAACATAGCAGTTGGGGCGGATGAGTCAGTAG
Sequence number 509: Bacillus anthracis pagA #32 
GAGATAAACGTTTTCATTATGATAGAAATAACATAGCAGTTGGGGCGGATGAGTCAGTAGTTAAGGAG
GCTCATAGAGAAGTAATTAATTCGTCAACAGAGGGATTATTGTTAAATATTG
Sequence number 510: Bacillus anthracis pagA #33 
TTAAGGAGGCTCATAGAGAAGTAATTAATTCGTCAACAGAGGGATTATTGTTAAATATTGATAAGGAT
ATAAGAAAAATATTATCAGGTTATATTGTAGAAATTGAAGATACTGAAGGGC
Sequence number 511: Bacillus anthracis pagA #34 
ATAAGGATATAAGAAAAATATTATCAGGTTATATTGTAGAAATTGAAGATACTGAAGGGCTTAAAGAA
GTTATAAATGACAGATATGATATGTTGAATATTTCTAGTTTACGGCAAGATG
Sequence number 512: Bacillus anthracis pagA #35 
TTAAAGAAGTTATAAATGACAGATATGATATGTTGAATATTTCTAGTTTACGGCAAGATGGAAAAACA
TTTATAGATTTTAAAAAATATAATGATAAATTACCGTTATATATAAGTAATC
Sequence number 513: Bacillus anthracis pagA #36 
GAAAAACATTTATAGATTTTAAAAAATATAATGATAAATTACCGTTATATATAAGTAATCCCAATTAT
AAGGTAAATGTATATGCTGTTACTAAAGAAAACACTATTATTAATCCTAGTG
Sequence number 514: Bacillus anthracis pagA #37 
CCAATTATAAGGTAAATGTATATGCTGTTACTAAAGAAAACACTATTATTAATCCTAGTGAGAATGGG
GATACTAGTACCAACGGGATCAAGAAAATTTTAATCTTTTCTAAAAAAGGCT
Sequence number 515: Bacillus anthracis pagA #38 
AGAATGGGGATACTAGTACCAACGGGATCAAGAAAATTTTAATCTTTTCTAAAAAAGGCTATGAGATA
GGATAAGGTAATTCTAGGTGATTTTTAAATTATCTAAAAAACAGTAAAATTA
Sequence number 516: Bacillus anthracis pagA #39 
ATGAGATAGGATAAGGTAATTCTAGGTGATTTTTAAATTATCTAAAAAACAGTAAAATTAAAACATAC
TCTTTTTGTAAGAAATACAAGGAGAGTATGTTTTAAACAGTAATCTAAATCA
Sequence number 517: Bacillus anthracis pagA #40 
AAACATACTCTTTTTGTAAGAAATACAAGGAGAGTATGTTTTAAACAGTAATCTAAATCATCATAATC
CTTTGAGATTGTTTGTAGGATCCATAAAAACTTAGAGAAAATGAAACATCCA
Sequence number 518: Bacillus anthracis pagA #41 
TCATAATCCTTTGAGATTGTTTGTAGGATCCATAAAAACTTAGAGAAAATGAAACATCCAATGATTCT
ATATACAATTGTGCGAAGTATCTGGAGTTTAGAAAGCACAGAAAAATTTAAT
Sequence number 519: Bacillus anthracis pagA #42 
ATGATTCTATATACAATTGTGCGAAGTATCTGGAGTTTAGAAAGCACAGAAAAATTTAATTAGAGGAC
AATTGCACATTCAATAAGATATCTATAATATATTGATGCTCAAAATAAAGGT
Sequence number 520: Bacillus anthracis pagA #43 
TAGAGGACAATTGCACATTCAATAAGATATCTATAATATATTGATGCTCAAAATAAAGGTTCATGCCT
TAAGGAATGTAGTAAAGGTGTGCTGTTCAGGTGCAGAAACGGTTCTATGTAT
Sequence number 521: Bacillus anthracis pagA #44 
TCATGCCTTAAGGAATGTAGTAAAGGTGTGCTGTTCAGGTGCAGAAACGGTTCTATGTATTAAGGTAA
GGGTGTCTTACTAACAGCATAGCAGAAATCTATATTGCGAAAAGACTTTAAT
Sequence number 522: Bacillus anthracis pagA #45 
TAAGGTAAGGGTGTCTTACTAACAGCATAGCAGAAATCTATATTGCGAAAAGACTTTAATTATTATAG
GTAGTTAGTAGATTAGACGAAACGAGCGCCAATGAACTAATTACACTACATT
Sequence number 523: Bacillus anthracis pagA #46 
TATTATAGGTAGTTAGTAGATTAGACGAAACGAGCGCCAATGAACTAATTACACTACATTAATGAGCA
AAAGTTAATACGGTAAATAGATAGTATGATTGAAATTGATAAGGACCACAAG
Sequence number 524: Bacillus anthracis pagA #47 
AATGAGCAAAAGTTAATACGGTAAATAGATAGTATGATTGAAATTGATAAGGACCACAAGAGCGTGGC
AAATATATTGAAAGAGAAAGCAACTGCCAAAAATATTCTTCTGTTGGAAAAG
Sequence number 525: Bacillus anthracis pagA #48 
AGCGTGGCAAATATATTGAAAGAGAAAGCAACTGCCAAAAATATTCTTCTGTTGGAAAAGATAAAAGT
GGTAATTTAGGAATATAGTTTATATAATTTATCACGATATAGGGACTATACT
Sequence number 526: Bacillus anthracis pagA #49 
ATAAAAGTGGTAATTTAGGAATATAGTTTATATAATTTATCACGATATAGGGACTATACTCTAAATCA
ATTAATAGAAAATATGAGGTTAAAGAATCTATACATTTCTTGTTTTATTTCT
Sequence number 527: Bacillus anthracis pagA #50 
CTAAATCAATTAATAGAAAATATGAGGTTAAAGAATCTATACATTTCTTGTTTTATTTCTTTATAATT
TAATTTAAAAATACTATAAAAATCCTAAATTAGATATTAAAAATAGTGTGCG
Sequence number 528: Bacillus anthracis pagA #51 
TTATAATTTAATTTAAAAATACTATAAAAATCCTAAATTAGATATTAAAAATAGTGTGCGAAAGTTCT
TAAAAACTTAATATTAAAATCATATTAATATTATACATTAGAATTATATAAT
Sequence number 529: Bacillus anthracis pagA #52 
AAAGTTCTTAAAAACTTAATATTAAAATCATATTAATATTATACATTAGAATTATATAATCAGTTTAT
TATACAAAACATCACAATGTACGAACATAATAATGTCATATTATTAAAAGGT
Sequence number 530: Bacillus anthracis pagA #53 
CAGTTTATTATACAAAACATCACAATGTACGAACATAATAATGTCATATTATTAAAAGGTTAAACTAC
ATTAAAACATAAAGAGAGAGGAATTGCTATGACAGTATTTGTAGATCATAAA
Sequence number 531: Bacillus anthracis pagA #54 
TAAACTACATTAAAACATAAAGAGAGAGGAATTGCTATGACAGTATTTGTAGATCATAAAATTGAATA
CATGAGTTTAGAAGATGATGCTGAACTTTTAAAAACAATGGCACATCCTATG
Sequence number 532: Bacillus anthracis pagA #55 
ATTGAATACATGAGTTTAGAAGATGATGCTGAACTTTTAAAAACAATGGCACATCCTATGCGTTTAAA
AATAGTCAATGAACTTTACAAACATAAAGCATTAAATGTAACGCAAATCATT
Sequence number 533: Bacillus anthracis pagA #56 
CGTTTAAAAATAGTCAATGAACTTTACAAACATAAAGCATTAAATGTAACGCAAATCATTCAAATCTT
AAAACTACCACAATCAACTGTATCCCAGCATTTATGTAAAATGAGAGGAAAA
Sequence number 534: Bacillus anthracis pagA #57 
CAAATCTTAAAACTACCACAATCAACTGTATCCCAGCATTTATGTAAAATGAGAGGAAAAGTTTTAAA
AAGAAATCGACAAGGTTTAGAGATATACTATAGCATTAATAATCCAAAAGTT
Sequence number 535: Bacillus anthracis pagA #58 CATTTATGTAAAATGAGAGGAAAAGTTTTAAAAAGAAATCGACAAGGTTTAGAGATATACTATAGCAT TAATAATCCAAAAGTTGAAGGGATTATTAAGTTGTTAAACCCTATCCAATAG
Sequence number 536: Bacillus anthracis capB #1 
TAAATCACTTTTGCTTGCTTTTTGCCAATTTGTTCTTCCATAATCATATCGATTAATGGCTCAGCTGC
ACCATGAATATTACCCACTCCATATACAATCCGATTTTTTAAATATGGACGC
Sequence number 537: Bacillus anthracis capB #2 
TCAGCTGCACCATGAATATTACCCACTCCATATACAATCCGATTTTTTAAATATGGACGCATACGAGA
CATAATTTCACTTGTTGACCAGCCTTCTAAGTTCCAATACTCTTGCGTTGGA
Sequence number 538: Bacillus anthracis capB #3 
ATACGAGACATAATTTCACTTGTTGACCAGCCTTCTAAGTTCCAATACTCTTGCGTTGGAATATCTCC
TTTTTCAAAAGCACTTGTAATAGGTGCAGTCGTTTCTCCAATCGCAATAACT
Sequence number 539: Bacillus anthracis capB #4 
ATATCTCCTTTTTCAAAAGCACTTGTAATAGGTGCAGTCGTTTCTCCAATCGCAATAACTATTTCCGC
TTTAATATATGGCAAAACATCCCTAGCAAACTGCTCAGTACGATCAACGCGG
Sequence number 540: Bacillus anthracis capB #5 
ATTTCCGCTTTAATATATGGCAAAACATCCCTAGCAAACTGCTCAGTACGATCAACGCGGTCAGGGCG
GCAATTCATAATTACAATTGGAGCTAGATTACTATATCCAAAATCATCCACA
Sequence number 541: Bacillus anthracis capB #6 
TCAGGGCGGCAATTCATAATTACAATTGGAGCTAGATTACTATATCCAAAATCATCCACACGTTCCCA
AATACGTAATGTTGATGAGGGATCATTCGCTGCAAAACCATTTACGAAGAAC
Sequence number 542: Bacillus anthracis capB #7 
CGTTCCCAAATACGTAATGTTGATGAGGGATCATTCGCTGCAAAACCATTTACGAAGAACGCAGGCTT
AGATTGGTCAGCAAAACGTGTAATTCTCATTGCTCCTGGATCCGGATGAGCA
Sequence number 543: Bacillus anthracis capB #8 
GCAGGCTTAGATTGGTCAGCAAAACGTGTAATTCTCATTGCTCCTGGATCCGGATGAGCATTCAACAT
ACCACGGAATGCTGTTTCCTCATCAATCCCAAGAGCCTCTGCTACCGCTAAA
Sequence number 544: Bacillus anthracis capB #9 
TTCAACATACCACGGAATGCTGTTTCCTCATCAATCCCAAGAGCCTCTGCTACCGCTAAAGCAAGCGA
TGCATTATCTGGGAAGACCATGTAATCAAATTTTCGTAAGAATTCTTCTGAA
Sequence number 545: Bacillus anthracis capB #10 
GCAAGCGATGCATTATCTGGGAAGACCATGTAATCAAATTTTCGTAAGAATTCTTCTGAAATTCTAGA
ATTATCCGCAACAATCACTTTTGTATTTCTCTCTTCTGCAACCTCTTTAAAG
Sequence number 546: Bacillus anthracis capB #11 
ATTCTAGAATTATCCGCAACAATCACTTTTGTATTTCTCTCTTCTGCAACCTCTTTAAAGTAATCCAA
GTATTCACTTTCAATAGTGACTAAATGTCCATTATATGGAATGGTAGCAGTG
Sequence number 547: Bacillus anthracis capB #12 
TAATCCAAGTATTCACTTTCAATAGTGACTAAATGTCCATTATATGGAATGGTAGCAGTGAAAGCTTC
AGCTACTTCGTCAAGTGTAGGTCCCATAACATCCATATGATCTTCTAAAACA
Sequence number 548: Bacillus anthracis capB #13 
AAAGCTTCAGCTACTTCGTCAAGTGTAGGTCCCATAACATCCATATGATCTTCTAAAACATTTACAAT
CACTCCAACATTTGCTTGAATCATTTTATTTTGGAAGATAATTTGATAATCG
Sequence number 549: Bacillus anthracis capB #14 
TTTACAATCACTCCAACATTTGCTTGAATCATTTTATTTTGGAAGATAATTTGATAATCGGGTTGAAC
TGCCATACATTCACAAATAAGTGCTTCTGCTTCTAAATCAGCAGCCTCTTTA
Sequence number 550: Bacillus anthracis capB #15 
GGTTGAACTGCCATACATTCACAAATAAGTGCTTCTGCTTCTAAATCAGCAGCCTCTTTAACTACCCT
GCGTTGCTCACCGATATTAGGACCTTCTTTACGGCGCTTAATCGGTTGCTCC
Sequence number 551: Bacillus anthracis capB #16 
ACTACCCTGCGTTGCTCACCGATATTAGGACCTTCTTTACGGCGCTTAATCGGTTGCTCCTCGTCAGT
AAACCAATATATCATTCGCGCAGATGTACCAGTTGTTTTCCCTACAGTCTTA
Sequence number 552: Bacillus anthracis capB #17 
TCGTCAGTAAACCAATATATCATTCGCGCAGATGTACCAGTTGTTTTCCCTACAGTCTTATATTTCGC
TTCTTGTACAACACCTGTAATTAGTCTTGTAACGGTAGATTTACCTCGAATT
Sequence number 553: Bacillus anthracis capB #18 
TATTTCGCTTCTTGTACAACACCTGTAATTAGTCTTGTAACGGTAGATTTACCTCGAATTCCATTTAT
GTTTACTCGAATTGGGATAGAATTGAGCCTTTTCTGATGGCAACGTTGTTCC
Sequence number 554: Bacillus anthracis capB #19 
CCATTTATGTTTACTCGAATTGGGATAGAATTGAGCCTTTTCTGATGGCAACGTTGTTCCCATATACC
ATAAATAATCAAAAACACTGTACATATACCTATTATGAAGATCATCTGTATT
Sequence number 555: Bacillus anthracis capB #20 
CATATACCATAAATAATCAAAAACACTGTACATATACCTATTATGAAGATCATCTGTATTAATCCCTT
CCTGCTTTTCGATTTCTTGCTTATTAAGATATTTCGACTTAAGTAGAGTCGT
Sequence number 556: Bacillus anthracis capB #21 
AATCCCTTCCTGCTTTTCGATTTCTTGCTTATTAAGATATTTCGACTTAAGTAGAGTCGTTGATACCA
ATTAAAAATTAGGTGGTTCCCTGCATATACAACACCGAATTCATCTGAGTAT
Sequence number 557: Bacillus anthracis capB #22 
TGATACCAATTAAAAATTAGGTGGTTCCCTGCATATACAACACCGAATTCATCTGAGTATAATTCTGA
AATCCTATGTTCAATGCGTATTGCCTCATCATGTTTCAATATTCTTACAATT
Sequence number 558: Bacillus anthracis capB #23 
AATTAGGTGGTTCCCTGCATATACAACACCGAATTCATCTGAGTATAATTCTGAAATCCTATGTTCAA
TGCGTATTGCCTCATCATGTTTCAATATTCTTACAATTTTTATGTTTTTCAT
Sequence number 559: Borrelia burgdorferi 16S rRNA #1 
TGTGCGATTTGTTATCTTGCCGCCATAAGAATGACAAGCGCTATCTGTTATCCCCATATAAGCTTCCG
TTTCAACAATTCTGGTAACTATTTCCTCTTTGTCGATCTTTCTGATCAACAA
Sequence number 560: Borrelia burgdorferi 16S rRNA #2 
AGCTTCCGTTTCAACAATTCTGGTAACTATTTCCTCTTTGTCGATCTTTCTGATCAACAAATTACCAA
GCAATAACTTTGCAACGGTAGTAGCATCTTGCAAAAAAAAATATCGATCCAT
Sequence number 561: Borrelia burgdorferi 16S rRNA #3 
ATTACCAAGCAATAACTTTGCAACGGTAGTAGCATCTTGCAAAAAAAAATATCGATCCATAAGCTCAA
TTTTAAAACAAAATAATAATAAAAATTATTACCAAAACACACATGTAAAATA
Sequence number 562: Borrelia burgdorferi 16S rRNA #4 
AAGCTCAATTTTAAAACAAAATAATAATAAAAATTATTACCAAAACACACATGTAAAATATGAAAACA
TATTGCCTAGAATAAAAATAAAAACTAAAATCATTTAATCACCCAGTAAATA
Sequence number 563: Borrelia burgdorferi 16S rRNA #5 TGAAAACATATTGCCTAGAATAAAAATAAAAACTAAAATCATTTAATCACCCAGTAAATACATATTTT AAATAAATTCTTGGTTGATTAATAAATTTTTTGATTAAAAATCAAAAAAT
Sequence number 564: Borrelia burgdorferi 16S rRNA #6 
CATATTTTAAATAAATTCTTGGTTGATTAATAAATTTTTTTGATTAAAAATCAAAAAAATTTAAAAAG
TTGAACTAGTTCAACTTAAATCAGCTTATATGAAAATGCTAATCTAAAATAC
Sequence number 565: Borrelia burgdorferi 16S rRNA #7 
TTAAAAAGTTGAACTAGTTCAACTTAAATCAGCTTATATGAAAATGCTAATCTAAAATACAAAATATC
GAATATTGACAAATATATAAAAATTATAAACTATATGCAATATCTCTTACAT
Sequence number 566: Borrelia burgdorferi 16S rRNA #8 
AAAATATCGAATATTGACAAATATATAAAAATTATAAACTATATGCAATATCTCTTACATCTTGAAAA
GATATAAGAGATATTGACAAAATCATAAGATTTAAATAAAATTTGTAAAGAA
Sequence number 567: Borrelia burgdorferi 16S rRNA #9 
CTTGAAAAGATATAAGAGATATTGACAAAATCATAAGATTTAAATAAAATTTGTAAAGAATTGATATG
CTTAAGGGATCATAGCTCAGGTGGTTAGAGCGCAGGTCTGATAAACCTGAGG
Sequence number 568: Borrelia burgdorferi 16S rRNA #10 
TTGATATGCTTAAGGGATCATAGCTCAGGTGGTTAGAGCGCAGGTCTGATAAACCTGAGGTCGGATGT
TCAACTCATCCTGGTCCCAATGTATTCCATTGTTTTAATTACGACAATACTG
Sequence number 569: Borrelia burgdorferi 16S rRNA #11 
TCGGATGTTCAACTCATCCTGGTCCCAATGTATTCCATTGTTTTAATTACGACAATACTGTTTTAAGC
TATAATTAATAAGAAAAACAAAAATTTATTCAATTTCTATATTAAGGTATAC
Sequence number 570: Borrelia burgdorferi 16S rRNA #12 
TTTTAAGCTATAATTAATAAGAAAAACAAAAATTTATTCAATTTCTATATTAAGGTATACACGATTAC
TAACAATTACAACTTCATGCCATTGAGTGTCGGGCAAATCCAAACTGAAATC
Sequence number 571: Borrelia burgdorferi 16S rRNA #13 
ACGATTACTAACAATTACAACTTCATGCCATTGAGTGTCGGGCAAATCCAAACTGAAATCTGCTTTAT
AGTATTTTGCTTAGCTTTTTTGAGTTATTGTTGCAAACTTGTAACCTAAAAC
Sequence number 572: Borrelia burgdorferi 16S rRNA #14 
TGCTTTATAGTATTTTGCTTAGCTTTTTTGAGTTATTGTTGCAAACTTGTAACCTAAAACATAAGAGC
GATAACGATTTAATGCAATCTTTGCCTTCCTCCAACTTATAACTGGCAATCT
Sequence number 573: Borrelia burgdorferi 16S rRNA #15 
ATAAGAGCGATAACGATTTAATGCAATCTTTGCCTTCCTCCAACTTATAACTGGCAATCTTATCCGAA
TCCCTACCACTAAATACTGGCAACGATAATAAGGATTTAAGCTCGCTACAAT
Sequence number 574: Borrelia burgdorferi 16S rRNA #16 
TATCCGAATCCCTACCACTAAATACTGGCAACGATAATAAGGATTTAAGCTCGCTACAATACTTGTCC
CTCTCTCAGACATCACACTTAGTAATCTACATTATTTCCTAACTAAAAACTA
Sequence number 575: Borrelia burgdorferi 16S rRNA #17 
ACTTGTCCCTCTCTCAGACATCACACTTAGTAATCTACATTATTTCCTAACTAAAAACTATTTACTAA
ATGCAGATCGTTTATAAAATAGGCTGCTGAAAAATACAATCCTGTTTAATCC
Sequence number 576: Borrelia burgdorferi 16S rRNA #18 
TTTACTAAATGCAGATCGTTTATAAAATAGGCTGCTGAAAAATACAATCCTGTTTAATCCTTGCACTT
TAGCGAAACAACTTCGGGTTTTATCGAAAGCTTGCTTTTGCTTCTAGACATT
Sequence number 577: Borrelia burgdorferi 16S rRNA #19 
TTGCACTTTAGCGAAACAACTTCGGGTTTTATCGAAAGCTTGCTTTTGCTTCTAGACATTTTTCTTAA
TATCAACAGATCTCAACTTAACCCAGAAATTTTAACTTCTTGTGTTTCACAT
Sequence number 578: Borrelia burgdorferi 16S rRNA #20 
TTTCTTAATATCAACAGATCTCAACTTAACCCAGAAATTTTAACTTCTTGTGTTTCACATTCCAACAA
TTACTTTAACAAAGTTACTTAAAAACATACTTAATTACAATTTAGGGCATAA
Sequence number 579: Borrelia burgdorferi 16S rRNA #21 
TCCAACAATTACTTTAACAAAGTTACTTAAAAACATACTTAATTACAATTTAGGGCATAAGCCCAATG
GAGGTTAAGGGACTCGAACCCTTGACCCTCGGCTTGCAAAGCCGATGCTCTA
Sequence number 580: Borrelia burgdorferi 16S rRNA #22 
GCCCAATGGAGGTTAAGGGACTCGAACCCTTGACCCTCGGCTTGCAAAGCCGATGCTCTAGCCAACTG
AGCTAAACCCCCAAAATGACCTAAAATTAAGTCTAAGCCTAATAAAACTATT
Sequence number 581: Borrelia burgdorferi 16S rRNA #23 
GCCAACTGAGCTAAACCCCCAAAATGACCTAAAATTAAGTCTAAGCCTAATAAAACTATTTAATATCA
ATAATTTAATACATAAACTTCTCTTAAACTTACATAAATAACACTGGTTTTA
Sequence number 582: Borrelia burgdorferi 16S rRNA #24 
TAATATCAATAATTTAATACATAAACTTCTCTTAAACTTACATAAATAACACTGGTTTTAAAAACTCT
CTTATCAAAAGAAAAGAGTAGGGAAGAGTTAATGGAATTAGCCTTAGTTTAT
Sequence number 583: Borrelia burgdorferi 16S rRNA #25 
AAAACTCTCTTATCAAAAGAAAAGAGTAGGGAAGAGTTAATGGAATTAGCCTTAGTTTATCTTTCTCT
TAGAAAGGAGGTGATCCAGCCGCACTTTCCAGTACGGCTACCTTGTTACGAC
Sequence number 584: Borrelia burgdorferi 16S rRNA #26 
CTTTCTCTTAGAAAGGAGGTGATCCAGCCGCACTTTCCAGTACGGCTACCTTGTTACGACTTCACCCC
CCTCACTAAACATACCTTAAATACCTTCCTCCCTTACGGGTTAGAATAATAG
Sequence number 585: Borrelia burgdorferi 16S rRNA #27 
TTCACCCCCCTCACTAAACATACCTTAAATACCTTCCTCCCTTACGGGTTAGAATAATAGCTTCGGGT
ATCCTCAACTCGGGTGGTGTGACGGGCGGTGTGTACAAGGCCCGAGAACGTA
Sequence number 586: Borrelia burgdorferi 16S rRNA #28 
CTTCGGGTATCCTCAACTCGGGTGGTGTGACGGGCGGTGTGTACAAGGCCCGAGAACGTATTCACCGT
ATCATTCTGATATACGATTACTAGCGATTCCAACTTCATGAAGTCGAGTTTC
Sequence number 587: Borrelia burgdorferi 16S rRNA #29 
TTCACCGTATCATTCTGATATACGATTACTAGCGATTCCAACTTCATGAAGTCGAGTTTCAGACTTCA
ATCCGGACTGAGACCTGCTTTATGCGTTTTGCTTCACATCACTGTTTCGCTT
Sequence number 588: Borrelia burgdorferi 16S rRNA #30 
AGACTTCAATCCGGACTGAGACCTGCTTTATGCGTTTTGCTTCACATCACTGTTTCGCTTCGCTTTGT
ACAGGCCATTGTAGCACGTGTGTAGCCCAGGACATAAGGGCCATGATGATTT
Sequence number 589: Borrelia burgdorferi 16S rRNA #31 
CGCTTTGTACAGGCCATTGTAGCACGTGTGTAGCCCAGGACATAAGGGCCATGATGATTTGACGTCAT
CCTCACCTTCCTCCGACTTATCACCGGCAGTCTTATCTGAGTCCCCACCATT
Sequence number 590: Borrelia burgdorferi 16S rRNA #32 
GACGTCATCCTCACCTTCCTCCGACTTATCACCGGCAGTCTTATCTGAGTCCCCACCATTACATGCTG
GTAACAGATAACAAGGGTTGCGCTCGTTGCGGGACTTAACCCAACACCTCAC
Sequence number 591: Borrelia burgdorferi 16S rRNA #33 ACATGCTGGTAACAGATAACAAGGGTTGCGCTCGTTGCGGGACTTAACCCAACACCTCACAGCACGAG CTGACGACAACCATGCAGCACCTGTATATAGACCCCAAACGGGGAATAATTA
Sequence number 592: Borrelia burgdorferi 16S rRNA #34 
AGCACGAGCTGACGACAACCATGCAGCACCTGTATATAGACCCCAAACGGGGAATAATTATCTCTAAC
TATATCCTATATATGTCAAGCCCTGGTAAGGTTCCTCGCGTATCATCGAATT
Sequence number 593: Borrelia burgdorferi 16S rRNA #35 
TCTCTAACTATATCCTATATATGTCAAGCCCTGGTAAGGTTCCTCGCGTATCATCGAATTAAACCACA
TGCTCCACCGCTTGTGCGGGCCCCCGTCAATTCCTTTGAGTTTCACTCTTGC
Sequence number 594: Borrelia burgdorferi 16S rRNA #36 
AAACCACATGCTCCACCGCTTGTGCGGGCCCCCGTCAATTCCTTTGAGTTTCACTCTTGCGAGCATAC
TCCCCAGGCGGCACACTTAACACGTTAGCTTCGGTACTAACTTTTAGTTAAC
Sequence number 595: Borrelia burgdorferi 16S rRNA #37 
GAGCATACTCCCCAGGCGGCACACTTAACACGTTAGCTTCGGTACTAACTTTTAGTTAACACCAAGTG
TGCATCGTTTACAGCGTAGACTACCAGGGTATCTAATCCTGTTTGCTCCCTA
Sequence number 596: Borrelia burgdorferi 16S rRNA #38 
ACCAAGTGTGCATCGTTTACAGCGTAGACTACCAGGGTATCTAATCCTGTTTGCTCCCTACGCTTTCG
TGACTCAGCGTCAGTCTTGACCCAGAAGTTCGCCTTCGCCTCCGGTATTCTT
Sequence number 597: Borrelia burgdorferi 16S rRNA #39 
CGCTTTCGTGACTCAGCGTCAGTCTTGACCCAGAAGTTCGCCTTCGCCTCCGGTATTCTTTCTGATAT
CAACAGATTCCACCCTTACACCAGAAATTCTAACTTCCTCTATCAGACTCTA
Sequence number 598: Borrelia burgdorferi 16S rRNA #40 
TCTGATATCAACAGATTCCACCCTTACACCAGAAATTCTAACTTCCTCTATCAGACTCTAGACATATA
GTTTCCAACATAGGTCCACAGTTGAGCTGTGGTATTTTATGCATAGACTTAT
Sequence number 599: Borrelia burgdorferi 16S rRNA #41 
GACATATAGTTTCCAACATAGGTCCACAGTTGAGCTGTGGTATTTTATGCATAGACTTATATATCCGC
CTACTCACCCTTTACGCCCAATAATCCCGAACAACGCTCGCCCCTTACGTAT
Sequence number 600: Borrelia burgdorferi 16S rRNA #42 
ATATCCGCCTACTCACCCTTTACGCCCAATAATCCCGAACAACGCTCGCCCCTTACGTATTACCGCGG
CTGCTGGCACGTAATTAGCCGGGGCTTATTCATAAATTAACGTCATCACTTT
Sequence number 601: Borrelia burgdorferi 16S rRNA #43 
TACCGCGGCTGCTGGCACGTAATTAGCCGGGGCTTATTCATAAATTAACGTCATCACTTTGTCATTTC
CTACAAAGCTTATTCCTCATTTATAAAAGAATTTTACAATCTTTCGACCTTC
Sequence number 602: Borrelia burgdorferi 16S rRNA #44 
GTCATTTCCTACAAAGCTTATTCCTCATTTATAAAAGAATTTTACAATCTTTCGACCTTCTTCATTCA
CGCAGTGTCGCTCCGTCAGGCTTTCGCCCATTGCGGAAGATTCTTAGCTGCT
Sequence number 603: Borrelia burgdorferi 16S rRNA #45 
TTCATTCACGCAGTGTCGCTCCGTCAGGCTTTCGCCCATTGCGGAAGATTCTTAGCTGCTGCCTCCCG
TAGGAGTCTGGACCGTATCTCAGTTCCAGTGTGACCGTTCACCCTCTCAGGC
Sequence number 604: Borrelia burgdorferi 16S rRNA #46 
GCCTCCCGTAGGAGTCTGGACCGTATCTCAGTTCCAGTGTGACCGTTCACCCTCTCAGGCCGGTTACT
TATCATTGCCTTGGTAGGCATTTACCCTACCAACTAGCTAATAAGACGCAGA
Sequence number 605: Borrelia burgdorferi 16S rRNA #47 
CGGTTACTTATCATTGCCTTGGTAGGCATTTACCCTACCAACTAGCTAATAAGACGCAGACTCATCTA
CAAGCGAAGCTTTAAAGGCTTCCTTTCATCAATTAACAAATTAATTGACCTT
Sequence number 606: Borrelia burgdorferi 16S rRNA #48 
CTCATCTACAAGCGAAGCTTTAAAGGCTTCCTTTCATCAATTAACAAATTAATTGACCTTATTCGGTA
TTAGCTACTATTTCTAATAGTTATCCCCATCTCATAGGTAGATCATCCACGC
Sequence number 607: Borrelia burgdorferi 16S rRNA #49 
ATTCGGTATTAGCTACTATTTCTAATAGTTATCCCCATCTCATAGGTAGATCATCCACGCGTTACTCA
CCCGTTCGCCACTGAATGTATTGCTACATCCCGTTTGACTTGCATGCTTAAG
Sequence number 608: Borrelia burgdorferi 16S rRNA #50 
GTTACTCACCCGTTCGCCACTGAATGTATTGCTACATCCCGTTTGACTTGCATGCTTAAGACGCACTG
CCAGCGTTAGTTCTAAGCCAGGATCAAACTCTTCGTTATTTTATTTTTTTAT
Sequence number 609: Borrelia burgdorferi 16S rRNA #51 
ACGCACTGCCAGCGTTAGTTCTAAGCCAGGATCAAACTCTTCGTTATTTTATTTTTTTATTTAAAAAA
ATTAACAGGTTTTGTTTATTCTTTTGGCTTTTAACCTTACTTAACTCTTCCC
Sequence number 610: Borrelia burgdorferi 16S rRNA #52 
TTAAAAAAATTAACAGGTTTTGTTTATTCTTTTGGCTTTTAACCTTACTTAACTCTTCCCTTCTCTCA
TCTTCCAATAATCATTTCAACCTTTAATACTTTAACAAAAGAAGAATCATAT
Sequence number 611: Borrelia burgdorferi 16S rRNA #53 
TTCTCTCATCTTCCAATAATCATTTCAACCTTTAATACTTTAACAAAAGAAGAATCATATTGTCAATA
CCTTTGCATTATTTTTATTAATATAAAATGTAAAAAAATAAATTTTATTAAT
Sequence number 612: Borrelia burgdorferi 16S rRNA #54 
TGTCAATACCTTTGCATTATTTTTATTAATATAAAATGTAAAAAAATAAATTTTATTAATAAACAGTG
ATAAAAGCATTAACAGCCACACTTTTCATTAACAGGCAAAGTTGTAAAAATT
Sequence number 613: Borrelia burgdorferi 16S rRNA #55 
AAACAGTGATAAAAGCATTAACAGCCACACTTTTCATTAACAGGCAAAGTTGTAAAAATTTTTTTTAA
TTTATTTCCCAATTTAGCTTTTAAATAATCAAAAAAGGTTCTGAAATTTTCA
Sequence number 614: Borrelia burgdorferi 16S rRNA #56 
TTTTTAATTTATTTCCCAATTTAGCTTTTAAATAATCAAAAAAGGTTCTGAAATTTTCATTATCTCC
TTTAAACTTAATCCACAGTTCTTTAAAGGTTAAAATTCCTTCCCTAAGATCC
Sequence number 615: Borrelia burgdorferi 16S rRNA #57 
TTATCTCCTTTAAACTTAATCCACAGTTCTTTAAAGGTTAAAATTCCTTCCCTAAGATCCTTGTAATT
GGAAAAAGCAGCAGCACAGTCGCTATACATTAAATTATAAAAATCAGAATCT
Sequence number 616: Borrelia burgdorferi 16S rRNA #58 
TTGTAATTGGAAAAAGCAGCAGCACAGTCGCTATACATTAAATTATAAAAATCAGAATCTTGTTTATC
TTTAAAAAAATCTATACTTGGTATTTTCTTGGCAAAGGCGAATCCTCTCTCA
Sequence number 617: Borrelia burgdorferi 16S rRNA #59 
TGTTTATCTTTAAAAAAATCTATACTTGGTATTTTCTTGGCAAAGGCGAATCCTCTCTCATAAGCCGT
GCCAGAATCAACATAATTAACCAAGGCTAAAACAATATCGCTACTAACTAGC
Sequence number 618: Borrelia burgdorferi 16S rRNA #60 
TAAGCCGTGCCAGAATCAACATAATTAACCAAGGCTAAAACAATATCGCTACTAACTAGCTCTTTTAA
ATCTATTTCTCTTATCTTCTCCCTTATATCTCTATTTACAAACTTATAATCA
Sequence number 619: Borrelia burgdorferi 16S rRNA #61 TCTTTTAAATCTATTTCTCTTATCTTCTCCCTTATATCTCTATTTACAAACTTATAATCAACCTTTTC AAGCAATCCCATCTTTTTGACAGCATGATGCTCTGGAGAAAATACTTCTAAA
Sequence number 620: Borrelia burgdorferi 16S rRNA #62 
ACCTTTTCAAGCAATCCCATCTTTTTGACAGCATGATGCTCTGGAGAAAATACTTCTAAATTAAACTC
TTCAAGAAATTTCAAAACCTCATCCCTTAGTTTAATTTCCTCTTCCTTAAAA
Sequence number 621: Borrelia burgdorferi 16S rRNA #63 
TTAAACTCTTCAAGAAATTTCAAAACCTCATCCCTTAGTTTAATTTCCTCTTCCTTAAAAAATGGAGA
GGCTAAATAAATCTTCATAAAAACCCCTATCAAAATTCAAGTATAACACAAT
Sequence number 622: Borrelia burgdorferi 16S rRNA #64 
AATGGAGAGGCTAAATAAATCTTCATAAAAACCCCTATCAAAATTCAAGTATAACACAATAAATCAAT
CGAGTAAAATCAAGAATAAATAAATTTTTAAAATCCAAGTAGAAAACATTTA
Sequence number 623: Borrelia burgdorferi 16S rRNA #65 
AAATCAATCGAGTAAAATCAAGAATAAATAAATTTTTAAAATCCAAGTAGAAAACATTTATTATGAAC
GACTTCTGCTGACTAGAATAGTAAACTCTCCTTTAACCTTATCCTTACTCGA
Sequence number 624: Borrelia burgdorferi 16S rRNA #66 
TTATGAACGACTTCTGCTGACTAGAATAGTAAACTCTCCTTTAACCTTATCCTTACTCGATTCAAAAT
ATTTTTTTAATTCTAAAGGTTTGCCAATTTGATATTCTTCATAAATTTTTGT
Sequence number 625: Borrelia burgdorferi 16S rRNA #67 
TTCAAAATATTTTTTTAATTCTAAAGGTTTGCCAATTTGATATTCTTCATAAATTTTTGTCATCTCGC
GACCAATAAGAACCTTCGCATCTAAACTAACAGAAGAAATTTCAACAAGCAA
Sequence number 626: Borrelia burgdorferi 16S rRNA #68 
CATCTCGCGACCAATAAGAACCTTCGCATCTAAACTAACAGAAGAAATTTCAACAAGCAATTTCAAAA
TTCTATGACCAGATTCAAGCAGAACAAAAGCATCTCCCCTTTTATATAGCTC
Sequence number 627: Borrelia burgdorferi 16S rRNA #69 
TTTCAAAATTCTATGACCAGATTCAAGCAGAACAAAAGCATCTCCCCTTTTATATAGCTCAGCAATTC
TTTTAAATCTTTTAAGGCCTTTGTTAGGCAAAAATCCCTCAAAAAACACTGA
Sequence number 628: Borrelia burgdorferi 16S rRNA #70 
AGCAATTCTTTTAAATCTTTTAAGGCCTTTGTTAGGCAAAAATCCCTCAAAAAACACTGATTTATCTC
TAAAAGGATTAACACTTACAATTGTATTAAAAGAACTTACTCCAGGAATTGG
Sequence number 629: Borrelia burgdorferi 16S rRNA #71 
TTTATCTCTAAAAGGATTAACACTTACAATTGTATTAAAAGAACTTACTCCAGGAATTGGACAAACTT
TGTATCCCTCTCTAAAAGCAGCAGCAACTAATAAGCTGCCCGGATCACTAAG
Sequence number 630: Borrelia burgdorferi 16S rRNA #72 
ACAAACTTTGTATCCCTCTCTAAAAGCAGCAGCAACTAATAAGCTGCCCGGATCACTAAGCCCTGGAG
TACCAGCATCGCTAACAAAAGCAACAGAATTTCCTTTTGCCAAATAATCCAA
Sequence number 631: Borrelia burgdorferi 16S rRNA #73 
CCCTGGAGTACCAGCATCGCTAACAAAAGCAACAGAATTTCCTTTTGCCAAATAATCCAATAGCAAGC
TTATCTTCTTGTTTTCTGTTACGGCATTACAAGAAATCATTTTTTTATTAAT
Sequence number 632: Borrelia burgdorferi 16S rRNA #74 
TAGCAAGCTTATCTTCTTGTTTTCTGTTACGGCATTACAAGAAATCATTTTTTTATTAATTTTATATC
GAGACAAAAGTTTGCTAGTTACTCTCGTATCTTCCGCAAAGATAACATTTAC
Sequence number 633: Borrelia burgdorferi 16S rRNA #75 
TTTATATCGAGACAAAAGTTTGCTAGTTACTCTCGTATCTTCCGCAAAGATAACATTTACCGATTTTA
AAACATCAATTGCTCTATAAGTAATATCTTCTAAATTGCCTATTGGCGTACC
Sequence number 634: Borrelia burgdorferi 16S rRNA #76 
TCCGCAAAGATAACATTTACCGATTTTAAAACATCAATTGCTCTATAAGTAATATCTTCTAAATTGCC
TATTGGCGTACCAACAATATATAACACGGCCCCTTTTAATTCCTCCAAAACT
Sequence number 635: Borrelia hermsii 16S rRNA #1 
AATCTTAAATCCAAATGAAAATATAGGTTTTGCCTCATACCAAGTTGAAGATTTCATTAAAGAAATAA
TTGATCCTATTCTTGAAAATAAATAAGGAATACTAATATTTAGAAACAAATC
Sequence number 636: Borrelia hermsii 16S rRNA #2 
AGAAATAATTGATCCTATTCTTGAAAATAAATAAGGAATACTAATATTTAGAAACAAATCTATTATCC
CTTATGTAAAATCGCCAAAGCTTATTCGCATACTCCTCACCTGCATAATCAA
Sequence number 637: Borrelia hermsii 16S rRNA #3 
TATTATCCCTTATGTAAAATCGCCAAAGCTTATTCGCATACTCCTCACCTGCATAATCAACATTTATT
CTCTTTGAGCATGCCACTTCAAAATCAAAAGATAAATCTTTTCTTAAGAAAA
Sequence number 638: Borrelia hermsii 16S rRNA #4 
CATTTATTCTCTTTGAGCATGCCACTTCAAAATCAAAAGATAAATCTTTTCTTAAGAAAAGTTCACAA
TTATTAAGAAGATCTCTATTATTAAACTTTAAATCTATATTTAAAAACTTAG
Sequence number 639: Borrelia hermsii 16S rRNA #5 
GTTCACAATTATTAAGAAGATCTCTATTATTAAACTTTAAATCTATATTTAAAAACTTAGTAAGCTTA
CCAGGCCCATTAGTTAACAATCCATCAACTTTTGGAGAAATAGGCTCAATAG
Sequence number 640: Borrelia hermsii 16S rRNA #6 
TAAGCTTACCAGGCCCATTAGTTAACAATCCATCAACTTTTGGAGAAATAGGCTCAATAGCTCTTATT
AAAACAGCATGGGGATTATGTTCATTAGATGCCACAACGTTTAACATGTAAT
Sequence number 641: Borrelia hermsii 16S rRNA #7 
CTCTTATTAAAACAGCATGGGGATTATGTTCATTAGATGCCACAACGTTTAACATGTAATACATACCA
TAAATCATATAAACATAAACATATCCCCCAATATTATACATGGCAATGGTAC
Sequence number 642: Borrelia hermsii 16S rRNA #8 
ACATACCATAAATCATATAAACATAAACATATCCCCCAATATTATACATGGCAATGGTACGATTTGTT
CTTCTTCCCCCATAAGCATGGCAGGCTTTATCTACTACACCCATATAAGCTT
Sequence number 643: Borrelia hermsii 16S rRNA #9 
GATTTGTTCTTCTTCCCCCATAAGCATGGCAGGCTTTATCTACTACACCCATATAAGCTTCCGTCTCG
ACAATTCTTGAAATAATTTCTTTGCCATCTATTTTTCTAATCAGCAAGTGCC
Sequence number 644: Borrelia hermsii 16S rRNA #10 
CCGTCTCGACAATTCTTGAAATAATTTCTTTGCCATCTATTTTTCTAATCAGCAAGTGCCCAAGCAAA
GACTCAGCCACAACAATAGCATCCTGCATAAAAAACTCTCTATTCATTAGTA
Sequence number 645: Borrelia hermsii 16S rRNA #11 
CAAGCAAAGACTCAGCCACAACAATAGCATCCTGCATAAAAAACTCTCTATTCATTAGTACAATTCTA
AACTAAAACAAAAATCAAATTCAAAACTTATTGTTTCAAAACTTATTGCAAT
Sequence number 646: Borrelia hermsii 16S rRNA #12 
CAATTCTAAACTAAAACAAAAATCAAATTCAAAACTTATTGTTTCAAAACTTATTGCAATTGGCACAA
GTGTTAAATAACAATTAAAAATAACAGACAATTATCATGGAAAAATAAAGCG
Sequence number 647: Borrelia hermsii 16S rRNA #13 TGGCACAAGTGTTAAATAACAATTAAAAATAACAGACAATTATCATGGAAAAATAAAGCGTATATTGA CAAACGCAATAAAGTTAAATAGAATCTTAAAGGGTAAGAATATTTTAGGGAT
Sequence number 648: Borrelia hermsii 16S rRNA #14 
TATATTGACAAACGCAATAAAGTTAAATAGAATCTTAAAGGGTAAGAATATTTTAGGGATCATAGCTC
AGGTGGTTAGAGCGCAGGTCTGATAAACCTGAGGTCGGAGGTTCAACTCCTC
Sequence number 649: Borrelia hermsii 16S rRNA #15 
CATAGCTCAGGTGGTTAGAGCGCAGGTCTGATAAACCTGAGGTCGGAGGTTCAACTCCTCCTGGTCCC
AATTTTACACTCAAATCCTTTTAAAGATCTAATGAGCTAGGTAAAATAGTTC
Sequence number 650: Borrelia hermsii 16S rRNA #16 
CTGGTCCCAATTTTACACTCAAATCCTTTTAAAGATCTAATGAGCTAGGTAAAATAGTTCAAAACGTA
TTTTTGAAAGTTTGTTCTTAAGTTTTAGTCAAGCTTCACATCTGCAATAAAA
Sequence number 651: Borrelia hermsii 16S rRNA #17 
AAAACGTATTTTTGAAAGTTTGTTCTTAAGTTTTAGTCAAGCTTCACATCTGCAATAAAAATTTATCA
CTAAACCACAAAGAATGGGTAATATAATAATTAAAACCACAAGCTTAAATAT
Sequence number 652: Borrelia hermsii 16S rRNA #18 
ATTTATCACTAAACCACAAAGAATGGGTAATATAATAATTAAAACCACAAGCTTAAATATTAAACCAT
GTATTATATTCATATATTATATTGATTCACTCAAGATAAACACAAACATCAA
Sequence number 653: Borrelia hermsii 16S rRNA #19 
TAAACCATGTATTATATTCATATATTATATTGATTCACTCAAGATAAACACAAACATCAACCATATTA
AACTTTACATGAAGTAATATTATTTCCTACAAACTACAAAAACTTAAATTAA
Sequence number 654: Borrelia hermsii 16S rRNA #20 
CCATATTAAACTTTACATGAAGTAATATTATTTCCTACAAACTACAAAAACTTAAATTAATCTAAATA
AGATAACTTAAAAATAACATGAATAACATATCGTTATTTTTTATTATCTATC
Sequence number 655: Borrelia hermsii 16S rRNA #21 
TCTAAATAAGATAACTTAAAAATAACATGAATAACATATCGTTATTTTTTATTATCTATCCTATAGAT
GCAATCTATTGACCTAATAAATTCTTAAACTTAAGCAAAAATTTTAAAAAAG
Sequence number 656: Borrelia hermsii 16S rRNA #22 
CTATAGATGCAATCTATTGACCTAATAAATTCTTAAACTTAAGCAAAAATTTTAAAAAAGAAATGTTT
TATTTGGACATCTAAATTTTAATTAGATTATTTTTAATCATTAAAGACAATT
Sequence number 657: Borrelia hermsii 16S rRNA #23 
AAATGTTTTATTTGGACATCTAAATTTTAATTAGATTATTTTTAATCATTAAAGACAATTTTAATTGA
TAAATCACAGGCTGATATTTAAAAGATACAACCTCCATTCATTTTTGCAATA
Sequence number 658: Borrelia hermsii 16S rRNA #24 
TTAATTGATAAATCACAGGCTGATATTTAAAAGATACAACCTCCATTCATTTTTGCAATAAACAAATG
GAGGTTAAGGGACTCGAACCCTTGACCCCCGGCTTGCAAAGCCGATGCTCTA
Sequence number 659: Borrelia hermsii 16S rRNA #25 
AACAAATGGAGGTTAAGGGACTCGAACCCTTGACCCCCGGCTTGCAAAGCCGATGCTCTAAACCAACT
GAGCTAAACCCCCAAAAAAATCTTTGAAAAGACAAAGGAAGAGTTAAAATAA
Sequence number 660: Borrelia hermsii 16S rRNA #26 
AACCAACTGAGCTAAACCCCCAAAAAAATCTTTGAAAAGACAAAGGAAGAGTTAAAATAATGCTTTAC
CTTAAATAATTAAATCATTTCTTTCTCTTAGAAAGGAGGTGATCCAGCCACA
Sequence number 661: Borrelia hermsii 16S rRNA #27 
TGCTTTACCTTAAATAATTAAATCATTTCTTTCTCTTAGAAAGGAGGTGATCCAGCCACACTTTCCAG
TACGGCTACCTTGTTACGACTTCACCCCCCTCACTAAACATACCTTAGATAC
Sequence number 662: Borrelia hermsii 16S rRNA #28 
CTTTCCAGTACGGCTACCTTGTTACGACTTCACCCCCCTCACTAAACATACCTTAGATACCTTCCTCC
CTTGCGGGTTAGAATAATAGCTTCGGGTATCCTCAACTCGGGTGGTGTGACG
Sequence number 663: Borrelia hermsii 16S rRNA #29 
CTTCCTCCCTTGCGGGTTAGAATAATAGCTTCGGGTATCCTCAACTCGGGTGGTGTGACGGGCGGTGT
GTACAAGGCCCGAGAACGTATTCACCGTATCATTCTGATATACGATTACTAG
Sequence number 664: Borrelia hermsii 16S rRNA #30 
GGCGGTGTGTACAAGGCCCGAGAACGTATTCACCGTATCATTCTGATATACGATTACTAGCGATTCCA
ACTTCATGAAGTCGAGTTTCAGACTTCAATCTGGACTGAGACCTGCTTTATG
Sequence number 665: Borrelia hermsii 16S rRNA #31 
CGATTCCAACTTCATGAAGTCGAGTTTCAGACTTCAATCTGGACTGAGACCTGCTTTATGCGTTTTGC
TTCACATCACTGTTTCGCGTCGCTTTGTACAGGCCATTGTAGCACGTGTGTA
Sequence number 666: Borrelia hermsii 16S rRNA #32 
CGTTTTGCTTCACATCACTGTTTCGCGTCGCTTTGTACAGGCCATTGTAGCACGTGTGTAGCCCAGGA
CATAAGGGCCATGATGATTTGACGTCATCCTCACCTTCCTCCGGCTTATCAC
Sequence number 667: Borrelia hermsii 16S rRNA #33 
GCCCAGGACATAAGGGCCATGATGATTTGACGTCATCCTCACCTTCCTCCGGCTTATCACCGGCAGTC
TCGTCTGAGTCCCCATCTTTACATGCTGGTAACAGACAATAAGGGTTGCGCT
Sequence number 668: Borrelia hermsii 16S rRNA #34 
CGGCAGTCTCGTCTGAGTCCCCATCTTTACATGCTGGTAACAGACAATAAGGGTTGCGCTCGTTGCGG
GACTTAACCCAACACCTCACAGCACGAGCTGACGACAACCATGCAGCACCTG
Sequence number 669: Borrelia hermsii 16S rRNA #35 
CGTTGCGGGACTTAACCCAACACCTCACAGCACGAGCTGACGACAACCATGCAGCACCTGTATACAGA
CCCCAAACGGGGAATAGTTATCTCTAACTACATCCTGTATATGTCAAGCCCT
Sequence number 670: Borrelia hermsii 16S rRNA #36 
TATACAGACCCCAAACGGGGAATAGTTATCTCTAACTACATCCTGTATATGTCAAGCCCTGGTAAGGT
TCCTCGCGTATCATCGAATTAAACCACATGCTCCACCGCTTGTGCGGGCCCC
Sequence number 671: Borrelia hermsii 16S rRNA #37 
GGTAAGGTTCCTCGCGTATCATCGAATTAAACCACATGCTCCACCGCTTGTGCGGGCCCCCGTCAATT
CCTTTGAGTTTCACTCTTGCGAGCATACTCCCCAGGCGGCACACTTAACACG
Sequence number 672: Borrelia hermsii 16S rRNA #38 
CGTCAATTCCTTTGAGTTTCACTCTTGCGAGCATACTCCCCAGGCGGCACACTTAACACGTTAGCTTC
GGTACTAACCTTTCGATTAACACCAAGTGTGCATCGTTTACAGCGTAGACTA
Sequence number 673: Borrelia hermsii 16S rRNA #39 
TTAGCTTCGGTACTAACCTTTCGATTAACACCAAGTGTGCATCGTTTACAGCGTAGACTACCAGGGTA
TCTAATCCTGTTTGCTCCCTACGCTTTCGTGACTCAGCGTCAGTCTTGACCC
Sequence number 674: Borrelia hermsii 16S rRNA #40 
CCAGGGTATCTAATCCTGTTTGCTCCCTACGCTTTCGTGACTCAGCGTCAGTCTTGACCCAGAAGTTC
GCCTTCGCCTCTGGTATTCTTCCTGATATCAACAGATTCCACCCTTACACCA
Sequence number 675: Borrelia hermsii 16S rRNA #41 AGAAGTTCGCCTTCGCCTCTGGTATTCTTCCTGATATCAACAGATTCCACCCTTACACCAGGAATTCT AACTTCCCCTATCAGACTCTAGTCATGCAGTTTCCAGCATATTCCCACAGTT
Sequence number 676: Borrelia hermsii 16S rRNA #42 
GGAATTCTAACTTCCCCTATCAGACTCTAGTCATGCAGTTTCCAGCATATTCCCACAGTTGAGCTGTG
GTATTTTACGCATAGACTTGCATATCCGCCTACTCACCCTTTACGCCCAATA
Sequence number 677: Borrelia hermsii 16S rRNA #43 
GAGCTGTGGTATTTTACGCATAGACTTGCATATCCGCCTACTCACCCTTTACGCCCAATAATCCCGAA
CAACGCTCGCCCCTTACGTATTACCGCGGCTGCTGGCACGTAATTAGCCGGG
Sequence number 678: Borrelia hermsii 16S rRNA #44 
ATCCCGAACAACGCTCGCCCCTTACGTATTACCGCGGCTGCTGGCACGTAATTAGCCGGGGCTTATTC
ATAAATTAACGTCATCACTTTGTCATTTCCTACAAAGCTTATTCCTCATTTA
Sequence number 679: Borrelia hermsii 16S rRNA #45 
GCTTATTCATAAATTAACGTCATCACTTTGTCATTTCCTACAAAGCTTATTCCTCATTTATAAAAGAA
CTTTACAATCTTTCGACCTTCTTCGTTCACGCAGTGTCGCTCCGTCAGGCTT
Sequence number 680: Borrelia hermsii 16S rRNA #46 
TAAAAGAACTTTACAATCTTTCGACCTTCTTCGTTCACGCAGTGTCGCTCCGTCAGGCTTTCGCCCAT
TGCGGAAGATTCTTAGCTGCTGCCTCCCGTAGGAGTCTGGACCGTATCTCAG
Sequence number 681: Borrelia hermsii 16S rRNA #47 
TCGCCCATTGCGGAAGATTCTTAGCTGCTGCCTCCCGTAGGAGTCTGGACCGTATCTCAGTTCCAGTG
TGACCGTTCACCCTCTCAGGCCGGTTACTTATCACAGCCTTGGTAGGCCTTT
Sequence number 682: Borrelia hermsii 16S rRNA #48 
TTCCAGTGTGACCGTTCACCCTCTCAGGCCGGTTACTTATCACAGCCTTGGTAGGCCTTTACCCCACC
AACTAGCTAATAAGACGCAGACTCATCTACAAGCGAAGCTTTAAAGGCTTCC
Sequence number 683: Borrelia hermsii 16S rRNA #49 
ACCCCACCAACTAGCTAATAAGACGCAGACTCATCTACAAGCGAAGCTTTAAAGGCTTCCTTTCATCA
ATTAACATCTTAATTGACCTTATTCGGTATTAGCTACTATTTCTAATAGTTA
Sequence number 684: Borrelia hermsii 16S rRNA #50 
TTTCATCAATTAACATCTTAATTGACCTTATTCGGTATTAGCTACTATTTCTAATAGTTATCCCCATC
TCATAGGTAGATTATCCACGCGTTACTCACCCGTTCGCCACTGAATGTATTG
Sequence number 685: Borrelia hermsii 16S rRNA #51 
TCCCCATCTCATAGGTAGATTATCCACGCGTTACTCACCCGTTCGCCACTGAATGTATTGCTACATTC
CGTCTGACTTGCATGCTTAAGACGCACTGCCAGCGTTAGTTCTAAGCCAGGA
Sequence number 686: Borrelia hermsii 16S rRNA #52 
CTACATTCCGTCTGACTTGCATGCTTAAGACGCACTGCCAGCGTTAGTTCTAAGCCAGGATCAAACTC
TTCGTTATTTTTATTTTCTTCTTTTAAAATTAACAGGTTATTCATTTTATTT
Sequence number 687: Borrelia hermsii 16S rRNA #53 
TCAAACTCTTCGTTATTTTATTTCTTCTTTAAAATTAACAGGTTATTCATTTATTTGGCTTATT
AAACCTTACTTAACTCTTCCCTTCTCTTCTCTTCCAATTCTCAATATCAACT
Sequence number 688: Borrelia hermsii 16S rRNA #54 
GGCTTATTAAACCTTACTTAACTCTTCCCTTCTCTTCTCTTCCAATTCTCAATATCAACTCTTACTAC
TTTATAACATACGTTAAGCTTTGTCAATACTTAAACATTTATTTTTTTATAT
Sequence number 689: Borrelia hermsii 16S rRNA #55 
CTTACTACTTTATAACATACGTTAAGCTTTGTCAATACTTAAACATTTATTTTTTTATATTACTATTT
TTTAACAGATTGTTTTCATGATCTTTTATTCTGCTATCTTGCACTAATCGCC
Sequence number 690: Borrelia hermsii 16S rRNA #56 
TACTATTTTTTAACAGATTGTTTTCATGATCTTTTATTCTGCTATCTTGCACTAATCGCCTAACAACA
CCTAAAACACTGCCTATACCCTCATATCCATTATAATGATTGCCTCTTAAAT
Sequence number 691: Borrelia hermsii 16S rRNA #57 
TAACAACACCTAAAACACTGCCTATACCCTCATATCCATTATAATGATTGCCTCTTAAATTAGCACCA
AAGACACTTTTAAATCCAGCTTTCAAAGCTGCAATAACCTTACTCTCAATAT
Sequence number 692: Borrelia hermsii 16S rRNA #58 
TAGCACCAAAGACACTTTTAAATCCAGCTTTCAAAGCTGCAATAACCTTACTCTCAATATTAGAGGAT
ACTTTAATTTCTCCTGAGAGAGAAATCTCACCTGTAAAGATTAACTCTTGAC
Sequence number 693: Borrelia hermsii 16S rRNA #59 
TAGAGGATACTTTAATTTCTCCTGAGAGAGAAATCTCACCTGTAAAGATTAACTCTTGACTGATAATA
ACATTAGTCTTAGCAGAAAATAATGCAACTAAAACGGCAAGTTCAATCTCTA
Sequence number 694: Borrelia hermsii 16S rRNA #60 
TGATAATAACATTAGTCTTAGCAGAAAATAATGCAACTAAAACGGCAAGTTCAATCTCTACATCATCA
ATCCTAAGCCCCCCAGAAACATTAACATAGATATCATCATTATTAAAATTAA
Sequence number 695: Borrelia hermsii 16S rRNA #61 
CATCATCAATCCTAAGCCCCCCAGAAACATTAACATAGATATCATCATTATTAAAATTAAGATTTAAA
TACTTACTAATAACAGCTAAAATTCTTGATATTTTTTTAGAATCTATTTTCT
Sequence number 696: Borrelia hermsii 16S rRNA #62 
GATTTAAATACTTACTAATAACAGCTAAAATTCTTGATATTTTTTTAGAATCTATTTTCTCTGAAAAA
ATTCTAGAAATATTCATGCCTGTCCTTGTAATTAAAGCCTGTATCTCAACAA
Sequence number 697: Borrelia hermsii 16S rRNA #63 
CTGAAAAAATTCTAGAAATATTCATGCCTGTCCTTGTAATTAAAGCCTGTATCTCAACAAATAAGACT
CTACTCCCCTCATTAATAATTCCAATAGCAATACCTGAGGATATCTCTTCTT
Sequence number 698: Borrelia hermsii 16S rRNA #64 
ATAAGACTCTACTCCCCTCATTAATAATTCCAATAGCAATACCTGAGGATATCTCTTCTTTCCTCTCC
AAAAAGATAGAAGATGGATCTTTAATCTCAACAAGTCCCAAACTCGTCATTT
Sequence number 699: Borrelia hermsii 16S rRNA #65 
TCCTCTCCAAAAAGATAGAAGATGGATCTTTAATCTCAACAAGTCCCAAACTCGTCATTTCAAAAATA
CCTATCTCATTAACAGTACCAAACCTATTCTTAGTAGCTCTAAGCATACGCA
Sequence number 700: Borrelia hermsii 16S rRNA #66 
CAAAAATACCTATCTCATTAACAGTACCAAACCTATTCTTAGTAGCTCTAAGCATACGCAAAGATTTT
TTTGCCTCTTCAAAATAAAAAACAGAATCTACCATATGCTCTATTATTTTTG
Sequence number 701: Borrelia hermsii 16S rRNA #67 
AAGATTTTTTTGCCTCTTCAAAATAAAAAACAGAATCTACCATATGCTCTATTATTTTTGGTCCCGCT
AAAATACCATCCTTTGTAACATGTCCCACTAAACATAAAGTTATATTCTTTC
Sequence number 702: Borrelia hermsii 16S rRNA #68 
GTCCCGCTAAAATACCATCCTTTGTAACATGTCCCACTAAACATAAAGTTATATTCTTTCCCTTTGCC
CACTCTACAAGCTTGTAAACACAATGTTTTAACTGAGCAACCCCGCCAAGAC
Sequence number 703: Borrelia hermsii 16S rRNA #69 CCTTTGCCCACTCTACAAGCTTGTAAACACAATGTTTTAACTGAGCAACCCCGCCAAGACTACCTTGC ACTTCTCTTGAATTCAAAGTTTGAATAGAATCAACAACTATAAAGTCAAGCT
Sequence number 704: Borrelia hermsii 16S rRNA #70 
TACCTTGCACTTCTCTTGAATTCAAAGTTTGAATAGAATCAACAACTATAAAGTCAAGCTCAATATTT
GCAAGCATTTCTATTAAAGCATCAACATCTACCTCATTAGTTATCAATATAT
Sequence number 705: Borrelia hermsii 16S rRNA #71 
CAATATTTGCAAGCATTTCTATTAAAGCATCAACATCTACCTCATTAGTTATCAATATATCAGAAGAA
ATTTTAAGCCTATTTGCTCTCAACTTAATTTGTGGAATTGATTCCTCACCCG
Sequence number 706: Borrelia hermsii 16S rRNA #72 
CAGAAGAAATTTTAAGCCTATTTGCTCTCAACTTAATTTGTGGAATTGATTCCTCACCCGCAAGATAA
AGCACATTCTTGCCATCAAGTGAGAGAATACTAGAAATCTGAAGTAAAAAG
Sequence number 707: Borrelia hermsii 16S rRNA #73 
CAAGATAAAGCACATTCTTGCCATCAAGTGAGAGAATACTAGAAATCTGAAGTAAAAAAGTTGATTTT
CCAATGCCAGGCTCTCCTGATATCAAAATTGCACTACCAATTACAATCCCAG
Sequence number 708: Borrelia hermsii 16S rRNA #74 
TTGATTTTCCAATGCCAGGCTCTCCTGATATCAAAATTGCACTACCAATTACAATCCCAGCACCAAGA
ACTCTATCAAACTCTTCAATGCCTGTTAAATACCTAACATTATCAACTTGCT
Sequence number 709: Borrelia hermsii 16S rRNA #75 
CACCAAGAACTCTATCAAACTCTTCAATGCCTGTTAAATACCTAACATTATCAACTTGCTTAAAATCT
TTTAAAGAAAAAATCTCACTTTTTGACTTACTTAAGTTTGTCCCATAACCAG
Sequence number 710: Borrelia hermsii 16S rRNA #76 
TAAAATCTTTTAAAGAAAAAATCTCACTTTTTGACTTACTTAAGTTTGTCCCATAACCAGATTTAGGT
TCAACATAATGCTCTAAACTTTCCCAACTAAAACACTCAGGACACTTCCCTA
Sequence number 711: Borrelia hermsii 16S rRNA #77 
ATTTAGGTTCAACATAATGCTCTAAACTTTCCCAACTAAAACACTCAGGACACTTCCCTAGCCATTTT
AAAGATTTATATCCACAATTTAAACATTTATAAATTTCCTTATCTTTCTTTT
Sequence number 712: Borrelia hermsii 16S rRNA #78 
CCTAGCCATTTTAAAGATTTATATCCACAATTTAAACATTTATAAATTTCCTTATCTTTCTTTTTTGC
CATAAAACCTCAATAGGTAAACTTTTTTTCATTTGGACAAGTGATAAAACTA
Sequence number 713: Chikungunya virus Whole genome #1 
TGAGACACACGTAGCCTACCAGTTTCTTACTGCTCTACTCTGCAAAGCAAGAGATTAAGAACCCATCA
TGGATCCTGTGTACGTGGACATAGACGCTGACAGCGCCTTTTTGAAGGCCCT
Sequence number 714: Chikungunya virus Whole genome #2 
ACCCATCATGGATCCTGTGTACGTGGACATAGACGCTGACAGCGCCTTTTTGAAGGCCCTGCAACGTG
CGTACCCCATGTTTGAGGTGGAACCTAGGCAGGTCACACCGAATGACCATGC
Sequence number 715: Chikungunya virus Whole genome #3 
GCAACGTGCGTACCCCATGTTTGAGGTGGAACCTAGGCAGGTCACACCGAATGACCATGCTAATGCTA
GAGCGTTCTCGCATCTAGCTATAAAACTAATAGAGCAGGAAATTGATCCCGA
Sequence number 716: Chikungunya virus Whole genome #4 
TAATGCTAGAGCGTTCTCGCATCTAGCTATAAAACTAATAGAGCAGGAAATTGATCCCGACTCAACCA
TCCTGGATATTGGTAGTGCGCCAGCAAGGAGGATGATGTCGGACAGGAAGTA
Sequence number 717: Chikungunya virus Whole genome #5 
CTCAACCATCCTGGATATTGGTAGTGCGCCAGCAAGGAGGATGATGTCGGACAGGAAGTACCACTGCG
TTTGCCCGATGCGCAGTGCAGAAGATCCCGAGAGACTCGCCAATTATGCGAG
Sequence number 718: Chikungunya virus Whole genome #6 
CCACTGCGTTTGCCCGATGCGCAGTGCAGAAGATCCCGAGAGACTCGCCAATTATGCGAGAAAGCTAG
CATCTGCCGCAGGAAAAGTCCTGGACAGAAACATCTCTGGAAAGATCGGGGA
Sequence number 719: Chikungunya virus Whole genome #7 
AAAGCTAGCATCTGCCGCAGGAAAAGTCCTGGACAGAAACATCTCTGGAAAGATCGGGGACTTACAAG
CAGTAATGGCCGTGCCAGACACGGAGACGCCAACATTCTGCTTACACACAGA
Sequence number 720: Chikungunya virus Whole genome #8 
CTTACAAGCAGTAATGGCCGTGCCAGACACGGAGACGCCAACATTCTGCTTACACACAGATGTATCAT
GTAGACAGAGAGCAGACGTCGCGATATACCAAGACGTCTATGCTGTACACGC
Sequence number 721: Chikungunya virus Whole genome #9 
TGTATCATGTAGACAGAGAGCAGACGTCGCGATATACCAAGACGTCTATGCTGTACACGCACCCACGT
CGCTATACCACCAGGCGATTAAAGGGGTCCGATTGGCGTACTGGGTAGGGTT
Sequence number 722: Chikungunya virus Whole genome #10 
ACCCACGTCGCTATACCACCAGGCGATTAAAGGGGTCCGATTGGCGTACTGGGTAGGGTTTGACACAA
CCCCGTTCATGTACAATGCCATGGCGGGTGCCTACCCCTCATACTCGACAAA
Sequence number 723: Chikungunya virus Whole genome #11 
TGACACAACCCCGTTCATGTACAATGCCATGGCGGGTGCCTACCCCTCATACTCGACAAATTGGGCAG
ATGAGCAGGTACTGAAGGCTAAGAACATAGGATTATGTTCAACAGACCTGAC
Sequence number 724: Chikungunya virus Whole genome #12 
TTGGGCAGATGAGCAGGTACTGAAGGCTAAGAACATAGGATTATGTTCAACAGACCTGACGGAAGGTA
GACGAGGCAAATTGTCTATTATGAGAGGAAAAAAGCTAGAACCGTGCGACCG
Sequence number 725: Chikungunya virus Whole genome #13 
GGAAGGTAGACGAGGCAAATTGTCTATTATGAGAGGAAAAAAGCTAGAACCGTGCGACCGTGTGCTGT
TCTCAGTAGGGTCAACGCTCTACCCGGAAAGCCGTAAGCTACTTAAGAGCTG
Sequence number 726: Chikungunya virus Whole genome #14 
TGTGCTGTTCTCAGTAGGGTCAACGCTCTACCCGGAAAGCCGTAAGCTACTTAAGAGCTGGCACCTAC
CATCGGTGTTCCATTTAAAGGGCAAGCTCAGCTTCACATGCCGCTGTGATAC
Sequence number 727: Chikungunya virus Whole genome #15 
GCACCTACCATCGGTGTTCCATTTAAAGGGCAAGCTCAGCTTCACATGCCGCTGTGATACAGTGGTTT
CGTGCGAAGGCTACGTCGTTAAGAGAATAACGATGAGCCCAGGCCTTTACGG
Sequence number 728: Chikungunya virus Whole genome #16 
AGTGGTTTCGTGCGAAGGCTACGTCGTTAAGAGAATAACGATGAGCCCAGGCCTTTACGGAAAAACCA
CAGGGTATGCGGTAACCCACCACGCAGACGGATTCCTGATGTGCAAGACCAC
Sequence number 729: Chikungunya virus Whole genome #17 
AAAAACCACAGGGTATGCGGTAACCCACCACGCAGACGGATTCCTGATGTGCAAGACCACCGACACGG
TTGACGGCGAAAGAGTGTCATTCTCGGTGTGCACGTACGTGCCGGCGACCAT
Sequence number 730: Chikungunya virus Whole genome #18 
CGACACGGTTGACGGCGAAAGAGTGTCATTCTCGGTGTGCACGTACGTGCCGGCGACCATTTGTGATC
AAATGACCGGCATCCTTGCTACAGAAGTCACGCCGGAGGATGCACAGAAGCT
Sequence number 731: Chikungunya virus Whole genome #19 TTGTGATCAAATGACCGGCATCCTTGCTACAGAAGTCACGCCGGAGGATGCACAGAAGCTGTTGGTGG GGCTGAACCAGAGAATAGTGGTTAACGGCAGAACGCAACGGAATACGAACAC
Sequence number 732: Chikungunya virus Whole genome #20 
GTTGGTGGGGCTGAACCAGAGAATAGTGGTTAACGGCAGAACGCAACGGAATACGAACACCATGAAAA
ACTATATGATTCCCGTGGTCGCCCAAGCCTTCAGTAAGTGGGCAAAGGAGTG
Sequence number 733: Chikungunya virus Whole genome #21 
CATGAAAAACTATATGATTCCCGTGGTCGCCCAAGCCTTCAGTAAGTGGGCAAAGGAGTGCCGGAAAG
ACATGGAAGATGAAAAACTCCTGGGGGTCAGAGAAAGAACACTGACCTGCTG
Sequence number 734: Chikungunya virus Whole genome #22 
CCGGAAAGACATGGAAGATGAAAAACTCCTGGGGGTCAGAGAAAGAACACTGACCTGCTGCTGTCTAT
GGGCATTTAAGAAGCAGAAAACACACACGGTCTACAAGAGGCCTGATACCCA
Sequence number 735: Chikungunya virus Whole genome #23 
CTGTCTATGGGCATTTAAGAAGCAGAAAACACACACGGTCTACAAGAGGCCTGATACCCAGTCAATTC
AGAAGGTTCAGGCCGAGTTTGACAGCTTTGTGGTACCGAGCCTGTGGTCGTC
Sequence number 736: Chikungunya virus Whole genome #24 
GTCAATTCAGAAGGTTCAGGCCGAGTTTGACAGCTTTGTGGTACCGAGCCTGTGGTCGTCCGGGTTGT
CAATCCCGTTGAGGACTAGAATCAAATGGTTGTTAAGCAAGGTGCCAAAAAC
Sequence number 737: Chikungunya virus Whole genome #25 
CGGGTTGTCAATCCCGTTGAGGACTAGAATCAAATGGTTGTTAAGCAAGGTGCCAAAAACCGACCTGA
CCCCATACAGCGGGGACGCCCAAGAAGCCCGGGACGCAGAAAAAGAAGCAGA
Sequence number 738: Chikungunya virus Whole genome #26 
CGACCTGACCCCATACAGCGGGGACGCCCAAGAAGCCCGGGACGCAGAAAAAGAAGCAGAGGAAGAAC
GAGAAGCAGAACTGACTCTTGAAGCCCTACCACCCCTTCAGGCAGCACAGGA
Sequence number 739: Chikungunya virus Whole genome #27 
GGAAGAACGAGAAGCAGAACTGACTCTTGAAGCCCTACCACCCCTTCAGGCAGCACAGGAAGATGTTC
AGGTCGAAATCGACGTGGAACAGCTTGAGGACAGAGCGGGTGCAGGAATAAT
Sequence number 740: Chikungunya virus Whole genome #28 
AGATGTTCAGGTCGAAATCGACGTGGAACAGCTTGAGGACAGAGCGGGTGCAGGAATAATAGAGACTC
CGAGAGGAGCTATCAAAGTTACTGCCCAACCAACAGACCACGTCGTGGGAGA
Sequence number 741: Chikungunya virus Whole genome #29 
AGAGACTCCGAGAGGAGCTATCAAAGTTACTGCCCAACCAACAGACCACGTCGTGGGAGAGTACTTGG
TTCTTTCCCCGCAGACCGTACTACGTAGCCAAAAGCTTAGCCTGATTCACGC
Sequence number 742: Chikungunya virus Whole genome #30 
GTACTTGGTTCTTTCCCCGCAGACCGTACTACGTAGCCAAAAGCTTAGCCTGATTCACGCTTTGGCGG
AGCAAGTGAAGACGTGCACGCACAGCGGACGAGCAGGGAGGTATGCGGTCGA
Sequence number 743: Chikungunya virus Whole genome #31 
TTTGGCGGAGCAAGTGAAGACGTGCACGCACAGCGGACGAGCAGGGAGGTATGCGGTCGAAGCGTACG
ACGGCAGAGTCCTAGTGCCCTCAGGCTACGCAATCTCGCCTGAAGACTTCCA
Sequence number 744: Chikungunya virus Whole genome #32 
AGCGTACGACGGCAGAGTCCTAGTGCCCTCAGGCTACGCAATCTCGCCTGAAGACTTCCAGAGCCTAA
GCGAAAGCGCAACGATGGTGTACAACGAAAGAGAGTTCGTAAACAGAAAGCT
Sequence number 745: Chikungunya virus Whole genome #33 
GAGCCTAAGCGAAAGCGCAACGATGGTGTACAACGAAAGAGAGTTCGTAAACAGAAAGCTACACCATA
TTGCGATGCATGGACCAGCCCTGAACACCGACGAAGAGTCGTATGAGCTGGT
Sequence number 746: Chikungunya virus Whole genome #34 
ACACCATATTGCGATGCATGGACCAGCCCTGAACACCGACGAAGAGTCGTATGAGCTGGTGAGGGCAG
AGAGGACAGAACACGAGTACGTCTACGACGTGGACCAGAGAAGATGCTGTAA
Sequence number 747: Chikungunya virus Whole genome #35 
GAGGGCAGAGAGGACAGAACACGAGTACGTCTACGACGTGGACCAGAGAAGATGCTGTAAGAAGGAAG
AAGCTGCAGGACTGGTACTGGTGGGCGACTTGACTAATCCGCCCTACCACGA
Sequence number 748: Chikungunya virus Whole genome #36 
GAAGGAAGAAGCTGCAGGACTGGTACTGGTGGGCGACTTGACTAATCCGCCCTACCACGAATTCGCAT
ATGAAGGGCTAAAAATCCGCCCTGCCTGCCCATACAAAATTGCAGTCATAGG
Sequence number 749: Chikungunya virus Whole genome #37 
ATTCGCATATGAAGGGCTAAAAATCCGCCCTGCCTGCCCATACAAAATTGCAGTCATAGGAGTCTTCG
GAGTACCAGGATCTGGCAAGTCAGCTATTATCAAGAACCTAGTTACCAGGCA
Sequence number 750: Chikungunya virus Whole genome #38 
AGTCTTCGGAGTACCAGGATCTGGCAAGTCAGCTATTATCAAGAACCTAGTTACCAGGCAAGACCTGG
TGACTAGCGGAAAGAAAGAAAACTGCCAAGAAATCACCACCGACGTGATGAG
Sequence number 751: Chikungunya virus Whole genome #39 
AGACCTGGTGACTAGCGGAAAGAAAGAAAACTGCCAAGAAATCACCACCGACGTGATGAGACAGAGAG
GTCTAGAGATATCTGCACGTACGGTTGACTCGCTGCTCTTGAATGGATGTAA
Sequence number 752: Chikungunya virus Whole genome #40 
ACAGAGAGGTCTAGAGATATCTGCACGTACGGTTGACTCGCTGCTCTTGAATGGATGTAACAGACCAG
TCGACGTGTTGTACGTAGACGAGGCGTTTGCGTGCCACTCTGGAACGTTACT
Sequence number 753: Chikungunya virus Whole genome #41 
CAGACCAGTCGACGTGTTGTACGTAGACGAGGCGTTTGCGTGCCACTCTGGAACGTTACTTGCATTGA
TCGCCTTGGTGAGACCAAGACAGAAAGTTGTACTTTGTGGTGACCCGAAGCA
Sequence number 754: Chikungunya virus Whole genome #42 
TGCATTGATCGCCTTGGTGAGACCAAGACAGAAAGTTGTACTTTGTGGTGACCCGAAGCAGTGCGGCT
TCTTCAATATGATGCAGATGAAAGTCAACTATAATCACAACATCTGCACCCA
Sequence number 755: Chikungunya virus Whole genome #43 
GTGCGGCTTCTTCAATATGATGCAGATGAAAGTCAACTATAATCACAACATCTGCACCCAAGTGTACC
ACAAAAGTATCTCCAGGCGGTGTACACTGCCTGTGACTGCCATTGTGTCATC
Sequence number 756: Chikungunya virus Whole genome #44 
AGTGTACCACAAAAGTATCTCCAGGCGGTGTACACTGCCTGTGACTGCCATTGTGTCATCGTTGCATT
ACGAAGGCAAAATGCGCACTACGAATGAGTACAACAAGCCGATTGTAGTGGA
Sequence number 757: Chikungunya virus Whole genome #45 
GTTGCATTACGAAGGCAAAATGCGCACTACGAATGAGTACAACAAGCCGATTGTAGTGGACACTACAG
GCTCAACAAAACCTGACCCTGGAGATCTCGTGTTAACGTGCTTCAGAGGATG
Sequence number 758: Chikungunya virus Whole genome #46 
CACTACAGGCTCAACAAAACCTGACCCTGGAGATCTCGTGTTAACGTGCTTCAGAGGATGGGTTAAAC
AACTGCAAATTGACTATCGTGGACACGAGGTCATGACAGCAGCCGCATCCCA
Sequence number 759: Chikungunya virus Whole genome #47 GGTTAAACAACTGCAAATTGACTATCGTGGACACGAGGTCATGACAGCAGCCGCATCCCAAGGGTTAA CCAGAAAAGGAGTTTACGCAGTTAGGCAAAAAGTTAACGAAAACCCGCTTTA
Sequence number 760: Chikungunya virus Whole genome #48 
AGGGTTAACCAGAAAAGGAGTTTACGCAGTTAGGCAAAAAGTTAACGAAAACCCGCTTTATGCATCAA
CGTCAGAGCACGTCAACGTACTCCTAACGCGTACGGAAGGTAAACTGGTATG
Sequence number 761: Chikungunya virus Whole genome #49 
TGCATCAACGTCAGAGCACGTCAACGTACTCCTAACGCGTACGGAAGGTAAACTGGTATGGAAGACAC
TCTCCGGTGACCCGTGGATAAAGACGCTGCAGAACCCACCGAAAGGAAACTT
Sequence number 762: Chikungunya virus Whole genome #50 
GAAGACACTCTCCGGTGACCCGTGGATAAAGACGCTGCAGAACCCACCGAAAGGAAACTTCAAAGCAA
CTATTAAGGAGTGGGAGGTGGAGCATGCATCAATAATGGCGGGCATCTGCAG
Sequence number 763: Chikungunya virus Whole genome #51 
CAAAGCAACTATTAAGGAGTGGGAGGTGGAGCATGCATCAATAATGGCGGGCATCTGCAGTCACCAAA
TGACCTTTGATACATTCCAAAACAAAGCCAACGTTTGTTGGGCTAAGAGTTT
Sequence number 764: Chikungunya virus Whole genome #52 
TCACCAAATGACCTTTGATACATTCCAAAACAAAGCCAACGTTTGTTGGGCTAAGAGTTTGGTCCCTA
TCCTCGAAACAGCGGGGATAAAACTAAACGACAGGCAGTGGTCCCAGATAAT
Sequence number 765: Chikungunya virus Whole genome #53 
GGTCCCTATCCTCGAAACAGCGGGGATAAAACTAAACGACAGGCAGTGGTCCCAGATAATTCAAGCCT
TCAAAGAAGACAAAGCATATTCACCCGAAGTAGCCCTGAATGAAATATGCAC
Sequence number 766: Chikungunya virus Whole genome #54 
TCAAGCCTTCAAAGAAGACAAAGCATATTCACCCGAAGTAGCCCTGAATGAAATATGCACGCGCATGT
ATGGGGTGGATCTAGACAGCGGGCTATTTTCTAAACCGTTGGTGTCTGTGTA
Sequence number 767: Chikungunya virus Whole genome #55 
GCGCATGTATGGGGTGGATCTAGACAGCGGGCTATTTTCTAAACCGTTGGTGTCTGTGTATTACGCGG
ATAACCACTGGGATAATAGGCCTGGAGGGAAGATGTTCGGATTCAACCCCGA
Sequence number 768: Chikungunya virus Whole genome #56 
TTACGCGGATAACCACTGGGATAATAGGCCTGGAGGGAAGATGTTCGGATTCAACCCCGAGGCAGCAT
CCATTCTAGAAAGAAAGTATCCATTTACAAAAGGGAAGTGGAACATCAACAA
Sequence number 769: Chikungunya virus Whole genome #57 
GGCAGCATCCATTCTAGAAAGAAAGTATCCATTTACAAAAGGGAAGTGGAACATCAACAAGCAGATCT
GCGTGACTACCAGGAGGATAGAAGACTTCAACCCTACCACCAACATTATACC
Sequence number 770: Chikungunya virus Whole genome #58 
GCAGATCTGCGTGACTACCAGGAGGATAGAAGACTTCAACCCTACCACCAACATTATACCGGCCAACA
GGAGACTACCACACTCATTAGTGGCCGAACACCGCCCAGTAAAAGGGGAAAG
Sequence number 771: Chikungunya virus Whole genome #59 
GGCCAACAGGAGACTACCACACTCATTAGTGGCCGAACACCGCCCAGTAAAAGGGGAAAGAATGGAAT
GGCTGGTTAACAAGATAAACGGCCACCACGTGCTCCTGGTCAGTGGCTGTAG
Sequence number 772: Chikungunya virus Whole genome #60 
AATGGAATGGCTGGTTAACAAGATAAACGGCCACCACGTGCTCCTGGTCAGTGGCTGTAGCCTTGCAC
TGCCTACTAAGAGAGTCACTTGGGTAGCGCCACTAGGTGTCCGCGGAGCGGA
Sequence number 773: Chikungunya virus Whole genome #61 
CCTTGCACTGCCTACTAAGAGAGTCACTTGGGTAGCGCCACTAGGTGTCCGCGGAGCGGACTATACAT
ACAACCTAGAGTTGGGTCTGCCAGCAACGCTTGGTAGGTATGACCTAGTGGT
Sequence number 774: Chikungunya virus Whole genome #62 
CTATACATACAACCTAGAGTTGGGTCTGCCAGCAACGCTTGGTAGGTATGACCTAGTGGTCATAAACA
TCCACACACCTTTTCGCATACACCATTATCAACAGTGCGTAGACCACGCAAT
Sequence number 775: Chikungunya virus Whole genome #63 
CATAAACATCCACACACCTTTTCGCATACACCATTATCAACAGTGCGTAGACCACGCAATGAAACTGC
AAATGCTCGGGGGTGACTCATTGAGACTGCTCAAACCGGGTGGCTCTCTATT
Sequence number 776: Chikungunya virus Whole genome #64 
GAAACTGCAAATGCTCGGGGGTGACTCATTGAGACTGCTCAAACCGGGTGGCTCTCTATTGATCAGAG
CATATGGTTACGCAGATAGAACCAGTGAACGAGTCATCTGCGTATTGGGACG
Sequence number 777: Chikungunya virus Whole genome #65 
GATCAGAGCATATGGTTACGCAGATAGAACCAGTGAACGAGTCATCTGCGTATTGGGACGCAAGTTTA
GATCATCTAGAGCGTTGAAACCACCATGTGTCACCAGCAACACTGAGATGTT
Sequence number 778: Chikungunya virus Whole genome #66 
CAAGTTTAGATCATCTAGAGCGTTGAAACCACCATGTGTCACCAGCAACACTGAGATGTTTTTTCTAT
TCAGCAACTTTGACAATGGCAGAAGGAATTTCACAACTCATGTCATGAACAA
Sequence number 779: Chikungunya virus Whole genome #67 
TTTTCTATTCAGCAACTTTGACAATGGCAGAAGGAATTTCACAACTCATGTCATGAACAATCAACTGA
ATGCAGCCTTTGTAGGACAGGCCACCCGAGCAGGATGTGCACCGTCGTACCG
Sequence number 780: Chikungunya virus Whole genome #68 
TCAACTGAATGCAGCCTTTGTAGGACAGGCCACCCGAGCAGGATGTGCACCGTCGTACCGGGTAAAAC
GCATGGATATCGCGAAGAACGATGAAGAGTGCGTAGTCAACGCCGCCAACCC
Sequence number 781: Chikungunya virus Whole genome #69 
GGTAAAACGCATGGATATCGCGAAGAACGATGAAGAGTGCGTAGTCAACGCCGCCAACCCTCGCGGGT
TACCAGGTGACGGTGTTTGCAAGGCAGTATACAAAAAATGGCCGGAGTCCTT
Sequence number 782: Chikungunya virus Whole genome #70 
TCGCGGGTTACCAGGTGACGGTGTTTGCAAGGCAGTATACAAAAAATGGCCGGAGTCCTTTAAGAACA
GTGCAACACCAGTGGGAACCGCAAAAACAGTCATGTGCGGTACGTATCCAGT
Sequence number 783: Chikungunya virus Whole genome #71 
TAAGAACAGTGCAACACCAGTGGGAACCGCAAAAACAGTCATGTGCGGTACGTATCCAGTAATCCACG
CCGTTGGACCAAACTTCTCTAATTATTCGGAGTCTGAAGGGGACCGAGAATT
Sequence number 784: Chikungunya virus Whole genome #72 
AATCCACGCCGTTGGACCAAACTTCTCTAATTATTCGGAGTCTGAAGGGGACCGAGAATTGGCGGCTG
CCTATCGAGAAGTCGCAAAGGAGGTAACTAGACTGGGAGTAAATAGTGTAGC
Sequence number 785: Chikungunya virus Whole genome #73 
GGCGGCTGCCTATCGAGAAGTCGCAAAGGAGGTAACTAGACTGGGAGTAAATAGTGTAGCTATACCTC
TCCTCTCCACAGGTGTATACTCAGGAGGGAAAGACAGGCTGACCCAGTCACT
Sequence number 786: Chikungunya virus Whole genome #74 
TATACCTCTCCTCTCCACAGGTGTATACTCAGGAGGGAAAGACAGGCTGACCCAGTCACTGAACCACC
TCTTTACAGCCATGGACTCGACGGATGCAGACGTGGTCATCTACTGCCGCGA
Sequence number 787: Chikungunya virus Whole genome #75 GAACCACCTCTTTACAGCCATGGACTCGACGGATGCAGACGTGGTCATCTACTGCCGCGACAAAGAAT GGGAGAAGAAAATATCTGAGGCCATACAGATGCGGACCCAAGTGGAGCTGCT
Sequence number 788: Chikungunya virus Whole genome #76 
CAAAGAATGGGAGAAGAAAATATCTGAGGCCATACAGATGCGGACCCAAGTGGAGCTGCTGGATGAGC
ACATCTCCATAGACTGCGATGTTGTTCGCGTGCACCCTGACAGCAGCTTGGC
Sequence number 789: Chikungunya virus Whole genome #77 
GGATGAGCACATCTCCATAGACTGCGATGTTGTTCGCGTGCACCCTGACAGCAGCTTGGCAGGCAGAA
AAGGATACAGCACCACGGAAGGCGCACTGTACTCATATCTAGAAGGGACCCG
Sequence number 790: Chikungunya virus Whole genome #78 
AGGCAGAAAAGGATACAGCACCACGGAAGGCGCACTGTACTCATATCTAGAAGGGACCCGTTTTCACC
AAACGGCAGTGGATATGGCAGAGATATATACTATGTGGCCAAAGCAAACAGA
Sequence number 791: Chikungunya virus Whole genome #79 
TTTTCACCAAACGGCAGTGGATATGGCAGAGATATATACTATGTGGCCAAAGCAAACAGAGGCCAACG
AGCAAGTTTGCCTATATGCCCTGGGGGAAAGTATTGAATCGATCAGGCAGAA
Sequence number 792: Chikungunya virus Whole genome #80 
GGCCAACGAGCAAGTTTGCCTATATGCCCTGGGGGAAAGTATTGAATCGATCAGGCAGAAATGCCCGG
TGGATGATGCAGATGCATCATCTCCCCCGAAAACTGTCCCGTGCCTCTGCCG
Sequence number 793: Chikungunya virus Whole genome #81 
ATGCCCGGTGGATGATGCAGATGCATCATCTCCCCCGAAAACTGTCCCGTGCCTCTGCCGTTACGCCA
TGACACCAGAACGCGTTACCCGACTTCGCATGAACCATGTCACAAGCATAAT
Sequence number 794: Chikungunya virus Whole genome #82 
TTACGCCATGACACCAGAACGCGTTACCCGACTTCGCATGAACCATGTCACAAGCATAATTGTGTGTT
CTTCGTTTCCCCTTCCAAAGTACAAAATAGAAGGAGTGCAAAAAGTCAAATG
Sequence number 795: Chikungunya virus Whole genome #83 
TGTGTGTTCTTCGTTTCCCCTTCCAAAGTACAAAATAGAAGGAGTGCAAAAAGTCAAATGCTCCAAGG
TAATGCTATTTGACCACAACGTGCCATCGCGCGTAAGTCCAAGGGAATACAG
Sequence number 796: Chikungunya virus Whole genome #84 
CTCCAAGGTAATGCTATTTGACCACAACGTGCCATCGCGCGTAAGTCCAAGGGAATACAGACCTTCCC
AGGAGTCTGTACAGGAAGCGAGTACGACCACGTCACTGACGCATAGCCAATT
Sequence number 797: Chikungunya virus Whole genome #85 
ACCTTCCCAGGAGTCTGTACAGGAAGCGAGTACGACCACGTCACTGACGCATAGCCAATTCGATCTAA
GCGTTGACGGCAAGATACTGCCCGTCCCGTCAGACCTGGATGCTGACGCCCC
Sequence number 798: Chikungunya virus Whole genome #86 
CGATCTAAGCGTTGACGGCAAGATACTGCCCGTCCCGTCAGACCTGGATGCTGACGCCCCAGCCCTAG
AACCAGCCCTTGACGACGGGGCGATACACACGTTGCCATCTGCAACCGGAAA
Sequence number 799: Chikungunya virus Whole genome #87 
AGCCCTAGAACCAGCCCTTGACGACGGGGCGATACACACGTTGCCATCTGCAACCGGAAACCTTGCGG
CCGTGTCTGACTGGGTAATGAGCACCGTACCTGTCGCGCCGCCCAGAAGAAG
Sequence number 800: Chikungunya virus Whole genome #88 
CCTTGCGGCCGTGTCTGACTGGGTAATGAGCACCGTACCTGTCGCGCCGCCCAGAAGAAGGCGAGGGA
GAAACCTGACTGTGACATGCGACGAGAGAGAAGGGAATATAACACCCATGGC
Sequence number 801: Chikungunya virus Whole genome #89 
GCGAGGGAGAAACCTGACTGTGACATGCGACGAGAGAGAAGGGAATATAACACCCATGGCTAGCGTCC
GATTCTTTAGGGCAGAGCTGTGTCCAGTCGTACAAGAAACAGCGGAGACGCG
Sequence number 802: Chikungunya virus Whole genome #90 
TAGCGTCCGATTCTTTAGGGCAGAGCTGTGTCCAGTCGTACAAGAAACAGCGGAGACGCGTGACACAG
CTATGTCTCTTCAGGCACCGCCGAGTACCGCCACGGAACTGAGTCACCCGCC
Sequence number 803: Chikungunya virus Whole genome #91 
TGACACAGCTATGTCTCTTCAGGCACCGCCGAGTACCGCCACGGAACTGAGTCACCCGCCGATCTCCT
TCGGTGCACCAAGCGAGACGTTCCCCATCACATTTGGGGACTTCAACGAAGG
Sequence number 804: Chikungunya virus Whole genome #92 
GATCTCCTTCGGTGCACCAAGCGAGACGTTCCCCATCACATTTGGGGACTTCAACGAAGGAGAAATCG
AAAGCTTGTCTTCTGAGCTACTAACTTTCGGAGACTTCCTACCCGGAGAAGT
Sequence number 805: Chikungunya virus Whole genome #93 
AGAAATCGAAAGCTTGTCTTCTGAGCTACTAACTTTCGGAGACTTCCTACCCGGAGAAGTGGATGATT
TGACAGATAGCGACTGGTCCACGTGCTCAGACACGGACGACGAGTTACGACT
Sequence number 806: Chikungunya virus Whole genome #94 
GGATGATTTGACAGATAGCGACTGGTCCACGTGCTCAGACACGGACGACGAGTTACGACTAGACAGGG
CAGGTGGGTATATATTCTCGTCGGACACTGGTCCAGGTCATTTACAACAGAA
Sequence number 807: Chikungunya virus Whole genome #95 
AGACAGGGCAGGTGGGTATATATTCTCGTCGGACACTGGTCCAGGTCATTTACAACAGAAGTCAGTAC
GCCAGTCAGTGCTGCCGGTGAACACCCTGGAGGAAGTCCACGAGGAGAAGTG
Sequence number 808: Chikungunya virus Whole genome #96 
GTCAGTACGCCAGTCAGTGCTGCCGGTGAACACCCTGGAGGAAGTCCACGAGGAGAAGTGTTACCCAC
CTAAGCTGGATGAAGCAAAGGAGCAACTACTACTTAAGAAACTCCAGGAGAG
Sequence number 809: Chikungunya virus Whole genome #97 
TTACCCACCTAAGCTGGATGAAGCAAAGGAGCAACTACTACTTAAGAAACTCCAGGAGAGTGCATCCA
TGGCCAACAGAAGCAGGTATCAGTCGCGCAAAGTAGAAAACATGAAAGCAAC
Sequence number 810: Chikungunya virus Whole genome #98 
TGCATCCATGGCCAACAGAAGCAGGTATCAGTCGCGCAAAGTAGAAAACATGAAAGCAACAATCATCC
AGAGACTAAAGAGAGGCTGTAGATTATACTTAATGTCAGAGACCCCAAAAGT
Sequence number 811: Chikungunya virus Whole genome #99 
AATCATCCAGAGACTAAAGAGAGGCTGTAGATTATACTTAATGTCAGAGACCCCAAAAGTCCCTACCT
ACCGGACCACATATCCGGCGCCTGTGTACTCGCCTCCGATTAACGTCCGACT
Sequence number 812: Chikungunya virus Whole genome #100 
CCCTACCTACCGGACCACATATCCGGCGCCTGTGTACTCGCCTCCGATTAACGTCCGACTGTCCAACC
CCGAGTCCGCAGTGGCAGCATGCAATGAGTTCTTGGCTAGAAACTATCCAAC
Sequence number 813: Chikungunya virus Whole genome #101 
GTCCAACCCCGAGTCCGCAGTGGCAGCATGCAATGAGTTCTTGGCTAGAAACTATCCAACTGTTTCAT
CATACCAAATCACCGACGAGTATGATGCATATCTAGACATGGTGGACGGGTC
Sequence number 814: Chikungunya virus Whole genome #102 
TGTTTCATCATACCAAATCACCGACGAGTATGATGCATATCTAGACATGGTGGACGGGTCGGAGAGTT
GTCTGGACCGAGCGACATTCAATCCGTCAAAACTTAGGAGCTACCCAAAACA
Sequence number 815: Chikungunya virus Whole genome #103 GGAGAGTTGTCTGGACCGAGCGACATTCAATCCGTCAAAACTTAGGAGCTACCCAAAACAGCACGCTT ACCACGCGCCCTCCATCAGAAGCGCTGTACCGTCCCCATTCCAGAACACACT
Sequence number 816: Chikungunya virus Whole genome #104 
GCACGCTTACCACGCGCCCTCCATCAGAAGCGCTGTACCGTCCCCATTCCAGAACACACTACAGAATG
TACTGGCAGCAGCCACGAAAAGAAACTGCAACGTCACACAGATGAGGGAATT
Sequence number 817: Chikungunya virus Whole genome #105 
ACAGAATGTACTGGCAGCAGCCACGAAAAGAAACTGCAACGTCACACAGATGAGGGAATTACCCACTT
TGGACTCAGCAGTATTCAACGTGGAGTGTTTCAAAAAATTCGCATGCAACCA
Sequence number 818: Chikungunya virus Whole genome #106 
ACCCACTTTGGACTCAGCAGTATTCAACGTGGAGTGTTTCAAAAAATTCGCATGCAACCAAGAATACT
GGGAAGAATTTGCTGCCAGCCCTATCAGGATAACAACTGAGAATTTAACAAC
Sequence number 819: Chikungunya virus Whole genome #107 
AGAATACTGGGAAGAATTTGCTGCCAGCCCTATCAGGATAACAACTGAGAATTTAACAACCTATGTTA
CTAAACTAAAGGGGCCAAAAGCAGCAGCGCTATTTGCAAGAACCCATAATCT
Sequence number 820: Chikungunya virus Whole genome #108 
CTATGTTACTAAACTAAAGGGGCCAAAAGCAGCAGCGCTATTTGCAAGAACCCATAATCTGCTGCCAC
TGCAGGAAGTGCCAATGGATAGGTTCACAGTAGACATGAAAAGGGATGTGAA
Sequence number 821: Chikungunya virus Whole genome #109 
GCTGCCACTGCAGGAAGTGCCAATGGATAGGTTCACAGTAGACATGAAAAGGGATGTGAAGGTGACTC
CTGGTACAAAGCACACAGAGGAAAGACCTAAGGTACAGGTTATACAGGCGGC
Sequence number 822: Chikungunya virus Whole genome #110 
GGTGACTCCTGGTACAAAGCACACAGAGGAAAGACCTAAGGTACAGGTTATACAGGCGGCTGAACCCT
TGGCAACAGCATACCTATGTGGGATTCACAGAGAGCTGGTTAGGAGGCTGAA
Sequence number 823: Chikungunya virus Whole genome #111 
TGAACCCTTGGCAACAGCATACCTATGTGGGATTCACAGAGAGCTGGTTAGGAGGCTGAACGCCGTCC
TCCTACCCAATGTACATACACTATTTGACATGTCTGCCGAGGATTTCGATGC
Sequence number 824: Chikungunya virus Whole genome #112 
CGCCGTCCTCCTACCCAATGTACATACACTATTTGACATGTCTGCCGAGGATTTCGATGCCATCATAG
CCGCACACTTTAAGCCAGGAGACACTGTTTTAGAAACGGACATAGCCTCCTT
Sequence number 825: Chikungunya virus Whole genome #113 
CATCATAGCCGCACACTTTAAGCCAGGAGACACTGTTTTAGAAACGGACATAGCCTCCTTTGATAAGA
GCCAAGATGATTCACTTGCGCTTACTGCTTTAATGCTGTTAGAGGATTTAGG
Sequence number 826: Chikungunya virus Whole genome #114 
TGATAAGAGCCAAGATGATTCACTTGCGCTTACTGCTTTAATGCTGTTAGAGGATTTAGGGGTGGATC
ACTCCCTGTTGGACTTGATAGAGGCTGCTTTCGGAGAGATTTCCAGCTGTCA
Sequence number 827: Chikungunya virus Whole genome #115 
GGTGGATCACTCCCTGTTGGACTTGATAGAGGCTGCTTTCGGAGAGATTTCCAGCTGTCATCTACCGA
CAGGTACGCGCTTCAAGTTCGGCGCCATGATGAAATCTGGTATGTTCCTAAC
Sequence number 828: Chikungunya virus Whole genome #116 
TCTACCGACAGGTACGCGCTTCAAGTTCGGCGCCATGATGAAATCTGGTATGTTCCTAACTCTGTTCG
TCAACACACTGCTAAATATCACCATCGCCAGCCGAGTGCTGGAAGATCGTCT
Sequence number 829: Chikungunya virus Whole genome #117 
TCTGTTCGTCAACACACTGCTAAATATCACCATCGCCAGCCGAGTGCTGGAAGATCGTCTGACAAAAT
CCGCGTGCGCAGCCTTCATCGGCGACGACAACATAATACATGGAGTCGTCTC
Sequence number 830: Chikungunya virus Whole genome #118 
GACAAAATCCGCGTGCGCAGCCTTCATCGGCGACGACAACATAATACATGGAGTCGTCTCCGATGAAT
TGATGGCAGCCAGATGCGCCACTTGGATGAACATGGAAGTGAAGATCATAGA
Sequence number 831: Chikungunya virus Whole genome #119 
CGATGAATTGATGGCAGCCAGATGCGCCACTTGGATGAACATGGAAGTGAAGATCATAGATGCAGTTG
TATCCCAGAAAGCCCCTTACTTTTGTGGAGGGTTTATACTGCACGATATCGT
Sequence number 832: Chikungunya virus Whole genome #120 
TGCAGTTGTATCCCAGAAAGCCCCTTACTTTTGTGGAGGGTTTATACTGCACGATATCGTGACAGGAA
CAGCTTGCAGAGTGGCAGACCCGCTAAAAAGGCTATTTAAACTGGGCAAACC
Sequence number 833: Chikungunya virus Whole genome #121 
GACAGGAACAGCTTGCAGAGTGGCAGACCCGCTAAAAAGGCTATTTAAACTGGGCAAACCGCTAGCGG
CAGGTGACGAACAAGATGAGGATAGAAGACGAGCGCTGGCTGACGAAGTGGT
Sequence number 834: Chikungunya virus Whole genome #122 
GCTAGCGGCAGGTGACGAACAAGATGAGGATAGAAGACGAGCGCTGGCTGACGAAGTGGTCAGATGGC
AACGAACAGGGCTAATTGATGAGTTGGAGAAAGCGGTATACTCTAGGTATGA
Sequence number 835: Chikungunya virus Whole genome #123 
CAGATGGCAACGAACAGGGCTAATTGATGAGTTGGAGAAAGCGGTATACTCTAGGTATGAAGTGCAGG
GTATATCAGTTGTGGTAATGTCCATGGCCACCTTTGCAAGCTCCAGATCCAA
Sequence number 836: Chikungunya virus Whole genome #124 
AGTGCAGGGTATATCAGTTGTGGTAATGTCCATGGCCACCTTTGCAAGCTCCAGATCCAACTTCGAGA
AGCTCAGAGGACCCGTCGTAACTTTGTACGGCGGTCCTAAATAGGTACGCAC
Sequence number 837: Chikungunya virus Whole genome #125 
CTTCGAGAAGCTCAGAGGACCCGTCGTAACTTTGTACGGCGGTCCTAAATAGGTACGCACTACAGCTA
CCTATTTTGCAGAAGCCGACAGTAAGTACCTAAACACTAATCAGCTACAATG
Sequence number 838: Chikungunya virus Whole genome #126 
TACAGCTACCTATTTTGCAGAAGCCGACAGTAAGTACCTAAACACTAATCAGCTACAATGGAGTTCAT
CCCAACCCAAACTTTTTACAACAGGAGGTACCAGCCTCGACCCTGGACTCCG
Sequence number 839: Chikungunya virus Whole genome #127 
GAGTTCATCCCAACCCAAACTTTTTACAACAGGAGGTACCAGCCTCGACCCTGGACTCCGCGCCCTAC
TATCCAAGTCATCAGGCCCAGACCGCGCCCGCAGAGGCAAGCTGGGCAACTT
Sequence number 840: Chikungunya virus Whole genome #128 
CGCCCTACTATCCAAGTCATCAGGCCCAGACCGCGCCCGCAGAGGCAAGCTGGGCAACTTGCCCAGCT
GATCTCAGCAGTTAATAAACTGACAATGCGCGCGGTACCCCAACAGAAGCCA
Sequence number 841: Chikungunya virus Whole genome #129 
GCCCAGCTGATCTCAGCAGTTAATAAACTGACAATGCGCGCGGTACCCCAACAGAAGCCACGCAGGAA
TCGGAAGAATAAGAAGCAAAAGCAAAAGCAACAGGCGCCACAAAACAACACA
Sequence number 842: Chikungunya virus Whole genome #130 
CGCAGGAATCGGAAGAATAAGAAGCAAAAGCAAAAGCAACAGGCGCCACAAAACAACACAAACCAAAA
GAAGCAGCCACCTAAAAAGAAACCAGCTCAAAAGAAAAAGAAGCCGGGCCGC
Sequence number 843: Chikungunya virus Whole genome #131 AACCAAAAGAAGCAGCCACCTAAAAAGAAACCAGCTCAAAAGAAAAAGAAGCCGGGCCGCAGAGAGAG GATGTGCATGAAAATCGAAAATGACTGTATTTTCGAAGTCAAGCACGAAGGT
Sequence number 844: Chikungunya virus Whole genome #132 
AGAGAGAGGATGTGCATGAAAATCGAAAATGACTGTATTTTCGAAGTCAAGCACGAAGGTAAGGTAAC
AGGTTACGCGTGCTTGGTGGGGGACAAAGTAATGAAACCAGCACACGTAAAG
Sequence number 845: Chikungunya virus Whole genome #133 
AAGGTAACAGGTTACGCGTGCTTGGTGGGGGACAAAGTAATGAAACCAGCACACGTAAAGGGGACCAT
CGATAACGCGGACCTGGCCAAATTGGCCTTTAAGCGGTCATCTAAGTACGAC
Sequence number 846: Chikungunya virus Whole genome #134 
GGGACCATCGATAACGCGGACCTGGCCAAATTGGCCTTTAAGCGGTCATCTAAGTACGACCTTGAATG
CGCGCAGATACCCGTGCACATGAAGTCCGACGCTTCGAAGTTCACCCATGAG
Sequence number 847: Chikungunya virus Whole genome #135 
CTTGAATGCGCGCAGATACCCGTGCACATGAAGTCCGACGCTTCGAAGTTCACCCATGAGAAACCGGA
GGGGTACTACAACTGGCACCACGGAGCAGTACAGTACTCAGGAGGCCGGTTC
Sequence number 848: Chikungunya virus Whole genome #136 
AAACCGGAGGGGTACTACAACTGGCACCACGGAGCAGTACAGTACTCAGGAGGCCGGTTCACCATCCC
TACAGGTGCGGGCAAACCAGGGGACAGCGGTAGACCGATCTTCGACAACAAG
Sequence number 849: Chikungunya virus Whole genome #137 
ACCATCCCTACAGGTGCGGGCAAACCAGGGGACAGCGGTAGACCGATCTTCGACAACAAGGGACGCGT
GGTGGCCATAGTCTTAGGAGGAGCTAATGAAGGAGCCCGTACAGCCCTCTCA
Sequence number 850: Chikungunya virus Whole genome #138 
GGACGCGTGGTGGCCATAGTCTTAGGAGGAGCTAATGAAGGAGCCCGTACAGCCCTCTCAGTGGTGAC
CTGGAATAAAGACATTGTCACTAAAATCACCCCTGAGGGAGCCGAAGAGTGG
Sequence number 851: Chikungunya virus Whole genome #139 
GTGGTGACCTGGAATAAAGACATTGTCACTAAAATCACCCCTGAGGGAGCCGAAGAGTGGAGTCTTGC
CATCCCAGTTATGTGCCTGTTGGCAAATACCACGTTCCCCTGCTCCCAGCCC
Sequence number 852: Chikungunya virus Whole genome #140 
AGTCTTGCCATCCCAGTTATGTGCCTGTTGGCAAATACCACGTTCCCCTGCTCCCAGCCCCCTTGCAT
ACCCTGCTGCTACGAAAAGGAACCGGAGGAAACCCTACGCATGCTTGAGGAC
Sequence number 853: Chikungunya virus Whole genome #141 
CCTTGCATACCCTGCTGCTACGAAAAGGAACCGGAGGAAACCCTACGCATGCTTGAGGACAACGTCAT
GAGACCTGGGTACTATCAGCTGCTACAAGCATCATTAACATGTTCTCCCCAC
Sequence number 854: Chikungunya virus Whole genome #142 
AACGTCATGAGACCTGGGTACTATCAGCTGCTACAAGCATCATTAACATGTTCTCCCCACCGCCAGCG
ACGCAGCACCAAGGACAACTTCAATGTCTATAAAGCCACAAGACCATACTTA
Sequence number 855: Chikungunya virus Whole genome #143 
CGCCAGCGACGCAGCACCAAGGACAACTTCAATGTCTATAAAGCCACAAGACCATACTTAGCTCACTG
TCCCGACTGTGGAGAAGGGCACTCGTGCCATAGTCCCGTAGCACTAGAACGC
Sequence number 856: Chikungunya virus Whole genome #144 
GCTCACTGTCCCGACTGTGGAGAAGGGCACTCGTGCCATAGTCCCGTAGCACTAGAACGCATCAGAAA
TGAAGCGACAGACGGGACGCTGAAAATCCAGGTCTCCTTGCAAATTGGAATA
Sequence number 857: Chikungunya virus Whole genome #145 
ATCAGAAATGAAGCGACAGACGGGACGCTGAAAATCCAGGTCTCCTTGCAAATTGGAATAGGGACGGA
TGATAGCCATGATTGGACCAAGCTGCGTTACATGGACAATCACATACCAGCA
Sequence number 858: Chikungunya virus Whole genome #146 
GGGACGGATGATAGCCATGATTGGACCAAGCTGCGTTACATGGACAATCACATACCAGCAGACGCAGG
GAGGGCCGGGCTATTTGTAAGAACATCAGCACCATGCACGATTACTGGAACA
Sequence number 859: Chikungunya virus Whole genome #147 
GACGCAGGGAGGGCCGGGCTATTTGTAAGAACATCAGCACCATGCACGATTACTGGAACAATGGGACA
CTTCATCCTGGCCCGATGTCCGAAAGGAGAAACTCTGACGGTGGGATTCACT
Sequence number 860: Chikungunya virus Whole genome #148 
ATGGGACACTTCATCCTGGCCCGATGTCCGAAAGGAGAAACTCTGACGGTGGGATTCACTGACAGTAG
GAAGATTAGTCACTCATGTACGCACCCATTTCACCACGACCCTCCTGTGATA
Sequence number 861: Chikungunya virus Whole genome #149 
GACAGTAGGAAGATTAGTCACTCATGTACGCACCCATTTCACCACGACCCTCCTGTGATAGGCCGGGA
AAAATTCCATTCCCGACCGCAGCACGGTAAAGAGCTACCTTGCAGCACGTAC
Sequence number 862: Chikungunya virus Whole genome #150 
GGCCGGGAAAAATTCCATTCCCGACCGCAGCACGGTAAAGAGCTACCTTGCAGCACGTACGTGCAGAG
CAACGCCGCAACTGCCGAGGAGATAGAGGTACACATGCCCCCAGACACCCCT
Sequence number 863: Chikungunya virus Whole genome #151 
GTGCAGAGCAACGCCGCAACTGCCGAGGAGATAGAGGTACACATGCCCCCAGACACCCCTGATCGCAC
ATTGCTGTCACAACAGTCCGGCAACGTAAAGATCACAGTCAACGGCCGGACG
Sequence number 864: Chikungunya virus Whole genome #152 
GATCGCACATTGCTGTCACAACAGTCCGGCAACGTAAAGATCACAGTCAACGGCCGGACGGTGCGGTA
TAAGTGTAATTGCGGTGGCTCAAATGAAGGACTAATAACTACAGATAAAGTG
Sequence number 865: Chikungunya virus Whole genome #153 
GTGCGGTATAAGTGTAATTGCGGTGGCTCAAATGAAGGACTAATAACTACAGATAAAGTGATTAATAA
CTGCAAGGTTGATCAATGTCATGCCGCGGTCACCAATCACAAAAAGTGGCAG
Sequence number 866: Chikungunya virus Whole genome #154 
ATTAATAACTGCAAGGTTGATCAATGTCATGCCGCGGTCACCAATCACAAAAAGTGGCAGTATAACTC
CCCTCTGGTCCCGCGTAACGCTGAACTCGGGGACCGAAAAGGAAAAATTCAC
Sequence number 867: Chikungunya virus Whole genome #155 
TATAACTCCCCTCTGGTCCCGCGTAACGCTGAACTCGGGGACCGAAAAGGAAAAATTCACATCCCGTT
TCCGCTGGCAAATGTAACATGCATGGTGCCTAAAGCAAGGAACCCCACCGTG
Sequence number 868: Chikungunya virus Whole genome #156 
ATCCCGTTTCCGCTGGCAAATGTAACATGCATGGTGCCTAAAGCAAGGAACCCCACCGTGACGTACGG
GAAAAACCAAGTCATCATGCTACTGTATCCTGACCACCCAACACTCCTGTCC
Sequence number 869: Chikungunya virus Whole genome #157 
ACGTACGGGAAAAACCAAGTCATCATGCTACTGTATCCTGACCACCCAACACTCCTGTCCTACCGGAG
TATGGGAGAAGAACCAAACTATCAAGAAGAGTGGGTGACGCACAAGAAGGAG
Sequence number 870: Chikungunya virus Whole genome #158 
TACCGGAGTATGGGAGAAGAACCAAACTATCAAGAAGAGTGGGTGACGCACAAGAAGGAGGTCGTGCT
AACCGTGCCGACTGAAGGGCTCGAGGTTACGTGGGGCAACAACGAGCCGTAT
Sequence number 871: Chikungunya virus Whole genome #159 GTCGTGCTAACCGTGCCGACTGAAGGGCTCGAGGTTACGTGGGGCAACAACGAGCCGTATAAGTATTG GCCGCAGTTATCTGCAAACGGTACAGCCCACGGCCACCCGCATGAGATAATC
Sequence number 872: Chikungunya virus Whole genome #160 
AAGTATTGGCCGCAGTTATCTGCAAACGGTACAGCCCACGGCCACCCGCATGAGATAATCTTGTACTA
TTATGAGCTGTACCCTACTATGACTGTAGTAGTTGTGTCAGTGGCCTCGTTC
Sequence number 873: Chikungunya virus Whole genome #161 
TTGTACTATTATGAGCTGTACCCTACTATGACTGTAGTAGTTGTGTCAGTGGCCTCGTTCATACTCCT
GTCGATGGTGGGTATGGCAGTGGGGATGTGCATGTGTGCACGACGCAGATGC
Sequence number 874: Chikungunya virus Whole genome #162 
ATACTCCTGTCGATGGTGGGTATGGCAGTGGGGATGTGCATGTGTGCACGACGCAGATGCATCACACC
ATACGAACTGACACCAGGAGCTACCGTCCCTTTCCTGCTTAGCCTAATATGC
Sequence number 875: Chikungunya virus Whole genome #163 
ATCACACCATACGAACTGACACCAGGAGCTACCGTCCCTTTCCTGCTTAGCCTAATATGCTGCATCAG
AACAGCTAAAGCGGCCACATACCAAGAGGCTGCGGTATACCTGTGGAACGAG
Sequence number 876: Chikungunya virus Whole genome #164 
TGCATCAGAACAGCTAAAGCGGCCACATACCAAGAGGCTGCGGTATACCTGTGGAACGAGCAGCAACC
TTTGTTTTGGCTACAAGCCCTTATTCCGCTGGCAGCCCTGATTGTCCTATGC
Sequence number 877: Chikungunya virus Whole genome #165 
CAGCAACCTTTGTTTTGGCTACAAGCCCTTATTCCGCTGGCAGCCCTGATTGTCCTATGCAACTGTCT
GAGACTCTTACCATGCTGTTGTAAAACGTTGGCTTTTTTAGCCGTAATGAGC
Sequence number 878: Chikungunya virus Whole genome #166 
AACTGTCTGAGACTCTTACCATGCTGTTGTAAAACGTTGGCTTTTTTAGCCGTAATGAGCATCGGTGC
CCACACTGTGAGCGCGTACGAACACGTAACAGTGATCCCGAACACGGTGGGA
Sequence number 879: Chikungunya virus Whole genome #167 
ATCGGTGCCCACACTGTGAGCGCGTACGAACACGTAACAGTGATCCCGAACACGGTGGGAGTACCGTA
TAAGACTCTAGTCAACAGACCGGGCTACAGCCCCATGGTACTGGAGATGGAG
Sequence number 880: Chikungunya virus Whole genome #168 
GTACCGTATAAGACTCTAGTCAACAGACCGGGCTACAGCCCCATGGTACTGGAGATGGAGCTACTGTC
AGTCACTTTGGAGCCAACGCTATCGCTTGATTACATCACGTGCGAATACAAA
Sequence number 881: Chikungunya virus Whole genome #169 
CTACTGTCAGTCACTTTGGAGCCAACGCTATCGCTTGATTACATCACGTGCGAATACAAAACCGTCAT
CCCGTCTCCGTACGTGAAATGCTGCGGTACAGCAGAGTGCAAGGACAAAAC
Sequence number 882: Chikungunya virus Whole genome #170 
ACCGTCATCCCGTCTCCGTACGTGAAATGCTGCGGTACAGCAGAGTGCAAGGACAAAAACCTACCTGA
CTACAGCTGTAAGGTCTTCACCGGCGTCTACCCATTTATGTGGGGCGGCGCC
Sequence number 883: Chikungunya virus Whole genome #171 
CTACCTGACTACAGCTGTAAGGTCTTCACCGGCGTCTACCCATTTATGTGGGGCGGCGCCTACTGCTT
CTGCGACGCTGAAAACACGCAATTGAGCGAAGCACATGTGGAGAAGTCCGAA
Sequence number 884: Chikungunya virus Whole genome #172 
TACTGCTTCTGCGACGCTGAAAACACGCAATTGAGCGAAGCACATGTGGAGAAGTCCGAATCATGCAA
AACAGAATTTGCATCAGCATACAGGGCTCATACCGCATCCGCATCAGCTAAG
Sequence number 885: Chikungunya virus Whole genome #173 
TCATGCAAAACAGAATTTGCATCAGCATACAGGGCTCATACCGCATCCGCATCAGCTAAGCTCCGCGT
CCTTTACCAAGGAAATAACATCACTGTAACTGCCTATGCAAACGGCGACCAT
Sequence number 886: Chikungunya virus Whole genome #174 
CTCCGCGTCCTTTACCAAGGAAATAACATCACTGTAACTGCCTATGCAAACGGCGACCATGCCGTCAC
AGTTAAGGACGCCAAATTCATTGTGGGGCCAATGTCTTCAGCCTGGACACCT
Sequence number 887: Chikungunya virus Whole genome #175 
GCCGTCACAGTTAAGGACGCCAAATTCATTGTGGGGCCAATGTCTTCAGCCTGGACACCTTTCGACAA
CAAAATCGTGGTGTACAAAGGTGACGTTTACAACATGGACTACCCGCCCTTT
Sequence number 888: Chikungunya virus Whole genome #176 
TTCGACAACAAAATCGTGGTGTACAAAGGTGACGTTTACAACATGGACTACCCGCCCTTTGGCGCAGG
AAGACCAGGACAATTTGGCGATATCCAAAGTCGCACGCCTGAGAGCAAAGAC
Sequence number 889: Chikungunya virus Whole genome #177 
GGCGCAGGAAGACCAGGACAATTTGGCGATATCCAAAGTCGCACGCCTGAGAGCAAAGACGTCTATGC
TAACACACAACTGGTACTGCAGAGACCGGCTGCGGGTACGGTACACGTGCCA
Sequence number 890: Chikungunya virus Whole genome #178 
GTCTATGCTAACACACAACTGGTACTGCAGAGACCGGCTGCGGGTACGGTACACGTGCCATACTCTCA
GGCACCATCTGGCTTTAAGTATTGGTTAAAAGAACGAGGGGCGTCGCTACAG
Sequence number 891: Chikungunya virus Whole genome #179 
TACTCTCAGGCACCATCTGGCTTTAAGTATTGGTTAAAAGAACGAGGGGCGTCGCTACAGCACACAGC
ACCATTTGGCTGCCAAATAGCAACAAACCCGGTAAGAGCGATGAACTGCGCC
Sequence number 892: Chikungunya virus Whole genome #180 
CACACAGCACCATTTGGCTGCCAAATAGCAACAAACCCGGTAAGAGCGATGAACTGCGCCGTAGGGAA
CATGCCCATCTCCATCGACATACCGGATGCGGCCTTCACTAGGGTCGTCGAC
Sequence number 893: Chikungunya virus Whole genome #181 
GTAGGGAACATGCCCATCTCCATCGACATACCGGATGCGGCCTTCACTAGGGTCGTCGACGCGCCCTC
TTTAACGGACATGTCATGCGAGGTACCAGCCTGCACCCATTCCTCAGACTTT
Sequence number 894: Chikungunya virus Whole genome #182 
GCGCCCTCTTTAACGGACATGTCATGCGAGGTACCAGCCTGCACCCATTCCTCAGACTTTGGGGGCGT
CGCCATTATTAAATATGCAGTCAGCAAGAAAGGCAAGTGTGCGGTGCATTCG
Sequence number 895: Chikungunya virus Whole genome #183 
GGGGGCGTCGCCATTATTAAATATGCAGTCAGCAAGAAAGGCAAGTGTGCGGTGCATTCGATGACCAA
CGCCGTCACTATCCGGGAAGCTGAGATAGAAGTTGAAGGGAATTCTCAGCTG
Sequence number 896: Chikungunya virus Whole genome #184 
ATGACCAACGCCGTCACTATCCGGGAAGCTGAGATAGAAGTTGAAGGGAATTCTCAGCTGCAAATCTC
TTTCTCGACGGCCTTGGCCAGCGCCGAATTCCGCGTACAAGTCTGTTCTACA
Sequence number 897: Chikungunya virus Whole genome #185 
CAAATCTCTTTCTCGACGGCCTTGGCCAGCGCCGAATTCCGCGTACAAGTCTGTTCTACACAAGTACA
CTGTGCAGCCGAGTGCCACCCTCCGAAGGACCACATAGTCAACTACCCGGCG
Sequence number 898: Chikungunya virus Whole genome #186 
CAAGTACACTGTGCAGCCGAGTGCCACCCTCCGAAGGACCACATAGTCAACTACCCGGCGTCACATAC
CACCCTCGGGGTCCAGGACATTTCCGCTACGGCGATGTCATGGGTGCAGAAG
Sequence number 899: Chikungunya virus Whole genome #187 TCACATACCACCCTCGGGTCCAGGACATTTCCGCTACGGCGATGTCATGGTGCAGAAGATCACGGG AGGTGTGGGACTGGTTGTCGCTGTTGCAGCACTGATTCTAATCGTGGTGCTA
Sequence number 900: Chikungunya virus Whole genome #188 
ATCACGGGAGGTGTGGGACTGGTTGTCGCTGTTGCAGCACTGATTCTAATCGTGGTGCTATGCGTGTC
GTTCAGCAGGCACTAACTTGACGACTAAGCATGAAGGTATATGTGTCCCCTA
Sequence number 901: Chikungunya virus Whole genome #189 
TGCGTGTCGTTCAGCAGGCACTAACTTGACGACTAAGCATGAAGGTATATGTGTCCCCTAAGAGACAC
ACCGTATATAGCTAATAATCTGTAGATCAAAGGGCTATATAACCCCTGAATA
Sequence number 902: Chikungunya virus Whole genome #190 
AGAGACACACCGTATATAGCTAATAATCTGTAGATCAAAGGGCTATATAACCCCTGAATAGTAACAAA
ATACAAAATCACTAAAAATTATAAAAAACAGAAAAATATATAAATAGGTATA
Sequence number 903: Chikungunya virus Whole genome #191 
GTAACAAAATACAAAATCACTAAAAATTATAAAAAACAGAAAAATATATAAATAGGTATACGTGTCCC
CTAAGAGACACATTGTATGTAGGTGATAAGTATAGATCAAAGGGCCGAACAA
Sequence number 904: Chikungunya virus Whole genome #192 
CGTGTCCCCTAAGAGACACATTGTATGTAGGTGATAAGTATAGATCAAAGGGCCGAACAACCCCTGAA
TAGTAACAAAATATAAAAATTAATAAAAATCATAAAATAGAAAAACCATAAA
Sequence number 905: Chikungunya virus Whole genome #193 
CCCCTGAATAGTAACAAAATATAAAAATTAATAAAAATCATAAAATAGAAAAACCATAAACAGAAGTA
GTTCAAAGGGCTATAAAAACCCCTGAATAGTAACAAAACATAAAACTAATAA
Sequence number 906: Chikungunya virus Whole genome #194 
CAGAAGTAGTTCAAAGGGCTATAAAAACCCCTGAATAGTAACAAAACATAAAACTAATAAAAATCAAA
TGAATACCATAATTGGCAAACGGAAGAGATGTAGGTACTTAAGCTTCCTAAA
Sequence number 907: Chikungunya virus Whole genome #195 
AAATCAAATGAATACCATAATTGGCAAACGGAAGAGATGTAGGTACTTAAGCTTCCTAAAAGCAGCCG
AACTCACTTTGAGATGTAGGCATAGCATACCGAACTCTTCCACGATTCTCCG
Sequence number 908: Chikungunya virus Whole genome #196 
TGAATACCATAATTGGCAAACGGAAGAGATGTAGGTACTTAAGCTTCCTAAAAGCAGCCGAACTCACT
TTGAGATGTAGGCATAGCATACCGAACTCTTCCACGATTCTCCGAACCCACA
Sequence number 909: Coxiella burnetii IS1111 #1 
TTAGCGAATAAAAATGCGCGGATCATTTGGGCGCTTTTAACACGCCAAGAAACGTATCGCTGTGGCGC
CTAAACACCGCCGTGGGTAAAAAAAAGAATTAACAAAAGGAGACACACCAAC
Sequence number 910: Coxiella burnetii IS1111 #2 
TGTGGCGCCTAAACACCGCCGTGGGTAAAAAAAAGAATTAACAAAAGGAGACACACCAACCGAGTTCG
AAACAATGAGGGCTGATGAAAGTAAGGTAAAACCTGAGGTTGATTAAGCTGA
Sequence number 911: Coxiella burnetii IS1111 #3 
CGAGTTCGAAACAATGAGGGCTGATGAAAGTAAGGTAAAACCTGAGGTTGATTAAGCTGATTCATACG
GTGGCTCTGTGAAGTCGATAGCCCGATAAGCATCAACCTTTCATAATTCATC
Sequence number 912: Coxiella burnetii IS1111 #4 
TTCATACGGTGGCTCTGTGAAGTCGATAGCCCGATAAGCATCAACCTTTCATAATTCATCAAGGCACC
AATGGTGGCCAATTTAAATTGTGATGCCGGATATACGAATGCAACCGCTTTC
Sequence number 913: Coxiella burnetii IS1111 #5 
AAGGCACCAATGGTGGCCAATTTAAATTGTGATGCCGGATATACGAATGCAACCGCTTTCTTTTCATC
AAAACTGATTGTTGACAACAAGGGTGGGTCTATATACGTATGAAGCGAAATA
Sequence number 914: Coxiella burnetii IS1111 #6 
TTTTCATCAAAACTGATTGTTGACAACAAGGGTGGGTCTATATACGTATGAAGCGAAATACGGGAAAA
GGCATGGCGCATATCATTACCCACAAGTCACAGCCATTTCGTCATTCCCGCG
Sequence number 915: Coxiella burnetii IS1111 #7 
CGGGAAAAGGCATGGCGCATATCATTACCCACAAGTCACAGCCATTTCGTCATTCCCGCGCAGGCGGG
AACTAGGTGCACCTTCGGTGCGCTGCGCACAGGTGCGCTGGATCCCCGCCTG
Sequence number 916: Coxiella burnetii IS1111 #8 
CAGGCGGGAACTAGGTGCACCTTCGGTGCGCTGCGCACAGGTGCGCTGGATCCCCGCCTGCGCGGGGA
TGACGAGGGGGGCGGGATGGCGAGGGGTGGGGAATGACGGGTTTTAAGTTTT
Sequence number 917: Coxiella burnetii IS1111 #9 
CGCGGGGATGACGAGGGGGGCGGGATGGCGAGGGGTGGGGAATGACGGGTTTTAAGTTTTTGTCTTTT
ACCACTGGGGGAGTGATATGGGTATGATGTCGTTTCCCTTACAAAACCGGTG
Sequence number 918: Coxiella burnetii IS1111 #10 
TGTCTTTTACCACTGGGGGAGTGATATGGGTATGATGTCGTTTCCCTTACAAAACCGGTGGGGCTTGA
AGGGGAAAGAAATTTAGGATATTAATGGAAGTCTGCCAATAATGCCTGGAGG
Sequence number 919: Coxiella burnetii IS1111 #11 
GGGCTTGAAGGGGAAAGAAATTTAGGATATTAATGGAAGTCTGCCAATAATGCCTGGAGGTTGCATTA
TGTACAGGTATGGAAACCTCTTCCAAACACCGATCCTTCGCTCACCACTCGA
Sequence number 920: Coxiella burnetii IS1111 #12 
TTGCATTATGTACAGGTATGGAAACCTCTTCCAAACACCGATCCTTCGCTCACCACTCGAAGATCAAT
TCTCTGACTATGTATTATGGCGGTTGGATTGTGAGCCATTCGTAGAGGTCTT
Sequence number 921: Coxiella burnetii IS1111 #13 
AGATCAATTCTCTGACTATGTATTATGGCGGTTGGATTGTGAGCCATTCGTAGAGGTCTTAGAAAGAA
CCTGCCGTATGAATGATGAAAAGCTGCGAACCCAAAACGTGGAAGCGACCCT
Sequence number 922: Coxiella burnetii IS1111 #14 
AGAAAGAACCTGCCGTATGAATGATGAAAAGCTGCGAACCCAAAACGTGGAAGCGACCCTATTTGATT
CGGCCAAGAAACCGGAAATTTCGTTTGCGGATTATATTTGGCGAATAGTCGC
Sequence number 923: Coxiella burnetii IS1111 #15 
ATTTGATTCGGCCAAGAAACCGGAAATTTCGTTTGCGGATTATATTTGGCGAATAGTCGCCTATGCGA
GACTGACCCATTCCCAAATGATCCATACGTTAATTTACCTCGATAGGTGTCA
Sequence number 924: Coxiella burnetii IS1111 #16 
CTATGCGAGACTGACCCATTCCCAAATGATCCATACGTTAATTTACCTCGATAGGTGTCAAGAAAATT
TTTTTTTAACTTCTTTAAATTTTTATCGCTTATTCCTAGTCGCTGCTTTAGT
Sequence number 925: Coxiella burnetii IS1111 #17 
AGAAAATTTTTTTTTAACTTCTTTAAATTTTTATCGCTTATTCCTAGTCGCTGCTTTAGTGGCACAAA
AATTTCATCAAGATGATTCTTTTTCCAACAAAAGTTTTGCTGATTTAGTAGG
Sequence number 926: Coxiella burnetii IS1111 #18 
GGCACAAAAATTTCATCAAGATGATTCTTTTTCCAACAAAAGTTTTGCTGATTTAGTAGGAATTACGG
TCAAAGAGTTAAATATACTTGAAGCAAAATTCTTATTTGCTATTAGTTTTAG
Sequence number 927: Coxiella burnetii IS1111 #19 AATTACGGTCAAAGAGTTAAATATACTTGAAGCAAAATTCTTATTTGCTATTAGTTTTAGTCTTTATG TTTTACCAAAAACCTATAAAGAATATAATCGAATTGTGGGTGGACAAAGAAT
Sequence number 928: Coxiella burnetii IS1111 #20 
TCTTTATGTTTTACCAAAAACCTATAAAGAATATAATCGAATTGTGGGTGGACAAAGAATTAACCGCC
AATTAGTGGAGGCAAGGCGATCGAATCTATCGTTTTTTTCTCTTTTCCCTCA
Sequence number 929: Coxiella burnetii IS1111 #21 
TAACCGCCAATTAGTGGAGGCAAGGCGATCGAATCTATCGTTTTTTTCTCTTTTCCCTCATACAGTTA
ATACCTTTCCTCACAGGAAGAGAAATTCTTATCACTTTTTACATCGCCCTCG
Sequence number 930: Coxiella burnetii IS1111 #22 
TACAGTTAATACCTTTCCTCACAGGAAGAGAAATTCTTATCACTTTTTACATCGCCCTCGATTCTAAT
ATTGCTTTTTACAAAAAGTCTCTTTAATACCGAATTTTCCGATAAATAAGGC
Sequence number 931: Coxiella burnetii IS1111 #23 
ATTCTAATATTGCTTTTTACAAAAAGTCTCTTTAATACCGAATTTTCCGATAAATAAGGCTAATAAAA
ATAGCAAGGGAATCATCGTAAGCGCAACTTGATAAGCATAGGTACTATAGAT
Sequence number 932: Coxiella burnetii IS1111 #24 
TAATAAAAATAGCAAGGGAATCATCGTAAGCGCAACTTGATAAGCATAGGTACTATAGATAGGAGCAT
TTCCCAACATTCGACCGTCCCAGAATTTATCCAACAATAAACCTACAATCGG
Sequence number 933: Coxiella burnetii IS1111 #25 
AGGAGCATTTCCCAACATTCGACCGTCCCAGAATTTATCCAACAATAAACCTACAATCGGTTGTAAAA
TAACACCGGCGAAAATAATACCCATATTGGTTAAGCCTGACGCAGTCCCCAC
Sequence number 934: Coxiella burnetii IS1111 #26 
TTGTAAAATAACACCGGCGAAAATAATACCCATATTGGTTAAGCCTGACGCAGTCCCCACGGCTTGAG
CATGATTATTATCGATGACCAGGCCGAAAGAGATGACCATGGCAGAACAGGC
Sequence number 935: Coxiella burnetii IS1111 #27 
GGCTTGAGCATGATTATTATCGATGACCAGGCCGAAAGAGATGACCATGGCAGAACAGGCCGCGCCGA
AGAGGAAGAGACAAACGGCTAACAGCATTTGAGGCAAAGCGGGAACATACAA
Sequence number 936: Coxiella burnetii IS1111 #28 
CGCGCCGAAGAGGAAGAGACAAACGGCTAACAGCATTTGAGGCAAAGCGGGAACATACAAAATTAAAG
AGGCGACAACAAATCCTAAAAGAGCAGCCGTAACCATGGGTAGCCGGCGTCG
Sequence number 937: Coxiella burnetii IS1111 #29 
AATTAAAGAGGCGACAACAAATCCTAAAAGAGCAGCCGTAACCATGGGTAGCCGGCGTCGTTGAATAG
CATTCGTCCACCAACCGAAAAAAGGCCCGCCGACGGCGATGCCAAGCCAGAT
Sequence number 938: Coxiella burnetii IS1111 #30 
TTGAATAGCATTCGTCCACCAACCGAAAAAAGGCCCGCCGACGGCGATGCCAAGCCAGATTAGAGAGA
GCAAAGCCGTGGTGTGAACATTCGTAAATTGATGGGAACTTACGAGAAATAA
Sequence number 939: Coxiella burnetii IS1111 #31 
TAGAGAGAGCAAAGCCGTGGTGTGAACATTCGTAAATTGATGGGAACTTACGAGAAATAAAGCGCCCC
ATGAAGTAGCGAACAATGCCGTCGGCCCCCAAATGGCAAAACCGAACAATGC
Sequence number 940: Coxiella burnetii IS1111 #32 
AGCGCCCCATGAAGTAGCGAACAATGCCGTCGGCCCCCAAATGGCAAAACCGAACAATGCAATCCACC
ATGATTGCGTCCGTTTAAAGACATCGCGTAAATAAAGACGGTTTTCTTGTGC
Sequence number 941: Coxiella burnetii IS1111 #33 
AATCCACCATGATTGCGTCCGTTTAAAGACATCGCGTAAATAAAGACGGTTTTCTTGTGCCAGCAATT
TAGCGGCGGCTTTGGTTAGGTGGGATTCCTTAGGTTTATCCCGAATAACTAT
Sequence number 942: Coxiella burnetii IS1111 #34 
CAGCAATTTAGCGGCGGCTTTGGTTAGGTGGGATTCCTTAGGTTTATCCCGAATAACTATCCAGTTAA
GGATGGTAAGTAAAAATCCCAATGCCGCAATAAAGTAAGTCGTTCGTTGCCA
Sequence number 943: Coxiella burnetii IS1111 #35 
CCAGTTAAGGATGGTAAGTAAAAATCCCAATGCCGCAATAAAGTAAGTCGTTCGTTGCCAACCATACA
GGTTTGCAAAATGCGCCACGGGCGCTGAGCCGATAACAGCGCCAGCGCACCC
Sequence number 944: Coxiella burnetii IS1111 #36 
ACCATACAGGTTTGCAAAATGCGCCACGGGCGCTGAGCCGATAACAGCGCCAGCGCACCCAAGCACTT
GCACCAGTCCCGCATACATGGCAAACCGCTTAGCACCCAACCAATAAGCGCC
Sequence number 945: Coxiella burnetii IS1111 #37 
AAGCACTTGCACCAGTCCCGCATACATGGCAAACCGCTTAGCACCCAACCAATAAGCGCCGACCCATA
AAGCGCCGAGATAAGCGATGGAAGCGGTAAATCCCATAATAAAACGAGCAAT
Sequence number 946: Coxiella burnetii IS1111 #38 
GACCCATAAAGCGCCGAGATAAGCGATGGAAGCGGTAAATCCCATAATAAAACGAGCAATGACAGCGA
GAGGAAAATAAGTAGTGGACGCAAAGATAAGCGTTGCTATCGAGCAAACTAG
Sequence number 947: Coxiella burnetii IS1111 #39 
GACAGCGAGAGGAAAATAAGTAGTGGACGCAAAGATAAGCGTTGCTATCGAGCAAACTAGAAGCCCTA
AAGAAAGCAAACGACGCGCGCCGAATTTATCGCACAACATGCCCGATGGAAT
Sequence number 948: Coxiella burnetii IS1111 #40 
AAGCCCTAAAGAAAGCAAACGACGCGCGCCGAATTTATCGCACAACATGCCCGATGGAATTTGCATCA
AGGCGTAGCCGTAAAAAAAACCGCTAAATAAAATACTGAGTTCACCCGCTTG
Sequence number 949: Coxiella burnetii IS1111 #41 
TTGCATCAAGGCGTAGCCGTAAAAAAAACCGCTAAATAAAATACTGAGTTCACCCGCTTGAATTTTGA
ACGCGTCTAATAACTCATGCGCAATAGCCATCGGCATGACGCGGACTAAAAA
Sequence number 950: Coxiella burnetii IS1111 #42 
AATTTTGAACGCGTCTAATAACTCATGCGCAATAGCCATCGGCATGACGCGGACTAAAAAATCGTACG
CGTAAAAGGAAGAAGCGACGATGCCTACAATTAATGGTGTCAGCCAAAGATG
Sequence number 951: Coxiella burnetii IS1111 #43 
ATCGTACGCGTAAAAGGAAGAAGCGACGATGCCTACAATTAATGGTGTCAGCCAAAGATGAAGTTTGG
TATTTTTCTTACTGATCAGCATAATTATCCTCTGTGCGAAATTTATGGCCCT
Sequence number 952: Coxiella burnetii IS1111 #44 
AAGTTTGGTATTTTTCTTACTGATCAGCATAATTATCCTCTGTGCGAAATTTATGGCCCTTTGTACTC
CCTCTCCAATGATGCCATAATAAATCAGATTATGAGGGGAAGGAGTCCCTAA
Sequence number 953: Coxiella burnetii IS1111 #45 
TTGTACTCCCTCTCCAATGATGCCATAATAAATCAGATTATGAGGGGAAGGAGTCCCTAATGTATTGT
CGTAAGCGAATATTCATCCGCTGTTTTTACATTTTAATGCTTACCTTTGGTC
Sequence number 954: Coxiella burnetii IS1111 #46 
TGTATTGTCGTAAGCGAATATTCATCCGCTGTTTTTACATTTTAATGCTTACCTTTGGTCTGCTTGGA
ATAGCATCCCACATTATTGCTCAAAATAATACCCGTAGATTAGCCATTTTAA
Sequence number 955: Coxiella burnetii IS1111 #47 TGCTTGGAATAGCATCCCACATTATTGCTCAAAATAATACCCGTAGATTAGCCATTTTAATCGACATA AAAGGCGGTATTGGTCCAGCCACCGCCGATTTTTTTCATCGCAGTCTAAAGC
Sequence number 956: Coxiella burnetii IS1111 #48 
TCGACATAAAAGGCGGTATTGGTCCAGCCACCGCCGATTTTTTTCATCGCAGTCTAAAGCAAGCCACT
GAAAAAAAAGCACAAGTTATTATTTTACAACTTGACACGCCTGGCGGTTTAG
Sequence number 957: Coxiella burnetii IS1111 #49 
AAGCCACTGAAAAAAAAGCACAAGTTATTATTTTACAACTTGACACGCCTGGCGGTTTAGTGACTGCC
ATGCGCGATATTATTAAAGATATCCTCGCCTCGCCCATCCCAGTCATCGGCT
Sequence number 958: Coxiella burnetii IS1111 #50 
TGACTGCCATGCGCGATATTATTAAAGATATCCTCGCCTCGCCCATCCCAGTCATCGGCTACGTAGCG
CCGAGCGGTGCCCATGCGGCGAGTGCGGGCACTTATATTTTATACGCCACTC
Sequence number 959: Coxiella burnetii IS1111 #51 
ACGTAGCGCCGAGCGGTGCCCATGCGGCGAGTGCGGGCACTTATATTTTATACGCCACTCATATCGCC
GCCATGGCCCCTGGAACCAATTTAGGGGCTGCGACGCCGATCGCTATTGGCA
Sequence number 960: Coxiella burnetii IS1111 #52 
ATATCGCCGCCATGGCCCCTGGAACCAATTTAGGGGCTGCGACGCCGATCGCTATTGGCACACCCGGC
GGCAGTTCTCCTGAAAAGGGTAACGAAAAAAAAGAAGGAGCTAAAAAAGCAC
Sequence number 961: Coxiella burnetii IS1111 #53 
CACCCGGCGGCAGTTCTCCTGAAAAGGGTAACGAAAAAAAAGAAGGAGCTAAAAAAGCACCGGCGCAA
AAAACCGCGGCTGAAAAAAAAGCCATTAGCGATGCGAGAGCCTATATTCGCA
Sequence number 962: Coxiella burnetii IS1111 #54 
CGGCGCAAAAAACCGCGGCTGAAAAAAAAGCCATTAGCGATGCGAGAGCCTATATTCGCAGTTTAGCG
CAACTGCGCGGTCGAAACGTGGAATGGGCAGAAAGTGCGGTCGTTGAAGCAG
Sequence number 963: Coxiella burnetii IS1111 #55 
GTTTAGCGCAACTGCGCGGTCGAAACGTGGAATGGGCAGAAAGTGCGGTCGTTGAAGCAGAAAGCCTT
TCCGCCGAAGAAGCATATAAAATTAAAGTAATTAATATCATTGCGTCGAATA
Sequence number 964: Coxiella burnetii IS1111 #56 
AAAGCCTTTCCGCCGAAGAAGCATATAAAATTAAAGTAATTAATATCATTGCGTCGAATATCCCTAAT
TTACTAGAACAAGCGAATGGCATAAAAATTAGCGTGCGCGGCGAAACGCAAA
Sequence number 965: Coxiella burnetii IS1111 #57 
TCCCTAATTTACTAGAACAAGCGAATGGCATAAAAATTAGCGTGCGCGGCGAAACGCAAACCGTAAAC
ACCGCCAATCTGGAAATAGAAAAAGTAGAACCCGATTGGCGAACGCGCTTTT
Sequence number 966: Coxiella burnetii IS1111 #58 
CCGTAAACACCGCCAATCTGGAAATAGAAAAAGTAGAACCCGATTGGCGAACGCGCTTTTTAAGCGTG
ATTACGGACTCCAGCGTTGCCTATATTTTATTGATGATCGGTTTTTATGGGA
Sequence number 967: Coxiella burnetii IS1111 #59 
TAAGCGTGATTACGGACTCCAGCGTTGCCTATATTTTATTGATGATCGGTTTTTATGGGATATTTTTT
GAATTTATTAATCCCGGTTTTATTGTTCCGGGGGTGGTGGGCGCTATTTCCT
Sequence number 968: Coxiella burnetii IS1111 #60 
TATTTTTTGAATTTATTAATCCCGGTTTTATTGTTCCGGGGGTGGTGGGCGCTATTTCCTTATTGTTA
GCGCTTTACGCGTTTCAATTGCTGCCAATCAATTACGCGGGTTTAGCGTTGA
Sequence number 969: Coxiella burnetii IS1111 #61 
TATTGTTAGCGCTTTACGCGTTTCAATTGCTGCCAATCAATTACGCGGGTTTAGCGTTGATTTTATTG
GGATTGGCTTTCATTGTTGCGGAAGCTTTCATGCCGAGTTTTGGCATGTTGG
Sequence number 970: Coxiella burnetii IS1111 #62 
TTTTATTGGGATTGGCTTTCATTGTTGCGGAAGCTTTCATGCCGAGTTTTGGCATGTTGGGTATTGGC
GGCGGTGTTGCATTTGTTATCGGTTCTATTATGCTAATGAGAACAGATGCAA
Sequence number 971: Coxiella burnetii IS1111 #63 
GTATTGGCGGCGGTGTTGCATTTGTTATCGGTTCTATTATGCTAATGAGAACAGATGCAATTGGCTTT
GGAATTCCCATGTATTTAATCATCGCTATCGCTATTGTCACCATAGCCTTCT
Sequence number 972: Coxiella burnetii IS1111 #64 
TTGGCTTTGGAATTCCCATGTATTTAATCATCGCTATCGCTATTGTCACCATAGCCTTCTTTTTACTC
GTTTTAAGTATCGCGTTTCGTTCGCGGGGGCGCCCGATTGTGAGTGGTCGAG
Sequence number 973: Coxiella burnetii IS1111 #65 
TTTTACTCGTTTTAAGTATCGCGTTTCGTTCGCGGGGGCGCCCGATTGTGAGTGGTCGAGAAGCGGTG
ATTGGAAAGCGCGGCGTTGTCAATGTCAGCGATAAACGCATGTGGGTGCGTA
Sequence number 974: Coxiella burnetii IS1111 #66 
AAGCGGTGATTGGAAAGCGCGGCGTTGTCAATGTCAGCGATAAACGCATGTGGGTGCGTATCGAAGGC
GAGCGTTGGCAATGTAAAAGTGATGTTCCCCTTCAAGATGGCGATGAAGTCG
Sequence number 975: Coxiella burnetii IS1111 #67 
TCGAAGGCGAGCGTTGGCAATGTAAAAGTGATGTTCCCCTTCAAGATGGCGATGAAGTCGTCGTTGAA
CGACTCGAAGGATTATTTTGTGTGGTCAAACCAGTGGAGAAAAAATAATGAT
Sequence number 976: Coxiella burnetii IS1111 #68 
TCGTTGAACGACTCGAAGGATTATTTTGTGTGGTCAAACCAGTGGAGAAAAAATAATGATGATTGTTC
TGATTGTAATAGCGGTATTGGTTTTACTGTTATTGTTTTCCGCTATCCATAT
Sequence number 977: Coxiella burnetii IS1111 #69 
GATTGTTCTGATTGTAATAGCGGTATTGGTTTTACTGTTATTGTTTTCCGCTATCCATATTCTGAAAG
AGTACGAGCGCGGCGTTATTTTCACTTTAGGTCGTTTTTGGAAAGTGAAGGG
Sequence number 978: Coxiella burnetii IS1111 #70 
TCTGAAAGAGTACGAGCGCGGCGTTATTTTCACTTTAGGTCGTTTTTGGAAAGTGAAGGGTCCAGGCT
TAATCATCGTCGTGCCGATTATTCAACAAATTGTTCGCACGCATTTGCGAAC
Sequence number 979: Coxiella burnetii IS1111 #71 
TCCAGGCTTAATCATCGTCGTGCCGATTATTCAACAAATTGTTCGCACGCATTTGCGAACAGTTGTGA
TGGATGTGCCTAGCCAGGATGTTATTTCCCGCGATAATGTTTCCGTGCGAGT
Sequence number 980: Coxiella burnetii IS1111 #72 
AGTTGTGATGGATGTGCCTAGCCAGGATGTTATTTCCCGCGATAATGTTTCCGTGCGAGTGAACGCGG
TTGTTTATTTTCGGGTGATTGATCCCGAGCGCGCGATTATTCAGGTTGAGGA
Sequence number 981: Coxiella burnetii IS1111 #73 
GAACGCGGTTGTTTATTTTCGGGTGATTGATCCCGAGCGCGCGATTATTCAGGTTGAGGATTATTACG
AAGCGACGAGTCAATTGGCGCAAACCACACTGCGTTCCGTATTGGGCCAACA
 
Sequence number 982: Coxiella burnetii IS1111 #74
TTATTACGAAGCGACGAGTCAATTGGCGCAAACCACACTGCGTTCCGTATTGGGCCAACACGAACTCG
ATGAAATGCTGGCCGAACGAGAGAAGTTAAACAAAGATATTCAAGAAATTTT
Sequence number 983: Coxiella burnetii IS1111 #75 CGAACTCGATGAAATGCTGGCCGAACGAGAGAAGTTAAACAAAGATATTCAAGAAATTTTAGATGCGG AAACAGACGCGTGGGGTATTAAAGTCGCCAACGTCGAAATTAAACATGTCGA
Sequence number 984: Coxiella burnetii IS1111 #76 
AGATGCGGAAACAGACGCGTGGGGTATTAAAGTCGCCAACGTCGAAATTAAACATGTCGATTTGGAAG
AAAGCATGGTTCGCGCCATCGCCCGCCAAGCCGAGGCTGAACGGGAACGACG
Sequence number 985: Coxiella burnetii IS1111 #77 
TTTGGAAGAAAGCATGGTTCGCGCCATCGCCCGCCAAGCCGAGGCTGAACGGGAACGACGGGCAAAAG
TGATTAACGCAGAAGGCGAATTTCAAGCCGCCCAACGCTTAAAAGAAGCCGC
Sequence number 986: Coxiella burnetii IS1111 #78 
GGCAAAAGTGATTAACGCAGAAGGCGAATTTCAAGCCGCCCAACGCTTAAAAGAAGCCGCTGAAATTC
TGGCAAAACAGCCGCAATCGTTACAATTGCGTTATATGCAAACGTTAATGGA
Sequence number 987: Coxiella burnetii IS1111 #79 
TGAAATTCTGGCAAAACAGCCGCAATCGTTACAATTGCGTTATATGCAAACGTTAATGGATTTAGCCA
GCGATAAAACTTCAACCATTGTCTTCCCCATGCCAATCGATATTTTAAAAAT
Sequence number 988: Coxiella burnetii IS1111 #80 
TTTAGCCAGCGATAAAACTTCAACCATTGTCTTCCCCATGCCAATCGATATTTTAAAAATTTTCGAAA
AGAAAGTAAGTGATTAATTATGTAGCCCAATGAAATGGACCCACCCCTTAAA
Sequence number 989: Coxiella burnetii IS1111 #81 
TTTCGAAAAGAAAGTAAGTGATTAATTATGTAGCCCAATGAAATGGACCCACCCCTTAAAGACGGCGT
CATAATGCGCCAACATAGAATTTCTATTTTCAAAAAAAGGAGAAGGTCCATG
Sequence number 990: Coxiella burnetii IS1111 #82 
GACGGCGTCATAATGCGCCAACATAGAATTTCTATTTTCAAAAAAAGGAGAAGGTCCATGAAAGATAT
TAAAATACTGGGTGTTGATATTGCAAAAGATGTTTTTCAACTGTGTGGAATT
Sequence number 991: Coxiella burnetii IS1111 #83 
AAAGATATTAAAATACTGGGTGTTGATATTGCAAAAGATGTTTTTCAACTGTGTGGAATTGATGAGTG
GGGTAAAGTGATCTACACGAGACGGGTTAAGCGTGCTCAGTATGTATCCACC
Sequence number 992: Coxiella burnetii IS1111 #84 
GATGAGTGGGGTAAAGTGATCTACACGAGACGGGTTAAGCGTGCTCAGTATGTATCCACCGTAGCCAG
TCTTAAGGTGGGCTGCGTGGTGATGGAAGCGTGTGGAGGAGCGAACCATTGG
Sequence number 993: Coxiella burnetii IS1111 #85 
GTAGCCAGTCTTAAGGTGGGCTGCGTGGTGATGGAAGCGTGTGGAGGAGCGAACCATTGGTATCGGAC
GTTTATGGGGATGGGTATCCCAACGCAGTTGATCAGTCCGCAGCACGTCAAA
Sequence number 994: Coxiella burnetii IS1111 #86 
TATCGGACGTTTATGGGGATGGGTATCCCAACGCAGTTGATCAGTCCGCAGCACGTCAAACCGTATGT
CAAAAGTAACAAGAATGATCGTAACGATGCGCAGGCGATAGCTGAAGCGGCT
Sequence number 995: Coxiella burnetii IS1111 #87 
CCGTATGTCAAAAGTAACAAGAATGATCGTAACGATGCGCAGGCGATAGCTGAAGCGGCTTCCCGCGC
CTCGATGCGGTTTGTGCGGGGTAAAACGGTGGAACAACAAGACGTTCAAGCG
Sequence number 996: Coxiella burnetii IS1111 #88 
TCCCGCGCCTCGATGCGGTTTGTGCGGGGTAAAACGGTGGAACAACAAGACGTTCAAGCGCTGTTAAA
GATACGCGATCGTTTAGTCAAAAGCCGCACGGCGCTGATCAATGAGATTCGG
Sequence number 997: Coxiella burnetii IS1111 #89 
CTGTTAAAGATACGCGATCGTTTAGTCAAAAGCCGCACGGCGCTGATCAATGAGATTCGGGGGTTGTT
GCAAGAATACGGACTCACGATGGCGCGTGGTGCCAAGCGATTTTATGAAGAG
Sequence number 998: Coxiella burnetii IS1111 #90 
GGGTTGTTGCAAGAATACGGACTCACGATGGCGCGTGGTGCCAAGCGATTTTATGAAGAGCTCCCGTT
GATTTTAGCGAGCGAAGCGGTGGGATTAACACCGCGGATGAAACGGGTGTTG
Sequence number 999: Coxiella burnetii IS1111 #91 
CTCCCGTTGATTTTAGCGAGCGAAGCGGTGGGATTAACACCGCGGATGAAACGGGTGTTGAATTGTTT
GTATACCGAATTGTTGAACCGGGACGAAGCGATTGGTGATTACGAGGAGGAA
Sequence number 1000: Coxiella burnetii IS1111 #92 
AATTGTTTGTATACCGAATTGTTGAACCGGGACGAAGCGATTGGTGATTACGAGGAGGAATTAAAAGC
GGTGGCAAAAGCCAATGAGGATTGTCAACGGGTACAGAGCATCCCGGGGGTG
Sequence number 1001: Coxiella burnetii IS1111 #93 
TTAAAAGCGGTGGCAAAAGCCAATGAGGATTGTCAACGGGTACAGAGCATCCCGGGGGTGGGTTATTT
AACGGCGCTCTCGGTTTATGCGAGCGTGGGTGACATTCATCAATTTCATCGT
Sequence number 1002: Coxiella burnetii IS1111 #94 
GGTTATTTAACGGCGCTCTCGGTTTATGCGAGCGTGGGTGACATTCATCAATTTCATCGTTCCCGGCA
GTTGTCGGCGTTTATTGGGTTGGTCCCTCGACAACATTCGAGTGGGAATAAG
Sequence number 1003: Coxiella burnetii IS1111 #95 
TCCCGGCAGTTGTCGGCGTTTATTGGGTTGGTCCCTCGACAACATTCGAGTGGGAATAAGGAGGTGTT
GTTGGGGATTAGTAAACGCGGCAATGTGATGTTAAGGACGTTATTGATTCAT
Sequence number 1004: Coxiella burnetii IS1111 #96 
GAGGTGTTGTTGGGGATTAGTAAACGCGGCAATGTGATGTTAAGGACGTTATTGATTCATGGCGCCCG
TGCGCTATTGCGTCATGTAAAAAATAAAACGGATAAAAAGAGTCTGTGGTTA
Sequence number 1005: Coxiella burnetii IS1111 #97 
GGCGCCCGTGCGCTATTGCGTCATGTAAAAAATAAAACGGATAAAAAGAGTCTGTGGTTAAAAGCACT
CATTGAGCGCCGCGGAATGAATCGCGCTTGTGTGGCGTTAGCGAATAAAAAT
Sequence number 1006: Coxiella burnetii IS1111 #98 
AAAGCACTCATTGAGCGCCGCGGAATGAATCGCGCTTGTGTGGCGTTAGCGAATAAAAATGCGCCGAT
CATTTGGGCGCTTTTAACACGCCAAGAAACGTATCGCTGTGGCGCCTAAACA
Sequence number 1007: Coxiella burnetii IS1111 #99 
GCGCCGATCATTTGGGCGCTTTTAACACGCCAAGAAACGTATCGCTGTGGCGCCTAAACACCGCCGTG
GGTAAAAAAAAGAATTAACAAAAGGAGACACACCAACCGAGTTCGAAACAAT
Sequence number 1008: Coxiella burnetii IS1111 #100 
CCGCCGTGGGTAAAAAAAAGAATTAACAAAAGGAGACACACCAACCGAGTTCGAAACAATGAGGGCTG
ATGAAAGTAAGGTAAAACCTGAGGTTGATTAAGCTGATTCATACGGTGGCTC
Sequence number 1009: Coxiella burnetii IS1111 #101 
GAGGGCTGATGAAAGTAAGGTAAAACCTGAGGTTGATTAAGCTGATTCATACGGTGGCTCTGTGAAGC
CGATAGCCCGATAAGCATCAACCTTGCATAATTCATCAAGGCACCAATGGTG
Sequence number 1010: Coxiella burnetii IS1111 #102 
TGTGAAGCCGATAGCCCGATAAGCATCAACCTTGCATAATTCATCAAGGCACCAATGGTGGCCAATTT
AAATTGTGATGCCGGATATACGAATGCAACCGCTTTCTTTTCATCAAAACTG
Sequence number 1011: Coxiella burnetii IS1111 #103 GCCAATTTAAATTGTGATGCCGGATATACGAATGCAACCGCTTTCTTTTCATCAAAACTGATTGTTGA CAACAAGGGTGGGTCCATATACGTATGACGCGAACGGAAGGCAGAAGACAGA
Sequence number 1012: Coxiella burnetii IS1111 #104 
ATTGTTGACAACAAGGGTGGGTCCATATACGTATGACGCGAACGGAAGGCAGAAGACAGAGGGCAGAA
GACAGAGGACAGAAGGCAGAAGACAGTGGTTGTGTCGCTACGCAACAACTAA
Sequence number 1013: Coxiella burnetii IS1111 #105 
GGGCAGAAGACAGAGGACAGAAGGCAGAAGACAGTGGTTGTGTCGCTACGCAACAACTAAATTTTATA
AATTAATTGTGGCGAAGCCACACGAACACTGTCCTCTGTCCTCTGCCTAGGG
Sequence number 1014: Coxiella burnetii IS1111 #106 
ATTTTATAAATTAATTGTGGCGAAGCCACACGAACACTGTCCTCTGTCCTCTGCCTAGGGAATCACGG
TATTTACTCATTTATTTTTCCTTTTTACTCTTTCTCGATGAGTCATTCACCT
Sequence number 1015: Coxiella burnetii IS1111 #107 
AATCACGGTATTTACTCATTTATTTTTCCTTTTTACTCTTTCTCGATGAGTCATTCACCTTCTTACGG
GTTTACATTGCTTGAGGTATTAATTGCCTGGTCTATTTTATCCGGTATTTTA
Sequence number 1016: Coxiella burnetii IS1111 #108 
TCTTACGGGTTTACATTGCTTGAGGTATTAATTGCCTGGTCTATTTTATCCGGTATTTTATTAAGTGT
TTTGTTTTTACAAATCGATGAGGTTCGCCATATTCGTCATGCTTATTATTAC
Sequence number 1017: Coxiella burnetii IS1111 #109 
TTAAGTGTTTTGTTTTTACAAATCGATGAGGTTCGCCATATTCGTCATGCTTATTATTACAGCGTAGC
TACGGTTCAATTACGATCTTTAATAGAACGACTGCGCGCGAATCAAACTTCC
Sequence number 1018: Coxiella burnetii IS1111 #110 
AGCGTAGCTACGGTTCAATTACGATCTTTAATAGAACGACTGCGCGCGAATCAAACTTCCGTTGCGCG
TGAACGGGAATGGAGGGATTGGAATAATGAGAATAAGGGGGTGTTGCCGCAA
Sequence number 1019: Coxiella burnetii IS1111 #111 
GTTGCGCGTGAACGGGAATGGAGGGATTGGAATAATGAGAATAAGGGGGTGTTGCCGCAAGGAGAAGG
GAATTATCGCTGTGAACGACGTATTTGCCACGTGACCGTTCATTAGCGGGAG
Sequence number 1020: Coxiella burnetii IS1111 #112 
GGAGAAGGGAATTATCGCTGTGAACGACGTATTTGCCACGTGACCGTTCATTAGCGGGAGAGAAAAAT
ACAAACTTTATCTTTGGTTGCAGCGGTATGAAATTGCAAACAGGGTTCAGTC
Sequence number 1021: Coxiella burnetii IS1111 #113 
AGAAAAATACAAACTTTATCTTTGGTTGCAGCGGTATGAAATTGCAAACAGGGTTCAGTCTAATTGAA
TTATTGGTGTCTCTGTTTTTAGGCTCTTTATTAATGATTATGCTTGTGAAAC
Sequence number 1022: Coxiella burnetii IS1111 #114 
TAATTGAATTATTGGTGTCTCTGTTTTTAGGCTCTTTATTAATGATTATGCTTGTGAAACTCTATTTC
AGCTTTCGTGAAACCGCACAATGGCAGGATACCCTAGCAGCCATTCAAGAAC
Sequence number 1023: Coxiella burnetii IS1111 #115 
TCTATTTCAGCTTTCGTGAAACCGCACAATGGCAGGATACCCTAGCAGCCATTCAAGAACGCGAACGG
TTTGGTGCGTATTTCTTAGCGCAACGCATTCGACAAGCGGGCGATTCGACTT
Sequence number 1024: Coxiella burnetii IS1111 #116 
GCGAACGGTTTGGTGCGTATTTCTTAGCGCAACGCATTCGACAAGCGGGCGATTCGACTTGTGAACCT
GGTGAAACGGTGGATCAATCAAATGCCGTTATAGGGTACGATTCAGATCATT
Sequence number 1025: Coxiella burnetii IS1111 #117 
GTGAACCTGGTGAAACGGTGGATCAATCAAATGCCGTTATAGGGTACGATTCAGATCATTTGCCGCCC
GATTTACGCAAAGAAAGCATTCCTAATTCTGACGTCATCGTTATTGGCGAAT
Sequence number 1026: Coxiella burnetii IS1111 #118 
TGCCGCCCGATTTACGCAAAGAAAGCATTCCTAATTCTGACGTCATCGTTATTGGCGAATGCTTGCGT
TATCACAATAAGTTACAATTTATAAAAACCGCTTATTTTATCAGCGATACGC
Sequence number 1027: Coxiella burnetii IS1111 #119 
GCTTGCGTTATCACAATAAGTTACAATTTATAAAAACCGCTTATTTTATCAGCGATACGCATCGCAAA
GAAAAGGGGAAACCAGTTTTTTTGTATGAAAAACCAATGGATGGGGCGCGGC
Sequence number 1028: Coxiella burnetii IS1111 #120 
ATCGCAAAGAAAAGGGGAAACCAGTTTTTTTGTATGAAAAACCAATGGATGGGGCGCGGCAGGAACTA
ATTGCCGACGTTAATAAATTAACCATTCACTATGGGGTTTCCTCTTCTGACC
Sequence number 1029: Coxiella burnetii IS1111 #121 
AGGAACTAATTGCCGACGTTAATAAATTAACCATTCACTATGGGGTTTCCTCTTCTGACCGTCAATCC
ATTCGTTATTACCACACTGCCGCCGCAATTAAAAACTGGGAAGCTATCAAAA
Sequence number 1030: Coxiella burnetii IS1111 #122 
GTCAATCCATTCGTTATTACCACACTGCCGCCGCAATTAAAAACTGGGAAGCTATCAAAAGCGTCAAT
CTAACGCTTTTTTTCATTCTAATTTAATGCTTCTTAAAAAATGGCATCTGTA
Sequence number 1031: Coxiella burnetii IS1111 #123 
GCGTCAATCTAACGCTTTTTTTCATTCTAATTTAATGCTTCTTAAAAAATGGCATCTGTATGTGACTT
TGCGAGAAAGATTTTGAAATCCCAACAGGCAGAGCAAGGAATGATACTGGTC
Sequence number 1032: Coxiella burnetii IS1111 #124 
TGTGACTTTGCGAGAAAGATTTTGAAATCCCAACAGGCAGAGCAAGGAATGATACTGGTCAGCGTACT
TTTATTCCTATTTGCGCTGAGTTTATTAATGACAGCGCATCTTGAACTTCAC
Sequence number 1033: Coxiella burnetii IS1111 #125 
AGCGTACTTTTATTCCTATTTGCGCTGAGTTTATTAATGACAGCGCATCTTGAACTTCACTTGCTCGA
AACTAAAATGAGTGAAAATAATTATCATCGAATTGTCACCTTTGAAGCAGCT
Sequence number 1034: Coxiella burnetii IS1111 #126 
TTGCTCGAAACTAAAATGAGTGAAAATAATTATCATCGAATTGTCACCTTTGAAGCAGCTGAAGCAGG
TTTGATGGCGGCTGAAGCTTCAATACAAGGAAAATCATTTTCACTACCGTCT
Sequence number 1035: Coxiella burnetii IS1111 #127 
GAAGCAGGTTTGATGGCGGCTGAAGCTTCAATACAAGGAAAATCATTTTCACTACCGTCTTCGGGGGC
GACAGTGAATTATCATATTCAGTTAATGGGCACGGACGCTTGTGATAAAAAA
Sequence number 1036: Coxiella burnetii IS1111 #128 
TCGGGGGCGACAGTGAATTATCATATTCAGTTAATGGGCACGGACGCTTGTGATAAAAAACGGTATCG
CATTACGGCTGATGCCCTTTTTCATCGAAGCAAAGTGGCTTTGCTCAGTGAG
Sequence number 1037: Coxiella burnetii IS1111 #129 
CGGTATCGCATTACGGCTGATGCCCTTTTTCATCGAAGCAAAGTGGCTTTGCTCAGTGAGTATGAATA
CTTATTGTCAACGACAAAACCAGCCTGTAAAAAAGAAGTAATTAATCGTCGC
Sequence number 1038: Coxiella burnetii IS1111 #130 
TATGAATACTTATTGTCAACGACAAAACCAGCCTGTAAAAAAGAAGTAATTAATCGTCGCACGTATTG
GAAAAGACTTTAAAAAGGGTTGAGCCTGTCGGCTCAACCTGCTATGTTTTAT
Sequence number 1039: Coxiella burnetii IS1111 #131 ACGTATTGGAAAAGACTTTAAAAAGGGTTGAGCCTGTCGGCTCAACCTGCTATGTTTTATTCTCAATA ATCGAAACCTGCGCCAGCCCATTTAATACTGCCGCGATACGAATGACATGTT
Sequence number 1040: Coxiella burnetii IS1111 #132 
TCTCAATAATCGAAACCTGCGCCAGCCCATTTAATACTGCCGCGATACGAATGACATGTTGAATGGAA
TGTTTGCTGAAACCAGCTTTAATTAATGTATTAGCATGGGCATCAATACACA
Sequence number 1041: Coxiella burnetii IS1111 #133 
GAATGGAATGTTTGCTGAAACCAGCTTTAATTAATGTATTAGCATGGGCATCAATACACAAGCCGCAA
CCATTGATGGCAGAAACGGCTAAGGAATAAAGTTCAAAATCTTTTTTATCGA
Sequence number 1042: Coxiella burnetii IS1111 #134 
AGCCGCAACCATTGATGGCAGAAACGGCTAAGGAATAAAGTTCAAAATCTTTTTTATCGATGCCAGGA
TTTGCGATTATATTCATGCGCAAATTAGCGGGTAGGCGTTGGTATTCACTGT
Sequence number 1043: Coxiella burnetii IS1111 #135 
TGCCAGGATTTGCGATTATATTCATGCGCAAATTAGCGGGTAGGCGTTGGTATTCACTGTCTTTTACC
AAATGCACGAAGCGATAATAAATATTATTCATCGCCATAATGCTGGCCGCCG
Sequence number 1044: Coxiella burnetii IS1111 #136 
CTTTTACCAAATGCACGAAGCGATAATAAATATTATTCATCGCCATAATGCTGGCCGCCGCTTTAATG
GCCTGTAAGGTCGATTCATCAAGCTCAGCTTCGGCTTTTTGAAAAATGAGCT
Sequence number 1045: Coxiella burnetii IS1111 #137 
CTTTAATGGCCTGTAAGGTCGATTCATCAAGCTCAGCTTCGGCTTTTTGAAAAATGAGCTCGATTAAC
TGACGATTTCGTGTAGCATAAGCAACGGCTAATGCAACACCTGTGATTTGTT
Sequence number 1046: Coxiella burnetii IS1111 #138 
CGATTAACTGACGATTTCGTGTAGCATAAGCAACGGCTAATGCAACACCTGTGATTTGTTGATTTGAT
AGTTCGCTCGAAGGCGACTCACTTAATACTTGCGTTAGATTTAATTTTAAAT
Sequence number 1047: Coxiella burnetii IS1111 #139 
GATTTGATAGTTCGCTCGAAGGCGACTCACTTAATACTTGCGTTAGATTTAATTTTAAATCTTTTGCA
TAATCGGGTAATTGATCTTTGTAAGTTTGTAACATGTTTATTCTCCTCACGT
Sequence number 1048: Coxiella burnetii IS1111 #140 
CTTTTGCATAATCGGGTAATTGATCTTTGTAAGTTTGTAACATGTTTATTCTCCTCACGTAGCCCAAT
GAAATGGACCCACCCCTTAAAGACGGCGTCATAATGCGCCAACATAGAATTT
Sequence number 1049: Coxiella burnetii IS1111 #141 
AGCCCAATGAAATGGACCCACCCCTTAAAGACGGCGTCATAATGCGCCAACATAGAATTTCTATTTTC
AAAAAAAGGAGAAGGTCCATGAAAGATATTAAAATACTGGGTGTTGATATTG
Sequence number 1050: Coxiella burnetii IS1111 #142 
CTATTTTCAAAAAAAGGAGAAGGTCCATGAAAGATATTAAAATACTGGGTGTTGATATTGCAAAAGAT
GTTTTTCAACTGTGTGGAATTGATGAGTGGGGTAAAGTGATCTACACGAGAC
Sequence number 1051: Coxiella burnetii IS1111 #143 
CAAAAGATGTTTTTCAACTGTGTGGAATTGATGAGTGGGGTAAAGTGATCTACACGAGACGGGTTAAG
CGTGCTCAGTATGTATCCACCGTAGCCAGTCTTAAGGTGGGCTGCGTGGTGA
Sequence number 1052: Coxiella burnetii IS1111 #144 
GGGTTAAGCGTGCTCAGTATGTATCCACCGTAGCCAGTCTTAAGGTGGGCTGCGTGGTGATGGAAGCG
TGTGGAGGAGCGAACCATTGGTATCGGACGTTTATGGGGATGGGTATCCCAA
Sequence number 1053: Coxiella burnetii IS1111 #145 
TGGAAGCGTGTGGAGGAGCGAACCATTGGTATCGGACGTTTATGGGGATGGGTATCCCAACGCAGTTG
ATCAGTCCGCAGCACGTCAAACCGTATGTCAAAAGTAACAAGAATGATCGTA
Sequence number 1054: Coxiella burnetii IS1111 #146 
CGCAGTTGATCAGTCCGCAGCACGTCAAACCGTATGTCAAAAGTAACAAGAATGATCGTAACGATGCG
CAGGCGATAGCTGAAGCGGCTTCCCGCGCCTCGATGCGGTTTGTGCGGGGTA
Sequence number 1055: Coxiella burnetii IS1111 #147 
ACGATGCGCAGGCGATAGCTGAAGCGGCTTCCCGCGCCTCGATGCGGTTTGTGCGGGGTAAAACGGTG
GAACAACAAGACGTTCAAGCGCTGTTAAAGATACGCGATCGTTTAGTCAAAA
Sequence number 1056: Coxiella burnetii IS1111 #148 
AAACGGTGGAACAACAAGACGTTCAAGCGCTGTTAAAGATACGCGATCGTTTAGTCAAAAGCCGCACG
GCGCTGATCAATGAGATTCGGGGGTTGTTGCAAGAATACGGACTCACGATGG
Sequence number 1057: Coxiella burnetii IS1111 #149 
GCCGCACGGCGCTGATCAATGAGATTCGGGGGTTGTTGCAAGAATACGGACTCACGATGGCGCGTGGT
GCCAAGCGATTTTATGAAGAGCTCCCGTTGATTTTAGCGAGCGAAGCGGTGG
Sequence number 1058: Coxiella burnetii IS1111 #150 
CGCGTGGTGCCAAGCGATTTTATGAAGAGCTCCCGTTGATTTTAGCGAGCGAAGCGGTGGGATTAACA
CCGCGGATGAAACGGGTGTTGAATTGTTTGTATACCGAATTGTTGAACCGGG
Sequence number 1059: Coxiella burnetii IS1111 #151 
GATTAACACCGCGGATGAAACGGGTGTTGAATTGTTTGTATACCGAATTGTTGAACCGGGACGAAGCG
ATTGGTGATTACGAGGAGGAATTAAAAGCGGTGGCAAAAGCCAATGAGGATT
Sequence number 1060: Coxiella burnetii IS1111 #152 
ACGAAGCGATTGGTGATTACGAGGAGGAATTAAAAGCGGTGGCAAAAGCCAATGAGGATTGTCAACGG
GTACAGAGCATCCCGGGGGTGGGTTATTTAACGGCGCTCTCGGTTTATGCGA
Sequence number 1061: Coxiella burnetii IS1111 #153 
GTCAACGGGTACAGAGCATCCCGGGGGTGGGTTATTTAACGGCGCTCTCGGTTTATGCGAGCGTGGTG
ACATTCATCAATTTCATCGTTCCCGGCAGTTGTCGGCGTTTATTGGGTTGGT
Sequence number 1062: Coxiella burnetii IS1111 #154 
GCGTGGTGACATTCATCAATTTCATCGTTCCCGGCAGTTGTCGGCGTTTATTGGGTTGGTCCCTCGAC
AACATTCGAGTGGGAATAAGGAGGTGTTGTTGGGGATTAGTAAACGCGGCAA
Sequence number 1063: Coxiella burnetii IS1111 #155 
CCCTCGACAACATTCGAGTGGGAATAAGGAGGTGTTGTTGGGGATTAGTAAACGCGGCAATGTGATGT
TAAGGACGTTATTGATTCATGGCGCCCGTGCGCTATTGCGTCATGTAAAAAA
Sequence number 1064: Coxiella burnetii IS1111 #156 
TGTGATGTTAAGGACGTTATTGATTCATGGCGCCCGTGCGCTATTGCGTCATGTAAAAAATAAAACGG
ATAAAAAGAGTCTGTGGTTAAAAGCACCCATTGAGCGCCGCGGAATGAATCG
Sequence number 1065: Coxiella burnetii IS1111 #157 
TAAAACGGATAAAAAGAGTCTGTGGTTAAAAGCACCCATTGAGCGCCGCGGAATGAATCGCGCTTGTG
TGGCGTTAGCGAATAAAAATGCGCGGATCATTTGGGCGCTTTTAACACGCCA
Sequence number 1066: Coxiella burnetii IS1111 #158 
CGCTTGTGTGGCGTTAGCGAATAAAAATGCGCGGATCATTTGGGCGCTTTTAACACGCCAAGAAACGT
ATCGCTGTGGCGCCTAAACACCGCCGTGGGTAAAAAAAAGAATTAACAAAAG
Sequence number 1067: Coxiella burnetii IS1111 #159 AGAAACGTATCGCTGTGGCGCCTAAACACCGCCGTGGGTAAAAAAAAGAATTAACAAAAGGAGACACA CCAACCGAGTTCGAAACAATGAGGGCTGATGAAAGTAAGGTAAAACCTGAGG
Sequence number 1068: Coxiella burnetii IS1111 #160 
GAGACACACCAACCGAGTTCGAAACAATGAGGGCTGATGAAAGTAAGGTAAAACCTGAGGTTGATTAA
GCTGATTCATACGGTGGCTCTGTGAAGCCGATAGCCCGATAAGCATCAACCT
Sequence number 1069: Coxiella burnetii IS1111 #161 
TTGATTAAGCTGATTCATACGGTGGCTCTGTGAAGCCGATAGCCCGATAAGCATCAACCTTGCATAAT
TCATCAAGGCACCAATGGTGGCCAATTTAAATTGTGATGCCGGATATACGAA
Sequence number 1070: Coxiella burnetii IS1111 #162 
TGCATAATTCATCAAGGCACCAATGGTGGCCAATTTAAATTGTGATGCCGGATATACGAATGCAACCG
CTTTCTTTTCATCAAAACTGATTGTTGACAACAAGGGTGGGTCCATATACGT
Sequence number 1071: Coxiella burnetii IS1111 #163 
TGCAACCGCTTTCTTTTCATCAAAACTGATTGTTGACAACAAGGGTGGGTCCATATACGTATGAAGCG
CAGCGAAATACGGGGAATTGCCTTTATTTCCCGGCATTTCGCTGCGCTTCAT
Sequence number 1072: Coxiella burnetii IS1111 #164 
ATGAAGCGCAGCGAAATACGGGGAATTGCCTTTATTTCCCGGCATTTCGCTGCGCTTCATACGGGCTA
CTTAGCGCGGTTAGGCAACCACAATTGTTTCTTCGCCTTGCTGCCAGTTGCA
Sequence number 1073: Coxiella burnetii IS1111 #165 
ACGGGCTACTTAGCGCGGTTAGGCAACCACAATTGTTTCTTCGCCTTGCTGCCAGTTGCAAGGGCAGA
GTTCATCGGTTTGTAAAGCGTCAAGAACGCGCAAAACCTCTTGAGGATTGCG
Sequence number 1074: Coxiella burnetii IS1111 #166 
AGGGCAGAGTTCATCGGTTTGTAAAGCGTCAAGAACGCGCAAAACCTCTTGAGGATTGCGGCCAACGT
TGAGGTCGTTCACCATTACAAAACGCACGATGTGGTTAGGATCAACGATAAA
Sequence number 1075: Coxiella burnetii IS1111 #167 
GCCAACGTTGAGGTCGTTCACCATTACAAAACGCACGATGTGGTTAGGATCAACGATAAAGGTGGCAC
GTTCCGCTACGCCTTCTTTTTCGTTCAAAATACCGAGGCGTTGTGACAAGTC
Sequence number 1076: Coxiella burnetii IS1111 #168 
GGTGGCACGTTCCGCTACGCCTTCTTTTTCGTTCAAAATACCGAGGCGTTGTGACAAGTCACGTCGGA
TGTCGGACAGCATGGGGAACGGTAATTGGCTAAGCTCTTCTTTTTCTCGCCG
Sequence number 1077: Coxiella burnetii IS1111 #169 
ACGTCGGATGTCGGACAGCATGGGGAACGGTAATTGGCTAAGCTCTTCTTTTTCTCGCCGCCAAGCTA
AATGGACAAATTCGGAGTCGACGCTGGCGCCGAGCACCTGCGCATCGCGGTC
Sequence number 1078: Coxiella burnetii IS1111 #170 
CCAAGCTAAATGGACAAATTCGGAGTCGACGCTGGCGCCGAGCACCTGCGCATCGCGGTCAGCAAATT
CGCCATTCAGTCGCCCAAATTCAGCAATTTCTGTCGGACATACAAAGGTAAA
Sequence number 1079: Coxiella burnetii IS1111 #171 
AGCAAATTCGCCATTCAGTCGCCCAAATTCAGCAATTTCTGTCGGACATACAAAGGTAAAGTCCTTAG
GCCAGAAGAAGAGGACGAGCCATTTGTTCGCATAGCTTTTTTCGTTGATTTC
Sequence number 1080: Coxiella burnetii IS1111 #172 
GTCCTTAGGCCAGAAGAAGAGGACGAGCCATTTGTTCGCATAGCTTTTTTCGTTGATTTCGGTAAAGG
CTTTGTTAACATCATTGCTGATTACAGCCTTTAATGAAAATGAAGGAAATTG
Sequence number 1081: Coxiella burnetii IS1111 #173 
GGTAAAGGCTTTGTTAACATCATTGCTGATTACAGCCTTTAATGAAAATGAAGGAAATTGTTCGCCGA
TTTTTATCATGGTTTTCTCCTGTAGAAATGGCAGTTGTTAACAGTACAAAGG
Sequence number 1082: Coxiella burnetii IS1111 #174 
TTCGCCGATTTTTATCATGGTTTTCTCCTGTAGAAATGGCAGTTGTTAACAGTACAAAGGTATTAAAA
CAAAGAATCTATGATTGATAAAATTGATTATATTTATTATACTAATAGAAAT
Sequence number 1083: Coxiella burnetii IS1111 #175 
TTTCTCCTGTAGAAATGGCAGTTGTTAACAGTACAAAGGTATTAAAACAAAGAATCTATGATTGATAA
AATTGATTATATTTATTATACTAATAGAAATTTTTTATCATACTTGGGTCTA
Sequence number 1084: Dengue virus 1 Whole genome #1 
AGTTGTTAGTCTACGTGGACCGACAAGAACAGTTTCGAATCGGAAGCTTGCTTAACGTAGTTCTAACA
GTTTTTTATTAGAGAGCAGATCTCTGATGAACAACCAACGGAAGAAGACGGG
Sequence number 1085: Dengue virus 1 Whole genome #2 
TTCTAACAGTTTTTTATTAGAGAGCAGATCTCTGATGAACAACCAACGGAAGAAGACGGGTCGACCGT
CTTTCAATATGCTGAAACGCGCGAGAAACCGCGTGTCAACTGGTTCACAGTT
Sequence number 1086: Dengue virus 1 Whole genome #3 
TCGACCGTCTTTCAATATGCTGAAACGCGCGAGAAACCGCGTGTCAACTGGTTCACAGTTGGCGAAGA
GATTCTCAAAAGGATTGCTCTCAGGCCAAGGACCCATGAAAATGGTGATGGC
Sequence number 1087: Dengue virus 1 Whole genome #4 
GGCGAAGAGATTCTCAAAAGGATTGCTCTCAGGCCAAGGACCCATGAAAATGGTGATGGCTTTCATAG
CATTCCTAAGATTTCTAGCCATACCCCCAACAGCAGGAATTTTGGCTAGATG
Sequence number 1088: Dengue virus 1 Whole genome #5 
TTTCATAGCATTCCTAAGATTTCTAGCCATACCCCCAACAGCAGGAATTTTGGCTAGATGGAGCTCAT
TCAAGAAGAATGGAGCGATCAAAGTGTTACGGGGTTTCAAAAAAGAGATCTC
Sequence number 1089: Dengue virus 1 Whole genome #6 
GAGCTCATTCAAGAAGAATGGAGCGATCAAAGTGTTACGGGGTTTCAAAAAAGAGATCTCAAGCATGC
TGAACATAATGAACAGGAGGAAAAGATCCGTGACCATGCTCCTCATGCTGCT
Sequence number 1090: Dengue virus 1 Whole genome #7 
AAGCATGCTGAACATAATGAACAGGAGGAAAAGATCCGTGACCATGCTCCTCATGCTGCTGCCCACAG
CTCTGGCGTTCCATTTGACCACACGAGGGGGAGAACCACACATGATAGTTAG
Sequence number 1091: Dengue virus 1 Whole genome #8 
GCCCACAGCTCTGGCGTTCCATTTGACCACACGAGGGGGAGAACCACACATGATAGTTAGCAAGCAGG
AAAGAGGAAAGTCACTCCTGTTTAAGACCTCTGCAGGTGTCAACATGTGCAC
Sequence number 1092: Dengue virus 1 Whole genome #9 
CAAGCAGGAAAGAGGAAAGTCACTCCTGTTTAAGACCTCTGCAGGTGTCAACATGTGCACCCTCATTG
CAATGGATTTGGGAGAGTTATGTGAGGATACAATGACCTACAAATGCCCCCG
Sequence number 1093: Dengue virus 1 Whole genome #10 
CCTCATTGCAATGGATTTGGGAGAGTTATGTGAGGATACAATGACCTACAAATGCCCCCGGATCACTG
AAGCGGAACCAGATGATGTTGATTGCTGGTGCAATGCCACGGACACATGGGT
Sequence number 1094: Dengue virus 1 Whole genome #11 
GATCACTGAAGCGGAACCAGATGATGTTGATTGCTGGTGCAATGCCACGGACACATGGGTGACCTATG
GGACGTGTTCTCAGACTGGCGAACACCGACGGGATAAACGTTCTGTCGCATT
Sequence number 1095: Dengue virus 1 Whole genome #12 GACCTATGGGACGTGTTCTCAGACTGGCGAACACCGACGGGATAAACGTTCTGTCGCATTGGCCCCAC ACGTGGGGCTTGGTCTCGAAACAAGAACCGAAACGTGGATGTCCTCTGAAGG
Sequence number 1096: Dengue virus 1 Whole genome #13 
GGCCCCACACGTGGGGCTTGGTCTCGAAACAAGAACCGAAACGTGGATGTCCTCTGAAGGCGCCTGGA
GACAGATACAAAAAGTGGAGACTTGGGCCTTGAGACACCCAGGATTCACGGT
Sequence number 1097: Dengue virus 1 Whole genome #14 
CGCCTGGAGACAGATACAAAAAGTGGAGACTTGGGCCTTGAGACACCCAGGATTCACGGTGATAGCCC
TTTTTCTAGCACATGCCATAGGAACATCCATTACCCAGAAAGGGATCATCTT
Sequence number 1098: Dengue virus 1 Whole genome #15 
GATAGCCCTTTTTCTAGCACATGCCATAGGAACATCCATTACCCAGAAAGGGATCATCTTCATTTTGC
TGATGCTGGTGACACCATCAATGGCCATGCGATGTGTGGGAATAGGTAACAG
Sequence number 1099: Dengue virus 1 Whole genome #16 
CATTTTGCTGATGCTGGTGACACCATCAATGGCCATGCGATGTGTGGGAATAGGTAACAGAGACTTCG
TTGAAGGATTGTCAGGAGCAACATGGGTGGATGTGGTACTGGAGCATGGAAG
Sequence number 1100: Dengue virus 1 Whole genome #17 
AGACTTCGTTGAAGGATTGTCAGGAGCAACATGGGTGGATGTGGTACTGGAGCATGGAAGCTGCGTCA
CCACCATGGCAAAAGATAAACCAACATTGGACATTGAACTCTTGAAGACGGA
Sequence number 1101: Dengue virus 1 Whole genome #18 
CTGCGTCACCACCATGGCAAAAGATAAACCAACATTGGACATTGAACTCTTGAAGACGGAGGTCACGA
ACCCTGCCGTCTTGCGCAAACTGTGCATTGAAGCTAAAATATCAAACACCAC
Sequence number 1102: Dengue virus 1 Whole genome #19 
GGTCACGAACCCTGCCGTCTTGCGCAAACTGTGCATTGAAGCTAAAATATCAAACACCACTACCGATT
CAAGATGTCCAACACAAGGAGAAGCTACACTGGTGGAAGAACAAGACGCGAA
Sequence number 1103: Dengue virus 1 Whole genome #20 
TACCGATTCAAGATGTCCAACACAAGGAGAAGCTACACTGGTGGAAGAACAAGACGCGAACTTTGTGT
GTCGACGAACATTCGTGGACAGAGGCTGGGGTAATGGTTGTGGACTATTCGG
Sequence number 1104: Dengue virus 1 Whole genome #21 
CTTTGTGTGTCGACGAACATTCGTGGACAGAGGCTGGGGTAATGGTTGTGGACTATTCGGGAAGGGAA
GCTTACTAACGTGTGCTAAGTTTAAGTGTGTGACAAAACTTGAAGGAAAGAT
Sequence number 1105: Dengue virus 1 Whole genome #22 
GAAGGGAAGCTTACTAACGTGTGCTAAGTTTAAGTGTGTGACAAAACTTGAAGGAAAGATAGTTCAAT
ATGAAAACTTAAAATATTCGGTGATAGTCACAGTCCACACTGGGGACCAGCA
Sequence number 1106: Dengue virus 1 Whole genome #23 
AGTTCAATATGAAAACTTAAAATATTCGGTGATAGTCACAGTCCACACTGGGGACCAGCACCAGGTAG
GAAATGAGACTACAGAACATGGAACAATTGCAACCATAACACCTCAAGCTCC
Sequence number 1107: Dengue virus 1 Whole genome #24 
CCAGGTAGGAAATGAGACTACAGAACATGGAACAATTGCAACCATAACACCTCAAGCTCCCACGTCGG
AAATACAGCTGACTGACTACGGAGCCCTTACATTGGACTGCTCACCTAGAAC
Sequence number 1108: Dengue virus 1 Whole genome #25 
CACGTCGGAAATACAGCTGACTGACTACGGAGCCCTTACATTGGACTGCTCACCTAGAACAGGGCTGG
ACTTTAATGAGATGGTGCTGTTGACAATGAAAgAAAAATCATGGCTTGTCCA
Sequence number 1109: Dengue virus 1 Whole genome #26 
AGGGCTGGACTTTAATGAGATGGTGCTGTTGACAATGAAAgAAAAATCATGGCTTGTCCACAAACAAT
GGTTTCTAGACTTACCATTGCCTTGGACCTCGGGGGCTTCAACATCTCAAGA
Sequence number 1110: Dengue virus 1 Whole genome #27 
CAAACAATGGTTTCTAGACTTACCATTGCCTTGGACCTCGGGGGCTTCAACATCTCAAGAGACTTGGA
ACAGACAAGATCTGCTGGTCACATTTAAGACAGCTCACGCAAAGAAGCAGGA
Sequence number 1111: Dengue virus 1 Whole genome #28 
GACTTGGAACAGACAAGATCTGCTGGTCACATTTAAGACAGCTCACGCAAAGAAGCAGGAAGTAGTCG
TACTGGGGTCACAAGAAGGAGCAATGCACACTGCGTTGACTGGGGCGACAGA
Sequence number 1112: Dengue virus 1 Whole genome #29 
AGTAGTCGTACTGGGGTCACAAGAAGGAGCAATGCACACTGCGTTGACTGGGGCGACAGAAATCCAGA
CGTCAGGAACGACGACAATCTTCGCAGGACACCTGAAATGTAGACTAAAAT
Sequence number 1113: Dengue virus 1 Whole genome #30 
AATCCAGACGTCAGGAACGACGACAATCTTCGCAGGACACCTGAAATGTAGACTAAAAATGGATAAAC
TGACTTTAAAAGGGGTGTCATATGTGATGTGCACAGGCTCATTTAAGCTAGA
Sequence number 1114: Dengue virus 1 Whole genome #31 
GGATAAACTGACTTTAAAAGGGGTGTCATATGTGATGTGCACAGGCTCATTTAAGCTAGAGAAGGAAG
TGGCTGAGACCCAGCATGGAACTGTCCTAGTGCAGGTTAAATATGAAGGAAC
Sequence number 1115: Dengue virus 1 Whole genome #32 
GAAGGAAGTGGCTGAGACCCAGCATGGAACTGTCCTAGTGCAGGTTAAATATGAAGGAACAGATGCAC
CATGCAAGATTCCAATTTCAACCCAAGATGAGAAAGGAGTGACCCAGAATGG
Sequence number 1116: Dengue virus 1 Whole genome #33 
AGATGCACCATGCAAGATTCCAATTTCAACCCAAGATGAGAAAGGAGTGACCCAGAATGGGAGATTGA
TAACAGCCAATCCCATAGTTACTGACAAAGAAAAACCAGTCAACATTGAAAC
Sequence number 1117: Dengue virus 1 Whole genome #34 
GAGATTGATAACAGCCAATCCCATAGTTACTGACAAAGAAAAACCAGTCAACATTGAAACAGAACCAC
CCTTTGGCGAGAGtTACATCGTGATAGGGGCGGGTGAAAAAGCTTTGAAACT
Sequence number 1118: Dengue virus 1 Whole genome #35 
AGAACCACCCTTTGGCGAGAGt TACATCGTGATAGGGGCGGGTGAAAAAGCTTTGAAACTAAGCTGGT
TCAAGAAAGGAAGCAGCATAGGGAAAATGTTCGAAGCCACCGCCCGGGGAGC
Sequence number 1119: Dengue virus 1 Whole genome #36 
AAGCTGGTTCAAGAAAGGAAGCAGCATAGGGAAAATGTTCGAAGCCACCGCCCGGGGAGCACGAAGGA
TGGCTATCCTGGGGGACACAGCATGGGACTTCGGTTCCATAGGAGGGGTGTT
Sequence number 1120: Dengue virus 1 Whole genome #37 
ACGAAGGATGGCTATCCTGGGGGACACAGCATGGGACTTCGGTTCCATAGGAGGGGTGTTCACATCTT
TGGGAAAATTGGTACACCAGGTTTTTGGAACCGCATATGGGGTCTTGTTCAG
Sequence number 1121: Dengue virus 1 Whole genome #38 
CACATCTTTGGGAAAATTGGTACACCAGGTTTTTGGAACCGCATATGGGGTCTTGTTCAGCGGTGTTT
CCTGGACCATGAAAATAGGAATAGGGGTTCTGCTGACATGGCTAGGATTGAA
Sequence number 1122: Dengue virus 1 Whole genome #39 
CGGTGTTTCCTGGACCATGAAAATAGGAATAGGGGTTCTGCTGACATGGCTAGGATTGAATTCAAGGA
GCACGTCACTCTCGATGACGTGCATTGCAGTTGGCATGGTCACACTGTACCT
Sequence number 1123: Dengue virus 1 Whole genome #40 TTCAAGGAGCACGTCACTCTCGATGACGTGCATTGCAGTTGGCATGGTCACACTGTACCTAGGAGTCA TGGTTCAAGCGGACTCGGGGTGTGTGATCAACTGGAAGGGCAGAGAACTCAA
Sequence number 1124: Dengue virus 1 Whole genome #41 
AGGAGTCATGGTTCAAGCGGACTCGGGGTGTGTGATCAACTGGAAGGGCAGAGAACTCAAGTGTGGAA
GTGGCATCTTTGTCACTAATGAAGTCCACACTTGGACAGAGCAATATAAATT
Sequence number 1125: Dengue virus 1 Whole genome #42 
GTGTGGAAGTGGCATCTTTGTCACTAATGAAGTCCACACTTGGACAGAGCAATATAAATTCCAGGCTG
ACTCCCCAAAAAGACTATCAGCAGCCATTGGGAAGGCATGGGAGGAAGGCGT
Sequence number 1126: Dengue virus 1 Whole genome #43 
CCAGGCTGACTCCCCAAAAAGACTATCAGCAGCCATTGGGAAGGCATGGGAGGAAGGCGTGTGTGGAA
TTCGATCAGCCACACGTCTTGAGAACATCATGTGGAAGCAAATATCAAATGA
Sequence number 1127: Dengue virus 1 Whole genome #44 
GTGTGGAATTCGATCAGCCACACGTCTTGAGAACATCATGTGGAAGCAAATATCAAATGAATTGAACC
ACATTCTACTTGAAAATGACATGAAATTCACAGTGGTTGTAGGAGACGCTAA
Sequence number 1128: Dengue virus 1 Whole genome #45 
ATTGAACCACATTCTACTTGAAAATGACATGAAATTCACAGTGGTTGTAGGAGACGCTAATGGAATCT
TGACCCAAGGAAAAAAGATGATTAGGCCACAACCCATGGAACACAAATACTC
Sequence number 1129: Dengue virus 1 Whole genome #46 
TGGAATCTTGACCCAAGGAAAAAAGATGATTAGGCCACAACCCATGGAACACAAATACTCATGGAAAA
GCTGGGGAAAAGCCAAAATCATAGGAGCAGACACACAGAATACTACCTTCAT
Sequence number 1130: Dengue virus 1 Whole genome #47 
ATGGAAAAGCTGGGGAAAAGCCAAAATCATAGGAGCAGACACACAGAATACTACCTTCATCATCGACG
GCCCAGATACTCCAGAATGCCCCGATGACCAGAGAGCATGGAACATTTGGGA
Sequence number 1131: Dengue virus 1 Whole genome #48 
CATCGACGGCCCAGATACTCCAGAATGCCCCGATGACCAGAGAGCATGGAACATTTGGGAAGTTGAGG
ACTATGGGTTTGGAGTTTTCACGACAAACATATGGCTGAAATTGCGTGACTC
Sequence number 1132: Dengue virus 1 Whole genome #49 
AGTTGAGGACTATGGGTTTGGAGTTTTCACGACAAACATATGGCTGAAATTGCGTGACTCCTACACCC
AAATGTGTGACCACCGGCTAATGTCAGCTGCCATCAAGGACAGCAAGGCAGT
Sequence number 1133: Dengue virus 1 Whole genome #50 
CTACACCCAAATGTGTGACCACCGGCTAATGTCAGCTGCCATCAAGGACAGCAAGGCAGTCCATGCTG
ACATGGGGTACTGGATAGAAAGTGAAAAGAACGAGACCTGGAAGCTaGCGAG
Sequence number 1134: Dengue virus 1 Whole genome #51 
CCATGCTGACATGGGGTACTGGATAGAAAGTGAAAAGAACGAGACCTGGAAGCTaGCGAGAGCCTCCT
TCATAGAAGTGAAGACATGCATCTGGCCAAGATCCCACACTCTATGGAGCAA
Sequence number 1135: Dengue virus 1 Whole genome #52 
AGCCTCCTTCATAGAAGTGAAGACATGCATCTGGCCAAGATCCCACACTCTATGGAGCAATGGAGTCT
TGGAAAGTGAAATGATAATCCCAAAGATATATGGAGGACCAATATCTCAGCA
Sequence number 1136: Dengue virus 1 Whole genome #53 
TGGAGTCTTGGAAAGTGAAATGATAATCCCAAAGATATATGGAGGACCAATATCTCAGCACAACTACA
GACCAGGGTATTTCACACAAACAGCAGGGCCATGGCACCTAGGCAAGTTGGA
Sequence number 1137: Dengue virus 1 Whole genome #54 
CAACTACAGACCAGGGTATTTCACACAAACAGCAGGGCCATGGCACCTAGGCAAGTTGGAATTAGACT
TTAATTTGTGTGAAGGCACCACAGTAGTTGTGGATGAACATTGTGGAAATCG
Sequence number 1138: Dengue virus 1 Whole genome #55 
ATTAGACTTTAATTTGTGTGAAGGCACCACAGTAGTTGTGGATGAACATTGTGGAAATCGAGGTCCAT
CTCTTAGAACTACAACAGTCACAGGAAAGATAATCCATGAATGGTGTTGCAG
Sequence number 1139: Dengue virus 1 Whole genome #56 
AGGTCCATCTCTTAGAACTACAACAGTCACAGGAAAGATAATCCATGAATGGTGTTGCAGATCCTGCA
CGCTACCCCCCCTACGCTTCAGAGGAGAAGACGGATGTTGGTATGGCATGGA
Sequence number 1140: Dengue virus 1 Whole genome #57 
ATCCTGCACGCTACCCCCCCTACGCTTCAGAGGAGAAGACGGATGTTGGTATGGCATGGAAATCAGAC
CAGTTAAGGAGAAGGAAGAGAATCTAGTCAGGTCAATGGTCTCTGCAGGGTC
Sequence number 1141: Dengue virus 1 Whole genome #58 
AATCAGACCAGTTAAGGAGAAGGAAGAGAATCTAGTCAGGTCAATGGTCTCTGCAGGGTCAGGAGAAG
TGGACAGTTTTTCATTGGGACTACTATGCGTATCAATAATGATTGAAGAAGT
Sequence number 1142: Dengue virus 1 Whole genome #59 
AGGAGAAGTGGACAGTTTTTCATTGGGACTACTATGCGTATCAATAATGATTGAAGAAGTGATGAGAT
CCAGATGGAGTAGAAAGATGCTGATGACTGGAACACTGGCTGTTTTCTTCCT
Sequence number 1143: Dengue virus 1 Whole genome #60 
GATGAGATCCAGATGGAGTAGAAAGATGCTGATGACTGGAACACTGGCTGTTTTCTTCCTTCTTATAA
TGGGACAACTGACATGGAATGATCTGATCAGGTTATGCATCATGGTTGGAGC
Sequence number 1144: Dengue virus 1 Whole genome #61 
TCTTATAATGGGACAACTGACATGGAATGATCTGATCAGGTTATGCATCATGGTTGGAGCCAATGTCT
CAGACAGAATGGGGATGGGAACAACGTACCTAGCCTTGATGGCCACTTTTAA
Sequence number 1145: Dengue virus 1 Whole genome #62 
CAATGTCTCAGACAGAATGGGGATGGGAACAACGTACCTAGCCTTGATGGCCACTTTTAAAATGAGAC
CGATGTTCGCTGTTGGGCTATTACTTCGCAGACTAACATCCAGAGAGGTGCT
Sequence number 1146: Dengue virus 1 Whole genome #63 
AATGAGACCGATGTTCGCTGTTGGGCTATTACTTCGCAGACTAACATCCAGAGAGGTGCTCCTTCTCA
CAATTGGACTAAGCCTGGTGGCATCCGTGGAGCTACCAAATTCTTTAGAGGA
Sequence number 1147: Dengue virus 1 Whole genome #64 
CCTTCTCACAATTGGACTAAGCCTGGTGGCATCCGTGGAGCTACCAAATTCTTTAGAGGAGCTAGGGG
ACGGACTTGCGATGGGCATCATGATGTTAAAATTGTTGACTGATTTTCAGTC
Sequence number 1148: Dengue virus 1 Whole genome #65 
GCTAGGGGACGGACTTGCGATGGGCATCATGATGTTAAAATTGTTGACTGATTTTCAGTCACACCAGT
TGTGGATTACCTTACTGTCTCTGACATTCATCAAAACAACTCTCTCACTGGA
Sequence number 1149: Dengue virus 1 Whole genome #66 
ACACCAGTTGTGGATTACCTTACTGTCTCTGACATTCATCAAAACAACTCTCTCACTGGATTATGCAT
GGAAGACAATGGCTATGGTATTGTCAATCGTATCTCTCTTTCCTCTATGTCT
Sequence number 1150: Dengue virus 1 Whole genome #67 
TTATGCATGGAAGACAATGGCTATGGTATTGTCAATCGTATCTCTCTTTCCTCTATGTCTATCCACGA
CCTCTCAAAAAACAACATGGCTTCCGGTGCTGCTAGGATCCTTTGGATGTAA
Sequence number 1151: Dengue virus 1 Whole genome #68 ATCCACGACCTCTCAAAAAACAACATGGCTTCCGGTGCTGCTAGGATCCTTTGGATGTAAACCACTAA CCATGTTTCTTATAACAGAAAACAAAATTTGGGGAAGGAAAAGTTGGCCCCT
Sequence number 1152: Dengue virus 1 Whole genome #69 
ACCACTAACCATGTTTCTTATAACAGAAAACAAAATTTGGGGAAGGAAAAGTTGGCCCCTCAATGAAG
GAATTATGGCTGTTGGAATAGTCAGCATTTTATTAAGTTCACTCCTCAAAAA
Sequence number 1153: Dengue virus 1 Whole genome #70 
CAATGAAGGAATTATGGCTGTTGGAATAGTCAGCATTTTATTAAGTTCACTCCTCAAAAATGACGTGC
CACTAGCCGGCCCACTAATAGCTGGAGGCATGCTGATAGCATGTTACGTCAT
Sequence number 1154: Dengue virus 1 Whole genome #71 
TGACGTGCCACTAGCCGGCCCACTAATAGCTGGAGGCATGCTGATAGCATGTTACGTCATATCCGGAA
GCTCAGCCGATTTATCATTGGAGAGAGCGGCTGAGGTCTCCTGGGAAGAAGA
Sequence number 1155: Dengue virus 1 Whole genome #72 
ATCCGGAAGCTCAGCCGATTTATCATTGGAGAGAGCGGCTGAGGTCTCCTGGGAAGAAGAAGCAGAAC
ACTCTGGTGCCTCACACAACATACTAGTGGAGGTCCAAGATGATGGAACTAT
Sequence number 1156: Dengue virus 1 Whole genome #73 
AGCAGAACACTCTGGTGCCTCACACAACATACTAGTGGAGGTCCAAGATGATGGAACTATGAAAATAA
AAGATGAAGAGAGAGATGATACGCTCACTATACTCCTCAAAGCAACTTTGTT
Sequence number 1157: Dengue virus 1 Whole genome #74 
GAAAATAAAAGATGAAGAGAGAGATGATACGCTCACTATACTCCTCAAAGCAACTTTGTTGGCAGTTT
CAGGGGTGTACCCAATGTCAATACCAGCAACCCTTTTTGTGTGGTATTTTTG
Sequence number 1158: Dengue virus 1 Whole genome #75 
GGCAGTTTCAGGGGTGTACCCAATGTCAATACCAGCAACCCTTTTTGTGTGGTATTTTTGGCAGAAAA
AGAAACAGAGATCAGGAGTCTTGTGGGACACACCCAGCCCTCCAGAAGTAGA
Sequence number 1159: Dengue virus 1 Whole genome #76 
GCAGAAAAAGAAACAGAGATCAGGAGTCTTGTGGGACACACCCAGCCCTCCAGAAGTAGAAAGAGCAG
TTCTTGATGATGGTATCTATAGAATCTTGCAAAGAGGACTGTTGGGTAGGTC
Sequence number 1160: Dengue virus 1 Whole genome #77 
AAGAGCAGTTCTTGATGATGGTATCTATAGAATCTTGCAAAGAGGACTGTTGGGTAGGTCCCAGGTAG
GAGTGGGAGTTTTTCAAGACGGCGTGTTCCACACAATGTGGCATGTCACCAG
Sequence number 1161: Dengue virus 1 Whole genome #78 
CCAGGTAGGAGTGGGAGTTTTTCAAGACGGCGTGTTCCACACAATGTGGCATGTCACCAGGGGGGCTG
TCCTCATGTACCAAGGGAAGAGGCTGGAACCAAGTTGGGCCAGTGTCAAAAA
Sequence number 1162: Dengue virus 1 Whole genome #79 
GGGGGCTGTCCTCATGTACCAAGGGAAGAGGCTGGAACCAAGTTGGGCCAGTGTCAAAAAAGACTTGA
TCTCATATGGAGGAGGTTGGAGGTTTCAAGGATCATGGAACACGGGAGAAGA
Sequence number 1163: Dengue virus 1 Whole genome #80 
AGACTTGATCTCATATGGAGGAGGTTGGAGGTTTCAAGGATCATGGAACACGGGAGAAGAAGTACAGG
TGATAGCTGTTGAACCAGGGAAAAACCCaAAGAATGTACAGACAACGCCAGG
Sequence number 1164: Dengue virus 1 Whole genome #81 
AGTACAGGTGATAGCTGTTGAACCAGGGAAAAACCCaAAGAATGTACAGACAACGCCAGGCACCTTCA
TGACCCCTGAAGGCGCAGTTGGAGCCATAGCCCTAGATTTCAAACCTGGCAC
Sequence number 1165: Dengue virus 1 Whole genome #82 
CACCTTCATGACCCCTGAAGGCGCAGTTGGAGCCATAGCCCTAGATTTCAAACCTGGCACATCTGGAT
CTCCCATCGTGAACAGAGAGAGAAAAATAGTAGGTCTTTATGGAAATGGAGT
Sequence number 1166: Dengue virus 1 Whole genome #83 
ATCTGGATCTCCCATCGTGAACAGAGAGAGAAAAATAGTAGGTCTTTATGGAAATGGAGTGGTGACAA
CAAGTGGAACCTACGTCAGTGCCATTGCCCAAGCTAAAGCGTCACAAGAAGG
Sequence number 1167: Dengue virus 1 Whole genome #84 
GGTGACAACAAGTGGAACCTACGTCAGTGCCATTGCCCAAGCTAAAGCGTCACAAGAAGGGCCTCTAC
CAGAGATTGAGGACGAGGTGTTCAAGAAAAGAAACCTAACAATAATGGACCT
Sequence number 1168: Dengue virus 1 Whole genome #85 
GCCTCTACCAGAGATTGAGGACGAGGTGTTCAAGAAAAGAAACCTAACAATAATGGACCTACATCCAG
GATCAGGAAAAACAAGAAGATATCTTCCAGCCATAGTCCGTGAGGCCATAAA
Sequence number 1169: Dengue virus 1 Whole genome #86 
ACATCCAGGATCAGGAAAAACAAGAAGATATCTTCCAGCCATAGTCCGTGAGGCCATAAAAAGAAAGC
TGCGCACGCTAATCTTGGCTCCCACAAGAGTTGTCGCCTCTGAGATGGCAGA
Sequence number 1170: Dengue virus 1 Whole genome #87 
AAGAAAGCTGCGCACGCTAATCTTGGCTCCCACAAGAGTTGTCGCCTCTGAGATGGCAGAGGCGCTCA
AAGGAATGCCAATAAGATATCAGACAACAGCAGTAAAGAGTGAACACACAGG
Sequence number 1171: Dengue virus 1 Whole genome #88 
GGCGCTCAAAGGAATGCCAATAAGATATCAGACAACAGCAGTAAAGAGTGAACACACAGGAAAGGAGA
TAGTCGATCTCATGTGCCACGCCACTTTCACCATGCGTCTCCTATCTCCAGT
Sequence number 1172: Dengue virus 1 Whole genome #89 
AAAGGAGATAGTCGATCTCATGTGCCACGCCACTTTCACCATGCGTCTCCTATCTCCAGTGAGAGTTC
CCAATTACAACATGATTATCATGGATGAAGCACACTTTACCGATCCAGCTAG
Sequence number 1173: Dengue virus 1 Whole genome #90 
GAGAGTTCCCAATTACAACATGATTATCATGGATGAAGCACACTTTACCGATCCAGCTAGCATAGCGG
CCAGAGGGTACATCTCAACCCGAGTGGGTATGGGTGAAGCAGCTGCGATCTT
Sequence number 1174: Dengue virus 1 Whole genome #91 
CATAGCGGCCAGAGGGTACATCTCAACCCGAGTGGGTATGGGTGAAGCAGCTGCGATCTTTATGACAG
CCACTCCCCCAGGATCGGTAGAGGCCTTTCCACAGAGCAATGCAGTTATTCA
Sequence number 1175: Dengue virus 1 Whole genome #92 
TATGACAGCCACTCCCCCAGGATCGGTAGAGGCCTTTCCACAGAGCAATGCAGTTATTCAAGATGAGG
AAAGAGACATTCCTGAGAGATCATGGAACTCAGGCTAcGACTGGATCACTGA
Sequence number 1176: Dengue virus 1 Whole genome #93 
AGATGAGGAAAGAGACATTCCTGAGAGATCATGGAACTCAGGCTAcGACTGGATCACTGATTTCCCAG
GAAAAACAGTCTGGTTTGTTCCAAGCATCAAATCAGGAAATGACATTGCCAA
Sequence number 1177: Dengue virus 1 Whole genome #94 
TTTCCCAGGAAAAACAGTCTGGTTTGTTCCAAGCATCAAATCAGGAAATGACATTGCCAACTGTTTAA
GAAAAAACGGGAAACGGGTGATCCAATTGAGCAGAAAAACCTTTGACACTGA
Sequence number 1178: Dengue virus 1 Whole genome #95 
CTGTTTAAGAAAAAACGGGAAACGGGTGATCCAATTGAGCAGAAAAACCTTTGACACTGAGTACCAGA
AAACAAAAAACAATGATTGGGACTATGTTGTCACAACAGACATTTCCGAAAT
Sequence number 1179: Dengue virus 1 Whole genome #96 GTACCAGAAAACAAAAAACAATGATTGGACTATGTTGTCACAACAGACATTTCCGAAATGGAGCAA ATTTCCGGGCCGACAGGGTAATAGACCCAAGGCGGTGTTTGAAGCCGGTAAT
Sequence number 1180: Dengue virus 1 Whole genome #97 
GGGAGCAAATTTCCGGGCCGACAGGGTAATAGACCCAAGGCGGTGTTTGAAGCCGGTAATACTAAAAG
ATGGCCCAGAGCGCGTCATTCTGGCCGGACCAATGCCAGTGACCGCGGCCAG
Sequence number 1181: Dengue virus 1 Whole genome #98 
ACTAAAAGATGGCCCAGAGCGCGTCATTCTGGCCGGACCAATGCCAGTGACCGCGGCCAGTGCTGCCC
AGAGAAGAGGAAGAATTGGAAGGAACCAAAACAAAGAAGGTGACCAGTATAT
Sequence number 1182: Dengue virus 1 Whole genome #99 
TGCTGCCCAGAGAAGAGGAAGAATTGGAAGGAACCAAAACAAAGAAGGTGACCAGTATATTTACATGG
GACAGCCTTTAAACAATGATGAGGACCACGCTCATTGGACAGAAGCAAAAAT
Sequence number 1183: Dengue virus 1 Whole genome #100 
TTACATGGGACAGCCTTTAAACAATGATGAGGACCACGCTCATTGGACAGAAGCAAAAATGCTCCTTG
ACAATATAAACACGCCAGAAGGAATTATCCCAGCCCTCTTTGAGCCAGAGAG
Sequence number 1184: Dengue virus 1 Whole genome #101 
GCTCCTTGACAATATAAACACGCCAGAAGGAATTATCCCAGCCCTCTTTGAGCCAGAGAGAGAAAAGA
GTGCAGCAATAGACGGGGAGTACAGACTGCGGGGAGAAGCAAGGAAAACGTT
Sequence number 1185: Dengue virus 1 Whole genome #102 
AGAAAAGAGTGCAGCAATAGACGGGGAGTACAGACTGCGGGGAGAAGCAAGGAAAACGTTCGTGGAGC
TAATGAGAAGAGGGGATTTACCAGTTTGGCTATCTTACAAAGTTGCCTCAGA
Sequence number 1186: Dengue virus 1 Whole genome #103 
CGTGGAGCTAATGAGAAGAGGGGATTTACCAGTTTGGCTATCTTACAAAGTTGCCTCAGAAGGCTTCC
AGTACTCCGACAGAAGATGGTGCTTTGACGGGGAAAGGAACAACCAGGTGTT
Sequence number 1187: Dengue virus 1 Whole genome #104 
AGGCTTCCAGTACTCCGACAGAAGATGGTGCTTTGACGGGGAAAGGAACAACCAGGTGTTGGAGGAGA
ATATGGACGTGGAGATCTGGACAAAAGAAGGAGAAAGAAAGAAATTGCGACC
Sequence number 1188: Dengue virus 1 Whole genome #105 
GGAGGAGAATATGGACGTGGAGATCTGGACAAAAGAAGGAGAAAGAAAGAAATTGCGACCCCGCTGGC
TGGATGCCAGAACCTACTCTGATCCACTGGCCCTGCGCGAGTTTAAAGAGTT
Sequence number 1189: Dengue virus 1 Whole genome #106 
CCGCTGGCTGGATGCCAGAACCTACTCTGATCCACTGGCCCTGCGCGAGTTTAAAGAGTTCGCAGCAG
GAAGAAGAAGTGTCTCAGGTGACCTAATATTAGAAATAGGGAAACTTCCACA
Sequence number 1190: Dengue virus 1 Whole genome #107 
CGCAGCAGGAAGAAGAAGTGTCTCAGGTGACCTAATATTAGAAATAGGGAAACTTCCACAGCATCTGA
CGCTAAGGGCCCAGAATGCTTTGGACAACTTGGTCATGTTGCACAATTCCGA
Sequence number 1191: Dengue virus 1 Whole genome #108 
GCATCTGACGCTAAGGGCCCAGAATGCTTTGGACAACTTGGTCATGTTGCACAATTCCGAACAAGGAG
GAAAAGCCTATAGACATGCTATGGAAGAACTACCAGACACCATAGAAACATT
Sequence number 1192: Dengue virus 1 Whole genome #109 
ACAAGGAGGAAAAGCCTATAGACATGCTATGGAAGAACTACCAGACACCATAGAAACATTGATGCTAC
TAGCTTTGATAGCTGTGCTGACTGGTGGAGTTACGTTGTTTTTCCTATCAGG
Sequence number 1193: Dengue virus 1 Whole genome #110 
GATGCTACTAGCTTTGATAGCTGTGCTGACTGGTGGAGTTACGTTGTTTTTCCTATCAGGAAGGGGTC
TAGGAAAAACATCCATTGGCCTACTTTGTGTAATGGCCTCAAGTGTACTGTT
Sequence number 1194: Dengue virus 1 Whole genome #111 
AAGGGGTCTAGGAAAAACATCCATTGGCCTACTTTGTGTAATGGCCTCAAGTGTACTGTTATGGATGG
CCAGTGTGGAGCCCCATTGGATAGCGGCCTCCATCATACTAGAGTTTTTTCT
Sequence number 1195: Dengue virus 1 Whole genome #112 
ATGGATGGCCAGTGTGGAGCCCCATTGGATAGCGGCCTCCATCATACTAGAGTTTTTTCTGATGGTGC
TGCTCATTCCAGAGCCAGACAGACAGCGTACTCCACAGGACAATCAACTAGC
Sequence number 1196: Dengue virus 1 Whole genome #113 
GATGGTGCTGCTCATTCCAGAGCCAGACAGACAGCGTACTCCACAGGACAATCAACTAGCATATGTGG
TGATAGGTTTGTTATTCATGATATTGACAGTGGCAGCCAATGAGATGGGATT
Sequence number 1197: Dengue virus 1 Whole genome #114 
ATATGTGGTGATAGGTTTGTTATTCATGATATTGACAGTGGCAGCCAATGAGATGGGATTACTGGAAA
CCACAAAGAAAGACCTGGGGATTGGCCATGTAGTCGCCGAAAACCACCACCA
Sequence number 1198: Dengue virus 1 Whole genome #115 
ACTGGAAACCACAAAGAAAGACCTGGGGATTGGCCATGTAGTCGCCGAAAACCACCACCATGCCACAA
TGCTAGACATAGACCTACATCCAGCTTCAGCCTGGACCCTCTATGCAGTAGC
Sequence number 1199: Dengue virus 1 Whole genome #116 
TGCCACAATGCTAGACATAGACCTACATCCAGCTTCAGCCTGGACCCTCTATGCAGTAGCCACAACAA
TCATCACTCCCATGATGAGACACACAATTGAAAACACAACGGCAAACATTTC
Sequence number 1200: Dengue virus 1 Whole genome #117 
CACAACAATCATCACTCCCATGATGAGACACACAATTGAAAACACAACGGCAAACATTTCCCTGACAG
CTATTGCAAATCAGGCGGCTATATTAATGGGACTTGACAAGGGATGGCCAAT
Sequence number 1201: Dengue virus 1 Whole genome #118 
CCTGACAGCTATTGCAAATCAGGCGGCTATATTAATGGGACTTGACAAGGGATGGCCAATATCGAAGA
TGGACATAGGAGTGCCACTTCTCGCCTTAGGGTGCTATTCCCAGGTGAACCC
Sequence number 1202: Dengue virus 1 Whole genome #119 
ATCGAAGATGGACATAGGAGTGCCACTTCTCGCCTTAGGGTGCTATTCCCAGGTGAACCCATTGACAC
TGACAGCGGCGGTGTTGATGTTAGTGGCCCATTATGCCATAATCGGACCTGG
Sequence number 1203: Dengue virus 1 Whole genome #120 
ATTGACACTGACAGCGGCGGTGTTGATGTTAGTGGCCCATTATGCCATAATCGGACCTGGACTGCAAG
CGAAAGCTACTAGAGAAGCCCAAAAAAGGACTGCGGCCGGAATAATGAAAAA
Sequence number 1204: Dengue virus 1 Whole genome #121 
ACTGCAAGCGAAAGCTACTAGAGAAGCCCAAAAAAGGACTGCGGCCGGAATAATGAAAAACCCAACTG
TAGACGGGATTGTTGCAATAGATTTGGATCCTGTGGTTTATGATGCAAAATT
Sequence number 1205: Dengue virus 1 Whole genome #122 
CCCAACTGTAGACGGGATTGTTGCAATAGATTTGGATCCTGTGGTTTATGATGCAAAATTTGAAAAAC
AGCTAGGCCAAATAATGTTACTAATACTCTGCACATCACAGATTCTCTTGAT
Sequence number 1206: Dengue virus 1 Whole genome #123 
TGAAAAACAGCTAGGCCAAATAATGTTACTAATACTCTGCACATCACAGATTCTCTTGATGAGGACTA
CATGGGCCTTATGTGAATCCATCACACTGGCTACTGGACCCTTGACCACTCT
Sequence number 1207: Dengue virus 1 Whole genome #124 GAGGACTACATGGGCCTTATGTGAATCCATCACACTGGCTACTGGACCCTTGACCACTCTCTGGGAGG GATCTCCTGGAAGATTCTGGAATACCACGATAGCAGTGTCCATGGCAAACAT
Sequence number 1208: Dengue virus 1 Whole genome #125 
CTGGGAGGGATCTCCTGGAAGATTCTGGAATACCACGATAGCAGTGTCCATGGCAAACATCTTCAGGG
GAAGTTATCTAGCAGGAGCAGGTCTGGCCTTCTCATTAATGAAATCTTTAGG
Sequence number 1209: Dengue virus 1 Whole genome #126 
CTTCAGGGGAAGTTATCTAGCAGGAGCAGGTCTGGCCTTCTCATTAATGAAATCTTTAGGAGGAGGTA
GGAGAGGCACGGGAGCCCAAGGGGAAACATTGGGAGAAAAATGGAAAAGACA
Sequence number 1210: Dengue virus 1 Whole genome #127 
AGGAGGTAGGAGAGGCACGGGAGCCCAAGGGGAAACATTGGGAGAAAAATGGAAAAGACAACTAAACC
AATTGAGCAAGTCAGAATTTAACACTTACAAAAGGAGTGGAATTATGGAGGT
Sequence number 1211: Dengue virus 1 Whole genome #128 
ACTAAACCAATTGAGCAAGTCAGAATTTAACACTTACAAAAGGAGTGGAATTATGGAGGTGGACAGAT
CTGAAGCCAAGGAAGGACTGAAAAGAGGAGAAACAACCAAACATGCAGTGTC
Sequence number 1212: Dengue virus 1 Whole genome #129 
GGACAGATCTGAAGCCAAGGAAGGACTGAAAAGAGGAGAAACAACCAAACATGCAGTGTCGAGAGGAA
CAGCCAAACTTAGGTGGTTTGTGGAGAGGAACCTTGTGAAACCAGAAGGGAA
Sequence number 1213: Dengue virus 1 Whole genome #130 
GAGAGGAACAGCCAAACTTAGGTGGTTTGTGGAGAGGAACCTTGTGAAACCAGAAGGGAAAGTGATAG
ACCTCGGTTGTGGAAGAGGTGGCTGGTCATATTACTGCGCTGGGCTGAAGAA
Sequence number 1214: Dengue virus 1 Whole genome #131 
AGTGATAGACCTCGGTTGTGGAAGAGGTGGCTGGTCATATTACTGCGCTGGGCTGAAGAAGGTCACAG
AAGTGAAGGGATACACAAAAGGAGGACCCGGTCATGAGGAACCTATCCCAAT
Sequence number 1215: Dengue virus 1 Whole genome #132 
GGTCACAGAAGTGAAGGGATACACAAAAGGAGGACCCGGTCATGAGGAACCTATCCCAATGGCGACTT
ATGGATGGAACATAGTGAAGCTACACTCTGGAAGGGATGTATTCTTTACACC
Sequence number 1216: Dengue virus 1 Whole genome #133 
GGCGACTTATGGATGGAACATAGTGAAGCTACACTCTGGAAGGGATGTATTCTTTACACCACCTGAGA
AATGTGACACCCTTTTGTGTGATATTGGTGAGTCCTCTCCGAATCCAACTAT
Sequence number 1217: Dengue virus 1 Whole genome #134 
ACCTGAGAAATGTGACACCCTTTTGTGTGATATTGGTGAGTCCTCTCCGAATCCAACTATAGAAGAAG
GAAGAACGTTACGTGTTCTAAAGATGGTGGAACCATGGCTCAGAGGAAACCA
Sequence number 1218: Dengue virus 1 Whole genome #135 
AGAAGAAGGAAGAACGTTACGTGTTCTAAAGATGGTGGAACCATGGCTCAGAGGAAACCAGTTTTGCA
TAAAAATCCTAAATCCTTACATGCCAAGTGTGGTAGAAACTCTGGAGCAAAT
Sequence number 1219: Dengue virus 1 Whole genome #136 
GTTTTGCATAAAAATCCTAAATCCTTACATGCCAAGTGTGGTAGAAACTCTGGAGCAAATGCAAAGAA
AACATGGAGGGATGCTAGTGCGAAACCCACTCTCAAGAAATTCTACCCATGA
Sequence number 1220: Dengue virus 1 Whole genome #137 
GCAAAGAAAACATGGAGGGATGCTAGTGCGAAACCCACTCTCAAGAAATTCTACCCATGAAATGTACT
GGGTTTCATGTGGAACAGGAAACATTGTGTCGGCAGTGAACATGACATCCAG
Sequence number 1221: Dengue virus 1 Whole genome #138 
AATGTACTGGGTTTCATGTGGAACAGGAAACATTGTGTCGGCAGTGAACATGACATCCAGAATGTTGC
TGAATCGGTTCACAATGACTCACAGGAAGCCAACGTATGAAAGAGACGTGGA
Sequence number 1222: Dengue virus 1 Whole genome #139 
AATGTTGCTGAATCGGTTCACAATGACTCACAGGAAGCCAACGTATGAAAGAGACGTGGACTTGGGCG
CTGGAACAAGACATGTGGCAGTGGAACCAGAGATAGCCAACCTAGACATTAT
Sequence number 1223: Dengue virus 1 Whole genome #140 
CTTGGGCGCTGGAACAAGACATGTGGCAGTGGAACCAGAGATAGCCAACCTAGACATTATTGGCCAGA
GGATAGAGAACATAAAAAATGAACATAAGTCAACATGGCATTATGATGAGGA
Sequence number 1224: Dengue virus 1 Whole genome #141 
TGGCCAGAGGATAGAGAACATAAAAAATGAACATAAGTCAACATGGCATTATGATGAGGACAATCCAT
ACAAAACATGGGCCTATCATGGATCATATGAGGTCAAGCCATCAGGGTCAGC
Sequence number 1225: Dengue virus 1 Whole genome #142 
CAATCCATACAAAACATGGGCCTATCATGGATCATATGAGGTCAAGCCATCAGGGTCAGCCTCATCCA
TGGTGAATGGCGTGGTGAGATTGCTCACCAAGCCATGGGATGTCATCCCCAT
Sequence number 1226: Dengue virus 1 Whole genome #143 
CTCATCCATGGTGAATGGCGTGGTGAGATTGCTCACCAAGCCATGGGATGTCATCCCCATGGTCACAC
AAATAGCTATGACTGATACCACACCCTTCGGACAACAGAGGGTTTTcAAAGA
Sequence number 1227: Dengue virus 1 Whole genome #144 
GGTCACACAAATAGCTATGACTGATACCACACCCTTCGGACAACAGAGGGTTTTcAAAGAGAAAGTTG
ACACACGCACACCAAGACCAAAACGAGGCACAGCACAAATTATGGAGGTGAC
Sequence number 1228: Dengue virus 1 Whole genome #145 
GAAAGTTGACACACGCACACCAAGACCAAAACGAGGCACAGCACAAATTATGGAGGTGACAGCCAAGT
GGTTGTGGGGATTACTCTCTAGAAATAAAAAACCCAGAATTTGCACAAGAGA
Sequence number 1229: Dengue virus 1 Whole genome #146 
AGCCAAGTGGTTGTGGGGATTACTCTCTAGAAATAAAAAACCCAGAATTTGCACAAGAGAGGAGTTCA
CAAGAAAAGTTAGGTCAAACGCAGCAATTGGAGCAGTGTTTGTTGATGAAAA
Sequence number 1230: Dengue virus 1 Whole genome #147 
GGAGTTCACAAGAAAAGTTAGGTCAAACGCAGCAATTGGAGCAGTGTTTGTTGATGAAAACCAATGGA
ACTCAGCAAAAGAAGCAGTGGAAGACGAAAGGTTTTGGGACCTCGTGCACAG
Sequence number 1231: Dengue virus 1 Whole genome #148 
CCAATGGAACTCAGCAAAAGAAGCAGTGGAAGACGAAAGGTTTTGGGACCTCGTGCACAGAGAGAGAG
AGCTTCATAAACAGGGAAAATGTGCCACGTGTGTCTACAATATGATGGGtAA
Sequence number 1232: Dengue virus 1 Whole genome #149 
AGAGAGAGAGCTTCATAAACAGGGAAAATGTGCCACGTGTGTCTACAATATGATGGGtAAAAGAGAGA
AAAAACTAGGAGAGTTTGGAAAGGCAAAGGGAAGTCGTGCAATATGGTACAT
Sequence number 1233: Dengue virus 1 Whole genome #150 
AAGAGAGAAAAAACTAGGAGAGTTTGGAAAGGCAAAGGGAAGTCGTGCAATATGGTACATGTGGTTGG
GAGCACGCTTTCTAGAGTTTGAAGCCCTTGGCTTCATGAATGAAGACCACTG
Sequence number 1234: Dengue virus 1 Whole genome #151 
GTGGTTGGGAGCACGCTTTCTAGAGTTTGAAGCCCTTGGCTTCATGAATGAAGACCACTGGTTCAGTA
GGGAGAATTCACTCAGTGGAGTGGAAGGAGAAGGACTACATAAACTTGGATA
Sequence number 1235: Dengue virus 1 Whole genome #152 GTTCAGTAGGGAGAATTCACTCAGTGGAGTGGAAGGAGAAGGACTACATAAACTTGGATACATACTTA GAGACATATCAAAGATTCCAGGGGGAAATATGTATGCAGACGACACAGCTGG
Sequence number 1236: Dengue virus 1 Whole genome #153 
CATACTTAGAGACATATCAAAGATTCCAGGGGGAAATATGTATGCAGACGACACAGCTGGATGGGACA
CGAGAATAACAGAGGATGATCTTCAGAATGAGGCTAAAATCACCGACATCAT
Sequence number 1237: Dengue virus 1 Whole genome #154 
ATGGGACACGAGAATAACAGAGGATGATCTTCAGAATGAGGCTAAAATCACCGACATCATGGAGCCCG
AACATGCTCTACTGGCCACGTCAATCTTCAAGCTGACCTATCAAAATAAAGT
Sequence number 1238: Dengue virus 1 Whole genome #155 
GGAGCCCGAACATGCTCTACTGGCCACGTCAATCTTCAAGCTGACCTATCAAAATAAAGTGGTAAGGG
TGCAAAGACCAGCAAAAAGTGGAACCGTGATGGACGTCATATCCAGACGAGA
Sequence number 1239: Dengue virus 1 Whole genome #156 
GGTAAGGGTGCAAAGACCAGCAAAAAGTGGAACCGTGATGGACGTCATATCCAGACGAGACCAGAGAG
GAAGTGGACAGGTCGGAACTTATGGCCTAAACACTTTCACTAACATGGAGGT
Sequence number 1240: Dengue virus 1 Whole genome #157 
CCAGAGAGGAAGTGGACAGGTCGGAACTTATGGCCTAAACACTTTCACTAACATGGAGGTCCAGCTAA
TAAGACAAATGGAATCTGAAGGAATCTTTTCACCCAGCGAATTGGAAAATTC
Sequence number 1241: Dengue virus 1 Whole genome #158 
CCAGCTAATAAGACAAATGGAATCTGAAGGAATCTTTTCACCCAGCGAATTGGAAAATTCAAATCTAG
CTGAGAGAGTTCTCGACTGGTTGGAAAAACATGGCGTCGAAAGGTTGAAAAG
Sequence number 1242: Dengue virus 1 Whole genome #159 
AAATCTAGCTGAGAGAGTTCTCGACTGGTTGGAAAAACATGGCGTCGAAAGGTTGAAAAGAATGGCAA
TCAGCGGAGATGACTGTGTGGTGAAACCAACTGATGACAGGTTTGCAACAGC
Sequence number 1243: Dengue virus 1 Whole genome #160 
AATGGCAATCAGCGGAGATGACTGTGTGGTGAAACCAACTGATGACAGGTTTGCAACAGCTTTAACAG
CCCTGAATGACATGGGAAAGGTGAGAAAAGACATACCGCAATGGGAACCTTC
Sequence number 1244: Dengue virus 1 Whole genome #161 
TTTAACAGCCCTGAATGACATGGGAAAGGTGAGAAAAGACATACCGCAATGGGAACCTTCAAAAGGAT
GGAATGACTGGCAGCAAGTGCCTTTCTGTTCACACCACTTCCACCAACTGAT
Sequence number 1245: Dengue virus 1 Whole genome #162 
AAAAGGATGGAATGACTGGCAGCAAGTGCCTTTCTGTTCACACCACTTCCACCAACTGATCATGAAGG
ATGGGAGGGAGATAGTGGTGCCATGCCGCAACCAAGATGAACTTGTGGGCAG
Sequence number 1246: Dengue virus 1 Whole genome #163 
CATGAAGGATGGGAGGGAGATAGTGGTGCCATGCCGCAACCAAGATGAACTTGTGGGCAGGGCCAGAG
TATCACAAGGCGCCGGGTGGAGCCTGAGAGAAACTGCTTGCCTAGGCAAGTC
Sequence number 1247: Dengue virus 1 Whole genome #164 
GGCCAGAGTATCACAAGGCGCCGGGTGGAGCCTGAGAGAAACTGCTTGCCTAGGCAAGTCATATGCAC
AAATGTGGCAGCTGATGTACTTCCATAGGAGAGACCTGAGACTGGCGGCCAA
Sequence number 1248: Dengue virus 1 Whole genome #165 
ATATGCACAAATGTGGCAGCTGATGTACTTCCATAGGAGAGACCTGAGACTGGCGGCCAATGCTATCT
GTTCAGCCGTTCCAGTTGATTGGGTcccaaccagccGCACCACCTGGTCGAT
Sequence number 1249: Dengue virus 1 Whole genome #166 
TGCTATCTGTTCAGCCGTTCCAGTTGATTGGGTcccaaccagccGCACCACCTGGTCGATCCATGCCC
ACCATCAATGGATGACAACAGAAGACATGTTGTCAGTATGGAATAGGGTTTG
Sequence number 1250: Dengue virus 1 Whole genome #167 
CCATGCCCACCATCAATGGATGACAACAGAAGACATGTTGTCAGTATGGAATAGGGTTTGGATAGAGG
AAAACCCATGGATGGAGGACAAAACTCATGTATCCAGCTGGGAAGATGTTCC
Sequence number 1251: Dengue virus 1 Whole genome #168 
GATAGAGGAAAACCCATGGATGGAGGACAAAACTCATGTATCCAGCTGGGAAGATGTTCCATATTTAG
GAAAAAGGGAAGATCAATGGTGTGGATCTCTGATAGGCTTAACAGCAAGAGC
Sequence number 1252: Dengue virus 1 Whole genome #169 
ATATTTAGGAAAAAGGGAAGATCAATGGTGTGGATCTCTGATAGGCTTAACAGCAAGAGCCACCTGGG
CCACCAACATACAAGTGGCCATAAACCAAGTGAGAAGGCTTATTGGGAATGA
Sequence number 1253: Dengue virus 1 Whole genome #170 
CACCTGGGCCACCAACATACAAGTGGCCATAAACCAAGTGAGAAGGCTTATTGGGAATGAGAATTATT
TAGACTACATGACATCAATGAAGAGATTCAAGAATGAGAGTGATTCCGAAGG
Sequence number 1254: Dengue virus 1 Whole genome #171 
GAATTATTTAGACTACATGACATCAATGAAGAGATTCAAGAATGAGAGTGATTCCGAAGGAGCCCTCT
GGTAAGCCAACACACTCACAAAATAAAGGAAAATAAAAAATCAAACAAGGCA
Sequence number 1255: Dengue virus 1 Whole genome #172 
AGCCCTCTGGTAAGCCAACACACTCACAAAATAAAGGAAAATAAAAAATCAAACAAGGCAAGAAGTCA
GGCCGGTTTAAGCCATAGTACGGTAAGAGCTATGCTGCCTGTGAGCCCCGTC
Sequence number 1256: Dengue virus 1 Whole genome #173 
AGAAGTCAGGCCGGTTTAAGCCATAGTACGGTAAGAGCTATGCTGCCTGTGAGCCCCGTCCAAGGACG
TAAAATGAAGTCAGGCCGAAAGCCACGGCTTGAGCAAACCGTGCTGCCTGTG
Sequence number 1257: Dengue virus 1 Whole genome #174 
CAAGGACGTAAAATGAAGTCAGGCCGAAAGCCACGGCTTGAGCAAACCGTGCTGCCTGTGGCTCCATC
GTGGGGATGTAAAAACCCGGGAGGCTGCAACCCATGGAAGCTGTACGCATGG
Sequence number 1258: Dengue virus 1 Whole genome #175 
GCTCCATCGTGGGGATGTAAAAACCCGGGAGGCTGCAACCCATGGAAGCTGTACGCATGGGGTAGCAG
ACTAGTGGTTAGAGGAGACCCCTCCCAAGATACAACGCAGCAGCGGGGCCCA
Sequence number 1259: Dengue virus 1 Whole genome #176 
GGTAGCAGACTAGTGGTTAGAGGAGACCCCTCCCAAGATACAACGCAGCAGCGGGGCCCAACACCAGG
GGAAGCTGTACCCTGGTGGTAAGGACTAGAGGTTAGAGGAGACCCCCCGCAC
Sequence number 1260: Dengue virus 1 Whole genome #177 
ACACCAGGGGAAGCTGTACCCTGGTGGTAAGGACTAGAGGTTAGAGGAGACCCCCCGCACAACAATAA
ACAGCATATTGACGCTGGGAGAGACCAGAGATCCTGCTGTCTCCACAGCATC
Sequence number 1261: Dengue virus 1 Whole genome #178 
CGCACAACAATAAACAGCATATTGACGCTGGGAGAGACCAGAGATCCTGCTGTCTCCACAGCATCATT
CCAGGCACAGAACGCCAGAAAATGGAATGGTGCTGTTGAATCAACAGGTTCT
Sequence number 1262: Dengue virus 2 Whole genome #1 
GTTCTAACAGTTTTTAATTAGAGCGCAGATCTCTGATGAATAACCAACGGAAAAAGGCGAGAAGTACG
CCTTTCAATATGCTGAAACGCGAGAGAAACCGCGTGTCAACTGTGCAACAGC
Sequence number 1263: Dengue virus 2 Whole genome #2 GAAGTACGCCTTTCAATATGCTGAAACGCGAGAGAAACCGCGTGTCAACTGTGCAACAGCTGACAAAG AGATTCTCACTTGGAATGCTGCAAGGACGCGGACCATTAAAACTGTTCATGG
Sequence number 1264: Dengue virus 2 Whole genome #3 
TGACAAAGAGATTCTCACTTGGAATGCTGCAAGGACGCGGACCATTAAAACTGTTCATGGCCCTTGTG
GCGTTCCTTCGTTTCCTAACAATCCCACCAACAGCAGGGATACTAAAAAGAT
Sequence number 1265: Dengue virus 2 Whole genome #4 
CCCTTGTGGCGTTCCTTCGTTTCCTAACAATCCCACCAACAGCAGGGATACTAAAAAGATGGGGAACG
ATCAAAAAATCAAAAGCTATCAATGTCTTGAGAGGGTTCAGGAAAGAGATTG
Sequence number 1266: Dengue virus 2 Whole genome #5 
GGGGAACGATCAAAAAATCAAAAGCTATCAATGTCTTGAGAGGGTTCAGGAAAGAGATTGGAAGGATG
CTGAACATCTTGAATAGGAGACGCAGGACAGCAGGCGTGATTGTTATGTTGA
Sequence number 1267: Dengue virus 2 Whole genome #6 
GAAGGATGCTGAACATCTTGAATAGGAGACGCAGGACAGCAGGCGTGATTGTTATGTTGATTCCAACA
GCGATGGCGTTCCATTTAACCACACGCAATGGAGAACCACACATGATCGTTG
Sequence number 1268: Dengue virus 2 Whole genome #7 
TTCCAACAGCGATGGCGTTCCATTTAACCACACGCAATGGAGAACCACACATGATCGTTGGTAGGCAG
GAGAAAGGGAAAAGTCTTCTGTTCAAAACAGAGGATGGTGTTAACATGTGTA
Sequence number 1269: Dengue virus 2 Whole genome #8 
GTAGGCAGGAGAAAGGGAAAAGTCTTCTGTTCAAAACAGAGGATGGTGTTAACATGTGTACCCTCATG
GCCATAGACCTTGGTGAATTGTGTGAAGATACAATCACGTACAAGTGTCCTC
Sequence number 1270: Dengue virus 2 Whole genome #9 
CCCTCATGGCCATAGACCTTGGTGAATTGTGTGAAGATACAATCACGTACAAGTGTCCTCTCCTCAGA
CAAAATGAACCAGAAGACATAGATTGTTGGTGCAACTCCACGTCCACATGGG
Sequence number 1271: Dengue virus 2 Whole genome #10 
TCCTCAGACAAAATGAACCAGAAGACATAGATTGTTGGTGCAACTCCACGTCCACATGGGTAACTTAT
GGGACATGTACCACCACAGGAGAACACAGAAGAGAAAAAAGATCAGTGGCGC
Sequence number 1272: Dengue virus 2 Whole genome #11 
TAACTTATGGGACATGTACCACCACAGGAGAACACAGAAGAGAAAAAAGATCAGTGGCGCTCGTTCCA
CATGTGGGTATGGGACTGGAGACACGAACTGAAACATGGATGTCATCAGAAG
Sequence number 1273: Dengue virus 2 Whole genome #12 
TCGTTCCACATGTGGGTATGGGACTGGAGACACGAACTGAAACATGGATGTCATCAGAAGGGGCCTGG
AAACATGTCCAGAGAATTGAAACCTGGATCTTAAGACATCCAGGCTTTACCA
Sequence number 1274: Dengue virus 2 Whole genome #13 
GGGCCTGGAAACATGTCCAGAGAATTGAAACCTGGATCTTAAGACATCCAGGCTTTACCATAATGGCA
GCAATCCTGGCATACACCATAGGAACGACACATTTCCAAAAGGCCTTGATTT
Sequence number 1275: Dengue virus 2 Whole genome #14 
TAATGGCAGCAATCCTGGCATACACCATAGGAACGACACATTTCCAAAAGGCCTTGATTTTCATCTTA
CTGACAGCTGTCGCTCCTTCAATGACAATGCGCTGCATAGGAATATCAAATA
Sequence number 1276: Dengue virus 2 Whole genome #15 
TCATCTTACTGACAGCTGTCGCTCCTTCAATGACAATGCGCTGCATAGGAATATCAAATAGAGACTTC
GTAGAAGGGGTTTCAGGAGGAAGCTGGGTTGACATAGTCTTGGAACATGGAA
Sequence number 1277: Dengue virus 2 Whole genome #16 
GAGACTTCGTAGAAGGGGTTTCAGGAGGAAGCTGGGTTGACATAGTCTTGGAACATGGAAGTTGTGTG
ACGACGATGGCAAAAAATAAACCAACACTGGATTTTGAACTGATAAAAACAG
Sequence number 1278: Dengue virus 2 Whole genome #17 
GTTGTGTGACGACGATGGCAAAAAATAAACCAACACTGGATTTTGAACTGATAAAAACAGAAGCCAAA
CAACCTGCCACTCTAAGGAAGTACTGTATAGAAGCAAAGCTGACCAACACAA
Sequence number 1279: Dengue virus 2 Whole genome #18 
AAGCCAAACAACCTGCCACTCTAAGGAAGTACTGTATAGAAGCAAAGCTGACCAACACAACAACAGAA
TCGCGTTGCCCAACACAAGGGGAACCCAGTCTAAATGAAGAGCAGGACAAAA
Sequence number 1280: Dengue virus 2 Whole genome #19 
CAACAGAATCGCGTTGCCCAACACAAGGGGAACCCAGTCTAAATGAAGAGCAGGACAAAAGGTTCATC
TGCAAACACTCCATGGTAGACAGAGGATGGGGAAATGGATGTGGATTATTTG
Sequence number 1281: Dengue virus 2 Whole genome #20 
GGTTCATCTGCAAACACTCCATGGTAGACAGAGGATGGGGAAATGGATGTGGATTATTTGGAAAGGGA
GGCATTGTGACCTGTGCTATGTTTACATGCAAAAAGAACATGGAAGGAAAAG
Sequence number 1282: Dengue virus 2 Whole genome #21 
GAAAGGGAGGCATTGTGACCTGTGCTATGTTTACATGCAAAAAGAACATGGAAGGAAAAGTCGTGCAG
CCAGAAAATTTGGAATACACCATCGTGATAACACCTCACTCAGGAGAAGAGC
Sequence number 1283: Dengue virus 2 Whole genome #22 
TCGTGCAGCCAGAAAATTTGGAATACACCATCGTGATAACACCTCACTCAGGAGAAGAGCACGCTGTA
GGCAATGACACAGGAAAGCATGGCAAGGAAATTAAAATAACACCACAGAGCT
Sequence number 1284: Dengue virus 2 Whole genome #23 
ACGCTGTAGGCAATGACACAGGAAAGCATGGCAAGGAAATTAAAATAACACCACAGAGCTCCATCACA
GAAGCAGAACTGACAGGCTATGGCACTGTCACGATGGAGTGCTCTCCGAGAA
Sequence number 1285: Dengue virus 2 Whole genome #24 
CCATCACAGAAGCAGAACTGACAGGCTATGGCACTGTCACGATGGAGTGCTCTCCGAGAACGGGCCTC
GACTTTAATGAGATGGTGCTGCTGCAGATGGAAGACAAAGCTTGGCTGGTGC
Sequence number 1286: Dengue virus 2 Whole genome #25 
CGGGCCTCGACTTTAATGAGATGGTGCTGCTGCAGATGGAAGACAAAGCTTGGCTGGTGCACAGGCAA
TGGTTCCTAGACCTGCCGTTACCATGGCTACCTGGAGCGGATACACAAGGAT
Sequence number 1287: Dengue virus 2 Whole genome #26 
ACAGGCAATGGTTCCTAGACCTGCCGTTACCATGGCTACCTGGAGCGGATACACAAGGATCAAATTGG
ATACAGAAAGAGACATTGGTCACTTTCAAAAATCCCCACGCGAAGAAACAGG
Sequence number 1288: Dengue virus 2 Whole genome #27 
CAAATTGGATACAGAAAGAGACATTGGTCACTTTCAAAAATCCCCACGCGAAGAAACAGGATGTCGTC
GTTTTAGGGTCTCAAGAAGGGGCCATGCACACGGCACTCACAGGGGCCACAG
Sequence number 1289: Dengue virus 2 Whole genome #28 
ATGTCGTCGTTTTAGGGTCTCAAGAAGGGGCCATGCACACGGCACTCACAGGGGCCACAGAAATCCAA
ATGTCATCAGGAAACTTACTGTTCACAGGACATCTCAAGTGCAGGCTGAGAA
Sequence number 1290: Dengue virus 2 Whole genome #29 
AAATCCAAATGTCATCAGGAAACTTACTGTTCACAGGACATCTCAAGTGCAGGCTGAGAATGGACAAA
CTACAGCTCAAAGGAATGTCATACTCTATGTGTACAGGAAAGTTTAAAATTG
Sequence number 1291: Dengue virus 2 Whole genome #30 TGGACAAACTACAGCTCAAAGGAATGTCATACTCTATGTGTACAGGAAAGTTTAAAATTGTGAAGGAA ATAGCAGAAACACAACATGGAACAATAGTTATCAGAGTACAATATGAAGGGG
Sequence number 1292: Dengue virus 2 Whole genome #31 
TGAAGGAAATAGCAGAAACACAACATGGAACAATAGTTATCAGAGTACAATATGAAGGGGACGGTTCT
CCATGCAAGATCCCTTTTGAGATAACAGATTTGGAAAAAAGACACGTCTTAG
Sequence number 1293: Dengue virus 2 Whole genome #32 
ACGGTTCTCCATGCAAGATCCCTTTTGAGATAACAGATTTGGAAAAAAGACACGTCTTAGGTCGCTTG
ATCACAGTTAACCCAATCGTAACAGAAAAAGATAGCCCAGTCAACATAGAAG
Sequence number 1294: Dengue virus 2 Whole genome #33 
GTCGCTTGATCACAGTTAACCCAATCGTAACAGAAAAAGATAGCCCAGTCAACATAGAAGCAGAACCT
CCATTTGGAGACAGCTACATCATCATAGGAGTAGAGCCGGGACAATTGAAAC
Sequence number 1295: Dengue virus 2 Whole genome #34 
CAGAACCTCCATTTGGAGACAGCTACATCATCATAGGAGTAGAGCCGGGACAATTGAAACTCAATTGG
TTTAAGAAGGGAAGTTCCATCGGCCAAATGTTTGAGACAACAATGAGAGGAG
Sequence number 1296: Dengue virus 2 Whole genome #35 
TCAATTGGTTTAAGAAGGGAAGTTCCATCGGCCAAATGTTTGAGACAACAATGAGAGGAGCAAAGAGA
ATGGCCATTTTAGGTGACACAGCCTGGGACTTTGGATCCCTGGGAGGAGTGT
Sequence number 1297: Dengue virus 2 Whole genome #36 
CAAAGAGAATGGCCATTTTAGGTGACACAGCCTGGGACTTTGGATCCCTGGGAGGAGTGTTTACATCC
ATAGGAAAGGCTCTCCACCAAGTTTTCGGAGCAATCTATGGGGCTGCTTTTA
Sequence number 1298: Dengue virus 2 Whole genome #37 
TTACATCCATAGGAAAGGCTCTCCACCAAGTTTTCGGAGCAATCTATGGGGCTGCTTTTAGTGGGGTC
TCATGGACTATGAAAATCCTCATAGGAGTCATCATCACATGGATAGGAATGA
Sequence number 1299: Dengue virus 2 Whole genome #38 
GTGGGGTCTCATGGACTATGAAAATCCTCATAGGAGTCATCATCACATGGATAGGAATGAATTCACGT
AGCACCTCACTGTCTGTATCGCTAGTATTGGTGGGCGTCGTGACATTGTACC
Sequence number 1300: Dengue virus 2 Whole genome #39 
ATTCACGTAGCACCTCACTGTCTGTATCGCTAGTATTGGTGGGCGTCGTGACATTGTACCTGGGAGCT
ATGGTGCAAGCTGATAGTGGTTGCGTTGTGAGCTGGAAAAACAAAGAACTGA
Sequence number 1301: Dengue virus 2 Whole genome #40 
TGGGAGCTATGGTGCAAGCTGATAGTGGTTGCGTTGTGAGCTGGAAAAACAAAGAACTGAAATGTGGC
AGCGGGATCTTCATTACAGATAACGTACACACATGGACAGAACAATACAAGT
Sequence number 1302: Dengue virus 2 Whole genome #41 
AATGTGGCAGCGGGATCTTCATTACAGATAACGTACACACATGGACAGAACAATACAAGTTCCAACCA
GAATCCCCTTCAAAACTAGCTTCAGCTATCCAAAAAGCTCATGAAGAGGGCA
Sequence number 1303: Dengue virus 2 Whole genome #42 
TCCAACCAGAATCCCCTTCAAAACTAGCTTCAGCTATCCAAAAAGCTCATGAAGAGGGCATTTGTGGA
ATCCGCTCAGTAACAAGATTGGAGAATCTGATGTGGAAACAAATAACACCAG
Sequence number 1304: Dengue virus 2 Whole genome #43 
TTTGTGGAATCCGCTCAGTAACAAGATTGGAGAATCTGATGTGGAAACAAATAACACCAGAATTGAAT
CATATTCTATCAGAAAATGAGGTAAAGTTGACCATTATGACAGGAGACATTA
Sequence number 1305: Dengue virus 2 Whole genome #44 
AATTGAATCATATTCTATCAGAAAATGAGGTAAAGTTGACCATTATGACAGGAGACATTAAAGGAATC
ATGCAGGCAGGAAAACGATCCTTGCGGCCCCAGCCCACTGAGCTGAAGTACT
Sequence number 1306: Dengue virus 2 Whole genome #45 
AAGGAATCATGCAGGCAGGAAAACGATCCTTGCGGCCCCAGCCCACTGAGCTGAAGTACTCATGGAAA
ACATGGGGAAAGGCGAAAATGCTCTCCACAGAGTCTCACAATCAGACCTTTC
Sequence number 1307: Dengue virus 2 Whole genome #46 
CATGGAAAACATGGGGAAAGGCGAAAATGCTCTCCACAGAGTCTCACAATCAGACCTTTCTTATTGAT
GGCCCTGAAACAGCAGAATGCCCCAACACAAACAGAGCTTGGAACTCGCTGG
Sequence number 1308: Dengue virus 2 Whole genome #47 
TTATTGATGGCCCTGAAACAGCAGAATGCCCCAACACAAACAGAGCTTGGAACTCGCTGGAAGTTGAA
GACTATGGTTTTGGAGTTTTCACCACCAATATATGGCTGAAATTGAGAGAAA
Sequence number 1309: Dengue virus 2 Whole genome #48 
AAGTTGAAGACTATGGTTTTGGAGTTTTCACCACCAATATATGGCTGAAATTGAGAGAAAAACAGGAT
GTATTTTGTGACTCAAAACTCATGTCAGCAGCCATTAAAGACAATAGAGCCG
Sequence number 1310: Dengue virus 2 Whole genome #49 
AACAGGATGTATTTTGTGACTCAAAACTCATGTCAGCAGCCATTAAAGACAATAGAGCCGTTCATGCC
GATATGGGTTATTGGATAGAAAGTGCACTCAATGACACATGGAAGATGGAGA
Sequence number 1311: Dengue virus 2 Whole genome #50 
TTCATGCCGATATGGGTTATTGGATAGAAAGTGCACTCAATGACACATGGAAGATGGAGAAAGCCTCC
TTCATTGAAGTTAAAAGCTGCCACTGGCCAAAGTCACACACCCTTTGGAGCA
Sequence number 1312: Dengue virus 2 Whole genome #51 
AAGCCTCCTTCATTGAAGTTAAAAGCTGCCACTGGCCAAAGTCACACACCCTTTGGAGCAATGGAGTA
TTAGAAAGTGAGATGATAATCCCAAAAAATTTTGCCGGGCCAGTGTCACAAC
Sequence number 1313: Dengue virus 2 Whole genome #52 
ATGGAGTATTAGAAAGTGAGATGATAATCCCAAAAAATTTTGCCGGGCCAGTGTCACAACACAACTAC
AGACCAGGCTACCATACACAAACAGCAGGACCTTGGCATCTAGGTAAGCTTG
Sequence number 1314: Dengue virus 2 Whole genome #53 
ACAACTACAGACCAGGCTACCATACACAAACAGCAGGACCTTGGCATCTAGGTAAGCTTGAGATGGAC
TTTGATCTCTGCGAAGGAACTACAGTGGTGGTGACTGAGGACTGTGGAAATA
Sequence number 1315: Dengue virus 2 Whole genome #54 
AGATGGACTTTGATCTCTGCGAAGGAACTACAGTGGTGGTGACTGAGGACTGTGGAAATAGAGGACCC
TCTTTAAGAACGACCACTGCCTCTGGAAAACTCATAACAGAATGGTGCTGCC
Sequence number 1316: Dengue virus 2 Whole genome #55 
GAGGACCCTCTTTAAGAACGACCACTGCCTCTGGAAAACTCATAACAGAATGGTGCTGCCGATCCTGC
ACACTACCACCTCTAAGATACAGAGGTGAGGATGGATGCTGGTACGGGATGG
Sequence number 1317: Dengue virus 2 Whole genome #56 
GATCCTGCACACTACCACCTCTAAGATACAGAGGTGAGGATGGATGCTGGTACGGGATGGAAATCAGA
CCATTGAAAGAGAAAGAGGAGAATTTGGTCAACTCCTTGGTCACAGCCGGAC
Sequence number 1318: Dengue virus 2 Whole genome #57 
AAATCAGACCATTGAAAGAGAAAGAGGAGAATTTGGTCAACTCCTTGGTCACAGCCGGACATGGGCAG
ATTGATAACTTTTCACTAGGAGTCTTGGGAATGGCACTGTTCCTGGAAGAAA
Sequence number 1319: Dengue virus 2 Whole genome #58 ATGGGCAGATTGATAACTTTTCACTAGGAGTCTTGGGAATGGCACTGTTCCTGGAAGAAATGCTCAGG ACCCGAATAGGAACGAAACATGCAATACTGCTAGTCGCAGTATCTTTTGTGA
Sequence number 1320: Dengue virus 2 Whole genome #59 
TGCTCAGGACCCGAATAGGAACGAAACATGCAATACTGCTAGTCGCAGTATCTTTTGTGACATTGATT
ACTGGGAACATGTCTTTTAGAGACCTGGGAAGAGTGATGGTTATGGTGGGCG
Sequence number 1321: Dengue virus 2 Whole genome #60 
CATTGATTACTGGGAACATGTCTTTTAGAGACCTGGGAAGAGTGATGGTTATGGTGGGCGCTACCATG
ACGGATGACATAGGTATGGGAGTGACTTATCTTGCCCTACTAGCAGCTTTTA
Sequence number 1322: Dengue virus 2 Whole genome #61 
CTACCATGACGGATGACATAGGTATGGGAGTGACTTATCTTGCCCTACTAGCAGCTTTTAAAGTTAGA
CCAACTTTTGCAGCTGGACTACTCTTGAGAAAACTGACCTCCAAGGAATTGA
Sequence number 1323: Dengue virus 2 Whole genome #62 
AAGTTAGACCAACTTTTGCAGCTGGACTACTCTTGAGAAAACTGACCTCCAAGGAATTGATGATGGCC
ACCATAGGAATCGCACTTCTTTCCCAAAGCACCATACCAGAGACCATTCTTG
Sequence number 1324: Dengue virus 2 Whole genome #63 
TGATGGCCACCATAGGAATCGCACTTCTTTCCCAAAGCACCATACCAGAGACCATTCTTGAACTGACC
GATGCATTAGCCTTGGGCATGATGGTCCTCAAAATAGTGAGAAATATGGAAA
Sequence number 1325: Dengue virus 2 Whole genome #64 
AACTGACCGATGCATTAGCCTTGGGCATGATGGTCCTCAAAATAGTGAGAAATATGGAAAAATACCAA
TTGGCAGTGACTATCATGGCTATTTCATGTGTCCCAAATGCAGTGATACTGC
Sequence number 1326: Dengue virus 2 Whole genome #65 
AATACCAATTGGCAGTGACTATCATGGCTATTTCATGTGTCCCAAATGCAGTGATACTGCAAAACGCA
TGGAAGGTGAGCTGCACAATATTGGCAGCGGTGTCCGTTTCACCACTGCTCT
Sequence number 1327: Dengue virus 2 Whole genome #66 
AAAACGCATGGAAGGTGAGCTGCACAATATTGGCAGCGGTGTCCGTTTCACCACTGCTCTTAACATCC
TCACAGCAGAAAGCGGATTGGATACCACTGGCATTGACGATAAAAGGTCTCA
Sequence number 1328: Dengue virus 2 Whole genome #67 
TAACATCCTCACAGCAGAAAGCGGATTGGATACCACTGGCATTGACGATAAAAGGTCTCAATCCAACA
GCCATTTTTCTAACAACTCTTTCGAGAACCAGCAAAAAAAGGAGCTGGCCGC
Sequence number 1329: Dengue virus 2 Whole genome #68 
ATCCAACAGCCATTTTTCTAACAACTCTTTCGAGAACCAGCAAAAAAAGGAGCTGGCCGCTAAATGAA
GCTATCATGGCAGTCGGGATGGTGAGCATTTTAGCCAGTTCTCTCCTAAAGA
Sequence number 1330: Dengue virus 2 Whole genome #69 
TAAATGAAGCTATCATGGCAGTCGGGATGGTGAGCATTTTAGCCAGTTCTCTCCTAAAGAATGATATT
CCCATGACAGGTCCATTAGTGGCTGGAGGGCTCCTTACCGTATGTTACGTGC
Sequence number 1331: Dengue virus 2 Whole genome #70 
ATGATATTCCCATGACAGGTCCATTAGTGGCTGGAGGGCTCCTTACCGTATGTTACGTGCTCACTGGA
CGATCGGCCGATTTGGAACTGGAGAGAGCTGCCGATGTAAAATGGGAAGATC
Sequence number 1332: Dengue virus 2 Whole genome #71 
TCACTGGACGATCGGCCGATTTGGAACTGGAGAGAGCTGCCGATGTAAAATGGGAAGATCAGGCAGAA
ATATCAGGAAGCAGCCCAATCCTATCAATAACAATATCAGAAGATGGCAGCA
Sequence number 1333: Dengue virus 2 Whole genome #72 
AGGCAGAAATATCAGGAAGCAGCCCAATCCTATCAATAACAATATCAGAAGATGGCAGCATGTCGATA
AAAAATGAAGAGGAAGAACAAACACTGACCATACTCATCAGAACGGGATTGT
Sequence number 1334: Dengue virus 2 Whole genome #73 
TGTCGATAAAAAATGAAGAGGAAGAACAAACACTGACCATACTCATCAGAACGGGATTGTTGGTGATC
TCAGGAGTCTTTCCAGTATCGATACCAATTACGGCAGCAGCATGGTACCTGT
Sequence number 1335: Dengue virus 2 Whole genome #74 
TGGTGATCTCAGGAGTCTTTCCAGTATCGATACCAATTACGGCAGCAGCATGGTACCTGTGGGAAGTG
AAGAAACAACGGGCTGGAGTATTGTGGGACGTCCCTTCACCCCCACCAGTGG
Sequence number 1336: Dengue virus 2 Whole genome #75 
GGGAAGTGAAGAAACAACGGGCTGGAGTATTGTGGGACGTCCCTTCACCCCCACCAGTGGAAAAAGCC
GAACTGGAGGATGGAGCCTACAGAATCAAGCAAAGAGGGATTCTTGGATATT
Sequence number 1337: Dengue virus 2 Whole genome #76 
AAAAAGCCGAACTGGAGGATGGAGCCTACAGAATCAAGCAAAGAGGGATTCTTGGATATTCTCAGATC
GGAGCCGGAGTTTACAAAGAAGGAACATTCCATACAATGTGGCACGTCACAC
Sequence number 1338: Dengue virus 2 Whole genome #77 
CTCAGATCGGAGCCGGAGTTTACAAAGAAGGAACATTCCATACAATGTGGCACGTCACACGTGGTGCT
GTTCTGATGCATAGAGGGAAGAGGATTGAACCATCATGGGCAGACGTCAAGA
Sequence number 1339: Dengue virus 2 Whole genome #78 
GTGGTGCTGTTCTGATGCATAGAGGGAAGAGGATTGAACCATCATGGGCAGACGTCAAGAAAGACCTA
ATATCATATGGAGGAGGTTGGAAGCTAGAAGGAGAATGGAAGGAAGGAGAGG
Sequence number 1340: Dengue virus 2 Whole genome #79 
AAGACCTAATATCATATGGAGGAGGTTGGAAGCTAGAAGGAGAATGGAAGGAAGGAGAGGAAGTCCAA
GTCCTGGCACTGGAACCTGGGAAAAATCCAAGAGCAGTCCAAACGAAACCTG
Sequence number 1341: Dengue virus 2 Whole genome #80 
AAGTCCAAGTCCTGGCACTGGAACCTGGGAAAAATCCAAGAGCAGTCCAAACGAAACCTGGAATTTTC
AAAACCAACACCGGAACCATAGGCGCCGTATCTCTGGACTTTTCCCCTGGAA
Sequence number 1342: Dengue virus 2 Whole genome #81 
GAATTTTCAAAACCAACACCGGAACCATAGGCGCCGTATCTCTGGACTTTTCCCCTGGAACGTCAGGA
TCTCCAATTGTCGACAGAAAAGGAAAAGTTGTGGGTCTTTACGGTAATGGTG
Sequence number 1343: Dengue virus 2 Whole genome #82 
CGTCAGGATCTCCAATTGTCGACAGAAAAGGAAAAGTTGTGGGTCTTTACGGTAATGGTGTTGTCACA
AGGAGTGGAGCATATGTAAGTGCCATAGCCCAGACAGAAAAAAGCATTGAAG
Sequence number 1344: Dengue virus 2 Whole genome #83 
TTGTCACAAGGAGTGGAGCATATGTAAGTGCCATAGCCCAGACAGAAAAAAGCATTGAAGACAATCCA
GAGATCGAAGATGACATTTTCCGAAAGAAAAGATTGACCATCATGGACCTCC
Sequence number 1345: Dengue virus 2 Whole genome #84 
ACAATCCAGAGATCGAAGATGACATTTTCCGAAAGAAAAGATTGACCATCATGGACCTCCATCCAGGA
GCAGGAAAGACAAAAAGATACCTTCCAGCCATAGTTAGAGAAGCCATAAAAC
Sequence number 1346: Dengue virus 2 Whole genome #85 
ATCCAGGAGCAGGAAAGACAAAAAGATACCTTCCAGCCATAGTTAGAGAAGCCATAAAACGTGGCTTG
AGAACATTGATCCTGGCTCCCACTAGAGTAGTGGCAGCTGAAATGGAGGAAG
Sequence number 1347: Dengue virus 2 Whole genome #86 GTGGCTTGAGAACATTGATCCTGGCTCCCACTAGAGTAGTGGCAGCTGAAATGGAGGAAGCTCTTAGA GGACTTCCAATAAGATACCAAACCCCAGCCATCAAAACCCAGCATACTGGC
Sequence number 1348: Dengue virus 2 Whole genome #87 
CTCTTAGAGGACTTCCAATAAGATACCAAACCCCAGCCATCAAAACCCAGCATACTGGGCGGGAGATC
GTGGACCTAATGTGTCATGCCACATTTACTATGAGGCTACTATCACCAGTCA
Sequence number 1349: Dengue virus 2 Whole genome #88 
GGGAGATCGTGGACCTAATGTGTCATGCCACATTTACTATGAGGCTACTATCACCAGTCAGAGTACCA
AATTACAACCTGATCATCATGGACGAAGCCCACTTCACAGACCCAGCAAGTA
Sequence number 1350: Dengue virus 2 Whole genome #89 
GAGTACCAAATTACAACCTGATCATCATGGACGAAGCCCACTTCACAGACCCAGCAAGTATAGCAGCT
AGAGGATACATTTCAACTCGAGTAGAGATGGGTGAAGCAGCCGGGATTTTCA
Sequence number 1351: Dengue virus 2 Whole genome #90 
TAGCAGCTAGAGGATACATTTCAACTCGAGTAGAGATGGGTGAAGCAGCCGGGATTTTCATGACAGCC
ACTCCTCCGGGAAGTAGAGACCCATTTCCTCAGAGCAATGCACCAATCATGG
Sequence number 1352: Dengue virus 2 Whole genome #91 
TGACAGCCACTCCTCCGGGAAGTAGAGACCCATTTCCTCAGAGCAATGCACCAATCATGGATGAGGAA
AGAGAAATTCCTGAGCGTTCGTGGAATTCAGGACACGAATGGGTCACAGATT
Sequence number 1353: Dengue virus 2 Whole genome #92 
ATGAGGAAAGAGAAATTCCTGAGCGTTCGTGGAATTCAGGACACGAATGGGTCACAGATTTTAAGGGG
AAGACTGTTTGGTTTGTTCCAAGTATAAAAGCAGGAAATGATATAGCAGCTT
Sequence number 1354: Dengue virus 2 Whole genome #93 
TTAAGGGGAAGACTGTTTGGTTTGTTCCAAGTATAAAAGCAGGAAATGATATAGCAGCTTGTCTTAGG
AAAAATGGAAAGAAAGTGATACAACTCAGTAGGAAGACTTTTGACTCTGAGT
Sequence number 1355: Dengue virus 2 Whole genome #94 
GTCTTAGGAAAAATGGAAAGAAAGTGATACAACTCAGTAGGAAGACTTTTGACTCTGAGTATGTCAAG
ACTAGAGCCAATGATTGGGACTTTGTGGTCACAACTGACATTTCAGAAATGG
Sequence number 1356: Dengue virus 2 Whole genome #95 
ATGTCAAGACTAGAGCCAATGATTGGGACTTTGTGGTCACAACTGACATTTCAGAAATGGGTGCCAAC
TTTAAGGCTGAGAGGGTTATAGACCCCAGACGTTGCATGAAACCAGTTATAC
Sequence number 1357: Dengue virus 2 Whole genome #96 
GTGCCAACTTTAAGGCTGAGAGGGTTATAGACCCCAGACGTTGCATGAAACCAGTTATACTAACAGAT
GGCGAAGAGCGGGTGATCTTGGCAGGACCAATGCCAGTGACCCACTCTAGTG
Sequence number 1358: Dengue virus 2 Whole genome #97 
TAACAGATGGCGAAGAGCGGGTGATCTTGGCAGGACCAATGCCAGTGACCCACTCTAGTGCAGCGCAA
AGAAGAGGGAGAATAGGAAGAAATCCAAAGAATGAAAATGACCAGTACATAT
Sequence number 1359: Dengue virus 2 Whole genome #98 
CAGCGCAAAGAAGAGGGAGAATAGGAAGAAATCCAAAGAATGAAAATGACCAGTACATATATATGGGG
GAACCTCTTGAAAATGATGAAGACTGTGCACATTGGAAAGAAGCTAAAATGC
Sequence number 1360: Dengue virus 2 Whole genome #99 
ATATGGGGGAACCTCTTGAAAATGATGAAGACTGTGCACATTGGAAAGAAGCTAAAATGCTCCTAGAT
AACATCAACACACCCGAAGGAATCATTCCTAGTATGTTCGAACCAGAGCGTG
Sequence number 1361: Dengue virus 2 Whole genome #100 
TCCTAGATAACATCAACACACCCGAAGGAATCATTCCTAGTATGTTCGAACCAGAGCGTGAAAAAGTG
GATGCCATTGATGGTGAATACCGCTTGAGAGGAGAAGCAAGGAAAACTTTTG
Sequence number 1362: Dengue virus 2 Whole genome #101 
AAAAAGTGGATGCCATTGATGGTGAATACCGCTTGAGAGGAGAAGCAAGGAAAACTTTTGTGGACCTA
ATGAGAAGAGGGGACTTACCAGTCTGGTTGGCCTACAAAGTGGCAGCTGAAG
Sequence number 1363: Dengue virus 2 Whole genome #102 
TGGACCTAATGAGAAGAGGGGACTTACCAGTCTGGTTGGCCTACAAAGTGGCAGCTGAAGGCATCAAC
TACGCAGACAGAAAGTGGTGTTTTGATGGAATCAAGAACAACCAAATACTGG
Sequence number 1364: Dengue virus 2 Whole genome #103 
GCATCAACTACGCAGACAGAAAGTGGTGTTTTGATGGAATCAAGAACAACCAAATACTGGAAGAAAAT
ATAGAAGTGGAAATCTGGACAAAAGAAGGGGAGAGGAAAAAATTAAAACCCA
Sequence number 1365: Dengue virus 2 Whole genome #104 
AAGAAAATATAGAAGTGGAAATCTGGACAAAAGAAGGGGAGAGGAAAAAATTAAAACCCAGATGGTTG
GATGCTAGGATCTACTCTGACCCACTGGCACTAAAAGAATTCAAGGAATTTG
Sequence number 1366: Dengue virus 2 Whole genome #105 
GATGGTTGGATGCTAGGATCTACTCTGACCCACTGGCACTAAAAGAATTCAAGGAATTTGCAGCTGGA
AGAAAATCTCTGACCCTGAACCTAATCACAGAAATGGGTAGGCTTCCAACTT
Sequence number 1367: Dengue virus 2 Whole genome #106 
CAGCTGGAAGAAAATCTCTGACCCTGAACCTAATCACAGAAATGGGTAGGCTTCCAACTTTCATGACT
CAGAAAGCAAGAAACGCACTGGACAACTTGGCTGTGCTGCATACGGCTGAGG
Sequence number 1368: Dengue virus 2 Whole genome #107 
TCATGACTCAGAAAGCAAGAAACGCACTGGACAACTTGGCTGTGCTGCATACGGCTGAGGCAGGTGGA
AGGGCGTACAATCATGCTCTCAGTGAACTGCCGGAGACCCTGGAAACACTGC
Sequence number 1369: Dengue virus 2 Whole genome #108 
CAGGTGGAAGGGCGTACAATCATGCTCTCAGTGAACTGCCGGAGACCCTGGAAACACTGCTTCTACTG
ACACTCCTGGCAACAGTCACAGGAGGAATCTTCTTATTCTTAATGAGCGGAA
Sequence number 1370: Dengue virus 2 Whole genome #109 
TTCTACTGACACTCCTGGCAACAGTCACAGGAGGAATCTTCTTATTCTTAATGAGCGGAAAAGGTGTA
GGGAAGATGACCCTGGGAATGTGTTGCATAATCACGGCTAGTATCCTCCTAT
Sequence number 1371: Dengue virus 2 Whole genome #110 
AAGGTGTAGGGAAGATGACCCTGGGAATGTGTTGCATAATCACGGCTAGTATCCTCCTATGGTATGCA
CAGATACAACCACACTGGATAGCAGCTTCAATAATACTGGAGTTTTTTCTCA
Sequence number 1372: Dengue virus 2 Whole genome #111 
GGTATGCACAGATACAACCACACTGGATAGCAGCTTCAATAATACTGGAGTTTTTTCTCATAGTTTTG
CTCATTCCAGAACCAGAAAAACAGAGAACACCCCAAGACAACCAATTGACCT
Sequence number 1373: Dengue virus 2 Whole genome #112 
TAGTTTTGCTCATTCCAGAACCAGAAAAACAGAGAACACCCCAAGACAACCAATTGACCTACGTTGTC
ATAGCTATTCTCACAGTGGTGGCCGCAACCATGGCAAACGAGATGGGTTTTC
Sequence number 1374: Dengue virus 2 Whole genome #113 
ACGTTGTCATAGCTATTCTCACAGTGGTGGCCGCAACCATGGCAAACGAGATGGGTTTTCTAGAAAAA
ACCAAGAAAGACCTCGGATTGGGAAGCATCACAACCCAGGAACCTGAGAGCA
Sequence number 1375: Dengue virus 2 Whole genome #114 TAGAAAAAACCAAGAAAGACCTCGGATTGGGAAGCATCACAACCCAGGAACCTGAGAGCAATATCCTG GACATAGATCTACGCCCTGCATCAGCATGGACGCTGTATGCCGTGGCTACAA
Sequence number 1376: Dengue virus 2 Whole genome #115 
ATATCCTGGACATAGATCTACGCCCTGCATCAGCATGGACGCTGTATGCCGTGGCTACAACATTTGTC
ACACCAATGTTGAGACATAGCATTGAAAATTCCTCAGTGAATGTCTCCCTAA
Sequence number 1377: Dengue virus 2 Whole genome #116 
CATTTGTCACACCAATGTTGAGACATAGCATTGAAAATTCCTCAGTGAATGTCTCCCTAACAGCCATT
GCTAACCAAGCTACAGTGCTAATGGGTCTTGGGAAAGGATGGCCATTGTCAA
Sequence number 1378: Dengue virus 2 Whole genome #117 
CAGCCATTGCTAACCAAGCTACAGTGCTAATGGGTCTTGGGAAAGGATGGCCATTGTCAAAGATGGAC
ATTGGAGTTCCCCTCCTTGCCATTGGATGCTATTCACAAGTCAACCCTATAA
Sequence number 1379: Dengue virus 2 Whole genome #118 
AGATGGACATTGGAGTTCCCCTCCTTGCCATTGGATGCTATTCACAAGTCAACCCTATAACTCTCACA
GCAGCTCTTCTTTTATTGGTAGCACATTATGCCATTATAGGGCCAGGACTTC
Sequence number 1380: Dengue virus 2 Whole genome #119 
CTCTCACAGCAGCTCTTCTTTTATTGGTAGCACATTATGCCATTATAGGGCCAGGACTTCAAGCAAAA
GCAACCAGAGAAGCTCAGAAAAGAGCAGCAGCAGGCATCATGAAAAACCCAA
Sequence number 1381: Dengue virus 2 Whole genome #120 
AAGCAAAAGCAACCAGAGAAGCTCAGAAAAGAGCAGCAGCAGGCATCATGAAAAACCCAACAGTAGAT
GGAATAACAGTGATTGACCTAGAACCAATACCCTATGATCCAAAATTTGAAA
Sequence number 1382: Dengue virus 2 Whole genome #121 
CAGTAGATGGAATAACAGTGATTGACCTAGAACCAATACCCTATGATCCAAAATTTGAAAAGCAGTTA
GGACAAGTAATGCTCCTAATTCTCTGCGTGACTCAAGTATTAATGATGAGGA
Sequence number 1383: Dengue virus 2 Whole genome #122 
AGCAGTTAGGACAAGTAATGCTCCTAATTCTCTGCGTGACTCAAGTATTAATGATGAGGACTACATGG
GCTCTATGTGAGGCTCTAACCTTAGCGACCGGGCCTATCTCCACACTGTGGG
Sequence number 1384: Dengue virus 2 Whole genome #123 
CTACATGGGCTCTATGTGAGGCTCTAACCTTAGCGACCGGGCCTATCTCCACACTGTGGGAAGGAAAT
CCAGGGAGGTTTTGGAACACTACCATTGCAGTGTCAATGGCTAACATCTTTA
Sequence number 1385: Dengue virus 2 Whole genome #124 
AAGGAAATCCAGGGAGGTTTTGGAACACTACCATTGCAGTGTCAATGGCTAACATCTTTAGGGGGAGC
TACTTGGCCGGAGCTGGACTTCTCTTTTCCATCATGAAAAACACAACAAACA
Sequence number 1386: Dengue virus 2 Whole genome #125 
GGGGGAGCTACTTGGCCGGAGCTGGACTTCTCTTTTCCATCATGAAAAACACAACAAACACAAGAAGA
GGAACTGGCAACATAGGAGAGACACTTGGAGAAAAATGGAAAAGTCGATTAA
Sequence number 1387: Dengue virus 2 Whole genome #126 
CAAGAAGAGGAACTGGCAACATAGGAGAGACACTTGGAGAAAAATGGAAAAGTCGATTAAACGCATTG
GGAAAAAGTGAATTTCAAGTCTACAAGAAAAGTGGAATCCAGGAAGTGGATA
Sequence number 1388: Dengue virus 2 Whole genome #127 
ACGCATTGGGAAAAAGTGAATTTCAAGTCTACAAGAAAAGTGGAATCCAGGAAGTGGATAGAACCCTA
GCAAAAGAAGGTATCAAAAGAGGAGAAACGGACCACCATGCTGTGTCGCGAG
Sequence number 1389: Dengue virus 2 Whole genome #128 
GAACCCTAGCAAAAGAAGGTATCAAAAGAGGAGAAACGGACCACCATGCTGTGTCGCGAGGTTCAGCT
AAACTGAGATGGTTCGTCGAGAGAAATATGGTCACACCGGAAGGGAAGGTGG
Sequence number 1390: Dengue virus 2 Whole genome #129 
GTTCAGCTAAACTGAGATGGTTCGTCGAGAGAAATATGGTCACACCGGAAGGGAAGGTGGTGGATCTC
GGTTGCGGCAGAGGGGGCTGGTCATACTATTGTGGGGGACTAAAGAATGTAA
Sequence number 1391: Dengue virus 2 Whole genome #130 
TGGATCTCGGTTGCGGCAGAGGGGGCTGGTCATACTATTGTGGGGGACTAAAGAATGTAAGAGAAGTC
AAAGGCCTAACAAAAGGAGGACCAGGACATGAAGAACCCATCCCCATGTCAA
Sequence number 1392: Dengue virus 2 Whole genome #131 
GAGAAGTCAAAGGCCTAACAAAAGGAGGACCAGGACATGAAGAACCCATCCCCATGTCAACATATGGG
TGGAATCTAGTGCGTTTGCAAAGTGGAGTTGACGTTTTCTTCACCCCGCCAG
Sequence number 1393: Dengue virus 2 Whole genome #132 
CATATGGGTGGAATCTAGTGCGTTTGCAAAGTGGAGTTGACGTTTTCTTCACCCCGCCAGAAAAGTGT
GATACATTGTTGTGTGACATAGGGGAGTCGTCGCCAAATCCCACGATAGAAG
Sequence number 1394: Dengue virus 2 Whole genome #133 
AAAAGTGTGATACATTGTTGTGTGACATAGGGGAGTCGTCGCCAAATCCCACGATAGAAGCAGGACGA
ACACTCAGAGTCCTCAACTTAGTGGAAAATTGGTTGAATAATAACACCCAAT
Sequence number 1395: Dengue virus 2 Whole genome #134 
CAGGACGAACACTCAGAGTCCTCAACTTAGTGGAAAATTGGTTGAATAATAACACCCAATTTTGCATA
AAGGTCCTCAACCCATATATGCCTTCAGTCATAGAAAAAATGGAAGCATTAC
Sequence number 1396: Dengue virus 2 Whole genome #135 
TTTGCATAAAGGTCCTCAACCCATATATGCCTTCAGTCATAGAAAAAATGGAAGCATTACAAAGGAAA
TATGGAGGAGCCTTAGTGAGGAATCCACTCTCACGAAACTCCACGCATGAAA
Sequence number 1397: Dengue virus 2 Whole genome #136 
AAAGGAAATATGGAGGAGCCTTAGTGAGGAATCCACTCTCACGAAACTCCACGCATGAAATGTACTGG
GTATCTAATGCCACCGGGAACATAGTGTCATCAGTGAACATGATTTCAAGGA
Sequence number 1398: Dengue virus 2 Whole genome #137 
TGTACTGGGTATCTAATGCCACCGGGAACATAGTGTCATCAGTGAACATGATTTCAAGGATGTTGATT
AACAGATTCACAATGAAACACAAGAAAGCCACTTACGAGCCAGATGTTGACC
Sequence number 1399: Dengue virus 2 Whole genome #138 
TGTTGATTAACAGATTCACAATGAAACACAAGAAAGCCACTTACGAGCCAGATGTTGACCTAGGAAGT
GGAACCCGCAACATTGGAGTTGAAAGTGAGATACCAAATCTAGACATAATAG
Sequence number 1400: Dengue virus 2 Whole genome #139 
TAGGAAGTGGAACCCGCAACATTGGAGTTGAAAGTGAGATACCAAATCTAGACATAATAGGAAAGAGA
ATAGAGAAAATAAAACAAGAGCATGAAACATCATGGCACTATGATCAAGATC
Sequence number 1401: Dengue virus 2 Whole genome #140 
GAAAGAGAATAGAGAAAATAAAACAAGAGCATGAAACATCATGGCACTATGATCAAGATCACCCATAC
AAAACGTGGGCTTACCACGGCAGCTATGAAACAAAACAAACTGGATCAGCAT
Sequence number 1402: Dengue virus 2 Whole genome #141 
ACCCATACAAAACGTGGGCTTACCACGGCAGCTATGAAACAAAACAAACTGGATCAGCATCATCTATG
GTGAACGGAGTGGTCAGATTGCTGACAAAACCTTGGGACGTCGTCCCTATGG
Sequence number 1403: Dengue virus 2 Whole genome #142 CATCTATGGTGAACGGAGTGGTCAGATTGCTGACAAAACCTTGGGACGTCGTCCCTATGGTGACACAG ATGGCAATGACGGACACGACTCCATTTGGACAACAGCGCGTTTTCAAAGAGA
Sequence number 1404: Dengue virus 2 Whole genome #143 
TGACACAGATGGCAATGACGGACACGACTCCATTTGGACAACAGCGCGTTTTCAAAGAGAAAGTAGAC
ACGAGAACCCAGGAACCGAAGGAAGGCACAAAGAAACTGATGAAAATTACGG
Sequence number 1405: Dengue virus 2 Whole genome #144 
AAGTAGACACGAGAACCCAGGAACCGAAGGAAGGCACAAAGAAACTGATGAAAATTACGGCAGAGTGG
CTTTGGAAAGAACTAGGAAAGAAAAAGACACCTAGGATGTGTACCAGAGAAG
Sequence number 1406: Dengue virus 2 Whole genome #145 
CAGAGTGGCTTTGGAAAGAACTAGGAAAGAAAAAGACACCTAGGATGTGTACCAGAGAAGAATTCACA
AGAAAAGTGAGAAGCAATGCAGCCTTGGGGGCCATATTCACTGATGAGAACA
Sequence number 1407: Dengue virus 2 Whole genome #146 
AATTCACAAGAAAAGTGAGAAGCAATGCAGCCTTGGGGGCCATATTCACTGATGAGAACAAATGGAAA
TCGGCACGTGAGGCTGTTGAAGATGGTAGGTTTTGGGAGCTGGTTGACAGGG
Sequence number 1408: Dengue virus 2 Whole genome #147 
AATGGAAATCGGCACGTGAGGCTGTTGAAGATGGTAGGTTTTGGGAGCTGGTTGACAGGGAAAGAAAT
CTCCATCTTGAAGGGAAGTGTGAAACATGTGTGTACAACATGATGGGAAAAA
Sequence number 1409: Dengue virus 2 Whole genome #148 
AAAGAAATCTCCATCTTGAAGGGAAGTGTGAAACATGTGTGTACAACATGATGGGAAAAAGAGAGAAG
AAACTAGGGGAGTTCGGCAAGGCAAAAGGTAGCAGAGCCATATGGTACATGT
Sequence number 1410: Dengue virus 2 Whole genome #149 
GAGAGAAGAAACTAGGGGAGTTCGGCAAGGCAAAAGGTAGCAGAGCCATATGGTACATGTGGCTTGGA
GCACGCTTCTTAGAGTTTGAAGCCCTAGGATTCCTGAATGAAGATCACTGGT
Sequence number 1411: Dengue virus 2 Whole genome #150 
GGCTTGGAGCACGCTTCTTAGAGTTTGAAGCCCTAGGATTCCTGAATGAAGATCACTGGTTCTCCAGA
GGGAACTCCCTGAGTGGAGTGGAAGGAGAAGGGCTGCACAGACTAGGCTATA
Sequence number 1412: Dengue virus 2 Whole genome #151 
TCTCCAGAGGGAACTCCCTGAGTGGAGTGGAAGGAGAAGGGCTGCACAGACTAGGCTATATTTTAAGA
GACGTGGGCAAGAAGGAAGGGGGAGCAATGTACGCCGATGATACAGCAGGAT
Sequence number 1413: Dengue virus 2 Whole genome #152 
TTTTAAGAGACGTGGGCAAGAAGGAAGGGGGAGCAATGTACGCCGATGATACAGCAGGATGGGACACA
AGAATCACACTAGAAGACTTAAAAAATGAAGAAATGGTAACAAACCACATGA
Sequence number 1414: Dengue virus 2 Whole genome #153 
GGGACACAAGAATCACACTAGAAGACTTAAAAAATGAAGAAATGGTAACAAACCACATGAAAGGAGAA
CACAAGAAACTAGCCGAGGCCATATTCAAACTAACGTACCAAAACAAGGTGG
Sequence number 1415: Dengue virus 2 Whole genome #154 
AAGGAGAACACAAGAAACTAGCCGAGGCCATATTCAAACTAACGTACCAAAACAAGGTGGTGCGTGTG
CAAAGACCAACGCCAAGAGGCACAGTAATGGATATCATATCGAGAAGAGACC
Sequence number 1416: Dengue virus 2 Whole genome #155 
TGCGTGTGCAAAGACCAACGCCAAGAGGCACAGTAATGGATATCATATCGAGAAGAGACCAAAGAGGC
AGTGGGCAAGTCGGCACCTATGGCCTTAATACTTTCACCAATATGGAAGCCC
Sequence number 1417: Dengue virus 2 Whole genome #156 
AAAGAGGCAGTGGGCAAGTCGGCACCTATGGCCTTAATACTTTCACCAATATGGAAGCCCAATTAATT
AGACAGATGGAGGGAGAAGGAATCTTCAAAAGCATTCAGCACCTGACAGCCA
Sequence number 1418: Dengue virus 2 Whole genome #157 
AATTAATTAGACAGATGGAGGGAGAAGGAATCTTCAAAAGCATTCAGCACCTGACAGCCACAGAAGAA
ATCGCTGTACAGAACTGGTTAGCAAGAGTGGGGCGTGAAAGGCTATCGAGAA
Sequence number 1419: Dengue virus 2 Whole genome #158 
CAGAAGAAATCGCTGTACAGAACTGGTTAGCAAGAGTGGGGCGTGAAAGGCTATCGAGAATGGCAATC
AGTGGGGATGATTGTGTTGTAAAGCCTATAGATGATAGATTTGCAAGTGCTT
Sequence number 1420: Dengue virus 2 Whole genome #159 
TGGCAATCAGTGGGGATGATTGTGTTGTAAAGCCTATAGATGATAGATTTGCAAGTGCTTTAACAGCT
CTAAATGACATGGGAAAAGTTAGAAAAGATATACAACAATGGGAACCTTCAA
Sequence number 1421: Dengue virus 2 Whole genome #160 
TAACAGCTCTAAATGACATGGGAAAAGTTAGAAAAGATATACAACAATGGGAACCTTCAAGAGGATGG
AACGATTGGACACAAGTGCCTTTCTGTTCACACCATTTTCATGAGTTAGTCA
Sequence number 1422: Dengue virus 2 Whole genome #161 
GAGGATGGAACGATTGGACACAAGTGCCTTTCTGTTCACACCATTTTCATGAGTTAGTCATGAAAGAT
GGTCGCGTGCTCGTAGTCCCATGCAGAAACCAAGATGAACTGATTGGTAGAG
Sequence number 1423: Dengue virus 2 Whole genome #162 
TGAAAGATGGTCGCGTGCTCGTAGTCCCATGCAGAAACCAAGATGAACTGATTGGTAGAGCCCGAATT
TCCCAGGGAGCTGGGTGGTCTTTGAAGGAGACGGCCTGTTTGGGGAAGTCTT
Sequence number 1424: Dengue virus 2 Whole genome #163 
CCCGAATTTCCCAGGGAGCTGGGTGGTCTTTGAAGGAGACGGCCTGTTTGGGGAAGTCTTACGCCCAA
ATGTGGACCCTGATGTACTTCCACAGACGTGACCTCAGACTGGCGGCAAATG
Sequence number 1425: Dengue virus 2 Whole genome #164 
ACGCCCAAATGTGGACCCTGATGTACTTCCACAGACGTGACCTCAGACTGGCGGCAAATGCCATTTGC
TCGGCAGTCCCGCCACATTGGGTTCCAACAAGTCGAACAACCTGGTCCATAC
Sequence number 1426: Dengue virus 2 Whole genome #165 
CCATTTGCTCGGCAGTCCCGCCACATTGGGTTCCAACAAGTCGAACAACCTGGTCCATACACGCCAAG
CATGAATGGATGACGACGGAAGACATGCTGGCAGTCTGGAACAGGGTGTGGA
Sequence number 1427: Dengue virus 2 Whole genome #166 
ACGCCAAGCATGAATGGATGACGACGGAAGACATGCTGGCAGTCTGGAACAGGGTGTGGATCCAAGAA
AACCCGTGGATGGAAGATAAAACTCCAGTGGAATCATGGGAAGAAGTCCCAT
Sequence number 1428: Dengue virus 2 Whole genome #167 
TCCAAGAAAACCCGTGGATGGAAGATAAAACTCCAGTGGAATCATGGGAAGAAGTCCCATACTTGGGA
AAAAGAGAAGACCAATGGTGCGGCTCATTGATTGGGCTAACAAGCAGGGCTA
Sequence number 1429: Dengue virus 2 Whole genome #168 
ACTTGGGAAAAAGAGAAGACCAATGGTGCGGCTCATTGATTGGGCTAACAAGCAGGGCTACCTGGGCA
AAGAACATCCAAACAGCAATAAATCAAGTCAGATCCCTTATAGGCAATGAGG
Sequence number 1430: Dengue virus 2 Whole genome #169 
CCTGGGCAAAGAACATCCAAACAGCAATAAATCAAGTCAGATCCCTTATAGGCAATGAGGAATACACA
GACTACATGCCATCCATGAAGAGATTCAGAAGGGAAGAGGAAGAGGCAGGTG
Sequence number 1431: Dengue virus 2 Whole genome #170 AATACACAGACTACATGCCATCCATGAAGAGATTCAGAAGGGAAGAGGAAGAGGCAGGTGTCCTGTGG TAAAAGGCAAAACTAACATGAAACAAGGCTAAAAGTCAGGTCGGATTAAGCC
Sequence number 1432: Dengue virus 2 Whole genome #171 
TCCTGTGGTAAAAGGCAAAACTAACATGAAACAAGGCTAAAAGTCAGGTCGGATTAAGCCATAGTACG
GGAAAAACTATGCTACCTGTGAGCCCCGTCCAAGGACGTTAAAAGAAGTCAG
Sequence number 1433: Dengue virus 2 Whole genome #172 
ATAGTACGGGAAAAACTATGCTACCTGTGAGCCCCGTCCAAGGACGTTAAAAGAAGTCAGGCCATCAC
AAAATGCCACAGCTTGAGTAAACTGTGCAGCCTGTAGCTCCACCTGAGGAGG
Sequence number 1434: Dengue virus 2 Whole genome #173 
GCCATCACAAAATGCCACAGCTTGAGTAAACTGTGCAGCCTGTAGCTCCACCTGAGGAGGTGTAAAAA
ACCTGGGAGGCCACAAACCATGGAAGCTGTACGCATGGTGTAGTGGACTAGC
Sequence number 1435: Dengue virus 2 Whole genome #174 
TGTAAAAAACCTGGGAGGCCACAAACCATGGAAGCTGTACGCATGGTGTAGTGGACTAGCGGTTAGAG
GAGACCCCTCCCTTACAAATCGCAGCAAACAACGGGGGCCCAAGGTGAGATG
Sequence number 1436: Dengue virus 2 Whole genome #175 
GGTTAGAGGAGACCCCTCCCTTACAAATCGCAGCAAACAACGGGGGCCCAAGGTGAGATGAAGCTGTA
ATCTCACCGGAAGGACTAGAGGTTAGAGGAGACCCCCCCAAAACAAAAAACA
Sequence number 1437: Dengue virus 2 Whole genome #176 
ATGAAGCTGTAATCTCACCGGAAGGACTAGAGGTTAGAGGAGACCCCCCCAAAACAAAAAACAGCATA
TTGACGCTGGGAAAGACCAGAGATCCTGCTGTCTCCTCAGCATCATTCCAGG
Sequence number 1438: Dengue virus 3 Whole genome #1 
AGTTGTTAGTCTACGTGGACCGACAAGAACAGTTTCGACTCGGAAGCTTGCTTAACGTAGTGCTAACA
GTTTTTTATTAGAGAGCAGATCTCTGATGAACAACCAACGGAAGAAGACGGG
Sequence number 1439: Dengue virus 3 Whole genome #2 
TGCTAACAGTTTTTTATTAGAGAGCAGATCTCTGATGAACAACCAACGGAAGAAGACGGGAAAACCGT
CTATCAATATGCTGAAACGCGTGAGAAACCGTGTGTCAACTGGATCACAGTT
Sequence number 1440: Dengue virus 3 Whole genome #3 
AAAACCGTCTATCAATATGCTGAAACGCGTGAGAAACCGTGTGTCAACTGGATCACAGTTGGCGAAGA
GATTCTCAAAAGGATTGCTGAACGGCCAGGGACCAATGAAATTGGTCATGGC
Sequence number 1441: Dengue virus 3 Whole genome #4 
GGCGAAGAGATTCTCAAAAGGATTGCTGAACGGCCAGGGACCAATGAAATTGGTCATGGCGTTCATAG
CCTTCCTTAGATTTCTGGCCATTCCACCAACAGCAGGAGTTTTGGCCAGATG
Sequence number 1442: Dengue virus 3 Whole genome #5 
GTTCATAGCCTTCCTTAGATTTCTGGCCATTCCACCAACAGCAGGAGTTTTGGCCAGATGGGGAACCT
TCAAGAAGTCGGGTGCCATTAAGGTTCTGAAAGGCTTCAAGAAGGAGATTTC
Sequence number 1443: Dengue virus 3 Whole genome #6 
GGGAACCTTCAAGAAGTCGGGTGCCATTAAGGTTCTGAAAGGCTTCAAGAAGGAGATTTCAAACATGC
TGAGCATAATCAACAAACGGAAAAAGACATCGCTCTGTCTCATGATGATATT
Sequence number 1444: Dengue virus 3 Whole genome #7 
AAACATGCTGAGCATAATCAACAAACGGAAAAAGACATCGCTCTGTCTCATGATGATATTGCCAGCAG
CACTTGCTTTCCACTTGACTTCACGAGATGGAGAGCCGCGGATGATTGTAGG
Sequence number 1445: Dengue virus 3 Whole genome #8 
GCCAGCAGCACTTGCTTTCCACTTGACTTCACGAGATGGAGAGCCGCGGATGATTGTAGGGAAGAATG
AAAGAGGGAAATCCCTACTTTTTAAGACAGCCTCTGGAATCAACATGTGCAC
Sequence number 1446: Dengue virus 3 Whole genome #9 
GAAGAATGAAAGAGGGAAATCCCTACTTTTTAAGACAGCCTCTGGAATCAACATGTGCACACTCATAG
CCATGGACTTGGGAGAAATGTGTGATGACACGGTCACTTACAAATGCCCCCA
Sequence number 1447: Dengue virus 3 Whole genome #10 
ACTCATAGCCATGGACTTGGGAGAAATGTGTGATGACACGGTCACTTACAAATGCCCCCACATTACCG
AAGTGGAACCTGAAGACATTGACTGCTGGTGCAACCTTACATCAACATGGGT
Sequence number 1448: Dengue virus 3 Whole genome #11 
CATTACCGAAGTGGAACCTGAAGACATTGACTGCTGGTGCAACCTTACATCAACATGGGTGACTTATG
GAACGTGTAACCAAGCTGGAGAGCATAGACGCGACAAGAGATCAGTGGCGTT
Sequence number 1449: Dengue virus 3 Whole genome #12 
GACTTATGGAACGTGTAACCAAGCTGGAGAGCATAGACGCGACAAGAGATCAGTGGCGTTAGCTCCCC
ATGTCGGCATGGGACTGGACACACGCACCCAAACCTGGATGTCGGCTGAAGG
Sequence number 1450: Dengue virus 3 Whole genome #13 
AGCTCCCCATGTCGGCATGGGACTGGACACACGCACCCAAACCTGGATGTCGGCTGAAGGAGCTTGGA
GACAAGTCGAGAAGGTAGAGACATGGGCCCTTAGGCATCCAGGGTTCACCAT
Sequence number 1451: Dengue virus 3 Whole genome #14 
AGCTTGGAGACAAGTCGAGAAGGTAGAGACATGGGCCCTTAGGCATCCAGGGTTCACCATACTAGCCC
TATTTCTTGCCCATTACATAGGCACCTCCTTGACCCAGAAGGTGGTTATTTT
Sequence number 1452: Dengue virus 3 Whole genome #15 
ACTAGCCCTATTTCTTGCCCATTACATAGGCACCTCCTTGACCCAGAAGGTGGTTATTTTTACACTAC
TAATGCTGGTCACCCCATCCATGACAATGAGATGTGTGGGAGTGGGAAATAG
Sequence number 1453: Dengue virus 3 Whole genome #16 
TACACTACTAATGCTGGTCACCCCATCCATGACAATGAGATGTGTGGGAGTGGGAAATAGAGATTTTG
TGGAAGGTCTGTCAGGAGCTACGTGGGTTGACGTGGTGCTAGAGCACGGGGG
Sequence number 1454: Dengue virus 3 Whole genome #17 
AGATTTTGTGGAAGGTCTGTCAGGAGCTACGTGGGTTGACGTGGTGCTAGAGCACGGGGGGTGTGTGA
CCACCATGGCTAAGAACAAGCCCACGCTGGATATAGAGCTTCAGAAGACCGA
Sequence number 1455: Dengue virus 3 Whole genome #18 
GTGTGTGACCACCATGGCTAAGAACAAGCCCACGCTGGATATAGAGCTTCAGAAGACCGAGGCCACCC
AACTGGCGACCCTAAGGAAGCTATGCATTGAGGGgAAAATTACCAACATAAC
Sequence number 1456: Dengue virus 3 Whole genome #19 
GGCCACCCAACTGGCGACCCTAAGGAAGCTATGCATTGAGGGgAAAATTACCAACATAACAACTGACT
CAAGATGTCCTACCCAAGGGGAAGCGGTTTTGCCTGAGGAGCAGGACCAGAA
Sequence number 1457: Dengue virus 3 Whole genome #20 
AACTGACTCAAGATGTCCTACCCAAGGGGAAGCGGTTTTGCCTGAGGAGCAGGACCAGAACTACGTGT
GTAAGCACACATACGTAGATAGAGGCTGGGGGAACGGTTGTGGTTTGTTTGG
Sequence number 1458: Dengue virus 3 Whole genome #21 
CTACGTGTGTAAGCACACATACGTAGATAGAGGCTGGGGGAACGGTTGTGGTTTGTTTGGCAAGGGAA
GCTTGGTAACATGTGCGAAATTTCAATGCCTGGAACCGATAGAGGGAAAAGT
Sequence number 1459: Dengue virus 3 Whole genome #22 CAAGGGAAGCTTGGTAACATGTGCGAAATTTCAATGCCTGGAACCGATAGAGGGAAAAGTAGTGCAAT ATGAGAACCTCAAATACACCGTCATCATTACAGTGCACACAGGAGACCAACA
Sequence number 1460: Dengue virus 3 Whole genome #23 
AGTGCAATATGAGAACCTCAAATACACCGTCATCATTACAGTGCACACAGGAGACCAACACCAGGTGG
GAAATGAAACGCAGGGAATCACGGCTGAGATAACGCCTCAGGCATCAACTAC
Sequence number 1461: Dengue virus 3 Whole genome #24 
CCAGGTGGGAAATGAAACGCAGGGAATCACGGCTGAGATAACGCCTCAGGCATCAACTACTGAAGCCA
TCTTGCCTGAATATGGAACCCTTGGGCTAGAATGCTCACCACGGACAGGTTT
Sequence number 1462: Dengue virus 3 Whole genome #25 
TGAAGCCATCTTGCCTGAATATGGAACCCTTGGGCTAGAATGCTCACCACGGACAGGTTTGGATTTCA
ATGAAATGATCTTACTAACAATGAAGAACAAAGCATGGATGGTACACAGACA
Sequence number 1463: Dengue virus 3 Whole genome #26 
GGATTTCAATGAAATGATCTTACTAACAATGAAGAACAAAGCATGGATGGTACACAGACAATGGTTTT
TTGATCTACCTCTACCATGGACATCAGGAGCCACGACAGAAACACCAACTTG
Sequence number 1464: Dengue virus 3 Whole genome #27 
ATGGTTTTTTGATCTACCTCTACCATGGACATCAGGAGCCACGACAGAAACACCAACTTGGAACAGGA
AGGAGCTCCTTGTGACATTCAAAAATGCACATGCGAAAAAACAAGAAGTAGT
Sequence number 1465: Dengue virus 3 Whole genome #28 
GAACAGGAAGGAGCTCCTTGTGACATTCAAAAATGCACATGCGAAAAAACAAGAAGTAGTTGTCCTTG
GATCGCAAGAGGGAGCAATGCATACCGCACTGACAGGAGCCACAGAAATCCA
Sequence number 1466: Dengue virus 3 Whole genome #29 
TGTCCTTGGATCGCAAGAGGGAGCAATGCATACCGCACTGACAGGAGCCACAGAAATCCAAAACTCAG
GAGGCACAAGCATTTTTGCGGGGCACTTAAAGTGTAGACTTAAGATGGACAA
Sequence number 1467: Dengue virus 3 Whole genome #30 
AAACTCAGGAGGCACAAGCATTTTTGCGGGGCACTTAAAGTGTAGACTTAAGATGGACAAATTGGAAC
TCAAGGGGATGAGCTATGCAATGTGCACGAACACCTTTGTGTTGAAGAAAGA
Sequence number 1468: Dengue virus 3 Whole genome #31 
ATTGGAACTCAAGGGGATGAGCTATGCAATGTGCACGAACACCTTTGTGTTGAAGAAAGAAGTCTCAG
AAACGCAGCATGGGACAATACTCATTAAGGTCGAGTACAAAGGGGAAGACGC
Sequence number 1469: Dengue virus 3 Whole genome #32 
AGTCTCAGAAACGCAGCATGGGACAATACTCATTAAGGTCGAGTACAAAGGGGAAGACGCACCTTGCA
AGATTCCTTTCTCCACAGAGGATGGACAAGGGAAAGCTCACAATGGCAGACT
Sequence number 1470: Dengue virus 3 Whole genome #33 
ACCTTGCAAGATTCCTTTCTCCACAGAGGATGGACAAGGGAAAGCTCACAATGGCAGACTGATCACAG
CCAACCCAGTGGTGACTAAGAAGGAGGAGCCTGTCAACATTGAGGCTGAACC
Sequence number 1471: Dengue virus 3 Whole genome #34 
GATCACAGCCAACCCAGTGGTGACTAAGAAGGAGGAGCCTGTCAACATTGAGGCTGAACCTCCTTTTG
GGGAAAGTAATATAGTAATTGGAACTGGAGACAATGCCTTGAAAATCAATTG
Sequence number 1472: Dengue virus 3 Whole genome #35 
TCCTTTTGGGGAAAGTAATATAGTAATTGGAACTGGAGACAATGCCTTGAAAATCAATTGGTACAAGA
AGGGAAGCTCTATTGGGAAGATGTTCGAGGCCACTGCCAGAGGTGCAAGGCG
Sequence number 1473: Dengue virus 3 Whole genome #36 
GTACAAGAAGGGAAGCTCTATTGGGAAGATGTTCGAGGCCACTGCCAGAGGTGCAAGGCGCATGGCCA
TCTTGGGAGACACAGCTTGGGACTTTGGATCAGTGGGTGGTGTTCTGAACTC
Sequence number 1474: Dengue virus 3 Whole genome #37 
CATGGCCATCTTGGGAGACACAGCTTGGGACTTTGGATCAGTGGGTGGTGTTCTGAACTCATTAGGCA
AAATGGTGCACCAAATATTCGGAAGTGCTTACACAGCCCTATTCAGTGGAGT
Sequence number 1475: Dengue virus 3 Whole genome #38 
ATTAGGCAAAATGGTGCACCAAATATTCGGAAGTGCTTACACAGCCCTATTCAGTGGAGTCTCTTGGG
TGATGAAAATTGGAATAGGTGTTCTCTTAACTTGGATAGGGTTGAATTCAAA
Sequence number 1476: Dengue virus 3 Whole genome #39 
CTCTTGGGTGATGAAAATTGGAATAGGTGTTCTCTTAACTTGGATAGGGTTGAATTCAAAAAACACAT
CCATGTCATTTTCATGCATTGCAATTGGAATCATCACACTCTATTTGGGAGC
Sequence number 1477: Dengue virus 3 Whole genome #40 
AAACACATCCATGTCATTTTCATGCATTGCAATTGGAATCATCACACTCTATTTGGGAGCTGTAGTAC
AAGCTGACATGGGGTGTGTCATAAACTGGAAAGGCAAAGAACTCAAATGCGG
Sequence number 1478: Dengue virus 3 Whole genome #41 
TGTAGTACAAGCTGACATGGGGTGTGTCATAAACTGGAAAGGCAAAGAACTCAAATGCGGAAGTGGAA
TTTTCGTCACCAACGAGGTCCATACCTGGACAGAGCAGTACAAATTCCAAGC
Sequence number 1479: Dengue virus 3 Whole genome #42 
AAGTGGAATTTTCGTCACCAACGAGGTCCATACCTGGACAGAGCAGTACAAATTCCAAGCGGATTCCC
CAAAAAGATTGGCGACAGCCATTGCAGGCGCTTGGGAAAATGGAGTGTGTGG
Sequence number 1480: Dengue virus 3 Whole genome #43 
GGATTCCCCAAAAAGATTGGCGACAGCCATTGCAGGCGCTTGGGAAAATGGAGTGTGTGGAATTAGGT
CAACAACCAGAATGGAGAATCTCTTGTGGAAGCAAGTAGCCAATGAACTGAA
Sequence number 1481: Dengue virus 3 Whole genome #44 
AATTAGGTCAACAACCAGAATGGAGAATCTCTTGTGGAAGCAAGTAGCCAATGAACTGAACTACATAT
TATGGGAAAACAACATCAAATTAACGGTAGTTGTGGGCGACACAATCGGGGT
Sequence number 1482: Dengue virus 3 Whole genome #45 
CTACATATTATGGGAAAACAACATCAAATTAACGGTAGTTGTGGGCGACACAATCGGGGTTTTAGAGC
AAGGAAAAAGAACACTAACACCACAACCCATGGAGCTAAAATACTCATGGAA
Sequence number 1483: Dengue virus 3 Whole genome #46 
TTTAGAGCAAGGAAAAAGAACACTAACACCACAACCCATGGAGCTAAAATACTCATGGAAAACGTGGG
GAAAGGCAAAAATAGTGACAGCAGAAACACAAAATTCCTCTTTCATAATAGA
Sequence number 1484: Dengue virus 3 Whole genome #47 
AACGTGGGGAAAGGCAAAAATAGTGACAGCAGAAACACAAAATTCCTCTTTCATAATAGACGGGCCAA
ACACACCGGAGTGTCCAAGTGCCTCAAGAGCATGGAATGTGTGGGAGGTAGA
Sequence number 1485: Dengue virus 3 Whole genome #48 
CGGGCCAAACACACCGGAGTGTCCAAGTGCCTCAAGAGCATGGAATGTGTGGGAGGTAGAAGATTACG
GATTCGGAGTCTTCACAACCAACATATGGCTGAAACTCCGAGAGGTGTACAC
Sequence number 1486: Dengue virus 3 Whole genome #49 
AGATTACGGATTCGGAGTCTTCACAACCAACATATGGCTGAAACTCCGAGAGGTGTACACCCAAATGT
GTGACCATAGGCTAATGTCGGCAGCCGTCAAGGATGAGAGGGCCGTGCATGC
Sequence number 1487: Dengue virus 3 Whole genome #50 CCAAATGTGTGACCATAGGCTAATGTCGGCAGCCGTCAAGGATGAGAGGGCCGTGCATGCCGACATGG GCTACTGGATAGAAAGCCAAAAGAATGGAAGTTGGAAGCTAGAAAAAGCATC
Sequence number 1488: Dengue virus 3 Whole genome #51 
CGACATGGGCTACTGGATAGAAAGCCAAAAGAATGGAAGTTGGAAGCTAGAAAAAGCATCCCTCATAG
AGGTGAAAACCTGCACATGGCCAAAATCACACACTCTTTGGAGCAACGGTGT
Sequence number 1489: Dengue virus 3 Whole genome #52 
CCTCATAGAGGTGAAAACCTGCACATGGCCAAAATCACACACTCTTTGGAGCAACGGTGTGCTAGAGA
GTGACATGATCATTCCAAAGAGTCTAGCTGGTCCTATCTCGCAACACAACTA
Sequence number 1490: Dengue virus 3 Whole genome #53 
GCTAGAGAGTGACATGATCATTCCAAAGAGTCTAGCTGGTCCTATCTCGCAACACAACTACAGGCCCG
GGTACCACACCCAAACGGCAGGACCCTGGCACTTAGGAAAATTGGAGCTGGA
Sequence number 1491: Dengue virus 3 Whole genome #54 
CAGGCCCGGGTACCACACCCAAACGGCAGGACCCTGGCACTTAGGAAAATTGGAGCTGGACTTCAACT
ATTGTGAAGGAACAACAGTTGTCATCACAGAAAATTGTGGGACAAGAGGCCC
Sequence number 1492: Dengue virus 3 Whole genome #55 
CTTCAACTATTGTGAAGGAACAACAGTTGTCATCACAGAAAATTGTGGGACAAGAGGCCCATCATTGA
GAACGACAACAGTGTCAGGAAAGTTGATACATGAATGGTGTTGCCGCTCGTG
Sequence number 1493: Dengue virus 3 Whole genome #56 
ATCATTGAGAACGACAACAGTGTCAGGAAAGTTGATACATGAATGGTGTTGCCGCTCGTGCACACTTC
CTCCCCTGCGATACATGGGAGAAGACGGCTGTTGGTATGGcATGGAAATCAG
Sequence number 1494: Dengue virus 3 Whole genome #57 
CACACTTCCTCCCCTGCGATACATGGGAGAAGACGGCTGTTGGTATGGcATGGAAATCAGGCCCATCA
GTGAGAAAGAAGAGAACATGGTAAAGTCTCTAGTCTCAGCGGGAAGTGGAAA
Sequence number 1495: Dengue virus 3 Whole genome #58 
GCCCATCAGTGAGAAAGAAGAGAACATGGTAAAGTCTCTAGTCTCAGCGGGAAGTGGAAAGGTAGACA
ACTTCACAATGGGCGTCTTGTGTTTGGCAATCCTCTTTGAAGAGGTGATGAG
Sequence number 1496: Dengue virus 3 Whole genome #59 
GGTAGACAACTTCACAATGGGCGTCTTGTGTTTGGCAATCCTCTTTGAAGAGGTGATGAGAGGAAAAT
TTGGGAAGAAACACATGATTGCAGGGGTTTTCCTCACGTTTGTGCTCCTTCT
Sequence number 1497: Dengue virus 3 Whole genome #60 
AGGAAAATTTGGGAAGAAACACATGATTGCAGGGGTTTTCCTCACGTTTGTGCTCCTTCTCTCAGGGC
AAATAACATGGAGAGACATGGCGCACACACTAATAATGATTGGGTCCAACGC
Sequence number 1498: Dengue virus 3 Whole genome #61 
CTCAGGGCAAATAACATGGAGAGACATGGCGCACACACTAATAATGATTGGGTCCAACGCCTCTGACA
GGATGGGAATGGGCGTCACCTATCTAGCTTTAATTGCAACATTTAAAATCCA
Sequence number 1499: Dengue virus 3 Whole genome #62 
CTCTGACAGGATGGGAATGGGCGTCACCTATCTAGCTTTAATTGCAACATTTAAAATCCAGCCATTCC
TGGCTTTGGGATTTTTCCTAAGAAAACTGACATCTAGAGAAAATTTACTGTT
Sequence number 1500: Dengue virus 3 Whole genome #63 
GCCATTCCTGGCTTTGGGATTTTTCCTAAGAAAACTGACATCTAGAGAAAATTTACTGTTAGGAGTTG
GACTGGCTATGGCAACAACGTTACAATTGCCAGAGGACATTGAACAAATGGC
Sequence number 1501: Dengue virus 3 Whole genome #64 
AGGAGTTGGACTGGCTATGGCAACAACGTTACAATTGCCAGAGGACATTGAACAAATGGCAAATGGAA
TCGCCCTGGGGCTCATGGCTCTTAAATTGATAACACAATTTGAAACATACCA
Sequence number 1502: Dengue virus 3 Whole genome #65 
AAATGGAATCGCCCTGGGGCTCATGGCTCTTAAATTGATAACACAATTTGAAACATACCAATTATGGA
CAGCATTAATCTCCTTAACGTGTTCAAATACAATCTTCACGTTGACTGTTGC
Sequence number 1503: Dengue virus 3 Whole genome #66 
ATTATGGACAGCATTAATCTCCTTAACGTGTTCAAATACAATCTTCACGTTGACTGTTGCTTGGAGAA
CAGCCACCCTGATTTTGGCTGGAGTTTCACTTTTACCAGTGTGCCAGTCTTC
Sequence number 1504: Dengue virus 3 Whole genome #67 
TTGGAGAACAGCCACCCTGATTTTGGCTGGAGTTTCACTTTTACCAGTGTGCCAGTCTTCGAGCATGA
GAAAAACAGACTGGCTTCCAATGACAGTGGCAGCTATGGGAGTCCCACCCCT
Sequence number 1505: Dengue virus 3 Whole genome #68 
GAGCATGAGAAAAACAGACTGGCTTCCAATGACAGTGGCAGCTATGGGAGTCCCACCCCTACCACTCT
TTATTTTTAGCTTGAAAGACACACTCAAAAGGAGAAGCTGGCCACTGAATGA
Sequence number 1506: Dengue virus 3 Whole genome #69 
ACCACTCTTTATTTTTAGCTTGAAAGACACACTCAAAAGGAGAAGCTGGCCACTGAATGAAGGGGTGA
TGGCTGTTGGACTTGTGAGCATTCTGGCCAGTTCTCTCCTTAGGAATGACGT
Sequence number 1507: Dengue virus 3 Whole genome #70 
AGGGGTGATGGCTGTTGGACTTGTGAGCATTCTGGCCAGTTCTCTCCTTAGGAATGACGTGCCCATGG
CTGGACCATTAGTGGCCGGGGGCTTGCTGATAGCGTGCTACGTCATAACTGG
Sequence number 1508: Dengue virus 3 Whole genome #71 
GCCCATGGCTGGACCATTAGTGGCCGGGGGCTTGCTGATAGCGTGCTACGTCATAACTGGCACGTCAG
CAGACCTTACAGTAGAAAAAGCAGCAGATGTAACATGGGAGGAAGAGGCTGA
Sequence number 1509: Dengue virus 3 Whole genome #72 
CACGTCAGCAGACCTTACAGTAGAAAAAGCAGCAGATGTAACATGGGAGGAAGAGGCTGAGCAAACAG
GAGTGTCCCACAACTTAATGATCACAGTTGATGATGATGGAACAATGAGAAT
Sequence number 1510: Dengue virus 3 Whole genome #73 
GCAAACAGGAGTGTCCCACAACTTAATGATCACAGTTGATGATGATGGAACAATGAGAATAAAAGATG
ATGAGACTGAGAATATTCTAACAGTGCTTTTGAAGACAGCATTATTAATAGT
Sequence number 1511: Dengue virus 3 Whole genome #74 
AAAAGATGATGAGACTGAGAATATTCTAACAGTGCTTTTGAAGACAGCATTATTAATAGTATCAGGTA
TCTTTCCATACTCCATACCTGCAACATTGTTGGTCTGGCATACTTGGCAAAA
Sequence number 1512: Dengue virus 3 Whole genome #75 
ATCAGGTATCTTTCCATACTCCATACCTGCAACATTGTTGGTCTGGCATACTTGGCAAAAGCAAACCC
AAAGATCCGGTGTTCTATGGGACGTACCCAGCCCCCCAGAGACACAGAAAGC
Sequence number 1513: Dengue virus 3 Whole genome #76 
GCAAACCCAAAGATCCGGTGTTCTATGGGACGTACCCAGCCCCCCAGAGACACAGAAAGCAGAACTGG
AAGAAGGGGTCTATAGGATCAAACAGCAAGGAATTTTTGGGAAAACCCAAGT
Sequence number 1514: Dengue virus 3 Whole genome #77 
AGAACTGGAAGAAGGGGTCTATAGGATCAAACAGCAAGGAATTTTTGGGAAAACCCAAGTAGGGGTTG
GAGTACAAAAAGACGGAGTCTTCCACACCATGTGGCACGTCACAAGAGGGGC
Sequence number 1515: Dengue virus 3 Whole genome #78 AGGGGTTGGAGTACAAAAAGACGGAGTCTTCCACACCATGTGGCACGTCACAAGAGGGGCAGTGTTGA CACATAATGGGAAAAGACTGGAACCAAATTGGGCTAGCGTGAAAAAAGATCT
Sequence number 1516: Dengue virus 3 Whole genome #79 
AGTGTTGACACATAATGGGAAAAGACTGGAACCAAATTGGGCTAGCGTGAAAAAAGATCTGATTTCAT
ACGGAGGAGGATGGAGATTGAGCGCACAATGGCAAAAGGGGGAGGAGGTGCA
Sequence number 1517: Dengue virus 3 Whole genome #80 
GATTTCATACGGAGGAGGATGGAGATTGAGCGCACAATGGCAAAAGGGGGAGGAGGTGCAGGTCATTG
CCGTAGAGCCTGGGAAGAATCCAAAGAACTTCCAAACCATGCCAGGCACATT
Sequence number 1518: Dengue virus 3 Whole genome #81 
GGTCATTGCCGTAGAGCCTGGGAAGAATCCAAAGAACTTCCAAACCATGCCAGGCACATTCCAGACTA
CAACAGGGGAAATAGGAGCAATTGCACTGGATTTCAAGCCTGGAACTTCAGG
Sequence number 1519: Dengue virus 3 Whole genome #82 
CCAGACTACAACAGGGGAAATAGGAGCAATTGCACTGGATTTCAAGCCTGGAACTTCAGGGTCTCCTA
TCATAAACAGAGAGGGAAAGGTAGTGGGACTATATGGCAATGGAGTGGTTAC
Sequence number 1520: Dengue virus 3 Whole genome #83 
GTCTCCTATCATAAACAGAGAGGGAAAGGTAGTGGGACTATATGGCAATGGAGTGGTTACAAAGAATG
GCGGCTACGTCAGTGGAATAGCGCAAACAAATGCAGAACCAGATGGACCGAC
Sequence number 1521: Dengue virus 3 Whole genome #84 
AAAGAATGGCGGCTACGTCAGTGGAATAGCGCAAACAAATGCAGAACCAGATGGACCGACGCCAGAGT
TGGAAGAAGAGATGTTCAAAAAGCGAAATCTAACCATAATGGATCTTCATCC
Sequence number 1522: Dengue virus 3 Whole genome #85 
GCCAGAGTTGGAAGAAGAGATGTTCAAAAAGCGAAATCTAACCATAATGGATCTTCATCCTGGGTCAG
GAAAGACACGGAAATACCTTCCAGCCATTGTTAGAGAGGCAATCAAGAGACG
Sequence number 1523: Dengue virus 3 Whole genome #86 
TGGGTCAGGAAAGACACGGAAATACCTTCCAGCCATTGTTAGAGAGGCAATCAAGAGACGTTTAAGAA
CTCTGATTTTGGCACCGACAAGGGTGGTTGCAGCTGAGATGGAAGAAGCATT
Sequence number 1524: Dengue virus 3 Whole genome #87 
TTTAAGAACTCTGATTTTGGCACCGACAAGGGTGGTTGCAGCTGAGATGGAAGAAGCATTGAAAGGGC
TCCCAATAAGGTACCAAACAACAGCAACAAAATCTGAACATACGGGAAGAGA
Sequence number 1525: Dengue virus 3 Whole genome #88 
GAAAGGGCTCCCAATAAGGTACCAAACAACAGCAACAAAATCTGAACATACGGGAAGAGAGATTGTTG
ATCTAATGTGCCACGCAACGTTCACAATGCGCTTGCTGTCACCAGTTAGGGT
Sequence number 1526: Dengue virus 3 Whole genome #89 
GATTGTTGATCTAATGTGCCACGCAACGTTCACAATGCGCTTGCTGTCACCAGTTAGGGTTCCAAATT
ACAACTTGATAATAATGGATGAGGCCCATTTCACAGACCCAGCCAGTATAGC
Sequence number 1527: Dengue virus 3 Whole genome #90 
TCCAAATTACAACTTGATAATAATGGATGAGGCCCATTTCACAGACCCAGCCAGTATAGCGGCCAGAG
GGTACATATCAACTCGCGTTGGAATGGGAGAGGCAGCCGCAATCTTCATGAC
Sequence number 1528: Dengue virus 3 Whole genome #91 
GGCCAGAGGGTACATATCAACTCGCGTTGGAATGGGAGAGGCAGCCGCAATCTTCATGACGGCAACAC
CCCCTGGAACAGCTGATGCCTTTCCTCAGAGCAACGCTCCAATTCAAGATGA
Sequence number 1529: Dengue virus 3 Whole genome #92 
GGCAACACCCCCTGGAACAGCTGATGCCTTTCCTCAGAGCAACGCTCCAATTCAAGATGAAGAAAGGG
ACATACCAGAACGCTCATGGAATTCAGGCAATGAATGGATTACCGACTTCGC
Sequence number 1530: Dengue virus 3 Whole genome #93 
AGAAAGGGACATACCAGAACGCTCATGGAATTCAGGCAATGAATGGATTACCGACTTCGCCGGGAAAA
CGGTGTGGTTTGTCCCCAGCATCAAAGCCGGAAATGACATAGCAAACTGCTT
Sequence number 1531: Dengue virus 3 Whole genome #94 
CGGGAAAACGGTGTGGTTTGTCCCCAGCATCAAAGCCGGAAATGACATAGCAAACTGCTTGCGAAAGA
ACGGGAAAAAGGTCATTCAACTTAGTAGGAAGACTTTTGAAACAGAATATCA
Sequence number 1532: Dengue virus 3 Whole genome #95 
GCGAAAGAACGGGAAAAAGGTCATTCAACTTAGTAGGAAGACTTTTGAAACAGAATATCAGAAGACTA
AACTAAATGACTGGGACTTTGTGGTGACAACTGACATTTCAGAAATGGGGGC
Sequence number 1533: Dengue virus 3 Whole genome #96 
GAAGACTAAACTAAATGACTGGGACTTTGTGGTGACAACTGACATTTCAGAAATGGGGGCCAATTTCA
AAGCAGATAGAGTGATCGACCCAAGAAGATGTCTAAAACCAGTGATCCTGAC
Sequence number 1534: Dengue virus 3 Whole genome #97 
CAATTTCAAAGCAGATAGAGTGATCGACCCAAGAAGATGTCTAAAACCAGTGATCCTGACAGATGGAC
CAGAGCGGGTGATCCTGGCTGGACCAATGCCAGTCACCGCGGCGAGTGCTGC
Sequence number 1535: Dengue virus 3 Whole genome #98 
AGATGGACCAGAGCGGGTGATCCTGGCTGGACCAATGCCAGTCACCGCGGCGAGTGCTGCGCAAAGGA
GAGGGAGAGTTGGCAGGAACCCACAAAAAGAAAATGACCAGTACATATTCAC
Sequence number 1536: Dengue virus 3 Whole genome #99 
GCAAAGGAGAGGGAGAGTTGGCAGGAACCCACAAAAAGAAAATGACCAGTACATATTCACGGGTCAGC
CTCTCAACAATGATGAAGACCATGCTCACTGGACAGAAGCAAAAATGCTGCT
Sequence number 1537: Dengue virus 3 Whole genome #100 
GGGTCAGCCTCTCAACAATGATGAAGACCATGCTCACTGGACAGAAGCAAAAATGCTGCTGGACAACA
TCAACACTCCAGAAGGAATCATACCAGCTCTCTTTGAACCAGAAAGGGAGAA
Sequence number 1538: Dengue virus 3 Whole genome #101 
GGACAACATCAACACTCCAGAAGGAATCATACCAGCTCTCTTTGAACCAGAAAGGGAGAAGTCAGCCG
CCATAGACGGTGAATATCGCCTGAAGGGTGAATCCAGGAAGACTTTCGTGGA
Sequence number 1539: Dengue virus 3 Whole genome #102 
GTCAGCCGCCATAGACGGTGAATATCGCCTGAAGGGTGAATCCAGGAAGACTTTCGTGGAACTCATGA
GGAGGGGTGACCTCCCAGTTTGGTTAGCCCACAAAGTAGCATCAGAAGGGAT
Sequence number 1540: Dengue virus 3 Whole genome #103 
ACTCATGAGGAGGGGTGACCTCCCAGTTTGGTTAGCCCACAAAGTAGCATCAGAAGGGATCAAATATA
CAGATAGGAAATGGTGCTTTGATGGACAGCGCAACAACCAAATTTTAGAGGA
Sequence number 1541: Dengue virus 3 Whole genome #104 
CAAATATACAGATAGGAAATGGTGCTTTGATGGACAGCGCAACAACCAAATTTTAGAGGAAAACATGG
ACGTGGAAATCTGGACAAAGGAAGGAGAAAAGAAAAAATTAAGACCTAGGTG
Sequence number 1542: Dengue virus 3 Whole genome #105 
AAACATGGACGTGGAAATCTGGACAAAGGAAGGAGAAAAGAAAAAATTAAGACCTAGGTGGCTTGATG
CCCGCACTTATTCAGATCCCTTAGCACTCAAGGAATTCAAGGACTTTGCGGC
Sequence number 1543: Dengue virus 3 Whole genome #106 GCTTGATGCCCGCACTTATTCAGATCCCTTAGCACTCAAGGAATTCAAGGACTTTGCGGCTGGCAGAA AGTCAATCGCCCTTGATCTTGTGACAGAAATAGGAAGAGTGCCTTCACACCT
Sequence number 1544: Dengue virus 3 Whole genome #107 
TGGCAGAAAGTCAATCGCCCTTGATCTTGTGACAGAAATAGGAAGAGTGCCTTCACACCTAGCCCACA
GAACGAGAAACGCTCTGGACAATCTGGTGATGCTGCACACGTCAGAACATGG
Sequence number 1545: Dengue virus 3 Whole genome #108 
AGCCCACAGAACGAGAAACGCTCTGGACAATCTGGTGATGCTGCACACGTCAGAACATGGCGGTAGGG
CCTACAGGCATGCGGTGGAGGAACTACCAGAAACAATGGAAACACTCCTACT
Sequence number 1546: Dengue virus 3 Whole genome #109 
CGGTAGGGCCTACAGGCATGCGGTGGAGGAACTACCAGAAACAATGGAAACACTCCTACTCTTAGGAC
TCATGATCTTACTGACAGGTGGAGCAATGCTTTTCTTGATATCAGGTAAAGG
Sequence number 1547: Dengue virus 3 Whole genome #110 
CTTAGGACTCATGATCTTACTGACAGGTGGAGCAATGCTTTTCTTGATATCAGGTAAAGGGATTGGAA
AGACTTCAATAGGACTTATTTGTGTGATTGCTTCCAGCGGCATGCTGTGGAT
Sequence number 1548: Dengue virus 3 Whole genome #111 
GATTGGAAAGACTTCAATAGGACTTATTTGTGTGATTGCTTCCAGCGGCATGCTGTGGATGGCCGAAA
TTCCACTCCAATGGATCGCGTCGGCTATAGTCCTGGAGTTTTTTATGATGGT
Sequence number 1549: Dengue virus 3 Whole genome #112 
GGCCGAAATTCCACTCCAATGGATCGCGTCGGCTATAGTCCTGGAGTTTTTTATGATGGTGTTGCTTA
TACCAGAACCAGAAAAGCAGAGAACCCCCCAAGACAACCAACTCGCATATGT
Sequence number 1550: Dengue virus 3 Whole genome #113 
GTTGCTTATACCAGAACCAGAAAAGCAGAGAACCCCCCAAGACAACCAACTCGCATATGTCGTGATAG
GCATACTTACAATGGCTGCAATAATAGCGGCCAATGAAATGGGACTATTGGA
Sequence number 1551: Dengue virus 3 Whole genome #114 
CGTGATAGGCATACTTACAATGGCTGCAATAATAGCGGCCAATGAAATGGGACTATTGGAAACTACAA
AGAGAGATCTAGGAATGTCTAAGGAACCAGGTGTTGTTTCTCCAACTAGCTA
Sequence number 1552: Dengue virus 3 Whole genome #115 
AACTACAAAGAGAGATCTAGGAATGTCTAAGGAACCAGGTGTTGTTTCTCCAACTAGCTATTTGGATG
TGGATTTGCACCCAGCATCAGCCTGGACATTGTACGCTGTGGCCACTACAGT
Sequence number 1553: Dengue virus 3 Whole genome #116 
TTTGGATGTGGATTTGCACCCAGCATCAGCCTGGACATTGTACGCTGTGGCCACTACAGTAATAACAC
CAATGTTAAGACATACCATAGAGAATTCTACAGCAAATGTGTCCCTGGCAGC
Sequence number 1554: Dengue virus 3 Whole genome #117 
AATAACACCAATGTTAAGACATACCATAGAGAATTCTACAGCAAATGTGTCCCTGGCAGCTATAGCCA
ACCAGGCGGTGGTTCTGATGGGTTTGGACAAAGGATGGCCAATATCAAAAT
Sequence number 1555: Dengue virus 3 Whole genome #118 
TATAGCCAACCAGGCGGTGGTTCTGATGGGTTTGGACAAAGGATGGCCAATATCAAAAATGGACTTAG
GCGTACCACTACTGGCATTGGGTTGCTATTCACAAGTGAACCCACTAACTCT
Sequence number 1556: Dengue virus 3 Whole genome #119 
GGACTTAGGCGTACCACTACTGGCATTGGGTTGCTATTCACAAGTGAACCCACTAACTCTAACAGCGG
CAGTACTTTTGCTAGTTACGCATTATGCTATTATAGGTCCAGGACTGCAGGC
Sequence number 1557: Dengue virus 3 Whole genome #120 
AACAGCGGCAGTACTTTTGCTAGTTACGCATTATGCTATTATAGGTCCAGGACTGCAGGCAAAAGCCA
CTCGTGAAGCTCAAAAAAGGACAGCTGCTGGAATAATGAAGAATCCAACGGT
Sequence number 1558: Dengue virus 3 Whole genome #121 
AAAAGCCACTCGTGAAGCTCAAAAAAGGACAGCTGCTGGAATAATGAAGAATCCAACGGTGGATGGGA
TAATAACAATAGACCTAGACCCTGTAATATATGATTCAAAATTTGAAAAGCA
Sequence number 1559: Dengue virus 3 Whole genome #122 
GGATGGGATAATAACAATAGACCTAGACCCTGTAATATATGATTCAAAATTTGAAAAGCAACTGGGAC
AGGTCATGCTCCTGGTTTTGTGTGCAGTTCAACTGTTGTTAATGAGAACATC
Sequence number 1560: Dengue virus 3 Whole genome #123 
ACTGGGACAGGTCATGCTCCTGGTTTTGTGTGCAGTTCAACTGTTGTTAATGAGAACATCATGGGCCT
TGTGTGAAGCTTTAACTTTAGCTACAGGACCAATAACAACACTCTGGGAAGG
Sequence number 1561: Dengue virus 3 Whole genome #124 
ATGGGCCTTGTGTGAAGCTTTAACTTTAGCTACAGGACCAATAACAACACTCTGGGAAGGATCACCTG
GGAAGTTTTGGAATACCACGATAGCTGTTTCCATGGCGAACATTTTTAGAGG
Sequence number 1562: Dengue virus 3 Whole genome #125 
ATCACCTGGGAAGTTTTGGAATACCACGATAGCTGTTTCCATGGCGAACATTTTTAGAGGGAGCTATT
TAGCAGGAGCTGGGCTTGCCTTTTCTATTATGAAATCAGTTGGAACAGGAAA
Sequence number 1563: Dengue virus 3 Whole genome #126 
GAGCTATTTAGCAGGAGCTGGGCTTGCCTTTTCTATTATGAAATCAGTTGGAACAGGAAAAAGAGGAA
CAGGCTCTCAAGGTGAAACTTTAGGAGAAAAATGGAAAAAGAAATTGAATCA
Sequence number 1564: Dengue virus 3 Whole genome #127 
AAGAGGAACAGGCTCTCAAGGTGAAACTTTAGGAGAAAAATGGAAAAAGAAATTGAATCAATTATCCC
GGAAAGAGTTTGACCTTTACAAGAAGTCTGGAATCACTGAAGTGGATAGAAC
Sequence number 1565: Dengue virus 3 Whole genome #128 
ATTATCCCGGAAAGAGTTTGACCTTTACAAGAAGTCTGGAATCACTGAAGTGGATAGAACAGAAGCCA
AAGAAGGGTTGAAAAGAGGAGAAACAACACATCATGCCGTGTCCAGGGGTAG
Sequence number 1566: Dengue virus 3 Whole genome #129 
AGAAGCCAAAGAAGGGTTGAAAAGAGGAGAAACAACACATCATGCCGTGTCCAGGGGTAGTGCAAAAC
TTCAATGGTTTGTGGAAAGGAACATGGTCATTCCCGAGGGAAGAGTCATAGA
Sequence number 1567: Dengue virus 3 Whole genome #130 
TGCAAAACTTCAATGGTTTGTGGAAAGGAACATGGTCATTCCCGAGGGAAGAGTCATAGACCTGGGCT
GTGGAAGAGGAGGCTGGTCATATTACTGTGCAGGACTGAAAAAAGTCACAGA
Sequence number 1568: Dengue virus 3 Whole genome #131 
CCTGGGCTGTGGAAGAGGAGGCTGGTCATATTACTGTGCAGGACTGAAAAAAGTCACAGAAGTGCGAG
GATACACAAAAGGCGGCCCAGGACACGAAGAACCAGTGCCTATGTCAACATA
Sequence number 1569: Dengue virus 3 Whole genome #132 
AGTGCGAGGATACACAAAAGGCGGCCCAGGACACGAAGAACCAGTGCCTATGTCAACATATGGATGGA
ACATAGTTAAATTAATGAGTGGAAAGGATGTGTTTTATCTTCCGCCTGAAAA
Sequence number 1570: Dengue virus 3 Whole genome #133 
TGGATGGAACATAGTTAAATTAATGAGTGGAAAGGATGTGTTTTATCTTCCGCCTGAAAAGTGTGACA
CCCTGTTGTGCGACATTGGAGAATCTTCACCAAGCCCAACAGTGGAAGAAAG
Sequence number 1571: Dengue virus 3 Whole genome #134 GTGTGACACCCTGTTGTGCGACATTGGAGAATCTTCACCAAGCCCAACAGTGGAAGAAAGCAGAACtA TAAGAGTTTTGAAGATGGTTGAACCATGGCTGAAAAACAACCAGTTTTGCAT
Sequence number 1572: Dengue virus 3 Whole genome #135 
CAGAACt ATAAGAGTTTTGAAGATGGTTGAACCATGGCTGAAAAACAACCAGTTTTGCATTAAAGTAT
TGAACCCTTACATGCCAGCCGTGATTGAGCACCTAGAAAGACTGCAAAGGAA
Sequence number 1573: Dengue virus 3 Whole genome #136 
TAAAGTATTGAACCCTTACATGCCAGCCGTGATTGAGCACCTAGAAAGACTGCAAAGGAAACACGGAG
GAATGCTTGTGAGAAATCCACTTTCACGAAACTCCACGCACGAAATGTACTG
Sequence number 1574: Dengue virus 3 Whole genome #137 
ACACGGAGGAATGCTTGTGAGAAATCCACTTTCACGAAACTCCACGCACGAAATGTACTGGATATCTA
ATGGTACAGGTAACATTGTCTCTTCAGTCAACATGATATCCAGATTGCTACT
Sequence number 1575: Dengue virus 3 Whole genome #138 
GATATCTAATGGTACAGGTAACATTGTCTCTTCAGTCAACATGATATCCAGATTGCTACTGAACAGGT
TCACGATGACACACAGGAGACCTACCATAGAGAAAGATGTGGATTTAGGAGC
Sequence number 1576: Dengue virus 3 Whole genome #139 
GAACAGGTTCACGATGACACACAGGAGACCTACCATAGAGAAAGATGTGGATTTAGGAGCAGGAACTC
GACATGTCAATGCGGAACCAGAAACACCCAACATGGATGTCATTGGGGAAAG
Sequence number 1577: Dengue virus 3 Whole genome #140 
AGGAACTCGACATGTCAATGCGGAACCAGAAACACCCAACATGGATGTCATTGGGGAAAGAATAAAAA
GGATCAAGGAGGAGCATAATTCAACATGGCACTATGATGACGAAAACCCCTA
Sequence number 1578: Dengue virus 3 Whole genome #141 
AATAAAAAGGATCAAGGAGGAGCATAATTCAACATGGCACTATGATGACGAAAACCCCTACAAAACGT
GGGCTTACCACGGATCTTATGAAGTCAAGGCCACAGGCTCAGCCTCCTCCAT
Sequence number 1579: Dengue virus 3 Whole genome #142 
CAAAACGTGGGCTTACCACGGATCTTATGAAGTCAAGGCCACAGGCTCAGCCTCCTCCATGATAAACG
GAGTCGTGAAACTTCTCACTAAACCATGGGATGTAGTGCCCATGGTGACACA
Sequence number 1580: Dengue virus 3 Whole genome #143 
GATAAACGGAGTCGTGAAACTTCTCACTAAACCATGGGATGTAGTGCCCATGGTGACACAGATGGCAA
TGACAGATACAACTCCATTTGGCCAGCAGAGAGTTTTTAAAGAGAAGGTGGA
Sequence number 1581: Dengue virus 3 Whole genome #144 
GATGGCAATGACAGATACAACTCCATTTGGCCAGCAGAGAGTTTTTAAAGAGAAGGTGGACACCAGGA
CACCCAGGCCCATGCCAGGGACAAGAAAGGTTATGGAGATCACAGCGGAGTG
Sequence number 1582: Dengue virus 3 Whole genome #145 
CACCAGGACACCCAGGCCCATGCCAGGGACAAGAAAGGTTATGGAGATCACAGCGGAGTGGCTCTGGA
GAACCCTGGGAAGGAACAAAAGACCCAGGTTATGCACAAGGGAAGAGTTCAC
Sequence number 1583: Dengue virus 3 Whole genome #146 
GCTCTGGAGAACCCTGGGAAGGAACAAAAGACCCAGGTTATGCACAAGGGAAGAGTTCACAAAAAAGG
TCAGAACCAACGCAGCCATGGGCGCCGTTTTCACAGAGGAGAACCAATGGGA
Sequence number 1584: Dengue virus 3 Whole genome #147 
AAAAAAGGTCAGAACCAACGCAGCCATGGGCGCCGTTTTCACAGAGGAGAACCAATGGGACAGTGCGA
AAGCCGCTGTTGAGGATGAAGATTTTTGGAAACTTGTGGACAGAGAACGTGA
Sequence number 1585: Dengue virus 3 Whole genome #148 
CAGTGCGAAAGCCGCTGTTGAGGATGAAGATTTTTGGAAACTTGTGGACAGAGAACGTGAACTCCACA
AATTGGGCAAGTGTGGAAGCTGTGTTTACAACATGATGGGCAAGAGAGAGAA
Sequence number 1586: Dengue virus 3 Whole genome #149 
ACTCCACAAATTGGGCAAGTGTGGAAGCTGTGTTTACAACATGATGGGCAAGAGAGAGAAGAAACTTG
GAGAGTTTGGCAAAGCAAAAGGCAGTAGAGCTATATGGTACATGTGGTTGGG
Sequence number 1587: Dengue virus 3 Whole genome #150 
GAAACTTGGAGAGTTTGGCAAAGCAAAAGGCAGTAGAGCTATATGGTACATGTGGTTGGGAGCCAGGT
ACCTTGAGTTCGAAGCCCTTGGATTCTTAAATGAAGATCACTGGTTCTCGCG
Sequence number 1588: Dengue virus 3 Whole genome #151 
AGCCAGGTACCTTGAGTTCGAAGCCCTTGGATTCTTAAATGAAGATCACTGGTTCTCGCGTGAAAACT
CTTACAGTGGAGTAGAAGGAGAAGGACTGCACAAGCTAGGCTACATATTAAG
Sequence number 1589: Dengue virus 3 Whole genome #152 
TGAAAACTCTTACAGTGGAGTAGAAGGAGAAGGACTGCACAAGCTAGGCTACATATTAAGGGACATTT
CCAAGATACCCGGAGGAGCCATGTATGCTGATGACACAGCTGGTTGGGACAC
Sequence number 1590: Dengue virus 3 Whole genome #153 
GGACATTTCCAAGATACCCGGAGGAGCCATGTATGCTGATGACACAGCTGGTTGGGACACAAGAATAA
CAGAAGATGACCTGCACAATGAGGAAAAGATCACACAGCAAATGGACCCTGA
Sequence number 1591: Dengue virus 3 Whole genome #154 
AAGAATAACAGAAGATGACCTGCACAATGAGGAAAAGATCACACAGCAAATGGACCCTGAACACAGGC
TGTTAGCGAACGCTATATTTAAGCTCACATACCAAAACAAAGTGGTCAAAGT
Sequence number 1592: Dengue virus 3 Whole genome #155 
ACACAGGCTGTTAGCGAACGCTATATTTAAGCTCACATACCAAAACAAAGTGGTCAAAGTTCAACGAC
CGACTCCAACAGGCACGGTAATGGACATTATATCTAGGAAAGACCAAAGAGG
Sequence number 1593: Dengue virus 3 Whole genome #156 
TCAACGACCGACTCCAACAGGCACGGTAATGGACATTATATCTAGGAAAGACCAAAGAGGTAGTGGAC
AGGTGGGAACTTATGGTCTGAACACATTTACTAACATGGAAGCCCAGTTAGT
Sequence number 1594: Dengue virus 3 Whole genome #157 
TAGTGGACAGGTGGGAACTTATGGTCTGAACACATTTACTAACATGGAAGCCCAGTTAGTCAGACAAA
TGGAAGGAGAAGGTGTGCTGTCAAAGGCAGACCTCGAGAACCCTCATCTGCC
Sequence number 1595: Dengue virus 3 Whole genome #158 
CAGACAAATGGAAGGAGAAGGTGTGCTGTCAAAGGCAGACCTCGAGAACCCTCATCTGCCAGAAAAGA
AAATTACACAATGGCTGGAAACCAAAGGAGTGGAgAGGTTAAAAAGAATGGC
Sequence number 1596: Dengue virus 3 Whole genome #159 
AGAAAAGAAAATTACACAATGGCTGGAAACCAAAGGAGTGGAGAGGTTAAAAAGAATGGCCATTAGCG
GGGATGATTGCGTAGTGAAACCAATCGATGACAGGTTCGCTAACGCCCTGCT
Sequence number 1597: Dengue virus 3 Whole genome #160 
CATTAGCGGGGATGATTGCGTAGTGAAACCAATCGATGACAGGTTCGCTAACGCCCTGCTTGCTCTGA
ACGATATGGGAAAGGTTCGGAAAGACATACCTCAATGGCAGCCATCAAAAGG
Sequence number 1598: Dengue virus 3 Whole genome #161 
TGCTCTGAACGATATGGGAAAGGTTCGGAAAGACATACCTCAATGGCAGCCATCAAAAGGATGGCATG
ACTGGCAGCAGGTTCCTTTCTGCTCCCACCACTTTCATGAATTGATCATGAA
Sequence number 1599: Dengue virus 3 Whole genome #162 ATGGCATGACTGGCAGCAGGTTCCTTTCTGCTCCCACCACTTTCATGAATTGATCATGAAAGATGGAA GAAAGTTGGTGGTTCCCTGCAGACCCCAGGACGAACTAATAGGAAGAGCAAG
Sequence number 1600: Dengue virus 3 Whole genome #163 
AGATGGAAGAAAGTTGGTGGTTCCCTGCAGACCCCAGGACGAACTAATAGGAAGAGCAAGAATCTCTC
AAGGAGCGGGATGGAGCCTTAGAGAAACCGCATGTTTAGGGAAAGCCTACGC
Sequence number 1601: Dengue virus 3 Whole genome #164 
AATCTCTCAAGGAGCGGGATGGAGCCTTAGAGAAACCGCATGTTTAGGGAAAGCCTACGCCCAAATGT
GGAGTCTCATGTATTTTCACAGAAGAGATCTCAGACTAGCGTCCAATGCCAT
Sequence number 1602: Dengue virus 3 Whole genome #165 
CCAAATGTGGAGTCTCATGTATTTTCACAGAAGAGATCTCAGACTAGCGTCCAATGCCATATGTTCAG
CAGTACCAGTCCACTGGGTCCCTACAAGTAGAACGACATGGTCTATTCATGC
Sequence number 1603: Dengue virus 3 Whole genome #166 
ATGTTCAGCAGTACCAGTCCACTGGGTCCCTACAAGTAGAACGACATGGTCTATTCATGCTCACCATC
AGTGGATGACCACAGAGGACATGCTTACTGTTTGGAACAGGGTGTGGATTGA
Sequence number 1604: Dengue virus 3 Whole genome #167 
TCACCATCAGTGGATGACCACAGAGGACATGCTTACTGTTTGGAACAGGGTGTGGATTGAGGACAATC
CATGGATGGAAGACAAAACTCCAATTACAACCTGGGAAAATGTTCCATACCT
Sequence number 1605: Dengue virus 3 Whole genome #168 
GGACAATCCATGGATGGAAGACAAAACTCCAATTACAACCTGGGAAAATGTTCCATACCTAGGGAAGA
GGGAAGACCAATGGTGTGGATCACTTATTGGTCTCACTTCCAGAGCAACCTG
Sequence number 1606: Dengue virus 3 Whole genome #169 
AGGGAAGAGGGAAGACCAATGGTGTGGATCACTTATTGGTCTCACTTCCAGAGCAACCTGGGCCCAGA
ACATACCCACAGCAATTCAACAGGTGAGAAGCCTTATAGGCAATGAAGAGTT
Sequence number 1607: Dengue virus 3 Whole genome #170 
GGCCCAGAACATACCCACAGCAATTCAACAGGTGAGAAGCCTTATAGGCAATGAAGAGTTTCTGGACT
ACATGCCTTCAATGAAGAGATTCAGGAAGGAGGAGGAGTCGGAGGGAGCCAT
Sequence number 1608: Dengue virus 3 Whole genome #171 
TCTGGACTACATGCCTTCAATGAAGAGATTCAGGAAGGAGGAGGAGTCGGAGGGAGCCATTTGGTAAA
CGTAGGAAGTGAAAAAGAGGCTGACTGTCAGGCCACCTTAAGCCACAGTACG
Sequence number 1609: Dengue virus 3 Whole genome #172 
TTGGTAAACGTAGGAAGTGAAAAAGAGGCTGACTGTCAGGCCACCTTAAGCCACAGTACGGAAGAAGC
TGTGCTGCCTGTGAGCCCCGTCCAAGGACGTTAAAAGAAGAAGTCAGGCCCC
Sequence number 1610: Dengue virus 3 Whole genome #173 
GAAGAAGCTGTGCTGCCTGTGAGCCCCGTCCAAGGACGTTAAAAGAAGAAGTCAGGCCCCAAAGCCAC
GGTTTGAGCAAACCGTGCTGCCTGTAGCTCCGTCGTGGGGACGTAAAACCTG
Sequence number 1611: Dengue virus 3 Whole genome #174 
AAAGCCACGGTTTGAGCAAACCGTGCTGCCTGTAGCTCCGTCGTGGGGACGTAAAACCTGGGAGGCTG
CAAACTGTGGAAGCTGTACGCACGGTGTAGCAGACTAGTGGTTAGAGGAGAC
Sequence number 1612: Dengue virus 3 Whole genome #175 
GGAGGCTGCAAACTGTGGAAGCTGTACGCACGGTGTAGCAGACTAGTGGTTAGAGGAGACCCCTCCCA
TGACACAACGCAGCAGCGGGGCCCGAGCACTGAGGGAAGCTGTACCTCCTTG
Sequence number 1613: Dengue virus 3 Whole genome #176 
CCCTCCCATGACACAACGCAGCAGCGGGGCCCGAGCACTGAGGGAAGCTGTACCTCCTTGCAAAGGAC
TAGAGGTTAGAGGAGACCCCCCGCAAACAAAAACAGCATATTGACGCTGGGA
Sequence number 1614: Dengue virus 3 Whole genome #177 
CAAAGGACTAGAGGTTAGAGGAGACCCCCCGCAAACAAAAACAGCATATTGACGCTGGGAGAGACCAG
AGATCCTGCTGTCTCCTCAGCATCATTCCAGGCACAGAACGCCAGAAAATGG
Sequence number 1615: Dengue virus 3 Whole genome #178 
CCCGCAAACAAAAACAGCATATTGACGCTGGGAGAGACCAGAGATCCTGCTGTCTCCTCAGCATCATT
CCAGGCACAGAACGCCAGAAAATGGAATGGTGCTGTTGAATCAACAGGTTCT
Sequence number 1616: Dengue virus 4 Whole genome #1 
AGTTGTTAGTCTGTGTGGACCGACAAGGACAGTTCCAAATCGGAAGCTTGCTTAACACAGTTCTAACA
GTTTGTTTAAATAGAGAGCAGATCTCTGGAAAAATGAACCAACGAAAAAAGG
Sequence number 1617: Dengue virus 4 Whole genome #2 
TTCTAACAGTTTGTTTAAATAGAGAGCAGATCTCTGGAAAAATGAACCAACGAAAAAAGGTGGTCAGA
CCACCTTTCAATATGCTGAAACGCGAGAGAAACCGCGTATCTACCCCTCAAG
Sequence number 1618: Dengue virus 4 Whole genome #3 
TGGTCAGACCACCTTTCAATATGCTGAAACGCGAGAGAAACCGCGTATCTACCCCTCAAGGGTTGGGA
AAGAGATTCTCAACCGGACTTTTCTCCGGGAAAGGACCCTTGCGGATGGTGC
Sequence number 1619: Dengue virus 4 Whole genome #4 
GGTTGGGAAAGAGATTCTCAACCGGACTTTTCTCCGGGAAAGGACCCTTGCGGATGGTGCTAGCATTC
ATCACGTTTTTGCGGGTCCTTTCCATCCCACCAACAGCAGGGATTCTGAAAA
Sequence number 1620: Dengue virus 4 Whole genome #5 
TAGCATTCATCACGTTTTTGCGGGTCCTTTCCATCCCACCAACAGCAGGGATTCTGAAAAGATGGGGA
CAGTTGAAAAAGAACAAGGCCGTCAAAATACTGATTGGATTCAGGAAGGAGA
Sequence number 1621: Dengue virus 4 Whole genome #6 
GATGGGGACAGTTGAAAAAGAACAAGGCCGTCAAAATACTGATTGGATTCAGGAAGGAGATAGGTCGC
ATGTTAAACATCTTGAATAGGAGAAGAAGGTCAACAATGACATTGCTGTGTT
Sequence number 1622: Dengue virus 4 Whole genome #7 
TAGGTCGCATGTTAAACATCTTGAATAGGAGAAGAAGGTCAACAATGACATTGCTGTGTTTGATTCCC
ACCGTAATGGCGTTTCACCTGTCAACAAGAGATGGCGAACCCCTCATGATAG
Sequence number 1623: Dengue virus 4 Whole genome #8 
TGATTCCCACCGTAATGGCGTTTCACCTGTCAACAAGAGATGGCGAACCCCTCATGATAGTGGCAAAA
CACGAAAGGGGGGGACCTCTTTTGTTTAAGATAACAGAAGGAATCAACAAAT
Sequence number 1624: Dengue virus 4 Whole genome #9 
TGGCAAAACACGAAAGGGGGGGACCTCTTTTGTTTAAGATAACAGAAGGAATCAACAAATGTACCCTT
ATTGCTATGGACCTGGGTGAAATGTGCGAAGACACTGTCACGTATAAGTGTC
Sequence number 1625: Dengue virus 4 Whole genome #10 
GTACCCTTATTGCTATGGACCTGGGTGAAATGTGCGAAGACACTGTCACGTATAAGTGTCCTCTACTG
GTTAACACCGAACCTGAGGACATTGACTGCTGGTGCAATCTCACGTCCACCT
Sequence number 1626: Dengue virus 4 Whole genome #11 
CTCTACTGGTTAACACCGAACCTGAGGACATTGACTGCTGGTGCAATCTCACGTCCACCTGGGTCATG
TACGGGACATGCACCCAGAACGGAGAACGGAGACGAGAGAAGCGCTCAGTAG
Sequence number 1627: Dengue virus 4 Whole genome #12 GGGTCATGTACGGGACATGCACCCAGAACGGAGAACGGAGACGAGAGAAGCGCTCAGTAGCTTTAACA CCACATTCAGGAATGGGATTGGAAACAAGAGCTGAGACATGGATGTCATCGG
Sequence number 1628: Dengue virus 4 Whole genome #13 
CTTTAACACCACATTCAGGAATGGGATTGGAAACAAGAGCTGAGACATGGATGTCATCGGAAGGGGCT
TGGAAACATGCTCAGAGAGTAGAAAGCTGGATACTCAGAAACCCAGGATTCG
Sequence number 1629: Dengue virus 4 Whole genome #14 
AAGGGGCTTGGAAACATGCTCAGAGAGTAGAAAGCTGGATACTCAGAAACCCAGGATTCGCGCTCTTG
GCCGGATTTATGGCTTACATGATTGGGCAAACAGGAATCCAGCGAACTGTTT
Sequence number 1630: Dengue virus 4 Whole genome #15 
CGCTCTTGGCCGGATTTATGGCTTACATGATTGGGCAAACAGGAATCCAGCGAACTGTTTTCTTTGTC
CTGATGATGCTAGTCGCCCCATCCTACGGAATGCGATGCGTAGGGGTAGGGA
Sequence number 1631: Dengue virus 4 Whole genome #16 
TCTTTGTCCTGATGATGCTAGTCGCCCCATCCTACGGAATGCGATGCGTAGGGGTAGGGAACAGAGAC
TTTGTGGAAGGAGTCTCGGGTGGAGCATGGGTCGACTTGGTGCTAGAACATG
Sequence number 1632: Dengue virus 4 Whole genome #17 
ACAGAGACTTTGTGGAAGGAGTCTCGGGTGGAGCATGGGTCGACTTGGTGCTAGAACATGGAGGATGC
GTCACAACCATGGCCCAGGGAAAACCAACCCTGGATTTTGAATTGACCAAGA
Sequence number 1633: Dengue virus 4 Whole genome #18 
GAGGATGCGTCACAACCATGGCCCAGGGAAAACCAACCCTGGATTTTGAATTGACCAAGACAACAGCC
AAGGAAGTGGCTCTGTTAAGAACCTATTGCATTGAAGCCTCGATATCAAACA
Sequence number 1634: Dengue virus 4 Whole genome #19 
CAACAGCCAAGGAAGTGGCTCTGTTAAGAACCTATTGCATTGAAGCCTCGATATCAAACATAACCACG
GCAACAAGATGTCCAACGCAACGCAATCACCGACTCGACCCATTGCAAGACC
Sequence number 1635: Dengue virus 4 Whole genome #20 
TAACCACGGCAACAAGATGTCCAACGCAACGCAATCACCGACTCGACCCATTGCAAGACCAACAGTAC
ATTTGCCGTAGAGACGTGGTAGACAGAGGGTGGGGCAATGGCTGTGGCTTAT
Sequence number 1636: Dengue virus 4 Whole genome #21 
AACAGTACATTTGCCGTAGAGACGTGGTAGACAGAGGGTGGGGCAATGGCTGTGGCTTATTTGGAAAA
GGAGGAGTTGTGACATGTGCGAAGTTTTTATGCTCGGGGAAAATAACAGGCA
Sequence number 1637: Dengue virus 4 Whole genome #22 
TTGGAAAAGGAGGAGTTGTGACATGTGCGAAGTTTTTATGCTCGGGGAAAATAACAGGCAATCTGGTC
CAAGTTGAAAACCTTGAATATACAGTAGTAGTAACAGTCCACAATGGAGACA
Sequence number 1638: Dengue virus 4 Whole genome #23 
ATCTGGTCCAAGTTGAAAACCTTGAATATACAGTAGTAGTAACAGTCCACAATGGAGACACCCATGCA
GTAGGAAATGACACGTCCAACCATGGAGTGACAGCCACGATAACTCCTAGGT
Sequence number 1639: Dengue virus 4 Whole genome #24 
CCCATGCAGTAGGAAATGACACGTCCAACCATGGAGTGACAGCCACGATAACTCCTAGGTCACCATCG
GTAGAAGTTAAATTGCCGGACTATGGAGAACTAACACTCGACTGCGAACCCA
Sequence number 1640: Dengue virus 4 Whole genome #25 
CACCATCGGTAGAAGTTAAATTGCCGGACTATGGAGAACTAACACTCGACTGCGAACCCAGGTCCGGA
ATTGATTTTAATGAGATGATCCTGATGAAAATGAAAAAGAAAACGTGGCTTG
Sequence number 1641: Dengue virus 4 Whole genome #26 
GGTCCGGAATTGATTTTAATGAGATGATCCTGATGAAAATGAAAAAGAAAACGTGGCTTGTGCATAAG
CAATGGTTTTTAGACCTACCTCTACCATGGACGGCAGGAGCAGACACATCAG
Sequence number 1642: Dengue virus 4 Whole genome #27 
TGCATAAGCAATGGTTTTTAGACCTACCTCTACCATGGACGGCAGGAGCAGACACATCAGAAGTTCAT
TGGAATCATAAAGAGAGAATGGTGACATTTAAGGTTCCTCATGCCAAGAGAC
Sequence number 1643: Dengue virus 4 Whole genome #28 
AAGTTCATTGGAATCATAAAGAGAGAATGGTGACATTTAAGGTTCCTCATGCCAAGAGACAGGATGTG
ACAGTGCTAGGATCTCAGGAGGGAGCTATGCACTCTGCCCTCGCCGGAGCCA
Sequence number 1644: Dengue virus 4 Whole genome #29 
AGGATGTGACAGTGCTAGGATCTCAGGAGGGAGCTATGCACTCTGCCCTCGCCGGAGCCACAGAAGTG
GATTCTGGTGATGGAAATCACATGTTTGCGGGACATCTCAAGTGCAAAGAAC
Sequence number 1645: Dengue virus 4 Whole genome #30 
CAGAAGTGGATTCTGGTGATGGAAATCACATGTTTGCGGGACATCTCAAGTGCAAAGAACAATGGCTA
AAATTGAGAATTAAAGGAATGTCATACACGATGTGTTCAGGAAAGTTCTCAA
Sequence number 1646: Dengue virus 4 Whole genome #31 
AATGGCTAAAATTGAGAATTAAAGGAATGTCATACACGATGTGTTCAGGAAAGTTCTCAATTGACAAA
GAGATGGCAGAAACACAGCATGGAACAACAGTGGTGAAAGTCAAGTATGAAG
Sequence number 1647: Dengue virus 4 Whole genome #32 
TTGACAAAGAGATGGCAGAAACACAGCATGGAACAACAGTGGTGAAAGTCAAGTATGAAGGCGCTGGA
GCTCCGTGTAAAGTCCCCATAGAGATAAGAGATGTGAACAAGGAAAAAGTGG
Sequence number 1648: Dengue virus 4 Whole genome #33 
GCGCTGGAGCTCCGTGTAAAGTCCCCATAGAGATAAGAGATGTGAACAAGGAAAAAGTGGTCGGGCGT
ATCATCTCATCTACCCCTTTTGCTGAGAATACCAACAGCGTAACCAACATAG
Sequence number 1649: Dengue virus 4 Whole genome #34 
TCGGGCGTATCATCTCATCTACCCCTTTTGCTGAGAATACCAACAGCGTAACCAACATAGAATTAGAA
CCCCCTTTTGGGGACAGCTACATAGTGATAGGTGTTGGGGAGAGTGCATTAA
Sequence number 1650: Dengue virus 4 Whole genome #35 
AATTAGAACCCCCTTTTGGGGACAGCTACATAGTGATAGGTGTTGGGGAGAGTGCATTAACACTCCAT
TGGTTCAGAAAAGGGAGCTCCATTGGCAAGATGTTTGAGTCCACATACAGAG
Sequence number 1651: Dengue virus 4 Whole genome #36 
CACTCCATTGGTTCAGAAAAGGGAGCTCCATTGGCAAGATGTTTGAGTCCACATACAGAGGTGCAAAA
CGAATGGCCATTCTAGGTGAAACAGCTTGGGATTTTGGTTCTGTTGGTGGAC
Sequence number 1652: Dengue virus 4 Whole genome #37 
GTGCAAAACGAATGGCCATTCTAGGTGAAACAGCTTGGGATTTTGGTTCTGTTGGTGGACTGTTCACA
TCATTGGGAAAGGCTGTACACCAGGTTTTTGGAAGTGTGTATACAACCATGT
Sequence number 1653: Dengue virus 4 Whole genome #38 
TGTTCACATCATTGGGAAAGGCTGTACACCAGGTTTTTGGAAGTGTGTATACAACCATGTTTGGAGGG
GTCTCATGGATGGTTAGAATCCTAATTGGGTTCTTAGTATTGTGGATTGGCA
Sequence number 1654: Dengue virus 4 Whole genome #39 
TTGGAGGGGTCTCATGGATGGTTAGAATCCTAATTGGGTTCTTAGTATTGTGGATTGGCACGAATTCA
AGAAACACTTCAATGGCAATGACGTGCATAGCTGTTGGAGGAATCACTCTGT
Sequence number 1655: Dengue virus 4 Whole genome #40 CGAATTCAAGAAACACTTCAATGGCAATGACGTGCATAGCTGTTGGAGGAATCACTCTGTTTCTAGGC TTCACAGTTCAAGCAGACATGGGTTGTGTGGTGTCATGGAATGGGAAGGAAT
Sequence number 1656: Dengue virus 4 Whole genome #41 
TTCTAGGCTTCACAGTTCAAGCAGACATGGGTTGTGTGGTGTCATGGAATGGGAAGGAATTAAAATGT
GGAAGCGGAATCTTTGTGGTTGACAACGTGCACACTTGGACAGAACAGTACA
Sequence number 1657: Dengue virus 4 Whole genome #42 
TAAAATGTGGAAGCGGAATCTTTGTGGTTGACAACGTGCACACTTGGACAGAACAGTACAAATTTCAA
CCAGAGTCCCCAGCGAGACTAGCGTCTGCAATATTGAATGCCCACAAAGATG
Sequence number 1658: Dengue virus 4 Whole genome #43 
AATTTCAACCAGAGTCCCCAGCGAGACTAGCGTCTGCAATATTGAATGCCCACAAAGATGGGGTCTGT
GGAATTAGATCAACCACGAGGCTGGAAAATGTCATGTGGAAGCAAATAACCA
Sequence number 1659: Dengue virus 4 Whole genome #44 
GGGTCTGTGGAATTAGATCAACCACGAGGCTGGAAAATGTCATGTGGAAGCAAATAACCAACGAGCTA
AACTATGTTCTCTGGGAAGGAGGACATGACCTCACTGTTGTGGCTGGGGACG
Sequence number 1660: Dengue virus 4 Whole genome #45 
ACGAGCTAAACTATGTTCTCTGGGAAGGAGGACATGACCTCACTGTTGTGGCTGGGGACGTGAAGGGG
GTGTTGACCAAAGGCAAGAGAGCACTCACACCTCCAGTGAATGATCTGAAAT
Sequence number 1661: Dengue virus 4 Whole genome #46 
TGAAGGGGGTGTTGACCAAAGGCAAGAGAGCACTCACACCTCCAGTGAATGATCTGAAATATTCATGG
AAAACATGGGGGAAAGCAAAAATCTTTACCCCAGAAGCAAGAAATAGTACGT
Sequence number 1662: Dengue virus 4 Whole genome #47 
ATTCATGGAAAACATGGGGGAAAGCAAAAATCTTTACCCCAGAAGCAAGAAATAGTACGTTTTTAATA
GACGGACCAGACACCCCCGAATGCCCCAATGAGCGAAGAGCATGGAACTTTT
Sequence number 1663: Dengue virus 4 Whole genome #48 
TTTTAATAGACGGACCAGACACCCCCGAATGCCCCAATGAGCGAAGAGCATGGAACTTTTTGGAGGTA
GAAGACTATGGATTTGGCATGTTCACGACCAACATATGGATGAAATTCCGAG
Sequence number 1664: Dengue virus 4 Whole genome #49 
TGGAGGTAGAAGACTATGGATTTGGCATGTTCACGACCAACATATGGATGAAATTCCGAGAAGGAAGT
TCAGAAGTGTGTGACCATAGATTAATGTCGGCGGCAATCAAAGACCAGAAAG
Sequence number 1665: Dengue virus 4 Whole genome #50 
AAGGAAGTTCAGAAGTGTGTGACCATAGATTAATGTCGGCGGCAATCAAAGACCAGAAAGCTGTGCAT
GCCGACATGGGTTATTGGATAGAGAGCTCAAAAAACCAGACCTGGCAGATAG
Sequence number 1666: Dengue virus 4 Whole genome #51 
CTGTGCATGCCGACATGGGTTATTGGATAGAGAGCTCAAAAAACCAGACCTGGCAGATAGAGAAAGCA
TCCCTTATTGAAGTGAAAACATGTCTGTGGCCCAAAACCCACACGCTGTGGA
Sequence number 1667: Dengue virus 4 Whole genome #52 
AGAAAGCATCCCTTATTGAAGTGAAAACATGTCTGTGGCCCAAAACCCACACGCTGTGGAGCAATGGA
GTGCTGGAAAGTCAGATGCTCATTCCAAGATCATATGCAGGCCCCTTTTCAC
Sequence number 1668: Dengue virus 4 Whole genome #53 
GCAATGGAGTGCTGGAAAGTCAGATGCTCATTCCAAGATCATATGCAGGCCCCTTTTCACAGCACAAT
TACCGTCAGGGCTATGCCACGCAAACCGTGGGCCCATGGCACCTAGGCAAAT
Sequence number 1669: Dengue virus 4 Whole genome #54 
AGCACAATTACCGTCAGGGCTATGCCACGCAAACCGTGGGCCCATGGCACCTAGGCAAATTAGAGATA
GACTTTGGAGAATGCCCCGGGACAACAGTCGCAATTCGGGAGGATTGTGACC
Sequence number 1670: Dengue virus 4 Whole genome #55 
TAGAGATAGACTTTGGAGAATGCCCCGGGACAACAGTCGCAATTCGGGAGGATTGTGACCACAGAGGC
CCATCTTTGAGAACCACCACCGCATCTGGAAAACTGGTCACGCAATGGTGCT
Sequence number 1671: Dengue virus 4 Whole genome #56 
ACAGAGGCCCATCTTTGAGAACCACCACCGCATCTGGAAAACTGGTCACGCAATGGTGCTGCCGCTCC
TGCACGATGCCTCCCTTAAGGTTTTTGGGAGAAGATGGATGTTGGTATGGGA
Sequence number 1672: Dengue virus 4 Whole genome #57 
GCCGCTCCTGCACGATGCCTCCCTTAAGGTTTTTGGGAGAAGATGGATGTTGGTATGGGATGGAGATC
AGGCCTTTGAGTGAAAAAGAAGAGAACATGGTAAAATCACAGGTAACGGCCG
Sequence number 1673: Dengue virus 4 Whole genome #58 
TGGAGATCAGGCCTTTGAGTGAAAAAGAAGAGAACATGGTAAAATCACAGGTAACGGCCGGACAGGGC
ACATCAGAAACTTTTTCTATGGGGCTGCTATGCCTGACCTTGTTTGTGGAGG
Sequence number 1674: Dengue virus 4 Whole genome #59 
GACAGGGCACATCAGAAACTTTTTCTATGGGGCTGCTATGCCTGACCTTGTTTGTGGAGGAATGCTTG
AGGAGAAGAGTCACTAGGAAACACATGATATTGGTTGTGGTAATCACCTTTT
Sequence number 1675: Dengue virus 4 Whole genome #60 
AATGCTTGAGGAGAAGAGTCACTAGGAAACACATGATATTGGTTGTGGTAATCACCTTTTGTGCTATC
ATCCTAGGAGGTCTTACATGGATGGATTTACTACGAGCCCTTATCATGTTAG
Sequence number 1676: Dengue virus 4 Whole genome #61 
GTGCTATCATCCTAGGAGGTCTTACATGGATGGATTTACTACGAGCCCTTATCATGTTAGGGGATACT
ATGTCTGGTAGAATAGGAGGACAGATTCACCTAGCCATCATGGCAGTGTTCA
Sequence number 1677: Dengue virus 4 Whole genome #62 
GGGATACTATGTCTGGTAGAATAGGAGGACAGATTCACCTAGCCATCATGGCAGTGTTCAAGATGTCA
CCAGGATATGTGCTGGGTGTGTTTTTAAGGAAACTCACTTCAAGAGAGACAG
Sequence number 1678: Dengue virus 4 Whole genome #63 
AGATGTCACCAGGATATGTGCTGGGTGTGTTTTTAAGGAAACTCACTTCAAGAGAGACAGCACTAATG
GTAATAGGAATGGCCATGACAACGGTGTTTTCAATCCCACATGACCTCATGG
Sequence number 1679: Dengue virus 4 Whole genome #64 
CACTAATGGTAATAGGAATGGCCATGACAACGGTGTTTTCAATCCCACATGACCTCATGGAACTCATT
GATGGAATATCATTGGGGTTGATATTACTAAAAATAGTAACACATTTTGATA
Sequence number 1680: Dengue virus 4 Whole genome #65 
AACTCATTGATGGAATATCATTGGGGTTGATATTACTAAAAATAGTAACACATTTTGATAACACCCAA
GTGGGAACCCTAGCCCTTTCCTTGACTTTTTTAAGATCAACAATACCATTAG
Sequence number 1681: Dengue virus 4 Whole genome #66 
ACACCCAAGTGGGAACCCTAGCCCTTTCCTTGACTTTTTTAAGATCAACAATACCATTAGTCATGGCT
TGGAGGACCATCATGGCTGTGTTCTTTGTGGTCACACTCATTCCTTTGTGCA
Sequence number 1682: Dengue virus 4 Whole genome #67 
TCATGGCTTGGAGGACCATCATGGCTGTGTTCTTTGTGGTCACACTCATTCCTTTGTGCAGGACAAGC
TGTCTCCAAAAACAGTCCCATTGGGTAGAAATAACAGCACTCATCCTGGGAG
Sequence number 1683: Dengue virus 4 Whole genome #68 GGACAAGCTGTCTCCAAAAACAGTCCCATTGGGTAGAAATAACAGCACTCATCCTGGGAGCCCAGGCT TTGCCAGTGTACCTAATGACTCTCATGAAAGGAGCCTCAAGGAGATCTTGGC
Sequence number 1684: Dengue virus 4 Whole genome #69 
CCCAGGCTTTGCCAGTGTACCTAATGACTCTCATGAAAGGAGCCTCAAGGAGATCTTGGCCTCTTAAT
GAGGGCATAATGGCAGTAGGTTTGGTAAGCCTCTTAGGAAGCGCCCTCTTGA
Sequence number 1685: Dengue virus 4 Whole genome #70 
CTCTTAATGAGGGCATAATGGCAGTAGGTTTGGTAAGCCTCTTAGGAAGCGCCCTCTTGAAGAATGAT
GTTCCTTTGGCTGGCCCAATGGTGGCAGGAGGCTTGCTTCTAGCGGCTTACG
Sequence number 1686: Dengue virus 4 Whole genome #71 
AGAATGATGTTCCTTTGGCTGGCCCAATGGTGGCAGGAGGCTTGCTTCTAGCGGCTTACGTAATGAGT
GGTAGCTCAGCAGACCTGTCACTAGAGAAAGCTGCCAACGTGCAGTGGGATG
Sequence number 1687: Dengue virus 4 Whole genome #72 
TAATGAGTGGTAGCTCAGCAGACCTGTCACTAGAGAAAGCTGCCAACGTGCAGTGGGATGAAATGGCG
GACATAACTGGCTCAAGCCCAATCATAGAAGTGAAGCAGGATGAAGATGGCT
Sequence number 1688: Dengue virus 4 Whole genome #73 
AAATGGCGGACATAACTGGCTCAAGCCCAATCATAGAAGTGAAGCAGGATGAAGATGGCTCTTTCTCC
ATACGGGACGTCGAGGAAACCAATATGATAACTCTCTTGGTGAAACTGGCAC
Sequence number 1689: Dengue virus 4 Whole genome #74 
CTTTCTCCATACGGGACGTCGAGGAAACCAATATGATAACTCTCTTGGTGAAACTGGCACTGATAACA
GTATCAGGTCTTTACCCCTTGGCAATTCCAGTCACAATGGCATTATGGTATA
Sequence number 1690: Dengue virus 4 Whole genome #75 
TGATAACAGTATCAGGTCTTTACCCCTTGGCAATTCCAGTCACAATGGCATTATGGTATATTTGGCAA
GTGAAGACACAAAGATCAGGAGCTCTGTGGGACGTCCCCTCACCCGCTGCCA
Sequence number 1691: Dengue virus 4 Whole genome #76 
TTTGGCAAGTGAAGACACAAAGATCAGGAGCTCTGTGGGACGTCCCCTCACCCGCTGCCACCCAGAAA
GCCACACTGTCTGAAGGAGTGTACAGGATCATGCAAAGAGGGCTGTTTGGGA
Sequence number 1692: Dengue virus 4 Whole genome #77 
CCCAGAAAGCCACACTGTCTGAAGGAGTGTACAGGATCATGCAAAGAGGGCTGTTTGGGAAGACTCAG
GTTGGAGTAGGAATACACATGGAAGGCGTATTTCACACAATGTGGCATGTGA
Sequence number 1693: Dengue virus 4 Whole genome #78 
AGACTCAGGTTGGAGTAGGAATACACATGGAAGGCGTATTTCACACAATGTGGCATGTGACAAGAGGA
TCAGTGATCTGTCATGAGACAGGGAGATTAGAGCCATCTTGGGCTGACGTCA
Sequence number 1694: Dengue virus 4 Whole genome #79 
CAAGAGGATCAGTGATCTGTCATGAGACAGGGAGATTAGAGCCATCTTGGGCTGACGTCAGGAGCGAC
ATGATATCATACGGTGGGGGATGGAGGCTTGGAGACAAATGGGACAAAGAAG
Sequence number 1695: Dengue virus 4 Whole genome #80 
GGAGCGACATGATATCATACGGTGGGGGATGGAGGCTTGGAGACAAATGGGACAAAGAAGAAGATGTT
CAGGTCTTAGCCATAGAACCAGGGAAAAATCCCAAACATGTCCAAACGAAAC
Sequence number 1696: Dengue virus 4 Whole genome #81 
AAGATGTTCAGGTCTTAGCCATAGAACCAGGGAAAAATCCCAAACATGTCCAAACGAAACCTGGCCTT
TTCAAGACCCTAACTGGAGAAATTGGAGCAGTAACTCTGGATTTCAAACCCG
Sequence number 1697: Dengue virus 4 Whole genome #82 
CTGGCCTTTTCAAGACCCTAACTGGAGAAATTGGAGCAGTAACTCTGGATTTCAAACCCGGAACGTCT
GGCTCTCCTATCATTAACAAGAAAGGGAAAGTTATTGGACTCTATGGAAATG
Sequence number 1698: Dengue virus 4 Whole genome #83 
GAACGTCTGGCTCTCCTATCATTAACAAGAAAGGGAAAGTTATTGGACTCTATGGAAATGGAGTAGTT
ACCAAATCAGGTGATTATGTCAGTGCCATAACGCAAGCCGAAAAAATTGGTG
Sequence number 1699: Dengue virus 4 Whole genome #84 
GAGTAGTTACCAAATCAGGTGATTATGTCAGTGCCATAACGCAAGCCGAAAAAATTGGTGAGCCAGAT
TATGAAGTAGATGAGGACATTTTTCGAAAAAAAAGAAAAACCATAATGGATT
Sequence number 1700: Dengue virus 4 Whole genome #85 
AGCCAGATTATGAAGTAGATGAGGACATTTTTCGAAAAAAAAGAAAAACCATAATGGATTTACACCCC
GGAGCTGGAAAGACAAAAAGAATTCTCCCATCTATAGTCAGAGAGGCTTTAA
Sequence number 1701: Dengue virus 4 Whole genome #86 
TACACCCCGGAGCTGGAAAGACAAAAAGAATTCTCCCATCTATAGTCAGAGAGGCTTTAAAAAGGAGG
CTGCGAACACTGATTTTGGCTCCCACGAGAGTGGTGGCGGCCGAGATGGAAG
Sequence number 1702: Dengue virus 4 Whole genome #87 
AAAGGAGGCTGCGAACACTGATTTTGGCTCCCACGAGAGTGGTGGCGGCCGAGATGGAAGAGGCACTA
CGTGGATTGCCAATCCGTTATCAGACTCCAGCTGTGAAATCAGAGCACACAG
Sequence number 1703: Dengue virus 4 Whole genome #88 
AGGCACTACGTGGATTGCCAATCCGTTATCAGACTCCAGCTGTGAAATCAGAGCACACAGGAAGAGAG
ATTGTAGACCTCATGTGTCATGCAACCTTCACAACAAGACTTTTGTCATCGA
Sequence number 1704: Dengue virus 4 Whole genome #89 
GAAGAGAGATTGTAGACCTCATGTGTCATGCAACCTTCACAACAAGACTTTTGTCATCGACCAGAGTT
CCAGCTTGCAACCTCATAGTGATGGATGAAGCACATTTCACTGACCCTTGCA
Sequence number 1705: Dengue virus 4 Whole genome #90 
CCAGAGTTCCAGCTTGCAACCTCATAGTGATGGATGAAGCACATTTCACTGACCCTTGCAGTGTTGCG
GCTAGAGGATACATTTCAACCAGGGTGGAAATGGGAGAGGCAGCAGCTATCT
Sequence number 1706: Dengue virus 4 Whole genome #91 
GTGTTGCGGCTAGAGGATACATTTCAACCAGGGTGGAAATGGGAGAGGCAGCAGCTATCTTCATGACT
GCAACCCCTCCTGGAGCGACAGATCCCTTCCCCCAGAGTAACAGCCCAATAG
Sequence number 1707: Dengue virus 4 Whole genome #92 
TCATGACTGCAACCCCTCCTGGAGCGACAGATCCCTTCCCCCAGAGTAACAGCCCAATAGAAGACATT
GAGAGGGAAATTCCAGAAAGGTCATGGAATACAGGGTTCGACTGGATAACAG
Sequence number 1708: Dengue virus 4 Whole genome #93 
AAGACATTGAGAGGGAAATTCCAGAAAGGTCATGGAATACAGGGTTCGACTGGATAACAGACTACCAA
GGGAAAACTGTGTGGTTTGTTCCCAGCATAAAAGCTGGAAATGACATTGCAA
Sequence number 1709: Dengue virus 4 Whole genome #94 
ACTACCAAGGGAAAACTGTGTGGTTTGTTCCCAGCATAAAAGCTGGAAATGACATTGCAAATTGTTTG
AGAAAGTCGGGAAAGAGAGTGATCCAGATGCTGTGGTGGTCCTTTGACACAG
Sequence number 1710: Dengue virus 4 Whole genome #95 
ATTGTTTGAGAAAGTCGGGAAAGAGAGTGATCCAGATGCTGTGGTGGTCCTTTGACACAGAGTATCCA
AAGACGAAACTCACGGACTGGGATTTTGTGGTCACCACAGACATATCTGAAA
Sequence number 1711: Dengue virus 4 Whole genome #96 AGTATCCAAAGACGAAACTCACGGACTGGGATTTTGTGGTCACCACAGACATATCTGAAATGGGGGCC AATTTCAGAGCTGGGAGAGTGATAGACCCCAGGAGATGCCTCAAGCCAGTTA
Sequence number 1712: Dengue virus 4 Whole genome #97 
TGGGGGCCAATTTCAGAGCTGGGAGAGTGATAGACCCCAGGAGATGCCTCAAGCCAGTTATCCTAACA
GATGGGCCAGAGAGAGTTATTCTAGCAGGTCCAATCCCAGTAACTCCAGCAA
Sequence number 1713: Dengue virus 4 Whole genome #98 
TCCTAACAGATGGGCCAGAGAGAGTTATTCTAGCAGGTCCAATCCCAGTAACTCCAGCAAGTGCCGCT
CAGAGAAGAGGGCGAATAGGTAGGAATCCAGCACAGGAAGATGACCAATATG
Sequence number 1714: Dengue virus 4 Whole genome #99 
GTGCCGCTCAGAGAAGAGGGCGAATAGGTAGGAATCCAGCACAGGAAGATGACCAATATGTTTTCTCC
GGAGACCCACTAAAAAATGATGAAGATCATGCTCACTGGACAGAAGCAAAGA
Sequence number 1715: Dengue virus 4 Whole genome #100 
TTTTCTCCGGAGACCCACTAAAAAATGATGAAGATCATGCTCACTGGACAGAAGCAAAGATGCTGCTT
GACAACATCTACACCCCTGAAGGGATAATTCCAACACTGTTTGGTCCGGAAA
Sequence number 1716: Dengue virus 4 Whole genome #101 
TGCTGCTTGACAACATCTACACCCCTGAAGGGATAATTCCAACACTGTTTGGTCCGGAAAGGGAAAAA
ACCCAAGCCATTGATGGAGAGTTTCGCCTCAGAGGGAACAAAAGGAAGACTT
Sequence number 1717: Dengue virus 4 Whole genome #102 
GGGAAAAAACCCAAGCCATTGATGGAGAGTTTCGCCTCAGAGGGAACAAAAGGAAGACTTTTGTGGAA
TTAATGAGGAGAGGAGACCTTCCGGTGTGGCTGAGCTACAAGGTAGCTTCTG
Sequence number 1718: Dengue virus 4 Whole genome #103 
TTGTGGAATTAATGAGGAGAGGAGACCTTCCGGTGTGGCTGAGCTACAAGGTAGCTTCTGCCGGTATC
TCTTACAAAGATCGAGAATGGTGCTTCACAGGAGAAAGGAATAACCAAATTT
Sequence number 1719: Dengue virus 4 Whole genome #104 
CCGGTATCTCTTACAAAGATCGAGAATGGTGCTTCACAGGAGAAAGGAATAACCAAATTTTAGAAGAA
AACATGGAGGTTGAAATTTGGACCAAAGAGGGAGAAAAGAAAAAGCTAAGGC
Sequence number 1720: Dengue virus 4 Whole genome #105 
TAGAAGAAAACATGGAGGTTGAAATTTGGACCAAAGAGGGAGAAAAGAAAAAGCTAAGGCCAAAATGG
TTAGATGCACGTGTGTACGCTGATCCCATGGCTTTGAAAGATTTCAAGGAGT
Sequence number 1721: Dengue virus 4 Whole genome #106 
CAAAATGGTTAGATGCACGTGTGTACGCTGATCCCATGGCTTTGAAAGATTTCAAGGAGTTTGCCAGT
GGAAGAAAGAGCATAACTCTCGACATTCTAACAGAGATTGCCAGTCTGCCAA
Sequence number 1722: Dengue virus 4 Whole genome #107 
TTGCCAGTGGAAGAAAGAGCATAACTCTCGACATTCTAACAGAGATTGCCAGTCTGCCAACTTACCTT
TCCTCTAAGGCCAAGCTGGCCCTTGACAACATAGTCATGCTCCACACAACAG
Sequence number 1723: Dengue virus 4 Whole genome #108 
CTTACCTTTCCTCTAAGGCCAAGCTGGCCCTTGACAACATAGTCATGCTCCACACAACAGAAAAAGGA
GGGAGGGCCTACCAACACGCCCTGAATGAACTCCCGGAGTCACTGGAAACAC
Sequence number 1724: Dengue virus 4 Whole genome #109 
AAAAAGGAGGGAGGGCCTACCAACACGCCCTGAATGAACTCCCGGAGTCACTGGAAACACTCATGCTT
GTAGCTCTACTAGGAGCCATGGGATCAGGCATCTTCCTGTTTTTCATGCAGG
Sequence number 1725: Dengue virus 4 Whole genome #110 
TCATGCTTGTAGCTCTACTAGGAGCCATGGGATCAGGCATCTTCCTGTTTTTCATGCAGGGGAAAGGA
ATAGGAAAATTGTCAATGGGTCTAATAGCTATAGCTGTGGCTAGTGGCTTGC
Sequence number 1726: Dengue virus 4 Whole genome #111 
GGAAAGGAATAGGAAAATTGTCAATGGGTCTAATAGCTATAGCTGTGGCTAGTGGCTTGCTCTGGGTA
GCAGAAATTCAGCCCCAGTGGATAGCAGCTTCAATCATACTGGAGTTCTTTC
Sequence number 1727: Dengue virus 4 Whole genome #112 
TCTGGGTAGCAGAAATTCAGCCCCAGTGGATAGCAGCTTCAATCATACTGGAGTTCTTTCTGATGGTG
CTGTTGATACCAGAACCAGAAAAACAGAGGACCCCACAAGACAATCAATTTA
Sequence number 1728: Dengue virus 4 Whole genome #113 
TGATGGTGCTGTTGATACCAGAACCAGAAAAACAGAGGACCCCACAAGACAATCAATTTATCTATGTC
ATATTGACCATTCTCACCATCATCGGTCTTATAGCAGCCAATGAGATGGGGC
Sequence number 1729: Dengue virus 4 Whole genome #114 
TCTATGTCATATTGACCATTCTCACCATCATCGGTCTTATAGCAGCCAATGAGATGGGGCTGATTGAA
AAAACAAAAACTGACTTTGGGTTTTACCAGGTAAAAACAGAAACCACTATTC
Sequence number 1730: Dengue virus 4 Whole genome #115 
TGATTGAAAAAACAAAAACTGACTTTGGGTTTTACCAGGTAAAAACAGAAACCACTATTCTTGATGTG
GATTTGAGACCAGCCTCAGCATGGACACTCTATGCGGTAGCCACCACCATTC
Sequence number 1731: Dengue virus 4 Whole genome #116 
TTGATGTGGATTTGAGACCAGCCTCAGCATGGACACTCTATGCGGTAGCCACCACCATTCTGACTCCC
ATGCTGAGACACACCATAGAAAACACGTCTGCCAACCTATCTCTAGCAGCCA
Sequence number 1732: Dengue virus 4 Whole genome #117 
TGACTCCCATGCTGAGACACACCATAGAAAACACGTCTGCCAACCTATCTCTAGCAGCCATTGCCAAT
CAAGCAGCTGTCCTAATGGGACTTGGAAAAGGATGGCCGCTCCACAGAATGG
Sequence number 1733: Dengue virus 4 Whole genome #118 
TTGCCAATCAAGCAGCTGTCCTAATGGGACTTGGAAAAGGATGGCCGCTCCACAGAATGGACCTTGGT
GTGCCGCTGTTAGCAATAGGATGTTATTCTCAAGTGAACCCAACAACCTCGA
Sequence number 1734: Dengue virus 4 Whole genome #119 
ACCTTGGTGTGCCGCTGTTAGCAATAGGATGTTATTCTCAAGTGAACCCAACAACCTCGACAGCATCT
TTAGTCAGTCTTTTACGCCATTATGCCATAATAGGTCCGGGATTGCAGGCAA
Sequence number 1735: Dengue virus 4 Whole genome #120 
CAGCATCTTTAGTCAGTCTTTTACGCCATTATGCCATAATAGGTCCGGGATTGCAGGCAAAAGCCACA
CGAGAGGCCCAGAAAAGGACAGCTGCTGGGATCATGAAGAACCCCACGGTGG
Sequence number 1736: Dengue virus 4 Whole genome #121 
AAGCCACACGAGAGGCCCAGAAAAGGACAGCTGCTGGGATCATGAAGAACCCCACGGTGGACGGGATA
ACAGTAATAGATCTAGAACCAATATCCTATGACCCAAAATTTGAAAAGCAAT
Sequence number 1737: Dengue virus 4 Whole genome #122 
ACGGGATAACAGTAATAGATCTAGAACCAATATCCTATGACCCAAAATTTGAAAAGCAATTAGGGCAA
GTCATGCTACTAGTCCTGTGTGTTGGACAATTACTTTTGATGAGAACAACAT
Sequence number 1738: Dengue virus 4 Whole genome #123 
TAGGGCAAGTCATGCTACTAGTCCTGTGTGTTGGACAATTACTTTTGATGAGAACAACATGGGCTCTC
TGCGAAGTCTTGACTTTGGCCACAGGACCAGCCTTGACCTTGTGGGAGGGCA
Sequence number 1739: Dengue virus 4 Whole genome #124 GGGCTCTCTGCGAAGTCTTGACTTTGGCCACAGGACCAGCCTTGACCTTGTGGGAGGGCAGGGGGGCA ACCCGGGGGAGGAAGACCATAGCTGTATCCACAGCCAACATTTTCAGAGGAA
Sequence number 1740: Dengue virus 4 Whole genome #125 
GGGGGGCAACCCGGGGGAGGAAGACCATAGCTGTATCCACAGCCAACATTTTCAGAGGAAGCTACTTG
GCAGGAGCTGGACTAGCTTTTTCGCTCATAAAGAATGCACAAACCCCTAGGA
Sequence number 1741: Dengue virus 4 Whole genome #126 
GCTACTTGGCAGGAGCTGGACTAGCTTTTTCGCTCATAAAGAATGCACAAACCCCTAGGAGGGGAACT
GGGACCACAGGAGAGACACTGGGAGAGAAGTGGAAGAGACAGCTAAACTCAT
Sequence number 1742: Dengue virus 4 Whole genome #127 
GGGGAACTGGGACCACAGGAGAGACACTGGGAGAGAAGTGGAAGAGACAGCTAAACTCATTAGACAGA
AAAGAGTTTGAAGAGTACAAAAGAAGTGGAATACAAGAAGTGGATAGGACTG
Sequence number 1743: Dengue virus 4 Whole genome #128 
TAGACAGAAAAGAGTTTGAAGAGTACAAAAGAAGTGGAATACAAGAAGTGGATAGGACTGAAGCCAAG
TCTGCCCTAAAAGATGGATCTAAAATCAAGCATGCAGTGTCCAGAGGGTCTA
Sequence number 1744: Dengue virus 4 Whole genome #129 
AAGCCAAGTCTGCCCTAAAAGATGGATCTAAAATCAAGCATGCAGTGTCCAGAGGGTCTAGTAAGATC
AGATGGATTGTTGAAAGAGGGATGGTAAAGCCAAAAGGGAAGGTCGTGGATC
Sequence number 1745: Dengue virus 4 Whole genome #130 
GTAAGATCAGATGGATTGTTGAAAGAGGGATGGTAAAGCCAAAAGGGAAGGTCGTGGATCTTGGTTGC
GGGAGGGGAGGATGGTCCTATTACATGGCGACACTCAAGAACGTGACTGAAG
Sequence number 1746: Dengue virus 4 Whole genome #131 
TTGGTTGCGGGAGGGGAGGATGGTCCTATTACATGGCGACACTCAAGAACGTGACTGAAGTGAAAGGA
TATACAAAAGGAGGTCCAGGACATGAGGAACCAATCCCCATGGCTACTTATG
Sequence number 1747: Dengue virus 4 Whole genome #132 
TGAAAGGATATACAAAAGGAGGTCCAGGACATGAGGAACCAATCCCCATGGCTACTTATGGCTGGAAC
TTGGTCAAACTCCATTCAGGGGTTGATGTGTTTTACAAACCCACTGAACAGG
Sequence number 1748: Dengue virus 4 Whole genome #133 
GCTGGAACTTGGTCAAACTCCATTCAGGGGTTGATGTGTTTTACAAACCCACTGAACAGGTGGACACC
TTGCTCTGTGATATTGGGGAGTCGTCCTCCAATCCGACAATAGAGGAAGGAA
Sequence number 1749: Dengue virus 4 Whole genome #134 
TGGACACCTTGCTCTGTGATATTGGGGAGTCGTCCTCCAATCCGACAATAGAGGAAGGAAGAACATTG
AGAGCCTTATCTAAAGAGGAGACATGGCTCTCATCAAAACCTGAGTTCTGCA
Sequence number 1750: Dengue virus 4 Whole genome #135 
GAACATTGAGAGCCTTATCTAAAGAGGAGACATGGCTCTCATCAAAACCTGAGTTCTGCATCAAAATC
CTCAACCCCTACATGCCAACAGTTATAGAAGAGCTGGAGAAACTCCAGAGAA
Sequence number 1751: Dengue virus 4 Whole genome #136 
TCAAAATCCTCAACCCCTACATGCCAACAGTTATAGAAGAGCTGGAGAAACTCCAGAGAAGATATGGT
GGAAGCCTCATCAGATGCCCTCTATCTAGGAATTCTACCCATGAGATGTATT
Sequence number 1752: Dengue virus 4 Whole genome #137 
GATATGGTGGAAGCCTCATCAGATGCCCTCTATCTAGGAATTCTACCCATGAGATGTATTGGGTGTCA
GGTGCGTCGGGAAACATCGTGAGTTCTGTAAACACAGTGTCAAAGATGCTGT
Sequence number 1753: Dengue virus 4 Whole genome #138 
GGGTGTCAGGTGCGTCGGGAAACATCGTGAGTTCTGTAAACACAGTGTCAAAGATGCTGTTGAACAGG
TTCACAACAAGGCATAGGAAACCTACTTATGAGAAGGACGTGGATCTTGGGG
Sequence number 1754: Dengue virus 4 Whole genome #139 
TGAACAGGTTCACAACAAGGCATAGGAAACCTACTTATGAGAAGGACGTGGATCTTGGGGCAGGAACA
AGAAGTGTCTCCACTGAAACAGAAAAACCAGACATGACAGTTATTGGGACAA
Sequence number 1755: Dengue virus 4 Whole genome #140 
CAGGAACAAGAAGTGTCTCCACTGAAACAGAAAAACCAGACATGACAGTTATTGGGACAATGGATTGG
AGATTGCAAGAAGAGCACAAAGAAACCTGGCACTATGACCAGGAAAATCCAT
Sequence number 1756: Dengue virus 4 Whole genome #141 
TGGATTGGAGATTGCAAGAAGAGCACAAAGAAACCTGGCACTATGACCAGGAAAATCCATACAGAACC
TGGGCGTATCATGGAAGCTATGAAGCTCCTTCGACAGGCTCAGCATCCTCCA
Sequence number 1757: Dengue virus 4 Whole genome #142 
ACAGAACCTGGGCGTATCATGGAAGCTATGAAGCTCCTTCGACAGGCTCAGCATCCTCCATGGTGAAC
GGGGTAGTAAAACTGCTGACAAAACCCTGGGATGTGATTCCAATGGTGACCC
Sequence number 1758: Dengue virus 4 Whole genome #143 
TGGTGAACGGGGTAGTAAAACTGCTGACAAAACCCTGGGATGTGATTCCAATGGTGACCCAGCTGGCA
ATGACAGACACAACCCCTTTTGGGCAACAAAGAGTGTTCAAGGAGAAGGTGG
Sequence number 1759: Dengue virus 4 Whole genome #144 
AGCTGGCAATGACAGACACAACCCCTTTTGGGCAACAAAGAGTGTTCAAGGAGAAGGTGGATACCAGA
ACACCACAACCAAAACCCGGCACACGAATGATTATGACCACGACAGCTAATT
Sequence number 1760: Dengue virus 4 Whole genome #145 
ATACCAGAACACCACAACCAAAACCCGGCACACGAATGATTATGACCACGACAGCTAATTGGCTGTGG
GCTCTCCTCGGGAAGAAGAAAAATCCCAGATTGTGTACAAGGGAAGAGTTCA
Sequence number 1761: Dengue virus 4 Whole genome #146 
GGCTGTGGGCTCTCCTCGGGAAGAAGAAAAATCCCAGATTGTGTACAAGGGAAGAGTTCATCTCAAAA
GTTAGGTCAAACGCAGCCATAGGCGCAGTCTTTCAGGAAGAGCAGGGATGGA
Sequence number 1762: Dengue virus 4 Whole genome #147 
TCTCAAAAGTTAGGTCAAACGCAGCCATAGGCGCAGTCTTTCAGGAAGAGCAGGGATGGACATCTGCC
AGTGAAGCTGTGAGTGACAGCCGGTTCTGGGAACTGGTTGACAAAGAAAGGG
Sequence number 1763: Dengue virus 4 Whole genome #148 
CATCTGCCAGTGAAGCTGTGAGTGACAGCCGGTTCTGGGAACTGGTTGACAAAGAAAGGGCTCTGCAC
CAGGAAGGAAAATGTGAATCGTGTGTCTACAACATGATGGGAAAACGTGAGA
Sequence number 1764: Dengue virus 4 Whole genome #149 
CTCTGCACCAGGAAGGAAAATGTGAATCGTGTGTCTACAACATGATGGGAAAACGTGAGAAAAAACTA
GGAGAGTTTGGTAGGGCCAAGGGAAGCCGAGCAATCTGGTACATGTGGCTGG
Sequence number 1765: Dengue virus 4 Whole genome #150 
AAAAACTAGGAGAGTTTGGTAGGGCCAAGGGAAGCCGAGCAATCTGGTACATGTGGCTGGGGGCGCGC
TTCCTGGAATTTGAAGCCCTGGGTTTCTTGAATGAAGATCACTGGTTTAGCA
Sequence number 1766: Dengue virus 4 Whole genome #151 
GGGCGCGCTTCCTGGAATTTGAAGCCCTGGGTTTCTTGAATGAAGATCACTGGTTTAGCAGAGAAAAC
TCATGGAGTGGAGTGGAAGGGGAAGGTCTGCATAGATTGGGATATATCTTGG
Sequence number 1767: Dengue virus 4 Whole genome #152 GAGAAAACTCATGGAGTGGAGTGGAAGGGGAAGGTCTGCATAGATTGGGATATATCTTGGAGGATATA GACAAGCAGGATGGAGATCTAATATATGCTGACGACACAGCAGGCTGGACA
Sequence number 1768: Dengue virus 4 Whole genome #153 
AGGATATAGACAAGCAGGATGGAGATCTAATATATGCTGACGACACAGCAGGCTGGGACACAAGAATC
ACTGAGGATGACCTTTTAAATGAAGAACTGATCACAGAACAGATGGCCCCCC
Sequence number 1769: Dengue virus 4 Whole genome #154 
CAAGAATCACTGAGGATGACCTTTTAAATGAAGAACTGATCACAGAACAGATGGCCCCCCATCACAAG
ACCCTAGCCAAAGCCATATTCAAACTAACCTATCAAAACAAAGTGGTGAAAG
Sequence number 1770: Dengue virus 4 Whole genome #155 
ATCACAAGACCCTAGCCAAAGCCATATTCAAACTAACCTATCAAAACAAAGTGGTGAAAGTTCTCAGA
CCCACACCGAAAGGAGCGGTGATGGACATCATATCCAGGAAAGACCAAAGAG
Sequence number 1771: Dengue virus 4 Whole genome #156 
TTCTCAGACCCACACCGAAAGGAGCGGTGATGGACATCATATCCAGGAAAGACCAAAGAGGTAGTGGA
CAGGTTGGAACATATGGTTTAAACACATTCACTAACATGGAAGTTCAACTCA
Sequence number 1772: Dengue virus 4 Whole genome #157 
GTAGTGGACAGGTTGGAACATATGGTTTAAACACATTCACTAACATGGAAGTTCAACTCATCCGTCAA
ATGGAAGCTGAAGGAGTCATCACACAAGACGACATGCAGAACCCAAAAGGGT
Sequence number 1773: Dengue virus 4 Whole genome #158 
TCCGTCAAATGGAAGCTGAAGGAGTCATCACACAAGACGACATGCAGAACCCAAAAGGGTTGAAAGAA
AGAGTTGAGAAATGGCTGACAGAGTGTGGCGTCGACAGGTTGAAGAGGATGG
Sequence number 1774: Dengue virus 4 Whole genome #159 
TGAAAGAAAGAGTTGAGAAATGGCTGACAGAGTGTGGCGTCGACAGGTTGAAGAGGATGGCAATCAGT
GGAGATGATTGCGTGGTGAAGCCCCTAGATGAGAGGTTCAGCACCTCCCTCC
Sequence number 1775: Dengue virus 4 Whole genome #160 
CAATCAGTGGAGATGATTGCGTGGTGAAGCCCCTAGATGAGAGGTTCAGCACCTCCCTCCTCTTCTTG
AACGACATGGGAAAGGTGAGGAAAGACATCCCGCAGTGGGAACCATCCAAGG
Sequence number 1776: Dengue virus 4 Whole genome #161 
TCTTCTTGAACGACATGGGAAAGGTGAGGAAAGACATCCCGCAGTGGGAACCATCCAAGGGATGGAAA
AACTGGCAAGAGGTTCCTTTTTGCTCCCACCATTTTCATAAGATCTTTATGA
Sequence number 1777: Dengue virus 4 Whole genome #162 
GATGGAAAAACTGGCAAGAGGTTCCTTTTTGCTCCCACCATTTTCATAAGATCTTTATGAAAGATGGC
CGCTCACTAGTTGTTCCATGCAGAAACCAGGATGAACTGATAGGAAGAGCCA
Sequence number 1778: Dengue virus 4 Whole genome #163 
AAGATGGCCGCTCACTAGTTGTTCCATGCAGAAACCAGGATGAACTGATAGGAAGAGCCAGAATCTCG
CAGGGGGCTGGATGGAGCCTAAGAGAAACAGCCTGTTCGGGCAAAGCTTACG
Sequence number 1779: Dengue virus 4 Whole genome #164 
GAATCTCGCAGGGGGCTGGATGGAGCCTAAGAGAAACAGCCTGTTCGGGCAAAGCTTACGCCCAAATG
TGGTCGCTTATGTATTTCCATAGAAGGGACCTGCGTTTGGCCTCCATGGCCA
Sequence number 1780: Dengue virus 4 Whole genome #165 
CCCAAATGTGGTCGCTTATGTATTTCCATAGAAGGGACCTGCGTTTGGCCTCCATGGCCATATGTTCA
GCAGTTCCAACGGAATGGTTTCCAACAAGCAGGACAACATGGTCAATCCACG
Sequence number 1781: Dengue virus 4 Whole genome #166 
TATGTTCAGCAGTTCCAACGGAATGGTTTCCAACAAGCAGGACAACATGGTCAATCCACGCTCACCAC
CAGTGGATGACCACTGAAGACATGCTCAAAGTGTGGAACAGGGTGTGGATAG
Sequence number 1782: Dengue virus 4 Whole genome #167 
CTCACCACCAGTGGATGACCACTGAAGACATGCTCAAAGTGTGGAACAGGGTGTGGATAGAAGACAAC
CCCAATATGACTGACAAGACTCCAGTCCGTTCGTGGGAGGACATACCTTACC
Sequence number 1783: Dengue virus 4 Whole genome #168 
AAGACAACCCCAATATGACTGACAAGACTCCAGTCCGTTCGTGGGAGGACATACCTTACCTAGGGAAA
AGAGAGGATCTGTGGTGTGGATCCCTGATTGGACTCTCTTCCAGAGCCACCT
Sequence number 1784: Dengue virus 4 Whole genome #169 
TAGGGAAAAGAGAGGATCTGTGGTGTGGATCCCTGATTGGACTCTCTTCCAGAGCCACCTGGGCGAAG
AACATTCACACGGCCATAACCCAGGTCAGGAATCTGATCGGAAAAGAGGAAT
Sequence number 1785: Dengue virus 4 Whole genome #170 
GGGCGAAGAACATTCACACGGCCATAACCCAGGTCAGGAATCTGATCGGAAAAGAGGAATACGTGGAT
TACATGCCAGTCATGAGAAGATACAGCGCTCCTTCCGAAAGTGAAGGAGTTC
Sequence number 1786: Dengue virus 4 Whole genome #171 
ACGTGGATTACATGCCAGTCATGAGAAGATACAGCGCTCCTTCCGAAAGTGAAGGAGTTCTGTAATTA
CTAATAACAAACACCAAAGAGACCATTGAAGTCAGGCCACTTGTGCCACGGC
Sequence number 1787: Dengue virus 4 Whole genome #172 
TGTAATTACTAATAACAAACACCAAAGAGACCATTGAAGTCAGGCCACTTGTGCCACGGCTTGAGCAA
ACCGTGCTGCCTGTAGCTCCGCCAATAATGGGAGGCGTAAAATTCCCAGGGA
Sequence number 1788: Dengue virus 4 Whole genome #173 
TTGAGCAAACCGTGCTGCCTGTAGCTCCGCCAATAATGGGAGGCGTAAAATTCCCAGGGAGGCCATGC
GCCACGGAAGCTGTACGCGTGGCATATTGGACTAGCGGTTAGAGGAGACCCC
Sequence number 1789: Dengue virus 4 Whole genome #174 
GGCCATGCGCCACGGAAGCTGTACGCGTGGCATATTGGACTAGCGGTTAGAGGAGACCCCTCCCATCA
CCGACAAAACGCAGCAAAAAAGGGGGCCCGAAGCCAGGAGGAAGCTGTACTT
Sequence number 1790: Dengue virus 4 Whole genome #175 
TCCCATCACCGACAAAACGCAGCAAAAAGGGGCCCGAAGCCAGGAGGAAGCTGTACTTCTGGTAGA
AGGACTAGAGGTTAGAGGAGACCCCCCCAACACAAAAACAGCATATTGACGC
Sequence number 1791: Dengue virus 4 Whole genome #176 
CTGGTAGAAGGACTAGAGGTTAGAGGAGACCCCCCCAACACAAAAACAGCATATTGACGCTGGGACAG
ACCAGAGATCCTGCTGTCTCTGCAACATCAATCCAGGCACAGAGCGCCGCGA
Sequence number 1792: Dengue virus 4 Whole genome #177 
CCCCAACACAAAAACAGCATATTGACGCTGGGACAGACCAGAGATCCTGCTGTCTCTGCAACATCAAT
CCAGGCACAGAGCGCCGCGAGATGGATTGGTGTTGTTGATCCAACAGGTTCT
Sequence number 1793: Ehrlichia chaffeensis 120 kDa protein gene #1 
TTTCCATACAGCCTCTGTAAATAGTTGTGTGGGGACTGAGAATATGTGAGAATTGTCTTGTATAAATT
CTTAATTACTGTACTGTAATAAAAAATATCGTATAAGTTGTTGTTCAGTATA
Sequence number 1794: Ehrlichia chaffeensis 120 kDa protein gene #2 
TATAAATTCTTAATTACTGTACTGTAATAAAAAATATCGTATAAGTTGTTGTTCAGTATAAAATTGTG
TCATAGTCACAATTTAGAAAAATGCAGCTTGATACATCATGAACATTTAGGT
Sequence number 1795: Ehrlichia chaffeensis 120 kDa protein gene #3 AAATTGTGTCATAGTCACAATTTAGAAAAATGCAGCTTGATACATCATGAACATTTAGGTCGGTATAG ATTTGCTAATAGCAAGTTTATATTACATAGAAAGCGTAATGCTATTCACTTA
Sequence number 1796: Ehrlichia chaffeensis 120 kDa protein gene #4 
CGGTATAGATTTGCTAATAGCAAGTTTATATTACATAGAAAGCGTAATGCTATTCACTTAAATTTTCA
TCAAAATAAATAAACTTATTTTTTGTGTACTTGTGGGATTCTGCGTCTGAAT
Sequence number 1797: Ehrlichia chaffeensis 120 kDa protein gene #5 
AATTTTCATCAAAATAAATAAACTTATTTTTTGTGTACTTGTGGGATTCTGCGTCTGAATATGGAGGT
TTAGCAGAAGTTATGTTGTTCCATTCTTTAGAGAATTTTTTATTTATTTCAT
Sequence number 1798: Ehrlichia chaffeensis 120 kDa protein gene #6 
ATGGAGGTTTAGCAGAAGTTATGTTGTTCCATTCTTTAGAGAATTTTTTATTTATTTCATAAAACTTT
TTAAGGTTTTTCTGTTCTTCGTTTAATGCGCTATCATTGCATTCTAATACGT
Sequence number 1799: Ehrlichia chaffeensis 120 kDa protein gene #7 
AAAACTTTTTAAGGTTTTTCTGTTCTTCGTTTAATGCGCTATCATTGCATTCTAATACGTCTCTTATA
AAATCATCTGGGACGATTGCATCGATGGGACATTCTGGGACACACACACCAC
Sequence number 1800: Ehrlichia chaffeensis 120 kDa protein gene #8 
CTCTTATAAAATCATCTGGGACGATTGCATCGATGGGACATTCTGGGACACACACACCACAGTCTATA
CATTGATCAGGATCTATTACTAACATATTAGCTCCTTCATAGAAACAATCTA
Sequence number 1801: Ehrlichia chaffeensis 120 kDa protein gene #9 
AGTCTATACATTGATCAGGATCTATTACTAACATATTAGCTCCTTCATAGAAACAATCTACCGGGCAT
ACTTCAACACAATCAGTGTATTTGCATCTTATGCACCTATCCGTTATAAAGT
Sequence number 1802: Ehrlichia chaffeensis 120 kDa protein gene #10 
*CCGGGCATACTTCAACACAATCAGTGTATTTGCATCTTATGCACCTATCCGTTATAAAGTGTGTCAT
TACAGGGTTATTAATAATAATGTAACGGTTTTTATTGTAATGTTTTTTCTTGC
Sequence number 1803: Ehrlichia chaffeensis 120 kDa protein gene #11 
GTGTCATTACAGGGTTATTAATAATAATGTAACGGTTTTTATTGTAATGTTTTTTCTTGCCAAGTTCA
ATTAGTATTTATTATTTTACAAGGTATAGATGTGGAGTTGTAGTTAAACTTA
Sequence number 1804: Ehrlichia chaffeensis 120 kDa protein gene #12 CAAGTTCAATTAGTATTTATTATTTTACAAGGTATAGATGTGGAGTTGTAGTTAAACTTATACATCGT AGAGTTAAGTAGTTTGGTTAATGTTTAGGTAACATCCTAATACGTATATGAG
Sequence number 1805: Ehrlichia chaffeensis 120 kDa protein gene #13 
TACATCGTAGAGTTAAGTAGTTTGGTTAATGTTTAGGTAACATCCTAATACGTATATGAGCTATCAAT
TCTATAGAGTATGTTATTTTATGATAGAGAATTGATTGTGGAGTTGGATTTG
Sequence number 1806: Ehrlichia chaffeensis 120 kDa protein gene #14 
CTATCAATTCTATAGAGTATGTTATTTTATGATAGAGAATTGATTGTGGAGTTGGATTTGGCAATACG
TTTAAAATTAAAGGAGATTTTTATGGATATTGATAATAGTAACATAAGTACA
Sequence number 1807: Ehrlichia chaffeensis 120 kDa protein gene #15 
GCAATACGTTTAAAATTAAAGGAGATTTTTATGGATATTGATAATAGTAACATAAGTACAGCCGATAT
ACGGAGTAATACTGATGGCTTGATAGACATAATTATGCGTATATTAGGTTTT
Sequence number 1808: Ehrlichia chaffeensis 120 kDa protein gene #16 
GCCGATATACGGAGTAATACTGATGGCTTGATAGACATAATTATGCGTATATTAGGTTTTGGTAATAA
GAATATTGTGCAACCACAGGATCTGGGTTCTGAAATTTATCAGCAAGAGCAA
Sequence number 1809: Ehrlichia chaffeensis 120 kDa protein gene #17 
GGTAATAAGAATATTGTGCAACCACAGGATCTGGGTTCTGAAATTTATCAGCAAGAGCAAGAAGATGA
CACAGTCTCTCAACCTTCATTAGAGCCATTTGTTGCAGAAAGTGAAGTTTCT
Sequence number 1810: Ehrlichia chaffeensis 120 kDa protein gene #18 
GAAGATGACACAGTCTCTCAACCTTCATTAGAGCCATTTGTTGCAGAAAGTGAAGTTTCTAAAGTTGA
ACAAGAAAAAACTAACCCTGAGGTTTTAATAAAAGATTTGCAAGATGTTGCG
Sequence number 1811: Ehrlichia chaffeensis 120 kDa protein gene #19 
AAAGTTGAACAAGAAAAAACTAACCCTGAGGTTTTAATAAAAGATTTGCAAGATGTTGCGAGTCATGA
ATCTGGTGTATCAGATCAGCCAGCTCAAGTTGTTACAGAAAGAGAAAATGAA
Sequence number 1812: Ehrlichia chaffeensis 120 kDa protein gene #20 
AGTCATGAATCTGGTGTATCAGATCAGCCAGCTCAAGTTGTTACAGAAAGAGAAAATGAAATTGAATC
CCATCAAGGAGAAACAGAAAAAGAAAGTGGAATAACTGAATCTCATCAGAAA
Sequence number 1813: Ehrlichia chaffeensis 120 kDa protein gene #21 
ATTGAATCCCATCAAGGAGAAACAGAAAAAGAAAGTGGAATAACTGAATCTCATCAGAAAGAAGATGA
AATAGTATCTCAATCTTCATCAGAGCCATTTGTTGCAGAAAGTGAAGTTTCT
Sequence number 1814: Ehrlichia chaffeensis 120 kDa protein gene #22 
GAAGATGAAATAGTATCTCAATCTTCATCAGAGCCATTTGTTGCAGAAAGTGAAGTTTCTAAAGTTGA
ACAAGAAGAAACTAACCCTGAAGTTTTAATAAAAGATTTGCAAGATGTTGCG
Sequence number 1815: Ehrlichia chaffeensis 120 kDa protein gene #23 
AAAGTTGAACAAGAAGAAACTAACCCTGAAGTTTTAATAAAAGATTTGCAAGATGTTGCGAGTCATGA
ATCTGGTGTATCAGATCAGCCAGCTCAAGTTGTTACAGAGAGAGAAAGTGAA
Sequence number 1816: Ehrlichia chaffeensis 120 kDa protein gene #24 
AGTCATGAATCTGGTGTATCAGATCAGCCAGCTCAAGTTGTTACAGAGAGAGAAAGTGAAATTGAATC
CCATCAAGGAGAAACAGAAAAAGAAAGTGGAATAACTGAATCTCATCAGAAA
Sequence number 1817: Ehrlichia chaffeensis 120 kDa protein gene #25 
ATTGAATCCCATCAAGGAGAAACAGAAAAAGAAAGTGGAATAACTGAATCTCATCAGAAAGAAGATGA
AATAGTATCTCAACCTTCATCAGAGCCATTTGTTGCAGAAAGTGAAGTTTCT
Sequence number 1818: Ehrlichia chaffeensis 120 kDa protein gene #26 
GAAGATGAAATAGTATCTCAACCTTCATCAGAGCCATTTGTTGCAGAAAGTGAAGTTTCTAAAGTTGA
ACAAGAAGAAACTAACCCTGAAGTTTTAATAAAAGATTTGCAAGATGTTGCG
Sequence number 1819: Ehrlichia chaffeensis 120 kDa protein gene #27 
GAAGATGAAATAGTATCTCAACCTTCATCAGAGCCATTTGTTGCAGAAAGTGAAGTTTCTAAAGTTGA
ACAAGAAAAAACTAACCCTGAAATTCTAGTAGAAGATTTGCCATTAGGTCAA
Sequence number 1820: Ehrlichia chaffeensis 120 kDa protein gene #28 
AAAGTTGAACAAGAAAAAACTAACCCTGAAATTCTAGTAGAAGATTTGCCATTAGGTCAAGTGATTCC
GGTTGTTGTAGAGAAAGATGAAATGTTTGCACCTTCATTTAATCCAATCGTT
Sequence number 1821: Ehrlichia chaffeensis 120 kDa protein gene #29 
GTGATTCCGGTTGTTGTAGAGAAAGATGAAATGTTTGCACCTTCATTTAATCCAATCGTTATAAAGGA
GGAAGATAAAGTTTGTGAAACTTGCGAACAAGAATTTGAGATTGTAAAGGAT
Sequence number 1822: Ehrlichia chaffeensis 120 kDa protein gene #30 
ATAAAGGAGGAAGATAAAGTTTGTGAAACTTGCGAACAAGAATTTGAGATTGTAAAGGATTCACAGAC
TGTAAAAGGTAGTGAAGATATAATATCACCTATCGAATGCTTAGAAAGTATG
Sequence number 1823: Ehrlichia chaffeensis 120 kDa protein gene #31 
TCACAGACTGTAAAAGGTAGTGAAGATATAATATCACCTATCGAATGCTTAGAAAGTATGGATTCTAT
AGTTTCAACAATATTTGAAAGTGGAATGTTATGTCCTATGTCAAAACCTGGA
Sequence number 1824: Ehrlichia chaffeensis 120 kDa protein gene #32 
GATTCTATAGTTTCAACAATATTTGAAAGTGGAATGTTATGTCCTATGTCAAAACCTGGACAGTATGT
TTGTGGGTATGAAATGTATATGTATGGATTTCAAGATGTGAAAGACTTATTA
Sequence number 1825: Ehrlichia chaffeensis 120 kDa protein gene #33 
CAGTATGTTTGTGGGTATGAAATGTATATGTATGGATTTCAAGATGTGAAAGACTTATTAGGTGGTTT
ATTAAGTAATGTTCCTGTGTGTTGTAATGTTAGCCTTTATTTTATGGAACAT
Sequence number 1826: Ehrlichia chaffeensis 120 kDa protein gene #34 
GGTGGTTTATTAAGTAATGTTCCTGTGTGTTGTAATGTTAGCCTTTATTTTATGGAACATAATTACTT
TACTAACCATGAGAATATTAATCACAATGTAGTAAATGATATTGTATAATTG
Sequence number 1827: Ehrlichia chaffeensis 120 kDa protein gene #35 
AATTACTTTACTAACCATGAGAATATTAATCACAATGTAGTAAATGATATTGTATAATTGTAAGGTTT
AGTCTTGAGATAGCAAGTGATGCTTTTATTAAGTATTGCTTGCTATTTTTTT
Sequence number 1828: Ehrlichia chaffeensis 120 kDa protein gene #36 
TAAGGTTTAGTCTTGAGATAGCAAGTGATGCTTTTATTAAGTATTGCTTGCTATTTTTTTGTTTATTT
ACCTGCTTTTTATATGGGAGAAATCATATATTAAGTGATTGAAAGTAAAAGT
Sequence number 1829: Ehrlichia chaffeensis 120 kDa protein gene #37 
GTTTATTTACCTGCTTTTTATATGGGAGAAATCATATATTAAGTGATTGAAAGTAAAAGTGGAATTTT
GCTGATATTATAGGTTATAAGCAATTTTTTGAAGTAAAAATGTATATAGTTA
Sequence number 1830: Ehrlichia chaffeensis 120 kDa protein gene #38 
GGAATTTTGCTGATATTATAGGTTATAAGCAATTTTTTGAAGTAAAAATGTATATAGTTAGTGATATG
TACTAAAAAGTAGCTGAATTTTATTAGTGAGTTAGCCATTAGTCTTTTGCCT
Sequence number 1831: Ehrlichia chaffeensis 120 kDa protein gene #39 
GTGATATGTACTAAAAAGTAGCTGAATTTTATTAGTGAGTTAGCCATTAGTCTTTTGCCTTTTTCAGA
AGAACTTTGCTGAAATGATTAAAACAGAGTGATAGTTGTTGTTAATGTAATT
Sequence number 1832: Ehrlichia chaffeensis 120 kDa protein gene #40 TTTTCAGAAGAACTTTGCTGAAATGATTAAAACAGAGTGATAGTTGTTGTTAATGTAATTGATAATAA ACGGTGATAGGAATATAAGTCTTCGTATATAGCTATTCAATTTAGTATAAAG
Sequence number 1833: Ehrlichia chaffeensis 120 kDa protein gene #41 
GATAATAAACGGTGATAGGAATATAAGTCTTCGTATATAGCTATTCAATTTAGTATAAAGGCGGAAAA
TTGGTGTGTGCATAATATCTAAGTGTTTGCTTAAGGTTTGAGTTTTGGTAAT
Sequence number 1834: Ehrlichia chaffeensis 120 kDa protein gene #42 
GCGGAAAATTGGTGTGTGCATAATATCTAAGTGTTTGCTTAAGGTTTGAGTTTTGGTAATTAAGAATT
AAGTGCTATCCTGCGCTATTGTAATATGTTTACGTATGTAAAAGTGGACAAA
Sequence number 1835: Ehrlichia chaffeensis 120 kDa protein gene #43 
TAAGAATTAAGTGCTATCCTGCGCTATTGTAATATGTTTACGTATGTAAAAGTGGACAAAGTAGACTT
ACCTATAGCGTGGTTGGGAATTAAAAATAAAAAGCTGTTCTGCGCTATTGTA
Sequence number 1836: Ehrlichia chaffeensis 120 kDa protein gene #44 
GTAGACTTACCTATAGCGTGGTTGGGAATTAAAAATAAAAAGCTGTTCTGCGCTATTGTAATATATTT
ATGTATGTAAAAGTATACAAAGTAAACTTACCTATGGTGCGGTTAGTAATTA
Sequence number 1837: Ehrlichia chaffeensis 120 kDa protein gene #45 
ATATATTTATGTATGTAAAAGTATACAAAGTAAACTTACCTATGGTGCGGTTAGTAATTAAGAATTAA
GTGCTATCCTGCGCTGTAATATGTTTACGTATGTAAAAGTGGACAAAGTAGA
Sequence number 1838: Ehrlichia chaffeensis 120 kDa protein gene #46 
AGAATTAAGTGCTATCCTGCGCTGTAATATGTTTACGTATGTAAAAGTGGACAAAGTAGACCTACCTA
TGGCGTGGTTGGGAATTAAGAATAAAAAGCTATTCTACGCTGTTATAATATA
Sequence number 1839: Ehrlichia chaffeensis 120 kDa protein gene #47 
CCTACCTATGGCGTGGTTGGGAATTAAGAATAAAAAGCTATTCTACGCTGTTATAATATATTTATGTA
TGTAAAAGTGTACAAAGTAAACTTACCTATGATGTAGTTGGGAATTAAGAAT
Sequence number 1840: Ehrlichia chaffeensis 120 kDa protein gene #48 
TTTATGTATGTAAAAGTGTACAAAGTAAACTTACCTATGATGTAGTTGGGAATTAAGAATAAAAAGCT
ATTCTACGCTGTTATAATATATTTATGTATGTAAAAGTATACAAAGTAAACT
Sequence number 1841: Ehrlichia chaffeensis 120 kDa protein gene #49 
AAAAAGCTATTCTACGCTGTTATAATATATTTATGTATGTAAAAGTATACAAAGTAAACTTACCTATG
GTGTAGTTGGTAATTAAGAATTAAATGCTATTCTGCGCTGTTATAATGTATT
Sequence number 1842: Ehrlichia chaffeensis 120 kDa protein gene #50 
TACCTATGGTGTAGTTGGTAATTAAGAATTAAATGCTATTCTGCGCTGTTATAATGTATTTACGTATG
TTAAAAGTGACAAAGTAAACTTACCTATGGTGTGAATTGAATGTTGGTTAAT
Sequence number 1843: Ehrlichia chaffeensis 120 kDa protein gene #51 
TACGTATGTTAAAAGTGACAAAGTAAACTTACCTATGGTGTGAATTGAATGTTGGTTAATTACACTTT
AATCACAGCTTGCGTCTTATAAGAAAATTACTGTTTATCATTTTTTATATCT
Sequence number 1844: Ehrlichia chaffeensis 120 kDa protein gene #52 
TACACTTTAATCACAGCTTGCGTCTTATAAGAAAATTACTGTTTATCATTTTTTATATCTTGATCCTT
GACAGGTTTTTCATTGTGATTGTCAAAATCATTTTCATGGTGTGAGTTGCTT
Sequence number 1845: Ehrlichia chaffeensis 120 kDa protein gene #53 
TGATCCTTGACAGGTTTTTCATTGTGATTGTCAAAATCATTTTCATGGTGTGAGTTGCTTATATTATT
ATTTTCATAGTCATCATCTTCATTACTATTGTTGTAATCGTTGTCTTCTTCA
Sequence number 1846: Ehrlichia chaffeensis 120 kDa protein gene #54 
ATATTATTATTTTCATAGTCATCATCTTCATTACTATTGTTGTAATCGTTGTCTTCTTCATCTTCTTC
TTCATCTTCATCTTCTTCATCGGTATAGTCATAGTCTTCTTCAGGCTGTTCG
Sequence number 1847: Ehrlichia chaffeensis 120 kDa protein gene #55 
TCTTCTTCTTCATCTTCATCTTCTTCATCGGTATAGTCATAGTCTTCTTCAGGCTGTTCGCTGTACTC
TTCATCTTCATCTATGTACTCATCTAAATCATCGAAGTTAAAATCATCATCT
Sequence number 1848: Ehrlichia chaffeensis 120 kDa protein gene #56 
CTGTACTCTTCATCTTCATCTATGTACTCATCTAAATCATCGAAGTTAAAATCATCATCTTCGTCTTC
ATCATCTTCATAATTTGTTTCTGTACTGTTATATATAGCACCTTCTTCTGTT
Sequence number 1849: Ehrlichia chaffeensis 120 kDa protein gene #57 
TCGTCTTCATCATCTTCATAATTTGTTTCTGTACTGTTATATATAGCACCTTCTTCTGTTAGATTATT
TTCAGTGTTGTTAGGTTTGTTTTCAGGAATATCACTTTCAATACTAGGGATA
Sequence number 1850: Ehrlichia chaffeensis 120 kDa protein gene #58 
AGATTATTTTCAGTGTTGTTAGGTTTGTTTTCAGGAATATCACTTTCAATACTAGGGATAGGGTCTTC
ATTTTCTTTTTTTGTTATTTTTACATTATTAGGATCGTAAGGTTCTTGAGTA
Sequence number 1851: Ehrlichia chaffeensis 120 kDa protein gene #59 
GGGTCTTCATTTCTTTTTTGTTATTTTACATTATTAGGATCGTAAGGTTCTTGAGTAGGTACGAA
AGTGCTTTTCAATAATTTTTTGTAAAGTTTTGTCTTCATAATATTTTATT
Sequence number 1852: Ehrlichia chaffeensis 120 kDa protein gene #60 
GGTACGAAAGTGCTTTTCAATAATTTTTTTGTAAAGTTTTTGTCTTCATAATATTTTATTTTTTTTGA
TTTTATAGGATAAGTAGACTCTATTAAAAGTATTTGTTCATCTCTAGGTAAC
Sequence number 1853: Ehrlichia chaffeensis 120 kDa protein gene #61 
TTTTTTGATTTTATAGGATAAGTAGACTCTATTAAAAGTATTTGTTCATCTCTAGGTAACATTATAAC
TTCTTGAGGTAATAATAACGCTCTTTGTGTTTCTGATATGTGTAATGAACGA
Sequence number 1854: Ehrlichia chaffeensis 120 kDa protein gene #62 
ATTATAACTTCTTGAGGTAATAATAACGCTCTTTGTGTTTCTGATATGTGTAATGAACGAGAGGCAGG
GTTTAAATCTAAAAATTTAGGTCTATTTAAAGATTCCTGATTAACTGTTTTA
Sequence number 1855: Ehrlichia chaffeensis 120 kDa protein gene #63 
GAGGCAGGGTTTAAATCTAAAAATTTAGGTCTATTTAAAGATTCCTGATTAACTGTTTTATTGCCTAT
TAGCTGTGATATTAGGTTTGCAGTTTCAATATTGTTTGCTGCAAAAGTAATT
Sequence number 1856: Ehrlichia chaffeensis 120 kDa protein gene #64 
TTGCCTATTAGCTGTGATATTAGGTTTGCAGTTTCAATATTGTTTGCTGCAAAAGTAATTCTATAAGT
AGAATTTGACAGAAATGAGTTCATTCCTGCTTCCTCATATATACCTTTAAGC
Sequence number 1857: Ehrlichia chaffeensis 120 kDa protein gene #65 
CTATAAGTAGAATTTGACAGAAATGAGTTCATTCCTGCTTCCTCATATATACCTTTAAGCTGTTCAGT
GTCTTGGATAATCAAAAATAATCTAACTCTATATCCACGGAAATATGCAATA
Sequence number 1858: Ehrlichia chaffeensis 120 kDa protein gene #66 
TGTTCAGTGTCTTGGATAATCAAAAATAATCTAACTCTATATCCACGGAAATATGCAATACCAGTTTG
AAATTGCTCCATCTTTCCTAATGTTGGAAACTCATCCATTAAAAACAATACA
Sequence number 1859: Ehrlichia chaffeensis 120 kDa protein gene #67 
CCAGTTTGAAATTGCTCCATCTTTCCTAATGTTGGAAACTCATCCATTAAAAACAATACACCGTAAGG
TTCATCATCCGATGGTAAAGTTCGACATAAAAACTCTGTTGCTTGCTGATAG
Sequence number 1860: Ehrlichia chaffeensis 120 kDa protein gene #68 CCGTAAGGTTCATCATCCGATGGTAAAGTTCGACATAAAAACTCTGTTGCTTGCTGATAGAATACCTG CATTAAAGGCCTAAGACGAGTTAAGTTGTCTGGTGTTAATCCAACATATACT
Sequence number 1861: Ehrlichia chaffeensis 120 kDa protein gene #69 
AATACCTGCATTAAAGGCCTAAGACGAGTTAAGTTGTCTGGTGTTAATCCAACATATACTGTTACTTT
TTTTCTTTTAAATTCTTGAATATTAAAATCACTTGAAGCTGTTGCTGTATCT
Sequence number 1862: Ehrlichia chaffeensis 120 kDa protein gene #70 
GTTACTTTTTTTCTTTTAAATTCTTGAATATTAAAATCACTTGAAGCTGTTGCTGTATCTATTAACGG
GTTTGCCCATAATTCTAAAGATGAGTTCATAGTTGATACTACACCTGAACGC
Sequence number 1863: Ehrlichia chaffeensis 120 kDa protein gene #71 
ATTAACGGGTTTGCCCATAATTCTAAAGATGAGTTCATAGTTGATACTACACCTGAACGCTCTTTATC
AGCTTTTTGTAAGAATGCAGCTATATTCATATATGCAACTGGGTGAATTTTT
Sequence number 1864: Ehrlichia chaffeensis 120 kDa protein gene #72 
TCTTTATCAGCTTTTTGTAAGAATGCAGCTATATTCATATATGCAACTGGGTGAATTTTTTTCCCTAT
AGTGTCTAATACAACTGCCAAGTTGTAGACTACGTCATCACTACGCATTGTT
Sequence number 1865: Ehrlichia chaffeensis 120 kDa protein gene #73 
TTCCCTATAGTGTCTAATACAACTGCCAAGTTGTAGACTACGTCATCACTACGCATTGTTCGTACAAC
TTCTCCAAAGGATTTTACTTTTTCTGGTACTGCTAGTAGATATAATACTACT
Sequence number 1866: Ehrlichia chaffeensis 120 kDa protein gene #74 
CGTACAACTTCTCCAAAGGATTTTACTTTTTCTGGTACTGCTAGTAGATATAATACTACTCCTACAAA
TAAACTACGTGCTTCGTTGTACCAAAAGTCTTGTTCAGGCATTATTAGATTG
Sequence number 1867: Ehrlichia chaffeensis 120 kDa protein gene #75 
CCTACAAATAAACTACGTGCTTCGTTGTACCAAAAGTCTTGTTCAGGCATTATTAGATTGGCGATTTT
TTGTACATCATCCACCATTTGTCCTGGTTTAGAGCTTATCCAATCCAATGGA
Sequence number 1868: Ehrlichia chaffeensis 120 kDa protein gene #76 
GCGATTTTTTGTACATCATCCACCATTTGTCCTGGTTTAGAGCTTATCCAATCCAATGGATTGTAACA
GTGACTTACCCCATCAGGTTGAGCTGGATTCCACACAAATACTTCTTGTCCT
Sequence number 1869: Ehrlichia chaffeensis 120 kDa protein gene #77 
TTGTAACAGTGACTTACCCCATCAGGTTGAGCTGGATTCCACACAAATACTTCTTGTCCTCTTTTTTT
CCTCCATCCACTTGTGAGATCATAGTTTTCTAATTTTATGTCATGTACTATT
Sequence number 1870: Ehrlichia chaffeensis 120 kDa protein gene #78 
CTTTTTTTCCTCCATCCACTTGTGAGATCATAGTTTTCTAATTTTATGTCATGTACTATTACAGAGTC
TTCCCAAAACAATAAGTTTGGTATTACAAAACCTACACCTTTCCCAGATCCG
Sequence number 1871: Ehrlichia chaffeensis 120 kDa protein gene #79 
ACAGAGTCTTCCCAAAACAATAAGTTTGGTATTACAAAACCTACACCTTTCCCAGATCCGGTTGGTGC
AAATAGTAAAGCATGTTGAAATCCATCAGCAATAAGATAACCTCTTTTGTCT
Sequence number 1872: Ehrlichia chaffeensis 120 kDa protein gene #80 
GTTGGTGCAAATAGTAAAGCATGTTGAAATCCATCAGCAATAAGATAACCTCTTTTGTCTTTTCCTAG
TAGTATACCTTTTCTACTACGTAATCCTATTTTTCGAATATCTTTTTCTGTT
Sequence number 1873: Ehrlichia chaffeensis 120 kDa protein gene #81 
TTTCCTAGTAGTATACCTTTTCTACTACGTAATCCTATTTTTCGAATATCTTTTTCTGTTGCCCATCT
TGAATCTCCATGTAATGATTCTTTCTTTTTAAATGGTCTCCAATCGAATAGT
Sequence number 1874: Ehrlichia chaffeensis 120 kDa protein gene #82 
GCCCATCTTGAATCTCCATGTAATGATTCTTTCTTTTTAAATGGTCTCCAATCGAATAGTATGTTTCT
TAAATTCCACAATACTATACTTAGGATACCTATTGGTATACATAGTGATGAT
Sequence number 1875: Ehrlichia chaffeensis 120 kDa protein gene #83 
ATGTTTCTTAAATTCCACAATACTATACTTAGGATACCTATTGGTATACATAGTGATGATATTAACTT
TACTAATAGTATTGCATCGTATGAAGAAGGATTATGCCACCAGTGTTCTACG
Sequence number 1876: Ehrlichia chaffeensis 120 kDa protein gene #84 
ATTAACTTTACTAATAGTATTGCATCGTATGAAGAAGGATTATGCCACCAGTGTTCTACGTAATTAAA
AACAGTAGGCCAAATTCTGTCTGGAAAGTCGCTGAAACTAGGATTTATAGCG
Sequence number 1877: Ehrlichia chaffeensis 120 kDa protein gene #85 
TAATTAAAAACAGTAGGCCAAATTCTGTCTGGAAAGTCGCTGAAACTAGGATTTATAGCGTTGAAATC
TAGATAGTCTGGTCCCCATACCATTAAGATAAATAAAACCCCTGATAGATAA
Sequence number 1878: Ehrlichia chaffeensis 120 kDa protein gene #86 
TTGAAATCTAGATAGTCTGGTCCCCATACCATTAAGATAAATAAAACCCCTGATAGATAAAAGCAAAA
TTCCAACCCGAAGAATGCTCCTAAGACCAGGAATAAAATATTACGTATGTGG
Sequence number 1879: Ehrlichia chaffeensis 120 kDa protein gene #87 
AAGCAAAATTCCAACCCGAAGAATGCTCCTAAGACCAGGAATAAAATATTACGTATGTGGTTTGCACT
TACACTATCCATAAAAATAACAACTTAATTATAAAACTTACGTATCATTTAA
Sequence number 1880: Ehrlichia chaffeensis 120 kDa protein gene #88 
TTTGCACTTACACTATCCATAAAAATAACAACTTAATTATAAAACTTACGTATCATTTAACCATTTTT
CTGTAAAGATTTAGTGAATAATATTTCTGATACATACCTTTTACCCTCTGAT
Sequence number 1881: Ehrlichia chaffeensis 120 kDa protein gene #89 
CCATTTTTCTGTAAAGATTTAGTGAATAATATTTCTGATACATACCTTTTACCCTCTGATCCTCTTTT
TAACTGTATTACAATATCTACTATATTAGTGATGTAATTAATTATTTGATCT
Sequence number 1882: Ehrlichia chaffeensis 120 kDa protein gene #90 
CCTCTTTTTAACTGTATTACAATATCTACTATATTAGTGATGTAATTAATTATTTGATCTGGAGGTAT
GCCAAGTCCTGCTTGCATAACCATTAATTTTAATTGTTCTAGTGCCATCATT
Sequence number 1883: Ehrlichia chaffeensis 120 kDa protein gene #91 
ACTGTATTACAATATCTACTATATTAGTGATGTAATTAATTATTTGATCTGGAGGTATGCCAAGTCCT
GCTTGCATAACCATTAATTTTAATTGTTCTAGTGCCATCATTGGTGTGTCAG
Sequence number 1884: Human herpesvirus 3 ORF38 #1 
TGTTTGCGCTAGTTTTAGCGGTGGTAATTCTTCCTCTTTGGACCACGGCTAATAAATCTTACGTAACA
CCAACCCCTGCGACTCGCTCTATCGGACATATGTCTGCTCTTCTACGAGAAT
Sequence number 1885: Human herpesvirus 3 ORF38 #2 
ACGTAACACCAACCCCTGCGACTCGCTCTATCGGACATATGTCTGCTCTTCTACGAGAATATTCCGAC
CGTAATATGTCTCTGAAATTAGAAGCCTTTTATCCTACTGGTTTCGATGAAG
Sequence number 1886: Human herpesvirus 3 ORF38 #3 
ATTCCGACCGTAATATGTCTCTGAAATTAGAAGCCTTTTATCCTACTGGTTTCGATGAAGAACTCATT
AAATCACTTCACTGGGGAAATGATAGAAAACACGTTTTCTTGGTTATTGTTA
Sequence number 1887: Human herpesvirus 3 ORF38 #4 
AACTCATTAAATCACTTCACTGGGGAAATGATAGAAAACACGTTTTCTTGGTTATTGTTAAGGTTAAC
CCTACAACACACGAAGGAGACGTCGGGCTGGTTATATTTCCAAAATACTTGT
Sequence number 1888: Human herpesvirus 3 ORF38 #5 AGGTTAACCCTACAACACACGAAGGAGACGTCGGGCTGGTTATATTTCCAAAATACTTGTTATCGCCA TACCATTTCAAAGCAGAACATCGAGCACCGTTTCCTGCTGGACGTTTTGGAT
Sequence number 1889: Human herpesvirus 3 ORF38 #6 
TATCGCCATACCATTTCAAAGCAGAACATCGAGCACCGTTTCCTGCTGGACGTTTTGGATTTCTTAGT
CACCCTGTGACACCCGACGTGAGCTTCTTTGACAGTTCGTTTGCGCCGTATT
Sequence number 1890: Human herpesvirus 3 ORF38 #7 
TTCTTAGTCACCCTGTGACACCCGACGTGAGCTTCTTTGACAGTTCGTTTGCGCCGTATTTAACTACG
CAACATCTTGTTGCGTTTACTACGTTCCCACCAAACCCCCTTGTATGGCATT
Sequence number 1891: Human herpesvirus 3 ORF38 #8 
TAACTACGCAACATCTTGTTGCGTTTACTACGTTCCCACCAAACCCCCTTGTATGGCATTTGGAAAGA
GCTGAGACCGCAGCAACTGCAGAAAGGCCGTTTGGGGTAAGTCTTTTACCCG
Sequence number 1892: Human herpesvirus 3 ORF38 #9 
TGGAAAGAGCTGAGACCGCAGCAACTGCAGAAAGGCCGTTTGGGGTAAGTCTTTTACCCGCTCGCCCA
ACAGTCCCCAAGAATACTATTCTGGAACATAAAGCGCATTTTGCTACATGGG
Sequence number 1893: Human herpesvirus 3 ORF38 #10 
CTCGCCCAACAGTCCCCAAGAATACTATTCTGGAACATAAAGCGCATTTTGCTACATGGGATGCCCTT
GCCCGACATACTTTTTTTTCTGCCGAAGCAATTATCACCAACTCAACGTTGA
Sequence number 1894: Human herpesvirus 3 ORF38 #11 
ATGCCCTTGCCCGACATACTTTTTTTTCTGCCGAAGCAATTATCACCAACTCAACGTTGAGAATACAC
GTTCCCCTTTTTGGGTCGGTATGGCCAATTCGATACTGGGCCACCGGTTCGG
Sequence number 1895: Human herpesvirus 3 ORF38 #12 
GAATACACGTTCCCCTTTTTGGGTCGGTATGGCCAATTCGATACTGGGCCACCGGTTCGGTGCTTCTC
ACAAGCGACTCGGGTCGTGTGGAAGTAAATATTGGTGTAGGATTTATGAGCT
Sequence number 1896: Human herpesvirus 3 ORF38 #13 
TGCTTCTCACAAGCGACTCGGGTCGTGTGGAAGTAAATATTGGTGTAGGATTTATGAGCTCGCTCATT
TCTTTATCCTCTGGACCACCGATAGAATTAATTGTTGTACCACATACAGTAA
Sequence number 1897: Human herpesvirus 3 ORF38 #14 
CGCTCATTTCTTTATCCTCTGGACCACCGATAGAATTAATTGTTGTACCACATACAGTAAAACTGAAC
GCGGTTACAAGCGACACCACATGGTTCCAGCTAAATCCACCGGGTCCGGATC
Sequence number 1898: Human herpesvirus 3 ORF38 #15 
AACTGAACGCGGTTACAAGCGACACCACATGGTTCCAGCTAAATCCACCGGGTCCGGATCCGGGGCCA
TCTTATCGAGTTTATTTACTTGGACGTGGGTTGGATATGAATTTTTCAAAGC
Sequence number 1899: Human herpesvirus 3 ORF38 #16 
CGGGGCCATCTTATCGAGTTTATTTACTTGGACGTGGGTTGGATATGAATTTTTCAAAGCATGCTACG
GTCGATATATGCGCATATCCCGAAGAGAGTTTGGATTACCGCTATCATTTAT
Sequence number 1900: Human herpesvirus 3 ORF38 #17 
ATGCTACGGTCGATATATGCGCATATCCCGAAGAGAGTTTGGATTACCGCTATCATTTATCCATGGCC
CACACGGAGGCTCTGCGGATGACAACGAAGGCGGATCAACATGACATAAACG
Sequence number 1901: Human herpesvirus 3 ORF38 #18 
CCATGGCCCACACGGAGGCTCTGCGGATGACAACGAAGGCGGATCAACATGACATAAACGAGGAAAGC
TATTACCATATCGCCGCAAGAATAGCCACATCAATTTTTGCGTTGTCGGAAA
Sequence number 1902: Human herpesvirus 3 ORF38 #19 
AGGAAAGCTATTACCATATCGCCGCAAGAATAGCCACATCAATTTTTGCGTTGTCGGAAATGGGCCGT
ACCACAGAATATTTTCTGTTAGATGAGATCGTAGATGTTCAGTATCAATTAA
Sequence number 1903: Human herpesvirus 3 ORF38 #20 
TGGGCCGTACCACAGAATATTTTCTGTTAGATGAGATCGTAGATGTTCAGTATCAATTAAAATTCCTT
AATTACATTTTAATGCGGATAGGAGCAGGAGCTCATCCCAACACTATATCCG
Sequence number 1904: Human herpesvirus 3 ORF38 #21 
AATTCCTTAATTACATTTTAATGCGGATAGGAGCAGGAGCTCATCCCAACACTATATCCGGAACCTCG
GATCTGATCTTTGCCGATCCATCGCAGCTTCATGACGAACTTTCACTTCTTT
Sequence number 1905: Human herpesvirus 3 ORF38 #22 
GAACCTCGGATCTGATCTTTGCCGATCCATCGCAGCTTCATGACGAACTTTCACTTCTTTTTGGTCAG
GTAAAACCCGCAAATGTCGATTATTTTATTTCATATGATGAAGCCCGTGATC
Sequence number 1906: Human herpesvirus 3 ORF38 #23 
TTGGTCAGGTAAAACCCGCAAATGTCGATTATTTTATTTCATATGATGAAGCCCGTGATCAACTAAAG
ACCGCATACGCGCTTTCCCGTGGTCAAGACCATGTGAATGCACTTTCTCTCG
Sequence number 1907: Human herpesvirus 3 ORF38 #24 
AACTAAAGACCGCATACGCGCTTTCCCGTGGTCAAGACCATGTGAATGCACTTTCTCTCGCCAGGCGT
GTTATAATGAGCATATACAAGGGGCTGCTTGTGAAGCAAAATTTAAATGCTA
Sequence number 1908: Human herpesvirus 3 ORF38 #25 
CCAGGCGTGTTATAATGAGCATATACAAGGGGCTGCTTGTGAAGCAAAATTTAAATGCTACAGAGAGG
CAGGCTTTATTTTTTGCCTCAATGATTTTATTAAATTTCCGCGAAGGACTAG
Sequence number 1909: Human herpesvirus 3 ORF38 #26 
CAGAGAGGCAGGCTTTATTTTTTGCCTCAATGATTTTATTAAATTTCCGCGAAGGACTAGAAAATTCA
TCTCGGGTATTAGACGGTCGCACAACTTTGCTTTTAATGACATCCATGTGTA
Sequence number 1910: Human herpesvirus 3 ORF38 #27 
AAAATTCATCTCGGGTATTAGACGGTCGCACAACTTTGCTTTTAATGACATCCATGTGTACGGCAGCT
CACGCCACGCAAGCAGCACTTAACATACAAGAAGGCCTGGCATACTTAAATC
Sequence number 1911: Human herpesvirus 3 ORF38 #28 
CGGCAGCTCACGCCACGCAAGCAGCACTTAACATACAAGAAGGCCTGGCATACTTAAATCCTTCAAAA
CACATGTTTACAATACCAAACGTATACAGTCCTTGTATGGGTTCCCTTCGTA
Sequence number 1912: Human herpesvirus 3 ORF38 #29 
CTTCAAAACACATGTTTACAATACCAAACGTATACAGTCCTTGTATGGGTTCCCTTCGTACAGACCTC
ACGGAAGAGATTCATGTTATGAATCTCCTGTCGGCAATACCAACACGCCCAG
Sequence number 1913: Human herpesvirus 3 ORF38 #30 
CAGACCTCACGGAAGAGATTCATGTTATGAATCTCCTGTCGGCAATACCAACACGCCCAGGACTTAAC
GAGGTATTGCATACCCAACTAGACGAATCTGAAATATTCGACGCGGCATTTA
Sequence number 1914: Human herpesvirus 3 ORF38 #31 
GACTTAACGAGGTATTGCATACCCAACTAGACGAATCTGAAATATTCGACGCGGCATTTAAAACCATG
ATGATTTTTACCACATGGACTGCCAAAGATTTGCATATACTCCACACCCATG
Sequence number 1915: Human herpesvirus 3 ORF38 #32 
AAACCATGATGATTTTTACCACATGGACTGCCAAAGATTTGCATATACTCCACACCCATGTACCAGAA
GTATTTACGTGTCAAGATGCAGCCGCGCGTAACGGAGAATATGTGCTCATTC
Sequence number 1916: Human herpesvirus 3 ORF38 #33 TACCAGAAGTATTTACGTGTCAAGATGCAGCCGCGCGTAACGGAGAATATGTGCTCATTCTTCCAGCT GTCCAGGGACACAGTTATGTGATTACACGAAACAAACCTCAAAGGGGTTTGG
Sequence number 1917: Human herpesvirus 3 ORF38 #34 
TTCCAGCTGTCCAGGGACACAGTTATGTGATTACACGAAACAAACCTCAAAGGGGTTTGGTATATTCC
CTGGCAGATGTGGATGTATATAACCCCATATCCGTTGTTTATTTAAGCAGGG
Sequence number 1918: Human herpesvirus 3 ORF38 #35 
TATATTCCCTGGCAGATGTGGATGTATATAACCCCATATCCGTTGTTTATTTAAGCAGGGATACTTGC
GTGTCTGAACATGGTGTCATAGAGACGGTCGCACTGCCCCATCCGGACAATT
Sequence number 1919: Human herpesvirus 3 ORF38 #36 
ATACTTGCGTGTCTGAACATGGTGTCATAGAGACGGTCGCACTGCCCCATCCGGACAATTTAAAAGAA
TGTTTGTATTGCGGAAGTGTTTTTCTTAGGTATCTAACCACGGGGGCGATTA
Sequence number 1920: Human herpesvirus 3 ORF38 #37 
TAAAAGAATGTTTGTATTGCGGAAGTGTTTTTCTTAGGTATCTAACCACGOGGGGCGATTATGGATAT
AATTATTATTGACAGCAAAGATACAGAACGACAACTAGCCGCTATGGGAAACT
Sequence number 1921: Human herpesvirus 3 ORF38 #38 
TGGATATAATTATTATTGACAGCAAAGATACAGAACGACAACTAGCCGCTATGGGAAACTCCACAATT
CCACCCTTCAATCCAGACATGCACGGGGATGACTCTAAGGCTGTGTTGTTGT
Sequence number 1922: Human herpesvirus 3 ORF38 #39 
CCACAATTCCACCCTTCAATCCAGACATGCACGGGGATGACTCTAAGGCTGTGTTGTTGTTTCCAAAC
GGAACTGTGGTAACGCTTCTAGGATTCGAACGACGACAAGCCATACGAATGT
Sequence number 1923: Human herpesvirus 3 ORF38 #40 
TTCCAAACGGAACTGTGGTAACGCTTCTAGGATTCGAACGACGACAAGCCATACGAATGTCGGGACAA
TACCTTGGGGCCTCTTTAGGAGGGGCGTTTCTGGCGGTAGTGGGGTTTGGTA
Sequence number 1924: Human herpesvirus 3 ORF38 #41 
CGGGACAATACCTTGGGGCCTCTTTAGGAGGGGCGTTTCTGGCGGTAGTGGGGTTTGGTATTATCGGA
TGGATGTTATGTGGAAATTCCCGCCTTCGAGAATATAATAAAATACCTCTGA
Sequence number 1925: Human herpesvirus 3 ORF38 #42 
TTATCGGATGGATGTTATGTGGAAATTCCCGCCTTCGAGAATATAATAAAATACCTCTGACATAAAAA
ACATGTATAATAAAAAGTCACTATAAACGTATTCTCTACAATACTTTATTCG
Sequence number 1926: Human herpesvirus 3 ORF38 #43 
CATAAAAAACATGTATAATAAAAAGTCACTATAAACGTATTCTCTACAATACTTTATTCGCGAATAAT
ACACACTACCTTTGGGTTTTTTTCCCGTCCCCAAATGGTGTTTGGTGCACTC
Sequence number 1927: Human herpesvirus 3 ORF38 #44 
CGAATAATACACACTACCTTTGGGTTTTTTTCCCGTCCCCAAATGGTGTTTGGTGCACTCTACCAAAA
AATAGAGCGCCTAAATATGCTATATAACGCCTCCCAGCAAAATACGGTTCAA
Sequence number 1928: Human herpesvirus 3 ORF38 #45 
TACCAAAAAATAGAGCGCCTAAATATGCTATATAACGCCTCCCAGCAAAATACGGTTCAAAGGCATTA
CCCGATATTGTATTGTAGTACAGGGCAATGGGAATTGATGATCCCAATAAAC
Sequence number 1929: Human herpesvirus 3 ORF38 #46 
AGGCATTACCCGATATTGTATTGTAGTACAGGGCAATGGGAATTGATGATCCCAATAAACGGCATAGA
CGCACAGCGCCGTTATAGCAGGGGTCTCCAGAGTACAGGGTATCTAAGTACC
Sequence number 1930: Human herpesvirus 3 ORF38 #47 
GGCATAGACGCACAGCGCCGTTATAGCAGGGGTCTCCAGAGTACAGGGTATCTAAGTACCGGGATATC
TCATACTCATGCCTTTCCGTGACAGAAACATCAACCGGAACAGTATCCGATA
Sequence number 1931: Human herpesvirus 3 ORF38 #48 
GGGATATCTCATACTCATGCCTTTCCGTGACAGAAACATCAACCGGAACAGTATCCGATAAACCAACT
CCTGTTTTTGCAAGGCGTAAAATTCGCACACCTTCCTTTTTTGCAAGATGTG
Sequence number 1932: Human herpesvirus 3 ORF38 #49 
AACCAACTCCTGTTTTTGCAAGGCGTAAAATTCGCACACCTTCCTTTTTTGCAAGATGTGACGTTTCC
TTGTAACAGGGAAGCTGGGGGAGTGGTAAGAACAACAAAGTTTCAGCCAACG
Sequence number 1933: Human herpesvirus 3 ORF38 #50 
ACGTTTCCTTGTAACAGGGAAGCTGGGGGAGTGGTAAGAACAACAAAGTTTCAGCCAACGTGCCAATA
AAGCCCACTTCCCTCAAGAGGCTGTTTGCTGTATCCACAATGGTCCGTATTA
Sequence number 1934: Human herpesvirus 3 ORF38 #51 
TGCCAATAAAGCCCACTTCCCTCAAGAGGCTGTTTGCTGTATCCACAATGGTCCGTATTAAATCTTGA
GCAACTTGATCCGTGTCATCATCACTGGGTAACGCGTTAACATAACTACGCG
Sequence number 1935: Human herpesvirus 3 ORF38 #52 
AATCTTGAGCAACTTGATCCGTGTCATCATCACTGGGTAACGCGTTAACATAACTACGCGTTAAATCT
TCAATAACGGCATAACAATTAAACGCTTCCCACCGAGACAGTATATATTGAA
Sequence number 1936: Human herpesvirus 3 ORF38 #53 
TTAAATCTTCAATAACGGCATAACAATTAAACGCTTCCCACCGAGACAGTATATATTGAACAATCACG
AACCGTTGACAGGACGTCAGATCACGTCCGTAAGCATGCCCGAAAAATGGAA
Sequence number 1937: Human herpesvirus 3 ORF38 #54 
CAATCACGAACCGTTGACAGGACGTCAGATCACGTCCGTAAGCATGCCCGAAAAATGGAAGTTCCCCC
CGTTCGCCATATACCGCAACAACTGCAGTATATATCGTCTCACGGGCTTCAT
Sequence number 1938: Human herpesvirus 3 ORF38 #55 
GTTCCCCCCGTTCGCCATATACCGCAACAACTGCAGTATATATCGTCTCACGGGCTTCATTAAGTTCA
TCTTCAAGTCCAGGCCATTTTCTGGCTTTAAATATAACCTCGTCCGCAAAAA
Sequence number 1939: Human herpesvirus 3 ORF38 #56 
TAAGTTCATCTTCAAGTCCAGGCCATTTTCTGGCTTTAAATATAACCTCGTCCGCAAAAAAAACCGCA
CATGATAACGCGCGGATACAATGAGTAGTGGCTTTATGGCGAGGATCCCAAA
Sequence number 1940: Human herpesvirus 3 ORF38 #57 
AAACCGCACATGATAACGCGCGGATACAATGAGTAGTGGCTTTATGGCGAGGATCCCAAATGTCCATT
ACCCGGGGGATGGTCCTAATCTGTACAAAGTTACTTAGTGTAATATGATCGG
Sequence number 1941: Human herpesvirus 3 ORF38 #58 
TGTCCATTACCCGGGGGATGGTCCTAATCTGTACAAAGTTACTTAGTGTAATATGATCGGACTTCTTA
CGCCGTCTAGGCTGTTTCTCAGAATACGGTTCACCCGAAATCGGCACATCAT
Sequence number 1942: Human herpesvirus 3 ORF38 #59 
ACTTCTTACGCCGTCTAGGCTGTTTCTCAGAATACGGTTCACCCGAAATCGGCACATCATCTGCTTTT
ACGTCTTCCGTAACCACATCAGCAGCGCGCCGACTAACAATTATACTTGTTT
Sequence number 1943: Human herpesvirus 3 ORF38 #60 
CTGCTTTTACGTCTTCCGTAACCACATCAGCAGCGCGCCGACTAACAATTATACTTGTTTTTTCATCG
TCGTTACTTCCGTTAAGCGCGTCTCGTATCTCGGGCGTCCCGTCGAATAATC
Sequence number 1944: Human herpesvirus 3 ORF38 #61 TTTCATCGTCGTTACTTCCGTTAAGCGCGTCTCGTATCTCGGGCGTCCCGTCGAATAATCCACTCACT AGCTCCTGCAAACTTTCTGGTAACTCCAACATACGCATATACACCAATGAAA
Sequence number 1945: Human herpesvirus 3 ORF38 #62 
CACTCACTAGCTCCTGCAAACTTTCTGGTAACTCCAACATACGCATATACACCAATGAAAAACTGGCT
TCGTTTGGTACGTACATAAAGCCATTTGTGGTATTAATGGCGGTGGGTGTTG
Sequence number 1946: Human herpesvirus 3 ORF38 #63 
AACTGGCTTCGTTTGGTACGTACATAAAGCCATTTGTGGTATTAATGGCGGTGGGTGTTGGAAACAAT
TTTAGCTTATTCTCGCGCGTAACATCTACCCCCGCCACCAATGTTAAATGCG
Sequence number 1947: Human herpesvirus 3 ORF38 #64 
GAAACAATTTTAGCTTATTCTCGCGCGTAACATCTACCCCCGCCACCAATGTTAAATGCGTCACGGGG
AGGGACACGAGATAATCTGCGAGCGTAGGGTCCTCCACTTCAACATCAAATG
Sequence number 1948: Human herpesvirus 3 ORF38 #65 
TCACGGGGAGGGACACGAGATAATCTGCGAGCGTAGGGTCCTCCACTTCAACATCAAATGTTCCGCAA
AGGTCGCGATCCACCGCCCCCGATCCCGCTGCAAGTAAGGCCACTCGATCCA
Sequence number 1949: Human herpesvirus 3 ORF38 #66 
TTCCGCAAAGGTCGCGATCCACCGCCCCCGATCCCGCTGCAAGTAAGGCCACTCGATCCAAAAACACG
CAGTTATTATTGGATGATACCGCCCATGTCTTCCCGGTGCGATTGAGCTCAC
Sequence number 1950: Human herpesvirus 3 ORF38 #67 
AAAACACGCAGTTATTATTGGATGATACCGCCCATGTCTTCCCGGTGCGATTGAGCTCACTTCGAACG
TAACTGGCAACAGATCTGTCACCGGGTCCGACCCCGCGAACAACATGTCCAA
Sequence number 1951: Human herpesvirus 3 ORF38 #68 
TTCGAACGTAACTGGCAACAGATCTGTCACCGGGTCCGACCCCGCGAACAACATGTCCAAATTTTGCG
ATCTCGCCTCCATGTTTGCGGGGTATGGAAATTAAGCATCCCCCGCATATAA
Sequence number 1952: Human herpesvirus 3 ORF38 #69 
ATTTTGCGATCTCGCCTCCATGTTTGCGGGGTATGGAAATTAAGCATCCCCCGCATATAAAATACGCC
CTGGTAGCACGCTCGTTAAAATAAAACGTTACGCCGTTATAAGATACGGTTG
Sequence number 1953: Human herpesvirus 3 ORF38 #70 
AATACGCCCTGGTAGCACGCTCGTTAAAATAAAACGTTACGCCGTTATAAGATACGGTTGAATGATAT
GGAAATTCCATATTAAAGCGTTTATCGGAACATTAACCTCGAACTTGCCGTC
Sequence number 1954: Human herpesvirus 3 ORF38 #71 
AATGATATGGAAATTCCATATTAAAGCGTTTATCGGAACATTAACCTCGAACTTGCCGTCCCGTGATC
GTGTGATCGCCAACCTTAGGTCCACACCGAATATGAGAAATATATAACTACA
Sequence number 1955: Human herpesvirus 3 ORF38 #72 
CCGTGATCGTGTGATCGCCAACCTTAGGTCCACACCGAATATGAGAAATATATAACTACACGCAAACA
TTCAAAACACCGTGGTATCATTAACGTCATATGAAAAGATCCAATCAATCCA
Sequence number 1956: Human herpesvirus 3 ORF38 #73 
CGCAAACATTCAAAACACCGTGGTATCATTAACGTCATATGAAAAGATCCAATCAATCCAATCAACCA
CACCTCCTACCGTTTAGCACGTCAGCTATGTGACATGCTCCAAACATACGTA
Sequence number 1957: Human herpesvirus 3 ORF38 #74 
ATCAACCACACCTCCTACCGTTTAGCACGTCAGCTATGTGACATGCTCCAAACATACGTAAACATTTA
GAGAGGGTGTTATAACAGTCTGTCAGGCGGGGTATATTCTACATAATACAAG
Sequence number 1958: Human herpesvirus 3 ORF38 #75 
AACATTTAGAGAGGGTGTTATAACAGTCTGTCAGGCGGGGTATATTCTACATAATACAAGGATCGGCT
TTAACTTTGTCAACATTTTTACTTTGGACTATAAACTGCGACTGAACGTTAT
Sequence number 1959: Human herpesvirus 3 ORF38 #76 
GATCGGCTTTAACTTTGTCAACATTTTTACTTTGGACTATAAACTGCGACTGAACGTTATGAACCCAC
CCCAAGCCCGCGTCTCGGAACAGACAAAGGACTTGCTTAGCGTTATGGTTAA
Sequence number 1960: Human herpesvirus 3 ORF38 #77 
GAACCCACCCCAAGCCCGCGTCTCGGAACAGACAAAGGACTTGCTTAGCGTTATGGTTAACCAGCACC
CCGAAGAGGACGCAAAAGTGTGTAAATCCAGTGATAATTCACCGCTTTATAA
Sequence number 1961: Human herpesvirus 3 ORF38 #78 
CCAGCACCCCGAAGAGGACGCAAAAGTGTGTAAATCCAGTGATAATTCACCGCTTTATAACACCATGG
TTATGTTATCGTATGGGGGTGATACGGACTTACTATTAAGCTCTGCATGTAC
Sequence number 1962: Human herpesvirus 3 ORF38 #79 
CACCATGGTTATGTTATCGTATGGGGGTGATACGGACTTACTATTAAGCTCTGCATGTACCCGCACAT
CTACCGTAAACAGGTCGGCGTTTACGCAACACTCCGTGTTTTATATTATATC
Sequence number 1963: Human herpesvirus 3 ORF38 #80 
CCGCACATCTACCGTAAACAGGTCGGCGTTTACGCAACACTCCGTGTTTTATATTATATCCACGGTGT
TGATTCAACCAATATGTTGTATCTTCTTTTTTTTTTACTATAAAGCGACACG
Sequence number 1964: Human herpesvirus 3 ORF38 #81 
CACGGTGTTGATTCAACCAATATGTTGTATCTTCTTTTTTTTTTACTATAAAGCGACACGCTGTATGC
TCTTATTCACAGCCGGGTTACTTCTGACGATTCTACATCACTTTCGACTTAT
Sequence number 1965: Human herpesvirus 3 ORF38 #82 
CTGTATGCTCTTATTCACAGCCGGGTTACTTCTGACGATTCTACATCACTTTCGACTTATTATTATGT
TATTGTGTGTCTACAGAAATATACGATCAGACCTGCTACCCTTATCTACATC
Sequence number 1966: Human herpesvirus 3 ORF38 #83 
TATTATGTTATTGTGTGTCTACAGAAATATACGATCAGACCTGCTACCCTTATCTACATCCCAGCAAC
TGCTGCTTGGAATTATTGTTGTGACTCGAACAATGCTATTTTGTATTACGGC
Sequence number 1967: Human herpesvirus 3 ORF38 #84 
CCAGCAACTGCTGCTTGGAATTATTGTTGTGACTCGAACAATGCTATTTTGTATTACGGCGTATTATA
CTCTTTTTATAGACACCCGGGTGTTCTTTTTGATTACCGGACACTTGCAAAG
Sequence number 1968: Human herpesvirus 3 ORF38 #85 
GTATTATACTCTTTTTATAGACACCCGGGTGTTCTTTTTGATTACCGGACACTTGCAAAGTGAGGTTA
TTTTTCCAGATAGCGTTTCAAAAATACTTCCTGTGTCGTGGGGTCCAAGTCC
Sequence number 1969: Human herpesvirus 3 ORF38 #86 
TGAGGTTATTTTTCCAGATAGCGTTTCAAAAATACTTCCTGTGTCGTGGGGTCCAAGTCCAGCCGTGT
TACTGGTAATGGCGGCAGTTATTTACGCTATGGACTGTTTGGTGGACACGGT
Sequence number 1970: Human herpesvirus 3 ORF38 #87 
AGCCGTGTTACTGGTAATGGCGGCAGTTATTTACGCTATGGACTGTTTGGTGGACACGGTATCCTTTA
TTGGGCCAAGGGTGTGGGTCCGTGTTATGTTAAAAACATCTATTTCGTTTTA
Sequence number 1971: Human herpesvirus 3 ORF38 #88 
AATGGCGGCAGTTATTTACGCTATGGACTGTTTGGTGGACACGGTATCCTTTATTGGGCCAAGGGTGT
GGGTCCGTGTTATGTTAAAAACATCTATTTCGTTTTAGTCCATTTCAATAAA
Sequence number 1972: Measles virus Whole genome #1 ACCAAACAAAGTTGGGTAAGGATAGATCAATCAATGATCATATTCTAGTGCACTTAGGATTCAAGATC CTATCATCAGGGACAGGAGCAGGGTTAGGGACATCCGAGATGGCCACACTTT
Sequence number 1973: Measles virus Whole genome #2 
TCAAGATCCTATCATCAGGGACAGGAGCAGGGTTAGGGACATCCGAGATGGCCACACTTTTAAGGAGC
TTAGCATTGTTCAAAAGAAACAAGGACAAACCACCCATTACATCAGGATCCG
Sequence number 1974: Measles virus Whole genome #3 
TAAGGAGCTTAGCATTGTTCAAAAGAAACAAGGACAAACCACCCATTACATCAGGATCCGGTGGAGCC
ATCAGAGGAATCAAACACATTATTATAGTACCAATCCCTGGAGATTCCTCAA
Sequence number 1975: Measles virus Whole genome #4 
GTGGAGCCATCAGAGGAATCAAACACATTATTATAGTACCAATCCCTGGAGATTCCTCAATTACCACT
CGATCCAGACTCCTGGACCGGTTGGTCAGGTTAATTGGAAACCCGGATGTGA
Sequence number 1976: Measles virus Whole genome #5 
TTACCACTCGATCCAGACTCCTGGACCGGTTGGTCAGGTTAATTGGAAACCCGGATGTGAGCGGGCCC
AAACTAACAGGAGCACTAATAGGTATATTATCCTTATTTGTGGAGTCTCCAG
Sequence number 1977: Measles virus Whole genome #6 
GCGGGCCCAAACTAACAGGAGCACTAATAGGTATATTATCCTTATTTGTGGAGTCTCCAGGTCAATTG
ATTCAGAGGATCACTGATGACCCTGACGTTAGCATAAGGCTGTTGGAGGTTG
Sequence number 1978: Measles virus Whole genome #7 
GTCAATTGATTCAGAGGATCACTGATGACCCTGACGTTAGCATAAGGCTGTTGGAGGTTGTCCAGAGC
GACCAGTCACAATCTGGCCTTACCTTCGCATCGAGAGGTACTAATATGGAGG
Sequence number 1979: Measles virus Whole genome #8 
TCCAGAGCGACCAGTCACAATCTGGCCTTACCTTCGCATCGAGAGGTACTAATATGGAGGATGAGGCG
GACCAGTACTTTTCACATGATGATCCAAGTAGTAGTGATCAATCCAGGTTCG
Sequence number 1980: Measles virus Whole genome #9 
ATGAGGCGGACCAGTACTTTTCACATGATGATCCAAGTAGTAGTGATCAATCCAGGTTCGGGTGGTTT
GAGAACAAGGAAATCTCAGATATTGAAGTGCAAGACCCTGAGGGCTTCAACA
Sequence number 1981: Measles virus Whole genome #10 
GGTGGTTTGAGAACAAGGAAATCTCAGATATTGAAGTGCAAGACCCTGAGGGCTTCAACATGATTCTG
GGTACCATCCTAGCTCAGATTTGGGTCTTGCTCGCAAAGGCGGTTACGGCTC
Sequence number 1982: Measles virus Whole genome #11 
TGATTCTGGGTACCATCCTAGCTCAGATTTGGGTCTTGCTCGCAAAGGCGGTTACGGCTCCAGACACA
GCAGCTGATTCAGAGCTAAGAAGATGGATCAAATACACCCAACAAAGAAGAG
Sequence number 1983: Measles virus Whole genome #12 
CAGACACAGCAGCTGATTCAGAGCTAAGAAGATGGATCAAATACACCCAACAAAGAAGAGTAGTTGGT
GAATTTAGATTGGAGAGAAAATGGTTGGATGTGGTGAGAAACAGGATTGCCG
Sequence number 1984: Measles virus Whole genome #13 
TAGTTGGTGAATTTAGATTGGAGAGAAAATGGTTGGATGTGGTGAGAAACAGGATTGCCGAGGACCTC
TCCTTACGCCGATTCATGGTCGCTCTAATCCTGGATATCAAGAGAACACCCG
Sequence number 1985: Measles virus Whole genome #14 
AGGACCTCTCCTTACGCCGATTCATGGTCGCTCTAATCCTGGATATCAAGAGAACACCCGGAAACAAA
CCCAGGATTGCTGAAATGATATGTGACATTGATACATATATCGTAGAGGCAG
Sequence number 1986: Measles virus Whole genome #15 
GAAACAAACCCAGGATTGCTGAAATGATATGTGACATTGATACATATATCGTAGAGGCAGGATTAGCC
AGTTTTATCCTGACTATTAAGTTTGGGATAGAAACCATGTATCCTGCTCTTG
Sequence number 1987: Measles virus Whole genome #16 
GATTAGCCAGTTTTATCCTGACTATTAAGTTTGGGATAGAAACCATGTATCCTGCTCTTGGACTGCAT
GAATTTGCTGGTGAATTATCCACACTTGAGTCCTTGATGAATCTTTACCAGC
Sequence number 1988: Measles virus Whole genome #17 
GACTGCATGAATTTGCTGGTGAATTATCCACACTTGAGTCCTTGATGAATCTTTACCAGCAAATGGGG
GAAACTGCACCCTATATGGTAATCCTGGAGAACTCAATTCAGAACAAGTTTA
Sequence number 1989: Measles virus Whole genome #18 
AAATGGGGGAAACTGCACCCTATATGGTAATCCTGGAGAACTCAATTCAGAACAAGTTTAGTGCAGGA
TCATACCCTCTGCTCTGGAGCTATGCCATGGGAGTAGGAGTGGAACTTGAAA
Sequence number 1990: Measles virus Whole genome #19 
GTGCAGGATCATACCCTCTGCTCTGGAGCTATGCCATGGGAGTAGGAGTGGAACTTGAAAACTCCATG
GGAGGTTTGAACTTCGGCCGATCTTACTTTGATCCGGCATATTTTAGATTAG
Sequence number 1991: Measles virus Whole genome #20 
ACTCCATGGGAGGTTTGAACTTCGGCCGATCTTACTTTGATCCGGCATATTTTAGATTAGGGCAAGAG
ATGGTAAGGAGGTCAGCTGGAAAGGTCAGTTCCACATTGGCATCTGAACTCG
Sequence number 1992: Measles virus Whole genome #21 
GGCAAGAGATGGTAAGGAGGTCAGCTGGAAAGGTCAGTTCCACATTGGCATCTGAACTCGGTATAACT
GCCGAGGATGCAAGGCTTGTTTCAGAGATTGCAATGCATACTACTGAGGACA
Sequence number 1993: Measles virus Whole genome #22 
GTATAACTGCCGAGGATGCAAGGCTTGTTTCAGAGATTGCAATGCATACTACTGAGGACAGGATCAGC
AGAGCAGTTGGACCCAGACAAGCCCAAGTGTCATTTCTACACGGTGATCAAA
Sequence number 1994: Measles virus Whole genome #23 
GGATCAGCAGAGCAGTTGGACCCAGACAAGCCCAAGTGTCATTTCTACACGGTGATCAAAGTGAGAAT
GAGCTGCCGAGATTGGGGGGTAAGGAGGACAGGAGGGTCAAACAGAACCGAG
Sequence number 1995: Measles virus Whole genome #24 
GTGAGAATGAGCTGCCGAGATTGGGGGGTAAGGAGGACAGGAGGGTCAAACAGAACCGAGGAGAAGCC
CGGGAGAGCCACAGAGAAACCGAGCCCAGCAGAGCAAGTGATGCGAGAGCTG
Sequence number 1996: Measles virus Whole genome #25 
GAGAAGCCCGGGAGAGCCACAGAGAAACCGAGCCCAGCAGAGCAAGTGATGCGAGAGCTGCCCATCCT
CCAACCGGCACACCCCTAGACATTGACACTGCATCGGAGTTCAGCCAAGATC
Sequence number 1997: Measles virus Whole genome #26 
CCCATCCTCCAACCGGCACACCCCTAGACATTGACACTGCATCGGAGTTCAGCCAAGATCCGCAGGAC
AGTCGAAGGTCAGCCGATGCCCTGCTTAGGCTGCAAGCCATGGCAGGAATCT
Sequence number 1998: Measles virus Whole genome #27 
CGCAGGACAGTCGAAGGTCAGCCGATGCCCTGCTTAGGCTGCAAGCCATGGCAGGAATCTCGGAAGAA
CAAGACTCAGACACGGACACCCCTAGAGTGTACAATGATAGAGACCTTCTAG
Sequence number 1999: Measles virus Whole genome #28 
CGGAAGAACAAGACTCAGACACGGACACCCCTAGAGTGTACAATGATAGAGACCTTCTAGACTAGGTG
CGAGGGGTCGAGGACCAGAACAACATCTGCCTACCCTCCATCATTGTTATAA
Sequence number 2000: Measles virus Whole genome #29 ACTAGGTGCGAGGGGTCGAGGACCAGAACAACATCTGCCTACCCTCCATCATTGTTATAAAAAACTTA GGAACCAGGTCCACACAGCCGCCAGCCCATCAACCATCCACTCCCACGATCG
Sequence number 2001: Measles virus Whole genome #30 
AAAACTTAGGAACCAGGTCCACACAGCCGCCAGCCCATCAACCATCCACTCCCACGATCGGAGCCGAT
GGCAGAGGAGCAGGCACGCCATGTCAAAAACGGACTGGAATGCATCCGGGCT
Sequence number 2002: Measles virus Whole genome #31 
GAGCCGATGGCAGAGGAGCAGGCACGCCATGTCAAAAACGGACTGGAATGCATCCGGGCTCTCAAAGC
CGAGCCCATCGGCACACTGGCCATCGAGGAAGCCATGGCAGCATGGTCAGAA
Sequence number 2003: Measles virus Whole genome #32 
CTCAAAGCCGAGCCCATCGGCACACTGGCCATCGAGGAAGCCATGGCAGCATGGTCAGAAATATCAGA
CAACCCAGGACAGGAGCGAGCCACCTGCAAGAAAGAGAAGGCAGGCAGTTCG
Sequence number 2004: Measles virus Whole genome #33 
ATATCAGACAACCCAGGACAGGAGCGAGCCACCTGCAAGAAAGAGAAGGCAGGCAGTTCGGGTCTCAG
CAAACCATGCCTCTCAGCAATTGGATCAACTGAAGGCAGTGCACCTCGCATC
Sequence number 2005: Measles virus Whole genome #34 
GGTCTCAGCAAACCATGCCTCTCAGCAATTGGATCAACTGAAGGCAGTGCACCTCGCATCCGCGGTCA
GGGATCTGGAGAGAGCGATGACGACGCTGAAACTTTGGGAGCCCCCTCAGGA
Sequence number 2006: Measles virus Whole genome #35 
CGCGGTCAGGGATCTGGAGAGAGCGATGACGACGCTGAAACTTTGGGAGCCCCCTCAGGAAATCTCCA
GGCATCAAGCGCTGGGTTACAGCGTTATTATGTTTATGATCACAGCGGTGAA
Sequence number 2007: Measles virus Whole genome #36 
AATCTCCAGGCATCAAGCGCTGGGTTACAGCGTTATTATGTTTATGATCACAGCGGTGAAGCGATTAA
GGGAATCCAAGATGCTGACTCTATCATGGTTCAATCAGGCCTTGAAGGTGAT
Sequence number 2008: Measles virus Whole genome #37 
GCGATTAAGGGAATCCAAGATGCTGACTCTATCATGGTTCAATCAGGCCTTGAAGGTGATAGCACCTT
CTCAGGAGGAGACAATGAATCTGAAAACAGCGATGTGGATATTGGCGAACCT
Sequence number 2009: Measles virus Whole genome #38 
AGCACCTTCTCAGGAGGAGACAATGAATCTGAAAACAGCGATGTGGATATTGGCGAACCTGATACCGA
GGGATATGCTATCACTGACCGGGGATCTGCTCCCATCTCTATGGGGTTCAGA
Sequence number 2010: Measles virus Whole genome #39 
GATACCGAGGGATATGCTATCACTGACCGGGGATCTGCTCCCATCTCTATGGGGTTCAGAGCTTCTGA
TGTTGAAACTGCAGAAGGAGGGGAGATCCACGAGCTCCTGAGACTCCAATCC
Sequence number 2011: Measles virus Whole genome #40 
GCTTCTGATGTTGAAACTGCAGAAGGAGGGGAGATCCACGAGCTCCTGAGACTCCAATCCAGAGGCAA
CAACTTTCTGAAGCCTGGGAAGACTCTCAATGTTCCCCCGCCTCCGGACCCC
Sequence number 2012: Measles virus Whole genome #41 
AGAGGCAACAACTTTCTGAAGCCTGGGAAGACTCTCAATGTTCCCCCGCCTCCGGACCCCGGTAGAGC
CGACACTTCTGAGACACCCATTAAAAAGGGCACAGACGCGAGATTAGCCTCA
Sequence number 2013: Measles virus Whole genome #42 
GGTAGAGCCGACACTTCTGAGACACCCATTAAAAAGGGCACAGACGCGAGATTAGCCTCATTTGGAAC
GGAGATCGCGTCTTTATTGACAGGTGGTGCAACCCAATGTGCTCGAAAGTCG
Sequence number 2014: Measles virus Whole genome #43 
TTTGGAACGGAGATCGCGTCTTTATTGACAGGTGGTGCAACCCAATGTGCTCGAAAGTCGCCCTCGGA
ACCATCAGGGCCAGGTGCACCTGCGGAGAATGTCCCCGAGTATGTGAGCAAT
Sequence number 2015: Measles virus Whole genome #44 
CCCTCGGAACCATCAGGGCCAGGTGCACCTGCGGAGAATGTCCCCGAGTATGTGAGCAATGCCGCACT
GATACAGGAGTGGACACCCGAATCTGGTACCACAATCTCCCCGAGATCCCAG
Sequence number 2016: Measles virus Whole genome #45 
GCCGCACTGATACAGGAGTGGACACCCGAATCTGGTACCACAATCTCCCCGAGATCCCAGAATAATGA
AGAAGGGGGAGAATACTATGATGATGAGCTGTTCTCTGATGTCCAAGATATC
Sequence number 2017: Measles virus Whole genome #46 
AATAATGAAGAAGGGGGAGAATACTATGATGATGAGCTGTTCTCTGATGTCCAAGATATCAAAACAGC
CCTGGCCAAAATACACGAGGACAACCAGAAGATAATCTCCAAGCTAGAATCA
Sequence number 2018: Measles virus Whole genome #47 
AAAACAGCCCTGGCCAAAATACACGAGGACAACCAGAAGATAATCTCCAAGCTAGAATCACTGCTGTT
ATTGAAAGGAGAAGTTGAGTCAATCAAGAAGCAGATCAACAAGCAAAATATC
Sequence number 2019: Measles virus Whole genome #48 
CTGCTGTTATTGAAAGGAGAAGTTGAGTCAATCAAGAAGCAGATCAACAAGCAAAATATCAGCATATC
CACCCTAGAAGGGCACCTCTCAAGCATCATGATAGCCATTCCTGGATTTGGG
Sequence number 2020: Measles virus Whole genome #49 
AGCATATCCACCCTAGAAGGGCACCTCTCAAGCATCATGATAGCCATTCCTGGATTTGGGAAGGATCC
CAACGACCCTACTGCAGATGTCGAAATCAATCCCGACTTGAAACCCATCATA
Sequence number 2021: Measles virus Whole genome #50 
AAGGATCCCAACGACCCTACTGCAGATGTCGAAATCAATCCCGACTTGAAACCCATCATAAGTAGAGA
TTCAGGCCGAGCACTGGCCGAAGTTCTCAAGAAACCCATTGCCAGCCGACAA
Sequence number 2022: Measles virus Whole genome #51 
AGTAGAGATTCAGGCCGAGCACTGGCCGAAGTTCTCAAGAAACCCATTGCCAGCCGACAACTCCAAGG
AATGACAAATGGACGGACCAGTTCCAGAGGACAGCTACTTAAGGAATTTCAA
Sequence number 2023: Measles virus Whole genome #52 
CTCCAAGGAATGACAAATGGACGGACCAGTTCCAGAGGACAGCTACTTAAGGAATTTCAACTAAAGCC
GATCGGGAAAAAGATGAGCTCAGCCGTCGGGTTTGTTCCTGACACCGGCCCT
Sequence number 2024: Measles virus Whole genome #53 
CTAAAGCCGATCGGGAAAAAGATGAGCTCAGCCGTCGGGTTTGTTCCTGACACCGGCCCTGCATCACG
CAGTGTAATCCGCTCCATTATAAAATCTAGCCGGCTAGAGGAGGATCGGAAG
Sequence number 2025: Measles virus Whole genome #54 
GCATCACGCAGTGTAATCCGCTCCATTATAAAATCTAGCCGGCTAGAGGAGGATCGGAAGCGTTACCT
GATGACTCTCCTTGATGATATCAAAGGAGCCAACGATCTTGCCAAGTTCCAC
Sequence number 2026: Measles virus Whole genome #55 
CGTTACCTGATGACTCTCCTTGATGATATCAAAGGAGCCAACGATCTTGCCAAGTTCCACCAGATGCT
GATGAAAATAATAATGAAGTAGCTACAGCTCAACTTACCTGCCAACCCCATG
Sequence number 2027: Measles virus Whole genome #56 
CAGATGCTGATGAAAATAATAATGAAGTAGCTACAGCTCAACTTACCTGCCAACCCCATGCCAGTCGA
CCCAACCAGTACAACCTAAGTCCATTATAAAAAACTTAGGAGCAAAGTGATT
Sequence number 2028: Measles virus Whole genome #57 CCAGTCGACCCAACCAGTACAACCTAAGTCCATTATAAAAAACTTAGGAGCAAAGTGATTGCCTCCCA AGCTCCACAATGACAGAGATCTACGACTTCGACAAGTCGGCATGGGACATCA
Sequence number 2029: Measles virus Whole genome #58 
GCCTCCCAAGCTCCACAATGACAGAGATCTACGACTTCGACAAGTCGGCATGGGACATCAAAGGGTCG
ATCGCTCCGATTCAACCCACCACATACAGTGATGGGAGGCTGGTGCCCCAGG
Sequence number 2030: Measles virus Whole genome #59 
AAGGGTCGATCGCTCCGATTCAACCCACCACATACAGTGATGGGAGGCTGGTGCCCCAGGTCAGAGTC
ATAGATCCTGGTCTAGGCGACAGGAAGGATGAATGCTTTATGTACATGTTTC
Sequence number 2031: Measles virus Whole genome #60 
TCAGAGTCATAGATCCTGGTCTAGGCGACAGGAAGGATGAATGCTTTATGTACATGTTTCTGCTGGGG
GTTGTTGAGGACAGCGATCCCCTAGGGCCCCCAATCGGGCGAGCATTTGGGT
Sequence number 2032: Measles virus Whole genome #61 
TGCTGGGGGTTGTTGAGGACAGCGATCCCCTAGGGCCCCCAATCGGGCGAGCATTTGGGTCCCTGCCC
CTAGGTGTTGGCAGATCCACAGCAAAACCCGAAGAACTCCTCAAGGAGGCCA
Sequence number 2033: Measles virus Whole genome #62 
CCCTGCCCCTAGGTGTTGGCAGATCCACAGCAAAACCCGAAGAACTCCTCAAGGAGGCCACTGAGCTT
GACATAGTTGTTAGACGTACAGCAGGGCTCAATGAAAAACTGGTGTTCTACA
Sequence number 2034: Measles virus Whole genome #63 
CTGAGCTTGACATAGTTGTTAGACGTACAGCAGGGCTCAATGAAAAACTGGTGTTCTACAACAACACT
CCACTAACCCTCCTCACACCTTGGAGAAAGGTCCTGACAACAGGGAGTGTCT
Sequence number 2035: Measles virus Whole genome #64 
ACAACACTCCACTAACCCTCCTCACACCTTGGAGAAAGGTCCTGACAACAGGGAGTGTCTTCAACGCA
AATCAAGTGTGCAATGCGGTTAATCTGATACCGCTGGATACCCCGCAGAGGT
Sequence number 2036: Measles virus Whole genome #65 
TCAACGCAAATCAAGTGTGCAATGCGGTTAATCTGATACCGCTGGATACCCCGCAGAGGTTCCGTGTT
GTTTATATGAGCATCACCCGTCTTTCAGATAACGGGTATTACACTGTTCCTA
Sequence number 2037: Measles virus Whole genome #66 
TCCGTGTTGTTTATATGAGCATCACCCGTCTTTCAGATAACGGGTATTACACTGTTCCTAGAAGAATG
CTGGAATTCAGATCAGTCAATGCAGTGGCCTTCAACCTGCTGGTGACCCTTA
Sequence number 2038: Measles virus Whole genome #67 
GAAGAATGCTGGAATTCAGATCAGTCAATGCAGTGGCCTTCAACCTGCTGGTGACCCTTAGGATTGAC
AAGGCAATTGGCCCTGGGAAGATCATCGACAATACAGAGCAACTTCCTGAGG
Sequence number 2039: Measles virus Whole genome #68 
GGATTGACAAGGCAATTGGCCCTGGGAAGATCATCGACAATACAGAGCAACTTCCTGAGGCAACATTT
ATGGTTCACATCGGGAACTTCAGGAGAAAGAAGAATGAAGTCTACTCTGCCG
Sequence number 2040: Measles virus Whole genome #69 
CAACATTTATGGTTCACATCGGGAACTTCAGGAGAAAGAAGAATGAAGTCTACTCTGCCGATTATTGC
AAAATGAAGATCGAAAAGATGGGTCTAGTTTTTGCACTTGGCGGGATAGGGG
Sequence number 2041: Measles virus Whole genome #70 
ATTATTGCAAAATGAAGATCGAAAAGATGGGTCTAGTTTTTGCACTTGGCGGGATAGGGGGCACCAGT
CTTCACATTAGAAGCACAGGCAAAATGAGCAAGACCCTCCATGCACAACTCG
Sequence number 2042: Measles virus Whole genome #71 
GCACCAGTCTTCACATTAGAAGCACAGGCAAAATGAGCAAGACCCTCCATGCACAACTCGGGTTCAAG
AAGACCTTATGTTACCCACTGATGGATATCAATGAAGACCTTAATCGATTAC
Sequence number 2043: Measles virus Whole genome #72 
GGTTCAAGAAGACCTTATGTTACCCACTGATGGATATCAATGAAGACCTTAATCGATTACTCTGGAGG
AGCAGATGCAAGATAGTAAGAATCCAGGCAGTCTTGCAGCCATCAGTTCCTC
Sequence number 2044: Measles virus Whole genome #73 
TCTGGAGGAGCAGATGCAAGATAGTAAGAATCCAGGCAGTCTTGCAGCCATCAGTTCCTCAAGAATTC
CGCATTTACGATGACGTGATCATAAATGATGATCAAGGACTCTTCAAAGTTC
Sequence number 2045: Measles virus Whole genome #74 
AAGAATTCCGCATTTACGATGACGTGATCATAAATGATGATCAAGGACTCTTCAAAGTTCTGTAGACC
GTAGTGCTCAGCAGTGCCCAAAAACAACCCCCCTCACAATGACAGTCAGAAT
Sequence number 2046: Measles virus Whole genome #75 
TGTAGACCGTAGTGCTCAGCAGTGCCCAAAAACAACCCCCCTCACAATGACAGTCAGAATTCCCGGAC
AAAAAAGCCCCCTCCGAGAGACTCCACGGACTAAGCGAGAGGCCAACTAGCA
Sequence number 2047: Measles virus Whole genome #76 
TCCCGGACAAAAAAGCCCCCTCCGAGAGACTCCACGGACTAAGCGAGAGGCCAACTAGCAGCCGACGG
CAAGCGCGAACACCAGGTGGCCCCAGCATAGGAGAGTCCCGACCCAGGGCCA
Sequence number 2048: Measles virus Whole genome #77 
GCCGACGGCAAGCGCGAACACCAGGTGGCCCCAGCATAGGAGAGTCCCGACCCAGGGCCACCACCAGC
TATCCCAATCCGCATCATCCTCGTGTGACCCCCGAGGACCAGCCTCCAAGGT
Sequence number 2049: Measles virus Whole genome #78 
CCACCAGCTATCCCAATCCGCATCATCCTCGTGTGACCCCCGAGGACCAGCCTCCAAGGTTGCCCCCG
ACCCAAACCACCAACCACATCCCCACCACCCCCGGGAAAGAAACCCCCAGCA
Sequence number 2050: Measles virus Whole genome #79 
TGCCCCCGACCCAAACCACCAACCACATCCCCACCACCCCCGGGAAAGAAACCCCCAGCAGCTGGAAG
GCCCCTCTCCCCCTCTCCCAACACAAGAACCCCACAACCGGATCGCACAAGC
Sequence number 2051: Measles virus Whole genome #80 
GCTGGAAGGCCCCTCTCCCCCTCTCCCAACACAAGAACCCCACAACCGGATCGCACAAGCGACCGAGG
TGACCCAACCACAGGCACCCGACTCCCCAGGCAAATCCTCTCCCCCCGGCAA
Sequence number 2052: Measles virus Whole genome #81 
GACCGAGGTGACCCAACCACAGGCACCCGACTCCCCAGGCAAATCCTCTCCCCCCGGCAAACTAAACA
AAACTTAGGGCCAAGGAACACACACACCCAACAGAACCCAGACCCCGGCCCA
Sequence number 2053: Measles virus Whole genome #82 
ACTAAACAAAACTTAGGGCCAAGGAACACACACACCCAACAGAACCCAGACCCCGGCCCACGGCACCG
CGCCCCCAACCCCCGACAACCAGAGGGAGCCCCCAACCAATCCCGCCGGCTC
Sequence number 2054: Measles virus Whole genome #83 
CGGCACCGCGCCCCCAACCCCCGACAACCAGAGGGAGCCCCCAACCAATCCCGCCGGCTCCCCCGGCG
CCCACAGGCAGGCACACCAACCCCCGAACAGACTCAGCACCCAGCCACCTAC
Sequence number 2055: Measles virus Whole genome #84 
CCCCGGCGCCCACAGGCAGGCACACCAACCCCCGAACAGACTCAGCACCCAGCCACCTACAATCCAAG
ACGGGGGGGCCCCCCAAAAAGGGCCCCCAGGGGCCGACAACCAGGGCAGC
Sequence number 2056: Measles virus Whole genome #85 AATCCAAGACGGGGGGCCCCCCAAAAAGGCCCCCAGGGGCCGACAACCAGGGCAGCGAGGAAGC CCACCCACACCACACACCATCACGGCAACCAGATCAGAACCCAGACCACCCT
Sequence number 2057: Measles virus Whole genome #86 
GAGGAAGCCCACCCACACCACACACCATCACGGCAACCAGATCAGAACCCAGACCACCCTGGGCCACC
AACTCCCAGACCCGGCCACCACCCCGCGGAAAGGGACGGCCACAACCCGCAC
Sequence number 2058: Measles virus Whole genome #87 
GGGCCACCAACTCCCAGACCCGGCCACCACCCCGCGGAAAGGGACGGCCACAACCCGCACACCCCAGC
CCCGATCCGGCGGGCAGCCACCCAACCCGAACCAGCACCCAAGAGCGACCCC
Sequence number 2059: Measles virus Whole genome #88 
ACCCCAGCCCCGATCCGGCGGGCAGCCACCCAACCCGAACCAGCACCCAAGAGCGACCCCCGAAGGAC
CCCCAAACCGCAAAGGACATCAGCATCCCACAGCCTCTCCAAGTCCCCCGGT
Sequence number 2060: Measles virus Whole genome #89 
CGAAGGACCCCCAAACCGCAAAGGACATCAGCATCCCACAGCCTCTCCAAGTCCCCCGGTCTCCCCCC
CTTCTCGAAGGGACAAAAAGATCAACCCACTATATCCGACGTCACTCAACTC
Sequence number 2061: Measles virus Whole genome #90 
CTCCCCCCCTTCTCGAAGGGACAAAAAGATCAACCCACTATATCCGACGTCACTCAACTCCCCACCCC
TAGAGAAGACACCAGGGATCCCAGAATCAAGACTCATCCAATATCCATCATG
Sequence number 2062: Measles virus Whole genome #91 
CCCACCCCTAGAGAAGACACCAGGGATCCCAGAATCAAGACTCATCCAATATCCATCATGGGTCTCAA
GGTGAACGTCTCTGCCGTATTCATGGCAGTACTGTTAACTCTCCAAACACCC
Sequence number 2063: Measles virus Whole genome #92 
GGTCTCAAGGTGAACGTCTCTGCCGTATTCATGGCAGTACTGTTAACTCTCCAAACACCCGCCGGTCA
AATTCATTGGGGCAATCTCTCTAAGATAGGGGTAGTAGGAATAGGAAGTGCA
Sequence number 2064: Measles virus Whole genome #93 
GCCGGTCAAATTCATTGGGGCAATCTCTCTAAGATAGGGGTAGTAGGAATAGGAAGTGCAAGCTACAA
AGTTATGACTCGTTCCAGCCATCAATCATTAGTCATAAAATTAATGCCCAAT
Sequence number 2065: Measles virus Whole genome #94 
AGCTACAAAGTTATGACTCGTTCCAGCCATCAATCATTAGTCATAAAATTAATGCCCAATATAACTCT
CCTCAATAACTGCACGAGGGTAGAGATTGCAGAATACAGGAGGCTACTAAGA
Sequence number 2066: Measles virus Whole genome #95 
ATAACTCTCCTCAATAACTGCACGAGGGTAGAGATTGCAGAATACAGGAGGCTACTAAGAACAGTTTT
GGAACCAATTAGAGATGCACTTAATGCAATGACCCAGAACATAAGGCCGGTT
Sequence number 2067: Measles virus Whole genome #96 
ACAGTTTTGGAACCAATTAGAGATGCACTTAATGCAATGACCCAGAACATAAGGCCGGTTCAGAGCGT
AGCTTCAAGTAGGAGACACAAGAGATTTGCGGGAGTAGTCCTGGCAGGTGCG
Sequence number 2068: Measles virus Whole genome #97 
CAGAGCGTAGCTTCAAGTAGGAGACACAAGAGATTTGCGGGAGTAGTCCTGGCAGGTGCGGCCCTAGG
TGTTGCCACATCTGCTCAGATAACAGCCGGCATTGCACTTCACCGGTCCATG
Sequence number 2069: Measles virus Whole genome #98 
GCCCTAGGTGTTGCCACATCTGCTCAGATAACAGCCGGCATTGCACTTCACCGGTCCATGCTGAACTC
TCAGGCCATCGACAATCTGAGAGCGAGCCTGGAAACTACTAATCAGGCAATT
Sequence number 2070: Measles virus Whole genome #99 
CTGAACTCTCAGGCCATCGACAATCTGAGAGCGAGCCTGGAAACTACTAATCAGGCAATTGAGGCAAT
CAGACAAGCAGGGCAGGAGATGATATTGGCTGTTCAGGGTGTCCAAGACTAC
Sequence number 2071: Measles virus Whole genome #100 
GAGGCAATCAGACAAGCAGGGCAGGAGATGATATTGGCTGTTCAGGGTGTCCAAGACTACATCAATAA
TGAGCTGATACCGTCTATGAACCAGCTGTCTTGTGATCTAATCGGTCAGAAG
Sequence number 2072: Measles virus Whole genome #101 
ATCAATAATGAGCTGATACCGTCTATGAACCAGCTGTCTTGTGATCTAATCGGTCAGAAGCTCGGGCT
CAAATTGCTTAGATACTATACAGAAATCCTGTCACTATTTGGCCCCAGCCTA
Sequence number 2073: Measles virus Whole genome #102 
CTCGGGCTCAAATTGCTTAGATACTATACAGAAATCCTGTCACTATTTGGCCCCAGCCTACGGGACCC
CATATCTGCGGAGATATCTATCCAGGCTTTGAGCTATGCACTTGGAGGAGAT
Sequence number 2074: Measles virus Whole genome #103 
CGGGACCCCATATCTGCGGAGATATCTATCCAGGCTTTGAGCTATGCACTTGGAGGAGATATCAATAA
GGTGTTAGAAAAGCTCGGATACAGTGGAGGCGATTTACTAGGCATCTTAGAG
Sequence number 2075: Measles virus Whole genome #104 
ATCAATAAGGTGTTAGAAAAGCTCGGATACAGTGGAGGCGATTTACTAGGCATCTTAGAGAGCAGAGG
AATAAAGGCTCGGATAACTCACGTCGACACAGAGTCCTACTTCATAGTCCTC
Sequence number 2076: Measles virus Whole genome #105 
AGCAGAGGAATAAAGGCTCGGATAACTCACGTCGACACAGAGTCCTACTTCATAGTCCTCAGTATAGC
CTATCCGACGCTGTCCGAGATTAAGGGGGTGATTGTCCACCGGCTAGAGGGG
Sequence number 2077: Measles virus Whole genome #106 
AGTATAGCCTATCCGACGCTGTCCGAGATTAAGGGGGTGATTGTCCACCGGCTAGAGGGGGTCTCGTA
TAACATAGGCTCTCAAGAGTGGTATACCACTGTGCCCAAGTATGTTGCAACC
Sequence number 2078: Measles virus Whole genome #107 
GTCTCGTATAACATAGGCTCTCAAGAGTGGTATACCACTGTGCCCAAGTATGTTGCAACCCAAGGGTA
CCTTATCTCGAATTTTGATGAGTCGTCATGTACTTTCATGCCAGAGGGGACT
Sequence number 2079: Measles virus Whole genome #108 
CAAGGGTACCTTATCTCGAATTTTGATGAGTCGTCATGTACTTTCATGCCAGAGGGGACTGTGTGCAG
CCAAAATGCCTTGTACCCGATGAGTCCTCTGCTCCAAGAATGCCTCCGGGGG
Sequence number 2080: Measles virus Whole genome #109 
GTGTGCAGCCAAAATGCCTTGTACCCGATGAGTCCTCTGCTCCAAGAATGCCTCCGGGGGTCCACCAA
GTCCTGTGCTCGTACACTTGTATCCGGGTCTTTTGGGAACCGGTTCATTTTA
Sequence number 2081: Measles virus Whole genome #110 
TCCACCAAGTCCTGTGCTCGTACACTTGTATCCGGGTCTTTTGGGAACCGGTTCATTTTATCACAAGG
GAACCTAATAGCCAATTGTGCATCAATTCTCTGTAAGTGTTACACAACAGGT
Sequence number 2082: Measles virus Whole genome #111 
TCACAAGGGAACCTAATAGCCAATTGTGCATCAATTCTCTGTAAGTGTTACACAACAGGTACGATTAT
TAATCAAGACCCTGACAAGATCCTAACATACATTGCTGCCGATCGCTGCCCG
Sequence number 2083: Measles virus Whole genome #112 
ACGATTATTAATCAAGACCCTGACAAGATCCTAACATACATTGCTGCCGATCGCTGCCCGGTAGTCGA
GGTGAACGGCGTGACCATCCAAGTCGGGAGCAGGAGGTATCCAGATGCTGTG
Sequence number 2084: Measles virus Whole genome #113 GTAGTCGAGGTGAACGGCGTGACCATCCAAGTCGGGAGCAGGAGGTATCCAGATGCTGTGTACTTGCA CAGAATTGACCTCGGTCCTCCCATATCATTGGAGAGATTGGACGTAGGGACA
Sequence number 2085: Measles virus Whole genome #114 
TACTTGCACAGAATTGACCTCGGTCCTCCCATATCATTGGAGAGATTGGACGTAGGGACAAATCTGGG
GAATGCAATTGCCAAACTGGAGGATGCCAAGGAATTGTTGGAATCATCGGAC
Sequence number 2086: Measles virus Whole genome #115 
AATCTGGGGAATGCAATTGCCAAACTGGAGGATGCCAAGGAATTGTTGGAATCATCGGACCAGATATT
GAGAAGTATGAAAGGTTTATCGAGCACTAGCATAGTCTACATCCTGATTGCA
Sequence number 2087: Measles virus Whole genome #116 
CAGATATTGAGAAGTATGAAAGGTTTATCGAGCACTAGCATAGTCTACATCCTGATTGCAGTGTGTCT
TGGAGGGTTGATAGGGATCCCCACTTTAATATGTTGCTGCAGGGGGCGTTGT
Sequence number 2088: Measles virus Whole genome #117 
GTGTGTCTTGGAGGGTTGATAGGGATCCCCACTTTAATATGTTGCTGCAGGGGGCGTTGTAACAAAAA
GGGAGAACAAGTTGGTATGTCAAGACCAGGCCTAAAGCCTGACCTTACGGGA
Sequence number 2089: Measles virus Whole genome #118 
AACAAAAAGGGAGAACAAGTTGGTATGTCAAGACCAGGCCTAAAGCCTGACCTTACGGGAACATCAAA
ATCCTATGTAAGATCGCTTTGATCCTCTACAACTCTTGAAACACAAGTGTCC
Sequence number 2090: Measles virus Whole genome #119 
ACATCAAAATCCTATGTAAGATCGCTTTGATCCTCTACAACTCTTGAAACACAAGTGTCCCAGAATTC
TCCTCTTAGTCATCAAGCAACCACCGTGCCCGACATCAAGCCACCCTGAAAT
Sequence number 2091: Measles virus Whole genome #120 
CAGAATTCTCCTCTTAGTCATCAAGCAACCACCGTGCCCGACATCAAGCCACCCTGAAATTTTCTCCG
GCTTCCCTCTAGCCGAACAATATCGATAATTAATTAAAACTTAGGGTGCAAG
Sequence number 2092: Measles virus Whole genome #121 
TTTCTCCGGCTTCCCTCTAGCCGAACAATATCGATAATTAATTAAAACTTAGGGTGCAAGACCATCCA
CAATGTCACCGCAACGAGACCGGATAAATGCCTTTTACAAAGATAACCCTTA
Sequence number 2093: Measles virus Whole genome #122 
ACCATCCACAATGTCACCGCAACGAGACCGGATAAATGCCTTTTACAAAGATAACCCTTATCCCAAGG
GAAGTAGGATAGTTATTAACAGAGAACATCTTATGATTGACAGACCCTATGT
Sequence number 2094: Measles virus Whole genome #123 
TCCCAAGGGAAGTAGGATAGTTATTAACAGAGAACATCTTATGATTGACAGACCCTATGTTCTGCTGG
CTGTTCTGTTCGTCATGTTTCTGAGCTTGATCGGATTGCTGGCAATTGCAGG
Sequence number 2095: Measles virus Whole genome #124 
TCTGCTGGCTGTTCTGTTCGTCATGTTTCTGAGCTTGATCGGATTGCTGGCAATTGCAGGCATTAGAC
TTCATCGAGCAGCCATCTACACCGCGGAGATCCATAAAAGCCTCAGTACCAA
Sequence number 2096: Measles virus Whole genome #125 
CATTAGACTTCATCGAGCAGCCATCTACACCGCGGAGATCCATAAAAGCCTCAGTACCAATCTAGATG
TGACTAACTCAATCGAGCATCAAGTCAAGGACGTGCTGACACCACTCTTTAA
Sequence number 2097: Measles virus Whole genome #126 
TCTAGATGTGACTAACTCAATCGAGCATCAAGTCAAGGACGTGCTGACACCACTCTTTAAGATCATCG
GGGATGAAGTGGGCCTGAGAACACCTCAGAGATTCACTGACCTAGTGAAATT
Sequence number 2098: Measles virus Whole genome #127 
GATCATCGGGGATGAAGTGGGCCTGAGAACACCTCAGAGATTCACTGACCTAGTGAAATTCATCTCGG
ACAAGATTAAATTCCTTAATCCGGATAGGGAGTACGACTTCAGAGATCTCAC
Sequence number 2099: Measles virus Whole genome #128 
CATCTCGGACAAGATTAAATTCCTTAATCCGGATAGGGAGTACGACTTCAGAGATCTCACTTGGTGCA
TCAATCCGCCAGAGAGGATCAAACTAGATTATGATCAATACTGTGCAGATGT
Sequence number 2100: Measles virus Whole genome #129 
TTGGTGCATCAATCCGCCAGAGAGGATCAAACTAGATTATGATCAATACTGTGCAGATGTGGCTGCTG
AAGAGCTCATGAATACATTGGTGAACTCAACTCTACTGGAGACCAGAACAAC
Sequence number 2101: Measles virus Whole genome #130 
GGCTGCTGAAGAGCTCATGAATACATTGGTGAACTCAACTCTACTGGAGACCAGAACAACCAATCAGT
TCCTAGCTGTCTCAAAGGGAAACTGCTCAGGGCCCACTACAATCAGAGGTCA
Sequence number 2102: Measles virus Whole genome #131 
CAATCAGTTCCTAGCTGTCTCAAAGGGAAACTGCTCAGGGCCCACTACAATCAGAGGTCAATTCTCAA
ACATGTCGCTGTCCTTGTTGGACTTGTACTTAGGTCGAGGTTACAATGTGTC
Sequence number 2103: Measles virus Whole genome #132 
ATTCTCAAACATGTCGCTGTCCTTGTTGGACTTGTACTTAGGTCGAGGTTACAATGTGTCATCTATAG
TCACTATAACATCCCAGGGAATGTATGGGGGAACCTACCTAGTTGAAAAGCC
Sequence number 2104: Measles virus Whole genome #133 
ATCTATAGTCACTATAACATCCCAGGGAATGTATGGGGGAACCTACCTAGTTGAAAAGCCTAATCTGA
ACAGCAAAGGGTCAGAGTTGTCACAACTGAGCATGTACCGAGTGTTTGAAGT
Sequence number 2105: Measles virus Whole genome #134 
TAATCTGAACAGCAAAGGGTCAGAGTTGTCACAACTGAGCATGTACCGAGTGTTTGAAGTAGGTGTTA
TCAGAAACCCGGGTTTGGGGGCTCCGGTGTTCCATATGACAAACTATTTTGA
Sequence number 2106: Measles virus Whole genome #135 
AGGTGTTATCAGAAACCCGGGTTTGGGGGCTCCGGTGTTCCATATGACAAACTATTTTGAGCAACCAG
TCAGTGATGGTCTCGGCAACTGTATGGTGGCTTTGGGGGAGCTCAAACTCGC
Sequence number 2107: Measles virus Whole genome #136 
GCAACCAGTCAGTGATGGTCTCGGCAACTGTATGGTGGCTTTGGGGGAGCTCAAACTCGCAGCCCTTT
GTCACGGGGACGATTCTATCACAATTCCCTATCGGGGATCAGGGAAAGGTGT
Sequence number 2108: Measles virus Whole genome #137 
AGCCCTTTGTCACGGGGACGATTCTATCACAATTCCCTATCGGGGATCAGGGAAAGGTGTCAGCTTCC
AGCTCGTCAAGCTGGGTGTCTGGAAATCCCCAACCGACATGCAATCCTGGGT
Sequence number 2109: Measles virus Whole genome #138 
CAGCTTCCAGCTCGTCAAGCTGGGTGTCTGGAAATCCCCAACCGACATGCAATCCTGGGTCCCCTTAT
CAACGGATGATCCAGTAGTAGACAGGCTTTACCTCTCATCTCACAGAGGTGT
Sequence number 2110: Measles virus Whole genome #139 
CCCCTTATCAACGGATGATCCAGTAGTAGACAGGCTTTACCTCTCATCTCACAGAGGTGTCATCGCTG
ACAATCAAGCAAAATGGGCTGTCCCGACAACACGAACAGATGACAAGTTGCG
Sequence number 2111: Measles virus Whole genome #140 
CATCGCTGACAATCAAGCAAAATGGGCTGTCCCGACAACACGAACAGATGACAAGTTGCGAATGGAGA
CATGCTTCCAGCAGGCGTGTAAAGGTAAAATCCAAGCACTCTGCGAGAATCC
Sequence number 2112: Measles virus Whole genome #141 AATGGAGACATGCTTCCAGCAGGCGTGTAAAGGTAAAATCCAAGCACTCTGCGAGAATCCCGAGTGGG TACCATTGAAGGATAACAGGATTCCTTCATACGGGGTCCTGTCTGTTGATCT
Sequence number 2113: Measles virus Whole genome #142 
CGAGTGGGTACCATTGAAGGATAACAGGATTCCTTCATACGGGGTCCTGTCTGTTGATCTGAGTTTGA
CAGTTGAGCTTAAAATCAAAATTGCTTCGGGATTCGGGCCATTGATCACACA
Sequence number 2114: Measles virus Whole genome #143 
GAGTTTGACAGTTGAGCTTAAAATCAAAATTGCTTCGGGATTCGGGCCATTGATCACACACGGCTCAG
GGATGGACCTATACAAATCCAACCGCAACAATGTGTATTGGCTGACTATTCC
Sequence number 2115: Measles virus Whole genome #144 
CGGCTCAGGGATGGACCTATACAAATCCAACCGCAACAATGTGTATTGGCTGACTATTCCGCCAATGA
GAAATCTAGCCTTAGGCGTAATCAACACATTGGAGTGGATACCGAGATTCAA
Sequence number 2116: Measles virus Whole genome #145 
GCCAATGAGAAATCTAGCCTTAGGCGTAATCAACACATTGGAGTGGATACCGAGATTCAAAGTTAGTC
CCAACCTCTTCACTGTCCCAATTAAGGAAGCAGGCGAAGACTGCCATGCCCC
Sequence number 2117: Measles virus Whole genome #146 
AGTTAGTCCCAACCTCTTCACTGTCCCAATTAAGGAAGCAGGCGAAGACTGCCATGCCCCAACATACC
TACCTGCGGAGGTGGACGGTGATGTCAAACTCAGTTCCAACCTGGTGATTCT
Sequence number 2118: Measles virus Whole genome #147 
AACATACCTACCTGCGGAGGTGGACGGTGATGTCAAACTCAGTTCCAACCTGGTGATTCTACCTGGTC
AAGATCTCCAATATGTTTTGGCAACCTACGACACCTCCAGGGTTGAGCATGC
Sequence number 2119: Measles virus Whole genome #148 
ACCTGGTCAAGATCTCCAATATGTTTTGGCAACCTACGACACCTCCAGGGTTGAGCATGCTGTGGTTT
ATTACGTTTACAGCCCAAGCCGCTCATTTTCCTACTTTTATCCTTTTAGGTT
Sequence number 2120: Measles virus Whole genome #149 
TGTGGTTTATTACGTTTACAGCCCAAGCCGCTCATTTTCCTACTTTTATCCTTTTAGGTTGCCTATAA
AGGGGGTCCCAATCGAACTACAAGTGGAATGCTTCACATGGGACCAAAAACT
Sequence number 2121: Measles virus Whole genome #150 
GCCTATAAAGGGGGTCCCAATCGAACTACAAGTGGAATGCTTCACATGGGACCAAAAACTCTGGTGCC
GTCACTTCTGTGTGCTTGCGGACTCAGAATCCGGTGGACATATCACTCACTC
Sequence number 2122: Measles virus Whole genome #151 
CTGGTGCCGTCACTTCTGTGTGCTTGCGGACTCAGAATCCGGTGGACATATCACTCACTCTGGGATGG
TTGGCATGGGAGTCAGCTGCACAGCTACCCGGGAAGATGGAACCAATCGCAG
Sequence number 2123: Measles virus Whole genome #152 
TGGGATGGTTGGCATGGGAGTCAGCTGCACAGCTACCCGGGAAGATGGAACCAATCGCAGATAAGGCT
GTTAGTGAACCAATCACATGATGTCGCCCAGACATCAGGCATACCCACTAGT
Sequence number 2124: Measles virus Whole genome #153 
ATAAGGCTGTTAGTGAACCAATCACATGATGTCGCCCAGACATCAGGCATACCCACTAGTGTGAAATA
GACATCAGAATTAAGAAAAACGTAGGGTCCAAGTAGTTCCCCATTATGGACT
Sequence number 2125: Measles virus Whole genome #154 
GTGAAATAGACATCAGAATTAAGAAAAACGTAGGGTCCAAGTAGTTCCCCATTATGGACTCGCTATCT
GTCAACCAGATCTTATACCCTGAAGTTCATCTGGATAGTCCGATAGTCACCA
Sequence number 2126: Measles virus Whole genome #155 
CGCTATCTGTCAACCAGATCTTATACCCTGAAGTTCATCTGGATAGTCCGATAGTCACCAATAAGATA
GTAGCCATCCTGGAGTATGCTAGACTCCCTCACGCTTACAGCTTGGAGGACC
Sequence number 2127: Measles virus Whole genome #156 
ATAAGATAGTAGCCATCCTGGAGTATGCTAGACTCCCTCACGCTTACAGCTTGGAGGACCCTACATTG
TGTCAGAACATCAAGCACCGCCTAAAAACCGGATTTTCCAACCAAATGATTA
Sequence number 2128: Measles virus Whole genome #157 
CTACATTGTGTCAGAACATCAAGCACCGCCTAAAAACCGGATTTTCCAACCAAATGATTATAAACAAT
GTGGAAGTTGGGAATGTCATCAAGTCCAAGCTTAGGAGTTATCCGACCCACT
Sequence number 2129: Measles virus Whole genome #158 
TAAACAATGTGGAAGTTGGGAATGTCATCAAGTCCAAGCTTAGGAGTTATCCGACCCACTCTCATATT
CCATATCCAAATTGTAATCAGGATTTATTTAACATAGAAGACAAAGAGTCAA
Sequence number 2130: Measles virus Whole genome #159 
CTCATATTCCATATCCAAATTGTAATCAGGATTTATTTAACATAGAAGACAAAGAGTCAACGAGGAAG
ATCCGTGAACTTCTCAAAAAGGGAAATTTGCTGTACTCCAAAGTCAGTGATA
Sequence number 2131: Measles virus Whole genome #160 
CGAGGAAGATCCGTGAACTTCTCAAAAAGGGAAATTTGCTGTACTCCAAAGTCAGTGATAAGGTTTTC
CAATGCTTGAGGGACATTAACTCACGGCTTGGCCTAGGCTCCGAATTGAGGG
Sequence number 2132: Measles virus Whole genome #161 
AGGTTTTCCAATGCTTGAGGGACATTAACTCACGGCTTGGCCTAGGCTCCGAATTGAGGGAGGACATC
AAGGAGAAAGTTACTAACTTGGGAGTTTACATGCACAGCTCCCAATGGTTTG
Sequence number 2133: Measles virus Whole genome #162 
AGGACATCAAGGAGAAAGTTACTAACTTGGGAGTTTACATGCACAGCTCCCAATGGTTTGAGCCCTTT
CTGTTTTGGTTTACAGTCAAGACCGAGATGAGGTCAGTGATTAAATCACAAA
Sequence number 2134: Measles virus Whole genome #163 
AGCCCTTTCTGTTTTGGTTTACAGTCAAGACCGAGATGAGGTCAGTGATTAAATCACAAACCCATACT
TGCCATAGGAGGAGACACACACCTGTATTCTTCACTGGTAGTTCAGTTGAGT
Sequence number 2135: Measles virus Whole genome #164 
CCCATACTTGCCATAGGAGGAGACACACACCTGTATTCTTCACTGGTAGTTCAGTTGAGTTGCTAATC
TCTCGTGACCTTGTTGCTATAATAAGTAGAGAGTCTCAACATGTATATTACC
Sequence number 2136: Measles virus Whole genome #165 
TGCTAATCTCTCGTGACCTTGTTGCTATAATAAGTAGAGAGTCTCAACATGTATATTACCTGACATTT
GAGCTGGTTTTGATGTATTGTGATGTCATAGAGGGGAGGTTAATGACAGATA
Sequence number 2137: Measles virus Whole genome #166 
TGACATTTGAGCTGGTTTTGATGTATTGTGATGTCATAGAGGGGAGGTTAATGACAGATACCGCTATG
ACCATTGATGCTAGGTATACTGAGCTTCTAGGAAGAGTCAGGTACATGTGGA
Sequence number 2138: Measles virus Whole genome #167 
CCGCTATGACCATTGATGCTAGGTATACTGAGCTTCTAGGAAGAGTCAGGTACATGTGGAAACTGATA
GATGGTTTCTTCCCTGCACTCGGTAATCCAACTTATCAAATTGTAGCCATGC
Sequence number 2139: Measles virus Whole genome #168 
AACTGATAGATGGTTTCTTCCCTGCACTCGGTAATCCAACTTATCAAATTGTAGCCATGCTGGAGCCA
CTTTCACTTGCTTACCTGCAACTGAGGGATATAACAGTAGAACTCAGAGGTG
Sequence number 2140: Measles virus Whole genome #169 TGGAGCCACTTTCACTTGCTTACCTGCAACTGAGGGATATAACAGTAGAACTCAGAGGTGCTTTCCTT AACCACTGCTTTACTGAAATACATGATGTTCTTGACCAAAACGGGTTTTCTG
Sequence number 2141: Measles virus Whole genome #170 
CTTTCCTTAACCACTGCTTTACTGAAATACATGATGTTCTTGACCAAAACGGGTTTTCTGATGAAGGT
ACTTATCATGAGTTAATTGAAGCTCTAGATTACATCTTCATAACTGATGATA
Sequence number 2142: Measles virus Whole genome #171 
ATGAAGGTACTTATCATGAGTTAATTGAAGCTCTAGATTACATCTTCATAACTGATGATATACATCTG
ACAGGGGAGATTTTTTCATTTTTTAGAAGTTTCGGCCACCCCAGACTTGAAG
Sequence number 2143: Measles virus Whole genome #172 
TACATCTGACAGGGGAGATTTTTTCATTTTTTAGAAGTTTCGGCCACCCCAGACTTGAAGCAGTAACG
GCTGCTGAAAATGTTAGGAAATACATGAATCAGCCCAAAGTCATTGTGTATG
Sequence number 2144: Measles virus Whole genome #173 
CAGTAACGGCTGCTGAAAATGTTAGGAAATACATGAATCAGCCCAAAGTCATTGTGTATGAGACACTG
ATGAAAGGTCATGCCATATTTTGTGGAATCATAATCAATGGCTATCGTGACA
Sequence number 2145: Measles virus Whole genome #174 
AGACACTGATGAAAGGTCATGCCATATTTTGTGGAATCATAATCAATGGCTATCGTGACAGGCACGGA
GGCAGTTGGCCGCCGCTGACCCTCCCCCTACATGCTGCTGACACAATCCGAA
Sequence number 2146: Measles virus Whole genome #175 
GGCACGGAGGCAGTTGGCCGCCGCTGACCCTCCCCCTACATGCTGCTGACACAATCCGAAATGCTCAA
GCTTCAGGTGAAGGGTTAACACATGAGCAGTGCGTTGATAACTGGAGATCCT
Sequence number 2147: Measles virus Whole genome #176 
ATGCTCAAGCTTCAGGTGAAGGGTTAACACATGAGCAGTGCGTTGATAACTGGAGATCCTTTGCTGGA
GTGAAATTTGGCTGCTTTATGCCTCTTAGCCTGGATAGTGATCTGACAATGT
Sequence number 2148: Measles virus Whole genome #177 
TTGCTGGAGTGAAATTTGGCTGCTTTATGCCTCTTAGCCTGGATAGTGATCTGACAATGTACCTAAAG
GATAAGGCACTTGCTGCTCTCCAAAGAGAATGGGATTCAGTTTACCCGAAAG
Sequence number 2149: Measles virus Whole genome #178 
ACCTAAAGGATAAGGCACTTGCTGCTCTCCAAAGAGAATGGGATTCAGTTTACCCGAAAGAGTTCCTG
CGTTACGACCCTCCCAAGGGAACCGGGTCCCGGAGGCTTGTAGATGTTTTCC
Sequence number 2150: Measles virus Whole genome #179 
AGTTCCTGCGTTACGACCCTCCCAAGGGAACCGGGTCCCGGAGGCTTGTAGATGTTTTCCTTAATGAT
TCGAGCTTTGACCCATATGATATGATAATGTATGTTGTGAGTGGAGCTTACC
Sequence number 2151: Measles virus Whole genome #180 
TTAATGATTCGAGCTTTGACCCATATGATATGATAATGTATGTTGTGAGTGGAGCTTACCTCCATGAC
CCTGAGTTCAACTTGTCCTACAGTCTGAAGGAGAAGGAGATCAAGGAGACAG
Sequence number 2152: Measles virus Whole genome #181 
TCCATGACCCTGAGTTCAACTTGTCCTACAGTCTGAAGGAGAAGGAGATCAAGGAGACAGGTAGACTT
TTTGCTAAAATGACTTACAAAATGAGAGCATGCCAAGTGATTGCTGAAAATC
Sequence number 2153: Measles virus Whole genome #182 
GTAGACTTTTTGCTAAAATGACTTACAAAATGAGAGCATGCCAAGTGATTGCTGAAAATCTAATCTCA
AACGGCATTGGCAAATATTTTAAGGACAATGGGATGGCCAAGGATGAACATG
Sequence number 2154: Measles virus Whole genome #183 
TAATCTCAAACGGCATTGGCAAATATTTTAAGGACAATGGGATGGCCAAGGATGAACATGATTTGACT
AAGGCACTCCACACTCTAGCTGTCTCAGGAATCCCCAAAGATCTCAAAGAAA
Sequence number 2155: Measles virus Whole genome #184 
ATTTGACTAAGGCACTCCACACTCTAGCTGTCTCAGGAATCCCCAAAGATCTCAAAGAAAGTCACAGA
GGGGGGCCAATCTTAAAAACCTACTCCCGAAACTCAGTTCACACAAGTACCA
Sequence number 2156: Measles virus Whole genome #185 
GTCACAGAGGGGGGCCAATCTTAAAAACCTACTCCCGAAACTCAGTTCACACAAGTACCAGGAACGTG
AGAGCAGCAAAAGGGTTTATAGGGTTCCCTCAAGTAATTCAGCAGGGCCAAG
Sequence number 2157: Measles virus Whole genome #186 
GGAACGTGAGAGCAGCAAAAGGGTTTATAGGGTTCCCTCAAGTAATTCAGCAGGGCCAAGACACTGAT
CATCCGGAGAATGTGGAAGCCTACGAGACAGTCAGTGCATTTATCACGACTG
Sequence number 2158: Measles virus Whole genome #187 
ACACTGATCATCCGGAGAATGTGGAAGCCTACGAGACAGTCAGTGCATTTATCACGACTGATCTCAAG
AAGTACTGCCTTAATTGGAGATATGAGACCATCAGCCTGTTTGCACAGAGGC
Sequence number 2159: Measles virus Whole genome #188 
ATCTCAAGAAGTACTGCCTTAATTGGAGATATGAGACCATCAGCCTGTTTGCACAGAGGCTAAATGAG
ATTTACGGATTACCCTCATTTTTTCAGTGGCTGCATAAGAGGCTCGAGACCT
Sequence number 2160: Measles virus Whole genome #189 
TAAATGAGATTTACGGATTACCCTCATTTTTTCAGTGGCTGCATAAGAGGCTCGAGACCTCTGTTCTG
TATGTAAGTGATCCTCATTGCCCCCCTGACCTTGACGCCCATATCCCGTTAT
Sequence number 2161: Measles virus Whole genome #190 
CTGTTCTGTATGTAAGTGATCCTCATTGCCCCCCTGACCTTGACGCCCATATCCCGTTATGCAAAGTC
CCGAATGACCAAATCTTCATTAAGTACCCTATGGGAGGTATAGAAGGGTATT
Sequence number 2162: Measles virus Whole genome #191 
GCAAAGTCCCGAATGACCAAATCTTCATTAAGTACCCTATGGGAGGTATAGAAGGGTATTGTCAGAAG
CTGTGGACCATCAGCACCATCCCCTATTTATACCTGGCTGCTTATGAGAGCG
Sequence number 2163: Measles virus Whole genome #192 
GTCAGAAGCTGTGGACCATCAGCACCATCCCCTATTTATACCTGGCTGCTTATGAGAGCGGAGTAAGG
ATTGCTTCGTTAGTGCAGGGGGACAATCAGACCATAGCCGTAACAAAAAGGG
Sequence number 2164: Measles virus Whole genome #193 
GAGTAAGGATTGCTTCGTTAGTGCAGGGGGACAATCAGACCATAGCCGTAACAAAAAGGGTACCCAGC
ACATGGCCTTACAACCTCAAGAAACGGGAAGCTGCTAGAGTAACTAGAGACT
Sequence number 2165: Measles virus Whole genome #194 
TACCCAGCACATGGCCTTACAACCTCAAGAAACGGGAAGCTGCTAGAGTAACTAGAGACTACTTTGTA
ATTCTTAGGCAAAGGCTACATGACATTGGTCATCACCTCAAGGCAAATGAGA
Sequence number 2166: Measles virus Whole genome #195 
ACTTTGTAATTCTTAGGCAAAGGCTACATGACATTGGTCATCACCTCAAGGCAAATGAGACAATTGTC
TCATCACATTTTTTTGTCTATTCAAAAGGAATATATTATGATGGGCTACTTG
Sequence number 2167: Measles virus Whole genome #196 
CAATTGTCTCATCACATTTTTTTGTCTATTCAAAAGGAATATATTATGATGGGCTACTTGTGTCCCAA
TCACTCAAGAGCATCGCAAGATGTGTATTCTGGTCAGAGACTATAGTTGATG
Sequence number 2168: Measles virus Whole genome #197 TGTCCCAATCACTCAAGAGCATCGCAAGATGTGTATTCTGGTCAGAGACTATAGTTGATGAGACAAGG GCAGCATGCAGCAATATTGCCACAACGATGGCTAAGAGCATCGAGAGAGGTT
Sequence number 2169: Measles virus Whole genome #198 
AGACAAGGGCAGCATGCAGCAATATTGCCACAACGATGGCTAAGAGCATCGAGAGAGGTTATGACCGT
TACCTTGCATATTCTCTGAATGTCCTAAAAGTGATACAGCAAATTCTGATCT
Sequence number 2170: Measles virus Whole genome #199 
ATGACCGTTACCTTGCATATTCTCTGAATGTCCTAAAAGTGATACAGCAAATTCTGATCTCCCTTGGC
TTCACAATCAATTCAACCATGACCCGGGATGTAGTCATACCACTCCTCACAA
Sequence number 2171: Measles virus Whole genome #200 
CCCTTGGCTTCACAATCAATTCAACCATGACCCGGGATGTAGTCATACCACTCCTCACAAACAACGAC
CTCTTAATAAGGATGGCACTATTGCCCGCCCCTATTGGGGGGATGAATTATT
Sequence number 2172: Measles virus Whole genome #201 
ACAACGACCTCTTAATAAGGATGGCACTATTGCCCGCCCCTATTGGGGGGATGAATTATTTGAATATG
AGCAGGCTGTTTGTCAGAAACATCGGTGATCCAGTAACATCATCAATTGCTG
Sequence number 2173: Measles virus Whole genome #202 
TGAATATGAGCAGGCTGTTTGTCAGAAACATCGGTGATCCAGTAACATCATCAATTGCTGATCTCAAG
AGAATGATTCTCGCCTCGCTAATGCCTGAAGAGACCCTCCATCAAGTAATGA
Sequence number 2174: Measles virus Whole genome #203 
ATCTCAAGAGAATGATTCTCGCCTCGCTAATGCCTGAAGAGACCCTCCATCAAGTAATGACACAACAA
CCAGGAGACTCTTCATTCCTAGACTGGGCTAGCGACCCTTACTCAGCGAATC
Sequence number 2175: Measles virus Whole genome #204 
CACAACAACCAGGAGACTCTTCATTCCTAGACTGGGCTAGCGACCCTTACTCAGCGAATCTTGTATGT
GTCCAAAGCATCACTAGACTCCTCAAGAACATAACTGCAAGGTTTGTCCTGA
Sequence number 2176: Measles virus Whole genome #205 
TTGTATGTGTCCAAAGCATCACTAGACTCCTCAAGAACATAACTGCAAGGTTTGTCCTGATCCATAGT
CCAAACCCAATGTTAAAAGGATTATTCCATGATGACAGCAAAGAAGAGGACG
Sequence number 2177: Measles virus Whole genome #206 
TCCATAGTCCAAACCCAATGTTAAAAGGATTATTCCATGATGACAGCAAAGAAGAGGACGAGGGACTG
GCGGCGTTCCTCATGGACAGGCATATTATAGTACCTAGGGCAGCTCATGAAA
Sequence number 2178: Measles virus Whole genome #207 
AGGGACTGGCGGCGTTCCTCATGGACAGGCATATTATAGTACCTAGGGCAGCTCATGAAATCCTGGAT
CATAGTGTCACAGGGGCAAGAGAGTCTATCGCAGGCATGCTGGACACCACAA
Sequence number 2179: Measles virus Whole genome #208 
TCCTGGATCATAGTGTCACAGGGGCAAGAGAGTCTATCGCAGGCATGCTGGACACCACAAAGGGCCTG
ATTCGAGCCAGCATGAGGAAGGGGGGGTTAACCTCTCGAGTGATAACTAGAT
Sequence number 2180: Measles virus Whole genome #209 
AGGGCCTGATTCGAGCCAGCATGAGGAAGGGGGGGTTAACCTCTCGAGTGATAACTAGATTGTCCAAT
TATGACTATGAACAATTCAGGGCAGGGATGGTGCTATTGACAGGAAGAAGGA
Sequence number 2181: Measles virus Whole genome #210 
TGTCCAATTATGACTATGAACAATTCAGGGCAGGGATGGTGCTATTGACAGGAAGAAGGAGAAATATC
CTCATTGACAAAGAGTCATGTTCAGTGCAGCTGGCGAGAGCTCTAAGAAGCC
Sequence number 2182: Measles virus Whole genome #211 
GAAATATCCTCATTGACAAAGAGTCATGTTCAGTGCAGCTGGCGAGAGCTCTAAGAAGCCATATGTGG
GCGAGGCTAGCTCGAGGACGGCCTATTTACGGCCTTGAGGTCCCTGATGTAC
Sequence number 2183: Measles virus Whole genome #212 
ATATGTGGGCGAGGCTAGCTCGAGGACGGCCTATTTACGGCCTTGAGGTCCCTGATGTACTAGAATCT
ATGCGAGGCCACCTTATTCGGCGTCACGAGACATGTGTCATCTGCGAGTGTG
Sequence number 2184: Measles virus Whole genome #213 
TAGAATCTATGCGAGGCCACCTTATTCGGCGTCACGAGACATGTGTCATCTGCGAGTGTGGATCAGTC
AATTACGGATGGTTTTTTGTCCCCTCGAGTTGCCAACTGGATGATATTGACA
Sequence number 2185: Measles virus Whole genome #214 
GATCAGTCAATTACGGATGGTTTTTTGTCCCCTCGAGTTGCCAACTGGATGATATTGACAAGGAAACA
TCATCCTTGAGAGTCCCCTATATTGGTTCTACCACTGATGAGAGAACAGACA
Sequence number 2186: Measles virus Whole genome #215 
AGGAAACATCATCCTTGAGAGTCCCCTATATTGGTTCTACCACTGATGAGAGAACAGACATGAAGCTT
GCCTTCGTAAGAGCCCCAAGTCGATCCTTGCGATCTGCTGTTAGAATAGCAA
Sequence number 2187: Measles virus Whole genome #216 
TGAAGCTTGCCTTCGTAAGAGCCCCAAGTCGATCCTTGCGATCTGCTGTTAGAATAGCAACAGTGTAC
TCATGGGCTTACGGTGATGATGATAGCTCCTGGAACGAAGCTTGGTTGTTGG
Sequence number 2188: Measles virus Whole genome #217 
CAGTGTACTCATGGGCTTACGGTGATGATGATAGCTCCTGGAACGAAGCTTGGTTGTTGGCAAAACAA
AGGGCCAATGTGAGCCTGGAGGAGCTAAGGGTGATCACTCCCATCTCAACTT
Sequence number 2189: Measles virus Whole genome #218 
CAAAACAAAGGGCCAATGTGAGCCTGGAGGAGCTAAGGGTGATCACTCCCATCTCAACTTCGACTAAT
TTAGCGCATAGGTTGAGGGATCGTAGTACTCAAGTGAAATACTCAGGTACAT
Sequence number 2190: Measles virus Whole genome #219 
CGACTAATTTAGCGCATAGGTTGAGGGATCGTAGTACTCAAGTGAAATACTCAGGTACATCCCTTGTC
CGAGTGGCGAGGTATACCACAATCTCCAACGACAATCTTTCATTTGTCATAT
Sequence number 2191: Measles virus Whole genome #220 
CCCTTGTCCGAGTGGCGAGGTATACCACAATCTCCAACGACAATCTTTCATTTGTCATATCAGATAAG
AAGGTTGATACTAACTTTATATACCAACAAGGAATGCTTCTAGGATTGGGTG
Sequence number 2192: Measles virus Whole genome #221 
CAGATAAGAAGGTTGATACTAACTTTATATACCAACAAGGAATGCTTCTAGGATTGGGTGTTTTAGAA
ACATTGTTCCGACTTGAGAAAAACACCGGACCATCCAACACGGTACTACATC
Sequence number 2193: Measles virus Whole genome #222 
TTTTAGAAACATTGTTCCGACTTGAGAAAAACACCGGACCATCCAACACGGTACTACATCTTCACGTC
GAAACAGATTGTTGCGTGATCCCGATGATAGATCATCCCAGAATACCCAGCT
Sequence number 2194: Measles virus Whole genome #223 
TTCACGTCGAAACAGATTGTTGCGTGATCCCGATGATAGATCATCCCAGAATACCCAGCTCCCGCAAG
CTAGAGCTGAGAGCAGAGCTATGTACCAACCCATTGATATATGATAATGCTC
Sequence number 2195: Measles virus Whole genome #224 
CCCGCAAGCTAGAGCTGAGAGCAGAGCTATGTACCAACCCATTGATATATGATAATGCTCCTTTAATT
GACAGAGATGCAACAAGGCTATACACCCAGAGCCATAGGAGGCACCTTGTGG
Sequence number 2196: Measles virus Whole genome #225 CTTTAATTGACAGAGATGCAACAAGGCTATACACCCAGAGCCATAGGAGGCACCTTGTGGAATTTGTT ACGTGGTCCACACCCCAACTATATCACATTCTAGCTAAGTCCACAGCACTAT
Sequence number 2197: Measles virus Whole genome #226 
AATTTGTTACGTGGTCCACACCCCAACTATATCACATTCTAGCTAAGTCCACAGCACTATCTATGATT
GACCTGATAACAAAATTTGAGAAGGACCATATGAATGAAATTTCAGCTCTTA
Sequence number 2198: Measles virus Whole genome #227 
CTATGATTGACCTGATAACAAAATTTGAGAAGGACCATATGAATGAAATTTCAGCTCTTATAGGAGAT
GATGATATTAATAGTTTCATAACTGAATTTCTGCTTATAGAGCCAAGACTGT
Sequence number 2199: Measles virus Whole genome #228 
TAGGAGATGATGATATTAATAGTTTCATAACTGAATTTCTGCTTATAGAGCCAAGACTGTTCACTATC
TACTTGGGCCAGTGTGCGGCCATTAATTGGGCATTTGATGTGCATTATCATA
Sequence number 2200: Measles virus Whole genome #229 
TCACTATCTACTTGGGCCAGTGTGCGGCCATTAATTGGGCATTTGATGTGCATTATCATAGACCATCA
GGAAAATATCAGATGGGTGAGCTGTTGTCATCGTTCCTTTCTAGAATGAGCA
Sequence number 2201: Measles virus Whole genome #230 
GACCATCAGGAAAATATCAGATGGGTGAGCTGTTGTCATCGTTCCTTTCTAGAATGAGCAAAGGAGTG
TTTAAGGTGCTTGTCAATGCTCTAAGCCACCCAAAGATCTACAAGAAATTCT
Sequence number 2202: Measles virus Whole genome #231 
AAGGAGTGTTTAAGGTGCTTGTCAATGCTCTAAGCCACCCAAAGATCTACAAGAAATTCTGGCATTGT
GGTATTATAGAGCCTATCCATGGTCCGTCACTTGATGCTCAAAACTTGCACA
Sequence number 2203: Measles virus Whole genome #232 
GGCATTGTGGTATTATAGAGCCTATCCATGGTCCGTCACTTGATGCTCAAAACTTGCACACAACTGTG
TGCAACATGATTTACACATGCTATATGACCTACCTAGACCTGTTGTTGAATG
Sequence number 2204: Measles virus Whole genome #233 
CAACTGTGTGCAACATGATTTACACATGCTATATGACCTACCTAGACCTGTTGTTGAATGAAGAGTTA
GACGAGTTCACATTTCTTTTGTGTGAAAGTGACGAGGATGTAGTACCGGACA
Sequence number 2205: Measles virus Whole genome #234 
AAGAGTTAGACGAGTTCACATTTCTTTTGTGTGAAAGTGACGAGGATGTAGTACCGGACAGATTTGAC
AACATCCAGGCAAAACACTTGTGTGTTCTAGCAGATTTGTACTGTCAACCAG
Sequence number 2206: Measles virus Whole genome #235 
GATTTGACAACATCCAGGCAAAACACTTGTGTGTTCTAGCAGATTTGTACTGTCAACCAGGGACCTGC
CCACCAATTCGAGGTCTAAGACCGGTGGAGAAATGTGCAGTTCTAACCAACC
Sequence number 2207: Measles virus Whole genome #236 
GGACCTGCCCACCAATTCGAGGTCTAAGACCGGTGGAGAAATGTGCAGTTCTAACCAACCATATCAAG
GCAGAGGCTAGATTATCTCCAGCAGGATCTTCGTGGAACATAAACCCAATTA
Sequence number 2208: Measles virus Whole genome #237 
ATATCAAGGCAGAGGCTAGATTATCTCCAGCAGGATCTTCGTGGAACATAAACCCAATTATTGTAGAC
CACTACTCATGCTCCCTGACTTATCTCCGGCGAGGCTCGATCAAACAGATAA
Sequence number 2209: Measles virus Whole genome #238 
TTGTAGACCACTACTCATGCTCCCTGACTTATCTCCGGCGAGGCTCGATCAAACAGATAAGATTGAGA
GTTGATCCAGGATTCATCTTCGACGCCCTCGCTGAGGCAAATGTCAGTCAGC
Sequence number 2210: Measles virus Whole genome #239 
GATTGAGAGTTGATCCAGGATTCATCTTCGACGCCCTCGCTGAGGCAAATGTCAGTCAGCCAAAAATA
GGCAGCAACAACATCTCAAATATGAGTATCAAGGATTTCAGACCCCCACACG
Sequence number 2211: Measles virus Whole genome #240 
CAAAAATAGGCAGCAACAACATCTCAAATATGAGTATCAAGGATTTCAGACCCCCACACGATGATGTT
GCCAAATTGCTCAAAGATATCAACACAAGCAAGCACAACCTTCCTATTTCAG
Sequence number 2212: Measles virus Whole genome #241 
ATGATGTTGCCAAATTGCTCAAAGATATCAACACAAGCAAGCACAACCTTCCTATTTCAGGGGGCAGT
CTCGCCAATTATGAAATCCATGCTTTCCGCAGAATCGGGTTGAACTCATCTG
Sequence number 2213: Measles virus Whole genome #242 
GGGGCAGTCTCGCCAATTATGAAATCCATGCTTTCCGCAGAATCGGGTTGAACTCATCTGCTTGCTAC
AAAGCTGTTGAGATATCAACATTAATTAGGAGATGCCTTGAGCCAGGGGAAG
Sequence number 2214: Measles virus Whole genome #243 
CTTGCTACAAAGCTGTTGAGATATCAACATTAATTAGGAGATGCCTTGAGCCAGGGGAAGACGGCTTG
TTCTTGGGTGAGGGATCGGGTTCTATGTTGGTCACTTATAAGGAGATACTTA
Sequence number 2215: Measles virus Whole genome #244 
ACGGCTTGTTCTTGGGTGAGGGATCGGGTTCTATGTTGGTCACTTATAAGGAGATACTTAAACTAAAC
AAGTGCTTCTATAATAGTGGGGTTTCCGCCAATTCTAGATCTGGTCAAAGGG
Sequence number 2216: Measles virus Whole genome #245 
AACTAAACAAGTGCTTCTATAATAGTGGGGTTTCCGCCAATTCTAGATCTGGTCAAAGGGAATTAGCA
CCCTACCCCTCCGAAGTCGGCCTTGTCGAACACAGAATGGGAGTGGGCAATA
Sequence number 2217: Measles virus Whole genome #246 
AATTAGCACCCTACCCCTCCGAAGTCGGCCTTGTCGAACACAGAATGGGAGTGGGCAATATTGTCAAG
GTGCTCTTTAACGGGAGGCCCGAAGTCACGTGGATAGGCAGTGTAGATTGCT
Sequence number 2218: Measles virus Whole genome #247 
TTGTCAAGGTGCTCTTTAACGGGAGGCCCGAAGTCACGTGGATAGGCAGTGTAGATTGCTTCAATTTC
ATAGTCAGTAATATCCCTACCTCTAGTGTGGGGTTTATCCATTCAGATATAG
Sequence number 2219: Measles virus Whole genome #248 
TCAATTTCATAGTCAGTAATATCCCTACCTCTAGTGTGGGGTTTATCCATTCAGATATAGAGACCTTA
CCTAACAAAGATACTATAGAGAAGCTAGAGGAATTGGCAGCCATCTTGTCGC
Sequence number 2220: Measles virus Whole genome #249 
AGACCTTACCTAACAAAGATACTATAGAGAAGCTAGAGGAATTGGCAGCCATCTTGTCGCTGGCTCTG
CTCCTGGGCAAAATAGGATCAATATTGGTGATTAAGCTTATGCCTTTCAGCG
Sequence number 2221: Measles virus Whole genome #250 
TGGCTCTGCTCCTGGGCAAAATAGGATCAATATTGGTGATTAAGCTTATGCCTTTCAGCGGGGATTTT
GTTCAAGGATTTATAAGTTATGTAGGGTCTCATTATAGGGAAGTGAACCTTG
Sequence number 2222: Measles virus Whole genome #251 
GGGATTTTGTTCAAGGATTTATAAGTTATGTAGGGTCTCATTATAGGGAAGTGAACCTTGTATACCCT
AGATACAGCAACTTCATATCTACTGAATCTTATTTGGTTATGGCAGATCTCA
Sequence number 2223: Measles virus Whole genome #252 
TATACCCTAGATACAGCAACTTCATATCTACTGAATCTTATTTGGTTATGGCAGATCTCAAGGCTAAC
CGGCTAATGAATCCTGAAAAGATTAAGCAGCAGATAATTGAATCATCTGTGC
Sequence number 2224: Measles virus Whole genome #253 AGGCTAACCGGCTAATGAATCCTGAAAAGATTAAGCAGCAGATAATTGAATCATCTGTGCGGACTTCA CCTGGACTTATAGGTCACATCCTATCCATTAAGCAACTAAGCTGCATACAAG
Sequence number 2225: Measles virus Whole genome #254 
GGACTTCACCTGGACTTATAGGTCACATCCTATCCATTAAGCAACTAAGCTGCATACAAGCAATTGTG
GGAGGCGCAGTTATTAGAGGTGATATCAACCCTACTCTGAAAAAACTTACAC
Sequence number 2226: Measles virus Whole genome #255 
CAATTGTGGGAGGCGCAGTTATTAGAGGTGATATCAACCCTACTCTGAAAAAACTTACACCTATAGAG
CAGGTGCTGATCAATTGCGGGTTGGCGATTAACGGACCCAAACTGTGCAAAG
Sequence number 2227: Measles virus Whole genome #256 
CTATAGAGCAGGTGCTGATCAATTGCGGGTTGGCGATTAACGGACCCAAACTGTGCAAAGAATTGATC
CACCATGATGTTGCCTCAGGGCAAGATGGATTGCTTAATTCTATACTCATCC
Sequence number 2228: Measles virus Whole genome #257 
AATTGATCCACCATGATGTTGCCTCAGGGCAAGATGGATTGCTTAATTCTATACTCATCCTCTACAGG
GAATTGGCAAGATTCAAGGACAACCAAAGAAGTCAACAGGGGATGTTCCACG
Sequence number 2229: Measles virus Whole genome #258 
TCTACAGGGAATTGGCAAGATTCAAGGACAACCAAAGAAGTCAACAGGGGATGTTCCACGCTTACCCC
GTATTGGTAAGTAGCAGACAACGAGAACTTATATCTAGGATCGCCCGCAAAT
Sequence number 2230: Measles virus Whole genome #259 
CTTACCCCGTATTGGTAAGTAGCAGACAACGAGAACTTATATCTAGGATCGCCCGCAAATTCTGGGGG
CACATTCTTCTTTACTCCGGGAACAGAAAGTTGATAAATAAGTTTATCCAGA
Sequence number 2231: Measles virus Whole genome #260 
TCTGGGGGCACATTCTTCTTTACTCCGGGAACAGAAAGTTGATAAATAAGTTTATCCAGAATCTCAAG
TCCGGTTATCTGATACTGGACTTACACCAGAATATCTTCGTTAAGAATCTAT
Sequence number 2232: Measles virus Whole genome #261 
ATCTCAAGTCCGGTTATCTGATACTGGACTTACACCAGAATATCTTCGTTAAGAATCTATCCAAGTCA
GAGAAACAGATTATCCTGTCGGGGGGTCTGAAACGTGAGTGGGTCTTTAAGG
Sequence number 2233: Measles virus Whole genome #262 
CCAAGTCAGAGAAACAGATTATCCTGTCGGGGGGTCTGAAACGTGAGTGGGTCTTTAAGGTAACAGTC
AAGGAGACCAAAGAATGGTATAAGTTACTCGGATACAGTGCTCTGATTAAGG
Sequence number 2234: Measles virus Whole genome #263 
TAACAGTCAAGGAGACCAAAGAATGGTATAAGTTACTCGGATACAGTGCTCTGATTAAGGACTAGATG
GTTGAACTCCGGAACCCTGAACCTTCCCAAGGTAGTTGGGCATTATTTGCAA
Sequence number 2235: Measles virus Whole genome #264 
TTAAGGACTAGATGGTTGAACTCCGGAACCCTGAACCTTCCCAAGGTAGTTGGGCATTATTTGCAATA
TATTAAAGAAAACTTTGAAAATACGAAGTTTCTATTCCCAGCTTTGTCTGGT
Sequence number 2236: Monkeypox virus WA F3L #1 
TAAAATACAATTATATCTTTTTCGATATTAATAAATCCGTGTCTCCCGGGTTTTTTATCTCTTTCAGT
ATGTGAATAGATAGGTATTTTATCTCTATTCATCATCGAATTTAAGAGATCC
Sequence number 2237: Monkeypox virus WA F3L #2 
CTTTCAGTATGTGAATAGATAGGTATTTTATCTCTATTCATCATCGAATTTAAGAGATCCGATAAACA
TTGTTTGTATTCTCCAGATGTCAGCATCTGATACAACAATATATGTGCACAT
Sequence number 2238: Monkeypox virus WA F3L #3 
GATAAACATTGTTTGTATTCTCCAGATGTCAGCATCTGATACAACAATATATGTGCACATAAACCTCT
GGCACTTATTTCATGTACCTTCCCCTTATCACTAAGGAGAATAGTATTTGAG
Sequence number 2239: Monkeypox virus WA F3L #4 
AAACCTCTGGCACTTATTTCATGTACCTTCCCCTTATCACTAAGGAGAATAGTATTTGAGAAATATGT
ATACATGATATTATCATGTATTAGATATACAGAATTTGTAACACTCTCGAAA
Sequence number 2240: Monkeypox virus WA F3L #5 
AAATATGTATACATGATATTATCATGTATTAGATATACAGAATTTGTAACACTCTCGAAATCACACGA
TGTGTCGGCGTTAAGATCTAATATATCACTCGATAACACATTTTCATCTAGA
Sequence number 2241: Monkeypox virus WA F3L #6 
TCACACGATGTGTCGGCGTTAAGATCTAATATATCACTCGATAACACATTTTCATCTAGATACACTAG
ACATTTTTTAAAGCTAAAATAGTCTTTAGTAGTAACAGTAACTATGCGATTA
Sequence number 2242: Monkeypox virus WA F3L #7 
TACACTAGACATTTTTTAAAGCTAAAATAGTCTTTAGTAGTAACAGTAACTATGCGATTATTTTCATC
GATGATACATTTCATCGGCATATTATTACGCGTACCATCAAAGACTATACCA
Sequence number 2243: Monkeypox virus WA F3L #8 
TTTTCATCGATGATACATTTCATCGGCATATTATTACGCGTACCATCAAAGACTATACCATGTGTATA
TCTAACGTATTCTAGCATAGTTGCCATACGTACATTAAACTTTTCAGGATCT
Sequence number 2244: Monkeypox virus WA F3L #9 
TGTGTATATCTAACGTATTCTAGCATAGTTGCCATACGTACATTAAACTTTTCAGGATCTTTGGATAG
ATCTTCCAATCTATCTATTTGAGAAAACATTTTTATCATGTTCAATAGTTGA
Sequence number 2245: Monkeypox virus WA F3L #10 
TTGGATAGATCTTCCAATCTATCTATTTGAGAAAACATTTTTATCATGTTCAATAGTTGAAACGTCGG
ATCCACTATATAGATATTATCTATAAAGATTTTAGGAACTATGTTCATGGTA
Sequence number 2246: Monkeypox virus WA F3L #11 
AACGTCGGATCCACTATATAGATATTATCTATAAAGATTTTAGGAACTATGTTCATGGTATCCTGGCG
AATATTAAAACTATCAATGATATGATTATCGTTTTCATCTTTTATCACCATA
Sequence number 2247: Monkeypox virus WA F3L #12 
TCCTGGCGAATATTAAAACTATCAATGATATGATTATCGTTTTCATCTTTTATCACCATATAGTTTCT
AAGATATGGGATTTTACTTAATATAATATTATTTCCCGTAATAAATTTTATT
Sequence number 2248: Monkeypox virus WA F3L #13 
TAGTTTCTAAGATATGGGATTTTACTTAATATAATATTATTTCCCGTAATAAATTTTATTAGAAATGC
CAAATCTATAAGAAAAGTCCTAGAATTAGTCTGAAGAATATCTATATCACCG
Sequence number 2249: Monkeypox virus WA F3L #14 
AGAAATGCCAAATCTATAAGAAAAGTCCTAGAATTAGTCTGAAGAATATCTATATCACCGTACCGTAT
ATTTGGATTAATTAGATATAGAGAATATGATCCGTAACATATACAACTTTTA
Sequence number 2250: Monkeypox virus WA F3L #15 
TACCGTATATTTGGATTAATTAGATATAGAGAATATGATCCGTAACATATACAACTTTTATTATGACG
TCTAAGATATTCTTCCATCAACTTATTAACATTTTTGACTAGGGAAGATACA
Sequence number 2251: Monkeypox virus WA F3L #16 
TTATGACGTCTAAGATATTCTTCCATCAACTTATTAACATTTTTGACTAGGGAAGATACATTATGACG
TCCCATTACTTTTGCCTTGTCTATTACAGCGACGTTCATAGAATTTAGCATA
Sequence number 2252: Monkeypox virus WA F3L #17 TTATGACGTCCCATTACTTTTGCCTTGTCTATTACAGCGACGTTCATAGAATTTAGCATATCTCTTGC CAATTCTTCCATTGATGTTACATTATAAGAAATTTTAGATGAAATTACATTT
Sequence number 2253: Monkeypox virus WA F3L #18 
TCTCTTGCCAATTCTTCCATTGATGTTACATTATAAGAAATTTTAGATGAAATTACATTTGGAGCTTT
AATAGTAAGAACTCCTAATATATCCGTGTATGTGGTCACTAATACAGATTGT
Sequence number 2254: Monkeypox virus WA F3L #19 
GGAGCTTTAATAGTAAGAACTCCTAATATATCCGTGTATGTGGTCACTAATACAGATTGTAGTTCTAT
AATCGTAAATAATTTACCTATATTATATGTTTGAGTTTGTTTAGAAAAGTAG
Sequence number 2255: Monkeypox virus WA F3L #20 
AGTTCTATAATCGTAAATAATTTACCTATATTATATGTTTGAGTTTGTTTAGAAAAGTAGCTAAGTAT
ACGATCTTTTATTTCTGATGCCGATGTATCAACATCGAAAAAAAATCTTTTT
Sequence number 2256: Monkeypox virus WA F3L #21 
CTAAGTATACGATCTTTTATTTCTGATGCCGATGTATCAACATCGAAAAAAAATCTTTTTTTATTCTT
TTTTACTAACGATACGAATATGTCTTTGTTAAAAACAGTTATTTTCTGAATA
Sequence number 2257: Monkeypox virus WA F3L #22 
TTATTCTTTTTTACTAACGATACGAATATGTCTTTGTTAAAAACAGTTATTTTCTGAATATTTCTAGC
TTGTAATTTTAACATATGATATTCGTTCACACTAGGTACTCTGCCTAAATAG
Sequence number 2258: Monkeypox virus WA F3L #23 
TTTCTAGCTTGTAATTTTAACATATGATATTCGTTCACACTAGGTACTCTGCCTAAATAGGTTTCTAT
AATCTTTAATGTAATATTAGGAAGAGTATTCTGATCAGGATTCCTATTCATT
Sequence number 2259: Monkeypox virus WA F3L #24 
GTTTCTATAATCTTTAATGTAATATTAGGAAGAGTATTCTGATCAGGATTCCTATTCATTTTGAGGAT
TTAAAACTCTGATTATTGTCTAATATGGTCTCAACACAAACTTTTTCACAGA
Sequence number 2260: Monkeypox virus WA F3L #25 
TTGAGGATTTAAAACTCTGATTATTGTCTAATATGGTCTCAACACAAACTTTTTCACAGAGTGATAGA
GTTTTTGATAACTCGTTTTTCTTAAGAAATATAAAACTACTGTCTCCAGAGC
Sequence number 2261: Monkeypox virus WA F3L #26 
GTGATAGAGTTTTTGATAACTCGTTTTTCTTAAGAAATATAAAACTACTGTCTCCAGAGCTCGCTCTA
TCTTTTATTTTATCTAATTCGATACAAACTCCTGATACTGGTTCAGAAAGTA
Sequence number 2262: Monkeypox virus WA F3L #27 
TCGCTCTATCTTTTATTTTATCTAATTCGATACAAACTCCTGATACTGGTTCAGAAAGTAATTCATTA
ATTTTCAGTCCTTTATAGAAGATATTTAATATAGATAATACAAAATCTTCAG
Sequence number 2263: Monkeypox virus WA F3L #28 
ATTCATTAATTTTCAGTCCTTTATAGAAGATATTTAATATAGATAATACAAAATCTTCAGTTCTTGAT
ATCGATCTGATTGATCCTAGAACTAGATATATTAATAACGTGCTCATTAGGC
Sequence number 2264: Monkeypox virus WA F3L #29 
TTCTTGATATCGATCTGATTGATCCTAGAACTAGATATATTAATAACGTGCTCATTAGGCAGTTTATG
GCAGCTTGATAATTAGATATAGTATATTCCAGTTCATATTTATTAGATACCG
Sequence number 2265: Monkeypox virus WA F3L #30 
AGTTTATGGCAGCTTGATAATTAGATATAGTATATTCCAGTTCATATTTATTAGATACCGCATTGCCC
AGATTTTGATATTCTATGAATTCCTCTGAAAATAAATCCAAAATAACTAGAC
Sequence number 2266: Monkeypox virus WA F3L #31 
CATTGCCCAGATTTTGATATTCTATGAATTCCTCTGAAAATAAATCCAAAATAACTAGACATTCTATT
TTTTGTGGATTAGTGTACTCTCTTCCCTCTATCATGTTCACTACTGGTGTCC
Sequence number 2267: Monkeypox virus WA F3L #32 
ATTCTATTTTTTGTGGATTAGTGTACTCTCTTCCCTCTATCATGTTCACTACTGGTGTCCACAATGAT
AAATATCTAGAGGGAATATAATATAGTCCATATGATGCCAATCTAGCAATGT
Sequence number 2268: Monkeypox virus WA F3L #33 
ACAATGATAAATATCTAGAGGGAATATAATATAGTCCATATGATGCCAATCTAGCAATGTCGAATAAC
TGTAATTTTATTCTTCGCTCTTCATTATGAATTGAATCTTGAGGTATAAACC
Sequence number 2269: Monkeypox virus WA F3L #34 
CGAATAACTGTAATTTTATTCTTCGCTCTTCATTATGAATTGAATCTTGAGGTATAAACCTAACACAA
ATTATATCATTAGACTTTTCGTATGTAATGTCTTTCATGTTATAAGTTTTTA
Sequence number 2270: Monkeypox virus WA F3L #35 
TAACACAAATTATATCATTAGACTTTTCGTATGTAATGTCTTTCATGTTATAAGTTTTTAATCCTGGA
ATAGAATCTATTTTAATGAGGCTTTTAAACGCAGCGTTCTCCAACGAGTCAA
Sequence number 2271: Monkeypox virus WA F3L #36 
ATCCTGGAATAGAATCTATTTTAATGAGGCTTTTAAACGCAGCGTTCTCCAACGAGTCAAAGCATAAT
ACTCTGTTGGTTTTCTTATATTCAATATTACGATTTTCTTCTTTGAATGGAA
Sequence number 2272: Monkeypox virus WA F3L #37 
AGCATAATACTCTGTTGGTTTTCTTATATTCAATATTACGATTTTCTTCTTTGAATGGAATAGGTTTT
TGAATTAGTTTATAATTACAACATAATAGATAAGGAAGTGTGTAAATAGTAC
Sequence number 2273: Monkeypox virus WA F3L #38 
TAGGTTTTTGAATTAGTTTATAATTACAACATAATAGATAAGGAAGTGTGTAAATAGTACGCGGAAAA
AACATAATAGCTCCCCTGTTTTCATCCATGGTTTTAAGTAAATGATCACTGG
Sequence number 2274: Monkeypox virus WA F3L #39 
GCGGAAAAAACATAATAGCTCCCCTGTTTTCATCCATGGTTTTAAGTAAATGATCACTGGCTTCTTTA
GTCAATGGATATTCGAACATTAACCGTTTCATCATCATTGGACAGAATCCAT
Sequence number 2275: Monkeypox virus WA F3L #40 
CTTCTTTAGTCAATGGATATTCGAACATTAACCGTTTCATCATCATTGGACAGAATCCATATTTCTTA
ATGTAAAGAGTGATCAAATCATTGTGTTTATTGTACCATCTTGTTGTAAATG
Sequence number 2276: Monkeypox virus WA F3L #41 
ATTTCTTAATGTAAAGAGTGATCAAATCATTGTGTTTATTGTACCATCTTGTTGTAAATGTGTATTCG
GTTATCGGATCTGCTCCTTTTTCTATTAAAGTATCGATATCGATCTCGTCTA
Sequence number 2277: Monkeypox virus WA F3L #42 
TGTATTCGGTTATCGGATCTGCTCCTTTTTCTATTAAAGTATCGATATCGATCTCGTCTAAGAATTCA
ACTATATCGACATATTTCATTTGTATACACATAACCATTACTAACGTAGAAT
Sequence number 2278: Monkeypox virus WA F3L #43 
AGAATTCAACTATATCGACATATTTCATTTGTATACACATAACCATTACTAACGTAGAATGTATAGGA
AGAGATGTAACGGGAACAGGGTTTGTTGATTCGCAAACTATTCTAATACATA
Sequence number 2279: Monkeypox virus WA F3L #44 
GTATAGGAAGAGATGTAACGGGAACAGGGTTTGTTGATTCGCAAACTATTCTAATACATAATTCTTCT
GTTAATACGTCTTGCACGTAATCTATTATAGATGCCAAGATATCTATATAAT
Sequence number 2280: Monkeypox virus WA F3L #45 ATTCTTCTGTTAATACGTCTTGCACGTAATCTATTATAGATGCCAAGATATCTATATAATTATTTTGT AAGATGATGTTAACAATGTGATCTATATAAGTAGTGTAATAATTCATGTATT
Sequence number 2281: Monkeypox virus WA F3L #46 
TATTTTGTAAGATGATGTTAACAATGTGATCTATATAAGTAGTGTAATAATTCATGTATTTCGATATA
TGTTCCAACTCTGTCTTTGTGATGTCTAGTTTCGTAATATCTATAGCGTCCT
Sequence number 2282: Monkeypox virus WA F3L #47 
TCGATATATGTTCCAACTCTGTCTTTGTGATGTCTAGTTTCGTAATATCTATAGCGTCCTCAAAAAAT
ATATTCGCATATATTCCCAAGTCTTCAGTTCTATCTTCTAAAAAATCTTCAA
Sequence number 2283: Monkeypox virus WA F3L #48 
CAAAAAATATATTCGCATATATTCCCAAGTCTTCAGTTCTATCTTCTAAAAAATCTTCAACGTATGGA
ATATAATAATCTATTTTACCTCTTCTGATGTCATTAATGATATAGTTTTTGA
Sequence number 2284: Monkeypox virus WA F3L #49 
CGTATGGAATATAATAATCTATTTTACCTCTTCTGATGTCATTAATGATATAGTTTTTGACACTATTT
TCCGTCAATTGATTCTTATTCACTATGTCTAAAAACCGGATAGCGTCCCTAG
Sequence number 2285: Monkeypox virus WA F3L #50 
CACTATTTTCCGTCAATTGATTCTTATTCACTATGTCTAAAAACCGGATAGCGTCCCTAGGACGAACT
ACTGCCATTAATATCTCTATTATAGCTTCTGGACATAAATCATCTATTATAC
Sequence number 2286: Monkeypox virus WA F3L #51 
GACGAACTACTGCCATTAATATCTCTATTATAGCTTCTGGACATAAATCATCTATTATACCAGAATTA
ATGGGAACTATTCCGTATCTATCTAACATAGTTTTAAGAAAGTCAGAATCTA
Sequence number 2287: Monkeypox virus WA F3L #52 
CAGAATTAATGGGAACTATTCCGTATCTATCTAACATAGTTTTAAGAAAGTCAGAATCTAAGACCTGA
TGTTCATATATTGGTTCATACATGAAATGATCTCTATTGATGATAGTGACTA
Sequence number 2288: Monkeypox virus WA F3L #53 
AGACCTGATGTTCATATATTGGTTCATACATGAAATGATCTCTATTGATGATAGTGACTATTTCATTC
TCTGAAAATTGGTAACTCATTCTATACACGCTTTCCTTGTTGATAAAGGATA
Sequence number 2289: Monkeypox virus WA F3L #54 
TTTCATTCTCTGAAAATTGGTAACTCATTCTATACACGCTTTCCTTGTTGATAAAGGATAGTATATAC
TCAATGGAATTTGTACCAACAAACTGTTCTCTTATGAATCGTATATCATCAT
Sequence number 2290: Monkeypox virus WA F3L #55 
GTATATACTCAATGGAATTTGTACCAACAAACTGTTCTCTTATGAATCGTATATCATCATCTGAAATG
ATCATGTAAGGCATACATTTAACAATAAGAGACTTGTCTCCTGTTATCAATA
Sequence number 2291: Monkeypox virus WA F3L #56 
CTGAAATGATCATGTAAGGCATACATTTAACAATAAGAGACTTGTCTCCTGTTATCAATATACTATTC
TTGTGATAATTTATGTGTGCGGCAAATTTGTCCACGTTCTTTAATTTTGTTA
Sequence number 2292: Monkeypox virus WA F3L #57 
TACTATTCTTGTGATAATTTATGTGTGCGGCAAATTTGTCCACGTTCTTTAATTTTGTTATAGTAGAT
ATCAAATCCAATGGAGATACAGTTCTTGGCTTAAACAGATATAGTTTTTCTG
Sequence number 2293: Monkeypox virus WA F3L #58 
TAGTAGATATCAAATCCAATGGAGATACAGTTCTTGGCTTAAACAGATATAGTTTTTCTGGAACGAAT
TCTACAACATTATTATAAAGGACTTTGGGTATATAAGTGGGATGAAATCCTA
Sequence number 2294: Monkeypox virus WA F3L #59 
GAACGAATTCTACAACATTATTATAAAGGACTTTGGGTATATAAGTGGGATGAAATCCTATTTTAATT
AATGCGATAGCCTTGTCCTCGTGCAGATATCCAAACGCTTTTGTGATAGTAT
Sequence number 2295: Monkeypox virus WA F3L #60 
TTTTAATTAATGCGATAGCCTTGTCCTCGTGCAGATATCCAAACGCTTTTGTGATAGTATGGCATTCA
TTGTCTAGAAACGCTCTACGAATATCTGTAACAGATATCATCTTTAGAGAAT
Sequence number 2296: Monkeypox virus WA F3L #61 
GGCATTCATTGTCTAGAAACGCTCTACGAATATCTGTAACAGATATCATCTTTAGAGAATACTAGTCG
CGTTAATAGTACTAAAATTTGTATTTTTTAATCTATCTCAATAAAAAATTAA
Sequence number 2297: Monkeypox virus WA F3L #62 
ACTAGTCGCGTTAATAGTACTAAAATTTGTATTTTTTAATCTATCTCAATAAAAAATTAATATGTATG
ATTCAATGTATAACTAAACTACTAACTGTTATTGATAACTAGAATCAGAATC
Sequence number 2298: Monkeypox virus WA F3L #63 
TATGTATGATTCAATGTATAACTAAACTACTAACTGTTATTGATAACTAGAATCAGAATCTAATGATG
ACATAACTAAGAAGTTTATCTACAGCCAATTTAGCTGCATTATTTTTAGCAT
Sequence number 2299: Monkeypox virus WA F3L #64 
TAATGATGACATAACTAAGAAGTTTATCTACAGCCAATTTAGCTGCATTATTTTTAGCATCTCGTTTA
GATTTTCCATCTGCCTTATCGAATACTCTTCCGTCAATGTCTACACAGGCAT
Sequence number 2300: Monkeypox virus WA F3L #65 
CTCGTTTAGATTTTCCATCTGCCTTATCGAATACTCTTCCGTCAATGTCTACACAGGCATAAAATGTA
GGAGAGTTACTAGGCCCCACTGATTCAATACGAAAAGACCAATCTCTCCTAG
Sequence number 2301: Monkeypox virus WA F3L #66 
AAAATGTAGGAGAGTTACTAGGCCCCACTGATTCAATACGAAAAGACCAATCTCTCCTAGTTATTTGA
CAGTACTCATTAATAACGGTGACAGGGTTAACACCTTTCCAATAAATAATTT
Sequence number 2302: Monkeypox virus WA F3L #67 
TTATTTGACAGTACTCATTAATAACGGTGACAGGGTTAACACCTTTCCAATAAATAATTTTTTTAACC
GGAATAACATCATCAAAAGACTTATGATCCTCTCTCATTGATTTTTCGCGGG
Sequence number 2303: Monkeypox virus WA F3L #68 
TTTTAACCGGAATAACATCATCAAAAGACTTATGATCCTCTCTCATTGATTTTTCGCGGGATACATCA
TCTATTATAGCATCAGCATCAGAATCTGTAGGCCGTGTATCAGCATCCATTG
Sequence number 2304: Monkeypox virus WA F3L #69 
ATACATCATCTATTATAGCATCAGCATCAGAATCTGTAGGCCGTGTATCAGCATCCATTGTCGTAGAC
CAACGAGGAGGAGTATCGTTGGAGCTGTAAACCATAGCACTACGTTGAAGAT
Sequence number 2305: Monkeypox virus WA F3L #70 
TCGTAGACCAACGAGGAGGAGTATCGTTGGAGCTGTAAACCATAGCACTACGTTGAAGATCATACAGA
GCTTTATTAACTTCTCGCTTCTCCATATTAAGTTGTTTAGTTAGTTGTACAG
Sequence number 2306: Monkeypox virus WA F3L #71 
CATACAGAGCTTTATTAACTTCTCGCTTCTCCATATTAAGTTGTTTAGTTAGTTGTACAGCAGTAGCT
CCTTAGTCCAATGTTTTTAATAACCGCACACAATCTCTGTGTCAGAACGCTC
Sequence number 2307: Monkeypox virus WA F3L #72 
CAGTAGCTCCTTAGTCCAATGTTTTTAATAACCGCACACAATCTCTGTGTCAGAACGCTCGTCAATAT
AGATCTTAGAAATTTTTTTAGAGAGAACTAACGCAACTAGCAATAAAACTGA
Sequence number 2308: Monkeypox virus WA F3L #73 GTCAATATAGATCTTAGAAATTTTTTTAGAGAGAACTAACGCAACTAGCAATAAAACTGATCTTATTT TATCATTTTTTTTATTCATCATCCTCTGGTGGTTCGTCGTTCCTATCGAATG
Sequence number 2309: Monkeypox virus WA F3L #74 
TCTTATTTTATCATTTTTTTTATTCATCATCCTCTGGTGGTTCGTCGTTCCTATCGAATGTAGCTCTG
ATTAACCCGTCATCTATAGGTGATGCTGGTTCTGGAGATTCTGGAGGAGATG
Sequence number 2310: Monkeypox virus WA F3L #75 
TAGCTCTGATTAACCCGTCATCTATAGGTGATGCTGGTTCTGGAGATTCTGGAGGAGATGGATTATTA
TCTGGAAGAATCTCTGTTATTTCCTTGTTTTCATGTATCGATTGCGTTGTAA
Sequence number 2311: Monkeypox virus WA F3L #76 
GATTATTATCTGGAAGAATCTCTGTTATTTCCTTGTTTTCATGTATCGATTGCGTTGTAACATTAAGA
TTGCGAAATGCTCTAAATTTGGGAGGCTTAAAGTGTTGTTTACAATCTCTAC
Sequence number 2312: Monkeypox virus WA F3L #77 
CATTAAGATTGCGAAATGCTCTAAATTTGGGAGGCTTAAAGTGTTGTTTACAATCTCTACACGCGTGT
CTAACTAATGGAGGTTCATCAGCGGCTCTAGTTTGAATCATCATCGGTGTAG
Sequence number 2313: Monkeypox virus WA F3L #78 
ACGCGTGTCTAACTAATGGAGGTTCATCAGCGGCTCTAGTTTGAATCATCATCGGTGTAGTATTCCTA
CTTTTACAGTTAGGACACGGTGTATTGTATTTCTCGTCGAGAACGTTAAAAT
Sequence number 2314: Monkeypox virus WA F3L #79 
TATTCCTACTTTTACAGTTAGGACACGGTGTATTGTATTTCTCGTCGAGAACGTTAAAATAATCGTTG
TAACTCACATCCTTTATTTTATCTATATTGTATTCTACTCCTTTCTTAATGC
Sequence number 2315: Monkeypox virus WA F3L #80 
AATCGTTGTAACTCACATCCTTTATTTTATCTATATTGTATTCTACTCCTTTCTTAATGCATTTTATA
CCGAACAAGAGATAGCGAAGGAATTCTTTTTCGGTACCGCTAGTACCCTTAA
Sequence number 2316: Monkeypox virus WA F3L #81 
ATTTTATACCGAACAAGAGATAGCGAAGGAATTCTTTTTCGGTACCGCTAGTACCCTTAATCATATCA
CATAGTGTTTTATATTCTAAATGTGTGGCAATGGACGGTTTATTTCTATACG
Sequence number 2317: Monkeypox virus WA F3L #82 
TCATATCACATAGTGTTTTATATTCTAAATGTGTGGCAATGGACGGTTTATTTCTATACGATAGTTTG
TTTTTGGAATCCTTTGAGTATTCTATACCAATATTATTCTTTGATTCGAATT
Sequence number 2318: Monkeypox virus WA F3L #83 
ATAGTTTGTTTTTGGAATCCTTTGAGTATTCTATACCAATATTATTCTTTGATTCGAATTTAGTTTCT
TCGATATTAGATTTTGTATTACCTATATTCTTGATGTAGTACTTTGATGATT
Sequence number 2319: Monkeypox virus WA F3L #84 
TAGTTTCTTCGATATTAGATTTTGTATTACCTATATTCTTGATGTAGTACTTTGATGATTTTTCCATG
GCCCATTCTATTAAGTTTTCCAAGTTGGCATCATCCACATATTGTGATAGTA
Sequence number 2320: Monkeypox virus WA F3L #85 
TTTCCATGGCCCATTCTATTAAGTTTTCCAAGTTGGCATCATCCACATATTGTGATAGTAATTCTCGG
ATATCAGTAGTGACTACCGCCATTGATATTTGTTCATTTGATGAGTAACTAC
Sequence number 2321: Monkeypox virus WA F3L #86 
TAAGTTTTCCAAGTTGGCATCATCCACATATTGTGATAGTAATTCTCGGATATCAGTAGTGACTACCG
CCATTGATATTTGTTCATTTGATGAGTAACTACTAATGTATACATTTTCCAT
Sequence number 2322: Orientia tsutsugamushi tsa56 #1 
TAATATTCCAACTACTTTACCATCTAGTAAAGCAACCTGTAGCTCTCTACCCTTGATATATTGCTCAA
CTAAAACCTTATTACCAAATTGAAATTTATAATCCTTAAATAGAAATTTATC
Sequence number 2323: Orientia tsutsugamushi tsa56 #2 
TTGCTCAACTAAAACCTTATTACCAAATTGAAATTTATAATCCTTAAATAGAAATTTATCTTCTAAAT
GTACAATTTTTACACCTATACTTGACCCCTGCTGTAGTGGCTTTATAACATA
Sequence number 2324: Orientia tsutsugamushi tsa56 #3 
TTCTAAATGTACAATTTTTACACCTATACTTGACCCCTGCTGTAGTGGCTTTATAACATATGGTCTTA
AAATAGGATCATTTATTACATTATCATTACTATTGATAGTTATACTGGCAGG
Sequence number 2325: Orientia tsutsugamushi tsa56 #4 
TGGTCTTAAAATAGGATCATTTATTACATTATCATTACTATTGATAGTTATACTGGCAGGAGTTTTAA
TCTTATAGCATTGTAATATAGTTTTAGCAATTTGTTTATCAAATGCAGCAGC
Sequence number 2326: Orientia tsutsugamushi tsa56 #5 
AGTTTTAATCTTATAGCATTGTAATATAGTTTTAGCAATTTGTTTATCAAATGCAGCAGCAGAAGTTT
TTACACCAGAATGAGTATATGGTATATTCATAATATTTAATAAACCAGGAAT
Sequence number 2327: Orientia tsutsugamushi tsa56 #6 
AGAAGTTTTTACACCAGAATGAGTATATGGTATATTCATAATATTTAATAAACCAGGAATACATCCAT
CCTCTCCATATGTACCAACTAGACAATTAAATACTGTATATGGTGGTGTAAT
Sequence number 2328: Orientia tsutsugamushi tsa56 #7 
ACATCCATCCTCTCCATATGTACCAACTAGACAATTAAATACTGTATATGGTGGTGTAATTTCAGATA
GTTTAACTGCAATATCTTGCCCAACATCTATACGAGTCACCTGATATCCATT
Sequence number 2329: Orientia tsutsugamushi tsa56 #8 
TTCAGATAGTTTAACTGCAATATCTTGCCCAACATCTATACGAGTCACCTGATATCCATTAGAGATAA
GAGCAGCTTGTACATTATCATTAGACATCAATGATACTTCTCTTTCTGCTGA
Sequence number 2330: Orientia tsutsugamushi tsa56 #9 
AGAGATAAGAGCAGCTTGTACATTATCATTAGACATCAATGATACTTCTCTTTCTGCTGACATACCAC
CATATAAGATTACTACATGCTTTTTGCCTGAACTAGTTATACTTTCAACAAC
Sequence number 2331: Orientia tsutsugamushi tsa56 #10 
CATACCACCATATAAGATTACTACATGCTTTTTGCCTGAACTAGTTATACTTTCAACAACAGAATTGC
GTCTTTGAACACTATAGGCTTTTTTTACCAACAATTTTAATCTCCCATTCTA
Sequence number 2332: Orientia tsutsugamushi tsa56 #11 
AGAATTGCGTCTTTGAACACTATAGGCTTTTTTTACCAACAATTTTAATCTCCCATTCTAATGTTATA
CCAGTATGGTCAAAAACATTTTTACGAATCAACTCCCCTAGATTTTCAATAT
Sequence number 2333: Orientia tsutsugamushi tsa56 #12 
ATGTTATACCAGTATGGTCAAAAACATTTTTACGAATCAACTCCCCTAGATTTTCAATATCGCTAGCT
GTAGCATTTCCATTATTAATTAAAAAATTGCAATGTAGATTTGACACTTGAG
Sequence number 2334: Orientia tsutsugamushi tsa56 #13 
CGCTAGCTGTAGCATTTCCATTATTAATTAAAAAATTGCAATGTAGATTTGACACTTGAGCACCACCA
ATACTATGCCCACGTAATCCTACCTTATCTATCAACTGCCAAGCTTTATAGT
Sequence number 2335: Orientia tsutsugamushi tsa56 #14 
CACCACCAATACTATGCCCACGTAATCCTACCTTATCTATCAACTGCCAAGCTTTATAGTTGTTAGTA
TTACGAAAAGCGCTACCAGCAGTTTTTTGGTTAATTGGCTGCGTTAATTTAC
Sequence number 2336: Orientia tsutsugamushi tsa56 #15 TGTTAGTATTACGAAAAGCGCTACCAGCAGTTTTTTGGTTAATTGGCTGCGTTAATTTACGAACTGTT TGTATTTTTGTAGCTTTTGACTAATACTTGTTTACTGTCATTATAACAAA
Sequence number 2337: Orientia tsutsugamushi tsa56 #16 
GAACTGTTTGTATTTTTTGTAGCTTTTGACTAATACTTGTTTTACTGTCATTATAACAAATGAGATTA
GTTTTTGTAAAGATTAAAAAGCCATTCACGATACATTGCCTATATTCAAAGT
Sequence number 2338: Orientia tsutsugamushi tsa56 #17 
TGAGATTAGTTTTTGTAAAGATTAAAAAGCCATTCACGATACATTGCCTATATTCAAAGTTTAAATCT
TTAGATAAAAATATATGCTTATTACCTAATCTATCTAAAGCCTCAACAGAAT
Sequence number 2339: Orientia tsutsugamushi tsa56 #18 
TTAAATCTTTAGATAAAAATATATGCTTATTACCTAATCTATCTAAAGCCTCAACAGAATAAACAACA
TCTTTAAATTCCTGTCCATAAGCTCCAGCATTCATAGCAATCCCCCCACCTA
Sequence number 2340: Orientia tsutsugamushi tsa56 #19 
AAACAACATCTTTAAATTCCTGTCCATAAGCTCCAGCATTCATAGCAATCCCCCCACCTATTGTGCCA
GGTATTCCTACTAAAAATTCTAAACCACCTAAGTTATGTTGTAGACAAAATT
Sequence number 2341: Orientia tsutsugamushi tsa56 #20 
TTGTGCCAGGTATTCCTACTAAAAATTCTAAACCACCTAAGTTATGTTGTAGACAAAATTTTGCAACA
TCATAGTTTAAAGTTCCAGCTCCAACACTAATAATATTATAGTGATTATTTT
Sequence number 2342: Orientia tsutsugamushi tsa56 #21 
TTGCAACATCATAGTTTAAAGTTCCAGCTCCAACACTAATAATATTATAGTGATTATTTTTAACAAAA
TTAATCTCATTGAAACTTCTACCTAATTTAATTGTTACACCATCAATACCAT
Sequence number 2343: Orientia tsutsugamushi tsa56 #22 
TAACAAAATTAATCTCATTGAAACTTCTACCTAATTTAATTGTTACACCATCAATACCATTATCTCTG
ATAAGTAAATTTGATCCTGCTCCTAATACTGTAATTTGAATATCAAATTGTA
Sequence number 2344: Orientia tsutsugamushi tsa56 #23 
TATCTCTGATAAGTAAATTTGATCCTGCTCCTAATACTGTAATTTGAATATCAAATTGTAAATTTTTT
AAAAAGCAAGACAGATCCTCCTCATCTTTAGGTTTATAAAAAACTTGACTAA
Sequence number 2345: Orientia tsutsugamushi tsa56 #24 
AATTTTTTAAAAAGCAAGACAGATCCTCCTCATCTTTAGGTTTATAAAAAACTTGACTAATTCCACCA
ACCTTAAACCAAGTCAGTTGAGATAGTTTGAAATTTTTTCTATATTCACCGT
Sequence number 2346: Orientia tsutsugamushi tsa56 #25 
TTCCACCAACCTTAAACCAAGTCAGTTGAGATAGTTTGAAATTTTTTCTATATTCACCGTTAATTTTT
GGTAGTGATATCACTGTAGACATGCTGCTTTAATAATTTCTGTAATTGCTGT
Sequence number 2347: Orientia tsutsugamushi tsa56 #26 
TAATTTTTGGTAGTGATATCACTGTAGACATGCTGCTTTAATAATTTCTGTAATTGCTGTGGCAATAT
TGCTGCAAATTTAGTAATATCTCCAGCTCCCATTAGTAGAATAATATCACAT
Sequence number 2348: Orientia tsutsugamushi tsa56 #27 
GGCAATATTGCTGCAAATTTAGTAATATCTCCAGCTCCCATTAGTAGAATAATATCACATGGTTTGAC
TTGCCTATAAATAATTTGTGGCAAATCATCTAAATTATTTATAGGCATAACA
Sequence number 2349: Orientia tsutsugamushi tsa56 #28 
GGTTTGACTTGCCTATAAATAATTTGTGGCAAATCATCTAAATTATTTATAGGCATAACATATACGCT
ATGATAATAACTCTTAATAGCATTAACTAAAGAAATATGAGATAAGTTATAT
Sequence number 2350: Orientia tsutsugamushi tsa56 #29 
TATACGCTATGATAATAACTCTTAATAGCATTAACTAAAGAAATATGAGATAAGTTATATGTTTCTTG
TTCTCCAGCATCGTATATTTGCGAAATATATAATTGATCTACACGTTGAAAG
Sequence number 2351: Orientia tsutsugamushi tsa56 #30 
GTTTCTTGTTCTCCAGCATCGTATATTTGCGAAATATATAATTGATCTACACGTTGAAAGCAATGAGT
AAACTCTGAAAATAACTGTTTTAATCTAGAGTAACGATGCGGTTGAAATACA
Sequence number 2352: Orientia tsutsugamushi tsa56 #31 
CAATGAGTAAACTCTGAAAATAACTGTTTTAATCTAGAGTAACGATGCGGTTGAAATACAGCTATAAC
CCTTCCCTGCTGCTTATCTGCAATACATCTTGCTGTATCAATTGTTGCTATA
Sequence number 2353: Orientia tsutsugamushi tsa56 #32 
GCTATAACCCTTCCCTGCTGCTTATCTGCAATACATCTTGCTGTATCAATTGTTGCTATAATTTCTGC
AGGATGGTGAGCATAGTCATCAATAACGGTAATGTTATTATACTCTCCGATC
Sequence number 2354: Orientia tsutsugamushi tsa56 #33 
ATTTCTGCAGGATGGTGAGCATAGTCATCAATAACGGTAATGTTATTATACTCTCCGATCTTTGTAAA
GCGCCTCGCAATACCTTTAAAATTTTTAAATCCATCCTTAATAATATTTAAG
Sequence number 2355: Orientia tsutsugamushi tsa56 #34 
TTTGTAAAGCGCCTCGCAATACCTTTAAAATTTTTAAATCCATCCTTAATAATATTTAAGTCTAAGTT
TAGCTCTACAGCAATAGCTATTGCAGCTAGTGAATTGAGAATGTTATGCTTA
Sequence number 2356: Orientia tsutsugamushi tsa56 #35 
TCTAAGTTTAGCTCTACAGCAATAGCTATTGCAGCTAGTGAATTGAGAATGTTATGCTTACCTAAAGC
TGGTAAAGTAACTTGCGTAATTATGACTTCATTATGAAAATTATATGCTTTA
Sequence number 2357: Orientia tsutsugamushi tsa56 #36 
CCTAAAGCTGGTAAAGTAACTTGCGTAATTATGACTTCATTATGAAAATTATATGCTTTAATTCTAAC
ATCATAGGTTGAATAACCATTATCAGTATTAATATTAAATGCCTGAATATGT
Sequence number 2358: Orientia tsutsugamushi tsa56 #37 
ATTCTAACATCATAGGTTGAATAACCATTATCAGTATTAATATTAAATGCCTGAATATGTGCATTCTT
AGAATCAATACCATAAGTAATAATCTTTCTAGATGTAATTTTAGCTATTAAA
Sequence number 2359: Orientia tsutsugamushi tsa56 #38 
GCATTCTTAGAATCAATACCATAAGTAATAATCTTTCTAGATGTAATTTTAGCTATTAAATTTTGTAC
TGTAATATCATCAATACAAGCTACTGCAAAACCGTCATATGGTAAATTAATA
Sequence number 2360: Orientia tsutsugamushi tsa56 #39 
TTTTGTACTGTAATATCATCAATACAAGCTACTGCAAAACCGTCATATGGTAAATTAATAATAAATTT
ACGAAAAGCACTAATCAAATTATCAAAATTTTTATAAAAATCTAAATGCTCA
Sequence number 2361: Orientia tsutsugamushi tsa56 #40 
ATAAATTTACGAAAAGCACTAATCAAATTATCAAAATTTTTATAAAAATCTAAATGCTCAACATCAAT
ATTAGTAATAACAGCAATCGTTGCAGGAATTTTAATAAAAGTTGCATCTGAT
Sequence number 2362: Orientia tsutsugamushi tsa56 #41 
ACATCAATATTAGTAATAACAGCAATCGTTGCAGGAATTTTAATAAAAGTTGCATCTGATTCATCAGC
TTCAGCAATGAAATAATCACCATCACCTAAATAAGCATTTGTGGCCCTATTA
Sequence number 2363: Orientia tsutsugamushi tsa56 #42 
TCATCAGCTTCAGCAATGAAATAATCACCATCACCTAAATAAGCATTTGTGGCCCTATTATTCATAAT
TCCACCAGTAATAACTGTAGGATGTAACCCACATGCTTCAAATATGCAAGCA
Sequence number 2364: Orientia tsutsugamushi tsa56 #43 TTCATAATTCCACCAGTAATAACTGTAGGATGTAACCCACATGCTTCAAATATGCAAGCAATCAATGC TGTTGTAGTAGTTTTGCCGTGAGATCCTGAAATTGCTATAGCAACCTTTGAT
Sequence number 2365: Orientia tsutsugamushi tsa56 #44 
ATCAATGCTGTTGTAGTAGTTTTGCCGTGAGATCCTGAAATTGCTATAGCAACCTTTGATCTCATTAG
CTCAGCTAACATCTCTGCTCTAGATATTATTGGTATTTTTGCTAAGATTGCA
Sequence number 2366: Orientia tsutsugamushi tsa56 #45 
CTCATTAGCTCAGCTAACATCTCTGCTCTAGATATTATTGGTATTTTTGCTAAGATTGCAGCCTGATA
TTCAGGGTTATTGTGTTTTATAGCAGAAGATATAACTACAATTCCTGCTCCA
Sequence number 2367: Orientia tsutsugamushi tsa56 #46 
GCCTGATATTCAGGGTTATTGTGTTTTATAGCAGAAGATATAACTACAATTCCTGCTCCATTAATATT
ATTAGCATTATGGCCATGTAATACTTCAATGCCTAATAATCTCAATCTTTCA
Sequence number 2368: Orientia tsutsugamushi tsa56 #47 
TTAATATTATTAGCATTATGGCCATGTAATACTTCAATGCCTAATAATCTCAATCTTTCAGTATTAAC
ATTACTAGTAAGATCTGACCCTCTAATCTTATAACCTAGATTGTACATTATT
Sequence number 2369: Orientia tsutsugamushi tsa56 #48 
GTATTAACATTACTAGTAAGATCTGACCCTCTAATCTTATAACCTAGATTGTACATTATTTCTGCAAT
ACCACTCATACCTATGCCACCGATACCAATAATATGCACAATACCAAAATTA
Sequence number 2370: Orientia tsutsugamushi tsa56 #49 
TCTGCAATACCACTCATACCTATGCCACCGATACCAATAATATGCACAATACCAAAATTACTTTTAAG
TCTTCGATTTGCTAACAATGTCATGCAAATAATACTTATATAAGCATGCCTT
Sequence number 2371: Orientia tsutsugamushi tsa56 #50 
CTTTTAAGTCTTCGATTTGCTAACAATGTCATGCAAATAATACTTATATAAGCATGCCTTTTTGACAT
GCTATTTTTTTTAAAATATACAGCCGTCTTCCATTTATCAGCTTATCAGCAT
Sequence number 2372: Orientia tsutsugamushi tsa56 #51 
TTTGACATGCTATTTTTTTTAAAATATACAGCCGTCTTCCATTTATCAGCTTATCAGCATTGCTTAAG
CATAAGCTGCAACTCAATATTAACTAAATAAGCAGCTAAAATTAGAAAAAGA
Sequence number 2373: Orientia tsutsugamushi tsa56 #52 
TGCTTAAGCATAAGCTGCAACTCAATATTAACTAAATAAGCAGCTAAAATTAGAAAAAGAAATTAATC
AAAATTTTTACATAATTTTTCATAATTTCTTTTTTTTCTATAATGTTATAAG
Sequence number 2374: Orientia tsutsugamushi tsa56 #53 
AATTAATCAAAATTTTTACATAATTTTTCATAATTTCTTTTTTTTCTATAATGTTATAAGAACTTAAA
AGCTAAATCTAACACTACATGATAAAGTATGGCCATTAAGATCATACGTCTT
Sequence number 2375: Orientia tsutsugamushi tsa56 #54 
AACTTAAAAGCTAAATCTAACACTACATGATAAAGTATGGCCATTAAGATCATACGTCTTAAAGTTAT
CATATGGCTTATCTTTCACATCTTCGCCTGTAATGCTTCCTATCCACATATA
Sequence number 2376: Orientia tsutsugamushi tsa56 #55 
AAAGTTATCATATGGCTTATCTTTCACATCTTCGCCTGTAATGCTTCCTATCCACATATACCTATATT
CAACATCTAAGTGCACTTCATCAATTACTTCAGCCGATATACCAGCCATAGC
Sequence number 2377: Orientia tsutsugamushi tsa56 #56 
CCTATATTCAACATCTAAGTGCACTTCATCAATTACTTCAGCCGATATACCAGCCATAGCTTCTTTAT
AAAAACCAACTTTATAACCTATCTTATGCTGTTCTATTGTACCACCAGTATA
Sequence number 2378: Orientia tsutsugamushi tsa56 #57 
TTCTTTATAAAAACCAACTTTATAACCTATCTTATGCTGTTCTATTGTACCACCAGTATATGAAGCAC
CAACCCCTCCACCAGCAAAAATTGACACTGGCCCTAATTTAATTAAATTAAG
Sequence number 2379: Orientia tsutsugamushi tsa56 #58 
TGAAGCACCAACCCCTCCACCAGCAAAAATTGACACTGGCCCTAATTTAATTAAATTAAGATAACACC
TTAATGCCAGAACTCCAGAGTTAAGAGATAACAAACTGCTATCTAGCTTATG
Sequence number 2380: Orientia tsutsugamushi tsa56 #59 
ATAACACCTTAATGCCAGAACTCCAGAGTTAAGAGATAACAAACTGCTATCTAGCTTATGATTAGGAT
TATTATTATTATCTAAGTCATTACCGCTAGCTTGTTGCTCTTCCTGTGAATT
Sequence number 2381: Orientia tsutsugamushi tsa56 #60 
ATTAGGATTATTATTATTATCTAAGTCATTACCGCTAGCTTGTTGCTCTTCCTGTGAATTACTTACAC
TTTGCTCTGGTTTAGTTGTAATATCAAAGAGCCACATAGCCCCAACTTCTGT
Sequence number 2382: Orientia tsutsugamushi tsa56 #61 
ACTTACACTTTGCTCTGGTTTAGTTGTAATATCAAAGAGCCACATAGCCCCAACTTCTGTCCTAAAAT
ATCGATTTACATAAGCCCCTAAACTTCCACCTATTCCAAATGAAAATTTGTT
Sequence number 2383: Orientia tsutsugamushi tsa56 #62 
CCTAAAATATCGATTTACATAAGCCCCTAAACTTCCACCTATTCCAAATGAAAATTTGTTAAACTCAA
GCTCTTTATTTTTAAAATGAGATTCCCAATTGGTTGGCATTAATCCAACCGC
Sequence number 2384: Orientia tsutsugamushi tsa56 #63 
AAACTCAAGCTCTTTATTTTTAAAATGAGATTCCCAATTGGTTGGCATTAATCCAACCGCACTAACCT
TAGCATAATAATTATATTCTAAATAGTCTAAATATTCTGATGAATTATTAGC
Sequence number 2385: Orientia tsutsugamushi tsa56 #64 
ACTAACCTTAGCATAATAATTATATTCTAAATAGTCTAAATATTCTGATGAATTATTAGCATGTACCA
AAGATGAACTTGATAATGCAGAAAGCGCAGTAATAGCATTTATTATTACTTT
Sequence number 2386: Orientia tsutsugamushi tsa56 #65 
ATGTACCAAAGATGAACTTGATAATGCAGAAAGCGCAGTAATAGCATTTATTATTACTTTTTTTATTT
TACTCATGAACTTTTACTTATTTTTTATGACTGTTGTTATTGATTTAAATGC
Sequence number 2387: Orientia tsutsugamushi tsa56 #66 
TTTTATTTTACTCATGAACTTTTACTTATTTTTTATGACTGTTGTTATTGATTTAAATGCTTCCTAAT
TACAAAAACTTCTCCATTTTAGGAGTATATTTCTATATATAATCCAACAAGC
Sequence number 2388: Orientia tsutsugamushi tsa56 #67 
TTCCTAATTACAAAAACTTCTCCATTTTAGGAGTATATTTCTATATATAATCCAACAAGCTTTAAGTT
AATTAAATAGCAAAGAATAAAGTTAATTTTTATTATTATAAAAACTCTGAAA
Sequence number 2389: Orientia tsutsugamushi tsa56 #68 
TTTAAGTTAATTAAATAGCAAAGAATAAAGTTAATTTTTATTATTATAAAAACTCTGAAAAAGTAGCT
AAAAAACCTGCAAAGGACTTTTAGCTGCTTTTTTTTTATAATTTTAAAGAAA
Sequence number 2390: Orientia tsutsugamushi tsa56 #69 
AAGTAGCTAAAAAACCTGCAAAGGACTTTTAGCTGCTTTTTTTTTATAATTTTAAAGAAAAACTAGAA
GTTATAGCGCACACATGCACTTGCAATAAACGCGTTTACTTGGTACTTATCT
Sequence number 2391: Orientia tsutsugamushi tsa56 #70 
AACTAGAAGTTATAGCGCACACATGCACTTGCAATAAACGCGTTTACTTGGTACTTATCTTCTACTTT
ACTGAATGAGTGCATATAACCAGCCTCTAAGTCCACACATACACCCTCAGCA
Sequence number 2392: Orientia tsutsugamushi tsa56 #71 TCTACTTTACTGAATGAGTGCATATAACCAGCCTCTAAGTCCACACATACACCCTCAGCAGCATTAAT TGCTACACCAAGTGCTCCTGATGCAACCATGCCTGTATACCCTTTAACATCC
Sequence number 2393: Orientia tsutsugamushi tsa56 #72 
GCATTAATTGCTACACCAAGTGCTCCTGATGCAACCATGCCTGTATACCCTTTAACATCCTTATTATC
TATTTTTCCATAAGTATAAGCTAACCCTGCACCAACACCACCGTATATTGAG
Sequence number 2394: Orientia tsutsugamushi tsa56 #73 
TTATTATCTATTTTTCCATAAGTATAAGCTAACCCTGCACCAACACCACCGTATATTGAGAATGATTC
AGTTGTAACTAGGTCAGCATAGAGTTTAACTTGGCCAACAACCATACTCAGA
Sequence number 2395: Orientia tsutsugamushi tsa56 #74 
AATGATTCAGTTGTAACTAGGTCAGCATAGAGTTTAACTTGGCCAACAACCATACTCAGATCAAACTC
TGTTTCTTTGACTTTTCCTTCTTTAGATTTTTCAGATGCTCCTTGTTGCTGC
Sequence number 2396: Orientia tsutsugamushi tsa56 #75 
TCAAACTCTGTTTCTTTGACTTTTCCTTCTTTAGATTTTTCAGATGCTCCTTGTTGCTGCTTGCAGTC
ACCTTTACCTTGATTCTTTGCATCTTCTTCTTGTTGGGCAGCTAATTTTTCC
Sequence number 2397: Orientia tsutsugamushi tsa56 #76 
TTGCAGTCACCTTTACCTTGATTCTTTGCATCTTCTTCTTGTTGGGCAGCTAATTTTTCCATGGCTTT
CCTAATTCCTGCATGACGCTGCAATTTAACAAGATCTTTATATAACTGCGCA
Sequence number 2398: Orientia tsutsugamushi tsa56 #77 
ATGGCTTTCCTAATTCCTGCATGACGCTGCAATTTAACAAGATCTTTATATAACTGCGCAATCTGATC
ACTGCCATTTAAAAGCCTAACAGCTGCTGCTGCTACTGCTTCTTGCGCTGTA
Sequence number 2399: Orientia tsutsugamushi tsa56 #78 
ATCTGATCACTGCCATTTAAAAGCCTAACAGCTGCTGCTGCTACTGCTTCTTGCGCTGTAGCTTGAGC
TTGCTGTTGCTGCCCTTGCCCCTGCTGTTGCTGTGCTTGCGGCGGCATGACA
Sequence number 2400: Orientia tsutsugamushi tsa56 #79 
GCTTGAGCTTGCTGTTGCTGCCCTTGCCCCTGCTGTTGCTGTGCTTGCGGCGGCATGACAAAATTCAA
GTGTATCTGATTAACAAAAGCATTATTAATATACCCATCAAAAGAATCTCTG
Sequence number 2401: Orientia tsutsugamushi tsa56 #80 
AAATTCAAGTGTATCTGATTAACAAAAGCATTATTAATATACCCATCAAAAGAATCTCTGAGTTCTTC
CAATGTATCACCTAATTCTTGGATTTTACTCTGTATCTGTTCGATAGATGCA
Sequence number 2402: Orientia tsutsugamushi tsa56 #81 
AGTTCTTCCAATGTATCACCTAATTCTTGGATTTTACTCTGTATCTGTTCGATAGATGCACTATTAGG
CAAACCAGTATCAGGAACATTAATACCAGCTATATCAGCAAATGGCTTTATA
Sequence number 2403: Orientia tsutsugamushi tsa56 #82 
CTATTAGGCAAACCAGTATCAGGAACATTAATACCAGCTATATCAGCAAATGGCTTTATATCACTATA
TATCTGAATAATTTTGTCACTCAAAACTTTGACAGGAGAAGCGCTAGGTTTA
Sequence number 2404: Orientia tsutsugamushi tsa56 #83 
TCACTATATATCTGAATAATTTTGTCACTCAAAACTTTGACAGGAGAAGCGCTAGGTTTATTAGCATT
TGATAATGCAGCAAGACCAACTACCAAACTCCTCCATTGCTCATGGTTATGT
Sequence number 2405: Orientia tsutsugamushi tsa56 #84 
TTAGCATTTGATAATGCAGCAAGACCAACTACCAAACTCCTCCATTGCTCATGGTTATGTATCGCAAA
ATTTGCAGGCTGATTTGCTCGCTGCGGTGGCTGTCCAACAGGATTAGGGTTG
Sequence number 2406: Orientia tsutsugamushi tsa56 #85 
ATCGCAAAATTTGCAGGCTGATTTGCTCGCTGCGGTGGCTGTCCAACAGGATTAGGGTTGCCCTGTGG
AATATTTAACAACACCGGATTTACCATCATATGCCCAGGATTATTAGGATCC
Sequence number 2407: Orientia tsutsugamushi tsa56 #86 
CCCTGTGGAATATTTAACAACACCGGATTTACCATCATATGCCCAGGATTATTAGGATCCTTCACCAT
ATAGTCAATACCAGCACAATTCTTTAACCAAGCGATCCTAGCTGCAGCACGC
Sequence number 2408: Orientia tsutsugamushi tsa56 #87 
TTCACCATATAGTCAATACCAGCACAATTCTTTAACCAAGCGATCCTAGCTGCAGCACGCTTCTGATC
ATTAAGCGGAGGCTGTGCAGCTTGCGCTTGCGCCTGAGGTATGTTAGGAATA
Sequence number 2409: Orientia tsutsugamushi tsa56 #88 
TTCTGATCATTAAGCGGAGGCTGTGCAGCTTGCGCTTGCGCCTGAGGTATGTTAGGAATATCAATCCC
AAAGTCACGATCAGCTATACTTATAGGCATCATAGTAGGCTGAGGTGGTGTA
Sequence number 2410: Orientia tsutsugamushi tsa56 #89 
TCAATCCCAAAGTCACGATCAGCTATACTTATAGGCATCATAGTAGGCTGAGGTGGTGTAAGTTTAAA
CGGCTTACGTATAGGAGCATCTGTCCCACCTCCAGAATCTGCCTTTATCTCA
Sequence number 2411: Orientia tsutsugamushi tsa56 #90 
AGTTTAAACGGCTTACGTATAGGAGCATCTGTCCCACCTCCAGAATCTGCCTTTATCTCACCTTTAGA
ATCTACCTCGCCTTTACCTACTTCAACCTCAGCGCTTATATTTCTAAGGTAC
Sequence number 2412: Orientia tsutsugamushi tsa56 #91 
CCTTTAGAATCTACCTCGCCTTTACCTACTTCAACCTCAGCGCTTATATTTCTAAGGTACATAACACC
TAGCTCTGCTCTAAATCCTGGTGCAATTGTCATACCCGCAGCTAATGTACCA
Sequence number 2413: Orientia tsutsugamushi tsa56 #92 
ATAACACCTAGCTCTGCTCTAAATCCTGGTGCAATTGTCATACCCGCAGCTAATGTACCACCAAATGG
CAGTCCAGTTGTTAATGACAAATGTTTTTTTCCCTCAGAATCAGTTGAATCC
Sequence number 2414: Orientia tsutsugamushi tsa56 #93 
CCAAATGGCAGTCCAGTTGTTAATGACAAATGTTTTTTTCCCTCAGAATCAGTTGAATCCAAGCGAGT
AGATTCTGCACCAGTAATCATTCCTCCAACAACTCCAACTTTAGCATAAGGA
Sequence number 2415: Orientia tsutsugamushi tsa56 #94 
AAGCGAGTAGATTCTGCACCAGTAATCATTCCTCCAACAACTCCAACTTTAGCATAAGGACCACACTC
TAATCCTACTTCATCCTCCAATTCTATCGCACTAGCTGAAAACGGCAACGAC
Sequence number 2416: Orientia tsutsugamushi tsa56 #95 
CCACACTCTAATCCTACTTCATCCTCCAATTCTATCGCACTAGCTGAAAACGGCAACGACAACGCAGA
CATTGCACTAGCAATTAACATAATTTTTTTCATTCTAATCTCCTTAAAAGAA
Sequence number 2417: Orientia tsutsugamushi tsa56 #96 
AACGCAGACATTGCACTAGCAATTAACATAATTTTTTTCATTCTAATCTCCTTAAAAGAATTAAATTT
ATTTTTTAGTGGTAACCATTCTAAACTAATTATTTAGCCACACTAACTTAGT
Sequence number 2418: Orientia tsutsugamushi tsa56 #97 
TTAAATTTATTTTTTAGTGGTAACCATTCTAAACTAATTATTTAGCCACACTAACTTAGTTTTATATC
AAAAAACTATTATTTAGTATACTAATATTTAATAGTACTTGTAAAATAATTT
Sequence number 2419: Orientia tsutsugamushi tsa56 #98 
TTTATATCAAAAAACTATTATTTAGTATACTAATATTTAATAGTACTTGTAAAATAATTTAAACAATC
TATAAAACACTGTGTTAAAAATATCGCTTCAAAGCGTTTTATCCATTGTAAA
Sequence number 2420: Orientia tsutsugamushi tsa56 #99 AAACAATCTATAAAACACTGTGTTAAAAATATCGCTTCAAAGCGTTTTATCCATTGTAAAATTTATTT TTATTTTTTATATATAACTTAAAGACAACATTCAATAGAACACTGCTCTTAT
Sequence number 2421: Orientia tsutsugamushi tsa56 #100 
ATTTATTTTTATTTTTTATATATAACTTAAAGACAACATTCAATAGAACACTGCTCTTATATATATGT
TTAAAGACACGTTCGAAAATACATTAATTATCTATTAGCACCTTGTTACAGT
Sequence number 2422: Orientia tsutsugamushi tsa56 #101 
ATATATGTTTAAAGACACGTTCGAAAATACATTAATTATCTATTAGCACCTTGTTACAGTTAACCTTA
GATAGCATAACACTACTTTTTACGCAACAACTACCTTCAATTATTAATAATT
Sequence number 2423: Orientia tsutsugamushi tsa56 #102 
TAACCTTAGATAGCATAACACTACTTTTTACGCAACAACTACCTTCAATTATTAATAATTTTTCTTGC
CTAAAAGTTTATTTTTAGCTAAAATAGCAACAGTTTTTCAAGCTAAATAATC
Sequence number 2424: Orientia tsutsugamushi tsa56 #103 
TTTCTTGCCTAAAAGTTTATTTTTAGCTAAAATAGCAACAGTTTTTCAAGCTAAATAATCAAGCCCTG
TCTTAATTATTATTTTATATAAAATCTTAAATCAAGATACATTAATTTAACC
Sequence number 2425: Orientia tsutsugamushi tsa56 #104 
AAGCCCTGTCTTAATTATTATTTTATATAAAATCTTAAATCAAGATACATTAATTTAACCACATAATT
ATACTAACATTGTATTATCTAAAATTAGCCCACATAAAAAATGAACAAACTT
Sequence number 2426: Orientia tsutsugamushi tsa56 #105 
ACATAATTATACTAACATTGTATTATCTAAAATTAGCCCACATAAAAAATGAACAAACTTTCTTTTCA
ACAAATAATAATGCACCTGCAGCAATTCTGGTGCAATCAAGGCTGTGCTATA
Sequence number 2427: Orientia tsutsugamushi tsa56 #106 
TCTTTTCAACAAATAATAATGCACCTGCAGCAATTCTGGTGCAATCAAGGCTGTGCTATATTGCAACC
ATATGACGTTGAAATTGGCGCAGGAACTTTTCATCCTGCAACAGCAATAAAA
Sequence number 2428: Orientia tsutsugamushi tsa56 #107 
TTGCAACCATATGACGTTGAAATTGGCGCAGGAACTTTTCATCCTGCAACAGCAATAAAATTGCTAGG
GCAAGCCAAATGGCGTGCAGCATATGTTCAGCCATCCAGGCGACCAACTGAT
Sequence number 2429: Orientia tsutsugamushi tsa56 #108 
TTGCTAGGGCAAGCCAAATGGCGTGCAGCATATGTTCAGCCATCCAGGCGACCAACTGATAGTAGATA
TGGCAGCCACCCTAATCGTTTACAGCAATACTACCAGTTTCAAGTCATAATT
Sequence number 2430: Orientia tsutsugamushi tsa56 #109 
AGTAGATATGGCAGCCACCCTAATCGTTTACAGCAATACTACCAGTTTCAAGTCATAATTAAGCCATC
TCCAGATAATATTCAAGCTCTTTACTTACATAGCTTAGAATATCTCAACATA
Sequence number 2431: Orientia tsutsugamushi tsa56 #110 
AAGCCATCTCCAGATAATATTCAAGCTCTTTACTTACATAGCTTAGAATATCTCAACATAAAAACTCA
AACTAATGATATTAGATTTGTAGAAGATGACTGGCAATCCCCTACCCTAGGA
Sequence number 2432: Orientia tsutsugamushi tsa56 #111 
AAAACTCAAACTAATGATATTAGATTTGTAGAAGATGACTGGCAATCCCCTACCCTAGGAGCCTCAGG
CTTAGGCTGGGAAGTATGGTGTAATGGAATGGAAATTTCTCAGATTACTTAC
Sequence number 2433: Orientia tsutsugamushi tsa56 #112 
GCCTCAGGCTTAGGCTGGGAAGTATGGTGTAATGGAATGGAAATTTCTCAGATTACTTACTTACAACA
AATAGCTAGCATAGAATGCAATCCAGTACCTATAGAAATCACTTATGGACTT
Sequence number 2434: Orientia tsutsugamushi tsa56 #113 
TTACAACAAATAGCTAGCATAGAATGCAATCCAGTACCTATAGAAATCACTTATGGACTTGAACGATT
AGCAATGTACATACAGGAAGTCGATGATATTAAACTACTAAATTGGAATAAT
Sequence number 2435: Orientia tsutsugamushi tsa56 #114 
GAACGATTAGCAATGTACATACAGGAAGTCGATGATATTAAACTACTAAATTGGAATAATCAATCAGG
AGAACTAGCTTTAACATATCAAATGGTTAACATAGAGAATGAACGGCAATTT
Sequence number 2436: Orientia tsutsugamushi tsa56 #115 
CAATCAGGAGAACTAGCTTTAACATATCAAATGGTTAACATAGAGAATGAACGGCAATTTTCTGCTTA
TAACCTTGAATACGCCAACACAAATATATTGTTTAAACAATTTGAAGACGCT
Sequence number 2437: Orientia tsutsugamushi tsa56 #116 
TCTGCTTATAACCTTGAATACGCCAACACAAATATATTGTTTAAACAATTTGAAGACGCTGAACAACA
ATGCCAAGATTTAGTCAAGCAAAATCTTCCTCTACCAGCATATGATTTCTGT
Sequence number 2438: Orientia tsutsugamushi tsa56 #117 
GAACAACAATGCCAAGATTTAGTCAAGCAAAATCTTCCTCTACCAGCATATGATTTCTGTTTAAAAGC
TAGCCACTTATTTAATCTACTTGATAGCAGAGGAGTAATCGGAGTTACTCAA
Sequence number 2439: Orientia tsutsugamushi tsa56 #118 
TTAAAAGCTAGCCACTTATTTAATCTACTTGATAGCAGAGGAGTAATCGGAGTTACTCAACGCACTAA
TTATATCGCTAGAATACGAACACTAGCTCAAACAGTATGTAATCAATATATA
Sequence number 2440: Orientia tsutsugamushi tsa56 #119 
CGCACTAATTATATCGCTAGAATACGAACACTAGCTCAAACAGTATGTAATCAATATATAAAAAATCT
TAATATATCAAATGCATAGTTACAGTTTAATATTAGAACTCTACTCTGAGGA
Sequence number 2441: Orientia tsutsugamushi tsa56 #120 
AAAAATCTTAATATATCAAATGCATAGTTACAGTTTAATATTAGAACTCTACTCTGAGGAAATTCCAG
CTAAAATGCAAAAAAAAGCTGAATTAGCATTTCAAGAAATTTTTACTAAAAA
Sequence number 2442: Orientia tsutsugamushi tsa56 #121 
AATTCCAGCTAAAATGCAAAAAAAAGCTGAATTAGCATTTCAAGAAATTTTTACTAAAAATTTCAAAT
TAGCTAATATTAGTTTTAATCACTTAAAAGTATATATAGGCCCATGCAGAGT
Sequence number 2443: Orientia tsutsugamushi tsa56 #122 
TTTCAAATTAGCTAATATTAGTTTTAATCACTTAAAAGTATATATAGGCCCATGCAGAGTGGTTCTAC
ATACAGTCTTTAGCAGCAAAGAACTAGCCGCTAAAGCAATTGAACATAAAGG
Sequence number 2444: Orientia tsutsugamushi tsa56 #123 
GGTTCTACATACAGTCTTTAGCAGCAAAGAACTAGCCGCTAAAGCAATTGAACATAAAGGCCCTAAAG
TCGGAGCAAAAAAATCAGCTGTTGAAGGATTTTGCAAAGCTAAGTCTATTTT
Sequence number 2445: Orientia tsutsugamushi tsa56 #124 
CCCTAAAGTCGGAGCAAAAAAATCAGCTGTTGAAGGATTTTGCAAAGCTAAGTCTATTTTAAGCAAGG
ACTTAACCATAAAAACATTTAATAATCAGCAGTTCTACGTTTATACTCATGT
Sequence number 2446: Orientia tsutsugamushi tsa56 #125 
AAGCAAGGACTTAACCATAAAAACATTTAATAATCAGCAGTTCTACGTTTATACTCATGTTACTCCAA
CTCAACCTATTAAAGATCTATTACCAGAAATCATATTAAATTCCATTAAAGA
Sequence number 2447: Orientia tsutsugamushi tsa56 #126 
TACTCCAACTCAACCTATTAAAGATCTATTACCAGAAATCATATTAAATTCCATTAAAGAATACACTT
GGCCAAAATCAATGAAATGGGGGAATTATCAGATTAATTGGGTTAGACCACT
Sequence number 2448: Orientia tsutsugamushi tsa56 #127 ATACACTTGGCCAAAATCAATGAAATGGGGGAATTATCAGATTAATTGGGTTAGACCACTTAGAAACA TTTTATGCATGTTCAATGGCGAAGAATTAAAAATCAGCTGGCATCATCTAAC
Sequence number 2449: Orientia tsutsugamushi tsa56 #128 
TAGAAACATTTTATGCATGTTCAATGGCGAAGAATTAAAAATCAGCTGGCATCATCTAACATCAAATA
ATAAAAGCTTTGGGCATAAATTCATGAGTCCAAATAGCTTTACCGTAAGCAC
Sequence number 2450: Orientia tsutsugamushi tsa56 #129 
ATCAAATAATAAAAGCTTTGGGCATAAATTCATGAGTCCAAATAGCTTTACCGTAAGCACATGGAAAG
ATTATACTAATAATTTAAGAGATAATTTTGTCATATTAGACAGATCAGAACG
Sequence number 2451: Orientia tsutsugamushi tsa56 #130 
ATGGAAAGATTATACTAATAATTTAAGAGATAATTTTGTCATATTAGACAGATCAGAACGACAAGCTA
TTATACTTAATTCCTTAGAAAAAACATGCCAAACTCTAGGCATCACCTTAAA
Sequence number 2452: Orientia tsutsugamushi tsa56 #131 
ACAAGCTATTATACTTAATTCCTTAGAAAAAACATGCCAAACTCTAGGCATCACCTTAAATTACAATA
ATAAATTACTTGATGAAGTTACTGGTTTAGTTGAATACCCTATCGTATTATA
Sequence number 2453: Orientia tsutsugamushi tsa56 #132 
TTACAATAATAAATTACTTGATGAAGTTACTGGTTTAGTTGAATACCCTATCGTATTATATGGCACAA
TACCTAATGAGTTTCTTAGCTTACCAGAAGAAATCATCATCACTTCTGTACA
Sequence number 2454: Orientia tsutsugamushi tsa56 #133 
TGGCACAATACCTAATGAGTTTCTTAGCTTACCAGAAGAAATCATCATCACTTCTGTACAAACTCATC
AAAAATATTTCACTACAAAAGATCATAGCAATAAACTAGCTCCATATTTTTT
Sequence number 2455: Orientia tsutsugamushi tsa56 #134 
AACTCATCAAAAATATTTCACTACAAAAGATCATAGCAATAAACTAGCTCCATATTTTTTATTTGTTA
CCAATATCAATACTGAAGATTACAGCAATATTATCAAAGGAAATGAAAAAGT
Sequence number 2456: Orientia tsutsugamushi tsa56 #135 
ATTTGTTACCAATATCAATACTGAAGATTACAGCAATATTATCAAAGGAAATGAAAAAGTCTTATCAT
CTCGTCTAACTGATGCTATATACTTTTTTAATCAAGACTGTCAACTTACATT
Sequence number 2457: Orientia tsutsugamushi tsa56 #136 
CTTATCATCTCGTCTAACTGATGCTATATACTTTTTTAATCAAGACTGTCAACTTACATTGCTTAGTA
AATTAGAAAAATTAAAAAAAGTAGTCTTTCACACTAAAATTGGTAGTATATT
Sequence number 2458: Orientia tsutsugamushi tsa56 #137 
GCTTAGTAAATTAGAAAAATTAAAAAAAGTAGTCTTTCACACTAAAATTGGTAGTATATTTGATAAAG
TTCAGAGAATAAAAAAAATATGCCATAAGCTTGCACCTGATGATCAAGAACT
Sequence number 2459: Orientia tsutsugamushi tsa56 #138 
TGATAAAGTTCAGAGAATAAAAAAAATATGCCATAAGCTTGCACCTGATGATCAAGAACTAGCTATTG
CAGCTGAACTGTGTAAATCAGATTTAGTCTCAGAAGTGGTACAAGAATTCCC
Sequence number 2460: Orientia tsutsugamushi tsa56 #139 
AGCTATTGCAGCTGAACTGTGTAAATCAGATTTAGTCTCAGAAGTGGTACAAGAATTCCCTAATCTAC
AAGGAATAATGGGAAAATACTATGCTTTAAATGACAAACTCAGCCCTGCAGT
Sequence number 2461: Orientia tsutsugamushi tsa56 #140 
TAATCTACAAGGAATAATGGGAAAATACTATGCTTTAAATGACAAACTCAGCCCTGCAGTCGCTAATA
TAATCGGCAACCACTACTATCCTACCAGAATTGAAGATGAAGTACCTAATGG
Sequence number 2462: Orientia tsutsugamushi tsa56 #141 
CGCTAATATAATCGGCAACCACTACTATCCTACCAGAATTGAAGATGAAGTACCTAATGGTAAGGCAG
CAATTCTTGCTTTAGCTGACAAGCTTGATAATTTAACTAGCCTATATTTAGC
Sequence number 2463: Orientia tsutsugamushi tsa56 #142 
TAAGGCAGCAATTCTTGCTTTAGCTGACAAGCTTGATAATTTAACTAGCCTATATTTAGCAGGAGAGC
GCGCTACTAGTTCAAAAGATCCATTTGCATTACGTAGATGCGCTTTTTCTAT
Sequence number 2464: Orientia tsutsugamushi tsa56 #143 
AGGAGAGCGCGCTACTAGTTCAAAAGATCCATTTGCATTACGTAGATGCGCTTTTTCTATCGCTAGAA
TAATTTTAACTAATAAAATACAAGTAAAATTATTAGATTTATTAACTTTCAT
Sequence number 2465: Orientia tsutsugamushi tsa56 #144 
CGCTAGAATAATTTTAACTAATAAAATACAAGTAAAATTATTAGATTTATTAACTTTCATTTATCAAC
TTCATGATATTTATACTAATGAGCTAGCAACCGAAGTACTACTATTCATAAA
Sequence number 2466: Orientia tsutsugamushi tsa56 #145 
TTATCAACTTCATGATATTTATACTAATGAGCTAGCAACCGAAGTACTACTATTCATAAAAGATAAAA
CAAAAAATTTACTTAATGCAAAATTTAATCATTTGTTAGTCGAAGCTATATT
Sequence number 2467: Orientia tsutsugamushi tsa56 #146 
AGATAAAACAAAAAATTTACTTAATGCAAAATTTAATCATTTGTTAGTCGAAGCTATATTTCAGCAAA
ACAACTATGATGATCTTACTGAGTTAGAATTAAGAATGAATATTCTTGATCA
Sequence number 2468: Orientia tsutsugamushi tsa56 #147 
TCAGCAAAACAACTATGATGATCTTACTGAGTTAGAATTAAGAATGAATATTCTTGATCATTTTTTAA
CCTCAACTAAAGGTCAAAACTTTATACAATCATATAAAAGAGCGTATAATAT
Sequence number 2469: Orientia tsutsugamushi tsa56 #148 
TTTTTTAACCTCAACTAAAGGTCAAAACTTTATACAATCATATAAAAGAGCGTATAATATCATTAAAG
ATGAAGAAATTCGTCAAGCAAAAATAGATGGTTACAAATTATTAACTAAACA
Sequence number 2470: Orientia tsutsugamushi tsa56 #149 
CATTAAAGATGAAGAAATTCGTCAAGCAAAAATAGATGGTTACAAATTATTAACTAAACAAGAAACCA
TCCTATATCAGCAAGCTATGAATTCAAAAGTTAGCCAATTATCTACTCAATT
Sequence number 2471: Orientia tsutsugamushi tsa56 #150 
AGAAACCATCCTATATCAGCAAGCTATGAATTCAAAAGTTAGCCAATTATCTACTCAATTCTCAGCTG
AAAGTTTTACTTTAGCTCTTGATCAATTATCAAATCTTAGCATACAAATTGC
Sequence number 2472: Orientia tsutsugamushi tsa56 #151 
CTCAGCTGAAAGTTTTACTTTAGCTCTTGATCAATTATCAAATCTTAGCATACAAATTGCAGAATTTT
TAGACAATGTCACTGTTAACACTACTGATCAGTATCTTACTGATACTCGCAA
Sequence number 2473: Orientia tsutsugamushi tsa56 #152 
AGAATTTTTAGACAATGTCACTGTTAACACTACTGATCAGTATCTTACTGATACTCGCAAGCTTATAT
TACAATATGTAGTACAGTTTTGCAATAATATAGCACCATTTGACAAAGTAAT
Sequence number 2474: Orientia tsutsugamushi tsa56 #153 
GCTTATATTACAATATGTAGTACAGTTTTGCAATAATATAGCACCATTTGACAAAGTAATAAAACATT
ATGTTAGCAAATATGAACAGTAATAATAATGATATAAATGTTGCTGCACAAT
Sequence number 2475: Orientia tsutsugamushi tsa56 #154 
AAAACATTATGTTAGCAAATATGAACAGTAATAATAATGATATAAATGTTGCTGCACAATTTATTGAA
CAAGGGAAATTAGTTATATTCCCTACTGAAACAGTGTATGGCATAGGAGCTG
Sequence number 2476: Orientia tsutsugamushi tsa56 #155 TTATTGAACAAGGGAAATTAGTTATATTCCCTACTGAAACAGTGTATGGCATAGGAGCTGATGCAACT AATGACAATGCTTGTCAAAGCATTTATAAAATCAAAAACAGACCAATGAATA
Sequence number 2477: Orientia tsutsugamushi tsa56 #156 
ATGCAACTAATGACAATGCTTGTCAAAGCATTTATAAAATCAAAAACAGACCAATGAATAATCCATTA
ATTATTCATGTCAGCAATATACAAGCTGCTCAAACTATAGGCATATTTAACA
Sequence number 2478: Orientia tsutsugamushi tsa56 #157 
ATCCATTAATTATTCATGTCAGCAATATACAAGCTGCTCAAACTATAGGCATATTTAACAATCTTGCT
ATTAAACTAGCAACCTTATGGCCAGGCCCAATTAGCTTAGTTGTACCACTTA
Sequence number 2479: Orientia tsutsugamushi tsa56 #158 
ATCTTGCTATTAAACTAGCAACCTTATGGCCAGGCCCAATTAGCTTAGTTGTACCACTTAATCAATCA
GCAGGAATTAGCAGAACAGCAACAGCAAATCTTGATACTGTAGCAATTAGAA
Sequence number 2480: Orientia tsutsugamushi tsa56 #159 
ATCAATCAGCAGGAATTAGCAGAACAGCAACAGCAAATCTTGATACTGTAGCAATTAGAATTCCATCT
CATAAGGTAGCTCTCATGTTGCTACAGTTAACACAAAAACCTATAGCTGCTC
Sequence number 2481: Orientia tsutsugamushi tsa56 #160 
TTCCATCTCATAAGGTAGCTCTCATGTTGCTACAGTTAACACAAAAACCTATAGCTGCTCCAAGCGCA
AACCCATCAGGCTATCTCAGCCCTACTACCTATCAACATGCCAATATCCATT
Sequence number 2482: Orientia tsutsugamushi tsa56 #161 
CAAGCGCAAACCCATCAGGCTATCTCAGCCCTACTACCTATCAACATGCCAATATCCATTTCCAAAAT
TCTCCAGACATATTCATTCTTAAGGGCGCTCAATCAACTTATGGATTAGAAT
Sequence number 2483: Orientia tsutsugamushi tsa56 #162 
TCCAAAATTCTCCAGACATATTCATTCTTAAGGGCGCTCAATCAACTTATGGATTAGAATCTACTATA
ATTGATACTACTGCTGATAGACCAACAATATTACGCCATGGATTCATTACAA
Sequence number 2484: Orientia tsutsugamushi tsa56 #163 
CTACTATAATTGATACTACTGCTGATAGACCAACAATATTACGCCATGGATTCATTACAACAGAAATA
ATAAAGACTATTACTGGGCAAAACCCTATTATAGCAGAAAAACATAACAAGC
Sequence number 2485: Orientia tsutsugamushi tsa56 #164 
CAGAAATAATAAAGACTATTACTGGGCAAAACCCTATTATAGCAGAAAAACATAACAAGCTTTTAAAA
GCTCCAGGAATGATGTTAAAGCATTATTCTCCTATTACTAAAATTAGGTTAA
Sequence number 2486: Orientia tsutsugamushi tsa56 #165 
TTTTAAAAGCTCCAGGAATGATGTTAAAGCATTATTCTCCTATTACTAAAATTAGGTTAAATGCAACA
AAGCTTCTTCCAAAAGAAGTAGGATTAAATTATGGGCCTAATACTCTAACTG
Sequence number 2487: Orientia tsutsugamushi tsa56 #166 
ATGCAACAAAGCTTCTTCCAAAAGAAGTAGGATTAAATTATGGGCCTAATACTCTAACTGGAAAATAC
TGCCTAAATCTTAGCCCAAACAGTAATTTAATGAATGCAGCAGCAAACTTAT
Sequence number 2488: Orientia tsutsugamushi tsa56 #167 
GAAAATACTGCCTAAATCTTAGCCCAAACAGTAATTTAATGAATGCAGCAGCAAACTTATATCATATG
CTTCATCAACTTGACAACTATGCACAAAATCCTATCAATGAGGTTGAGACTA
Sequence number 2489: Orientia tsutsugamushi tsa56 #168 
ATCATATGCTTCATCAACTTGACAACTATGCACAAAATCCTATCAATGAGGTTGAGACTATCGCTATA
GCTCAAATACCAAATATAGGCATAGGAATTGCTATCAATGACAAGCTTAATC
Sequence number 2490: Orientia tsutsugamushi tsa56 #169 
TCGCTATAGCTCAAATACCAAATATAGGCATAGGAATTGCTATCAATGACAAGCTTAATCGCGCAAGC
TCATATTAACCCCAGAATAAGACAAGAGAAAGTATAAAAGGTATAGAATAAA
Sequence number 2491: Orientia tsutsugamushi tsa56 #170 
GCGCAAGCTCATATTAACCCCAGAATAAGACAAGAGAAAGTATAAAAGGTATAGAATAAAAGATATAC
AAAGATAAAATGTTGAGTTACAAAGAAAATTTAAATAAAAATAAAATAACTC
Sequence number 2492: Orientia tsutsugamushi tsa56 #171 
AGATATACAAAGATAAAATGTTGAGTTACAAAGAAAATTTAAATAAAAATAAAATAACTCAACATGAA
AAAATGTATTATAACAGTATACTATTTAATAGATAATTCTTGCAAGATATAT
Sequence number 2493: Orientia tsutsugamushi tsa56 #172 
AACATGAAAAAATGTATTATAACAGTATACTATTTAATAGATAATTCTTGCAAGATATATCAAGAGTG
AGAAAGAAAGAGATTAATACCAAGTAATAATCAAAAAAAAACAAAGATGGAA
Sequence number 2494: Orientia tsutsugamushi tsa56 #173 
CAAGAGTGAGAAAGAAAGAGATTAATACCAAGTAATAATCAAAAAAAAACAAAGATGGAAAGTTATTT
TTAACTGAGTTATTAACAATACTGATATATTAGACCTCTTTCGAAACTGGTT
Sequence number 2495: Orientia tsutsugamushi tsa56 #174 
AGTTATTTTTAACTGAGTTATTAACAATACTGATATATTAGACCTCTTTCGAAACTGGTTGACGTTGT
TCAAAATTATGAATGCCAGAGATCAAATTAAATCTAAGACCTAATCTTTTAC
Sequence number 2496: Orientia tsutsugamushi tsa56 #175 
GACGTTGTTCAAAATTATGAATGCCAGAGATCAAATTAAATCTAAGACCTAATCTTTTACGTCTATTT
TGATATTTATCAGCAATAATTTTGAACTACGTCAACCAGTTTCGAAAGAGAT
Sequence number 2497: Orientia tsutsugamushi tsa56 #176 
GTCTATTTTGATATTTATCAGCAATAATTTTGAACTACGTCAACCAGTTTCGAAAGAGATCTATTTTT
ATTTATCTCAATGTAAGGATTTTAAAAATTATTATCTATATTACTTGTGTCA
Sequence number 2498: Orientia tsutsugamushi tsa56 #177 
CTATTTTTATTTATCTCAATGTAAGGATTTTAAAAATTATTATCTATATTACTTGTGTCATTAAGTAT
AAAAAAGAAAGCTAGTATACAGTGCATGACCTTAGGATAATAGCCTGAATTT
Sequence number 2499: Orientia tsutsugamushi tsa56 #178 
AAAAATTATTATCTATATTACTTGTGTCATTAAGTATAAAAAAGAAAGCTAGTATACAGTGCATGACC
TTAGGATAATAGCCTGAATTTTAGATAAGTACCTAGCTTTTTTGAAAACTAA
Sequence number 2500: Rickettsia akari rOmpA #1 
AACCCGCATTATAAAACTATCGGCAGTTTTTCTAATATTATCTAACATAATTTTTTGTCTAAGTTTTT
AAGTGAGAATATGTCACACCCGCGGGGCAATGCATCCTCAGCATTACGGAAT
Sequence number 2501: Rickettsia akari rOmpA #2 
AAGTTTTTAAGTGAGAATATGTCACACCCGCGGGGCAATGCATCCTCAGCATTACGGAATGACATTTA
AGCAGCTTCTCAAGACTGCATTATAAAGCAAAAAAGCCTATATAGTCAAACT
Sequence number 2502: Rickettsia akari rOmpA #3 
GACATTTAAGCAGCTTCTCAAGACTGCATTATAAAGCAAAAAAGCCTATATAGTCAAACTATATAGGC
TTTTTTATTATTTTTGTAGATGTTCGATGTCATTTCTGTATGGTTCAGTTTT
Sequence number 2503: Rickettsia akari rOmpA #4 
ATATAGGCTTTTTTATTATTTTTGTAGATGTTCGATGTCATTTCTGTATGGTTCAGTTTTTCTGTCAT
TGCAATGAAATTACAAAGTAATTGACGCAGCAATCTCAGAAGTTTGTTATTA
Sequence number 2504: Rickettsia akari rOmpA #5 TCTGTCATTGCAATGAAATTACAAAGTAATTGACGCAGCAATCTCAGAAGTTTGTTATTATTTCATGA GATTGCCACGCAGCCTATGGCTTGCTCGCAATGACGACCTAGAGCATATTAG
Sequence number 2505: Rickettsia akari rOmpA #6 
TTTCATGAGATTGCCACGCAGCCTATGGCTTGCTCGCAATGACGACCTAGAGCATATTAGAAGTTTAT
ACGAACTTTTACACTACCTTGCTGAGTAAAATACTTACTTCCGATATTGGTA
Sequence number 2506: Rickettsia akari rOmpA #7 
AAGTTTATACGAACTTTTACACTACCTTGCTGAGTAAAATACTTACTTCCGATATTGGTATCGTAGTT
AATACCGTATTCCATCATTTGATGCTTAGCAGTAATACCAACACCGATATCA
Sequence number 2507: Rickettsia akari rOmpA #8 
TCGTAGTTAATACCGTATTCCATCATTTGATGCTTAGCAGTAATACCAACACCGATATCAAAGCTTGT
TTTGCTTTGCTTAAAGCTGTTGGTTGGGAAAGGAGCAATCATACCTTGTAAT
Sequence number 2508: Rickettsia akari rOmpA #9 
AAGCTTGTTTTGCTTTGCTTAAAGCTGTTGGTTGGGAAAGGAGCAATCATACCTTGTAATCTCGCATC
AATCGCCGGAACTTTATTCTTGAATGCATAATCAACCATTGCGTACAGCTCA
Sequence number 2509: Rickettsia akari rOmpA #10 
CTCGCATCAATCGCCGGAACTTTATTCTTGAATGCATAATCAACCATTGCGTACAGCTCAGGAGTTAA
CACTACTTCACCTACATTTATATTACTTAATACCTTAGCACCGAGTAAACCA
Sequence number 2510: Rickettsia akari rOmpA #11 
GGAGTTAACACTACTTCACCTACATTTATATTACTTAATACCTTAGCACCGAGTAAACCATCGAAAGT
GTTATAGTTCTTGCCTTTAACTGTAAGATTTTTGTTAGTAGTACCGGTTTCT
Sequence number 2511: Rickettsia akari rOmpA #12 
TCGAAAGTGTTATAGTTCTTGCCTTTAACTGTAAGATTTTTGTTAGTAGTACCGGTTTCTTTATAACC
CTTATCTTTGATAGCCGAATATCTAAGCCCAACTAGAGGTGTTAAGCTAATG
Sequence number 2512: Rickettsia akari rOmpA #13 
TTATAACCCTTATCTTTGATAGCCGAATATCTAAGCCCAACTAGAGGTGTTAAGCTAATGTTTTCACG
CACCATATAGGTATAACCAGCCATTAACTAACCTGTATAGCTTTCTGATTTA
Sequence number 2513: Rickettsia akari rOmpA #14 
TTTTCACGCACCATATAGGTATAACCAGCCATTAACTAACCTGTATAGCTTTCTGATTTATACTTACC
GCTTGCGGTTTGATAGCCGACAGTCTCTAGTGCCGTCGCAATAACACATCTT
Sequence number 2514: Rickettsia akari rOmpA #15 
TACTTACCGCTTGCGGTTTGATAGCCGACAGTCTCTAGTGCCGTCGCAATAACACATCTTGATTTGCT
TCTTATCTTGTTATCAGAGTAAGATGCTATAGCTTTAACGCAAAGATTTGCA
Sequence number 2515: Rickettsia akari rOmpA #16 
GATTTGCTTCTTATCTTGTTATCAGAGTAAGATGCTATAGCTTTAACGCAAAGATTTGCATAAGGTAC
GTTATATAAACCGTATAAAGAGTAAATATTACTCTCTACCTCATTCTTATCA
Sequence number 2516: Rickettsia akari rOmpA #17 
TAAGGTACGTTATATAAACCGTATAAAGAGTAAATATTACTCTCTACCTCATTCTTATCACCTGTCTT
ATTATTTTTCAGTTTGATATCAGTATCGGCTCCAGCTCTTGTATATGCAAGT
Sequence number 2517: Rickettsia akari rOmpA #18 
CCTGTCTTATTATTTTTCAGTTTGATATCAGTATCGGCTCCAGCTCTTGTATATGCAAGTCCGAGTGC
TAAATCATCCTTAACTAAGCCATCAAAACCTATAGTACCACCGATTGTATCA
Sequence number 2518: Rickettsia akari rOmpA #19 
CCGAGTGCTAAATCATCCTTAACTAAGCCATCAAAACCTATAGTACCACCGATTGTATCAGACTTATA
ACCGCTTATATTGTTATGCATCTTCTGCATTGCATTACCGACAAATGAGCTC
Sequence number 2519: Rickettsia akari rOmpA #20 
GACTTATAACCGCTTATATTGTTATGCATCTTCTGCATTGCATTACCGACAAATGAGCTCCAAACTTA
GCATCTCTGCCCTCATCACAAGCAGCTACAGCAACCGGCTCTATTGCATCCA
Sequence number 2520: Rickettsia akari rOmpA #21 
CAAACTTAGCATCTCTGCCCTCATCACAAGCAGCTACAGCAACCGGCTCTATTGCATCCATTCTAGCA
TTTAGATTAATTAAGCTACTTGAGATATTACTGAGTATAACTTGATTATTAA
Sequence number 2521: Rickettsia akari rOmpA #22 
TTCTAGCATTTAGATTAATTAAGCTACTTGAGATATTACTGAGTATAACTTGATTATTAATAGCAGCT
ATAGTATCGCTAAGCTTTACAACGTCTTGCATGAGCTGAGTTACAGTATCTG
Sequence number 2522: Rickettsia akari rOmpA #23 
TAGCAGCTATAGTATCGCTAAGCTTTACAACGTCTTGCATGAGCTGAGTTACAGTATCTGCTTCTTGG
ATTGGACTCAGTTTACCCCAATTATCGAAAGCTCAACATGCATCTGAACCTC
Sequence number 2523: Rickettsia akari rOmpA #24 
CTTCTTGGATTGGACTCAGTTTACCCCAATTATCGAAAGCTCAACATGCATCTGAACCTCTTTGTGCT
TGCTCCATTAACTCAAGCGATTTCTTTACATTTGCAGCATTTGTGATATTTG
Sequence number 2524: Rickettsia akari rOmpA #25 
TTTGTGCTTGCTCCATTAACTCAAGCGATTTCTTTACATTTGCAGCATTTGTGATATTTGCAAGCGGC
CCACCAGGTTTAAAATATGCTTCTATCACTTCTTCCGCCATATCCTCTGCAA
Sequence number 2525: Rickettsia akari rOmpA #26 
CAAGCGGCCCACCAGGTTTAAAATATGCTTCTATCACTTCTTCCGCCATATCCTCTGCAAATAACGTC
AAAGTCGATTCATCAAAAGTAAAGTTGACAAAGCGGTTGTAATTATTAACGG
Sequence number 2526: Rickettsia akari rOmpA #27 
ATAACGTCAAAGTCGATTCATCAAAAGTAAAGTTGACAAAGCGGTTGTAATTATTAACGGTTACTTTA
ACATTACCTGCGGGAGTAGGCTTTCATCCGCCTGCTGTTTCTGAAGATATTA
Sequence number 2527: Rickettsia akari rOmpA #28 
TTACTTTAACATTACCTGCGGGAGTAGGCTTTCATCCGCCTGCTGTTTCTGAAGATATTACCGTATAT
TTTGTATCCGGGCTTATATTATTAATGTCAAAATTAGTAGCAGTAACAACAA
Sequence number 2528: Rickettsia akari rOmpA #29 
CCGTATATTTTGTATCCGGGCTTATATTATTAATGTCAAAATTAGTAGCAGTAACAACAAGTTCTAAA
GTTGAAACTCCTGATAAATCAAGAGTACTACCTGATTTAATTAAGATATTAC
Sequence number 2529: Rickettsia akari rOmpA #30 
GTTCTAAAGTTGAAACTCCTGATAAATCAAGAGTACTACCTGATTTAATTAAGATATTACCGTCTAAC
TTATCTGCACCATCAAAAGTAGTATTTAAGGTTAGTGTATCGGTAAAGCTAC
Sequence number 2530: Rickettsia akari rOmpA #31 
CGTCTAACTTATCTGCACCATCAAAAGTAGTATTTAAGGTTAGTGTATCGGTAAAGCTACCGGTACCG
GTATATGTTATTTGGCTTGCACCTAAAGTAAGTGTAGTACCGCTACCTGTTA
Sequence number 2531: Rickettsia akari rOmpA #32 
CGGTACCGGTATATGTTATTTGGCTTGCACCTAAAGTAAGTGTAGTACCGCTACCTGTTATATTACTA
TTAAAAGCTAGGCTATTACCGAAATTAATAGTAGCATTGTTAACTGCAAAGC
Sequence number 2532: Rickettsia akari rOmpA #33 TATTACTATTAAAAGCTAGGCTATTACCGAAATTAATAGTAGCATTGTTAACTGCAAAGCTGGTAGCC GTTACATTTTTAGCAAAATTAGTGATAGAAGCTTTAGCTAAAGTAACTGCAC
Sequence number 2533: Rickettsia akari rOmpA #34 
TGGTAGCCGTTACATTTTTAGCAAAATTAGTGATAGAAGCTTTAGCTAAAGTAACTGCACCAGTGTTA
GTAAATGTATCGGCAAAACTTGTAGTACCGCAGAGCGTCGCCGTACCTTTTG
Sequence number 2534: Rickettsia akari rOmpA #35 
CAGTGTTAGTAAATGTATCGGCAAAACTTGTAGTACCGCAGAGCGTCGCCGTACCTTTTGCATTAACA
CTGCTTGCAAAGTTTATTATGCCTGCCGTTGTGATATCACCCACCGAATTAG
Sequence number 2535: Rickettsia akari rOmpA #36 
CATTAACACTGCTTGCAAAGTTTATTATGCCTGCCGTTGTGATATCACCCACCGAATTAGCCACAGTC
CCTACAACACCACTAACACTGCCGCCATTCGTGAAGTTTAACTTCAGAGCCT
Sequence number 2536: Rickettsia akari rOmpA #37 
CCACAGTCCCTACAACACCACTAACACTGCCGCCATTCGTGAAGTTTAACTTCAGAGCCTGACCACCG
GTTTTATTTATGCTGCCTGTTAAGTTAAAGTTTGCAGGTAATGTGAAGAGTG
Sequence number 2537: Rickettsia akari rOmpA #38 
GACCACCGGTTTTATTTATGCTGCCTGTTAAGTTAAAGTTTGCAGGTAATGTGAAGAGTGCAGTACCG
TTACCTTGGATTTCAGTGATGCTAGCATTACCGCCTTGGGTTATGCTTACAG
Sequence number 2538: Rickettsia akari rOmpA #39 
CAGTACCGTTACCTTGGATTTCAGTGATGCTAGCATTACCGCCTTGGGTTATGCTTACAGCCCCGGCA
CCTACTTTTAACATAGCAATATTATTAATAGTACTTGCAACAGTTGCGATGA
Sequence number 2539: Rickettsia akari rOmpA #40 
CCCCGGCACCTACTTTTAACATAGCAATATTATTAATAGTACTTGCAACAGTTGCGATGATTGTACCA
TTAACTCTGTTACTGCTTGTAATACTACCGGTTATATTTTTATTTGCACCAA
Sequence number 2540: Rickettsia akari rOmpA #41 
TTGTACCATTAACTCTGTTACTGCTTGTAATACTACCGGTTATATTTTTATTTGCACCAAGTATTATA
ACTGCTGCTTGATTATTGAAATCTACACTATCGTTAATATTACCGTTTTGAG
Sequence number 2541: Rickettsia akari rOmpA #42 
GTATTATAACTGCTGCTTGATTATTGAAATCTACACTATCGTTAATATTACCGTTTTGAGTTAAAGCA
GTAGCAGCACCGAATGATAAATTAGAGTTGATATCCTTATTATAGCCGGCAC
Sequence number 2542: Rickettsia akari rOmpA #43 
TTAAAGCAGTAGCAGCACCGAATGATAAATTAGAGTTGATATCCTTATTATAGCCGGCACTGATAATT
CTAAATTGTTAGCAAATATATCAGTATCTATTTAGAATGTGCTGTTTCCTCT
Sequence number 2543: Rickettsia akari rOmpA #44 
TGATAATTCTAAATTGTTAGCAAATATATCAGTATCTATTTAGAATGTGCTGTTTCCTCTAAATTCTA
ATGCTTTTAATTTATTTACTACAGTACCGTAAGTAGCATTTCCGACATTTGT
Sequence number 2544: Rickettsia akari rOmpA #45 
AAATTCTAATGCTTTTAATTTATTTACTACAGTACCGTAAGTAGCATTTCCGACATTTGTAAGAATTA
CTTGCCCGCCCGCAGCTCCTGAATCTACAGCAAGTTTACCTTTATTTGGATC
Sequence number 2545: Rickettsia akari rOmpA #46 
AAGAATTACTTGCCCGCCCGCAGCTCCTGAATCTACAGCAAGTTTACCTTTATTTGGATCTCCAGGAT
CAAGTGTGGCATTTAATGTTATTGTACTATCACCGGCTGCACTATTTTGTAA
Sequence number 2546: Rickettsia akari rOmpA #47 
TCCAGGATCAAGTGTGGCATTTAATGTTATTGTACTATCACCGGCTGCACTATTTTGTAATACTAACT
CTGCATCTTCATGGACAAAATTGATAGTCTGACCGTTTGCGAAAATTCCTAC
Sequence number 2547: Rickettsia akari rOmpA #48 
TACTAACTCTGCATCTTCATGGACAAAATTGATAGTCTGACCGTTTGCGAAAATTCCTACATTAGCGC
CATTTGCATCAATCGTATAAGAACCAGCAGCGGCAGGAAGTCCAAGACTCGC
Sequence number 2548: Rickettsia akari rOmpA #49 
ATTAGCGCCATTTGCATCAATCGTATAAGAACCAGCAGCGGCAGGAAGTCCAAGACTCGCATTTGCTG
CACCTATATTTATTACATTAATTTGGTTAGTAATACCGCATACATCAACAAA
Sequence number 2549: Rickettsia akari rOmpA #50 
ATTTGCTGCACCTATATTTATTACATTAATTTGGTTAGTAATACCGCATACATCAACAAACTGCATAG
TATTTATGATATTAAATGCGAGAACATTTGTAGTATCTATATTATTAAAGGC
Sequence number 2550: Rickettsia akari rOmpA #51 
CTGCATAGTATTTATGATATTAAATGCGAGAACATTTGTAGTATCTATATTATTAAAGGCTACATTCC
CTAAAACATTTAATGATGCTAGTTCATTTCCTGCCGTACATATTGCTTTAGC
Sequence number 2551: Rickettsia akari rOmpA #52 
TACATTCCCTAAAACATTTAATGATGCTAGTTCATTTCCTGCCGTACATATTGCTTTAGCTGCACAAT
CATCTTGTAAGGTTATAAGGTTTTCTGTACCAAGAAGAAGTAACATACCACC
Sequence number 2552: Rickettsia akari rOmpA #53 
TGCACAATCATCTTGTAAGGTTATAAGGTTTTCTGTACCAAGAAGAAGTAACATACCACCACCGGTTG
TAAACGCCGGTAAATCATTTTTAATACAACTCTATCATCTGTAGCACTATTG
Sequence number 2553: Rickettsia akari rOmpA #54 
ACCGGTTGTAAACGCCGGTAAATCATTTTTAATACAACTCTATCATCTGTAGCACTATTGTTAACTAA
AAAAAGCTTTGAGTTTGCACCTTTAAAATCAATAACTTGAGCGTTTAATATC
Sequence number 2554: Rickettsia akari rOmpA #55 
TTAACTAAAAAAAGCTTTGAGTTTGCACCTTTAAAATCAATAACTTGAGCGTTTAATATCTCAATATC
AGCGTTCTTAACATTTATTGCAAAAGTCTTATTATCTTGAATGTTAATTTGT
Sequence number 2555: Rickettsia akari rOmpA #56 
TCAATATCAGCGTTCTTAACATTTATTGCAAAAGTCTTATTATCTTGAATGTTAATTTGTGCAACCGT
CCCGATACTAGCATTATCTGCAGTAACCAAACCGAGAGTATTAATATTAATT
Sequence number 2556: Rickettsia akari rOmpA #57 
GCAACCGTCCCGATACTAGCATTATCTGCAGTAACCAAACCGAGAGTATTAATATTAATTGTAGCATT
ATTACTGTTTGCTATAGTTCTGCTTAATGTATAACTCTTACCGACAGGACCG
Sequence number 2557: Rickettsia akari rOmpA #58 
GTAGCATTATTACTGTTTGCTATAGTTCTGCTTAATGTATAACTCTTACCGACAGGACCGTTCAATTC
TAAAGTACCCCCCTGAGCATTCACACCGAAATTGATATTTGTAGCTATCAAA
Sequence number 2558: Rickettsia akari rOmpA #59 
TTCAATTCTAAAGTACCCCCCTGAGCATTCACACCGAAATTGATATTTGTAGCTATCAAATTACCGCC
GACTTGTATCGTACCGACTGCTCCACCACCGGCAATATCATCAAAATTTACG
Sequence number 2559: Rickettsia akari rOmpA #60 
TTACCGCCGACTTGTATCGTACCGACTGCTCCACCACCGGCAATATCATCAAAATTTACGATTTGTGC
ATAAATATCACTTGTAAAACAAGATCCGCAGCACCATTATCAACTTTAGCAT
Sequence number 2560: Rickettsia akari rOmpA #61 ATTTGTGCATAAATATCACTTGTAAAACAAGATCCGCAGCACCATTATCAACTTTAGCATTCAGAACT TTTAGAGATGCTGCATTTGTACCTACCGAGCCGGTAACCCATACTCCAGCGT
Sequence number 2561: Rickettsia akari rOmpA #62 
TCAGAACTTTTAGAGATGCTGCATTTGTACCTACCGAGCCGGTAACCCATACTCCAGCGTTGTTGCAC
TTATTGCTCCTACTGTACTTACTCCGCCTGCATTACCGTTATTAAACGTTAC
Sequence number 2562: Rickettsia akari rOmpA #63 
TGTTGCACTTATTGCTCCTACTGTACTTACTCCGCCTGCATTACCGTTATTAAACGTTACGGCATACA
AAGTTTTGGTCCAAGCGATTGCTCCGGTAAAACTTGTTCGAGTATTAACAGT
Sequence number 2563: Rickettsia akari rOmpA #64 
GGCATACAAAGTTTTGGTCCAAGCGATTGCTCCGGTAAAACTTGTTCGAGTATTAACAGTTATTTCAC
TATGATCATGATCATCACTATCTATAGCTACTGCAATCTTTATTAATGCTAG
Sequence number 2564: Rickettsia akari rOmpA #65 
TATTTCACTATGATCATGATCATCACTATCTATAGCTACTGCAATCTTTATTAATGCTAGAGTTGCAG
ATTGTATAGTTAAGCCTGCAGTGGCACCGCCTATGGTAATAGCTCCTCAACC
Sequence number 2565: Rickettsia akari rOmpA #66 
AGTTGCAGATTGTATAGTTAAGCCTGCAGTGGCACCGCCTATGGTAATAGCTCCTCAACCTATATAAT
CAGCAGCAGCATCAAAACCATGATTTGCAGCAACTGCTTCAGTACCGGTTAA
Sequence number 2566: Rickettsia akari rOmpA #67 
TATATAATCAGCAGCAGCATCAAAACCATGATTTGCAGCAACTGCTTCAGTACCGGTTAAAATTAAGC
TTTTGCCGTCAGTGATAGTAACAGGCAACAAGTTATCACATGTAACAATAGA
Sequence number 2567: Rickettsia akari rOmpA #68 
AATTAAGCTTTTGCCGTCAGTGATAGTAACAGGCAACAAGTTATCACATGTAACAATAGAACTGACGC
TAGTATTTTGAGTAATATTTAGAGCTACTTGAATAGTACCTGCAACATTTAT
Sequence number 2568: Rickettsia akari rOmpA #69 
ACTGACGCTAGTATTTTGAGTAATATTTAGAGCTACTTGAATAGTACCTGCAACATTTATAGCAGTAA
TAATACGATCTGCTTCATCTGCAGTGATAGTATGATCACCACCGTAAGTAAA
Sequence number 2569: Rickettsia akari rOmpA #70 
AGCAGTAATAATACGATCTGCTTCATCTGCAGTGATAGTATGATCACCACCGTAAGTAAATGCATCAT
CGTCTTGAGGACCGTCAGAAGGATGACCATTAGCGGCTCCTCCAGCCGTTAT
Sequence number 2570: Rickettsia akari rOmpA #71 
TGCATCATCGTCTTGAGGACCGTCAGAAGGATGACCATTAGCGGCTCCTCCAGCCGTTATATCATTCA
AATTATTGGCAACAGCAAAATCACTAAATACTGCATCATTAACAGAAATAAA
Sequence number 2571: Rickettsia akari rOmpA #72 
ATCATTCAAATTATTGGCAACAGCAAAATCACTAAATACTGCATCATTAACAGAAATAAAACCAGAAG
CGACACCACATGCCACGCTACCACTCAGCATTATCGCTGCGGTTGAGGTAGT
Sequence number 2572: Rickettsia akari rOmpA #73 
ACCAGAAGCGACACCACATGCCACGCTACCACTCAGCATTATCGCTGCGGTTGAGGTAGTGAATAAAG
CTGTTTTAAGACTTTTTTGAATTGCTTTTTGAAATAATTTTGGAGAAATATT
Sequence number 2573: Rickettsia akari rOmpA #74 
GAATAAAGCTGTTTTAAGACTTTTTTGAATTGCTTTTTGAAATAATTTTGGAGAAATATTCACCATAA
TGTAAACTTTAATAAAAATTAAATAATTTAATAATTAAATATAAGTGTTTAA
Sequence number 2574: Rickettsia akari rOmpA #75 
CACCATAATGTAAACTTTAATAAAAATTAAATAATTTAATAATTAAATATAAGTGTTTAAATATGTCT
ATTATAATTAATAATTATATACAATATTTTACAAATTTATATGCGGCATTTG
Sequence number 2575: Rickettsia akari rOmpA #76 
ATATGTCTATTATAATTAATAATTATATACAATATTTTACAAATTTATATGCGGCATTTGCATTCTGC
AGGGGCGGTAGCTTTGTTGCATAGATCGGTAAAACCTACTCAATGTCATTCC
Sequence number 2576: Rickettsia akari rOmpA #77 
CATTCTGCAGGGGCGGTAGCTTTGTTGCATAGATCGGTAAAACCTACTCAATGTCATTCCCGCGTAAG
CAGAACCTTGTTGCATGGTTCGAGAAATCTACTGAGTAGTAGACCGTGCTCA
Sequence number 2577: Rickettsia akari rOmpA #78 
CGCGTAAGCAGAACCTTGTTGCATGGTTCGAGAAATCTACTGAGTAGTAGACCGTGCTCAAACCACGG
GGTGACAGCTAAAAGTACTGGATCCACACTTTCACTGGGATAACATTAAGAA
Sequence number 2578: Rickettsia akari rOmpA #79 
AACCACGGGGTGACAGCTAAAAGTACTGGATCCACACTTTCACTGGGATAACATTAAGAATACGAGAA
TAACATTTACCTCTCAGGCAGTAATATCTCCCTCTTACCTGTATGATTCGGT
Sequence number 2579: Rickettsia akari rOmpA #80 
TACGAGAATAACATTTACCTCTCAGGCAGTAATATCTCCCTCTTACCTGTATGATTCGGTGGTGAGAC
TATTCCATCTTTCTCCATCTTCTCAACTAAGTTGGCAGCTTTGTTATAGCCG
Sequence number 2580: Rickettsia akari rOmpA #81 
GGTGAGACTATTCCATCTTTCTCCATCTTCTCAACTAAGTTGGCAGCTTTGTTATAGCCGATTCTAAG
CGACCTTTGGATATAGCTAATGGAAGATTTACGTTCGTCACGCACTATCTGA
Sequence number 2581: Rickettsia akari rOmpA #82 
ATTCTAAGCGACCTTTGGATATAGCTAATGGAAGATTTACGTTCGTCACGCACTATCTGAACAGCTTT
TTTGTAAAGCACTGCATCAGACGTGCCATCAACGATATCAATTCTACTATCG
Sequence number 2582: Rickettsia akari rOmpA #83 
ACAGCTTTTTTGTAAAGCACTGCATCAGACGTGCCATCAACGATATCAATTCTACTATCGTCTTCTTC
AGGCTGCTCGGTAACGGCAGAGATATATTCAGGCGCACCGGTCTCTTTCAAA
Sequence number 2583: Rickettsia akari rOmpA #84 
TCTTCTTCAGGCTGCTCGGTAACGGCAGAGATATATTCAGGCGCACCGGTCTCTTTCAAATATCCTGT
AATTTTCTCGATTTCAACTTCATTAACAAACGGTCCATGTACCCTACTTATC
Sequence number 2584: Rickettsia akari rOmpA #85 
TATCCTGTAATTTTCTCGATTTCAACTTCATTAACAAACGGTCCATGTACCCTACTTATCTTTGCAGT
ATTCCCCATAAATAGCATATCACCCATACCTAGTAACTGCTCGGAGCCTTGC
Sequence number 2585: Rickettsia akari rOmpA #86 
TTTGCAGTATTCCCCATAAATAGCATATCACCCATACCTAGTAACTGCTCGGAGCCTTGCTCGCCTAA
AATCGTTCGGCTATCGATTTTAGATGTAACTTTAAAGCTAATACGACTTGGG
Sequence number 2586: Rickettsia akari rOmpA #87 
TCGCCTAAAATCGTTCGGCTATCGATTTTAGATGTAACTTTAAAGCTAATACGACTTGGGAAATTGGC
TTTAATGACACCGGTAATAACGTCTACAGAAGGTCTTTGCGTTGCCATGATA
Sequence number 2587: Rickettsia akari rOmpA #88 
AAATTGGCTTTAATGACACCGGTAATAACGTCTACAGAAGGTCTTTGCGTTGCCATGATAATATGGAT
ACCTGCCGCTCTTGCCATTTGAGCAAGCCTTTGAATCAGCATTTCAATATCC
Sequence number 2588: Rickettsia akari rOmpA #89 ATATGGATACCTGCCGCTCTTGCCATTTGAGCAAGCCTTTGAATCAGCATTTCAATATCCTTACCGGC AACTAGCATTAAATCAGCCATCTCATCAACAATAACCACAATGTAAGGTAGT
Sequence number 2589: Rickettsia akari rOmpA #90 
TTACCGGCAACTAGCATTAAATCAGCCATCTCATCAACAATAACCACAATGTAAGGTAGTTTCTCCAT
ATTCATCGTAACGGTTTCGTAAATAGGCTTACCTGTTTCAGGATCAAAACCC
Sequence number 2590: Rickettsia akari rOmpA #91 
TTCTCCATATTCATCGTAACGGTTTCGTAAATAGGCTTACCTGTTTCAGGATCAAAACCCGTTTGAAT
CGAACGCTCAATTACTCTATTCTCTTTCACCGCTTCTAAGATTTTTGCATTG
Sequence number 2591: Rickettsia akari rOmpA #92 
GTTTGAATCGAACGCTCAATTACTCTATTCTCTTTCACCGCTTCTAAGATTTTTGCATTGTAACCTGC
AATATTCTTAACACCGATATTGCTCATAATTCTATAGCGGTTTTCCATTTCC
Sequence number 2592: Rickettsia akari rOmpA #93 
TAACCTGCAATATTCTTAACACCGATATTGCTCATAATTCTATAGCGGTTTTCCATTTCCTTAACCGC
CCATTTAAGAGCAACTACAGCCTTAGAAGGTTCAGTTACTACAGGAGTGAGT
Sequence number 2593: Rickettsia akari rOmpA #94 
TTAACCGCCCATTTAAGAGCAACTACAGCCTTAGAAGGTTCAGTTACTACAGGAGTGAGTAAATGGGG
TATTCCGTCATAAGCGGATAATTCAAGCATTTTCGGGTCAATCATGATAAAG
Sequence number 2594: Rickettsia akari rOmpA #95 
AAATGGGGTATTCCGTCATAAGCGGATAATTCAAGCATTTTCGGGTCAATCATGATAAAGCGGCATTC
TTCAGGAGTATAGCGGTATAAGAGCGAAACTATCATCGCGTTAATCCCAACT
Sequence number 2595: Rickettsia akari rOmpA #96 
CGGCATTCTTCAGGAGTATAGCGGTATAAGAGCGAAACTATCATCGCGTTAATCCCAACTGATTTACC
AGAACCGGTTGTTCCTGCAACAAGCAAGTGAGGCATCTTCGCAAGATCGGCA
Sequence number 2596: Rickettsia akari rOmpA #97 
GATTTACCAGAACCGGTTGTTCCTGCAACAAGCAAGTGAGGCATCTTCGCAAGATCGGCAATCAGTGG
CTTACCGACTAAATCTTTACCTAAGACTAACGGTAATAAAGTTGATTTGTCT
Sequence number 2597: Rickettsia akari rOmpA #98 
ATCAGTGGCTTACCGACTAAATCTTTACCTAAGACTAACGGTAATAAAGTTGATTTGTCTTGGTATTC
AGGCGTCTCTATCAACTCTTTCACACAAAAGAATTCACGCTGCTTATTAGGG
Sequence number 2598: Rickettsia akari rOmpA #99 
TGGTATTCAGGCGTCTCTATCAACTCTTTCACACAAAAGAATTCACGCTGCTTATTAGGGAGCTCAAT
ACCAAGTACGTTTTTGCCGGGTATTACCGCTATCCTTGTTGATAAAGCAGAT
Sequence number 2599: Rickettsia akari rOmpA #100 
AGCTCAATACCAAGTACGTTTTTGCCGGGTATTACCGCTATCCTTGTTGATAAAGCAGATAGTGAGCG
TGCTATATCGTCAGATAATCCTACTACTCTTGATGTTTTAGTACCAGCTGCC
Sequence number 2600: Rickettsia akari rOmpA #101 
AGTGAGCGTGCTATATCGTCAGATAATCCTACTACTCTTGATGTTTTAGTACCAGCTGCCGGCTCAAA
CTCATATTGAGTTACCACCGGTCCTTGATTGATATTAATTATATGCCCTTTA
Sequence number 2601: Rickettsia akari rOmpA #102 
GATGTTTTAGTACCAGCTGCCGGCTCAAACTCATATTGAGTTACCACCGGTCCTTGATTGATATTAAT
TATATGCCCTTTAACTCCAAAATCATTCAGCACGGTTAGTAGCTCCTCGGCT
Sequence number 2602: Rickettsia conorii rOmpA #1 
GAAGCAATCTCAGGATATTTGACGAGATTGCCACGTTGCTTCGCTCCTCGCAATGACGCTTCTCAAAA
CTACATTATAAAGCAAAAAAGCCTATATAGTCAAACTATATAGGCTTTTTTT
Sequence number 2603: Rickettsia conorii rOmpA #2 
TCTCAAAACTACATTATAAAGCAAAAAAGCCTATATAGTCAAACTATATAGGCTTTTTTTATGATTAT
AGAACGGTTAATTAAAAATTAACACGAACTTTCACACTACCTTGCTGAGCGA
Sequence number 2604: Rickettsia conorii rOmpA #3 
ATGATTATAGAACGGTTAATTAAAAATTAACACGAACTTTCACACTACCTTGCTGAGCGAAATACTTA
CTTCCGATATTGGTATCGTAGTTAATCCCGTATTCCATCATTTTATGCTTAG
Sequence number 2605: Rickettsia conorii rOmpA #4 
AATACTTACTTCCGATATTGGTATCGTAGTTAATCCCGTATTCCATCATTTTATGCTTAGCAGTAACA
CCGACACCAACATCAAAACTTGTTTTGCTTTGCTTAAAGCTGTTGGTTGGAA
Sequence number 2606: Rickettsia conorii rOmpA #5 
CAGTAACACCGACACCAACATCAAAACTTGTTTTGCTTTGCTTAAAGCTGTTGGTTGGAAGAGGAGCA
GTCATACCTTGTAATCTTGCATCAATCGCCGAAACTTTATTCTTGAATGCAT
Sequence number 2607: Rickettsia conorii rOmpA #6 
GAGGAGCAGTCATACCTTGTAATCTTGCATCAATCGCCGAAACTTTATTCTTGAATGCATAATCGACC
ATTGCGTAAAGCTCAGGTGTTAGCACTATTTCATTGACATTGATATTACTTG
Sequence number 2608: Rickettsia conorii rOmpA #7 
AATCGACCATTGCGTAAAGCTCAGGTGTTAGCACTATTTCATTGACATTGATATTACTTGATACTTTA
GCACCGAGTAAACCGTCGAAAGTATTATAGTTCTTGCCTTTAACGGTAAGAT
Sequence number 2609: Rickettsia conorii rOmpA #8 
ATACTTTAGCACCGAGTAAACCGTCGAAAGTATTATAGTTCTTGCCTTTAACGGTAAGATTTTGGTAA
GTAGTACCGGTTTCCTTATAGCTCTTATCTTTGATAGTCGAATATCTAAGCC
Sequence number 2610: Rickettsia conorii rOmpA #9 
TTTGGTAAGTAGTACCGGTTTCCTTATAGCTCTTATCTTTGATAGTCGAATATCTAAGCCCAGCTAAC
GGTGTTAAGTTAATGTTCTCAGACATCATATAGGTATAACCAGCCATTAACT
Sequence number 2611: Rickettsia conorii rOmpA #10 
CAGCTAACGGTGTTAAGTTAATGTTCTCAGACATCATATAGGTATAACCAGCCATTAACTGACCTGTA
TAGCTTTCGGATTTATACTTACCGTTTGCAGTTTGATAACCGACAGTCTCTA
Sequence number 2612: Rickettsia conorii rOmpA #11 
GACCTGTATAGCTTTCGGATTTATACTTACCGTTTGCAGTTTGATAACCGACAGTCTCTAGTGTTGTT
GCAATAACACGTCTTGATTTGCTTCTTATCTTATTATCTGAGTAAGATGCTA
Sequence number 2613: Rickettsia conorii rOmpA #12 
GTGTTGTTGCAATAACACGTCTTGATTTGCTTCTTATCTTATTATCTGAGTAAGATGCTATAGCTTCA
ACGAAGAGATTTTCATAAGGTACACTATATAAACCGTATAAAGAATAGATGT
Sequence number 2614: Rickettsia conorii rOmpA #13 
TAGCTTCAACGAAGAGATTTTCATAAGGTACACTATATAAACCGTATAAAGAATAGATGTTGCTCTCT
ACCTTATTCTTATCGCCCGTTTTATTATTTTTTAGCTTAATGTCAGTATCGG
Sequence number 2615: Rickettsia conorii rOmpA #14 
TGCTCTCTACCTTATTCTTATCGCCCGTTTTATTATTTTTTAGCTTAATGTCAGTATCGGCTCTTGTA
TATGCAAGTCCGAGTACTAGATCATCGCTAACGAAGCCGTCAAAACCTATAG
Sequence number 2616: Rickettsia conorii rOmpA #15 CTCTTGTATATGCAAGTCCGAGTACTAGATCATCGCTAACGAAGCCGTCAAAACCTATAGTGCCACCA GTTGTATCAGACTTATAACCACTTATACTATTACACATCTTCTGCGTTGCAT
Sequence number 2617: Rickettsia conorii rOmpA #16 
TGCCACCAGTTGTATCAGACTTATAACCACTTATACTATTACACATCTTCTGCGTTGCATTACCGACA
AACGGGCTTATCCACGCACCAAACTTAGCATCCATATCTTCATCACCAGAAG
Sequence number 2618: Rickettsia conorii rOmpA #17 
TACCGACAAACGGGCTTATCCACGCACCAAACTTAGCATCCATATCTTCATCACCAGAAGAAACAGGA
CCTTTATTCCCTGCTTGTACTTTATCCATTCTAGCATTTAGAGCAGTTATAT
Sequence number 2619: Rickettsia conorii rOmpA #18 
AAACAGGACCTTTATTCCCTGCTTGTACTTTATCCATTCTAGCATTTAGAGCAGTTATATTACTTGAT
ATATTACTCGCTACAACTTGATTATTAACGGCAGCTATAGTGTCGCTAGGTT
Sequence number 2620: Rickettsia conorii rOmpA #19 
TACTTGATATATTACTCGCTACAACTTGATTATTAACGGCAGCTATAGTGTCGCTAGGTTTTACAACA
TCTTGCATGAGATGAGTTGTAGCATCTGCTTCCTGTAGCGGTGTCATTAGAC
Sequence number 2621: Rickettsia conorii rOmpA #20 
TTACAACATCTTGCATGAGATGAGTTGTAGCATCTGCTTCCTGTAGCGGTGTCATTAGACCAAAGTTA
TTGAAAGCTTGACGTGCATCTGAACCATTGGGAGCATCCTCCATTAACTCAA
Sequence number 2622: Rickettsia conorii rOmpA #21 
CAAAGTTATTGAAAGCTTGACGTGCATCTGAACCATTGGGAGCATCCTCCATTAACTCAAGCGATTTC
TTTATATTTGCAGCATTTGGGATATTTGCAAGCGGTCCACCCGGTGCAAAAT
Sequence number 2623: Rickettsia conorii rOmpA #22 
GCGATTTCTTTATATTTGCAGCATTTGGGATATTTGCAAGCGGTCCACCCGGTGCAAAATCTTCATCT
ATAACACCTGCAGCTATATCCTCTGCAAATAACGTTAAAGTCGATGCATCAA
Sequence number 2624: Rickettsia conorii rOmpA #23 
CTTCATCTATAACACCTGCAGCTATATCCTCTGCAAATAACGTTAAAGTCGATGCATCAAAAGTAAAG
TCAACAAAACGGTTGTCATTATTAATAGTTATTTTAACATTCTCTTTAGGAG
Sequence number 2625: Rickettsia conorii rOmpA #24 
AAGTAAAGTCAACAAAACGGTTGTCATTATTAATAGTTATTTTAACATTCTCTTTAGGAGTAGGCTTT
AAACCACCTGCTGTTTCTGCAGATATTACCGTATATTTTGTATCTGGGCTTA
Sequence number 2626: Rickettsia conorii rOmpA #25 
TAGGCTTTAAACCACCTGCTGTTTCTGCAGATATTACCGTATATTTTGTATCTGGGCTTATATTATTC
ATGTCGAAATTAGTAGCAGTAACAACAAGTGCTAAATTTGAAACCCCTGATA
Sequence number 2627: Rickettsia conorii rOmpA #26 
TATTATTCATGTCGAAATTAGTAGCAGTAACAACAAGTGCTAAATTTGAAACCCCTGATAAATCAAGA
GTACTACCTGATTTAATTAAGATATTACCACCTGACTTAGCTGCTCCGTCAA
Sequence number 2628: Rickettsia conorii rOmpA #27 
AATCAAGAGTACTACCTGATTTAATTAAGATATTACCACCTGACTTAGCTGCTCCGTCAAAAGTAGTA
TTTAAGGTTAGCGTATCGGTAAAGCTACCGGTACCAGTATATGTTACTTGAT
Sequence number 2629: Rickettsia conorii rOmpA #28 
AAGTAGTATTTAAGGTTAGCGTATCGGTAAAGCTACCGGTACCAGTATATGTTACTTGATTTGCACCT
AAAGTAAGTGTAGTACCGCTACCTGTTATATTACTATTAAAGGCTAGGCTAT
Sequence number 2630: Rickettsia conorii rOmpA #29 
TTGCACCTAAAGTAAGTGTAGTACCGCTACCTGTTATATTACTATTAAAGGCTAGGCTATTGCCGAAA
TTAATAGTAGCACTGTTAGCTACAAAGCTGGTAGCCGTTACATTTTTAGCAA
Sequence number 2631: Rickettsia conorii rOmpA #30 
TGCCGAAATTAATAGTAGCACTGTTAGCTACAAAGCTGGTAGCCGTTACATTTTTAGCAAAACTAGTG
ATAGAACCTTTGGCTAAAGTAACCGCACCTGTATTAGTGAATGTATGGGCAA
Sequence number 2632: Rickettsia conorii rOmpA #31 
AACTAGTGATAGAACCTTTGGCTAAAGTAACCGCACCTGTATTAGTGAATGTATGGGCAAAACTTGTA
GTACCGCCAAGTGTCGCCGTACCTTTTGCGTTAACACTGCTTGCAAAACTTG
Sequence number 2633: Rickettsia conorii rOmpA #32 
AACTTGTAGTACCGCCAAGTGTCGCCGTACCTTTTGCGTTAACACTGCTTGCAAAACTTGTAGCACCT
GCCGTTGTGATATCACCAACCGAATTAGCCGCAGTCCCTACAACACCGCTAA
Sequence number 2634: Rickettsia conorii rOmpA #33 
TAGCACCTGCCGTTGTGATATCACCAACCGAATTAGCCGCAGTCCCTACAACACCGCTAACACTACCG
CCATTCATGAAGTTTAGCTTCAGAGCCTGACCACCGGTTTTATTTATGCTGC
Sequence number 2635: Rickettsia conorii rOmpA #34 
CACTACCGCCATTCATGAAGTTTAGCTTCAGAGCCTGACCACCGGTTTTATTTATGCTGCCTGTTAAC
TTAAAGTGTGCAGGTAATGTTAAAGTATTTGTACCTGTACCTTGGATTTCGC
Sequence number 2636: Rickettsia conorii rOmpA #35 
CTGTTAACTTAAAGTGTGCAGGTAATGTTAAAGTATTTGTACCTGTACCTTGGATTTCGCCAATTTTA
ACGTTACCGCCTTTGGCAATAGTTACATTTCCTGCACCTACTTTTAACATAG
Sequence number 2637: Rickettsia conorii rOmpA #36 
CAATTTTAACGTTACCGCCTTTGGCAATAGTTACATTTCCTGCACCTACTTTTAACATAGCAATCCCG
TTTACTCTATTAAGATTACTTGCACCTAAAACTATTAACGTACCGTTATTAG
Sequence number 2638: Rickettsia conorii rOmpA #37 
CAATCCCGTTTACTCTATTAAGATTACTTGCACCTAAAACTATTAACGTACCGTTATTAGTACCACCG
GTATTTTTGACTGTTCCCGCAACATTTACATTGTTATTTAATGTTACCGTAC
Sequence number 2639: Rickettsia conorii rOmpA #38 
TACCACCGGTATTTTTGACTGTTCCCGCAACATTTACATTGTTATTTAATGTTACCGTACCGTTTTTT
GCATTAAAATCTAAAAAACCTCCAATATTACCGGTTTGAGTTAATTGAACAG
Sequence number 2640: Rickettsia conorii rOmpA #39 
CGTTTTTTGCATTAAAATCTAAAAAACCTCCAATATTACCGGTTTGAGTTAATTGAACAGCATCCTTA
AATAAAACTACATTTGCCGTAGTAGCTCCAAGTTCTAATTGACCTGTAATAT
Sequence number 2641: Rickettsia conorii rOmpA #40 
CATCCTTAAATAAAACTACATTTGCCGTAGTAGCTCCAAGTTCTAATTGACCTGTAATATCAAGCACT
ATATTTGTTGTATTAAAAGTGGTACCTGCCGTGCCAAAATCTCCCTCTCCTT
Sequence number 2642: Rickettsia conorii rOmpA #41 
CAAGCACTATATTTGTTGTATTAAAAGTGGTACCTGCCGTGCCAAAATCTCCCTCTCCTTTGAAAACT
ATATCTTGTAACTTATGAGCTCCACCAAACGTCTTGCCTCCGGCTATTGTTA
Sequence number 2643: Rickettsia conorii rOmpA #42 
TGAAAACTATATCTTGTAACTTATGAGCTCCACCAAACGTCTTGCCTCCGGCTATTGTTAATTTTTTT
CCTGCAGTTACAGAATTTAATATTACTATACCCTCATCGTCATTATCAGGGT
Sequence number 2644: Rickettsia conorii rOmpA #43 ATTTTTTTCCTGCAGTTACAGAATTTAATATTACTATACCCTCATCGTCATTATCAGGGTCTATATTC GCACCTAGTGTGATCGTACGGTCGTTTCCTGAACTATTTTGTAATATTAATT
Sequence number 2645: Rickettsia conorii rOmpA #44 
CTATATTCGCACCTAGTGTGATCGTACGGTCGTTTCCTGAACTATTTTGTAATATTAATTGAGCATCC
GCATGTGCAAATTTAATATTTCCTGCCGGTATATTTAAATTACCATTATTTG
Sequence number 2646: Rickettsia conorii rOmpA #45 
GAGCATCCGCATGTGCAAATTTAATATTTCCTGCCGGTATATTTAAATTACCATTATTTGCATCAATC
GTATAAGTAGCATCGTTTATTTGTATAGTACCAGCATTAAATGCTGTACTAC
Sequence number 2647: Rickettsia conorii rOmpA #46 
CATCAATCGTATAAGTAGCATCGTTTATTTGTATAGTACCAGCATTAAATGCTGTACTACTAGTAAAA
TCTGCATTATTGTTAATAAGCACGTTCTGTATACCTTCTAAATTTACATCGT
Sequence number 2648: Rickettsia conorii rOmpA #47 
TAGTAAAATCTGCATTATTGTTAATAAGCACGTTCTGTATACCTTCTAAATTTACATCGTCAGTTATT
GTAACGGCATTATAAATAAGTAAAGTGTTAAATTTATTACCGCCTACATCAC
Sequence number 2649: Rickettsia conorii rOmpA #48 
CAGTTATTGTAACGGCATTATAAATAAGTAAAGTGTTAAATTTATTACCGCCTACATCACCGATATTT
CTAGCGGCACCTGCTACATTACTCCCAATATTCAGACCATTAACCCCACCAT
Sequence number 2650: Rickettsia conorii rOmpA #49 
CGATATTTCTAGCGGCACCTGCTACATTACTCCCAATATTCAGACCATTAACCCCACCATCAAAGACT
ACCGTACCTTCATCAACACCGGGAGCTACTAAATCAGCTGCTAATAATATAT
Sequence number 2651: Rickettsia conorii rOmpA #50 
CAAAGACTACCGTACCTTCATCAACACCGGGAGCTACTAAATCAGCTGCTAATAATATATTATTTACT
CCGACTCCGGTTAAGTTAGAAAGTACTAAAGCAGAATCTAGAGCTCTAAAAT
Sequence number 2652: Rickettsia conorii rOmpA #51 
TATTTACTCCGACTCCGGTTAAGTTAGAAAGTACTAAAGCAGAATCTAGAGCTCTAAAATGAATATCT
TGAGCATTTAATATAGTAACATCACCAGCACTTGCATCAATTGCAAAAAGAT
Sequence number 2653: Rickettsia conorii rOmpA #52 
GAATATCTTGAGCATTTAATATAGTAACATCACCAGCACTTGCATCAATTGCAAAAAGATTACCGGCT
CCAATATTGATTTCTGCAACTGTTCCTATAGTTAAATGATATGCCGTAAGTA
Sequence number 2654: Rickettsia conorii rOmpA #53 
TACCGGCTCCAATATTGATTTCTGCAACTGTTCCTATAGTTAAATGATATGCCGTAAGTAACTTTGTA
TTAACATTTAATATAGCATTATTGCCGTTTGCTATAGCTCCTTTAAAATAAT
Sequence number 2655: Rickettsia conorii rOmpA #54 
ACTTTGTATTAACATTTAATATAGCATTATTGCCGTTTGCTATAGCTCCTTTAAAATAATAAGGGATT
GCGTTACCACCACCATCAAGAGGTCCGTTAAACTCTAAAGTACTCCTAGCTC
Sequence number 2656: Rickettsia conorii rOmpA #55 
AAGGGATTGCGTTACCACCACCATCAAGAGGTCCGTTAAACTCTAAAGTACTCCTAGCTCCAAAATCT
ATATTATTCGCATCTAGACTTCCTCCAGCTTGTAATGTTACACCTGCTCCTA
Sequence number 2657: Rickettsia conorii rOmpA #56 
CAAAATCTATATTATTCGCATCTAGACTTCCTCCAGCTTGTAATGTTACACCTGCTCCTACATTCACT
GTTGCTAATGCATTTGTATTACCTATATTGCCAGTTACTGTACTATCACCGG
Sequence number 2658: Rickettsia conorii rOmpA #57 
CATTCACTGTTGCTAATGCATTTGTATTACCTATATTGCCAGTTACTGTACTATCACCGGTAAACGTT
ACTATACCATTATTTGCATTACCGGTATTATCTATCGCTCCGGTCACCACTA
Sequence number 2659: Rickettsia conorii rOmpA #58 
TAAACGTTACTATACCATTATTTGCATTACCGGTATTATCTATCGCTCCGGTCACCACTACAGGATTC
GTAAATGTCACTGCTGACGCATTATCTGTTAACTTAGTCGTAGTAGCTTTAA
Sequence number 2660: Rickettsia conorii rOmpA #59 
CAGGATTCGTAAATGTCACTGCTGACGCATTATCTGTTAACTTAGTCGTAGTAGCTTTAATAATCGCT
CCCCCTAACGTGGCTTTACCTGCTCCTACACTTATCGTGGCTAATGCATTTG
Sequence number 2661: Rickettsia conorii rOmpA #60 
TAATCGCTCCCCCTAACGTGGCTTTACCTGCTCCTACACTTATCGTGGCTAATGCATTTGTATTACCT
ATATTGCCAGTTACTGTACTATCACCGGTAAACGTTACTATACCATTATTTG
Sequence number 2662: Rickettsia conorii rOmpA #61 
TATTACCTATATTGCCAGTTACTGTACTATCACCGGTAAACGTTACTATACCATTATTTGCATTACCG
GTATTATCTATCGCTCCGGTCACCACTACAGGATTCGTAAATGTCACTGCTG
Sequence number 2663: Rickettsia conorii rOmpA #62 
CATTACCGGTATTATCTATCGCTCCGGTCACCACTACAGGATTCGTAAATGTCACTGCTGACGCATTA
TCCGTTAAGTTTATTGTATTCGATTTTACCACTCCGCCTTGTACTCGTAGCA
Sequence number 2664: Rickettsia conorii rOmpA #63 
ACGCATTATCCGTTAAGTTTATTGTATTCGATTTTACCACTCCGCCTTGTACTCGTAGCAAACCTGCT
CCTACATTCACTGTTGCTAATGCATTTGTATTACCTATATTGCCAGTTACTG
Sequence number 2665: Rickettsia conorii rOmpA #64 
AACCTGCTCCTACATTCACTGTTGCTAATGCATTTGTATTACCTATATTGCCAGTTACTGTACTATCA
CCGGTAAACGTCGCTATACCATTATTTGCATTACCGGTATTATCTATCGCTC
Sequence number 2666: Rickettsia conorii rOmpA #65 
TACTATCACCGGTAAACGTCGCTATACCATTATTTGCATTACCGGTATTATCTATCGCTCCGGTCACC
ACTACAGGATTCGTAAATGTCACTGCTGACGCATTATCTGTTAACTTAGTCG
Sequence number 2667: Rickettsia conorii rOmpA #66 
CGGTCACCACTACAGGATTCGTAAATGTCACTGCTGACGCATTATCTGTTAACTTAGTCGTAGTAGCT
TTAATAACCGCTCCCCCTAACGTGGCTTTACCTGCTCCTACACTTATCGTGG
Sequence number 2668: Rickettsia conorii rOmpA #67 
TAGTAGCTTTAATAACCGCTCCCCCTAACGTGGCTTTACCTGCTCCTACACTTATCGTGGCTAATGCA
TTTGTATTACCTATATTCCCGGTTACTTGACTTAATGCACCATTTAAATTTA
Sequence number 2669: Rickettsia conorii rOmpA #68 
CTAATGCATTTGTATTACCTATATTCCCGGTTACTTGACTTAATGCACCATTTAAATTTAAGACACCT
ACATTATCAACGCCTGTGGTGTTATCAATCGCACCTGTTAATACTGCATTTA
Sequence number 2670: Rickettsia conorii rOmpA #69 
AGACACCTACATTATCAACGCCTGTGGTGTTATCAATCGCACCTGTTAATACTGCATTTACATTTGTA
AGGGTTAATACCGACGCAGCATTCGTTAATTTAGTCGTAGTAGCTTTAATAA
Sequence number 2671: Rickettsia conorii rOmpA #70 
CATTTGTAAGGGTTAATACCGACGCAGCATTCGTTAATTTAGTCGTAGTAGCTTTAATAACCGCTCCC
TCTAACGTGGCTATACCTGCTCCTACATTCACTGTTGCTAATGCATTTGTAT
Sequence number 2672: Rickettsia conorii rOmpA #71 CCGCTCCCTCTAACGTGGCTATACCTGCTCCTACATTCACTGTTGCTAATGCATTTGTATTACCTATA TTGCCAGTTACTGTACTATTACCGGTAAACGTTACTATACCATTATTTGCAT
Sequence number 2673: Rickettsia conorii rOmpA #72 
TACCTATATTGCCAGTTACTGTACTATTACCGGTAAACGTTACTATACCATTATTTGCATTACCGGTA
TTATCTATCGCTCCGGTCACCACTACAGGATTCGTAAATGTCACTGCTGACG
Sequence number 2674: Rickettsia conorii rOmpA #73 
TACCGGTATTATCTATCGCTCCGGTCACCACTACAGGATTCGTAAATGTCACTGCTGACGCATTATCT
GTTAACTTAGTCGTAGTAGCTTTAATAATCGCTCCCCCTAACGTGGCTTTAC
Sequence number 2675: Rickettsia conorii rOmpA #74 
CATTATCTGTTAACTTAGTCGTAGTAGCTTTAATAATCGCTCCCCCTAACGTGGCTTTACCTGCTCCT
ACACTTATCGTGGCTAATGCATTTGTATTACCTATATTGCCAGTTACTGTAC
Sequence number 2676: Rickettsia conorii rOmpA #75 
CTGCTCCTACACTTATCGTGGCTAATGCATTTGTATTACCTATATTGCCAGTTACTGTACTATCACCG
GTAAACGTTACTATACCATTATTTGCATTACCGGTATTATCTATCGCTCCGG
Sequence number 2677: Rickettsia conorii rOmpA #76 
TATCACCGGTAAACGTTACTATACCATTATTTGCATTACCGGTATTATCTATCGCTCCGGTCACCACT
ACAGGATTCGTAAATGTCACTGCTGACGCATTATCCGTTAACTTAGTCGTAG
Sequence number 2678: Rickettsia conorii rOmpA #77 
TCACCACTACAGGATTCGTAAATGTCACTGCTGACGCATTATCCGTTAACTTAGTCGTAGTAGCTTTA
ATAATCGCTCCCCCTAACGTGGCTTTACCTGCTCCTACACTTATCGTGGCTA
Sequence number 2679: Rickettsia conorii rOmpA #78 
TAGCTTTAATAATCGCTCCCCCTAACGTGGCTTTACCTGCTCCTACACTTATCGTGGCTAATGCATTT
GTATTACCTATATTGCCAGTTACTGTACTATCACCGGTAAACGTTACTATAC
Sequence number 2680: Rickettsia conorii rOmpA #79 
ATGCATTTGTATTACCTATATTGCCAGTTACTGTACTATCACCGGTAAACGTTACTATACCATTATTT
GCATTACCGGTATTATCTATCGCTCCGGTCACCACTACAGGATTCGTAAATG
Sequence number 2681: Rickettsia conorii rOmpA #80 
CATTATTTGCATTACCGGTATTATCTATCGCTCCGGTCACCACTACAGGATTCGTAAATGTCACTGCT
GACGCATTATCCGTTAACTTAGTCGTAGTAGCTTTAATAATCGCTCCCCCTA
Sequence number 2682: Rickettsia conorii rOmpA #81 
TCACTGCTGACGCATTATCCGTTAACTTAGTCGTAGTAGCTTTAATAATCGCTCCCCCTAACGTGGCT
TTACCTGCTCCTACACTTATCGTGGCTAATGCATTTGTATTACCTATATTGC
Sequence number 2683: Rickettsia conorii rOmpA #82 
ACGTGGCTTTACCTGCTCCTACACTTATCGTGGCTAATGCATTTGTATTACCTATATTGCCAGTTACT
GTACTATCACCGGTAAACGTTACTATACCATTATTTGCATTACCGGTATTAT
Sequence number 2684: Rickettsia conorii rOmpA #83 
CAGTTACTGTACTATCACCGGTAAACGTTACTATACCATTATTTGCATTACCGGTATTATCTATCGCT
CCGGTCACCACTACAGGATTCGTAAATGTCACTGCTGACGCATTATCCGTTA
Sequence number 2685: Rickettsia conorii rOmpA #84 
CTATCGCTCCGGTCACCACTACAGGATTCGTAAATGTCACTGCTGACGCATTATCCGTTAACTTAGTC
GTAGTAGCTTTAATAATCGCTCCCCCTAACGTGGCTTTACCTGCTCCTACAC
Sequence number 2686: Rickettsia conorii rOmpA #85 
ACTTAGTCGTAGTAGCTTTAATAATCGCTCCCCCTAACGTGGCTTTACCTGCTCCTACACTTATCGTG
GCTAATGCATTTGTATTACCTATATTGCCAGTTACTGTACTATCACCGGTAA
Sequence number 2687: Rickettsia conorii rOmpA #86 
TTATCGTGGCTAATGCATTTGTATTACCTATATTGCCAGTTACTGTACTATCACCGGTAAACGTTACT
ATACCATTATTTGCATTACCGGTATTATCTATCGCTCCGGTCACCACTACAG
Sequence number 2688: Rickettsia conorii rOmpA #87 
ACGTTACTATACCATTATTTGCATTACCGGTATTATCTATCGCTCCGGTCACCACTACAGGATTCGTA
AATGTCACTGCTGACGCATTATCTGTTAACTTAGTCGTAGTAGCTTTAATAA
Sequence number 2689: Rickettsia conorii rOmpA #88 
GATTCGTAAATGTCACTGCTGACGCATTATCTGTTAACTTAGTCGTAGTAGCTTTAATAACCGCTCCC
CCTAACGTGGCTTTACCTGCTCCTACACTTATCGTGGCTAATGCATTTGTAT
Sequence number 2690: Rickettsia conorii rOmpA #89 
CCGCTCCCCCTAACGTGGCTTTACCTGCTCCTACACTTATCGTGGCTAATGCATTTGTATTACCTATA
TTCCCGGTTACTTGACTTAATGCACCATTTAAATTTAAGACACCTACATTAT
Sequence number 2691: Rickettsia conorii rOmpA #90 
TACCTATATTCCCGGTTACTTGACTTAATGCACCATTTAAATTTAAGACACCTACATTATCAACGCCT
GTGGTGTTATCAATCGCACCTGTTAATACTGCATTTACATTTGTAAGGGTTA
Sequence number 2692: Rickettsia conorii rOmpA #91 
CAACGCCTGTGGTGTTATCAATCGCACCTGTTAATACTGCATTTACATTTGTAAGGGTTAATACCGAC
GCAGCATTCGTTAATTTAGTCGTAGTAGCTTTAATAATCGCTCCCTCTAACG
Sequence number 2693: Rickettsia conorii rOmpA #92 
ATACCGACGCAGCATTCGTTAATTTAGTCGTAGTAGCTTTAATAATCGCTCCCTCTAACGTGGCTATA
CCTGCTCCTACATTCACTGTTGCTAACGCATTTGTATTACCTATTGTTCCGT
Sequence number 2694: Rickettsia conorii rOmpA #93 
TGGCTATACCTGCTCCTACATTCACTGTTGCTAACGCATTTGTATTACCTATTGTTCCGTTAATGGCA
GCATCTGTCTTGACAGTTATTATACCTCCTCCATTTATATTGCCTGCAAGTG
Sequence number 2695: Rickettsia conorii rOmpA #94 
TAATGGCAGCATCTGTCTTGACAGTTATTATACCTCCTCCATTTATATTGCCTGCAAGTGTTATCTTT
GCCGGGGCTGCAGATTGTATAATTAGTGCAGCATTCGCTCCCCCTAAAGCTA
Sequence number 2696: Rickettsia conorii rOmpA #95 
TTATCTTTGCCGGGGCTGCAGATTGTATAATTAGTGCAGCATTCGCTCCCCCTAAAGCTATATTTCCT
AAACCTGTATAATTATCGGCAGGAGCACCAAAACCATGATTTGCAGCATCAG
Sequence number 2697: Rickettsia conorii rOmpA #96 
TATTTCCTAAACCTGTATAATTATCGGCAGGAGCACCAAAACCATGATTTGCAGCATCAGCATTATTA
CCGTTTAAAGTTAAGCTTTTGCCGGCAGTAATAGTAACAGGCAACAAGTTAC
Sequence number 2698: Rickettsia conorii rOmpA #97 
CATTATTACCGTTTAAAGTTAAGCTTTTGCCGGCAGTAATAGTAACAGGCAACAAGTTACCTCCCGTT
ACAATCGAACCAACGACGGTATTTTGAGCAATATTTAGACCTATGGGAGTAG
Sequence number 2699: Rickettsia conorii rOmpA #98 
CTCCCGTTACAATCGAACCAACGACGGTATTTTGAGCAATATTTAGACCTATGGGAGTAGTATCCGCA
ACATTTATAGCCGTAATAATATGATCGGCTACATCTGCAGTGATAGTATAAT
Sequence number 2700: Rickettsia conorii rOmpA #99 TATCCGCAACATTTATAGCCGTAATAATATGATCGGCTACATCTGCAGTGATAGTATAATCACCACCG TAAGTAAATGCCCAATTGTTTTGAGGACCGCGAGCAGGAGTACCATTAGCTA
Sequence number 2701: Rickettsia conorii rOmpA #100 
CACCACCGTAAGTAAATGCCCAATTGTTTTGAGGACCGCGAGCAGGAGTACCATTAGCTACCCCTGCA
GCCGTTATCTCATTCCAATTATTGCCAACATTATCACTAAATGCTGCATTAT
Sequence number 2702: Rickettsia conorii rOmpA #101 
CCCCTGCAGCCGTTATCTCATTCCAATTATTGCCAACATTATCACTAAATGCTGCATTATTATTAGTA
GCAATAACACCTGAAACAGCAATACCGAGTGCCCCGCTACTACTCAGCATTA
Sequence number 2703: Rickettsia conorii rOmpA #102 
TATTAGTAGCAATAACACCTGAAACAGCAATACCGAGTGCCCCGCTACTACTCAGCATTATCGCTGCG
GTTGAGGTGGTGAATAAAGCGGCTTTAAGACCTTGTTGTATTGCTTTTTGAA
Sequence number 2704: Rickettsia conorii rOmpA #103 
TCGCTGCGGTTGAGGTGGTGAATAAAGCGGCTTTAAGACCTTGTTGTATTGCTTTTTGAAATAATTTT
GGAGAAATATTCGCCATATACGTAAACCTTAATAAAAATTAACTCACTTAAA
Sequence number 2705: Rickettsia conorii rOmpA #104 
ATAATTTTGGAGAAATATTCGCCATATACGTAAACCTTAATAAAAATTAACTCACTTAAAAAGTGTTA
TAACGCAACTTTTTTAGTATGTCTATTATAATTAATAACATAGATGATATTT
Sequence number 2706: Rickettsia conorii rOmpA #105 
ATTAACTCACTTAAAAAGTGTTATAACGCAACTTTTTTAGTATGTCTATTATAATTAATAACATAGAT
GATATTTTTATGTATAGATTGTCATTCCGTAGCGACGTTGGCCTTGTTGCAT
Sequence number 2707: Rickettsia japonica rOmpA #1 
TTTTTATGATTATAGAACGGTTAATTAAAAATTAACACGAACTTTTACACTACCTTGCTGAGCGAAAT
ACTTACTTCCGATATTGGTATCGTAGTTAATCCCATATTCCATCATTTTATG
Sequence number 2708: Rickettsia japonica rOmpA #2 
AGCGAAATACTTACTTCCGATATTGGTATCGTAGTTAATCCCATATTCCATCATTTTATGCTTAGCAG
TAACACCGACACCGACATCAAAACTTGTTTTGCTTTGCTTAAAGCTGTTGGT
Sequence number 2709: Rickettsia japonica rOmpA #3 
CTTAGCAGTAACACCGACACCGACATCAAAACTTGTTTTGCTTTGCTTAAAGCTGTTGGTTGGGAGAG
GAGCAGTCATACCTTGTAATCTTGCATCAATCGCCGAAACTTTATTCTTGAA
Sequence number 2710: Rickettsia japonica rOmpA #4 
TGGGAGAGGAGCAGTCATACCTTGTAATCTTGCATCAATCGCCGAAACTTTATTCTTGAATGCATAAT
CGACCATTGCGTAAAGCTCAGGTGTTAGCACTATTTCATTGACATTGATATT
Sequence number 2711: Rickettsia japonica rOmpA #5 
TGCATAATCGACCATTGCGTAAAGCTCAGGTGTTAGCACTATTTCATTGACATTGATATTACTTGATA
CTTTAGCACCGAGTAAACCGTCGAAAGTATTATAGTGCTTGCCTTTAACGGT
Sequence number 2712: Rickettsia japonica rOmpA #6 
ACTTGATACTTTAGCACCGAGTAAACCGTCGAAAGTATTATAGTGCTTGCCTTTAACGGTAAGATTTT
GGTAAGTAGTACCGGTTTCCTTATAGCCCTTATCTTTGATAGTCGAATATCT
Sequence number 2713: Rickettsia japonica rOmpA #7 
AAGATTTTGGTAAGTAGTACCGGTTTCCTTATAGCCCTTATCTTTGATAGTCGAATATCTAAGCCCAG
CTAGCGGTGTTAAGTTAATGTTCTCAGGCATCATATAGGTATAACCAGCCAT
Sequence number 2714: Rickettsia japonica rOmpA #8 
AAGCCCAGCTAGCGGTGTTAAGTTAATGTTCTCAGGCATCATATAGGTATAACCAGCCATTAACTGAC
CTGTATAGCTTTCGGATTTATAATTACCGTTTGCAGTTTGATAACCGACAGT
Sequence number 2715: Rickettsia japonica rOmpA #9 
TAACTGACCTGTATAGCTTTCGGATTTATAATTACCGTTTGCAGTTTGATAACCGACAGTCTCTAGTG
CCGTTGCAATAACACGTCTTGATTTGCTTCTTATCTTATTATCTGAGTAAGA
Sequence number 2716: Rickettsia japonica rOmpA #10 
CTCTAGTGCCGTTGCAATAACACGTCTTGATTTGCTTCTTATCTTATTATCTGAGTAAGATGCTATAG
CTTCAACGAAGAGATTTTCATAAGGTACATTATATAAACCGTATAAAGAATA
Sequence number 2717: Rickettsia japonica rOmpA #11 
TGCTATAGCTTCAACGAAGAGATTTTCATAAGGTACATTATATAAACCGTATAAAGAATAGATGTTGC
TCTCTACCTTATTCTTATCGCCCGTTTTATTATTTTTTAGCTTAATGTCAGT
Sequence number 2718: Rickettsia japonica rOmpA #12 
GATGTTGCTCTCTACCTTATTCTTATCGCCCGTTTTATTATTTTTTAGCTTAATGTCAGTATCGGATC
TTGTATATGCAAGTCCGAGTGCTAGATCATCGCTAACGAAGCCGTCAAAACC
Sequence number 2719: Rickettsia japonica rOmpA #13 
ATCGGATCTTGTATATGCAAGTCCGAGTGCTAGATCATCGCTAACGAAGCCGTCAAAACCTATAGTGC
CACCAGTTGTATCAGACTTATAACCACTTATATTGTTACACATCTTCTGCTT
Sequence number 2720: Rickettsia japonica rOmpA #14 
TATAGTGCCACCAGTTGTATCAGACTTATAACCACTTATATTGTTACACATCTTCTGCTTTGCATTAC
CGACAAACGGGCTTATCCACGCACCAAACTTAGCATCCATATCTTCATCACC
Sequence number 2721: Rickettsia japonica rOmpA #15 
TGCATTACCGACAAACGGGCTTATCCACGCACCAAACTTAGCATCCATATCTTCATCACCGGAAGAAA
CAGGACCTTTATTCGCTACTTGTACTTTATCCATTCTAGCATTTAGAGCAGT
Sequence number 2722: Rickettsia japonica rOmpA #16 
GGAAGAAACAGGACCTTTATTCGCTACTTGTACTTTATCCATTCTAGCATTTAGAGCAGTTATATTAC
TTGATATATTACTTGCTACAACTTGATTATTAACGGCAGCTATAGTATCGCT
Sequence number 2723: Rickettsia japonica rOmpA #17 
TATATTACTTGATATATTACTTGCTACAACTTGATTATTAACGGCAGCTATAGTATCGCTAGGTTTTA
CAACATCTTGAATGAGATGAGTTGTAGCATCTGCTTCCTGTAGCGGTGTCAT
Sequence number 2724: Rickettsia japonica rOmpA #18 
AGGTTTTACAACATCTTGAATGAGATGAGTTGTAGCATCTGCTTCCTGTAGCGGTGTCATTAGACCAA
AGTTATTGAAAGCTTGACGTGCATCTGAACCATTGGGAGCATCCTCCATTAA
Sequence number 2725: Rickettsia japonica rOmpA #19 
TAGACCAAAGTTATTGAAAGCTTGACGTGCATCTGAACCATTGGGAGCATCCTCCATTAACTCAAGCG
ATTTCTTTATATTTGCAGCATTTGGGATACTTACAAGCGGTCCACCCGGTGC
Sequence number 2726: Rickettsia japonica rOmpA #20 
CTCAAGCGATTTCTTTATATTTGCAGCATTTGGGATACTTACAAGCGGTCCACCCGGTGCAAAATCTT
CATCTATAACATCTGCAGCTATATCCTCTGCAAATAACGTTAAAGTCGATGC
Sequence number 2727: Rickettsia japonica rOmpA #21 
AAAATCTTCATCTATAACATCTGCAGCTATATCCTCTGCAAATAACGTTAAAGTCGATGCATCAAAAG
TAAAATCAACAAAACGGTTGTCATTATTAATAGTTATTTTAACATTCTCTTT
Sequence number 2728: Rickettsia japonica rOmpA #22 ATCAAAAGTAAAATCAACAAAACGGTTGTCATTATTAATAGTTATTTTAACATTCTCTTTAGGAGTAG GCTTTAAATCACCTGCTGTTTCTGCAGATATTACCGTATATTTTGTATCTGG
Sequence number 2729: Rickettsia japonica rOmpA #23 
AGGAGTAGGCTTTAAATCACCTGCTGTTTCTGCAGATATTACCGTATATTTTGTATCTGGGCTTATAT
TATTCATGTCGAAATTAGTAGCAGTAACAACAAGTGCTAAAGTTGAAACCCC
Sequence number 2730: Rickettsia japonica rOmpA #24 
GCTTATATTATTCATGTCGAAATTAGTAGCAGTAACAACAAGTGCTAAAGTTGAAACCCCTGATAAAT
CAAGAGTACTACCTGATTTAATTAAGATATTACCACCTGACTTAGCTGCTCC
Sequence number 2731: Rickettsia japonica rOmpA #25 
TGATAAATCAAGAGTACTACCTGATTTAATTAAGATATTACCACCTGACTTAGCTGCTCCATCAAAAG
TAGTATTTAAGGTTAGCGTATCGGTAAAGCTACCGGTGCCAGTATATGTTAC
Sequence number 2732: Rickettsia japonica rOmpA #26 
ATCAAAAGTAGTATTTAAGGTTAGCGTATCGGTAAAGCTACCGGTGCCAGTATATGTTACGTGATTTG
CACCTAAAGTAATTGTAGTACCGCTACCTGTTATATTACTATTAAAGGCTAG
Sequence number 2733: Rickettsia japonica rOmpA #27 
GTGATTTGCACCTAAAGTAATTGTAGTACCGCTACCTGTTATATTACTATTAAAGGCTAGGCTATTGC
CGAAATTAATAGTAGCACTGTTAGCTACAAAGCTGGTAGCTGTTACATTTTT
Sequence number 2734: Rickettsia japonica rOmpA #28 
GCTATTGCCGAAATTAATAGTAGCACTGTTAGCTACAAAGCTGGTAGCTGTTACATTTTTAGCAAAAC
TAGTGATAGAACCTTTGGCTAAAGTAACCGCACCTGTATTAGTGAATGTATT
Sequence number 2735: Rickettsia japonica rOmpA #29 
AGCAAAACTAGTGATAGAACCTTTGGCTAAAGTAACCGCACCTGTATTAGTGAATGTATTGGCAAAAC
TTGTAGTACCGCCAAGTGTCGCTGTACCTTTTGCGTTAACACTGCTTGCAAA
Sequence number 2736: Rickettsia japonica rOmpA #30 
GGCAAAACTTGTAGTACCGCCAAGTGTCGCTGTACCTTTTGCGTTAACACTGCTTGCAAAACTTGTAG
CACCTGCCGTTGTGATATCACCAACCGAATTAGCCGCAGTCCCTACAACACC
Sequence number 2737: Rickettsia japonica rOmpA #31 
ACTTGTAGCACCTGCCGTTGTGATATCACCAACCGAATTAGCCGCAGTCCCTACAACACCTTTAACAC
TACCGCCATTCGTGAAGTTTAGCTTCAGAGCCTGACCACCGGTTTTATTTAT
Sequence number 2738: Rickettsia japonica rOmpA #32 
TTTAACACTACCGCCATTCGTGAAGTTTAGCTTCAGAGCCTGACCACCGGTTTTATTTATGCTGCCTG
TTAAGTTAAAGTTTGCAGGTAATGTTAAAGTATTTGTGCCTGTACCTTGGAT
Sequence number 2739: Rickettsia japonica rOmpA #33 
GCTGCCTGTTAAGTTAAAGTTTGCAGGTAATGTTAAAGTATTTGTGCCTGTACCTTGGATTTCGCCGA
TTTTAACGTTACCGCCTTTGGCAATAGTTACATTTCCTGCACCTACTTTTAA
Sequence number 2740: Rickettsia japonica rOmpA #34 
TTCGCCGATTTTAACGTTACCGCCTTTGGCAATAGTTACATTTCCTGCACCTACTTTTAACATAGCAA
TCCCGTTTACTCTATTAAGATTACTTGCACCTAAAACTATTAACGTACCGTT
Sequence number 2741: Rickettsia japonica rOmpA #35 
CATAGCAATCCCGTTTACTCTATTAAGATTACTTGCACCTAAAACTATTAACGTACCGTTATTAGTAC
CGCCGGTATTTTGGACTGCTCCCGCAACACTTACATTGTTATTTAATATTAC
Sequence number 2742: Rickettsia japonica rOmpA #36 
ATTAGTACCGCCGGTATTTTGGACTGCTCCCGCAACACTTACATTGTTATTTAATATTACCGTACCGT
TTTTTGCATTAAAATCTAAGAAACCGCCAATATGACCGGTTTGAGTTAATTG
Sequence number 2743: Rickettsia japonica rOmpA #37 
CGTACCGTTTTTTGCATTAAAATCTAAGAAACCGCCAATATGACCGGTTTGAGTTAATTGAACAGCAT
CATTAAATAAAACTACATTTGCCTTAGCAGCTCCAAGTTCTAATTTACCTGT
Sequence number 2744: Rickettsia japonica rOmpA #38 
AACAGCATCATTAAATAAAACTACATTTGCCTTAGCAGCTCCAAGTTCTAATTTACCTGTAATATCAA
GCTCTATATTTGTTGTATTAAAAGTGGTACCTGCCGCGCTGCAATCTCCCGC
Sequence number 2745: Rickettsia japonica rOmpA #39 
AATATCAAGCTCTATATTTGTTGTATTAAAAGTGGTACCTGCCGCGCTGCAATCTCCCGCTCCTTTGA
AAACTATAGTTTGTAACTTATGAGCTCCACCAAACGTCTTGCCTCCGGCTAT
Sequence number 2746: Rickettsia japonica rOmpA #40 
TCCTTTGAAAACTATAGTTTGTAACTTATGAGCTCCACCAAACGTCTTGCCTCCGGCTATCGTTAATT
TTTTTCCTGCAGTTACAGAATTTAATATTACTACACCCTCATTGTCATTATC
Sequence number 2747: Rickettsia japonica rOmpA #41 
CGTTAATTTTTTTCCTGCAGTTACAGAATTTAATATTACTACACCCTCATTGTCATTATCAGGGTCTA
TATTCGCACCTAGTGTGATCGTACGGTCGTTTCCTGAACTATTTTGTAATAT
Sequence number 2748: Rickettsia japonica rOmpA #42 
AGGGTCTATATTCGCACCTAGTGTGATCGTACGGTCGTTTCCTGAACTATTTTGTAATATTAATTGAG
CATCCGCATGTGCAAATTGAATATTTCCTGCCGGTATATTTAAATTACCATT
Sequence number 2749: Rickettsia japonica rOmpA #43 
TAATTGAGCATCCGCATGTGCAAATTGAATATTTCCTGCCGGTATATTTAAATTACCATTATTTGCAT
CAATCGTATAAGTAGCATCGTTTATTTGTATAATACCAGCATTAAATGCTGT
Sequence number 2750: Rickettsia japonica rOmpA #44 
ATTTGCATCAATCGTATAAGTAGCATCGTTTATTTGTATAATACCAGCATTAAATGCTGTACTACTAG
TAAAATCTGCATTATTTTCAATACGCACGTTCTGTATACCTTCTAAATTTAC
Sequence number 2751: Rickettsia japonica rOmpA #45 
ACTACTAGTAAAATCTGCATTATTTTCAATACGCACGTTCTGTATACCTTCTAAATTTACATCGTCAG
TTATTGTAACGGCATTATAAATAAGTAAAGTGTTAAATTTATTACCGCCTCC
Sequence number 2752: Rickettsia japonica rOmpA #46 
ATCGTCAGTTATTGTAACGGCATTATAAATAAGTAAAGTGTTAAATTTATTACCGCCTCCATCACCGA
TATTTCTAGCGGTACCTGCTACATTACTCCCAATATTCAGGCCATTAACCCC
Sequence number 2753: Rickettsia japonica rOmpA #47 
ATCACCGATATTTCTAGCGGTACCTGCTACATTACTCCCAATATTCAGGCCATTAACCCCACCATCAA
AGACTACATTACCTTCATCAGCACCGGGAGCTACTAAATCAGCTGCTAATAA
Sequence number 2754: Rickettsia japonica rOmpA #48 
ACCATCAAAGACTACATTACCTTCATCAGCACCGGGAGCTACTAAATCAGCTGCTAATAATATATTTT
TTGCTCCGACTCCGGTGAAGTTAGAAAGTACTAAAGCAGAATCTGGAACTCT
Sequence number 2755: Rickettsia japonica rOmpA #49 
TATATTTTTGCTCCGACTCCGGTGAAGTTAGAAAGTACTAAAGCAGAATCTGGAACTCTAAAATTAA
TATCTTGAGCATTTAATATAGTAACATCACCAGCACTTGCATCAATTGTAAA
Sequence number 2756: Rickettsia japonica rOmpA #50 AAAATTAATATCTTGAGCATTTAATATAGTAACATCACCAGCACTTGCATCAATTGTAAAAAGATTAC CGGCTCCAATATTAATTTCTGCAACTGTTCCTATAGTTGAATGATATGCCGT
Sequence number 2757: Rickettsia japonica rOmpA #51 
AAGATTACCGGCTCCAATATTAATTTCTGCAACTGTTCCTATAGTTGAATGATATGCCGTAAGTGACT
TTGTATTAACATTTAATATAGCGTTATTGCCGTTTGCTATAGCTCCTTTAAA
Sequence number 2758: Rickettsia japonica rOmpA #52 
AAGTGACTTTGTATTAACATTTAATATAGCGTTATTGCCGTTTGCTATAGCTCCTTTAAAATAATAAG
GGATTATGTTACCACCACCATCAAGAGGTCCGTTAAACTCTAAAATACTCCT
Sequence number 2759: Rickettsia japonica rOmpA #53 
ATAATAAGGGATTATGTTACCACCACCATCAAGAGGTCCGTTAAACTCTAAAATACTCCTGGCTCCAA
AATCTATATTATTCGCAGCTAGGCTTCCTCCAGCTTTTAATGTTACTCCTGC
Sequence number 2760: Rickettsia japonica rOmpA #54 
GGCTCCAAAATCTATATTATTCGCAGCTAGGCTTCCTCCAGCTTTTAATGTTACTCCTGCTCCTACAT
TCACTGTTGCTAATGCATTTGTATTACCTATATTGCCAGTTACTGTACTATT
Sequence number 2761: Rickettsia japonica rOmpA #55 
TCCTACATTCACTGTTGCTAATGCATTTGTATTACCTATATTGCCAGTTACTGTACTATTACCGGTAA
ACGTTGCTATACCATTATTTGCATTACCGGTATTATCTAGCGCCCCGGTCAC
Sequence number 2762: Rickettsia japonica rOmpA #56 
ACCGGTAAACGTTGCTATACCATTATTTGCATTACCGGTATTATCTAGCGCCCCGGTCACCACTACAG
GATTCGTAAATGTCACTGCTGACGCATTATCCGTTAAGTTTATTGCATTCGC
Sequence number 2763: Rickettsia japonica rOmpA #57 
CACTACAGGATTCGTAAATGTCACTGCTGACGCATTATCCGTTAAGTTTATTGCATTCGCTTTTACCA
CTCCGCCTTGTACTTGTAGCAAACCTGCTCCTACATTCACTGTTGCTAATGC
Sequence number 2764: Rickettsia japonica rOmpA #58 
TTTTACCACTCCGCCTTGTACTTGTAGCAAACCTGCTCCTACATTCACTGTTGCTAATGCATTTGTAT
TACCTATATTGCCAGTTACTGTACTATTACCGGTAAACGTTGCTATACCATT
Sequence number 2765: Rickettsia japonica rOmpA #59 
ATTTGTATTACCTATATTGCCAGTTACTGTACTATTACCGGTAAACGTTGCTATACCATTATTTGCAT
TACCGGTATTATCTAGCGCTCCGGTCACCACTACAGGATTCGTAAATGTCAC
Sequence number 2766: Rickettsia japonica rOmpA #60 
ATTTGCATTACCGGTATTATCTAGCGCTCCGGTCACCACTACAGGATTCGTAAATGTCACTGCTGACG
CATTATCCGTTAAGTTTATTGCATTCGCTTTTACCACTCCGCCTTGTACTTG
Sequence number 2767: Rickettsia japonica rOmpA #61 
TGCTGACGCATTATCCGTTAAGTTTATTGCATTCGCTTTTACCACTCCGCCTTGTACTTGTAGCAAAC
CTGCTCCTACATTCACTGTTGCTAATGCATTTGTATTACCTATATTGCCAGT
Sequence number 2768: Rickettsia japonica rOmpA #62 
TAGCAAACCTGCTCCTACATTCACTGTTGCTAATGCATTTGTATTACCTATATTGCCAGTTACTGTAC
TATTACCGGTAAACGTTGCTATACCATTATTTGCATTACCGGTATTATCTAG
Sequence number 2769: Rickettsia japonica rOmpA #63 
TACTGTACTATTACCGGTAAACGTTGCTATACCATTATTTGCATTACCGGTATTATCTAGCGCCCCGG
TCACCACTACAGGATTCGTAAATGTCACTGCTGACGCAGCATTTGTTAATTT
Sequence number 2770: Rickettsia japonica rOmpA #64 
CGCCCCGGTCACCACTACAGGATTCGTAAATGTCACTGCTGACGCAGCATTTGTTAATTTAGTCGTAG
TAGCTTTAATAACCGCCCCCCCTAACGTGGCTGTACCTGCTCCTACATTTAT
Sequence number 2771: Rickettsia japonica rOmpA #65 
AGTCGTAGTAGCTTTAATAACCGCCCCCCCTAACGTGGCTGTACCTGCTCCTACATTTATCGTGGCTA
ATGCATTTGTATTACCTATATTCCCGCTTACTTGACTTAATGCACCATTTAA
Sequence number 2772: Rickettsia japonica rOmpA #66 
CGTGGCTAATGCATTTGTATTACCTATATTCCCGCTTACTTGACTTAATGCACCATTTAAATTTAAGA
CACCTACATTATCACCGCCTGTGGTGTTATCAATCTCACCTGTTAATACTGC
Sequence number 2773: Rickettsia japonica rOmpA #67 
ATTTAAGACACCTACATTATCACCGCCTGTGGTGTTATCAATCTCACCTGTTAATACTGCATTTGCAT
TTGTAAGGGTTAATACCGACGCAGCATTTGTTAATTTAGTCGTAGTAGCTTT
Sequence number 2774: Rickettsia japonica rOmpA #68 
ATTTGCATTTGTAAGGGTTAATACCGACGCAGCATTTGTTAATTTAGTCGTAGTAGCTTTAATAACCG
CTCCCTCTAACGTGGCTGTACCTGCTCCTACATTTATCGTGGCTAATGCATT
Sequence number 2775: Rickettsia japonica rOmpA #69 
AATAACCGCTCCCTCTAACGTGGCTGTACCTGCTCCTACATTTATCGTGGCTAATGCATTTGTATTAC
CTATATTCCCGCTTACTTGACTTAATGCACCATTTAAATTTAAGACACCTAC
Sequence number 2776: Rickettsia japonica rOmpA #70 
TGTATTACCTATATTCCCGCTTACTTGACTTAATGCACCATTTAAATTTAAGACACCTACATTATCAC
CGCCTGTGGTGTTATCAATCTCACCTGTTAATACTGCATTTGCATTTGTAAG
Sequence number 2777: Rickettsia japonica rOmpA #71 
ATTATCACCGCCTGTGGTGTTATCAATCTCACCTGTTAATACTGCATTTGCATTTGTAAGGGTTAATA
CCGACGCAGCATTTGTTAATTTAGTCGTAGTAGCTTTAATAACCGCTCCCTC
Sequence number 2778: Rickettsia japonica rOmpA #72 
GGTTAATACCGACGCAGCATTTGTTAATTTAGTCGTAGTAGCTTTAATAACCGCTCCCTCTAACGTGG
CTGTACCTGCTCCTACATTTATCGTGGCTAATGCATTTGTATTACCTATATT
Sequence number 2779: Rickettsia japonica rOmpA #73 
TAACGTGGCTGTACCTGCTCCTACATTTATCGTGGCTAATGCATTTGTATTACCTATATTCCCGCTTA
CTTGACTTAATGCACCATTTAAATTTAAGACACCTACATTATCACCGCCTGT
Sequence number 2780: Rickettsia japonica rOmpA #74 
CCCGCTTACTTGACTTAATGCACCATTTAAATTTAAGACACCTACATTATCACCGCCTGTGGTGTTAT
CAATCTCACCTGTTAATACTGCATTTGCATTTGTAAGGGTTAATACCGACGC
Sequence number 2781: Rickettsia japonica rOmpA #75 
GGTGTTATCAATCTCACCTGTTAATACTGCATTTGCATTTGTAAGGGTTAATACCGACGCAGCATTTG
TTAATTTAGTCGTAGTAGCTTTAATAACCGCTCCCTCTAACGTGGCTGTACC
Sequence number 2782: Rickettsia japonica rOmpA #76 
AGCATTTGTTAATTTAGTCGTAGTAGCTTTAATAACCGCTCCCTCTAACGTGGCTGTACCTGCTCCTA
CATTTATCGTGGCTAATGCATTTGTATTACCTATATTGCCAGTTACTGTACT
Sequence number 2783: Rickettsia japonica rOmpA #77 
TGCTCCTACATTTATCGTGGCTAATGCATTTGTATTACCTATATTGCCAGTTACTGTACTATTACCGG
TAAACGTTGCTATACCATTATTTGCATTACCGGTATTATCTAGCGCTCCGGT
Sequence number 2784: Rickettsia japonica rOmpA #78 ATTACCGGTAAACGTTGCTATACCATTATTTGCATTACCGGTATTATCTAGCGCTCCGGTTACCACTA CAGGATTCGTAAATGTCACTGCTGACGCATTATCCGTTAAGTTTATTGCATT
Sequence number 2785: Rickettsia japonica rOmpA #79 
TACCACTACAGGATTCGTAAATGTCACTGCTGACGCATTATCCGTTAAGTTTATTGCATTCGCTTTTA
CCACTCCGCCTTGTACTTGTAGCAAACCTGCTCCTACATTCACTGTTGCTAA
Sequence number 2786: Rickettsia japonica rOmpA #80 
CGCTTTTACCACTCCGCCTTGTACTTGTAGCAAACCTGCTCCTACATTCACTGTTGCTAATGCATTTG
TATTACCTATATTGCCAGTTACTGTACTATTACCGGTAAACGTTGCTATACC
Sequence number 2787: Rickettsia japonica rOmpA #81 
TGCATTTGTATTACCTATATTGCCAGTTACTGTACTATTACCGGTAAACGTTGCTATACCATTATTTG
CATTACCGGTATTATCTAGCGCTCCGGTCACCACTACAGGATTCGTAAATGT
Sequence number 2788: Rickettsia japonica rOmpA #82 
ATTATTTGCATTACCGGTATTATCTAGCGCTCCGGTCACCACTACAGGATTCGTAAATGTCACTGCTG
ACGCATTATCCGTTAAGTTTATTGCATTCGCTTTTACCACTCCGCCTTGTAC
Sequence number 2789: Rickettsia japonica rOmpA #83 
CACTGCTGACGCATTATCCGTTAAGTTTATTGCATTCGCTTTTACCACTCCGCCTTGTACTTGTAGCA
AACCTGCTCCTACATTCACTGTTGCTAATGCATTTGTATTACCTATATTGCC
Sequence number 2790: Rickettsia japonica rOmpA #84 
TTGTAGCAAACCTGCTCCTACATTCACTGTTGCTAATGCATTTGTATTACCTATATTGCCAGTTACTG
TACTATTACCGGTAAACGTTGCTATACCATTATTTGCATTACCGGTATTATC
Sequence number 2791: Rickettsia japonica rOmpA #85 
AGTTACTGTACTATTACCGGTAAACGTTGCTATACCATTATTTGCATTACCGGTATTATCTAGCGCCC
CGGTCACCACTACAGGATTCGTAAATGTCACTGCTGACGCATTATCCGTTAA
Sequence number 2792: Rickettsia japonica rOmpA #86 
TAGCGCCCCGGTCACCACTACAGGATTCGTAAATGTCACTGCTGACGCATTATCCGTTAAGTTTATTG
CATTCGCTTTTACCACTCCGCCTTGTACTTGTAGCAAACCTGCTCCTACATT
Sequence number 2793: Rickettsia japonica rOmpA #87 
GTTTATTGCATTCGCTTTTACCACTCCGCCTTGTACTTGTAGCAAACCTGCTCCTACATTCACTGTTG
CTAATGCATTTGTATTACCTATATTGCCAGTTACTGTACTATTACCGGTAAA
Sequence number 2794: Rickettsia japonica rOmpA #88 
CACTGTTGCTAATGCATTTGTATTACCTATATTGCCAGTTACTGTACTATTACCGGTAAACGTTGCTA
TACCATTATTTGCATTACCGGTATTATCTAGCGCTCCGGTCACCACTACAGG
Sequence number 2795: Rickettsia japonica rOmpA #89 
CGTTGCTATACCATTATTTGCATTACCGGTATTATCTAGCGCTCCGGTCACCACTACAGGATTCGTAA
ATGTCACTGCTGACGCATTATCCGTTAAGTTTATTGCATTCGCTTTTACCAC
Sequence number 2796: Rickettsia japonica rOmpA #90 
ATTCGTAAATGTCACTGCTGACGCATTATCCGTTAAGTTTATTGCATTCGCTTTTACCACTCCGCCTT
GTACTTGTAGCAAACCTGCTCCTACATTCACTGTTGCTAATGCATTTGTATT
Sequence number 2797: Rickettsia japonica rOmpA #91 
TCCGCCTTGTACTTGTAGCAAACCTGCTCCTACATTCACTGTTGCTAATGCATTTGTATTACCTATAT
TGCCAGTTACTGTACTATTACCGGTAAACGTTGCTATACCATTATTTGCATT
Sequence number 2798: Rickettsia japonica rOmpA #92 
ACCTATATTGCCAGTTACTGTACTATTACCGGTAAACGTTGCTATACCATTATTTGCATTACCGGTAT
TATCTAGCGCCCCGGTCACCACTACAGGATTCGTAAATGTCACTGCTGACGC
Sequence number 2799: Rickettsia japonica rOmpA #93 
ACCGGTATTATCTAGCGCCCCGGTCACCACTACAGGATTCGTAAATGTCACTGCTGACGCATTATCCG
TTAAGTTTATTGCATTCGCTTTTACCACTCCGCCTTGTACTTGTAGCAAACC
Sequence number 2800: Rickettsia japonica rOmpA #94 
ATTATCCGTTAAGTTTATTGCATTCGCTTTTACCACTCCGCCTTGTACTTGTAGCAAACCTGCTCCTA
CATTCACTGTTGCTAATGCATTTGTATTACCTATATTCCCGCTTACTTGACT
Sequence number 2801: Rickettsia japonica rOmpA #95 
TGCTCCTACATTCACTGTTGCTAATGCATTTGTATTACCTATATTCCCGCTTACTTGACTTAATGCAC
CATTTAAATTTAAGACACCTACATTATCACCGCCTGTGGTGTTATCAATCTC
Sequence number 2802: Rickettsia japonica rOmpA #96 
TAATGCACCATTTAAATTTAAGACACCTACATTATCACCGCCTGTGGTGTTATCAATCTCACCTGTTA
ATACTGCATTTGCATTTGTAAGGGTTAATACCGACGCAGCATTTGTTAATTT
Sequence number 2803: Rickettsia japonica rOmpA #97 
ACCTGTTAATACTGCATTTGCATTTGTAAGGGTTAATACCGACGCAGCATTTGTTAATTTAGTCGTAG
TAGCTTTAATAACCGCTCCCTCTAACGTGGCTTTACCTGCTCCTACATTTAT
Sequence number 2804: Rickettsia japonica rOmpA #98 
AGTCGTAGTAGCTTTAATAACCGCTCCCTCTAACGTGGCTTTACCTGCTCCTACATTTATCGTGGCTA
ATGTATTTGTATTACCTATATTCCCGCTTACTTGACTTAATGCACCATTTAA
Sequence number 2805: Rickettsia japonica rOmpA #99 
CGTGGCTAATGTATTTGTATTACCTATATTCCCGCTTACTTGACTTAATGCACCATTTAAATTTAAGA
CACCTACATTATCACCGCCTGTGGTGTTATCAATCTCACCTGTTAATACTGC
Sequence number 2806: Rickettsia japonica rOmpA #100 
ATTTGCATTTGTAAGGGTTAATACCGACGCAGCATTTGTTAATTTAGTCGTAGTAGCTTTAATAACCG
CTCCCTCTAACGTGGCTTTACCTGCTCCTACATTTATCGTGGCTAATGTATT
Sequence number 2807: Rickettsia japonica rOmpA #101 
AATAACCGCTCCCTCTAACGTGGCTTTACCTGCTCCTACATTTATCGTGGCTAATGTATTTGTATTAC
CTATATTCCCGCTTACTTGACTTAATGCACCATTTAAATTTAAGACACCTAC
Sequence number 2808: Rickettsia japonica rOmpA #102 
ATTATCACCGCCTGTGGTGTTATCAATCTCACCTGTTAATACTGCATTTGCATTTGTAAGGGTTAATA
CCGACGCAGCATTTGTTAATTTAGTCGTAGTAGCTTTAATAACCGCTCCCCC
Sequence number 2809: Rickettsia japonica rOmpA #103 
GGTTAATACCGACGCAGCATTTGTTAATTTAGTCGTAGTAGCTTTAATAACCGCTCCCCCTAACGTGG
CTTTACCTGCTCCTACATTTATCGTGGCTAATGCATTTGTATTACCTATTGT
Sequence number 2810: Rickettsia japonica rOmpA #104 
TAACGTGGCTTTACCTGCTCCTACATTTATCGTGGCTAATGCATTTGTATTACCTATTGTTCCGTTAA
TGGCAGCATCTGTCTTGACAGTTATTATACCTCCTCCATCTATATTTCCTGC
Sequence number 2811: Rickettsia japonica rOmpA #105 
TCCGTTAATGGCAGCATCTGTCTTGACAGTTATTATACCTCCTCCATCTATATTTCCTGCAAGTGTTA
TATTTGCCGGGGTTGCAGATTGTATAATTAGTTCAGCATTCACTCCCCCTAA
Sequence number 2812: Rickettsia japonica rOmpA #106 AAGTGTTATATTTGCCGGGGTTGCAGATTGTATAATTAGTTCAGCATTCACTCCCCCTAAAGTTATAT TTCCTAAACCTGTATAATTATCGGCAGGAGCATTAAAACCATGATTTGCAGC
Sequence number 2813: Rickettsia japonica rOmpA #107 
AGTTATATTTCCTAAACCTGTATAATTATCGGCAGGAGCATTAAAACCATGATTTGCAGCAACAGCAT
TATTACCATTTAAAGTTAAGCTTTTGCCGGCAGTAATAGTAACAGGCAACAA
Sequence number 2814: Rickettsia japonica rOmpA #108 
AACAGCATTATTACCATTTAAAGTTAAGCTTTTGCCGGCAGTAATAGTAACAGGCAACAAGTTACCTC
CCGTTATAATCGAACCAACGACGGTATTTTGAGCAATATTTAGACCTACGGG
Sequence number 2815: Rickettsia japonica rOmpA #109 
GTTACCTCCCGTTATAATCGAACCAACGACGGTATTTTGAGCAATATTTAGACCTACGGGATTAGTAT
TCGCAACATTTATAGCCTTAATAATACAATTGACTACATCTGCAGTGATAGT
Sequence number 2816: Rickettsia japonica rOmpA #110 
ATTAGTATTCGCAACATTTATAGCCTTAATAATACAATTGACTACATCTGCAGTGATAGTATAATCAC
CACCGTAAGTAAATGCCCAATTGTTTTGAGGACCACCAGCAGGATTACCATC
Sequence number 2817: Rickettsia japonica rOmpA #111 
ATAATCACCACCGTAAGTAAATGCCCAATTGTTTTGAGGACCACCAGCAGGATTACCATCAGCTTCCC
CTCTAGCCGTTATCTGATTCCAATTATTGGCAGCAGCATCATTACTAAATGC
Sequence number 2818: Rickettsia japonica rOmpA #112 
AGCTTCCCCTCTAGCCGTTATCTGATTCCAATTATTGGCAGCAGCATCATTACTAAATGCTGCATCAT
TATTAGTAGCAATAACACCTGCAGCAACACCGAGTGCCCCACTACTACTCAG
Sequence number 2819: Rickettsia japonica rOmpA #113 
TGCATCATTATTAGTAGCAATAACACCTGCAGCAACACCGAGTGCCCCACTACTACTCAGCATTATCG
CTGCGGTTGAGGTGGTGAATAAAGCGGCTTTAAGACCTTGTTGTATTGCTTT
Sequence number 2820: Rickettsia japonica rOmpA #114 
CATTATCGCTGCGGTTGAGGTGGTGAATAAAGCGGCTTTAAGACCTTGTTGTATTGCTTTTTGAAATA
ATTTTGGAGAAATATTTGCCATATGTAAACCTTAATAAAAATTAAATCACTT
Sequence number 2821: Rickettsia prowazekii gltA #1 
TAATCGGTTATGATCTCTTGCATATCTCTTGGTAGTTCACAAGAAAATGCCATAATATCATTACTAGT
TGGATGTGTAAAACTTAAATACCAAGAATGAAGAGCTTGGCGTTTAAAAGCT
Sequence number 2822: Rickettsia prowazekii gltA #2 
TTACTAGTTGGATGTGTAAAACTTAAATACCAAGAATGAAGAGCTTGGCGTTTAAAAGCTATTAATTT
AGCTTTTAGTTTAGGTGGTGAATTAATAATTTTACGGTTGTTATTACCATAA
Sequence number 2823: Rickettsia prowazekii gltA #3 
ATTAATTTAGCTTTTAGTTTAGGTGGTGAATTAATAATTTTACGGTTGTTATTACCATAAGTTTGGTC
ACCAACTACTGAATGCTTTAAATTACTTAACTGTACTCTGATTTGGTGCGTT
Sequence number 2824: Rickettsia prowazekii gltA #4 
GTTTGGTCACCAACTACTGAATGCTTTAAATTACTTAACTGTACTCTGATTTGGTGCGTTCTCCCGGT
ACTAAGCTTGCATTCCACCATACTAAGAGTACCGCCGTAAAATAGCTCTAAA
Sequence number 2825: Rickettsia prowazekii gltA #5 
CTCCCGGTACTAAGCTTGCATTCCACCATACTAAGAGTACCGCCGTAAAATAGCTCTAAAGTCTTGTA
ATACGTAACAGCTTTTTTGCCGCCGTATTTTAATATTGTCATTTTTTTTCGA
Sequence number 2826: Rickettsia prowazekii gltA #6 
GTCTTGTAATACGTAACAGCTTTTTTGCCGCCGTATTTTAATATTGTCATTTTTTTTCGATTATTTCT
ATTACGACCTACATTATTCTTAATTATTCCTTCTAGTGGATTAATTACTCCC
Sequence number 2827: Rickettsia prowazekii gltA #7 
TTATTTCTATTACGACCTACATTATTCTTAATTATTCCTTCTAGTGGATTAATTACTCCCCAAACTAA
TGCTTTATATTTCCGTATTACCTGCCTTTGTTTTATCTGATTGGCAAGCAGA
Sequence number 2828: Rickettsia prowazekii gltA #8 
CAAACTAATGCTTTATATTTCCGTATTACCTGCCTTTGTTTTATCTGATTGGCAAGCAGAATATGTGT
TTTATTATTTTTAGCAACAACCATTAAACCTGTAGTGTCTTTATCTAAACGA
Sequence number 2829: Rickettsia prowazekii gltA #9 
ATATGTGTTTTATTATTTTTAGCAACAACCATTAAACCTGTAGTGTCTTTATCTAAACGATGTACTAT
GCCTGGTCTTTCTGCTGAACCAATATCTGATAGATACTTTGTATGGTGCAGT
Sequence number 2830: Rickettsia prowazekii gltA #10 
TGTACTATGCCTGGTCTTTCTGCTGAACCAATATCTGATAGATACTTTGTATGGTGCAGTAATGCGTT
AACAAGAGTATTATCGTGATGTCTTATACCTGGATGTACAGTCATACCTGCT
Sequence number 2831: Rickettsia prowazekii gltA #11 
AATGCGTTAACAAGAGTATTATCGTGATGTCTTATACCTGGATGTACAGTCATACCTGCTGCTTTGTT
AATTACTATCAAATCATCATCTTCATAAATTATATCAAGTGTTATATTTGCT
Sequence number 2832: Rickettsia prowazekii gltA #12 
GCTTTGTTAATTACTATCAAATCATCATCTTCATAAATTATATCAAGTGTTATATTTGCTGCTGTAAT
TTTTAGCTCTTCAGGCTCTTTAAAAGAAAATAATATAGTATCATTCTCCTTG
Sequence number 2833: Rickettsia prowazekii gltA #13 
GCTGTAATTTTTAGCTCTTCAGGCTCTTTAAAAGAAAATAATATAGTATCATTCTCCTTGACCAAAAC
ATCAGAATCAGAAATAATCACACCATTAACTTGTACACAATAATTCTTAATA
Sequence number 2834: Rickettsia prowazekii gltA #14 
ACCAAAACATCAGAATCAGAAATAATCACACCATTAACTTGTACACAATAATTCTTAATAGCTTTTTG
AACTTGATTTCTTGAGACCTTTTTCAGGAGTAATGATAAAGCTTTATCAAGC
Sequence number 2835: Rickettsia prowazekii gltA #15 
GCTTTTTGAACTTGATTTCTTGAGACCTTTTTCAGGAGTAATGATAAAGCTTTATCAAGCCTTGTGTT
GTTTAGTTCCTTTGGTACATTATAGGTTAACATATTTTATTTTAAGATAATG
Sequence number 2836: Rickettsia prowazekii gltA #16 
CTTGTGTTGTTTAGTTCCTTTGGTACATTATAGGTTAACATATTTTATTTTAAGATAATGAATGATAT
GTTTAAATGGTTAAATGCAATCGGTGTTGAATATTATTGCTACGAACACACA
Sequence number 2837: Rickettsia prowazekii gltA #17 
AATGATATGTTTAAATGGTTAAATGCAATCGGTGTTGAATATTATTGCTACGAACACACACCACAATT
AAAAATATCTCACAATACTGCAATCACTGAAAAAAAACAAGTTAAACTGATG
Sequence number 2838: Rickettsia prowazekii gltA #18 
CCACAATTAAAAATATCTCACAATACTGCAATCACTGAAAAAAAACAAGTTAAACTGATGCATACAAC
AGAAGCGGTATTTAATAATAATGTTCATGATAATATAACGTTATCAAGATCT
Sequence number 2839: Rickettsia prowazekii gltA #19 
CATACAACAGAAGCGGTATTTAATAATAATGTTCATGATAATATAACGTTATCAAGATCTCTTGCAGA
CAAAGCTAATAATATCGTAGAACTAAGAGAATCTCTACTAAATTTTAATGGT
Sequence number 2840: Rickettsia prowazekii gltA #20 CTTGCAGACAAAGCTAATAATATCGTAGAACTAAGAGAATCTCTACTAAATTTTAATGGTTGTGAACT TAAGAAGTTTGCTACTAATACAGTTTTCGGTGATGGTAATCCACAAGCTAAC
Sequence number 2841: Rickettsia prowazekii gltA #21 
TGTGAACTTAAGAAGTTTGCTACTAATACAGTTTTCGGTGATGGTAATCCACAAGCTAACATTATGTT
AATAGGCGAAGCTCCAGGGAATACTGAGGATTTAAAAGGAATACCTTTTTGC
Sequence number 2842: Rickettsia prowazekii gltA #22 
ATTATGTTAATAGGCGAAGCTCCAGGGAATACTGAGGATTTAAAAGGAATACCTTTTTGCGGTGAAAG
TGGTAATTTACTTGATAATATGTTGTATGCTATAGGTATTTCACGGAATAAT
Sequence number 2843: Rickettsia prowazekii gltA #23 
GGTGAAAGTGGTAATTTACTTGATAATATGTTGTATGCTATAGGTATTTCACGGAATAATTTTTATAT
TACTAATATGGTATTTTGGCGTCCTCCTGCTAATAGACAACCAACTCTAGAA
Sequence number 2844: Rickettsia prowazekii gltA #24 
TTTTATATTACTAATATGGTATTTTGGCGTCCTCCTGCTAATAGACAACCAACTCTAGAAGAAGTTGA
TATTTGCAGACCTTTCGTAGAAAAACATATTGCTCTAATTAATCCTAAGTTG
Sequence number 2845: Rickettsia prowazekii gltA #25 
GAAGTTGATATTTGCAGACCTTTCGTAGAAAAACATATTGCTCTAATTAATCCTAAGTTGCTTATTTT
AGTCGGTAGCACTGCTGCAACGAGTTTACTTGGAAAAAATGCATGTATTACT
Sequence number 2846: Rickettsia prowazekii gltA #26 
CTTATTTTAGTCGGTAGCACTGCTGCAACGAGTTTACTTGGAAAAAATGCATGTATTACTAAAATTAG
ACAAGAATATTATTTTTATACAAATAAATATATATCAACTCCTATTCAAACT
Sequence number 2847: Rickettsia prowazekii gltA #27 
AAAATTAGACAAGAATATTATTTTTATACAAATAAATATATATCAACTCCTATTCAAACTACTGCAAT
CTTTCACCCTGCATACTTACTTAGACAACCTATGCAAAAACGTACCTCTTGG
Sequence number 2848: Rickettsia prowazekii gltA #28 
ACTGCAATCTTTCACCCTGCATACTTACTTAGACAACCTATGCAAAAACGTACCTCTTGGTATGATCT
ACTAAAGATTAAGGAATATTTGGTGAATAATAGAGTCATTGCATAACTTGTA
Sequence number 2849: Rickettsia prowazekii gltA #29 
TATGATCTACTAAAGATTAAGGAATATTTGGTGAATAATAGAGTCATTGCATAACTTGTATATCTAAT
ACTATTATATGTTTTCTATGTATTTAAGCTTAATATGTGTATGACAAATACA
Sequence number 2850: Rickettsia prowazekii gltA #30 
TATCTAATACTATTATATGTTTTCTATGTATTTAAGCTTAATATGTGTATGACAAATACAATTTTTAT
AACAGCAAACTGTATTTAAGATTTGTTAGAAATTAGATCTCATAACACTGAG
Sequence number 2851: Rickettsia prowazekii gltA #31 
ATTTTTATAACAGCAAACTGTATTTAAGATTTGTTAGAAATTAGATCTCATAACACTGAGATTAGTGC
ACTTAGAATATGAATTTTTTAAATAAAATGAATGTCATATGAGCTTATGTTT
Sequence number 2852: Rickettsia prowazekii gltA #32 
ATTAGTGCACTTAGAATATGAATTTTTTAAATAAAATGAATGTCATATGAGCTTATGTTTTTTGTTAA
AGGTTATATTAAATGCATATAATAAAGATACACAAGAAGAAATTTATCAATG
Sequence number 2853: Rickettsia prowazekii gltA #33 
TTTGTTAAAGGTTATATTAAATGCATATAATAAAGATACACAAGAAGAAATTTATCAATGAAATTGTT
TGTGTTATGTGGTAAAGTTTACAGATTCTTTAAAAGAAAAATTGATAGTATG
Sequence number 2854: Rickettsia prowazekii gltA #34 
AAATTGTTTGTGTTATGTGGTAAAGTTTACAGATTCTTTAAAAGAAAAATTGATAGTATGGTATATTG
AAGTGTTAGTGCAATATTTCATGGATCAAAATAAGGAGTTATTTTTTGTTAC
Sequence number 2855: Rickettsia prowazekii gltA #35 
GTATATTGAAGTGTTAGTGCAATATTTCATGGATCAAAATAAGGAGTTATTTTTTGTTACTGTTTGTT
ATTGTTAATAAAGCTAATATAAAAATGTAATCTGAACTTTAACAATAATCTT
Sequence number 2856: Rickettsia prowazekii gltA #36 
TGTTTGTTATTGTTAATAAAGCTAATATAAAAATGTAATCTGAACTTTAACAATAATCTTTATCAAAG
CTTTTAACTTATAGATGAAAGTAGAAGAGTATGCGTTTTTAATTTTCATGAA
Sequence number 2857: Rickettsia prowazekii gltA #37 
TATCAAAGCTTTTAACTTATAGATGAAAGTAGAAGAGTATGCGTTTTTAATTTTCATGAACCTTACAC
GTTGAGCAAGATATCAAAAAAATTTATAAATCGTAATTCATTTCTAATTTCC
Sequence number 2858: Rickettsia prowazekii gltA #38 
CCTTACACGTTGAGCAAGATATCAAAAAAATTTATAAATCGTAATTCATTTCTAATTTCCTAAAATTA
GGGTTATACTGACTGAATGAGGTATATACATTATGACTAATGGCAATAATAA
Sequence number 2859: Rickettsia prowazekii gltA #39 
TAAAATTAGGGTTATACTGACTGAATGAGGTATATACATTATGACTAATGGCAATAATAATAACTTAG
AATTTGCAGAATTAAAAATTAGAGGTAAACTATTTAAGTTACCTATACTTAA
Sequence number 2860: Rickettsia prowazekii gltA #40 
TAACTTAGAATTTGCAGAATTAAAAATTAGAGGTAAACTATTTAAGTTACCTATACTTAAAGCAAGTA
TCGGTAAAGATGTAATCGATATAAGTAGGGTATCTGCGGAAGCCGATTACTT
Sequence number 2861: Rickettsia prowazekii gltA #41 
AGCAAGTATCGGTAAAGATGTAATCGATATAAGTAGGGTATCTGCGGAAGCCGATTACTTTACTTATG
ATCCGGGTTTTATGTCTACTGCTTCTTGTCAATCTACTATCACATATATAGA
Sequence number 2862: Rickettsia prowazekii gltA #42 
TACTTATGATCCGGGTTTTATGTCTACTGCTTCTTGTCAATCTACTATCACATATATAGACGGTGATA
AAGGCATATTATGGTATCGAGGATATGATATTAAAGACTTAGCTGAGAAAAG
Sequence number 2863: Rickettsia prowazekii gltA #43 
CGGTGATAAAGGCATATTATGGTATCGAGGATATGATATTAAAGACTTAGCTGAGAAAAGTGATTTTT
TAGAAGTGGCATATTTGATGATTTATGGGGAGCTACCAAGTAGTGATCAGTA
Sequence number 2864: Rickettsia prowazekii gltA #44 
TGATTTTTTAGAAGTGGCATATTTGATGATTTATGGGGAGCTACCAAGTAGTGATCAGTATTGTAATT
TTACTAAAAAGGTTGCTCATCATTCATTAGTGAATGAAAGATTACACTATTT
Sequence number 2865: Rickettsia prowazekii gltA #45 
TTGTAATTTTACTAAAAAGGTTGCTCATCATTCATTAGTGAATGAAAGATTACACTATTTATTTCAAA
CCTTTTGTAGTTCTTCTCATCCTATGGCTATTATGCTTGCAGCTGTTGGTTC
Sequence number 2866: Rickettsia prowazekii gltA #46 
ATTTCAAACCTTTTGTAGTTCTTCTCATCCTATGGCTATTATGCTTGCAGCTGTTGGTTCTCTTTCAG
CATTCTATCCTGATTTATTAAATTTTAATGAAACAGACTATGAACTTACCGC
Sequence number 2867: Rickettsia prowazekii gltA #47 
TCTTTCAGCATTCTATCCTGATTTATTAAATTTTAATGAAACAGACTATGAACTTACCGCTATTAGAA
TGATTGCTAAGATACCTACTATCGCTGCAATGTCTTATAAATATTCTATAGG
Sequence number 2868: Rickettsia prowazekii gltA #48 TATTAGAATGATTGCTAAGATACCTACTATCGCTGCAATGTCTTATAAATATTCTATAGGGCAACCGT TTATTTATCCTGATAATTCATTAGATTTACCGAAAATTTCTACATATGAT
Sequence number 2869: Rickettsia prowazekii gltA #49 
GCAACCGTTTATTTATCCTGATAATTCATTAGATTTTACCGAAAATTTTCTACATATGATGTTTGCAA
CTCCTTGTACTAAATATAAAGTAAATCCAATAATAAAAAATGCTCTTAATAA
Sequence number 2870: Rickettsia prowazekii gltA #50 
GTTTGCAACTCCTTGTACTAAATATAAAGTAAATCCAATAATAAAAAATGCTCTTAATAAGATATTTA
TCTTACATGCAGACCATGAGCAGAATGCTTCTACTTCAACAGTTCGGATTGC
Sequence number 2871: Rickettsia prowazekii gltA #51 
GATATTTATCTTACATGCAGACCATGAGCAGAATGCTTCTACTTCAACAGTTCGGATTGCTGGCTCAT
CAGGAGCTAATCCTTTTGCATGTATTAGCACTGGTATTGCATCACTTTGGGG
Sequence number 2872: Rickettsia prowazekii gltA #52 
TGGCTCATCAGGAGCTAATCCTTTTGCATGTATTAGCACTGGTATTGCATCACTTTGGGGGCCTGCTC
ACGGCGGGGCTAATGAAGCAGTGATAAATATGCTTAAAGAAATTGGCAGTTC
Sequence number 2873: Rickettsia prowazekii gltA #53 
GCCTGCTCACGGCGGGGCTAATGAAGCAGTGATAAATATGCTTAAAGAAATTGGCAGTTCTGAGAATA
TTCCTAAATATGTAGCTAAAGCTAAAGATAAGAATGATCCATTTAGGTTAAT
Sequence number 2874: Rickettsia prowazekii gltA #54 
TGAGAATATTCCTAAATATGTAGCTAAAGCTAAAGATAAGAATGATCCATTTAGGTTAATGGGTTTTG
GTCATCGAGTATATAAAAGCTATGACCCGCGTGCCGCAGTACTTAAAGAAAC
Sequence number 2875: Rickettsia prowazekii gltA #55 
GGGTTTTGGTCATCGAGTATATAAAAGCTATGACCCGCGTGCCGCAGTACTTAAAGAAACTTGTAAAG
AAGTATTAAATGAATTAGGTCAGTTAGACAATAATCCGCTGTTACAAATAGC
Sequence number 2876: Rickettsia prowazekii gltA #56 
TTGTAAAGAAGTATTAAATGAATTAGGTCAGTTAGACAATAATCCGCTGTTACAAATAGCAATAGAAC
TTGAAGCTCTCGCTCTTAAAGATGAATATTTTATTGAAAGAAAATTATATCC
Sequence number 2877: Rickettsia prowazekii gltA #57 
AATAGAACTTGAAGCTCTCGCTCTTAAAGATGAATATTTTATTGAAAGAAAATTATATCCAAATGTTG
ATTTTTATTCAGGCATTATCTATAAAGCTATGGGTATACCGTCGCAAATGTT
Sequence number 2878: Rickettsia prowazekii gltA #58 
AAATGTTGATTTTTATTCAGGCATTATCTATAAAGCTATGGGTATACCGTCGCAAATGTTCACTGTAC
TTTTTGCAATAGCAAGAACCGTAGGTTGGATGGCACAATGGAAAGAAATGCA
Sequence number 2879: Rickettsia prowazekii gltA #59 
CACTGTACTTTTTGCAATAGCAAGAACCGTAGGTTGGATGGCACAATGGAAAGAAATGCACGAAGATC
CTGAACAAAAAATCAGTAGACCTAGACAGCTTTACACTGGTTATCTACATAG
Sequence number 2880: Rickettsia prowazekii gltA #60 
CGAAGATCCTGAACAAAAAATCAGTAGACCTAGACAGCTTTACACTGGTTATCTACATAGAGAGTATA
AGTGTATTGTAGAAAGAAAGTGACGCATTAAAATTGTATGCATTAGCAATTT
Sequence number 2881: Rickettsia prowazekii gltA #61 
AGAGTATAAGTGTATTGTAGAAAGAAAGTGACGCATTAAAATTGTATGCATTAGCAATTTAGTGATTT
AGATGAAGCATTAAGAAAATCTAATTAATTCATCTAAAATTTTTTAGGAACA
Sequence number 2882: Rickettsia prowazekii gltA #62 
AGTGATTTAGATGAAGCATTAAGAAAATCTAATTAATTCATCTAAAATTTTTTAGGAACATATATTAA
TAAAAAGATGTTTTTTATACTATCATTGTCCAATGTACTATAATTTTTATTT
Sequence number 2883: Rickettsia prowazekii gltA #63 
TATATTAATAAAAAGATGTTTTTTATACTATCATTGTCCAATGTACTATAATTTTTATTTTTCAAACA
TTCCAAAACCTAGTAAAATTATAATATGAAGGTTGAATACTATTCAATAAAT
Sequence number 2884: Rickettsia prowazekii gltA #64 
TTCAAACATTCCAAAACCTAGTAAAATTATAATATGAAGGTTGAATACTATTCAATAAATTCACATCT
GTGGTTCTTTCAGAAAATTAAGAAGAAATTTATATTATAACTAACAACTTTT
Sequence number 2885: Rickettsia prowazekii gltA #65 
TCACATCTGTGGTTCTTTCAGAAAATTAAGAAGAAATTTATATTATAACTAACAACTTTTAAATTTTT
AAACACTTATACGACTAACAAAGAAATTAAAATGCATTTTATTGATGAAGTT
Sequence number 2886: Rickettsia prowazekii gltA #66 
AAATTTTTAAACACTTATACGACTAACAAAGAAATTAAAATGCATTTTATTGATGAAGTTAAAATATA
TATAAAAGGTGGAAACGGCGGCAATGGTTGTATTAGTTTTCATCGTGAGAAA
Sequence number 2887: Rickettsia prowazekii gltA #67 
AAAATATATATAAAAGGTGGAAACGGCGGCAATGGTTGTATTAGTTTTCATCGTGAGAAATTTATTGA
TAGAGGTGGACCAGATGGTGGAGATGGTGGATTTGGTGGCAGTGTTATTTTT
Sequence number 2888: Rickettsia prowazekii gltA #68 
TTTATTGATAGAGGTGGACCAGATGGTGGAGATGGTGGATTTGGTGGCAGTGTTATTTTTAGAAGTAA
TCACCATATTAATACACTAGTGAATTATCGATATCAACAACATTTTACCGCA
Sequence number 2889: Rickettsia prowazekii gltA #69 
AGAAGTAATCACCATATTAATACACTAGTGAATTATCGATATCAACAACATTTTACCGCAGAAAACGG
CGAAAACGGTAAAGGGTCAAATCGAAGTGGAAAATCAGGAAAATCATTAATT
Sequence number 2890: Rickettsia prowazekii gltA #70 
GAAAACGGCGAAAACGGTAAAGGGTCAAATCGAAGTGGAAAATCAGGAAAATCATTAATTCTTGATGT
TCCAGTCGGCACTCAAATTTTTTCTCAGGATGGCGATATATTATTGTATGAT
Sequence number 2891: Rickettsia prowazekii gltA #71 
CTTGATGTTCCAGTCGGCACTCAAATTTTTTCTCAGGATGGCGATATATTATTGTATGATTTCACTGA
AGATGAAAAAAGTTTTGAAATAATTAAAGGAGGATGTGGTGGTCTTGGTAAC
Sequence number 2892: Rickettsia prowazekii gltA #72 
TTCACTGAAGATGAAAAAAGTTTTGAAATAATTAAAGGAGGATGTGGTGGTCTTGGTAACAGCCATTT
CAAAACATCTGTTAATCAAGCACCAAGAAAGAGTACAGAAGGGGAAATTGCT
Sequence number 2893: Rickettsia prowazekii gltA #73 
AGCCATTTCAAAACATCTGTTAATCAAGCACCAAGAAAGAGTACAGAAGGGGAAATTGCTGAAGAAAT
GTGGATTCATTTGAGACTAAAACTACTCTCTGATGTCGGACTTATTGGATTA
Sequence number 2894: Rickettsia prowazekii gltA #74 
GAAGAAATGTGGATTCATTTGAGACTAAAACTACTCTCTGATGTCGGACTTATTGGATTACCCAATGC
CGGTAAATCTACTTTTTTATCTGTTGTAACGGCAGCAAAACCTAAGATTGCT
Sequence number 2895: Rickettsia prowazekii gltA #75 
CCCAATGCCGGTAAATCTACTTTTTTATCTGTTGTAACGGCAGCAAAACCTAAGATTGCTGATTATCC
ATTTACTACTTTAGTGCCGAATCTTGGAGTTGTATATATTGATGATGAAGAG
Sequence number 2896: Rickettsia prowazekii gltA #76 GATTATCCATTTACTACTTTAGTGCCGAATCTTGGAGTTGTATATATTGATGATGAAGAGTTTGTGAT AGCTGATATTCCAGGTTTGATTGCCGGAGCTCATCAAGGGTACGGACTAGGA
Sequence number 2897: Rickettsia prowazekii gltA #77 
TTTGTGATAGCTGATATTCCAGGTTTGATTGCCGGAGCTCATCAAGGGTACGGACTAGGAGATAAATT
CTTAAAGCATATAGAAAGATGTAATGTATTAATACATTTAATAGATGGTGCT
Sequence number 2898: Rickettsia prowazekii gltA #78 
GATAAATTCTTAAAGCATATAGAAAGATGTAATGTATTAATACATTTAATAGATGGTGCTAGTAATAA
TGTAATTGCAGATTATAATACGGTACGTTTTGAACTTGAGTCATATTCTGAT
Sequence number 2899: Rickettsia prowazekii gltA #79 
AGTAATAATGTAATTGCAGATTATAATACGGTACGTTTTGAACTTGAGTCATATTCTGATTATCTAAA
AAATAAAATCGAAATTATATGTCTTAATAAATGTGATGTTCTTGTTGATGAA
Sequence number 2900: Rickettsia prowazekii gltA #80 
TATCTAAAAAATAAAATCGAAATTATATGTCTTAATAAATGTGATGTTCTTGTTGATGAAGAAATACA
AAAAAAAATAAAAAAGCTTCAAAAAGTTACTAATAAAGTAGTGCACCCTATC
Sequence number 2901: Rickettsia prowazekii gltA #81 
GAAATACAAAAAAAAATAAAAAAGCTTCAAAAAGTTACTAATAAAGTAGTGCACCCTATCTCTACTTA
TAATAATCAAGGTGTGAATAAAATAGTTAAACTTGCTTTAGAGATTATAAAA
Sequence number 2902: Rickettsia prowazekii gltA #82 
TCTACTTATAATAATCAAGGTGTGAATAAAATAGTTAAACTTGCTTTAGAGATTATAAAAAATCAAAA
ATACTAATATAGTATTAGCTAATCTACGATTACTAATCATGACTTAGTTGTA
Sequence number 2903: Rickettsia prowazekii gltA #83 
AATCAAAAATACTAATATAGTATTAGCTAATCTACGATTACTAATCATGACTTAGTTGTATTATACAG
TAATGAAAATTAATAAATTTTAAATTAAGCTAATACGTTGTTCTTCGGTTTC
Sequence number 2904: Rickettsia prowazekii gltA #84 
TTATACAGTAATGAAAATTAATAAATTTTAAATTAAGCTAATACGTTGTTCTTCGGTTTCAAGATATT
TCTCTAGTTCTTCCTTAGTATATCTCTTTTCTTTAAGTTGTATTTCATTATT
Sequence number 2905: Rickettsia prowazekii gltA #85 
AAGATATTTCTCTAGTTCTTCCTTAGTATATCTCTTTTCTTTAAGTTGTATTTCATTATTAAATATAT
ATTGTACAGTTTTATCTTCTAATGCAGGACCTTTAAGTCCCTCAATCGCTCT
Sequence number 2906: Rickettsia prowazekii gltA #86 
AAATATATATTGTACAGTTTTATCTTCTAATGCAGGACCTTTAAGTCCCTCAATCGCTCTAGGATTAT
TTTTATAAAAATCAAATATCATATTTTCTTGTCCTGGAAAATTACGTGCTTG
Sequence number 2907: Rickettsia prowazekii gltA #87 
AGGATTATTTTTATAAAAATCAAATATCATATTTTCTTGTCCTGGAAAATTACGTGCTTGCTGCATAA
TAATTTTTTTGAAATCATCAGGCTCTAGTTGCAAATTTTTAAATTTTGCGTA
Sequence number 2908: Rickettsia prowazekii gltA #88 
CTGCATAATAATTTTTTTGAAATCATCAGGCTCTAGTTGCAAATTTTTAAATTTTGCGTATTCAGCAA
GTAATAAACCTATTCGTACACGACGTAGAGCTAATTTATTATAATATTCTGT
Sequence number 2909: Rickettsia prowazekii gltA #89 
TTCAGCAAGTAATAAACCTATTCGTACACGACGTAGAGCTAATTTATTATAATATTCTGTTATTTCTT
TAGAGGATTTACCGTTTAATAATGATCCATCATGTTTATTTTTATCAGTTTC
Sequence number 2910: Rickettsia prowazekii gltA #90 
TATTTCTTTAGAGGATTTACCGTTTAATAATGATCCATCATGTTTATTTTTATCAGTTTCAGACTTTA
AAATATTTTTTTCTTGCTCTAATAAAGATTCAGGTACATCAAAATCTAACAA
Sequence number 2911: Rickettsia prowazekii gltA #91 
AGACTTTAAAATATTTTTTTCTTGCTCTAATAAAGATTCAGGTACATCAAAATCTAACAATTTTTCTA
ACTTATCAAATAAGTTCATTTTCATTATAGTATTAATAGCTTCTTCTGATTC
Sequence number 2912: Rickettsia prowazekii gltA #92 
TTTTTCTAACTTATCAAATAAGTTCATTTTCATTATAGTATTAATAGCTTCTTCTGATTCGTTTTCTA
TTTGTTTAGCAAAATGTGTACGTAATTCTTCAAGGCTATTACTTTGGAATTT
Sequence number 2913: Rickettsia prowazekii gltA #93 
GTTTTCTATTTGTTTAGCAAAATGTGTACGTAATTCTTCAAGGCTATTACTTTGGAATTTTTTAGCAA
ACTCCTCATCAATAATAGTAGGTTCTGCAGTATGTACTGCTTTAATTTGTAC
Sequence number 2914: Rickettsia prowazekii gltA #94 
TTTAGCAAACTCCTCATCAATAATAGTAGGTTCTGCAGTATGTACTGCTTTAATTTGTACTACAAAAT
GAGCATCTTTACCTGATAAATTTTTATCATGATAATTTTCAGGAAAAGTAAC
Sequence number 2915: Rickettsia prowazekii gltA #95 
TACAAAATGAGCATCTTTACCTGATAAATTTTTATCATGATAATTTTCAGGAAAAGTAACGTTAACAT
CTACTTCATTACCTGTTTTAGAACCTATTAATTGTTGTTCAAAACCTTGAAT
Sequence number 2916: Rickettsia prowazekii gltA #96 
GTTAACATCTACTTCATTACCTGTTTTAGAACCTATTAATTGTTGTTCAAAACCTTGAATAAGGGTAT
TACTACCTATTACTACCTTAAAATCATTTAATTTGCCACCATCAAAAGCTCT
Sequence number 2917: Rickettsia prowazekii gltA #97 
AAGGGTATTACTACCTATTACTACCTTAAAATCATTTAATTTGCCACCATCAAAAGCTCTATCCTTAA
TATATCCAATTGCATCAATAGTAACCTGATCACCATCTTTTATTTTTGTTTT
Sequence number 2918: Rickettsia prowazekii gltA #98 
ATCCTTAATATATCCAATTGCATCAATAGTAACCTGATCACCATCTTTTATTTTTGTTTTACTTTCTT
TAGTATAACATTTTGTTAATGCAGCAAGTTTTTCTAATTGTATTTCAACATC
Sequence number 2919: Rickettsia prowazekii gltA #99 
ACTTTCTTTAGTATAACATTTTGTTAATGCAGCAAGTTTTTCTAATTGTATTTCAACATCTTGAGAGT
TTACTGCTAATTTTGGACGATCTAGTGAAATTTTCTTTAAATCCGGAATGGT
Sequence number 2920: Rickettsia prowazekii gltA #100 
TTGAGAGTTTACTGCTAATTTTGGACGATCTAGTGAAATTTTCTTTAAATCCGGAATGGTAATTTTAG
GCAACAACTCTATTTTTACTGTAAATTCTAAATCTTTATCGGGTTCGTTTTG
Sequence number 2921: Rickettsia prowazekii gltA #101 
AATTTTAGGCAACAACTCTATTTTTACTGTAAATTCTAAATCTTTATCGGGTTCGTTTTGTAATTCTT
CAATTTTTGGTCTACCTATTATATTTAGATTATACTCTTTAATAATGTGATT
Sequence number 2922: Rickettsia prowazekii gltA #102 
TAATTCTTCAATTTTTGGTCTACCTATTATATTTAGATTATACTCTTTAATAATGTGATTTACTAAAT
TGTTAATTCTTTTTTCTATTATATCATGCCTAACAGAAGTACCGTATTTCTT
Sequence number 2923: Rickettsia prowazekii gltA #103 
TACTAAATTGTTAATTCTTTTTTCTATTATATCATGCCTAACAGAAGTACCGTATTTCTTCTTAACAA
TTGAAACAGGTACTTTACCAGCTCTAAAACCAGCTACTTTAACTTTTTTGGT
Sequence number 2924: Rickettsia prowazekii gltA #104 CTTAACAATTGAAACAGGTACTTTACCAGCTCTAAAACCAGCTACTTTAACTTTTTTGGTTAAATCAA GTAATTCTTTTTGTATGTCATCATCTATCTCACTTAAAGGAGTAGAGATTCT
Sequence number 2925: Rickettsia prowazekii gltA #105 
TTTAACTTTTTTGGTTAAATCAAGTAATTCTTTTTGTATGTCATCATCTATCTCACTTAAAGGAGTAG
AGATTCTTGCATGAAAATTCAATCCTTCGTTTTTTAATACGATAATTCCCAT
Sequence number 2926: Rickettsia typhi gltA #1 
ATTACGTACACATTTTGCTAAACAAATAGAAAATGAGTCAGAAGAAGCTATTAATACTATAATGAAAA
TGAACTTATTTGATAAGTTAGAAAAATTGTTAGATTTTGATGTACCAGAATC
Sequence number 2927: Rickettsia typhi gltA #2 
AATGAAAATGAACTTATTTGATAAGTTAGAAAAATTGTTAGATTTTGATGTACCAGAATCTTTATTAG
AGCAAGAAAAAAATATTTTAAAATCCGAAACCGATAAAAATAAACATGATGG
Sequence number 2928: Rickettsia typhi gltA #3 
TTTATTAGAGCAAGAAAAAAATATTTTAAAATCCGAAACCGATAAAAATAAACATGATGGATCATTAT
TAAATGGGAAATCCTCTAAAGAAATAATAGAATATTATAATAAATTAGCACT
Sequence number 2929: Rickettsia typhi gltA #4 
ATCATTATTAAATGGGAAATCCTCTAAAGAAATAATAGAATATTATAATAAATTAGCACTACGACGTG
TACGCATAGGCTTATTACTTGCTGAGTACGCAAAATTTAAAAATTTACAACT
Sequence number 2930: Rickettsia typhi gltA #5 
ACGACGTGTACGCATAGGCTTATTACTTGCTGAGTACGCAAAATTTAAAAATTTACAACTAGAGCCTG
ATGATTTGAAAAAAGTTATTATGCAGCAAGCACGTAATTTTCCTGGACAAGA
Sequence number 2931: Rickettsia typhi gltA #6 
AGAGCCTGATGATTTGAAAAAAGTTATTATGCAGCAAGCACGTAATTTTCCTGGACAAGAAAATATGA
TATTTGATTTTTACAAAAATAATCCTAGAGCCATTGAAGGGCTTAGAGGTCC
Sequence number 2932: Rickettsia typhi gltA #7 
AAATATGATATTTGATTTTTACAAAAATAATCCTAGAGCCATTGAAGGGCTTAGAGGTCCTGCATTAG
AAGATAAAACTGTACAATATATATTTAATAATGAAATACAACTTAAAGAAAA
Sequence number 2933: Rickettsia typhi gltA #8 
TGCATTAGAAGATAAAACTGTACAATATATATTTAATAATGAAATACAACTTAAAGAAAAGAGATATT
CTAAGGAAGAGCTAGAAAAATATCTTGAAACCGAAGAACAGCAACGTATTAG
Sequence number 2934: Rickettsia typhi gltA #9 
GAGATATTCTAAGGAAGAGCTAGAAAAATATCTTGAAACCGAAGAACAGCAACGTATTAGTTTAATTT
AAAGTTAATTAATTGTCATTGCTATAATACAACGTAGAACAATTAAGTCATG
Sequence number 2935: Rickettsia typhi gltA #10 
TTTAATTTAAAGTTAATTAATTGTCATTGCTATAATACAACGTAGAACAATTAAGTCATGATTAGTAA
TCGTAGATTTAGGTAGAAATAATAAATTTAAATCAGATTTTCTTATTTACTA
Sequence number 2936: Rickettsia typhi gltA #11 
ATTAGTAATCGTAGATTTAGGTAGAAATAATAAATTTAAATCAGATTTTCTTATTTACTATAGTTTTA
CTTTACAAAAATTGGTGCACGTAGTTAATACTTAAATATTACTATATTTGAT
Sequence number 2937: Rickettsia typhi gltA #12 
TAGTTTTACTTTACAAAAATTGGTGCACGTAGTTAATACTTAAATATTACTATATTTGATTTTTTATA
ATCTCTAAAGCAAGTTTAACTATTTTATTCACACCTAGATTAGTATAAGTAG
Sequence number 2938: Rickettsia typhi gltA #13 
TTTTTATAATCTCTAAAGCAAGTTTAACTATTTTATTCACACCTAGATTAGTATAAGTAGAAATAGGG
TGCACTACTTTATTAGTAACTCTCTGAAGCTTCTTTATTTTTTTTTGTATTT
Sequence number 2939: Rickettsia typhi gltA #14 
AAATAGGGTGCACTACTTTATTAGTAACTCTCTGAAGCTTCTTTATTTTTTTTTGTATTTCCTCATCA
GTAAGAACATCACATTTATTAATACATATGATTTCGATTTTATTTTTTAAAT
Sequence number 2940: Rickettsia typhi gltA #15 
CCTCATCAGTAAGAACATCACATTTATTAATACATATGATTTCGATTTTATTTTTTAAATAATCCGAA
TATGATTCAAGTTCAAAACGTACAGTATCATAATCTGCGATTACATTATGAC
Sequence number 2941: Rickettsia typhi gltA #16 
AATCCGAATATGATTCAAGTTCAAAACGTACAGTATCATAATCTGCGATTACATTATGACTAGAACCA
TCTATTAAATGTATTAATACATTACATCTTTCTATATGCTTTAAGAATTTAT
Sequence number 2942: Rickettsia typhi gltA #17 
TAGAACCATCTATTAAATGTATTAATACATTACATCTTTCTATATGCTTTAAGAATTTATCTCCAAGT
CCGTGTCCTTGGTGAGCTCCTGCAATCAAACCTGGAATATCAGCTATTACAA
Sequence number 2943: Rickettsia typhi gltA #18 
CTCCAAGTCCGTGTCCTTGGTGAGCTCCTGCAATCAAACCTGGAATATCAGCTATTACAAACTCTTCA
TCATTAATATATACAACTCCAAGATTTGGCACTAAAGTAGTAAATGGATAAT
Sequence number 2944: Rickettsia typhi gltA #19 
ACTCTTCATCATTAATATATACAACTCCAAGATTTGGCACTAAAGTAGTAAATGGATAATCAGCAATC
TTAGGTTTCGCTGCTGTTACAACAGATAAAAAAGTAGATTTACCTGCATTGG
Sequence number 2945: Rickettsia typhi gltA #20 
CAGCAATCTTAGGTTTCGCTGCTGTTACAACAGATAAAAAAGTAGATTTACCTGCATTGGGTAACCCA
ACAAGCCCAACATCAGAAAGTAGTTTTAGTCTCAAATGAATCCACATTTCTT
Sequence number 2946: Rickettsia typhi gltA #21 
GTAACCCAACAAGCCCAACATCAGAAAGTAGTTTTAGTCTCAAATGAATCCACATTTCTTGCGCAATT
TCTCCTTCTGTACGCTTTCTTGGCGCTTGATTAACAGATGTTTTGAAATGGC
Sequence number 2947: Rickettsia typhi gltA #22 
GCGCAATTTCTCCTTCTGTACGCTTTCTTGGCGCTTGATTAACAGATGTTTTGAAATGGCTGTTACCA
AGACCACCACATCCTCCTTTAATTATTTCAAAACTTTTTTCATCTTCCGTGA
Sequence number 2948: Rickettsia typhi gltA #23 
TGTTACCAAGACCACCACATCCTCCTTTAATTATTTCAAAACTTTTTTCATCTTCCGTGAAATCATGC
AATAATATATTGCCGTCCTCAGAAAAAATTTGAGTACCGATTGGAACATCAA
Sequence number 2949: Rickettsia typhi gltA #24 
AATCATGCAATAATATATTGCCGTCCTCAGAAAAAATTTGAGTACCGATTGGAACATCAAGAATTAAT
GATTTTCCTGATTTACCACTGCGATTTGAACCTTTGCCGTTTCCACCGTTTT
Sequence number 2950: Rickettsia typhi gltA #25 
GAATTAATGATTTTCCTGATTTACCACTGCGATTTGAACCTTTGCCGTTTCCACCGTTTTCTGCGATA
AAATGCTGTTGATATCGATAGTTCACTAATGTATTAATATGGTGATTACTTC
Sequence number 2951: Rickettsia typhi gltA #26 
CTGCGATAAAATGCTGTTGATATCGATAGTTCACTAATGTATTAATATGGTGATTACTTCTAAAAATA
ACACTGCCACCAAATCCACCATCTCCACCATCCGGTCCACCTCTGTCAACAA
Sequence number 2952: Rickettsia typhi gltA #27 TAAAAATAACACTGCCACCAAATCCACCATCTCCACCATCCGGTCCACCTCTGTCAACAAATTTCTCA CGATGAAAACTAATACAACCATTGCCGCCGTTTCCACCTTTTATATATATTT
Sequence number 2953: Rickettsia typhi gltA #28 
ATTTCTCACGATGAAAACTAATACAACCATTGCCGCCGTTTCCACCTTTTATATATATTTTAACTTCA
TCAATAAAATGCATTTTACTTTCTTGTTAGTGATATAAGTGTTTTAAAATTT
Sequence number 2954: Rickettsia typhi gltA #29 
TAACTTCATCAATAAAATGCATTTTACTTTCTTGTTAGTGATATAAGTGTTTTAAAATTTAAAAGTTG
TTAGTAAGCTTTAATTATTATAATATAAATTATAAATTTCCTCTTAATCTTC
Sequence number 2955: Rickettsia typhi gltA #30 
AAAAGTTGTTAGTAAGCTTTAATTATTATAATATAAATTATAAATTTCCTCTTAATCTTCTGATTAAT
CTTCCAAAAGGATTGCAGATGTGAATTTATTTAATGGTATTCTATCTTGATT
Sequence number 2956: Rickettsia typhi gltA #31 
TGATTAATCTTCCAAAAGGATTGCAGATGTGAATTTATTTAATGGTATTCTATCTTGATTATAGAATG
TGATATTATTCTAATTTTTCTAGGTTTCCGTAATATTTAAAAAATAAAAATT
Sequence number 2957: Rickettsia typhi gltA #32 
ATAGAATGTGATATTATTCTAATTTTTCTAGGTTTCCGTAATATTTAAAAAATAAAAATTATCGCGCA
TTGGACAACACATAATACAAAAAGCATCTTTTTTATTAAAATGATGATTTTT
Sequence number 2958: Rickettsia typhi gltA #33 
ATCGCGCATTGGACAACACATAATACAAAAAGCATCTTTTTTATTAAAATGATGATTTTTGCTAAAAA
ATTTTAGATGAAGTAATTATATTTCTAATTCATCATCTAAATCACTAAATTT
Sequence number 2959: Rickettsia typhi gltA #34 
GCTAAAAAATTTTAGATGAAGTAATTATATTTCTAATTCATCATCTAAATCACTAAATTTATCACCTT
TATATCTCATATAATTTTAGTTTGACTTTATGAGACAAATTTATTAATAACC
Sequence number 2960: Rickettsia typhi gltA #35 
ATCACCTTTATATCTCATATAATTTTAGTTTGACTTTATGAGACAAATTTATTAATAACCTAGTAAAT
AGAATTTTAATTCGTCACTTTCTTTCTACAATACACTTATATTCTCTATGTA
Sequence number 2961: Rickettsia typhi gltA #36 
TAGTAAATAGAATTTTAATTCGTCACTTTCTTTCTACAATACACTTATATTCTCTATGTACATAACCA
GTGTAAAGTTGTCTAGGTCTACTGATTTTTTGTTCAGGATCTTCGTGCATTT
Sequence number 2962: Rickettsia typhi gltA #37 
CATAACCAGTGTAAAGTTGTCTAGGTCTACTGATTTTTTGTTCAGGATCTTCGTGCATTTCTTTCCAT
TGCGCCATCCAACCTACGGTTCTTGCTATTGCAAAAAGTACAGTGAACATTT
Sequence number 2963: Rickettsia typhi gltA #38 
CTTTCCATTGCGCCATCCAACCTACGGTTCTTGCTATTGCAAAAAGTACAGTGAACATTTGCGACGGT
ATACCCATAGCTTTATAGATAATGCCTGAATAAAAATCAACATTTGGATATA
Sequence number 2964: Rickettsia typhi gltA #39 
GCGACGGTATACCCATAGCTTTATAGATAATGCCTGAATAAAAATCAACATTTGGATATAATTTTCTC
TCAATAAAATATTCATCTTTAAGAGCAAGAGCTTCAAGTTCTATTGCTATTT
Sequence number 2965: Rickettsia typhi gltA #40 
ATTTTCTCTCAATAAAATATTCATCTTTAAGAGCAAGAGCTTCAAGTTCTATTGCTATTTGTAACAAC
GGATTATTTTCTAACTGCCCTAGTTCATTTAATACTTCTTTACAAGTTTCTT
Sequence number 2966: Rickettsia typhi gltA #41 
GTAACAACGGATTATTTTCTAACTGCCCTAGTTCATTTAATACTTCTTTACAAGTTTCTTTAAGTACT
GCAGCACGCGGGTCATAACTTTTATATACTCGATGACCAAAACCCATTAACC
Sequence number 2967: Rickettsia typhi gltA #42 
TAAGTACTGCAGCACGCGGGTCATAACTTTTATATACTCGATGACCAAAACCCATTAACCTAAATGGA
TCATTTTTGTCTTTAGCTTTAGCTACATATTTAGGAATATTCTCAGAACTAC
Sequence number 2968: Rickettsia typhi gltA #43 
TAAATGGATCATTTTTGTCTTTAGCTTTAGCTACATATTTAGGAATATTCTCAGAACTACCAATTTCT
TTAAGCATATTTATCACTGCTTCATTAGCTCCACCGTGAGCAGGCCCCCAAA
Sequence number 2969: Rickettsia typhi gltA #44 
CAATTTCTTTAAGCATATTTATCACTGCTTCATTAGCTCCACCGTGAGCAGGCCCCCAAAGTGATGCA
ATACCAGTGCTAATACATGCAAAAGGATTAGCTCCTGATGAGCCAGCAATTC
Sequence number 2970: Rickettsia typhi gltA #45 
GTGATGCAATACCAGTGCTAATACATGCAAAAGGATTAGCTCCTGATGAGCCAGCAATTCGTACTGTT
GAAGTAGAAGCATTCTGCTCATGGTCTGCATGTAAGATAAATATCTTATTAA
Sequence number 2971: Rickettsia typhi gltA #46 
GTACTGTTGAAGTAGAAGCATTCTGCTCATGGTCTGCATGTAAGATAAATATCTTATTAAGCGCATTT
TTTATTATTGGATTTACTTTATATTTAGTACAAGGAGTTGCAAACATCATAT
Sequence number 2972: Rickettsia typhi gltA #47 
GCGCATTTTTTATTATTGGATTTACTTTATATTTAGTACAAGGAGTTGCAAACATCATATGTAGAAAA
TTTTCGGTAAAATCTAATGAATTATCAGGATAAATAAATGGCTGCCCTATAG
Sequence number 2973: Rickettsia typhi gltA #48 
GTAGAAAATTTTCGGTAAAATCTAATGAATTATCAGGATAAATAAATGGCTGCCCTATAGAATATTTA
TAAGACATTGCAGCGATAGTAGGTATCTTAGCAATCATTCTAATAGCGGTAA
Sequence number 2974: Rickettsia typhi gltA #49 
AATATTTATAAGACATTGCAGCGATAGTAGGTATCTTAGCAATCATTCTAATAGCGGTAAGTTCATAG
TCTGTTTCATTAAAATTCAATAAATCAGGATAAAATGCTGAAAGAGAACCAA
Sequence number 2975: Rickettsia typhi gltA #50 
GTTCATAGTCTGTTTCATTAAAATTCAATAAATCAGGATAAAATGCTGAAAGAGAACCAACAGCTGCA
AGCATAATAGCCATAGGATGAGAAGAACTACAAAAGGTTTGAAATAAATAGT
Sequence number 2976: Rickettsia typhi gltA #51 
CAGCTGCAAGCATAATAGCCATAGGATGAGAAGAACTACAAAAGGTTTGAAATAAATAGTGTAATCTT
TCATTTACTAATGCATGATGAGCAATCTTTGTAGTAAAATTATGATACTGCT
Sequence number 2977: Rickettsia typhi gltA #52 
GTAATCTTTCATTTACTAATGCATGATGAGCAATCTTTGTAGTAAAATTATGATACTGCTCACTACTT
GGTAGTTCCCCATAAATCATCAAATATGCCACTTCTAAAAAATCACTTTTCT
Sequence number 2978: Rickettsia typhi gltA #53 
CACTACTTGGTAGTTCCCCATAAATCATCAAATATGCCACTTCTAAAAAATCACTTTTCTCAGCTAAG
TCTTTAATATCATATCCTCGATGCCATAAGATTCCTTTATCACCGTCTATAT
Sequence number 2979: Rickettsia typhi gltA #54 
CAGCTAAGTCTTTAATATCATATCCTCGATGCCATAAGATTCCTTTATCACCGTCTATATATGTGATG
GTAGATTGACAAGAAGCAGTAGACATAAAACCTGGATCGTAAGTAAAGCAAT
Sequence number 2980: Rickettsia typhi gltA #55 ATGTGATGGTAGATTGACAAGAAGCAGTAGACATAAAACCTGGATCGTAAGTAAAGCAATCGGCTTCC GCAGATACCCTACTTATATCTATTACATCTTTACCTATACTTGCTTTAAGTA
Sequence number 2981: Rickettsia typhi gltA #56 
CGGCTTCCGCAGATACCCTACTTATATCTATTACATCTTTACCTATACTTGCTTTAAGTATAGGTAAT
TTAAACATTTTACCTCTAATTTTTAATTCCGCAAATTCTAAATTATTATTAT
Sequence number 2982: Rickettsia typhi gltA #57 
TAGGTAATTTAAACATTTTACCTCTAATTTTTAATTCCGCAAATTCTAAATTATTATTATTTCCATTA
GTCATAATGTATATACCTTATTTTACATCAGTAAGTATAACCCTAATTATAG
Sequence number 2983: Rickettsia typhi gltA #58 
TTCCATTAGTCATAATGTATATACCTTATTTTACATCAGTAAGTATAACCCTAATTATAGGAAATTAG
AAATGAATTATGATTTATAAATTTTCTTTGATATCTTACTCAACGTGTAAGG
Sequence number 2984: Rickettsia typhi gltA #59 
GAAATTAGAAATGAATTATGATTTATAAATTTTCTTTGATATCTTACTCAACGTGTAAGGTTAATGAA
AATTAAAAACACATACACCTCTATTTTCGCTATAAGTTAAAAGCTTTTTTGA
Sequence number 2985: Rickettsia typhi gltA #60 
TTAATGAAAATTAAAAACACATACACCTCTATTTTCGCTATAAGTTAAAAGCTTTTTTGAATAAAAAT
GCAATTTTCTAGATAATATTTTTATATTAGCTTTATTATATGCTATACCTCC
Sequence number 2986: Rickettsia typhi gltA #61 
ATAAAAATGCAATTTTCTAGATAATATTTTTATATTAGCTTTATTATATGCTATACCTCCTCCTCTTC
TTACTATATCTTTTACAGAAAAAATAACTCCTTATTGTGATCCATAAAATAT
Sequence number 2987: Rickettsia typhi gltA #62 
TCCTCTTCTTACTATATCTTTTACAGAAAAAATAACTCCTTATTGTGATCCATAAAATATTACGCTAA
CACTTCAATATACCATACTAATATTTTTTTCTTTTAAAAAATCTGTAACCAC
Sequence number 2988: Rickettsia typhi gltA #63 
TACGCTAACACTTCAATATACCATACTAATATTTTTTTCTTTTAAAAAATCTGTAACCACATAATACA
AACAATTTCAGTGATAAATTTCTTTTACTATTTCTAAAGTATTTTTATACTT
Sequence number 2989: Rickettsia typhi gltA #64 
ATAATACAAACAATTTCAGTGATAAATTTCTTTTACTATTTCTAAAGTATTTTTATACTTTATTTATC
TTATATTACATGCATTTAATAACAGAATAATTTTATAAAAAATATGAGCTGC
Sequence number 2990: Rickettsia typhi gltA #65 
TATTTATCTTATATTACATGCATTTAATAACAGAATAATTTTATAAAAAATATGAGCTGCATTTGATT
TAATAAATTTCAAGTACACTAATCTAGTGTCATGATATCTAATTTTCTAACA
Sequence number 2991: Rickettsia typhi gltA #66 
ATTTGATTTAATAAATTTCAAGTACACTAATCTAGTGTCATGATATCTAATTTTCTAACAAAAATATT
TACTGTTTGCTGCTATAAACATTGTATTTGTTACACATATATTACGCTTAAA
Sequence number 2992: Rickettsia typhi gltA #67 
AAAATATTTACTGTTTGCTGCTATAAACATTGTATTTGTTACACATATATTACGCTTAAATATATGGT
ACGCTTAAATATATGGAAAAATATATATAGTATTAGATGTACAAGTTATGCA
Sequence number 2993: Rickettsia typhi gltA #68 
TATATGGTACGCTTAAATATATGGAAAAATATATATAGTATTAGATGTACAAGTTATGCAATAACTCT
ATTATTCACTAAATATTCCTTAATCTTTAGTAGATCATACCAGGAGGTGCGT
Sequence number 2994: Rickettsia typhi gltA #69 
ATAACTCTATTATTCACTAAATATTCCTTAATCTTTAGTAGATCATACCAGGAGGTGCGTTTTTGCAT
AGGTTGTCTCAGTAAATATGCAGGGTGAAAGATTGCAGTAGTTTGAATAGGA
Sequence number 2995: Rickettsia typhi gltA #70 
TTTTGCATAGGTTGTCTCAGTAAATATGCAGGGTGAAAGATTGCAGTAGTTTGAATAGGAGTTGATAT
ATATTTATTTGTATAAAAGTAATATTCTTGTCTAATTTTAGTAATACATGAA
Sequence number 2996: Rickettsia typhi gltA #71 
GTTGATATATATTTATTTGTATAAAAGTAATATTCTTGTCTAATTTTAGTAATACATGAATTTTTTCC
AAGTAAACTAGTTGCAGCAGTGCTACCAACTAGAATAAGTAACTTAGGATTA
Sequence number 2997: Rickettsia typhi gltA #72 
TTTTTTCCAAGTAAACTAGTTGCAGCAGTGCTACCAACTAGAATAAGTAACTTAGGATTAATTAGGGC
AATATGTTTTTCTACAAAAGGTCTACAAATATCAACTTCTTCCAGAGTCGGT
Sequence number 2998: Rickettsia typhi gltA #73 
ATTAGGGCAATATGTTTTTCTACAAAAGGTCTACAAATATCAACTTCTTCCAGAGTCGGTTGTCTATT
AGCAGGAGGACGCCAAAATACCATATTAGTAATATAAAAATTATTTTTCCGT
Sequence number 2999: Rickettsia typhi gltA #74 
TGTCTATTAGCAGGAGGACGCCAAAATACCATATTAGTAATATAAAAATTATTTTTCCGTGAAATCCC
TATAGCATACAACATATTATCAAGTAAATTACCACTTTCACCACAAAAAGGT
Sequence number 3000: Rickettsia typhi gltA #75 
GAAATCCCTATAGCATACAACATATTATCAAGTAAATTACCACTTTCACCACAAAAAGGTATCCCTTT
TAAATCTTCAGTATTCCCTGGAGCTTCACCTATTAACATAATTTTAGCTTGT
Sequence number 3001: Rickettsia typhi gltA #76 
ATCCCTTTTAAATCTTCAGTATTCCCTGGAGCTTCACCTATTAACATAATTTTAGCTTGTGGATTACC
ATCACCGAAAACTGTATTAGTAGCAAATTTCTTAAGTTCGCAACCATTAAAA
Sequence number 3002: Rickettsia typhi gltA #77 
GGATTACCATCACCGAAAACTGTATTAGTAGCAAATTTCTTAAGTTCGCAACCATTAAAATTTAGTAG
AGATTCTCGTAGTTCTGCGATATTATTAGCTTTATCTGCAAGAGATCTTGAT
Sequence number 3003: Rickettsia typhi gltA #78 
TTTAGTAGAGATTCTCGTAGTTCTGCGATATTATTAGCTTTATCTGCAAGAGATCTTGATAAAGTGAT
ATTATCATGAACATTATTATTACATGCCGCTTCTGTTGTATGCATAAGTTCT
Sequence number 3004: Rickettsia typhi gltA #79 
AAAGTGATATTATCATGAACATTATTATTACATGCCGCTTCTGTTGTATGCATAAGTTCTACTTGTTG
CTTTTCAGTGAGTTCAGTATTATTAGGTATTTTTTGTGGTGTGTGTTCGTAG
Sequence number 3005: Rickettsia typhi gltA #80 
ACTTGTTGCTTTTCAGTGAGTTCAGTATTATTAGGTATTTTTTGTGGTGTGTGTTCGTAGCAATAATA
TTCAACACCGATTGCATTTAACCATTTAAAAATATCATTCATTATCTTACAA
Sequence number 3006: Rickettsia typhi gltA #81 
CAATAATATTCAACACCGATTGCATTTAACCATTTAAAAATATCATTCATTATCTTACAATAAAATAT
GTTAACATATCATATACCAAAGGAACTAAACAACACAAGGCTTGATAAAGCT
Sequence number 3007: Rickettsia typhi gltA #82 
TAAAATATGTTAACATATCATATACCAAAGGAACTAAACAACACAAGGCTTGATAAAGCTTTATCATC
ACTCCTTGAAAAGGTCTCAAGAAATCAAGTTCAAAAAGCTATTAAGAATTCT
Sequence number 3008: Rickettsia typhi gltA #83 TTATCATCACTCCTTGAAAAGGTCTCAAGAAATCAAGTTCAAAAAGCTATTAAGAATTCTTGTGTACA AATTAATGATGTGATTATTTCTGATTCTGATGTTTTGGTCAAGGAGAATGAT
Sequence number 3009: Rickettsia typhi gltA #84 
TGTGTACAAATTAATGATGTGATTATTTCTGATTCTGATGTTTTGGTCAAGGAGAATGATACTATATT
ATTTTCTTTTAAAGAGCCTGAAGAACTAAAAATTACATCAGCAAATATAACA
Sequence number 3010: Rickettsia typhi gltA #85 
ACTATATTATTTTCTTTTAAAGAGCCTGAAGAACTAAAAATTACATCAGCAAATATAACACTTGATAT
AATTTATGAAGATGATGATTTGATAGTAATTAACAAAGCAGCAGGTATAACT
Sequence number 3011: Rickettsia typhi gltA #86 
CTTGATATAATTTATGAAGATGATGATTTGATAGTAATTAACAAAGCAGCAGGTATAACTGTACATCC
AGGTGCAAAACATCATGATAATACTCTTGTTAATGCATTACTGCACCATACA
Sequence number 3012: Rickettsia typhi gltA #87 
GTACATCCAGGTGCAAAACATCATGATAATACTCTTGTTAATGCATTACTGCACCATACAAAGAATTT
ATCAGATATTGGTTCAGCAGAAAGGCCAGGAATAGTACATCGTTTAGATAAA
Sequence number 3013: Rickettsia typhi gltA #88 
AAGAATTTATCAGATATTGGTTCAGCAGAAAGGCCAGGAATAGTACATCGTTTAGATAAAGAAACTAC
AGGTTTGATGGTTGTTGCTAAAAATAATAAAGCTCATATGCTACTTGCCAAA
Sequence number 3014: Rickettsia typhi gltA #89 
GAAACTACAGGTTTGATGGTTGTTGCTAAAAATAATAAAGCTCATATGCTACTTGCCAAACAGATAGA
ACAAAGGCAGGTGATACGAAAATATAAAGCATTAGTTTGGGGGGTAATTAAT
Sequence number 3015: Rickettsia typhi gltA #90 
CAGATAGAACAAAGGCAGGTGATACGAAAATATAAAGCATTAGTTTGGGGGGTAATTAATCCACTAGA
AGGAATAATTAATAATAATATAGGTCGTAATAGAAATAACCGCAAAAAAATG
Sequence number 3016: Rickettsia typhi gltA #91 
CCACTAGAAGGAATAATTAATAATAATATAGGTCGTAATAGAAATAACCGCAAAAAAATGACAATATT
AAAATACGGTGGAAAAAAAGCTGTTACGCATTACAAGACTTTAGAGCTATTT
Sequence number 3017: Rickettsia typhi gltA #92 
GAAATAACCGCAAAAAAATGACAATATTAAAATACGGTGGAAAAAAAGCTGTTACGCATTACAAGACT
TTAGAGCTATTTTACGGCGGCACTCTTAGTATGGTGGAATGCAAGCTTAGTA
Sequence number 3018: Rubella virus Whole genome #1 
CAATGGGAGCTATCGGACCTCGCTTAGGACTCCTAATCCCATGGAGAAACTCCTAGATGAGGTTCTTG
CCCCCGGTGGGCCTTACAACTTAACCGTCGGCAGTTGGGTAAGAGACCACGT
Sequence number 3019: Rubella virus Whole genome #2 
GGTTCTTGCCCCCGGTGGGCCTTACAACTTAACCGTCGGCAGTTGGGTAAGAGACCACGTCCGCTCAA
TTGTCGAGGGCGCGTGGGAAGTGCGCGATGTTGTTACCGCTGCCCAAAAGCG
Sequence number 3020: Rubella virus Whole genome #3 
CCGCTCAATTGTCGAGGGCGCGTGGGAAGTGCGCGATGTTGTTACCGCTGCCCAAAAGCGGGCCATCG
TAGCCGTGATACCCAGACCTGTGTTCACGCAGATGCAGGTTAGCGATCACCC
Sequence number 3021: Rubella virus Whole genome #4 
GGCCATCGTAGCCGTGATACCCAGACCTGTGTTCACGCAGATGCAGGTTAGCGATCACCCAGCACTCC
ACGCAATTTCGCGGTACACCCGCCGCCATTGGATCGAGTGGGGCCCTAAAGA
Sequence number 3022: Rubella virus Whole genome #5 
AGCACTCCACGCAATTTCGCGGTACACCCGCCGCCATTGGATCGAGTGGGGCCCTAAAGAAGCCCTAC
ACGTCCTCATCGACCCAAGCCCGGGCTTGCTCCGCGAGGTCGCTCGCGTTGA
Sequence number 3023: Rubella virus Whole genome #6 
AGCCCTACACGTCCTCATCGACCCAAGCCCGGGCTTGCTCCGCGAGGTCGCTCGCGTTGAGCGCCGTT
GGGTCGCACTGTGCCTCCACAGGACGGCACGCAAACTCGCCACCGCCCTGGC
Sequence number 3024: Rubella virus Whole genome #7 
GCGCCGTTGGGTCGCACTGTGCCTCCACAGGACGGCACGCAAACTCGCCACCGCCCTGGCCGAGACGG
CCAGCGAGGCGTGGCACGCCGACTACGTGTGCGCGCTGCGTGGCGCACCGAG
Sequence number 3025: Rubella virus Whole genome #8 
CGAGACGGCCAGCGAGGCGTGGCACGCCGACTACGTGTGCGCGCTGCGTGGCGCACCGAGCGGCCCCT
TCTACGTCCACCCTGAGGACGTCCCGCACGGCGGCCGCGCCGTGGCGGACAG
Sequence number 3026: Rubella virus Whole genome #9 
CGGCCCCTTCTACGTCCACCCTGAGGACGTCCCGCACGGCGGCCGCGCCGTGGCGGACAGATGCCTGC
TCTACTACACACCCATGCAGATGTGCGAGCTGATGCGCACCATTGACGCCAC
Sequence number 3027: Rubella virus Whole genome #10 
ATGCCTGCTCTACTACACACCCATGCAGATGTGCGAGCTGATGCGCACCATTGACGCCACCTTGCTCG
TGGCGGTTGACTTGTGGCCGGTCGCCCTTGCGGCCCACGTCGGCGACGACTG
Sequence number 3028: Rubella virus Whole genome #11 
CTTGCTCGTGGCGGTTGACTTGTGGCCGGTCGCCCTTGCGGCCCACGTCGGCGACGACTGGGACGACC
TGGGCATCGCCTGGCATCTCGACCATGACGGCGGCTGCCCCGCCGATTGCCG
Sequence number 3029: Rubella virus Whole genome #12 
GGACGACCTGGGCATCGCCTGGCATCTCGACCATGACGGCGGCTGCCCCGCCGATTGCCGCGGAGCCG
GCGCTGGGCCCACGCCCGGCTACACCCGCCCCTGCACCACACGCATCTACCA
Sequence number 3030: Rubella virus Whole genome #13 
CGGAGCCGGCGCTGGGCCCACGCCCGGCTACACCCGCCCCTGCACCACACGCATCTACCAAGTCTTGC
CGGACACCGCCCACCCCGGGCGCCTCTACCGATGCGGGCCCCGCCTGTGGAC
Sequence number 3031: Rubella virus Whole genome #14 
AGTCTTGCCGGACACCGCCCACCCCGGGCGCCTCTACCGATGCGGGCCCCGCCTGTGGACGCGCGATT
GCGCCGTGGCCGAACTCTCATGGGAGGTTGCCCAACACTGCGGGCACCAGGC
Sequence number 3032: Rubella virus Whole genome #15 
GCGCGATTGCGCCGTGGCCGAACTCTCATGGGAGGTTGCCCAACACTGCGGGCACCAGGCGCGCGTGC
GCGCCGTGCGATGCACCCTCCCCATCCGCCACGTGCGCAGCCTCCAACCCAG
Sequence number 3033: Rubella virus Whole genome #16 
GCGCGTGCGCGCCGTGCGATGCACCCTCCCCATCCGCCACGTGCGCAGCCTCCAACCCAGCGCGCGGG
TCCGACTCCCGGACCTCGTCCATCTCGCCGAGGTGGGCCGGTGGCGGTGGTT
Sequence number 3034: Rubella virus Whole genome #17 
CGCGCGGGTCCGACTCCCGGACCTCGTCCATCTCGCCGAGGTGGGCCGGTGGCGGTGGTTCAGCCTCC
CCCGCCCCGTGTTCCAGCGCATGCTGTCCTACTGCAAGACCCTGAGCCCCGA
Sequence number 3035: Rubella virus Whole genome #18 
CAGCCTCCCCCGCCCCGTGTTCCAGCGCATGCTGTCCTACTGCAAGACCCTGAGCCCCGACGCGTACT
ACAGCGAGCGCGTGTTCAAGTTTAAGAACGCTCTGAGCCACAGCATCACGCT
Sequence number 3036: Rubella virus Whole genome #19 CGCGTACTACAGCGAGCGCGTGTTCAAGTTTAAGAACGCTCTGAGCCACAGCATCACGCTCGCGGGCA ATGTGCTGCAAGAAGGGTGGAAGGGCACGTGCGCCGAGGAAGACGCGCTGTG
Sequence number 3037: Rubella virus Whole genome #20 
CGCGGGCAATGTGCTGCAAGAAGGGTGGAAGGGCACGTGCGCCGAGGAAGACGCGCTGTGCGCATACG
TGGCCTTCCGCGCGTGGCAGTCTAACGCCAGGTTGGCGGGGATCATGAAAGG
Sequence number 3038: Rubella virus Whole genome #21 
CGCATACGTGGCCTTCCGCGCGTGGCAGTCTAACGCCAGGTTGGCGGGGATCATGAAAGGCGCCAAGC
GCTGCGCCGCCGACTCTTTGAGCGTGGCCGGCTGGCTGGACACCATTTGGGA
Sequence number 3039: Rubella virus Whole genome #22 
CGCCAAGCGCTGCGCCGCCGACTCTTTGAGCGTGGCCGGCTGGCTGGACACCATTTGGGACGCCATTA
AGCGATTCTTCGGCAGCGTGCCCCTCGCCGAGCGCATGGAGGAGTGGGAACA
Sequence number 3040: Rubella virus Whole genome #23 
CGCCATTAAGCGATTCTTCGGCAGCGTGCCCCTCGCCGAGCGCATGGAGGAGTGGGAACAGGACGCCG
CGGTCGCCGCCTTCGACCGCGGCCCCCTCGAGGACGGCGGGCGCCACTTGGA
Sequence number 3041: Rubella virus Whole genome #24 
GGACGCCGCGGTCGCCGCCTTCGACCGCGGCCCCCTCGAGGACGGCGGGCGCCACTTGGACACCGTGC
AACCCCCAAAATCGCCGCCCCGCCCTGAGATCGCCGCGACCTGGATCGTTCA
Sequence number 3042: Rubella virus Whole genome #25 
CACCGTGCAACCCCCAAAATCGCCGCCCCGCCCTGAGATCGCCGCGACCTGGATCGTTCACGCAGCCA
GCGCAGACCGCCATTGCGCGTGCGCTCCCCGCTGCGACGCCCCGCGCGAACG
Sequence number 3043: Rubella virus Whole genome #26 
CGCAGCCAGCGCAGACCGCCATTGCGCGTGCGCTCCCCGCTGCGACGCCCCGCGCGAACGTCCTTCCG
CGCCCGCCGGCCCGCCCGATGACGAGGCGCTCATCCCGCCGTGGCTGTTCGC
Sequence number 3044: Rubella virus Whole genome #27 
TCCTTCCGCGCCCGCCGGCCCGCCCGATGACGAGGCGCTCATCCCGCCGTGGCTGTTCGCCGAGCGCC
GTGCCCTCCGCTGCCGCGAGTGGGACTTCGAGGCTCTCCGCGCGCGCGCCGA
Sequence number 3045: Rubella virus Whole genome #28 
CGAGCGCCGTGCCCTCCGCTGCCGCGAGTGGACTTCGAGGCTCTCCGCGCGCGCCGATACGGCGG
CCGCGCCCGCCCCGCTGGCCCACGCCCTGCGCGGTACCCCACTGTGCTCTA
Sequence number 3046: Rubella virus Whole genome #29 
TACGGCGGCCGCGCCCGCCCCGCTGGCCCCACGCCCTGCGCGGTACCCCACTGTGCTCTACCGCCACC
CCGCCCACCACGGCCCGTGGCTCACCCTTGACGAGCCGGGCGAGGCTGACGC
Sequence number 3047: Rubella virus Whole genome #30 
CCGCCACCCCGCCCACCACGGCCCGTGGCTCACCCTTGACGAGCCGGGCGAGGCTGACGCGGCCCTGG
TCTTATGCGACCCGCTCGGCCAGCCGCTCCGGGGCCCCGAACGCCATTTCGC
Sequence number 3048: Rubella virus Whole genome #31 
GGCCCTGGTCTTATGCGACCCGCTCGGCCAGCCGCTCCGGGGCCCCGAACGCCATTTCGCCGCCGGCG
CGCATATGTGCGCGCAGGCGCGGGGGCTCCAGGCTTTCGTCCGTGTCGTGCC
Sequence number 3049: Rubella virus Whole genome #32 
CGCCGGCGCGCATATGTGCGCGCAGGCGCGGGGGCTCCAGGCTTTCGTCCGTGTCGTGCCTCCACCCG
AGCGCCCCTGGGCCGACGGGGGCGCCAGAGCGTGGGCAAAGTTCTTCCGCGG
Sequence number 3050: Rubella virus Whole genome #33 
TCCACCCGAGCGCCCCTGGGCCGACGGGGGCGCCAGAGCGTGGGCAAAGTTCTTCCGCGGCTGCGCCT
GGGCGCAGCGCTTGCTCGGCGAGCCGGCAGTCATGCACCTCCCATACACCGA
Sequence number 3051: Rubella virus Whole genome #34 
CTGCGCCTGGGCGCAGCGCTTGCTCGGCGAGCCGGCAGTCATGCACCTCCCATACACCGATGGCGATG
TGCCACAGCTGATCGCGCTGGCCTTGCGCACGCTGGCCCAACAGGGGGCCGC
Sequence number 3052: Rubella virus Whole genome #35 
TGGCGATGTGCCACAGCTGATCGCGCTGGCCTTGCGCACGCTGGCCCAACAGGGGGCCGCCTTGGCAC
TCTCCGTGCGTGACCTGCCCGGGGGTGCAGCGTTCGACGCAAATGCGGTCAC
Sequence number 3053: Rubella virus Whole genome #36 
CTTGGCACTCTCCGTGCGTGACCTGCCCGGGGGTGCAGCGTTCGACGCAAATGCGGTCACCGCCGCTG
TGCGCGCTGGCCCCGGCCAGTCCGCGGCCACGTCACCGCCACCTGGCGACCC
Sequence number 3054: Rubella virus Whole genome #37 
CGCCGCTGTGCGCGCTGGCCCCGGCCAGTCCGCGGCCACGTCACCGCCACCTGGCGACCCCCCGCCGC
CGCGCCGCGCACGGCGTTCGCAACGGCACTCGGACGCCCGCGGCACTCCGCC
Sequence number 3055: Rubella virus Whole genome #38 
CCCGCCGCCGCGCCGCGCACGGCGTTCGCAACGGCACTCGGACGCCCGCGGCACTCCGCCCCCCGCGC
CTGCGCCCGACCCGCCGCCGCCCGCCCCCAGCCCGCCCGCGCCACCCCGCGC
Sequence number 3056: Rubella virus Whole genome #39 
CCCCGCGCCTGCGCCCGACCCGCCGCCGCCCGCCCCCAGCCCGCCCGCGCCACCCCGCGCGGGTGACT
CGGCCCCTCCCACCCCTGCGGGGCCGGCGGATCGCGCGCGCGACGCCGAGCT
Sequence number 3057: Rubella virus Whole genome #40 
GGGTGACTCGGCCCCTCCCACCCCTGCGGGGCCGGCGGATCGCGCGCGCGACGCCGAGCTGGAGGTCG
CCTGCGAACCGAGCGGCCCCCCCGCGGCAGCCAAGGCAGACCCAGACAGCGA
Sequence number 3058: Rubella virus Whole genome #41 
GGAGGTCGCCTGCGAACCGAGCGGCCCCCCCGCGGCAGCCAAGGCAGACCCAGACAGCGACATCGTTG
AGAGTTACGCCCGCGCCGCCGGACCCGTGCACCTCCGAGTCCGCGACATCAT
Sequence number 3059: Rubella virus Whole genome #42 
CATCGTTGAGAGTTACGCCCGCGCCGCCGGACCCGTGCACCTCCGAGTCCGCGACATCATGGACCCAC
CGCCCGGCTGCAAGGTTGTGGTCAACGCCGCCAACGAGGGGCTGCTGGCCGG
Sequence number 3060: Rubella virus Whole genome #43 
GGACCCACCGCCCGGCTGCAAGGTTGTGGTCAACGCCGCCAACGAGGGGCTGCTGGCCGGCTCTGGCG
TGTGTGGCGCCATCTTTGCCAACGCCACGGCGGCCCTCGCTGCAGACTGCCG
Sequence number 3061: Rubella virus Whole genome #44 
CTCTGGCGTGTGTGGCGCCATCTTTGCCAACGCCACGGCGGCCCTCGCTGCAGACTGCCGGCGCCTCG
CCCCATGCCCCACCGGCGAGGCAGTGGCGACACCCGGCCATGGCTGCGGGTA
Sequence number 3062: Rubella virus Whole genome #45 
GCGCCTCGCCCCATGCCCCACCGGCGAGGCAGTGGCGACACCCGGCCATGGCTGCGGGTACACCCACA
TCATCCACGCCGTTGCGCCGCGGCGTCCTCGGGACCCCGCCGCCCTCGAGGA
Sequence number 3063: Rubella virus Whole genome #46 
CACCCACATCATCCACGCCGTTGCGCCGCGGCGTCCTCGGGACCCCGCCGCCCTCGAGGAGGGCGAAG
CACTGCTCGAGCGCGCCTACCGCAGCATCGTCGCGCTGGCCGCTGCGCGCAG
Sequence number 3064: Rubella virus Whole genome #47 GGGCGAAGCACTGCTCGAGCGCGCCTACCGCAGCATCGTCGCGCTGGCCGCTGCGCGCAGGTGGGCGT GTGTCGCGTGCCCCCTCCTCGGCGCTGGCGTCTACGGCTGGTCTGCTGCGGA
Sequence number 3065: Rubella virus Whole genome #48 
GTGGGCGTGTGTCGCGTGCCCCCTCCTCGGCGCTGGCGTCTACGGCTGGTCTGCTGCGGAGTCCCTCC
GAGCCGCGCTTGCGGCTACGCGCGCCGAGCCCGCCGAGCGCGTGAGCCTGCA
Sequence number 3066: Rubella virus Whole genome #49 
GTCCCTCCGAGCCGCGCTTGCGGCTACGCGCGCCGAGCCCGCCGAGCGCGTGAGCCTGCACATCTGCC
ACCCCGACCGCGCCACGCTGACGCACGCCTCCGTGCTCGTCGGCGCGGGGCT
Sequence number 3067: Rubella virus Whole genome #50 
CATCTGCCACCCCGACCGCGCCACGCTGACGCACGCCTCCGTGCTCGTCGGCGCGGGGCTCGCTGCCA
GGCGCGTTAGTCCTCCCCCGGCCGAGCCCCTCGCACCTTGCCCCGCCGGTGC
Sequence number 3068: Rubella virus Whole genome #51 
CGCTGCCAGGCGCGTTAGTCCTCCCCCGGCCGAGCCCCTCGCACCTTGCCCCGCCGGTGCTCCGGGCC
CGCCGGCTCAGCGCAGCTCGTCGCCTCCAGCGGCACCCCTTGGGGATGCCAC
Sequence number 3069: Rubella virus Whole genome #52 
TCCGGGCCCGCCGGCTCAGCGCAGCTCGTCGCCTCCAGCGGCACCCCTTGGGGATGCCACCGCGCCCG
AGCCTCGCGGATGCCAGGGGTGCGAACTCTGCCGGTACACGCGCGTCACCAA
Sequence number 3070: Rubella virus Whole genome #53 
CGCGCCCGAGCCTCGCGGATGCCAGGGGTGCGAACTCTGCCGGTACACGCGCGTCACCAACGACCGCG
CCTATGTCAACCTGTGGCTCGAACGCGACCGCGGCGCCACCAGCTGGGCGAT
Sequence number 3071: Rubella virus Whole genome #54 
CGACCGCGCCTATGTCAACCTGTGGCTCGAACGCGACCGCGGCGCCACCAGCTGGGCGATGCGCATTC
CCGAGGTGGTCGTCTACGGCCCGGAGCACCTCGCCACGCATTTCCCATTAAA
Sequence number 3072: Rubella virus Whole genome #55 
GCGCATTCCCGAGGTGGTCGTCTACGGCCCGGAGCACCTCGCCACGCATTTCCCATTAAACCACTACA
GTGTGCTCAAGCCCGCGGAGGTTAGGCCCCCGCGAGGCATGTGCGGGAGTGA
Sequence number 3073: Rubella virus Whole genome #56 
CCACTACAGTGTGCTCAAGCCCGCGGAGGTTAGGCCCCCGCGAGGCATGTGCGGGAGTGACATGTGGC
GCTGCCGCGGCTGGCAGGGCATGCCGCAAGTGCGCTGCACCCCCTCCAACGC
Sequence number 3074: Rubella virus Whole genome #57 
CATGTGGCGCTGCCGCGGCTGGCAGGGCATGCCGCAAGTGCGCTGCACCCCCTCCAACGCTCACGCCG
CCCTGTGCCGCACAGGCGTGCCCCCTCGAGTGAGCACGCGAGGCAGCGAGCT
Sequence number 3075: Rubella virus Whole genome #58 
TCACGCCGCCCTGTGCCGCACAGGCGTGCCCCCTCGAGTGAGCACGCGAGGCAGCGAGCTAGACCCAA
ACACCTGCTGGCTCCGCGCCGCCGCCAACGTTGCGCAGGCTGCGCGCCTG
Sequence number 3076: Rubella virus Whole genome #59 
AGACCCAAACACCTGCTGGCTCCGCGCCGCCGCCAACGTTGCGCAGGCTGCGCGCGCCTGCGGCGCCT
ACACGAGTGCCGGGTGCCCCAAGTGCGCCTACGGCCGCGCCCTGAGCGAAGC
Sequence number 3077: Rubella virus Whole genome #60 
CGGCGCCTACACGAGTGCCGGGTGCCCCAAGTGCGCCTACGGCCGCGCCCTGAGCGAAGCCCGCACTC
ACGAGGACTTTGCCGCCCTGAGCCAGCGGTGGAGCGCTAGCCACGCCGATGC
Sequence number 3078: Rubella virus Whole genome #61 
CCGCACTCACGAGGACTTTGCCGCCCTGAGCCAGCGGTGGAGCGCTAGCCACGCCGATGCCTCCCCTG
ACGGCACCGGAGATCCCCTCGACCCCCTGATGGAGACCGTGGGATGCGCCTG
Sequence number 3079: Rubella virus Whole genome #62 
CTCCCCTGACGGCACCGGAGATCCCCTCGACCCCCTGATGGAGACCGTGGGATGCGCCTGTTCGCGCG
TGTGGGTCGGCTCCGAGCATGAGGCCCCGCCCGACCACCTCCTCGTGTCCCT
Sequence number 3080: Rubella virus Whole genome #63 
TTCGCGCGTGTGGGTCGGCTCCGAGCATGAGGCCCCGCCCGACCACCTCCTCGTGTCCCTCCACCGTG
CCCCCAACGGCCCCTGGGGCGTAGTGCTCGAGGTGCGCGCGCGCCCCGAAGG
Sequence number 3081: Rubella virus Whole genome #64 
CCACCGTGCCCCCAACGGCCCCTGGGGCGTAGTGCTCGAGGTGCGCGCGCGCCCCGAAGGGGGCAACC
CCACCGGCCATTTCGTCTGCGCGGTTGGCGGTGGCCCACGCCGCGTCTCGGA
Sequence number 3082: Rubella virus Whole genome #65 
GGGCAACCCCACCGGCCATTTCGTCTGCGCGGTTGGCGGTGGCCCACGCCGCGTCTCGGACCGTCCCC
ACCTTTGGCTCGCGGTCCCCCTGTCTCGGGGCGGCGGCACCTGTGCCGCGAC
Sequence number 3083: Rubella virus Whole genome #66 
CCGTCCCCACCTTTGGCTCGCGGTCCCCCTGTCTCGGGGCGGCGGCACCTGTGCCGCGACCGACGAGG
GGCTGGCCCAGGCGTACTACGACGACCTCGAGGTGCGTCGCCTCGGGGATGA
Sequence number 3084: Rubella virus Whole genome #67 
CGACGAGGGGCTGGCCCAGGCGTACTACGACGACCTCGAGGTGCGTCGCCTCGGGGATGACGCCATGG
CCCGGGCGGCCCTCGCATCAGTCCAACGCCCTCGCAAAGGCCCCTACAACAT
Sequence number 3085: Rubella virus Whole genome #68 
CGCCATGGCCCGGGCGGCCCTCGCATCAGTCCAACGCCCTCGCAAAGGCCCCTACAACATCAGAGTAT
GGAACATGGCCGCGGGCGCTGGCAAGACCACCCGCATCCTCGCTGCCTTCAC
Sequence number 3086: Rubella virus Whole genome #69 
CAGAGTATGGAACATGGCCGCGGGCGCTGGCAAGACCACCCGCATCCTCGCTGCCTTCACGCGCGAAG
ACCTTTACGTCTGCCCCACCAATGCGCTCCTGCACGAGATTCAGGCCAAACT
Sequence number 3087: Rubella virus Whole genome #70 
GCGCGAAGACCTTTACGTCTGCCCCACCAATGCGCTCCTGCACGAGATTCAGGCCAAACTCCGCGCGC
GCGACATCGACATCAAGAACGCCGCCACCTACGAACGCGCGCTGACCAAACC
Sequence number 3088: Rubella virus Whole genome #71 
CCGCGCGCGCGACATCGACATCAAGAACGCCGCCACCTACGAACGCGCGCTGACCAAACCGCTCGCCG
CCTACCGCCGCATCTACATCGATGAGGCGTTCACCCTCGGCGGTGAGTACTG
Sequence number 3089: Rubella virus Whole genome #72 
GCTCGCCGCCTACCGCCGCATCTACATCGATGAGGCGTTCACCCTCGGCGGTGAGTACTGCGCGTTCG
TTGCCAGTCAAACCACCGCGGAGGTGATCTGCGTCGGTGACCGAGACCAGTG
Sequence number 3090: Rubella virus Whole genome #73 
CGCGTTCGTTGCCAGTCAAACCACCGCGGAGGTGATCTGCGTCGGTGACCGAGACCAGTGCGGCCCAC
ATTACGCCAACAACTGCCGCACCCCCGTCCCTGACCGCTGGCCTACCGAGCG
Sequence number 3091: Rubella virus Whole genome #74 
CGGCCCACATTACGCCAACAACTGCCGCACCCCCGTCCCTGACCGCTGGCCTACCGAGCGCTCACGAC
ACACTTGGCGCTTCCCCGACTGCTGGGCCGCCCGCCTGCGCGCGGGGCTCGA
Sequence number 3092: Rubella virus Whole genome #75 CTCACGACACACTTGGCGCTTCCCCGACTGCTGGGCCGCCCGCCTGCGCGCGGGGCTCGATTACGACA TCGAGGGCGAGCGCGCCGGCACCTTCGCCTGCAACCTTTGGGACGGCCGCCA
Sequence number 3093: Rubella virus Whole genome #76 
TTACGACATCGAGGGCGAGCGCGCCGGCACCTTCGCCTGCAACCTTTGGGACGGCCGCCAGGTCGACC
TTCACCTCGCCTTCTCGCGCGAAACCGTGCGCCGCCTTCACGAAGCTGGCAT
Sequence number 3094: Rubella virus Whole genome #77 
GGTCGACCTTCACCTCGCCTTCTCGCGCGAAACCGTGCGCCGCCTTCACGAAGCTGGCATACGCGCGT
ACACCGTGCGCGAGGCCCAGGGTATGAGCGTTAGCACCGCCTGCATCCACGT
Sequence number 3095: Rubella virus Whole genome #78 
ACGCGCGTACACCGTGCGCGAGGCCCAGGGTATGAGCGTTAGCACCGCCTGCATCCACGTAGGCAGAG
ACGGCACAGACGTTGCCCTGGCGCTGACACGCGACCTCGCCATCGTCAGCCT
Sequence number 3096: Rubella virus Whole genome #79 
AGGCAGAGACGGCACAGACGTTGCCCTGGCGCTGACACGCGACCTCGCCATCGTCAGCCTGACCCGGG
CCTCCGACGCACTCTACCTCCACGAGCTCGAGGACGGCTCACTGCGCGCTGC
Sequence number 3097: Rubella virus Whole genome #80 
GACCCGGGCCTCCGACGCACTCTACCTCCACGAGCTCGAGGACGGCTCACTGCGCGCTGCGGGGCTCA
GCGCGTTCCTTGACGCCGGGGCACTGGCGGAGCTCAAGGAGGTTCCCGCCGG
Sequence number 3098: Rubella virus Whole genome #81 
GGGGCTCAGCGCGTTCCTTGACGCCGGGGCACTGGCGGAGCTCAAGGAGGTTCCCGCCGGCATCGACC
GCGTTGTCGCCGTCGAACAGGCACCACCACCGTTGCCGCCCGCCGACGGCAT
Sequence number 3099: Rubella virus Whole genome #82 
CATCGACCGCGTTGTCGCCGTCGAACAGGCACCACCACCGTTGCCGCCCGCCGACGGCATCCCCGAGG
CCCAAGACGTGCCGCCCTTCTGCCCCCGCACTCTGGAGGAGCTCGTCTTCGG
Sequence number 3100: Rubella virus Whole genome #83 
CCCCGAGGCCCAAGACGTGCCGCCCTTCTGCCCCCGCACTCTGGAGGAGCTCGTCTTCGGCCGTGCTG
GCCACCCCCACTACGCGGACCTCAACCGCGTCACTGAGGGCGAGCGGGAAGT
Sequence number 3101: Rubella virus Whole genome #84 
CCGTGCTGGCCACCCCCACTACGCGGACCTCAACCGCGTCACTGAGGGCGAGCGGGAAGTGCGGTATA
TGCGCATCTCGCGTCACCTGCTCAACAAGAATCACACCGAGATGCCCGGGAC
Sequence number 3102: Rubella virus Whole genome #85 
GCGGTATATGCGCATCTCGCGTCACCTGCTCAACAAGAATCACACCGAGATGCCCGGGACGGAACGCG
TCCTCAGTGCCGTTTGCGCCGTGCGGCGCTACCGCGCGGGCGAGGATGGGTC
Sequence number 3103: Rubella virus Whole genome #86 
GGAACGCGTCCTCAGTGCCGTTTGCGCCGTGCGGCGCTACCGCGCGGGCGAGGATGGGTCGACCCTTC
GCACTGCTGTGGCCCGCCAGCACCCGCGCCCTTTCCGCCAGATCCCACCCCC
Sequence number 3104: Rubella virus Whole genome #87 
GACCCTTCGCACTGCTGTGGCCCGCCAGCACCCGCGCCCTTTCCGCCAGATCCCACCCCCGCGCGTCA
CCGCCGGGGTCGCTCAGGAGTGGCGCATGACGTACTTGCGGGAACGGATCGA
Sequence number 3105: Rubella virus Whole genome #88 
GCGCGTCACCGCCGGGGTCGCTCAGGAGTGGCGCATGACGTACTTGCGGGAACGGATCGACCTCACTG
ATGTCTACACGCAGATGGGCGTGGCCGCGCGGGAGCTGACCGACCGCTACGC
Sequence number 3106: Rubella virus Whole genome #89 
CCTCACTGATGTCTACACGCAGATGGGCGTGGCCGCGCGGGAGCTGACCGACCGCTACGCGCGCCGCT
ACCCCGAGATTTTCGCCGGCATGTGTACCGCCCAGAGCCTGAGCGTCCCCGC
Sequence number 3107: Rubella virus Whole genome #90 
GCGCCGCTACCCCGAGATTTTCGCCGGCATGTGTACCGCCCAGAGCCTGAGCGTCCCCGCCTTCCTCA
AGGCCACCCTGAAGTGCGTAGACGCCGCCCTCGGCCCCAGGGACACCGAGGA
Sequence number 3108: Rubella virus Whole genome #91 
CTTCCTCAAGGCCACCCTGAAGTGCGTAGACGCCGCCCTCGGCCCCAGGGACACCGAGGACTGCCACG
CCGCTCAGGGGAAAGCCGGCCTTGAGATTCGGGCGTGGGCCAAGGAGTGGGT
Sequence number 3109: Rubella virus Whole genome #92 
CTGCCACGCCGCTCAGGGGAAAGCCGGCCTTGAGATTCGGGCGTGGGCCAAGGAGTGGGTTCAGGTCA
TGTCCCCGCATTTCCGTGCGATCCAGAAGATCATCATGCGCGCCCTGCGCCC
Sequence number 3110: Rubella virus Whole genome #93 
TCAGGTCATGTCCCCGCATTTCCGTGCGATCCAGAAGATCATCATGCGCGCCCTGCGCCCGCAATTCC
TTGTGGCCGCTGGCCATACGGAGCCCGAGGTCGATGCGTGGTGGCAGGCTCA
Sequence number 3111: Rubella virus Whole genome #94 
GCAATTCCTTGTGGCCGCTGGCCATACGGAGCCCGAGGTCGATGCGTGGTGGCAGGCTCACTACACCA
CCAATGCCATCGAAGTCGACTTCACCGAGTTCGACATGAACCAGACCCTCGC
Sequence number 3112: Rubella virus Whole genome #95 
CTACACCACCAATGCCATCGAAGTCGACTTCACCGAGTTCGACATGAACCAGACCCTCGCCACTCGGG
ACGTCGAGCTCGAGATTAGCGCCGCTCTCTTGGGCCTCCCCTGCGCCGAAGA
Sequence number 3113: Rubella virus Whole genome #96 
CACTCGGGACGTCGAGCTCGAGATTAGCGCCGCTCTCTTGGGCCTCCCCTGCGCCGAAGACTACCGCG
CGCTCCGCGCCGGCAGCTACTGCACCTTGCGCGAACTGGGCACCACTGAGAC
Sequence number 3114: Rubella virus Whole genome #97 
CTACCGCGCGCTCCGCGCCGGCAGCTACTGCACCTTGCGCGAACTGGGCACCACTGAGACCGGCTGCG
AGCGCACAAGCGGCGAGCCCGCCACGCTGCTGCACAACACCACCGTGGCCAT
Sequence number 3115: Rubella virus Whole genome #98 
CGGCTGCGAGCGCACAAGCGGCGAGCCCGCCACGCTGCTGCACAACACCACCGTGGCCATGTGCATGG
CTATGCGCATGGTCCCCAAAGGCGTGCGCTGGGCTGGGATCTTCCAGGGCGA
Sequence number 3116: Rubella virus Whole genome #99 
GTGCATGGCTATGCGCATGGTCCCCAAAGGCGTGCGCTGGGCTGGGATCTTCCAGGGCGACGATATGG
TCATCTTCCTCCCTGAGGGCGCGCGCAGCGCGGCACTCAAGTGGACCCCCGC
Sequence number 3117: Rubella virus Whole genome #100 
CGATATGGTCATCTTCCTCCCTGAGGGCGCGCGCAGCGCGGCACTCAAGTGGACCCCCGCCGAGGTGG
GCTTGTTCGGCTTCCACATCCCGGTGAAGCATGTGAGCACCCCTACCCCCAG
Sequence number 3118: Rubella virus Whole genome #101 
CGAGGTGGGCTTGTTCGGCTTCCACATCCCGGTGAAGCATGTGAGCACCCCTACCCCCAGTTTCTGCG
GGCACGTCGGCACTGCGGCCGGCCTCTTCCATGATGTCATGCACCAGGCGAT
Sequence number 3119: Rubella virus Whole genome #102 
TTTCTGCGGGCACGTCGGCACTGCGGCCGGCCTCTTCCATGATGTCATGCACCAGGCGATTAAGGTGC
TTTGCCGCCGTTTCGACCCGGACGTGCTTGAAGAACAGCAGGTGGCCCTCCT
Sequence number 3120: Rubella virus Whole genome #103 TAAGGTGCTTTGCCGCCGTTTCGACCCGGACGTGCTTGAAGAACAGCAGGTGGCCCTCCTCGACCGCC TCCGGGGGGTCTACGCGGCCTTGCCTGACACCGTTGCCGCCAATGCTGCGTA
Sequence number 3121: Rubella virus Whole genome #104 
CGACCGCCTCCGGGGGGTCTACGCGGCCTTGCCTGACACCGTTGCCGCCAATGCTGCGTACTACGACT
ACAGTGCGGAGCGCGTCCTCGCCATCGTGCGCGAACTCACCGCGTACGCGCG
Sequence number 3122: Rubella virus Whole genome #105 
CTACGACTACAGTGCGGAGCGCGTCCTCGCCATCGTGCGCGAACTCACCGCGTACGCGCGGGGGCGCG
GCCTCGACCACCCGGCCACCATCGGCGCGCTCGAGGAGATCCAGACCCCCTA
Sequence number 3123: Rubella virus Whole genome #106 
GGGGCGCGGCCTCGACCACCCGGCCACCATCGGCGCGCTCGAGGAGATCCAGACCCCCTACGCGCGCG
CCAACCTCCACGACGCTGACTAACGCCCCTGTACGTGGGGCCTTTAATCTTA
Sequence number 3124: Rubella virus Whole genome #107 
CGCGCGCGCCAACCTCCACGACGCTGACTAACGCCCCTGTACGTGGGGCCTTTAATCTTACCTACTCC
AACCAGGTCATCACCCACCGTTGTTTCGCCGCATCTGGTGGGTACCGCACTC
Sequence number 3125: Rubella virus Whole genome #108 
CCTACTCCAACCAGGTCATCACCCACCGTTGTTTCGCCGCATCTGGTGGGTACCGCACTCTTGCCATT
CGGGAGAGCCCCAGGGTGCCCGAATGGCTTCCACTACCCCCATCACCATGGA
Sequence number 3126: Rubella virus Whole genome #109 
TTGCCATTCGGGAGAGCCCCAGGGTGCCCGAATGGCTTCCACTACCCCCATCACCATGGAGGACCTCC
AGAAGGCCCTCGAGGCACAATCACGCGCCCTGCGCGCGGAACTCGCTGCCGG
Sequence number 3127: Rubella virus Whole genome #110 
GGACCTCCAGAAGGCCCTCGAGGCACAATCACGCGCCCTGCGCGCGGAACTCGCTGCCGGCGCCTCGC
AGACGCGCCGGCCGCGGCCGCCGCGACAGCGCGACTCCAGCACCTCCGGAGA
Sequence number 3128: Rubella virus Whole genome #111 
CGCCTCGCAGACGCGCCGGCCGCGGCCGCCGCGACAGCGCGACTCCAGCACCTCCGGAGATGACTCCG
GCCGTGACTCCGGAGGGCCCCGCCGCCGCCGCGGCAACCGGGGCCGTGGCCA
Sequence number 3129: Rubella virus Whole genome #112 
TGACTCCGGCCGTGACTCCGGAGGGCCCCGCCGCCGCCGCGGCAACCGGGGCCGTGGCCAGCGCGGGG
ACTGGTCCAGGGCCCCGCCCCCCCCGGAAGAGCGGCAAGAAAGTCGCTCCCA
Sequence number 3130: Rubella virus Whole genome #113 
GCGCGGGGACTGGTCCAGGGCCCCGCCCCCCCCGGAAGAGCGGCAAGAAAGTCGCTCCCAGACTCCGG
CTCCGAAGCCACCGCGGGCGCCGCCACAGCAGCCTCAACCCCCGCGTATGCA
Sequence number 3131: Rubella virus Whole genome #114 
GACTCCGGCTCCGAAGCCACCGCGGGCGCCGCCACAGCAGCCTCAACCCCCGCGTATGCAAACCGGGC
GTGGGGGCTCTGCCCCGCGCCCCGAGCTGGGGCCGCCGACCAACCCGTTCCA
Sequence number 3132: Rubella virus Whole genome #115 
AACCGGGCGTGGGGGCTCTGCCCCGCGCCCCGAGCTGGGGCCGCCGACCAACCCGTTCCAAGCAGCCG
TGGCGCGTGGCCTGCGCCCGCCGCTCCACGACCCTGACACCGAGGCACCCAC
Sequence number 3133: Rubella virus Whole genome #116 
AGCAGCCGTGGCGCGTGGCCTGCGCCCGCCGCTCCACGACCCTGACACCGAGGCACCCACCGAGGCCT
GCGTAACCTCATGGCTTTGGAGCGAAGGCGAAGGCGCGGTCTTCTACCGCGT
Sequence number 3134: Rubella virus Whole genome #117 
CGAGGCCTGCGTAACCTCATGGCTTTGGAGCGAAGGCGAAGGCGCGGTCTTCTACCGCGTCGACCTAC
ACTTCACCAACCTGGGCACCCCCCCCCTCGACGAGGACGGCCGCTGGGACCC
Sequence number 3135: Rubella virus Whole genome #118 
CGACCTACACTTCACCAACCTGGGCACCCCCCCCCTCGACGAGGACGGCCGCTGGGACCCCGCGCTCA
TGTACAACCCCTGCGGGCCCGAGCCGCCCGCTCACGTCGTACGCGCGTACAA
Sequence number 3136: Rubella virus Whole genome #119 
CGCGCTCATGTACAACCCCTGCGGGCCCGAGCCGCCCGCTCACGTCGTACGCGCGTACAATCAACCCG
CCGGCGACGTCAGGGGCGTTTGGGGTAAAGGCGAGCGCACTTACGCCGAGCA
Sequence number 3137: Rubella virus Whole genome #120 
TCAACCCGCCGGCGACGTCAGGGGCGTTTGGGGTAAAGGCGAGCGCACTTACGCCGAGCAGGATTTCC
GCGTCGGTGGCACGCGCTGGCACCGACTGCTGCGCATGCCAGTGCGCGGCCT
Sequence number 3138: Rubella virus Whole genome #121 
GGATTTCCGCGTCGGTGGCACGCGCTGGCACCGACTGCTGCGCATGCCAGTGCGCGGCCTCGACGGCG
ACAGCGCCCCGCTTCCCCCCCACACCACCGAGCGCATTGAGACCCGCTCGGC
Sequence number 3139: Rubella virus Whole genome #122 
CGACGGCGACAGCGCCCCGCTTCCCCCCCACACCACCGAGCGCATTGAGACCCGCTCGGCGCGCCATC
CTTGGCGCATCCGCTTTGGCGCTCCCCAGGCCTTCCTTGCCGGGCTCTTGCT
Sequence number 3140: Rubella virus Whole genome #123 
GCGCCATCCTTGGCGCATCCGCTTTGGCGCTCCCCAGGCCTTCCTTGCCGGGCTCTTGCTCGCCGCGG
TCGCCGTCGGCACCGCGCGCGCCGGGCTCCAGCCCCGCGCTGACATGGCGGC
Sequence number 3141: Rubella virus Whole genome #124 
CGCCGCGGTCGCCGTCGGCACCGCGCGCGCCGGGCTCCAGCCCCGCGCTGACATGGCGGCCCCGCCTG
CGCCGCCGCAGCCCCCCCGTGCGCACGGGCAGCACTACGGCCATCACCACCA
Sequence number 3142: Rubella virus Whole genome #125 
CCCGCCTGCGCCGCCGCAGCCCCCCCGTGCGCACGGGCAGCACTACGGCCATCACCACCATCAGCTGC
CGTTCCTCGGGCACGACGGCCATCACGGCGGCACCCTGCGCGTCGGCCAGCA
Sequence number 3143: Rubella virus Whole genome #126 
TCAGCTGCCGTTCCTCGGGCACGACGGCCATCACGGCGGCACCCTGCGCGTCGGCCAGCATCACCGAA
ACGCCAGCGACGTGCTGCCCGGCCACTGGCTCCAAGGCGGCTGGGGCTGCTA
Sequence number 3144: Rubella virus Whole genome #127 
TCACCGAAACGCCAGCGACGTGCTGCCCGGCCACTGGCTCCAAGGCGGCTGGGGCTGCTACAACCTGA
GCGACTGGCACCAGGGCACTCATGTCTGTCACACCAAGCACATGGACTTCTG
Sequence number 3145: Rubella virus Whole genome #128 
CAACCTGAGCGACTGGCACCAGGGCACTCATGTCTGTCACACCAAGCACATGGACTTCTGGTGTGTGG
AGCACGACCGACCGCCGCCTGCGACCCCGACGCCTCTCACCACCGCGGCGAA
Sequence number 3146: Rubella virus Whole genome #129 
GTGTGTGGAGCACGACCGACCGCCGCCTGCGACCCCGACGCCTCTCACCACCGCGGCGAACGCCACGA
CCGCCGCCACCCCCACCACTGTGCCGGCCCCTTGCCACGCCGGCCTCAATGA
Sequence number 3147: Rubella virus Whole genome #130 
CGCCACGACCGCCGCCACCCCCACCACTGTGCCGGCCCCTTGCCACGCCGGCCTCAATGACAGCTGCG
GCGGCTTCTTGTCAGGGTGCGGGCCGATGCGCCTGCGCCACGGCGCCGACAC
Sequence number 3148: Rubella virus Whole genome #131 CAGCTGCGGCGGCTTCTTGTCAGGGTGCGGGCCGATGCGCCTGCGCCACGGCGCCGACACCCGGTGCG GTCGGCTGATCTGCGGGCTGTCCACCACCGCCCAGTACCCGCCTACCCGGTT
Sequence number 3149: Rubella virus Whole genome #132 
CCGGTGCGGTCGGCTGATCTGCGGGCTGTCCACCACCGCCCAGTACCCGCCTACCCGGTTCGGCTGCG
TTATGCGGTGGGGCCTTCCCCCTTGGGAACTGGTCGTCCTTACCGCCCGCCC
Sequence number 3150: Rubella virus Whole genome #133 
CGGCTGCGTTATGCGGTGGGGCCTTCCCCCTTGGGAACTGGTCGTCCTTACCGCCCGCCCCGAAGACG
GCTGGACTTGCCGCGGCGTGCCCGCCCATCCAGGTACCCGCTGCCCCAAACT
Sequence number 3151: Rubella virus Whole genome #134 
CGAAGACGGCTGGACTTGCCGCGGCGTGCCCGCCCATCCAGGTACCCGCTGCCCCAAACTGGTGAGCC
CCATGGGACGCGCGACCTGCTCCCCAGCCTCGGCCCTCTGGCTCGCCACAGC
Sequence number 3152: Rubella virus Whole genome #135 
GGTGAGCCCCATGGGACGCGCGACCTGCTCCCCAGCCTCGGCCCTCTGGCTCGCCACAGCCAATGCGC
TGTCTCTTGATCACGCCCTTGCGGCCGCCGTCCTGCTGGTCCCGTGGGTCCT
Sequence number 3153: Rubella virus Whole genome #136 
CAATGCGCTGTCTCTTGATCACGCCCTTGCGGCCGCCGTCCTGCTGGTCCCGTGGGTCCTGATATTTA
TGGTGTGCCGCCGCGCCTGCCGCCGCCGCGGCGCCGCCGCCGCCCTTACCGC
Sequence number 3154: Rubella virus Whole genome #137 
GATATTTATGGTGTGCCGCCGCGCCTGCCGCCGCCGCGGCGCCGCCGCCCTTACCGCGGTCGTCC
TGCAGGGTTACAACCCCCCCGCCTACGGCGAGGAGGCTTTCACCTACCTCTG
Sequence number 3155: Rubella virus Whole genome #138 
GGTCGTCCTGCAGGGTTACAACCCCCCCGCCTACGGCGAGGAGGCTTTCACCTACCTCTGTACTGCAC
CGGGGTGCGCCACGCAAACACCTGTCCCTGTGCGCCTCGCTGGCGTCCGCTT
Sequence number 3156: Rubella virus Whole genome #139 
TACTGCACCGGGGTGCGCCACGCAAACACCTGTCCCTGTGCGCCTCGCTGGCGTCCGCTTTGAGTCCA
AGATTGTGGACGGCGGCTGCTTTGCCCCATGGGACCTCGAGGCCACTGGAGC
Sequence number 3157: Rubella virus Whole genome #140 
TGAGTCCAAGATTGTGGACGGCGGCTGCTTTGCCCCATGGGACCTCGAGGCCACTGGAGCCTGCATTT
GCGAGATCCCCACTGACGTCTCGTGCGAGGGCTTGGGGGCCTGGGTACCCAC
Sequence number 3158: Rubella virus Whole genome #141 
CTGCATTTGCGAGATCCCCACTGACGTCTCGTGCGAGGGCTTGGGGGCCTGGGTACCCACAGCCCCTT
GCGCGCGCATTTGGAACGGCACACAGCGCGCGTGCACCTTTTGGGCCGTCAA
Sequence number 3159: Rubella virus Whole genome #142 
AGCCCCTTGCGCGCGCATTTGGAACGGCACACAGCGCGCGTGCACCTTTTGGGCCGTCAACGCCTACT
CTTCTGGTGGGTACGCGCAGCTGGCCTCTTACTTCAACCCTGGCGGTAGCTA
Sequence number 3160: Rubella virus Whole genome #143 
CGCCTACTCTTCTGGTGGGTACGCGCAGCTGGCCTCTTACTTCAACCCTGGCGGTAGCTACTACAAGC
AGTACCATCCTACCGCGTGCGAGGTTGAGCCCGCCTTCGGACACAGCGACGC
Sequence number 3161: Rubella virus Whole genome #144 
CTACAAGCAGTACCATCCTACCGCGTGCGAGGTTGAGCCCGCCTTCGGACACAGCGACGCGGCCTGCT
GGGGCTTCCCCACCGACACTGTGATGAGCGTGTTCGCCCTTGCCAGCTACGT
Sequence number 3162: Rubella virus Whole genome #145 
GGCCTGCTGGGGCTTCCCCACCGACACTGTGATGAGCGTGTTCGCCCTTGCCAGCTACGTCCAGCACC
CTCACAAGACCGTCCGGGTCAAGTTCCATACAGAGACCAGGACCGTCTGGCA
Sequence number 3163: Rubella virus Whole genome #146 
CCAGCACCCTCACAAGACCGTCCGGGTCAAGTTCCATACAGAGACCAGGACCGTCTGGCAACTCTCCG
TTGCCGGCGTGTCGTGCAATGTCACCACTGAACACCCATTCTGCAACACGCC
Sequence number 3164: Rubella virus Whole genome #147 
ACTCTCCGTTGCCGGCGTGTCGTGCAATGTCACCACTGAACACCCATTCTGCAACACGCCGCACGGAC
AACTCGAGGTCCAGGTCCCGCCCGACCCCGGGGACCTGGTTGAGTACATTAT
Sequence number 3165: Rubella virus Whole genome #148 
GCACGGACAACTCGAGGTCCAGGTCCCGCCCGACCCCGGGGACCTGGTTGAGTACATTATGAATTACA
CCGGCAATCAGCAGTCCCGGTGGGGCCTTGGGAGCCCGAATTGCCACGGCCC
Sequence number 3166: Rubella virus Whole genome #149 
GAATTACACCGGCAATCAGCAGTCCCGGTGGGGCCTTGGGAGCCCGAATTGCCACGGCCCCGACTGGG
CCTCCCCGGTTTGTCAGCGCCATTCCCCTGACTGCTCGCGGCTTGTGGGGGC
Sequence number 3167: Rubella virus Whole genome #150 
CGACTGGGCCTCCCCGGTTTGTCAGCGCCATTCCCCTGACTGCTCGCGGCTTGTGGGGGCCACGCCAG
AGCGTCCCCGGCTTCGCCTGGTCGACGCCGACGACCCCCTGCTGCGCACTGC
Sequence number 3168: Rubella virus Whole genome #151 
CACGCCAGAGCGTCCCCGGCTTCGCCTGGTCGACGCCGACGACCCCCTGCTGCGCACTGCCCCTGGGC
CCGGCGAGGTGTGGGTCACGCCTGTCATAGGCTCTCAGGCGCGCAAGTGCGG
Sequence number 3169: Rubella virus Whole genome #152 
CCCTGGGCCCGGCGAGGTGTGGGTCACGCCTGTCATAGGCTCTCAGGCGCGCAAGTGCGGACTCCACA
TACGCGCTGGACCGTACGGCCATGCTACCGTCGAAATGCCCGAGTGGATCCA
Sequence number 3170: Rubella virus Whole genome #153 
ACTCCACATACGCGCTGGACCGTACGGCCATGCTACCGTCGAAATGCCCGAGTGGATCCACGCCCACA
CCACCAGCGACCCCTGGCACCCACCGGGCCCCTTGGGGCTGAAATTCAAGAC
Sequence number 3171: Rubella virus Whole genome #154 
CGCCCACACCACCAGCGACCCCTGGCACCCACCGGGCCCCTTGGGGCTGAAATTCAAGACCGTTCGCC
CGGTTGCCCTGCCACGCGCGTTAGCGCCACCCCGCAATGTGCGTGTGACCGG
Sequence number 3172: Rubella virus Whole genome #155 
CGTTCGCCCGGTTGCCCTGCCACGCGCGTTAGCGCCACCCCGCAATGTGCGTGTGACCGGGTGTTACC
AGTGCGGTACTCCCGCGCTGGTGGAAGGCCTTGCCCCCGGGGGGGGCAATTG
Sequence number 3173: Rubella virus Whole genome #156 
GTGTTACCAGTGCGGTACTCCCGCGCTGGTGGAAGGCCTTGCCCCCGGGGGGGGCAATTGCCATCTCA
CTGTCAATGGCGAGGACGTCGGCGCCTTCCCCCCTGGGAAGTTCGTCACCGC
Sequence number 3174: Rubella virus Whole genome #157 
CCATCTCACTGTCAATGGCGAGGACGTCGGCGCCTTCCCCCCTGGGAAGTTCGTCACCGCCGCCCTCC
TCAACACCCCCCCGCCCTACCAAGTCAGCTGCGGGGGCGAGAGCGATCGCGC
Sequence number 3175: Rubella virus Whole genome #158 
CGCCCTCCTCAACACCCCCCCGCCCTACCAAGTCAGCTGCGGGGGCGAGAGCGATCGCGCGAGCGCGC
GGGTTATTGACCCCGCCGCGCAATCGTTTACCGGCGTGGTGTATGGCACACA
Sequence number 3176: Rubella virus Whole genome #159 GAGCGCGCGGGTTATTGACCCCGCCGCGCAATCGTTTACCGGCGTGGTGTATGGCACACACACCACCG CTGTGTCGGAGACCCGGCAGACCTGGGCGGAGTGGGCTGCTGCCCATTGGTG
Sequence number 3177: Rubella virus Whole genome #160 
CACCACCGCTGTGTCGGAGACCCGGCAGACCTGGGCGGAGTGGGCTGCTGCCCATTGGTGGCAGCTCA
CTCTGGGCGCCATCTGCACCCTCCTATTTGCTGGCTTACTTGCTTGCTGTGC
Sequence number 3178: Rubella virus Whole genome #161 
GCAGCTCACTCTGGGCGCCATCTGCACCCTCCTATTTGCTGGCTTACTTGCTTGCTGTGCCAAATGCT
TGTACTACTTGCGCGGCGCTATAGCGCCGCGCTAGTGGGCCCCCGCGCGAAA
Sequence number 3179: Rubella virus Whole genome #162 
CTTACTTGCTTGCTGTGCCAAATGCTTGTACTACTTGCGCGGCGCTATAGCGCCGCGCTAGTGGGCCC
CCGCGCGAAACCCGCATCAGCCCACTAGATTCCCGCACCTGTTGCTGCATAG
Sequence number 3180: Vaccinia virus B10R #1 
TGTATAAAAAACTAATAACGTTTTTATTTGTAATAGGTGCAGTAGCATCCTATTCGAATAATGAGTAC
ACTCCGTTTAATAAACTGAGTGTAAAACTCTATATAGATGGAGTAGATAATA
Sequence number 3181: Vaccinia virus B10R #2 
ATGAGTACACTCCGTTTAATAAACTGAGTGTAAAACTCTATATAGATGGAGTAGATAATATAGAAAAT
TCATATACTGATGATAATAATGAATTGGTGTTAAATTTTAAAGAGTACACAA
Sequence number 3182: Vaccinia virus B10R #3 
TAGAAAATTCATATACTGATGATAATAATGAATTGGTGTTAAATTTTAAAGAGTACACAATTTCTATT
ATTACAGAGTCATGCGACGTCGGATTTGATTCCATAGATATAGATGTTATAA
Sequence number 3183: Vaccinia virus B10R #4 
TTTCTATTATTACAGAGTCATGCGACGTCGGATTTGATTCCATAGATATAGATGTTATAAACGACTAT
AAAATTATTGATATGTATACCATTGACTCGTCTACTATTCAACGCAGAGGTC
Sequence number 3184: Vaccinia virus B10R #5 
ACGACTATAAAATTATTGATATGTATACCATTGACTCGTCTACTATTCAACGCAGAGGTCACACGTGT
AGAATATCTACCAAATTATCATGCCATTATGATAAGTACCCTTATATTCACA
Sequence number 3185: Vaccinia virus B10R #6 
ACACGTGTAGAATATCTACCAAATTATCATGCCATTATGATAAGTACCCTTATATTCACAAATATGAT
GGTGATGAGCGACAATATTCTATTACTGCAGAGGGAAAATGCTATAAAGGAA
Sequence number 3186: Vaccinia virus B10R #7 
AATATGATGGTGATGAGCGACAATATTCTATTACTGCAGAGGGAAAATGCTATAAAGGAATAAAATAT
GAAATAAGTATGATCAACGATGATACTCTATTGAGAAAACATACTCTTAAAA
Sequence number 3187: Vaccinia virus B10R #8 
TAAAATATGAAATAAGTATGATCAACGATGATACTCTATTGAGAAAACATACTCTTAAAATTGGATCT
ACTTATATATTTGATCGTCATGGACATAGTAATACATATTATTCAAAATATG
Sequence number 3188: Vaccinia virus B10R #9 
TTGGATCTACTTATATATTTGATCGTCATGGACATAGTAATACATATTATTCAAAATATGATTTTTAA
AAATTTAAAATATATTATCACTTCAGTGACAGTAGTCAAATAACAAACAACA
Sequence number 3189: Vaccinia virus B10R #10 
ATTTTTAAAAATTTAAAATATATTATCACTTCAGTGACAGTAGTCAAATAACAAACAACACCATGAGA
TATATTATAATTCTCGCAGTTTTGTTCATTAATAGTATACATGCTAAAATAA
Sequence number 3190: Vaccinia virus B10R #11 
CCATGAGATATATTATAATTCTCGCAGTTTTGTTCATTAATAGTATACATGCTAAAATAACTAGTTAT
AAGTTTGAATCCGTCAATTTTGATTCCAAAATTGAATGGACTGGGGATGGTC
Sequence number 3191: Vaccinia virus B10R #12 
CTAGTTATAAGTTTGAATCCGTCAATTTTGATTCCAAAATTGAATGGACTGGGGATGGTCTATACAAT
ATATCCCTTAAAAATTATGGCATCAAGACGTGGCAAACAATGTATACAAATG
Sequence number 3192: Vaccinia virus B10R #13 
TATACAATATATCCCTTAAAAATTATGGCATCAAGACGTGGCAAACAATGTATACAAATGTACCAGAA
GGAACATACGACATATCCGCATTTCCAAAGAATGATTTCGTATCTTTCTGGG
Sequence number 3193: Vaccinia virus B10R #14 
TACCAGAAGGAACATACGACATATCCGCATTTCCAAAGAATGATTTCGTATCTTTCTGGGTTAAATTT
GAACAAGGCGATTATAAAGTGGAAGAGTATTGTACGGGACTATGCGTCGAAG
Sequence number 3194: Vaccinia virus B10R #15 
TTAAATTTGAACAAGGCGATTATAAAGTGGAAGAGTATTGTACGGGACTATGCGTCGAAGTAAAAATT
GGACCACCGACTGTAACATTAACTGAATACGACGACCATATCAATTTGTACA
Sequence number 3195: Vaccinia virus B10R #16 
TAAAAATTGGACCACCGACTGTAACATTAACTGAATACGACGACCATATCAATTTGTACATCGAGCAT
CCGTATGCTACTAGAGGTAGCAAAAAGATTCCTATTTACAAACGCGGTGACA
Sequence number 3196: Vaccinia virus B10R #17 
TCGAGCATCCGTATGCTACTAGAGGTAGCAAAAAGATTCCTATTTACAAACGCGGTGACATGTGTGAT
ATCTACTTGTTGTATACGGCTAACTTCACATTCGGAGATTCTAAAGAACCAG
Sequence number 3197: Vaccinia virus B10R #18 
TGTGTGATATCTACTTGTTGTATACGGCTAACTTCACATTCGGAGATTCTAAAGAACCAGTACCATAT
GATATCGATGACTACGATTGCACGTCTACAGGTTGCAGCATAGACTTTGTCA
Sequence number 3198: Vaccinia virus B10R #19 
TACCATATGATATCGATGACTACGATTGCACGTCTACAGGTTGCAGCATAGACTTTGTCACAACAGAA
AAAGTGTGCGTGACAGCACAGGGAGCCACAGAAGGGTTTCTCGAAAAAATTA
Sequence number 3199: Vaccinia virus B10R #20 
CAACAGAAAAAGTGTGCGTGACAGCACAGGGAGCCACAGAAGGGTTTCTCGAAAAAATTACTCCATGG
AGTTCGGAAGTATGTCTGACACCTAAAAAGAGTGTATATACATGCGCAATTA
Sequence number 3200: Vaccinia virus B10R #21 
CTCCATGGAGTTCGGAAGTATGTCTGACACCTAAAAAGAGTGTATATACATGCGCAATTAGATCCAAA
GAAGATGTTCCCAATTTCAAGGACAAAATGGCCAGAGTTATCAAGAGAAAAT
Sequence number 3201: Vaccinia virus B10R #22 
GATCCAAAGAAGATGTTCCCAATTTCAAGGACAAAATGGCCAGAGTTATCAAGAGAAAATTTAATAAA
CAGTCTCAATCTTATTTAACTAAATTTCTCGGTAGCACATCAAATGATGTTA
Sequence number 3202: Vaccinia virus B10R #23 
TTAATAAACAGTCTCAATCTTATTTAACTAAATTTCTCGGTAGCACATCAAATGATGTTACCACTTTT
CTTAGCATGCTTAACTTGACTAAATATTCATAACTAATTTTTATTAATGATA
Sequence number 3203: Vaccinia virus B10R #24 
CCACTTTTCTTAGCATGCTTAACTTGACTAAATATTCATAACTAATTTTTATTAATGATACAAAAACG
AAATAAAACTGCATATTATACACTGGTTAACGCCCTTATAGGCTCTAACCAT
Sequence number 3204: Vaccinia virus B10R #25 CAAAAACGAAATAAAACTGCATATTATACACTGGTTAACGCCCTTATAGGCTCTAACCATTTTCAAGA TGAGGTCCCTGATTATAGTCCTTCTGTTCCCCTCTATCATCTACTCCATGTC
Sequence number 3205: Vaccinia virus B10R #26 
TTTCAAGATGAGGTCCCTGATTATAGTCCTTCTGTTCCCCTCTATCATCTACTCCATGTCTATTAGAC
GATGTGAGAAGACTGAAGAGGAAACATGGGGATTGAAAATAGGGTTGTGTAT
Sequence number 3206: Vaccinia virus B10R #27 
TATTAGACGATGTGAGAAGACTGAAGAGGAAACATGGGGATTGAAAATAGGGTTGTGTATAATTGCCA
AAGATTTTTATCCCGAAAGAACTGATTGCAGTGTTCATCTCCCAACTGCAAG
Sequence number 3207: Vaccinia virus B10R #28 
AATTGCCAAAGATTTTTATCCCGAAAGAACTGATTGCAGTGTTCATCTCCCAACTGCAAGTGAAGGAT
TGATAACTGAAGGCAATGGATTCAGGGATATACGAAACACCGATAAATTATA
Sequence number 3208: Vaccinia virus B10R #29 
TGAAGGATTGATAACTGAAGGCAATGGATTCAGGGATATACGAAACACCGATAAATTATAAAAAAAGC
AATGTGTCCGCTGTTTCCGTTAATAATACTATTTTCGTAACTGGCGGATTAT
Sequence number 3209: Vaccinia virus B10R #30 
AAAAAAGCAATGTGTCCGCTGTTTCCGTTAATAATACTATTTTCGTAACTGGCGGATTATTCATAAAT
AACTCTAATAGCACGATCGTGGTTAACAATATGGAAAAACTTGACATTTATA
Sequence number 3210: Vaccinia virus B10R #31 
TCATAAATAACTCTAATAGCACGATCGTGGTTAACAATATGGAAAAACTTGACATTTATAAAGACAAA
CAATGGTCGATTATAGAAATGCCTATGGCTAGGGTATATCACGGCATCGACT
Sequence number 3211: Vaccinia virus B10R #32 
AAGACAAACAATGGTCGATTATAGAAATGCCTATGGCTAGGGTATATCACGGCATCGACTCGACATTT
GGAATGTTATATTTTGCCGGAGGTCTATCCGTTACCGAACAATATGGTAATT
Sequence number 3212: Vaccinia virus B10R #33 
CGACATTTGGAATGTTATATTTTGCCGGAGGTCTATCCGTTACCGAACAATATGGTAATTTAGAGAAA
AACAACGAGATATCTTGTTACAATCCTAGAACGAATAAGTGGTTTGATATTT
Sequence number 3213: Vaccinia virus B10R #34 
TAGAGAAAAACAACGAGATATCTTGTTACAATCCTAGAACGAATAAGTGGTTTGATATTTCATATACT
ATTTATAAGATATCCATATCATCATTGTGTAAACTAAATAACGTCTTCTATG
Sequence number 3214: Vaccinia virus B10R #35 
CATATACTATTTATAAGATATCCATATCATCATTGTGTAAACTAAATAACGTCTTCTATGTATTTAGT
AAGGACATTGGATATGTGGAAAAGTATGATGGTGCATGGAAGTTAGTACATG
Sequence number 3215: Vaccinia virus B10R #36 
TATTTAGTAAGGACATTGGATATGTGGAAAAGTATGATGGTGCATGGAAGTTAGTACATGATCGTCTC
CCCGCTATAAAGGCATTATCAACTTCTCCTTATTGATTGAAAATGAAAATAT
Sequence number 3216: Vaccinia virus B10R #37 
ATCGTCTCCCCGCTATAAAGGCATTATCAACTTCTCCTTATTGATTGAAAATGAAAATATAAATAGTT
TTTATGTATAGCAGTATTACCCTATAGTTTTATTGCTTACTACTAACATGGA
Sequence number 3217: Vaccinia virus B10R #38 
AAATAGTTTTTATGTATAGCAGTATTACCCTATAGTTTTATTGCTTACTACTAACATGGATACAGATG
TTACAAATGTAGAAGATATCATAAATGAAATAGATAGAGAGAAAGAAGAAAT
Sequence number 3218: Vaccinia virus B10R #39 
TACAGATGTTACAAATGTAGAAGATATCATAAATGAAATAGATAGAGAGAAAGAAGAAATACTAAAAA
ATGTAGAAATTGAAAATAATAAAAACATTAACAAGAATCATCCCAATGAATA
Sequence number 3219: Vaccinia virus B10R #40 
ACTAAAAAATGTAGAAATTGAAAATAATAAAAACATTAACAAGAATCATCCCAATGAATATATTAGAG
AAGCACTCGTTATTAATACCAGTAGTAATAGTGATTCCATTGATAAAGAAGT
Sequence number 3220: Vaccinia virus B10R #41 
TATTAGAGAAGCACTCGTTATTAATACCAGTAGTAATAGTGATTCCATTGATAAAGAAGTTATAGAAT
GTATCTGTCACGATGTAGGAATATAGATCATATCTACTAATTTTTATAATCG
Sequence number 3221: Vaccinia virus B10R #42 
TATAGAATGTATCTGTCACGATGTAGGAATATAGATCATATCTACTAATTTTTATAATCGATACAAAA
CATAAAAAACAACTCGTTATTACATAGCAGGCATGGAATCCTTCAAGTATTG
Sequence number 3222: Vaccinia virus B10R #43 
ATACAAAACATAAAAAACAACTCGTTATTACATAGCAGGCATGGAATCCTTCAAGTATTGTTTTGATA
ACGATGGCAAGAAATGGATTATCGGAAATACTTTATATTCTGGTAATTCAAT
Sequence number 3223: Vaccinia virus B10R #44 
TTTTGATAACGATGGCAAGAAATGGATTATCGGAAATACTTTATATTCTGGTAATTCAATACTATATA
AGGTCAGAAAAAATTTCACTAGTTCGTTCTACAATTACGTAATGAAGATAGA
Sequence number 3224: Vaccinia virus B10R #45 
ACTATATAAGGTCAGAAAAAATTTCACTAGTTCGTTCTACAATTACGTAATGAAGATAGATCACAAAT
CACACAAGCCATTGTTGTCTGAAATACGATTCTATATATCTGTATTGGATCC
Sequence number 3225: Vaccinia virus B10R #46 
TCACAAATCACACAAGCCATTGTTGTCTGAAATACGATTCTATATATCTGTATTGGATCCTTTGACTA
TCGACAACTGGACACGGGAACGTGGTATAAAGTATTTGGCTATTCCAGATCT
Sequence number 3226: Vaccinia virus B10R #47 
TTTGACTATCGACAACTGGACACGGGAACGTGGTATAAAGTATTTGGCTATTCCAGATCTGTATGGAA
TTGGAGAAACCGATGATTATATGTTCTTCGTTATAAAAAATTCGGGAAGAGT
Sequence number 3227: Vaccinia virus B10R #48 
GTATGGAATTGGAGAAACCGATGATTATATGTTCTTCGTTATAAAAAATTCGGGAAGAGTATTCGCCC
CAAAGGATACTGAATCAGTCTTCGAAGCATGTGTCACTATGATAAACACGTT
Sequence number 3228: Vaccinia virus B10R #49 
ATTCGCCCCAAAGGATACTGAATCAGTCTTCGAAGCATGTGTCACTATGATAAACACGTTAGAGTTTA
TACACTCTCGAGGATTTACCCATGGAAAAATAGAACCGAGGAATATACTGAT
Sequence number 3229: Vaccinia virus B10R #50 
AGAGTTTATACACTCTCGAGGATTTACCCATGGAAAAATAGAACCGAGGAATATACTGATTAGAAATA
AACGTCTTTCACTAATTGACTATTCTAGAACTAACAAACTATACAAGAGTGG
Sequence number 3230: Vaccinia virus B10R #51 
TAGAAATAAACGTCTTTCACTAATTGACTATTCTAGAACTAACAAACTATACAAGAGTGGAAACTCAC
ATATAGATTACAACGAGGACATGATAACTTCAGGAAATATCAATTATATGTG
Sequence number 3231: Vaccinia virus B10R #52 
AAACTCACATATAGATTACAACGAGGACATGATAACTTCAGGAAATATCAATTATATGTGTGTAGACA
ATCATCTTGGAGCAACAGTTTCAAAACGAGGAGATTTAGAAATGTTGGGATA
Sequence number 3232: Vaccinia virus B10R #53 TGTAGACAATCATCTTGGAGCAACAGTTTCAAAACGAGGAGATTTAGAAATGTTGGGATATTGCATGA TAGAATGGTTCGGTGGCAAACTTCCATGGAAAAACGAAAGTAGTATAAAAGT
Sequence number 3233: Vaccinia virus B10R #54 
TTGCATGATAGAATGGTTCGGTGGCAAACTTCCATGGAAAAACGAAAGTAGTATAAAAGTAATAAAAC
AAAAAAAAGAATATAAAAAATTTATAGCTACTTTCTTTGAGGACTGTTTTCC
Sequence number 3234: Vaccinia virus B10R #55 
AATAAAACAAAAAAAAGAATATAAAAAATTTATAGCTACTTTCTTTGAGGACTGTTTTCCTGAAGGAA
ATGAACCTCTGGAATTAGTTAGATATATAGAATTAGTATACACGTTAGATTA
Sequence number 3235: Vaccinia virus B10R #56 
TGAAGGAAATGAACCTCTGGAATTAGTTAGATATATAGAATTAGTATACACGTTAGATTATTCTCAAA
CTCCTAATTATGACAGACTACGTAAACTGTTTATACAAGATTGAAATTATAT
Sequence number 3236: Vaccinia virus B10R #57 
TTCTCAAACTCCTAATTATGACAGACTACGTAAACTGTTTATACAAGATTGAAATTATATTCTTTTTT
TATAGAGTGTGGTAGTGTTACGGATATCTAATATTAATATTAGACTATCTCT
Sequence number 3237: Vaccinia virus B10R #58 
TCTTTTTTTATAGAGTGTGGTAGTGTTACGGATATCTAATATTAATATTAGACTATCTCTATCGCGCT
ACACGACCAATATCGATTACTATGGATATCTTCAGGGAAATCGCATCTTCTA
Sequence number 3238: Vaccinia virus B10R #59 
ATCGCGCTACACGACCAATATCGATTACTATGGATATCTTCAGGGAAATCGCATCTTCTATGAAAGGA
GAGAATGTATTTATTTCTCCAGCGTCAATCTCGTCAGTATTGACAATACTGT
Sequence number 3239: Vaccinia virus B10R #60 
TGAAAGGAGAGAATGTATTTATTTCTCCAGCGTCAATCTCGTCAGTATTGACAATACTGTATTATGGA
GCTAATGGATCCACTGCTGAACAGCTATCAAAATATGTAGAAACGGAGGAGA
Sequence number 3240: Vaccinia virus B10R #61 
ATTATGGAGCTAATGGATCCACTGCTGAACAGCTATCAAAATATGTAGAAACGGAGGAGAACACGGAT
AAGGTTAGCGCTCAGAATATCTCATTCAAATCCATGAATAAAGTATATGGGC
Sequence number 3241: Vaccinia virus B10R #62 
ACACGGATAAGGTTAGCGCTCAGAATATCTCATTCAAATCCATGAATAAAGTATATGGGCGATATTCT
GCCGTGTTTAAAGATTCCTTTTTGAGAAAAATTGGCGATAAGTTTCAAACTG
Sequence number 3242: Vaccinia virus B10R #63 
GATATTCTGCCGTGTTTAAAGATTCCTTTTTGAGAAAAATTGGCGATAAGTTTCAAACTGTTGACTTC
ACTGATTGTCGCACTATAGATGCAATCAACAAGTGTGTAGATATCTTTACTG
Sequence number 3243: Vaccinia virus B10R #64 
TTGACTTCACTGATTGTCGCACTATAGATGCAATCAACAAGTGTGTAGATATCTTTACTGAGGGGAAA
ATCAATCCACTATTGGATGAACCATTGTCTCCTAGCAATTAGTGCCGTATAC
Sequence number 3244: Vaccinia virus B10R #65 
AGGGGAAAATCAATCCACTATTGGATGAACCATTGTCTCCTAGCAATTAGTGCCGTATACTTTAAAGC
AAAATGGTTGACGCCATTCGAAAAGGAATTTACCAGTGATTATCCCTTTTAC
Sequence number 3245: Vaccinia virus B10R #66 
TTTAAAGCAAAATGGTTGACGCCATTCGAAAAGGAATTTACCAGTGATTATCCCTTTTACGTATCTCC
GACGGAAATGGTAGATGTAAGTATGATGTCTATGTACGGCAAGGCATTTAAT
Sequence number 3246: Vaccinia virus B10R #67 
GTATCTCCGACGGAAATGGTAGATGTAAGTATGATGTCTATGTACGGCAAGGCATTTAATCACGCATC
TGTAAAGGAATCATTCGGCAACTTTTCAATCATAGAACTGCCATATGTTGGA
Sequence number 3247: Vaccinia virus B10R #68 
CACGCATCTGTAAAGGAATCATTCGGCAACTTTTCAATCATAGAACTGCCATATGTTGGAGATACTAG
TATGATGGTCATTCTTCCAGACAAGATTGATGGATTAGAATCCATAGAACAA
Sequence number 3248: Vaccinia virus B10R #69 
GATACTAGTATGATGGTCATTCTTCCAGACAAGATTGATGGATTAGAATCCATAGAACAAAATCTAAC
AGATACAAATTTTAAGAAATGGTGTGACTTTATGGATGCTATGTTTATAGAT
Sequence number 3249: Vaccinia virus B10R #70 
AATCTAACAGATACAAATTTTAAGAAATGGTGTGACTTTATGGATGCTATGTTTATAGATGTTCACAT
TCCCAAGTTTAAGGTAACAGGCTCGTATAATCTGGTGGATACTCTAGTAAAG
Sequence number 3250: Vaccinia virus B10R #71 
GTTCACATTCCCAAGTTTAAGGTAACAGGCTCGTATAATCTGGTGGATACTCTAGTAAAGTCAGGACT
GACAGAGGTGTTCGGTTCAACTGGAGATTATAGCAATATGTGTAATTCAGAT
Sequence number 3251: Vaccinia virus B10R #72 
TCAGGACTGACAGAGGTGTTCGGTTCAACTGGAGATTATAGCAATATGTGTAATTCAGATGTGAGTGT
CGACGCTATGATCCACAAAACGTATATAGATGTCAATGAAGAGTATACAGAA
Sequence number 3252: Vaccinia virus B10R #73 
GTGAGTGTCGACGCTATGATCCACAAAACGTATATAGATGTCAATGAAGAGTATACAGAAGCAGCTGC
AGCAACTTGTGCACTGGTGTCAGACTGTGCATCAACAATTACAAATGAGTTC
Sequence number 3253: Vaccinia virus B10R #74 
GCAGCTGCAGCAACTTGTGCACTGGTGTCAGACTGTGCATCAACAATTACAAATGAGTTCTGTGTAGA
TCATCCGTTCATCTATGTGATTAGGCATGTTGATGGCAAAATTCTTTTCGTT
Sequence number 3254: Vaccinia virus B10R #75 
TGTGTAGATCATCCGTTCATCTATGTGATTAGGCATGTTGATGGCAAAATTCTTTTCGTTGGTAGATA
TTGCTCTCCAACAACTAATTGTTAACCATTTTTTTTAAAAAAATAGAAAAAA
Sequence number 3255: Vaccinia virus B10R #76 
GGTAGATATTGCTCTCCAACAACTAATTGTTAACCATTTTTTTTAAAAAAATAGAAAAAACATGTGGT
ATTAGTGCAGGTCGTTATTCTTCCAATTGCAATTGGTAAGATGACGGCCAAC
Sequence number 3256: Vaccinia virus B10R #77 
CATGTGGTATTAGTGCAGGTCGTTATTCTTCCAATTGCAATTGGTAAGATGACGGCCAACTTTAGTAC
CCACGTCTTTTCACCACAGCACTGTGGATGTGACAGACTGACCAGTATTGAT
Sequence number 3257: Vaccinia virus B10R #78 
TTTAGTACCCACGTCTTTTCACCACAGCACTGTGGATGTGACAGACTGACCAGTATTGATGACGTCAA
ACAATGTTTGACTGAATATATTTATTGGTCGTCCTATGCATACCGCAACAGG
Sequence number 3258: Vaccinia virus B10R #79 
GACGTCAAACAATGTTTGACTGAATATATTTATTGGTCGTCCTATGCATACCGCAACAGGCAATGCGC
TGGACAGTTGTATTCCACACTCCTCTCTTTTAGAGATGATGCGGAATTAGTG
Sequence number 3259: Vaccinia virus B10R #80 
CAATGCGCTGGACAGTTGTATTCCACACTCCTCTCTTTTAGAGATGATGCGGAATTAGTGTTCATCGA
CATTCGCGAGCTGGTAAAACATATGCCGTGGGATGATGTCAAAGATTGTGCA
Sequence number 3260: Vaccinia virus B10R #81 TTCATCGACATTCGCGAGCTGGTAAAACATATGCCGTGGGATGATGTCAAAGATTGTGCAGAAATCAT CCGTTGTTATATACCGGATGAGCAAAAAACCATCAGAGAGATTTCGGCCATC
Sequence number 3261: Vaccinia virus B10R #82 
GAAATCATCCGTTGTTATATACCGGATGAGCAAAAAACCATCAGAGAGATTTCGGCCATCATCGGACT
TTGTGCATATGCTGCTACTTACTGGGGAGGTGAAGACCATCCCACTAGTAAC
Sequence number 3262: Vaccinia virus B10R #83 
ATCGGACTTTGTGCATATGCTGCTACTTACTGGGGAGGTGAAGACCATCCCACTAGTAACAGTCTGAA
CGCATTGTTTGTGATGCTTGAGATGCTAAATTACGTGGATTATAACATCATA
Sequence number 3263: Vaccinia virus B10R #84 
GCTGCTACTTACTGGGGAGGTGAAGACCATCCCACTAGTAACAGTCTGAACGCATTGTTTGTGATGCT
TGAGATGCTAAATTACGTGGATTATAACATCATATTCCGGCGTATGAATTGA
Sequence number 3264: Yersinia pestis Chromosomal hypothetical gene #1 
TGGATATTACCGCGTTTTATCAGACGTTTTTTGATGAAGCAGATGAACTGTTGGCAGATATGGAACAG
CATTTGTTATTGCTGGACCCGCTGACACCTGACAATGAACAGCTTAATGCTA
Sequence number 3265: Yersinia pestis Chromosomal hypothetical gene #2 
TGGAACAGCATTTGTTATTGCTGGACCCGCTGACACCTGACAATGAACAGCTTAATGCTATTTTCCGT
GCTGCACATTCGATCAAGGGCGGTGCCGCTACTTTTGGTTTTACGGTATTGC
Sequence number 3266: Yersinia pestis Chromosomal hypothetical gene #3 
TTTTCCGTGCTGCACATTCGATCAAGGGCGGTGCCGCTACTTTTGGTTTTACGGTATTGCAAGAGACA
ACTCATCTATTGGAAAACCTGTTGGATGGTGCTCGCCGCGACGAGATGCGCT
Sequence number 3267: Yersinia pestis Chromosomal hypothetical gene #4 
AAGAGACAACTCATCTATTGGAAAACCTGTTGGATGGTGCTCGCCGCGACGAGATGCGCTTAAGCACT
GAGATCATCAACCTGTTTTTGGAAACGAAAGATATTATGCAGGAACAGTTGG
Sequence number 3268: Yersinia pestis Chromosomal hypothetical gene #5 
TAAGCACTGAGATCATCAACCTGTTTTTGGAAACGAAAGATATTATGCAGGAACAGTTGGACGCCTAC
AAAACCTCTCAGGAACCTCATTCGGAAAGCTTTGAGTACATTTGTCAGGCGT
Sequence number 3269: Yersinia pestis Chromosomal hypothetical gene #6 
ACGCCTACAAAACCTCTCAGGAACCTCATTCGGAAAGCTTTGAGTACATTTGTCAGGCGTTGCGCCAA
TTGGCACTTGATGCTTTAGATCAACCTACAACCGAAGATCAACCTACAACCG
Sequence number 3270: Yersinia pestis Chromosomal hypothetical gene #7 
TGCGCCAATTGGCACTTGATGCTTTAGATCAACCTACAACCGAAGATCAACCTACAACCGAAAATCAA
TCCGCTACCGATCAGCACAGTACGGCCGCCAATACGCAAGCAACGGCTTCCA
Sequence number 3271: Yersinia pestis Chromosomal hypothetical gene #8 
AAAATCAATCCGCTACCGATCAGCACAGTACGGCCGCCAATACGCAAGCAACGGCTTCCACCCGCGTA
TCATCTTCTCTCGCTGGCAACACATCACCCGCGTTGCTTCAAGGCGGGATGC
Sequence number 3272: Yersinia pestis Chromosomal hypothetical gene #9 
CCCGCGTATCATCTTCTCTCGCTGGCAACACATCACCCGCGTTGCTTCAAGGCGGGATGCGTATTCGT
TTGTCTGGCCTGAAAGCGCCAGAAATCTCTCTGATGCTGGAGGAACTGGGTA
Sequence number 3273: Yersinia pestis Chromosomal hypothetical gene #10 
GTATTCGTTTGTCTGGCCTGAAAGCGCCAGAAATCTCTCTGATGCTGGAGGAACTGGGTAATCTGGGC
GAGGTGCAAGATCCCCATCAGGGCGCTGATAGCCTTGAGGCCACCTTAATAA
Sequence number 3274: Yersinia pestis Chromosomal hypothetical gene #11 
ATCTGGGCGAGGTGCAAGATCCCCATCAGGGCGCTGATAGCCTTGAGGCCACCTTAATAACCTCCGTC
AGTGAAGAGGATATTAGCGCGGTATTGTGCTTTGTTCTGGAACCTGAGCAGA
Sequence number 3275: Yersinia pestis Chromosomal hypothetical gene #12 
CCTCCGTCAGTGAAGAGGATATTAGCGCGGTATTGTGCTTTGTTCTGGAACCTGAGCAGATTAGCTTT
GTGTCCGCAGGGGACACCGAGCAGATTTATGCTGAGCAGACCGTTGAATCAG
Sequence number 3276: Yersinia pestis Chromosomal hypothetical gene #13 
TTAGCTTTGTGTCCGCAGGGGACACCGAGCAGATTTATGCTGAGCAGACCGTTGAATCAGAACAATCA
TCTGAGCCAGAACGCTTGTTTGAGCCAGCAATAACCGAGGCATCGGTGATCG
Sequence number 3277: Yersinia pestis Chromosomal hypothetical gene #14 
AACAATCATCTGAGCCAGAACGCTTGTTTGAGCCAGCAATAACCGAGGCATCGGTGATCGCAGAATCG
GTTCAGCCGGTGGCAACACCTGCCGCAACACCTGCCGTTGAAGTCCAAAGCA
Sequence number 3278: Yersinia pestis Chromosomal hypothetical gene #15 
CAGAATCGGTTCAGCCGGTGGCAACACCTGCCGCAACACCTGCCGTTGAAGTCCAAAGCACGCTGAAA
GCTGATGTGCATGCTGCACCAGCTAACGCAGAACAGGTCAGGCCTAAAGCGA
Sequence number 3279: Yersinia pestis Chromosomal hypothetical gene #16 
CGCTGAAAGCTGATGTGCATGCTGCACCAGCTAACGCAGAACAGGTCAGGCCTAAAGCGAAAGCCAGT
GAATCGACCAGTATCCGTGTAGCGGTAGAAAAAGTGGACCAGCTGATTAACC
Sequence number 3280: Yersinia pestis Chromosomal hypothetical gene #17 
AAGCCAGTGAATCGACCAGTATCCGTGTAGCGGTAGAAAAAGTGGACCAGCTGATTAACCTGGTCGGC
GAATTGGTTATTACCCAATCGATGCTCGCTCAGCGCTCAAGTTCGCTGGATC
Sequence number 3281: Yersinia pestis Chromosomal hypothetical gene #18 
TGGTCGGCGAATTGGTTATTACCCAATCGATGCTCGCTCAGCGCTCAAGTTCGCTGGATCCGGTGATC
AACGGTGATTTACTGAATAGCATGGGGCAGTTGGAACGTAACGCCCGCGATT
Sequence number 3282: Yersinia pestis Chromosomal hypothetical gene #19 
CGGTGATCAACGGTGATTTACTGAATAGCATGGGGCAGTTGGAACGTAACGCCCGCGATTTGCAAGAA
TCCGTCATGTCTATCCGGATGATGCCAATGGAGTATGTATTTAGCCGCTTCC
Sequence number 3283: Yersinia pestis Chromosomal hypothetical gene #20 
TGCAAGAATCCGTCATGTCTATCCGGATGATGCCAATGGAGTATGTATTTAGCCGCTTCCCACGTTTG
GTCCGTGATTTAGCCAGCAAGCTCAACAAACAAGTTGAATTGACGCTGTTAG
Sequence number 3284: Yersinia pestis Chromosomal hypothetical gene #21 
CACGTTTGGTCCGTGATTTAGCCAGCAAGCTCAACAAACAAGTTGAATTGACGCTGTTAGGCAGTTCG
ACAGAACTGGATAAGAGTTTGATTGAGCGCATTATCGATCCTCTGACCCACT
Sequence number 3285: Yersinia pestis Chromosomal hypothetical gene #22 
GCAGTTCGACAGAACTGGATAAGAGTTTGATTGAGCGCATTATCGATCCTCTGACCCACTTGGTGCGC
AACAGTCTGGACCACGGTATTGAAGAGCCGGCTACTCGTATTGCTGCGGGGA
Sequence number 3286: Yersinia pestis Chromosomal hypothetical gene #23 
TGGTGCGCAACAGTCTGGACCACGGTATTGAAGAGCCGGCTACTCGTATTGCTGCGGGGAAATCGCCA
GTGGGCAACCTGACACTGTCAGCTGAACATCAGGGCGGAAATATTTGTATCG
Sequence number 3287: Yersinia pestis Chromosomal hypothetical gene #24 
AATCGCCAGTGGGCAACCTGACACTGTCAGCTGAACATCAGGGCGGAAATATTTGTATCGAAGTCATT
GATGATGGTGCTGGCCTGAATCGACAGAGAATTTTGGCGAAAGCACAGTCTC
Sequence number 3288: Yersinia pestis Chromosomal hypothetical gene #25 
AAGTCATTGATGATGGTGCTGGCCTGAATCGACAGAGAATTTTGGCGAAAGCACAGTCTCAAGGGCTG
GCAGTCAACGAAAACATGAGCGATGAAGACGTTGGGATGCTTATTTTTGCAC
Sequence number 3289: Yersinia pestis Chromosomal hypothetical gene #26 
AAGGGCTGGCAGTCAACGAAAACATGAGCGATGAAGACGTTGGGATGCTTATTTTTGCACAAGGATTC
TCTACTGCCGAACAAGTCACGGATGTGTCAGGGCGTGGTGTCGGGATGGATG
Sequence number 3290: Yersinia pestis Chromosomal hypothetical gene #27 
AAGGATTCTCTACTGCCGAACAAGTCACGGATGTGTCAGGGCGTGGTGTCGGGATGGATGTGGTCAAA
CGTAACATCCAGGAAATGGGCGGCCATGTACAGGTCAGTTTTCAGGCCGGTT
Sequence number 3291: Yersinia pestis Chromosomal hypothetical gene #28 
TGGTCAAACGTAACATCCAGGAAATGGGCGGCCATGTACAGGTCAGTTTTCAGGCCGGTTTAGGTACC
TCAATCCGTATCCTGCTGCCATTAACGCTGGCTATCCTTGATGGCATGTCGG
Sequence number 3292: Yersinia pestis Chromosomal hypothetical gene #29 
TAGGTACCTCAATCCGTATCCTGCTGCCATTAACGCTGGCTATCCTTGATGGCATGTCGGTTAAAGTC
AGCAATGAAGTCTTTATCTTGCCATTGAATGCCGTGATGGAGTCACTGCAAC
Sequence number 3293: Yersinia pestis Chromosomal hypothetical gene #30 
TTAAAGTCAGCAATGAAGTCTTTATCTTGCCATTGAATGCCGTGATGGAGTCACTGCAACCCTTGGCC
GAAGACCTACATCCATTAGCCGGTGGTGAGCGCGTGCTGCAAGTTCGCGGTG
Sequence number 3294: Yersinia pestis Chromosomal hypothetical gene #31 
CCTTGGCCGAAGACCTACATCCATTAGCCGGTGGTGAGCGCGTGCTGCAAGTTCGCGGTGAATTTCTA
CCATTGGTTGAATTGTACCGCGTCTTTGATGTTAAAAATGCCAAGACGGAAG
Sequence number 3295: Yersinia pestis Chromosomal hypothetical gene #32 
AATTTCTACCATTGGTTGAATTGTACCGCGTCTTTGATGTTAAAAATGCCAAGACGGAAGCCACGCAA
GGCATCGTTGTGATTCTACAAAGTGCCGGGCGCCGCTATGCACTGCTGGTAG
Sequence number 3296: Yersinia pestis Chromosomal hypothetical gene #33 
CCACGCAAGGCATCGTTGTGATTCTACAAAGTGCCGGGCGCCGCTATGCACTGCTGGTAGACCAACTG
ATTGGTCAGCATCAGGTGGTCGTGAAAAATCTGGAAAGTAACTATCGGAAAG
Sequence number 3297: Yersinia pestis Chromosomal hypothetical gene #34 
ACCAACTGATTGGTCAGCATCAGGTGGTCGTGAAAAATCTGGAAAGTAACTATCGGAAAGTGCCGGGT
ATTTCTGCCGCCACCATTTTGGGTGATGGTAGCGTCGCGCTAATCGTTGATG
Sequence number 3298: Yersinia pestis Chromosomal hypothetical gene #35 
TGCCGGGTATTTCTGCCGCCACCATTTTGGGTGATGGTAGCGTCGCGCTAATCGTTGATGTGTCAGCG
TTGCAGGCCTTAAACCGTGAAAAACGCGTCGCGGCTGATGAGGTGGTTACCG
Sequence number 3299: Yersinia pestis Chromosomal hypothetical gene #36 
TGTCAGCGTTGCAGGCCTTAAACCGTGAAAAACGCGTCGCGGCTGATGAGGTGGTTACCGCATAGCGC
ACAGGAATTTTTCCTTATAAAAACAGGTAATACCCTAAATAATTTGAGTGCA
Sequence number 3300: Yersinia pestis Chromosomal hypothetical gene #37 
CATAGCGCACAGGAATTTTTCCTTATAAAAACAGGTAATACCCTAAATAATTTGAGTGCAGCAAGCCA
CCACCGGCCACTTTAAATATGACGGGTATCCAGGGCGGTGAAATATGTTTCC
Sequence number 3301: Yersinia pestis Chromosomal hypothetical gene #38 
GCAAGCCACCACCGGCCACTTTAAATATGACGGGTATCCAGGGCGGTGAAATATGTTTCCGGTCCTGA
AAAACATAAATTCAACCTATTGATTGAAGGGTAAAAAACATGGCAGGACTAG
Sequence number 3302: Yersinia pestis Chromosomal hypothetical gene #39 
GGTCCTGAAAAACATAAATTCAACCTATTGATTGAAGGGTAAAAAACATGGCAGGACTAGCAACCGTC
ACGAAACTGGCTGGCGAAACGGTAGGACAAGAATTCCTGATTTTTACGCTGG
Sequence number 3303: Yersinia pestis Chromosomal hypothetical gene #40 
CAACCGTCACGAAACTGGCTGGCGAAACGGTAGGACAAGAATTCCTGATTTTTACGCTGGGCGATGAA
GAGTACGGCATCGATATTCTGAAAGTACAAGAGATCCGAGGCTATGATCAGG
Sequence number 3304: Yersinia pestis Chromosomal hypothetical gene #41 
GCGATGAAGAGTACGGCATCGATATTCTGAAAGTACAAGAGATCCGAGGCTATGATCAGGTGACTCGT
ATTGCTAACACTCCTGCGTTTATTAAAGGTGTGACCAACTTACGCGGCGTCA
Sequence number 3305: Yersinia pestis Chromosomal hypothetical gene #42 
TGACTCGTATTGCTAACACTCCTGCGTTTATTAAAGGTGTGACCAACTTACGCGGCGTCATTGTGCCT
ATCATTGATCTGCGGGTTAAATTCGCCCAGCAAGGTGTCACTTATAACGAAA
Sequence number 3306: Yersinia pestis Chromosomal hypothetical gene #43 
TTGTGCCTATCATTGATCTGCGGGTTAAATTCGCCCAGCAAGGTGTCACTTATAACGAAAATACCGTG
GTGATTGTGCTGAATTTTGGTCAACGCGTGGTGGGTATTGTCGTTGATGGTG
Sequence number 3307: Yersinia pestis Chromosomal hypothetical gene #44 
ATACCGTGGTGATTGTGCTGAATTTTGGTCAACGCGTGGTGGGTATTGTCGTTGATGGTGTGTCAGAT
GTCCTGTCGTTGACCAACGAGCAGATCCGTCCGGCACCTGAATTTGCTGTGA
Sequence number 3308: Yersinia pestis Chromosomal hypothetical gene #45 
TGTCAGATGTCCTGTCGTTGACCAACGAGCAGATCCGTCCGGCACCTGAATTTGCTGTGACTCTCGCA
ACAGAATATCTGACCGGTTTAGGCTCGCTTGGCGAAAGAATGTTGATCCTGG
Sequence number 3309: Yersinia pestis Chromosomal hypothetical gene #46 
CTCTCGCAACAGAATATCTGACCGGTTTAGGCTCGCTTGGCGAAAGAATGTTGATCCTGGTTGATATC
GAAAAACTGCTGAGCAGTGAAGAGATGTCGTTGATTGATAGTGTTTCTAAGG
Sequence number 3310: Yersinia pestis Chromosomal hypothetical gene #47 
TTGATATCGAAAAACTGCTGAGCAGTGAAGAGATGTCGTTGATTGATAGTGTTTCTAAGGCGTGATAA
TAGTCTTTTTAATATGTGACAATAGTGTTGCTAAGGTGTGATTGAAGCGTGG
Sequence number 3311: Yersinia pestis Chromosomal hypothetical gene #48 
CGTGATAATAGTCTTTTTAATATGTGACAATAGTGTTGCTAAGGTGTGATTGAAGCGTGGAGTTTACG
CACGTTGAGTGATCACCTGAGCCAATCGGCCTTAACCAAGCAGGGGAGGCGG
Sequence number 3312: Yersinia pestis Chromosomal hypothetical gene #49 
AGTTTACGCACGTTGAGTGATCACCTGAGCCAATCGGCCTTAACCAAGCAGGGGAGGCGGTCCTCCTG
CCCCTACGGCTTGCGCGAAATATTGCCCTACGGGTAAAGCGTCAGCGTCTTT
Sequence number 3313: Yersinia pestis Chromosomal hypothetical gene #50 
TCCTCCTGCCCCTACGGCTTGCGCGAAATATTGCCCTACGGGTAAAGCGTCAGCGTCTTTCGGGCTGA
TGAATAGAGGACTATTCAATGACTCCTGAAATAAAGAACTGAACCGCCATTG
Sequence number 3314: Yersinia pestis Chromosomal hypothetical gene #51 
CGGGCTGATGAATAGAGGACTATTCAATGACTCCTGAAATAAAGAACTGAACCGCCATTGCATATTGC
TCGGGCGGTTCTTTATGACAAGTCATGATGTTGAGATAGTCATTTTATTTAG
Sequence number 3315: Yersinia pestis Chromosomal hypothetical gene #52 
CATATTGCTCGGGCGGTTCTTTATGACAAGTCATGATGTTGAGATAGTCATTTTATTTAGGCTACAGC
TACTGCACGCATTCTTTTTGTTAAAATAGTGGCACAAATAAAACCAAGAAGA
Sequence number 3316: Yersinia pestis Chromosomal hypothetical gene #53 
GCTACAGCTACTGCACGCATTCTTTTTGTTAAAATAGTGGCACAAATAAAACCAAGAAGACTAAACGC
TACCATGAGTGAAAATATGTAGTTGTACCCCTGAATCCCCTCAAAATTATCT
Sequence number 3317: Yersinia pestis Chromosomal hypothetical gene #54 
CTAAACGCTACCATGAGTGAAAATATGTAGTTGTACCCCTGAATCCCCTCAAAATTATCTAATAATGA
GCCATAAAGTGCGTAGGCAAACATGGACGGCATATAGCCGATAATACACCCG
Sequence number 3318: Yersinia pestis Chromosomal hypothetical gene #55 
AATAATGAGCCATAAAGTGCGTAGGCAAACATGGACGGCATATAGCCGATAATACACCCGAAGGCCAT
TGCCGAACCTGCATGTTCACGAGAAGTACCAATTTCATCCATCGGGGCGAAG
Sequence number 3319: Yersinia pestis Chromosomal hypothetical gene #56 
AAGGCCATTGCCGAACCTGCATGTTCACGAGAAGTACCAATTTCATCCATCGGGGCGAAGAAGATTGC
GCGCTGAGAGAAGATAATGGCTCCGAAGCCGAGTGTTGCCATCATACCCAAG
Sequence number 3320: Yersinia pestis Chromosomal hypothetical gene #57 
AAGATTGCGCGCTGAGAGAAGATAATGGCTCCGAAGCCGAGTGTTGCCATCATACCCAAGTATACGTT
CATCGAATCATGGGGTAATTGGATGAATAAGATCATCGCTATGGCCGAAATT
Sequence number 3321: Yersinia pestis Chromosomal hypothetical gene #58 
TATACGTTCATCGAATCATGGGGTAATTGGATGAATAAGATCATCGCTATGGCCGAAATTAAAAATGT
CCATTTCAGGTAAACTGTCGGTGATTTTGCGACTTTGTCAGCGAGGAATCCC
Sequence number 3322: Yersinia pestis Chromosomal hypothetical gene #59 
AAAAATGTCCATTTCAGGTAAACTGTCGGTGATTTTGCGACTTTGTCAGCGAGGAATCCCCCAACAGG
TCCTCCTACCATTTTGAGACCGTACTGATTAATGATCCCGTAAGCGCCAATA
Sequence number 3323: Yersinia pestis Chromosomal hypothetical gene #60 
CCAACAGGTCCTCCTACCATTTTGAGACCGTACTGATTAATGATCCCGTAAGCGCCAATAAGAGCGAC
TGGCAATCCATAAATATCTTTGAGGAATGGTATGAAATAGGTTAAGCCGCAA
Sequence number 3324: Yersinia pestis Chromosomal hypothetical gene #61 
AGAGCGACTGGCAATCCATAAATATCTTTGAGGAATGGTATGAAATAGGTTAAGCCGCAATAGGCGGA
ATAGACAAAGAAGATACAGAAAGAAGCCAACCACAAGTTAGGGCTTTTAATA
Sequence number 3325: Yersinia pestis Chromosomal hypothetical gene #62 
TAGGCGGAATAGACAAAGAAGATACAGAAAGAAGCCAACCACAAGTTAGGGCTTTTAATAACATCTTT
GATCCCCGCCAAAACATGTTTATTAACTTTATCTGCATTATCTACCTTAATC
Sequence number 3326: Yersinia pestis Chromosomal hypothetical gene #63 
ACATCTTTGATCCCCGCCAAAACATGTTTATTAACTTTATCTGCATTATCTACCTTAATCTTGTCATC
ACTGTCTACGATAAACCAAGTAATGACGCCAACCAATATCGTGGCTATCGTG
Sequence number 3327: Yersinia pestis Chromosomal hypothetical gene #64 
TTGTCATCACTGTCTACGATAAACCAAGTAATGACGCCAACCAATATCGTGGCTATCGTGTAATAGAT
TAGCCCGGCCTGCATCCCCGCTTTACCTTCACCAAACTGAACAAAAACAAAC
Sequence number 3328: Yersinia pestis Chromosomal hypothetical gene #65 
TAATAGATTAGCCCGGCCTGCATCCCCGCTTTACCTTCACCAAACTGAACAAAAACAAACAGGGCACC
AGAAGCGATAAGTACATCAATCACACCGCGACCCGCTTCCAGAAAACCAAAC
Sequence number 3329: Yersinia pestis Chromosomal hypothetical gene #66 
AGGGCACCAGAAGCGATAAGTACATCAATCACACCGCGACCCGCTTCCAGAAAACCAAACATGCGTCC
TTGTTCTTCTTTGGTTCCCAGCAACCTTACTGCTTTCAACAGTACAGGCCAG
Sequence number 3330: Yersinia pestis Chromosomal hypothetical gene #67 
ATGCGTCCTTGTTCTTCTTTGGTTCCCAGCAACCTTACTGCTTTCAACAGTACAGGCCAGTAAACCAC
TTCCCCAAAAAATGCCATAGCACCAAAGGCGATGAGATAACCGGTAAACGGT
Sequence number 3331: Yersinia pestis Chromosomal hypothetical gene #68 
TAAACCACTTCCCCAAAAAATGCCATAGCACCAAAGGCGATGAGATAACCGGTAAACGGTGGAAGCGT
GGCTAAGTATGCGCCACAGAGGCCCACACCGATCAACCCTACGGGCAGTAAG
Sequence number 3332: Yersinia pestis Chromosomal hypothetical gene #69 
GGAAGCGTGGCTAAGTATGCGCCACAGAGGCCCACACCGATCAACCCTACGGGCAGTAAGATCTTTTT
CGAAAAACGATCAGCAATATAAAGAGAACTGAATAAGCCAACGGTCTGAATG
Sequence number 3333: Yersinia pestis Chromosomal hypothetical gene #70 
ATCTTTTTCGAAAAACGATCAGCAATATAAAGAGAACTGAATAAGCCAACGGTCTGAATGATGGCATA
TACGGTAAAACCAAAACCAATCTGTGTATTCGTTAGCCCCCAGTCTTGTTGC
Sequence number 3334: Yersinia pestis Chromosomal hypothetical gene #71 
ATGGCATATACGGTAAAACCAAAACCAATCTGTGTATTCGTTAGCCCCCAGTCTTGTTGCATAGGGAT
GTAAAACACATCTTTCATACTGGAGAGTTTGAAAATCGTCCCTCCACCCAAA
Sequence number 3335: Yersinia pestis Chromosomal hypothetical gene #72 
ATAGGGATGTAAAACACATCTTTCATACTGGAGAGTTTGAAAATCGTCCCTCCACCCAAAATGAGGAA
GCAAAGCTTTGTCCATTTATTCACAATATTCCTACCTTATTTTAGAGAGACA
Sequence number 3336: Yersinia pestis Chromosomal hypothetical gene #73 
ATGAGGAAGCAAAGCTTTGTCCATTTATTCACAATATTCCTACCTTATTTTAGAGAGACAATAGCCTC
TACTTCAATGAGAACGTCTTTTGGTAGCCGAGCGACTTCAACACATGAACGG
Sequence number 3337: Yersinia pestis Chromosomal hypothetical gene #74 
ATAGCCTCTACTTCAATGAGAACGTCTTTTGGTAGCCGAGCGACTTCAACACATGAACGGGCAGGGAA
TGTCCCTGATGGGAAATAGCCCGCGTAAATGGTATTGAATGACTGAAAATTG
Sequence number 3338: Yersinia pestis Chromosomal hypothetical gene #75 
GCAGGGAATGTCCCTGATGGGAAATAGCCCGCGTAAATGGTATTGAATGACTGAAAATTGTTCATATC
ACTGATAAAGCAAGTGGTTTTAACCACGTCATTCATATAACTACCGGTGGCT
Sequence number 3339: Yersinia pestis Chromosomal hypothetical gene #76 
TTCATATCACTGATAAAGCAAGTGGTTTTAACCACGTCATTCATATAACTACCGGTGGCTTCCACAAT
AGCCTTTAGGTTTTTCATGGCTTGAATGGTTTGCTCTGTAATATCAATACCT
Sequence number 3340: Yersinia pestis Chromosomal hypothetical gene #77 
TCCACAATAGCCTTTAGGTTTTTCATGGCTTGAATGGTTTGCTCTGTAATATCAATACCTACGACTGA
TCCATCTTTTTCACTGAAAGGGCAGCAACCGGAAATAAAAAGCAAATTACCG
Sequence number 3341: Yersinia pestis Chromosomal hypothetical gene #78 
ACGACTGATCCATCTTTTTCACTGAAAGGGCAGCAACCGGAAATAAAAAGCAAATTACCGGCGACAAC
GCCTTGGGAGTATGGTCCGATAGCTGCTGGTGCGTTTTGAGTTTTAATAATC
Sequence number 3342: Yersinia pestis Chromosomal hypothetical gene #79 
GCGACAACGCCTTGGGAGTATGGTCCGATAGCTGCTGGTGCGTTTTGAGTTTTAATAATCATTCGACG
ATTCCTGTTGTGGTGTTGGGGCAATATTTATCACGCAGTGCTTTGAGCACTT
Sequence number 3343: Yersinia pestis Chromosomal hypothetical gene #80 
ATTCGACGATTCCTGTTGTGGTGTTGGGGCAATATTTATCACGCAGTGCTTTGAGCACTTGAACCGAT
GTATAAACACAGTGGTCCTGAGCTTGCATAAGCTCTTGTAGTAACTCTGGCG
Sequence number 3344: Yersinia pestis Chromosomal hypothetical gene #81 
GAACCGATGTATAAACACAGTGGTCCTGAGCTTGCATAAGCTCTTGTAGTAACTCTGGCGATATTTTG
TCGGGGTAGTAATATTCCAGTGGTTTAATTCGGCTGGAGTCAAAAGGAGGGG
Sequence number 3345: Yersinia pestis Chromosomal hypothetical gene #82 
ATATTTTGTCGGGGTAGTAATATTCCAGTGGTTTAATTCGGCTGGAGTCAAAAGGAGGGGTTTTTACT
TCAAAAGTCCCTTTTAGTGTACTGACTTCATTGGGGTTGCAAGGTCGAGAAA
Sequence number 3346: Yersinia pestis Chromosomal hypothetical gene #83 
TTTTTACTTCAAAAGTCCCTTTTAGTGTACTGACTTCATTGGGGTTGCAAGGTCGAGAAAATGCAGAG
GCATAAGGAAAGCAGGTTTCAATATTGACCCGAGTTATGCCTGATTTTTCAA
Sequence number 3347: Yersinia pestis Chromosomal hypothetical gene #84 
ATGCAGAGGCATAAGGAAAGCAGGTTTCAATATTGACCCGAGTTATGCCTGATTTTTCAACCAATATT
CTGAAGCAATTATCAATATCGATACTCCCTTCACCGATAGGAACGCCACATA
Sequence number 3348: Yersinia pestis Chromosomal hypothetical gene #85 
CCAATATTCTGAAGCAATTATCAATATCGATACTCCCTTCACCGATAGGAACGCCACATACCACTAAT
TGCTCGTCATGTTGAGTAACAATGTGATCTTTAAAATGGGTACTGTAAGTAA
Sequence number 3349: Yersinia pestis Chromosomal hypothetical gene #86 
CCACTAATTGCTCGTCATGTTGAGTAACAATGTGATCTTTAAAATGGGTACTGTAAGTAAAGGGGGCA
AGTTGGCTGACAGTGACAACGGGCTCTTCCCAAGCCATCATGCCATTTCCTA
Sequence number 3350: Yersinia pestis Chromosomal hypothetical gene #87 
AGGGGGCAAGTTGGCTGACAGTGACAACGGGCTCTTCCCAAGCCATCATGCCATTTCCTATATCGCAA
TGTGCCCCGACCCATAGGGAGTCTACCGCTTTTACAATTTGGATAATCTCGT
Sequence number 3351: Yersinia pestis Chromosomal hypothetical gene #88 
TATCGCAATGTGCCCCGACCCATAGGGAGTCTACCGCTTTTACAATTTGGATAATCTCGTCGGCAGTT
TCATCTTCATGATTTTCAACGGCCAGACGAATTCGATTTGCCTGAAGTAGAG
Sequence number 3352: Yersinia pestis Chromosomal hypothetical gene #89 
CGGCAGTTTCATCTTCATGATTTTCAACGGCCAGACGAATTCGATTTGCCTGAAGTAGAGGAACCAGT
GATTTCAACTCATGAATAATGCGTGGATATTCTAGGGGCTCATACTGACCAC
Sequence number 3353: Yersinia pestis Chromosomal hypothetical gene #90 
GAACCAGTGATTTCAACTCATGAATAATGCGTGGATATTCTAGGGGCTCATACTGACCACGACGGAAC
ATGTAGGTTCGAATAACATCCGCACCCAGTGCAGATGCAAATTGCACGGCTT
Sequence number 3354: Yersinia pestis Chromosomal hypothetical gene #91 
GACGGAACATGTAGGTTCGAATAACATCCGCACCCAGTGCAGATGCAAATTGCACGGCTTTACTAATC
GCTTTAACCGACGTAAAACGGGTATCGATTTCACAATAAAGATTGCGTTGTT
Sequence number 3355: Yersinia pestis Chromosomal hypothetical gene #92 
TACTAATCGCTTTAACCGACGTAAAACGGGTATCGATTTCACAATAAAGATTGCGTTGTTCGATAGCG
TGTCTCACCCGTGCAAGATATGTTGGGTCATCGCTACTCAACACACCAAATT
Sequence number 3356: Yersinia pestis Chromosomal hypothetical gene #93 
CGATAGCGTGTCTCACCCGTGCAAGATATGTTGGGTCATCGCTACTCAACACACCAAATTCAGGGTGA
AGGCCCTCATCTGGAATGATATTTATTTCTACGCCATCCAGTCCTAAACTCC
Sequence number 3357: Yersinia pestis Chromosomal hypothetical gene #94 
CAGGGTGAAGGCCCTCATCTGGAATGATATTTATTTCTACGCCATCCAGTCCTAAACTCCAGGCTTTA
TCAATGAAATCAAAAATATCCATTTTCCCCTGCTGGAAAAAAAGATGATAGG
Sequence number 3358: Yersinia pestis Chromosomal hypothetical gene #95 
AGGCTTTATCAATGAAATCAAAAATATCCATTTTCCCCTGCTGGAAAAAAAGATGATAGGACTCAGTC
TCTAAACCAATTTTCATAGGATTATCTCCATTAATGACTAATTTGGAGTTTA
Sequence number 3359: Yersinia pestis Chromosomal hypothetical gene #96 
ACTCAGTCTCTAAACCAATTTTCATAGGATTATCTCCATTAATGACTAATTTGGAGTTTAGGGTTTAA
AGAAAGTCATAACCGTGACAAAGGTAATGTTTTTGTATTCTTAGTATAAATA
Sequence number 3360: Yersinia pestis Chromosomal hypothetical gene #97 
GGGTTTAAAGAAAGTCATAACCGTGACAAAGGTAATGTTTTTGTATTCTTAGTATAAATATATTTTAC
TAAGTGTACTGTTTAGATACTGTATTGGACAGAGGGCAGCGAGAGGTGTAGC
Sequence number 3361: Yersinia pestis Chromosomal hypothetical gene #98 
TATTTTACTAAGTGTACTGTTTAGATACTGTATTGGACAGAGGGCAGCGAGAGGTGTAGCATGATACA
CACGACAATTTTTCTTGGGTAACATCATGAGAGATATATTAAATAAAGCATT
Sequence number 3362: Yersinia pestis Chromosomal hypothetical gene #99 
ATGATACACACGACAATTTTTCTTGGGTAACATCATGAGAGATATATTAAATAAAGCATTTACTGATG
ATGATCGGAGATTATTACAGTCTTATATTCCCTTCATAGAGTGCTTGGGACA
Sequence number 3363: Yersinia pestis Chromosomal hypothetical gene #100 
TACTGATGATGATCGGAGATTATTACAGTCTTATATTCCCTTCATAGAGTGCTTGGGACAGTTTTTTG
GTGAGTACTGTGAAATTGTACTGCACAGTTTTGAAGATCTTCATCGTTCCGT
Sequence number 3364: Yersinia pestis Chromosomal hypothetical gene #101 
GTTTTTTGGTGAGTACTGTGAAATTGTACTGCACAGTTTTGAAGATCTTCATCGTTCCGTGATCCACA
TTGTGAATGGGCATGTTACTGGCAGAGAGTTAGGTGCGCCGGTCACCAATAT
Sequence number 3365: Yersinia pestis Chromosomal hypothetical gene #102 
GATCCACATTGTGAATGGGCATGTTACTGGCAGAGAGTTAGGTGCGCCGGTCACCAATATAGCCTTGG
AAAAACTGACAGAATTCAAATCATCAAAAGAGATGTGGGATGTTTATTTCAG
Sequence number 3366: Yersinia pestis Chromosomal hypothetical gene #103 
AGCCTTGGAAAAACTGACAGAATTCAAATCATCAAAAGAGATGTGGGATGTTTATTTCAGCAATAAAG
ATACGCGTCCTTTTAAGTCTTCATCCACTCTGATAGTTAACAAACAGGGCGA
Sequence number 3367: Yersinia pestis Chromosomal hypothetical gene #104 
CAATAAAGATACGCGTCCTTTTAAGTCTTCATCCACTCTGATAGTTAACAAACAGGGCGATCCAATAG
GAATGATATGCATGAATATGGCGATGGATATGCCAATGAATGCTTTTATCGA
Sequence number 3368: Yersinia pestis Chromosomal hypothetical gene #105 
TCCAATAGGAATGATATGCATGAATATGGCGATGGATATGCCAATGAATGCTTTTATCGATATATTCA
TGAAAGCCAATAATAGTTGTAGAAATGAGAGTTTTACACAGGATGTCAATAA
Sequence number 3369: Yersinia pestis Chromosomal hypothetical gene #106 
TATATTCATGAAAGCCAATAATAGTTGTAGAAATGAGAGTTTTACACAGGATGTCAATAACCTGGTCC
AAAGCCACCTTGAACCGGTTAAGAGTATGGTTTATGCGAATAAAGACATCCC
Sequence number 3370: Yersinia pestis Chromosomal hypothetical gene #107 
CCTGGTCCAAAGCCACCTTGAACCGGTTAAGAGTATGGTTTATGCGAATAAAGACATCCCTAGTAAGA
ACAAAAATTTCGAAATAATCAAAGAATTAAATGAAACGGGTTTATTTGAACT
Sequence number 3371: Yersinia pestis Chromosomal hypothetical gene #108 
TAGTAAGAACAAAAATTTCGAAATAATCAAAGAATTAAATGAAACGGGTTTATTTGAACTCCCCGTAA
CAAATAAAATTGTCTCCGCAAGTCTGGGGATTAGTCCGGTGACCATTTATAA
Sequence number 3372: Yersinia pestis Chromosomal hypothetical gene #109 
CCCCGTAACAAATAAAATTGTCTCCGCAAGTCTGGGGATTAGTCCGGTGACCATTTATAAACATTTAA
GAACGCTAAATGCACAAAAAGTCAGTGAGTGAGGTCCTCGACTTTGGCTATG
Sequence number 3373: Yersinia pestis Chromosomal hypothetical gene #110 
ACATTTAAGAACGCTAAATGCACAAAAAGTCAGTGAGTGAGGTCCTCGACTTTGGCTATGATAGTGCC
TTCCCTGATGTTCGGTTGGCCGAATAGTATCGCCATAAATTTTATGGCGAAG
Sequence number 3374: Yersinia pestis Chromosomal hypothetical gene #111 
ATAGTGCCTTCCCTGATGTTCGGTTGGCCGAATAGTATCGCCATAAATTTTATGGCGAAGTAACATTA
ATATGGAGAAGTAACATTAATATGGCAAAATAACATTAACATTGCAAAATAA
Sequence number 3375: Yersinia pestis Chromosomal hypothetical gene #112 
TAACATTAATATGGAGAAGTAACATTAATATGGCAAAATAACATTAACATTGCAAAATAACATTAAAC
TGACACGATTAATTAACAATTATTGTTCTTGGTGATGTTAATGTAATTATTG
Sequence number 3376: Yersinia pestis Chromosomal hypothetical gene #113 
CATTAAACTGACACGATTAATTAACAATTATTGTTCTTGGTGATGTTAATGTAATTATTGTTGGTGTT
ATTTGTTATTCAATTTATTTTAATTAATGTGTTTAGGATATTTCCTACAAAA
Sequence number 3377: Yersinia pestis Chromosomal hypothetical gene #114 
TTGGTGTTATTTGTTATTCAATTTATTTTAATTAATGTGTTTAGGATATTTCCTACAAAAAAACAGTA
TGGTCCTATTATCCCCTTCCTTGAGCCCTGTTATCCTTTTTCGAATATATTC
Sequence number 3378: Yersinia pestis Chromosomal hypothetical gene #115 
AAACAGTATGGTCCTATTATCCCCTTCCTTGAGCCCTGTTATCCTTTTTCGAATATATTCCATCTGAA
TGACTTAATCGTCGACAGACAGCACTTCCAACGTTGATCGAACGCTTCAACT
Sequence number 3379: Yersinia pestis Chromosomal hypothetical gene #116 
CATCTGAATGACTTAATCGTCGACAGACAGCACTTCCAACGTTGATCGAACGCTTCAACTCTTTGCTG
ACAATATTTAGTGAGAAGGAGAGAAAATATTTTTCAGGCGATATGTCGTATC
Sequence number 3380: Yersinia pestis Chromosomal hypothetical gene #117 
CTTTGCTGACAATATTTAGTGAGAAGGAGAGAAAATATTTTTCAGGCGATATGTCGTATCGGTAATAA
ACTGGGGCAGCTAATAGGGTAATTCTATGTTGAAACATTTCAGACTGGGTCT
Sequence number 3381: Yersinia pestis Chromosomal hypothetical gene #118 
GGTAATAAACTGGGGCAGCTAATAGGGTAATTCTATGTTGAAACATTTCAGACTGGGTCTTTTTACCG
CAAATGTTCAGGCGGATACAGGGAGCCAACACTCCTTTAGCCGTGTTCCAGC
Sequence number 3382: Yersinia pestis Chromosomal hypothetical gene #119 
TTTTACCGCAAATGTTCAGGCGGATACAGGGAGCCAACACTCCTTTAGCCGTGTTCCAGCAGCCGCGA
TTAATCCCATAACATTAGCGATTGTTGTGGCGTTTTCTACTATTGCTTTACC
Sequence number 3383: Yersinia pestis Chromosomal hypothetical gene #120 
AGCCGCGATTAATCCCATAACATTAGCGATTGTTGTGGCGTTTTCTACTATTGCTTTACCACCAATGG
CATTAGCAGCATGCACCAGCCCCGGGGTGGGAACCTATGTCTGCGAAGGTGA
Sequence number 3384: Yersinia pestis Chromosomal hypothetical gene #121 
ACCAATGGCATTAGCAGCATGCACCAGCCCCGGGGTGGGAACCTATGTCTGCGAAGGTGAAAATACCG
ACGGCATCATCCTGAGCGGCACGGATATTGCGGTTGAGACCCAACCTGGTTT
Sequence number 3385: Yersinia pestis Chromosomal hypothetical gene #122 
AAATACCGACGGCATCATCCTGAGCGGCACGGATATTGCGGTTGAGACCCAACCTGGTTTCAGCATAA
TAGTTCCTGAAAATGGAGACCCTGCACTCTCGCTAGTCGGTTCTGGCACCAT
Sequence number 3386: Yersinia pestis Chromosomal hypothetical gene #123 
CAGCATAATAGTTCCTGAAAATGGAGACCCTGCACTCTCGCTAGTCGGTTCTGGCACCATCAGCTACC
TTGATACCAACAGTTCTGCGCTTGATACCACGGGTGCCGATTCTTTGTATAT
Sequence number 3387: Yersinia pestis Chromosomal hypothetical gene #124 
CAGCTACCTTGATACCAACAGTTCTGCGCTTGATACCACGGGTGCCGATTCTTTGTATATCCAGAATG
ATACGCACACCACTGGGCAATCGTCGTCCATTAATGTTCAGACAAACGGTTC
Sequence number 3388: Yersinia pestis Chromosomal hypothetical gene #125 
CCAGAATGATACGCACACCACTGGGCAATCGTCGTCCATTAATGTTCAGACAAACGGTTCAATGGGTA
GCGGTATCAATATTAGCAACCATAGCGGTGCCGACTCGACAGTACAGGTGGA
Sequence number 3389: Yersinia pestis Chromosomal hypothetical gene #126 
AATGGGTAGCGGTATCAATATTAGCAACCATAGCGGTGCCGACTCGACAGTACAGGTGGATGTTTCCG
GCACATTATTTGGTGATCTAAATGGTTCTCCCGCCCTTTTAATCTATTCATC
Sequence number 3390: Yersinia pestis Chromosomal hypothetical gene #127 
TGTTTCCGGCACATTATTTGGTGATCTAAATGGTTCTCCCGCCCTTTTAATCTATTCATCGGCGGAGA
ATAATTCCACTATTCTCATGAATATCAATACTATTTTCGGCGACGTGGGTAT
Sequence number 3391: Yersinia pestis Chromosomal hypothetical gene #128 
GGCGGAGAATAATTCCACTATTCTCATGAATATCAATACTATTTTCGGCGACGTGGGTATTCGAAGCG
ATAACACTTCATTCAATGGAACAGCCATCACTAACGTTGATATTGCTAATGA
Sequence number 3392: Yersinia pestis Chromosomal hypothetical gene #129 
TCGAAGCGATAACACTTCATTCAATGGAACAGCCATCACTAACGTTGATATTGCTAATGATATTAATG
CTACATATTCGGGTGCCACTATCAACAACAGCGGTAGCGGCGGTACCAGTGT
Sequence number 3393: Yersinia pestis Chromosomal hypothetical gene #130 
TATTAATGCTACATATTCGGGTGCCACTATCAACAACAGCGGTAGCGGCGGTACCAGTGTGGTTAATT
TCAACTCAAAAAGCATCATTACAGAATTGGACGCCTTAAATATTTATAATAC
Sequence number 3394: Yersinia pestis Chromosomal hypothetical gene #131 
GGTTAATTTCAACTCAAAAAGCATCATTACAGAATTGGACGCCTTAAATATTTATAATACCAATTATT
CAGGGGCGGTTATAACCAATGTCGATATCGATGGTGATGTCATTTCAACGAC
Sequence number 3395: Yersinia pestis Chromosomal hypothetical gene #132 
CAATTATTCAGGGGCGGTTATAACCAATGTCGATATCGATGGTGATGTCATTTCAACGACTAGCCAGG
CGACCTCTTTTTATAACGATGCCTACAACGGCTCTGCAAACTTTACGTTTCG
Sequence number 3396: Yersinia pestis Chromosomal hypothetical gene #133 
TAGCCAGGCGACCTCTTTTTATAACGATGCCTACAACGGCTCTGCAAACTTTACGTTTCGTGCGAACA
ATGTCACTGGTGAATATTCCGGCATTTCTATCAATAATAGCAGTCATAACAG
Sequence number 3397: Yersinia pestis Chromosomal hypothetical gene #134 
TGCGAACAATGTCACTGGTGAATATTCCGGCATTTCTATCAATAATAGCAGTCATAACAGTGCTAATA
ACAACCATGATAATGCGGTGATAACCGATATCCTGTTGACGGGGGATCTTAC
Sequence number 3398: Yersinia pestis Chromosomal hypothetical gene #135 
TGCTAATAACAACCATGATAATGCGGTGATAACCGATATCCTGTTGACGGGGGATCTTACGTCTACTT
CAGGATCGGGCCTCCAGATAAACTCTTATATGGACGATGGGGATATCAAGGT
Sequence number 3399: Yersinia pestis Chromosomal hypothetical gene #136 
GTCTACTTCAGGATCGGGCCTCCAGATAAACTCTTATATGGACGATGGGGATATCAAGGTATCAGCCC
AGTTGGAGAACATCTATTCTTACTATGAAGCCCTCAGTGTGCGTGCGGATAC
Sequence number 3400: Yersinia pestis Chromosomal hypothetical gene #137 
ATCAGCCCAGTTGGAGAACATCTATTCTTACTATGAAGCCCTCAGTGTGCGTGCGGATACCCTAACTG
GCAACCTGCAGTTTAACATCGATGTTTCGGGCAATATTGTTGCTGAAAATGG
Sequence number 3401: Yersinia pestis Chromosomal hypothetical gene #138 
CCTAACTGGCAACCTGCAGTTTAACATCGATGTTTCGGGCAATATTGTTGCTGAAAATGGCCTCGGTT
TCATGGTGATGGGGGGCGCTTCCGAAGGCAACTCCACTATGATCATCAATGC
Sequence number 3402: Yersinia pestis Chromosomal hypothetical gene #139 
CCTCGGTTTCATGGTGATGGGGGGCGCTTCCGAAGGCAACTCCACTATGATCATCAATGCGAATAACA
TTAGTTCTGGTAGTCAAGCACTCAATATTTACAACTATAGCGGTTTAGGTTC
Sequence number 3403: Yersinia pestis Chromosomal hypothetical gene #140 
GAATAACATTAGTTCTGGTAGTCAAGCACTCAATATTTACAACTATAGCGGTTTAGGTTCGGCATTTA
CTGCCGTTACCGCAACCGGGCATCTCGTCTCTGAACAGGGGATAGGGGCAAT
Sequence number 3404: Yersinia pestis Chromosomal hypothetical gene #141 
GGCATTTACTGCCGTTACCGCAACCGGGCATCTCGTCTCTGAACAGGGGATAGGGGCAATGTTCAGTA
CCTATGTCAGTCAAGGGGATGCTACCGCTGTCATCAATTTAAATGACATCAC
Sequence number 3405: Yersinia pestis Chromosomal hypothetical gene #142 
GTTCAGTACCTATGTCAGTCAAGGGGATGCTACCGCTGTCATCAATTTAAATGACATCACTACTGCGG
GCAGTGGTGTAAAGATAGACACCATAGCGAATGGGGGGAATTCAACCACCTA
Sequence number 3406: Yersinia pestis Chromosomal hypothetical gene #143 
TACTGCGGGCAGTGGTGTAAAGATAGACACCATAGCGAATGGGGGGAATTCAACCACCTATCTCACCG
TGGTGGGCCAGATTAACTCTAGTTTGTATGATGGTATTGATCTTCGTGCGAC
Sequence number 3407: Yersinia pestis Chromosomal hypothetical gene #144 
TCTCACCGTGGTGGGCCAGATTAACTCTAGTTTGTATGATGGTATTGATCTTCGTGCGACGGCTACTG
AAGGTAATACCCTGGTCAGCATTGACGTCAATAATATCGCCAGTGAATACGA
Sequence number 3408: Yersinia pestis Chromosomal hypothetical gene #145 
GGCTACTGAAGGTAATACCCTGGTCAGCATTGACGTCAATAATATCGCCAGTGAATACGATGCTATCC
ACCTCGATAACAAGAACTACACCACAGGCGCAGACAACGGCACCTCGACCAT
Sequence number 3409: Yersinia pestis Chromosomal hypothetical gene #146 
TGCTATCCACCTCGATAACAAGAACTACACCACAGGCGCAGACAACGGCACCTCGACCATTGATCTGA
TCACCCGGGGCGCGCTGGTTTCGCAGCAGGGCTACGGAATTAATATTGAAAC
Sequence number 3410: Yersinia pestis Chromosomal hypothetical gene #147 
TGATCTGATCACCCGGGGCGCGCTGGTTTCGCAGCAGGGCTACGGAATTAATATTGAAACCAATACCG
CAGACACCTATGTCACCGTGGGCGGCTTGGTGCACGGCGGCAATGGCACCGC
Sequence number 3411: Yersinia pestis Chromosomal hypothetical gene #148 
CAATACCGCAGACACCTATGTCACCGTGGGCGGCTTGGTGCACGGCGGCAATGGCACCGCAATCGGCA
TTCATCGGCTTGATAACATTCAAACATCGGCCACGTTAGAGCTGCAATCTGG
Sequence number 3412: Yersinia pestis Chromosomal hypothetical gene #149 
AATCGGCATTCATCGGCTTGATAACATTCAAACATCGGCCACGTTAGAGCTGCAATCTGGCTATGCCC
TTGAAGGCGTTACGCAGGCACTGGTCTTCAATGGCAGTTATGCGGAGATCAA
Sequence number 3413: Yersinia pestis Chromosomal hypothetical gene #150 
CTATGCCCTTGAAGGCGTTACGCAGGCACTGGTCTTCAATGGCAGTTATGCGGAGATCAATGATGCCG
CGCTGGATCTGGCAAACAGCCATCTGGTGCTGGGCGGAGCAGGAGACGCCGC
Sequence number 3414: Yersinia pestis Chromosomal hypothetical gene #151 
TGATGCCGCGCTGGATCTGGCAAACAGCCATCTGGTGCTGGGCGGAGCAGGAGACGCCGCTTTCGATC
TCACGCGTATTGATAACCGTGAAGAGGCCATTCTGGATGGCGACCCGAACCG
Sequence number 3415: Yersinia pestis Chromosomal hypothetical gene #152 
TTTCGATCTCACGCGTATTGATAACCGTGAAGAGGCCATTCTGGATGGCGACCCGAACCGGATCATCG
GCTTCGGTACCCTGACCAAAACCAACAACAGCATCTGGACGTTAACCGGCGC
Sequence number 3416: Yersinia pestis Chromosomal hypothetical gene #153 
GATCATCGGCTTCGGTACCCTGACCAAAACCAACAACAGCATCTGGACGTTAACCGGCGCCAATATGG
CCGACGGTGACGCCAATGCCTTCCTGTCGGCCAATATCGCCGGGGGGATTTT
Sequence number 3417: Yersinia pestis Chromosomal hypothetical gene #154 
CAATATGGCCGACGGTGACGCCAATGCCTTCCTGTCGGCCAATATCGCCGGGGGGATTTTGGTGCTGG
ATAACGCCACGCTGGGCCTGACACCTGACGCTGGGGCACTCACTGGAGCCAC
Sequence number 3418: Yersinia pestis Chromosomal hypothetical gene #155 
GGTGCTGGATAACGCCACGCTGGGCCTGACACCTGACGCTGGGGCACTCACTGGAGCCACAGTAAACC
GCCTCAGTGCTGCCGATATCGCCGCTGACCCGACGCTAGTGGCTACCGAAAC
Sequence number 3419: Yersinia pestis Chromosomal hypothetical gene #156 
AGTAAACCGCCTCAGTGCTGCCGATATCGCCGCTGACCCGACGCTAGTGGCTACCGAAACCGGTGCAT
TAACCCTGGCTGAAGGCGGGGCGTTGTCCTCGCTCGGTGACTCGGTTCTGAG
Sequence number 3420: Yersinia pestis Chromosomal hypothetical gene #157 
CGGTGCATTAACCCTGGCTGAAGGCGGGGCGTTGTCCTCGCTCGGTGACTCGGTTCTGAGCGGTAACC
TTATCAGCGCCGGTGGGATCCTGCTGTCAAACCACTATACCGGCGGCAATGG
Sequence number 3421: Yersinia pestis Chromosomal hypothetical gene #158 
CGGTAACCTTATCAGCGCCGGTGGGATCCTGCTGTCAAACCACTATACCGGCGGCAATGGTGCCGCTA
CCGACGATCGGCTGACCGTGACCGGGACTTATTTTGGTGAAAATAACGGTTC
Sequence number 3422: Yersinia pestis Chromosomal hypothetical gene #159 
TGCCGCTACCGACGATCGGCTGACCGTGACCGGGACTTATTTTGGTGAAAATAACGGTTCCGGTGAAG
GGGCCTGGCTGGCACTCGATACGGTGCTGGGGGATGACGATTCTGCCACCGA
Sequence number 3423: Yersinia pestis Chromosomal hypothetical gene #160 
CGGTGAAGGGGCCTGGCTGGCACTCGATACGGTGCTGGGGGATGACGATTCTGCCACCGACCGGTTAG
TGATCAACGGCGATGCCACCGGCACCACCTCGGTCCGGGTGAACAATGCGGG
Sequence number 3424: Yersinia pestis Chromosomal hypothetical gene #161 
CCGGTTAGTGATCAACGGCGATGCCACCGGCACCACCTCGGTCCGGGTGAACAATGCGGGCGGTCTGG
GCGATAAAACCCTCAATGGCATCAACCTGATCACCGTGGACGGTCTGGCGCA
Sequence number 3425: Yersinia pestis Chromosomal hypothetical gene #162 
CGGTCTGGGCGATAAAACCCTCAATGGCATCAACCTGATCACCGTGGACGGTCTGGCGCAGGATGACA
CCTTCCTACTGGCCGGGGACTATGTCACCACGGATGGCTATCAGGCGGTGGT
Sequence number 3426: Yersinia pestis Chromosomal hypothetical gene #163 
GGATGACACCTTCCTACTGGCCGGGGACTATGTCACCACGGATGGCTATCAGGCGGTGGTGGGCGGGG
CGTATGCCTACACCTTACAGGCCGACGGGGAAGCCGCCACTGCGGGGCGCAA
Sequence number 3427: Yersinia pestis Chromosomal hypothetical gene #164 
GGGCGGGGCGTATGCCTACACCTTACAGGCCGACGGGGAAGCCGCCACTGCGGGGCGCAACTGGTATC
TCTCTTCAGAACTGATGTTAACCGAGGGGGTACGCTATCAGGCGGGCGTGCC
Sequence number 3428: Yersinia pestis Chromosomal hypothetical gene #165 
CTGGTATCTCTCTTCAGAACTGATGTTAACCGAGGGGGTACGCTATCAGGCGGGCGTGCCGCTGTATG
AACAATATCCGCAGGTGCTGGCCGCCCTGAATACCCTGCCGACGCTGCAACA
Sequence number 3429: Yersinia pestis Chromosomal hypothetical gene #166 
GCTGTATGAACAATATCCGCAGGTGCTGGCCGCCCTGAATACCCTGCCGACGCTGCAACAGCGTGTCG
GTAACCGTTACGGGGCGCCGGGCGCGCTGGCAGACCTGGACTTTGACAATAA
Sequence number 3430: Yersinia pestis Chromosomal hypothetical gene #167 
GCGTGTCGGTAACCGTTACGGGGCGCCGGGCGCGCTGGCAGACCTGGACTTTGACAATAATCAATGGG
CCTGGGGCCGTATTGAAGGGAGCCACCAGGTCACCGACCCGGCCCGCTCCAC
Sequence number 3431: Yersinia pestis Chromosomal hypothetical gene #168 
TCAATGGGCCTGGGGCCGTATTGAAGGGAGCCACCAGGTCACCGACCCGGCCCGCTCCACCAGTGGTT
CACAACGCGAGATTGATGTGTGGAAGTTGCAGACCGGCATTGATGTGCCGCT
Sequence number 3432: Yersinia pestis Chromosomal hypothetical gene #169 
CAGTGGTTCACAACGCGAGATTGATGTGTGGAAGTTGCAGACCGGCATTGATGTGCCGCTGTATCAGA
GCCAGGACGGTTCACTGCTGACCGGCGGGGTAAACTTCTCCTACGGTAAAGC
Sequence number 3433: Yersinia pestis Chromosomal hypothetical gene #170 
GTATCAGAGCCAGGACGGTTCACTGCTGACCGGCGGGGTAAACTTCTCCTACGGTAAAGCCAAAGCGG
ATATCCACTCATTCTTTGGTGATGGCCGCATCAACAGCGCAGGTTACGGTCT
Sequence number 3434: Yersinia pestis Chromosomal hypothetical gene #171 
CAAAGCGGATATCCACTCATTCTTTGGTGATGGCCGCATCAACAGCGCAGGTTACGGTCTTGGCACCA
GCCTGACCTGGTATGGCAATAACGGCGTGTATGTGGATGGCCAGTTGCAGAC
Sequence number 3435: Yersinia pestis Chromosomal hypothetical gene #172 
TGGCACCAGCCTGACCTGGTATGGCAATAACGGCGTGTATGTGGATGGCCAGTTGCAGACGATGTGGT
TTGACAGCGACCTGAGCTCCCGTACCGCAGGGCATGCAGTGGCCAGCGGTAA
Sequence number 3436: Yersinia pestis Chromosomal hypothetical gene #173 
GATGTGGTTTGACAGCGACCTGAGCTCCCGTACCGCAGGGCATGCAGTGGCCAGCGGTAACAATGGTC
GCGGGTATACCTCGGCGATAGAAGCCGGTAAAGGTTACGCACTGGGTAACGG
Sequence number 3437: Yersinia pestis Chromosomal hypothetical gene #174 
CAATGGTCGCGGGTATACCTCGGCGATAGAAGCCGGTAAAGGTTACGCACTGGGTAACGGGTTGTCAC
TGACCCCGCAGATGCAGGTGACCTACTCGCGGGTCGATTTCGATACCTTCCG
Sequence number 3438: Yersinia pestis Chromosomal hypothetical gene #175 
GTTGTCACTGACCCCGCAGATGCAGGTGACCTACTCGCGGGTCGATTTCGATACCTTCCGCGATCCGT
TTGATAGCGAAGTTTCGCTGCAAGAGGGTGACAGCCTGCGGGGCCGCATCGG
Sequence number 3439: Yersinia pestis Chromosomal hypothetical gene #176 
CGATCCGTTTGATAGCGAAGTTTCGCTGCAAGAGGGTGACAGCCTGCGGGGCCGCATCGGTGTCTCAC
TGGATAAGGAAACGACCTGGAGCGCGAAAGACGGCACCACCCGCCGCTCACA
Sequence number 3440: Yersinia pestis Chromosomal hypothetical gene #177 
TGTCTCACTGGATAAGGAAACGACCTGGAGCGCGAAAGACGGCACCACCCGCCGCTCACACATTTACA
GTCATCTCGATCTGCACAATGAGTTCCTCAATGGCAGTAAAGTGCAGGTCTC
Sequence number 3441: Yersinia pestis Chromosomal hypothetical gene #178 
CATTTACAGTCATCTCGATCTGCACAATGAGTTCCTCAATGGCAGTAAAGTGCAGGTCTCGGGGGTGG
AGTTCGCCACCCGGGATGAGCGTCAGTCGGTGGGGTTAGGCGCGGGCGGCAC
Sequence number 3442: Yersinia pestis Chromosomal hypothetical gene #179 
GGGGGTGGAGTTCGCCACCCGGGATGAGCGTCAGTCGGTGGGGTTAGGCGCGGGCGGCACCTATGAAT
GGCAGAATGGTCGCTACGCGGTTTACGGCAATGTCAATCTGCTGGGGGCTAC
Sequence number 3443: Yersinia pestis Chromosomal hypothetical gene #180 
CTATGAATGGCAGAATGGTCGCTACGCGGTTTACGGCAATGTCAATCTGCTGGGGGCTACACGGAATG
TCAGTGACAACTATGCCGTCGGCGGTACGATAGGTGCACGGGTGAGCTGGTA
Sequence number 3444: Yersinia pestis Chromosomal hypothetical gene #181 
ACGGAATGTCAGTGACAACTATGCCGTCGGCGGTACGATAGGTGCACGGGTGAGCTGGTAATACAGGA
CGGTTTTGTTATATCGGGGTATCAGTGCGCCGAGTAGGGCACTGATAACGCA
Sequence number 3445: Yersinia pestis Chromosomal hypothetical gene #182 
ATACAGGACGGTTTTGTTATATCGGGGTATCAGTGCGCCGAGTAGGGCACTGATAACGCATTTAGTAT
ACTTAATATTAGGTCGTCATACCGACGGTGTGCAGAAGAAAGTCGAGTTGCT
Sequence number 3446: Yersinia pestis Chromosomal hypothetical gene #183 
TTTAGTATACTTAATATTAGGTCGTCATACCGACGGTGTGCAGAAGAAAGTCGAGTTGCTGTTGAAAT
CAATGACGAAATAGCCTTGTTTCAGATCTCATTTATAAAAAAATAGTTACAG
Sequence number 3447: Yersinia pestis Chromosomal hypothetical gene #184 
GTTGAAATCAATGACGAAATAGCCTTGTTTCAGATCTCATTTATAAAAAAATAGTTACAGATAAATTG
TAGGAAAATTCACTCAATGAACTTCCAATATTCTTTGTTTAATTTTTATGAT
Sequence number 3448: Yersinia pestis Chromosomal hypothetical gene #185 
ATAAATTGTAGGAAAATTCACTCAATGAACTTCCAATATTCTTTGTTTAATTTTTATGATGTTTATAA
AAGCCTTTAATAATGTGTGCTTATATTGGCTTTGTAGGAAATATCTTACGTA
Sequence number 3449: Yersinia pestis Chromosomal hypothetical gene #186 
GTTTATAAAAGCCTTTAATAATGTGTGCTTATATTGGCTTTGTAGGAAATATCTTACGTATCAATATG
ATGAACGGTTGCTTTTGAATTTTCACTGCTTACATACTGTTACATTTAACCC
Sequence number 3450: Yersinia pestis Chromosomal hypothetical gene #187 
TCAATATGATGAACGGTTGCTTTTGAATTTTCACTGCTTACATACTGTTACATTTAACCCATTGGTGT
TAGCTGGCTAACTTTTCTACACTGCCGCCGGCGAGTATTTAATTGGTAGATT
Sequence number 3451: Yersinia pestis Chromosomal hypothetical gene #188 
ATTGGTGTTAGCTGGCTAACTTTTCTACACTGCCGCCGGCGAGTATTTAATTGGTAGATTAAGGCGTG
TGAAAAATACCTGTCAGATACTGTTTCATGCTATCCGATGAATGGATAGGGT
Sequence number 3452: Yersinia pestis Chromosomal hypothetical gene #189 
AAGGCGTGTGAAAAATACCTGTCAGATACTGTTTCATGCTATCCGATGAATGGATAGGGTTATCTCAT
AGAGAATTTTTTACTTAATAAACGCTGAGTTGGATACATAATGGAGTATTGG
Sequence number 3453: Yersinia pestis Chromosomal hypothetical gene #190 
TATCTCATAGAGAATTTTTTACTTAATAAACGCTGAGTTGGATACATAATGGAGTATTGGCTGTTAAT
TTTTTTACTTATCTTCTTGTTTGTCATCTATATGCAAATAAGTGCGTTATCC
Sequence number 3454: Yersinia pestis Chromosomal hypothetical gene #191 
CTGTTAATTTTTTTACTTATCTTCTTGTTTGTCATCTATATGCAAATAAGTGCGTTATCCAACTCGGT
AAAATATATACGAGAAGAGATAGGCAGGCTGCGCAATGAGCTTGCTCCACGC
Sequence number 3455: Yersinia pestis Chromosomal hypothetical gene #192 
AACTCGGTAAAATATATACGAGAAGAGATAGGCAGGCTGCGCAATGAGCTTGCTCCACGCTTGCCTGA
ACCTACCGTGGCTACTGTTGTGGCCACTGTTAAGGAAGAGATCACGCCAACG
Sequence number 3456: Yersinia pestis Chromosomal hypothetical gene #193 
TTGCCTGAACCTACCGTGGCTACTGTTGTGGCCACTGTTAAGGAAGAGATCACGCCAACGAATTTGGC
AGATGATGTGGCCGCATCCGAGGTATGGACCCCTGATTACGCAACCTATCAG
Sequence number 3457: Yersinia pestis Chromosomal hypothetical gene #194 
AATTTGGCAGATGATGTGGCCGCATCCGAGGTATGGACCCCTGATTACGCAACCTATCAGAATTATCT
ACTCACCAAACAGCAAAGCCAGATTAAAGAGATGGCTCTAGCCTATGCCGCA
Sequence number 3458: Yersinia pestis Chromosomal hypothetical gene #195 
AATTATCTACTCACCAAACAGCAAAGCCAGATTAAAGAGATGGCTCTAGCCTATGCCGCAAAAAAAGA
ACCCGCTCGGCCAATAGAATATAGGATTGCTGACCGTCAGGTGGTGCTTGAG
Sequence number 3459: Yersinia pestis Chromosomal hypothetical gene #196 
AAAAAAGAACCCGCTCGGCCAATAGAATATAGGATTGCTGACCGTCAGGTGGTGCTTGAGCCACAGGA
TGGGGCTCCTCAAGAATCGCCGAAAATGGATGCAGGTCCTGCCAGTGGTCAT
Sequence number 3460: Yersinia pestis Chromosomal hypothetical gene #197 
CCACAGGATGGGGCTCCTCAAGAATCGCCGAAAATGGATGCAGGTCCTGCCAGTGGTCATCCACAGAA
ACCCGATGTGTGGACAAAATTGGAAAATGTCCTGTTTTCTTTAAAATCACCG
Sequence number 3461: Yersinia pestis Chromosomal hypothetical gene #198 
CCACAGAAACCCGATGTGTGGACAAAATTGGAAAATGTCCTGTTTTCTTTAAAATCACCGGAGAGTTT
CTGGAAACGCATCGAGCAAGAGTTTGCCGCGCGTTGGATGGTGTGGATCGGC
Sequence number 3462: Yersinia pestis Chromosomal hypothetical gene #199 
GAGAGTTTCTGGAAACGCATCGAGCAAGAGTTTGCCGCGCGTTGGATGGTGTGGATCGGCGGTATGAT
CATGGCACTGGGTGTGATTTTCCTGCTGAAAGCAGGCAGCGAACAGGGTCTG
Sequence number 3463: Yersinia pestis Chromosomal hypothetical gene #200 
GGTATGATCATGGCACTGGGTGTGATTTTCCTGCTGAAAGCAGGCAGCGAACAGGGTCTGTTCGGGCC
AAACTTGCGGATTGGTACCGCCATTGTGCTGAGTGTGCTGATGGTTGTTGGG
Sequence number 3464: Yersinia pestis Chromosomal hypothetical gene #201 
TTCGGGCCAAACTTGCGGATTGGTACCGCCATTGTGCTGAGTGTGCTGATGGTTGTTGGGGGGGAGTG
GCTCAGGCGTTCACGTTTTCAGTTGCCCTTGGTCAATGCCGGTTATATCCCG
Sequence number 3465: Yersinia pestis Chromosomal hypothetical gene #202 
GGGGAGTGGCTCAGGCGTTCACGTTTTCAGTTGCCCTTGGTCAATGCCGGTTATATCCCGGCGGCGCT
GAGTGGGGCCGGTATTCTCGGTCTATTTGCCTCAATGCTGGCGGCTAAATAT
Sequence number 3466: Yersinia pestis Chromosomal hypothetical gene #203 
GCGGCGCTGAGTGGGGCCGGTATTCTCGGTCTATTTGCCTCAATGCTGGCGGCTAAATATCTCTACGA
CATGTTCCCGATGCCCGTTCTCATGCTGTTGCTCAGCCTGATTTCTCTGTTG
Sequence number 3467: Yersinia pestis Chromosomal hypothetical gene #204 
CTCTACGACATGTTCCCGATGCCCGTTCTCATGCTGTTGCTCAGCCTGATTTCTCTGTTGGCGATGGT
GCTGGCATTGTGGCAGGGGCCATTTATGGCGGCCTTGGGGCTGCTTGGCGCC
Sequence number 3468: Yersinia pestis Chromosomal hypothetical gene #205 
GCGATGGTGCTGGCATTGTGGCAGGGGCCATTTATGGCGGCCTTGGGGCTGCTTGGCGCCTATACCGT
CCCGTTATTTGTTTCGACGGGCAGCGGTAATGTCACCGGGCTGTTGGCCTAC
Sequence number 3469: Yersinia pestis Chromosomal hypothetical gene #206 
TATACCGTCCCGTTATTTGTTTCGACGGGCAGCGGTAATGTCACCGGGCTGTTGGCCTACCTGTTCCT
GGTTAGTTTGGCGTCTATCGGCCTGATGAGCCGTGTCTATCGGCACTGGCTA
Sequence number 3470: Yersinia pestis Chromosomal hypothetical gene #207 
CTGTTCCTGGTTAGTTTGGCGTCTATCGGCCTGATGAGCCGTGTCTATCGGCACTGGCTATGGCTGGG
GGCGATGGTCGGAAACTATCTATGGCTTTTGGTTTCTTTATTCATAGCAACG
Sequence number 3471: Yersinia pestis Chromosomal hypothetical gene #208 
TGGCTGGGGGCGATGGTCGGAAACTATCTATGGCTTTTGGTTTCTTTATTCATAGCAACGTCCGAACA
CCAGTTAGCGCGTTCACTATTCCTGCTAGCAACCACATACGGCTTCCTGGCC
Sequence number 3472: Yersinia pestis Chromosomal hypothetical gene #209 
TCCGAACACCAGTTAGCGCGTTCACTATTCCTGCTAGCAACCACATACGGCTTCCTGGCCTGGCCACA
TCTGGGGTGGGCGCTAAAAAGCAAAATACGTTTTCGCCATAATGACTGGAAA
Sequence number 3473: Yersinia pestis Chromosomal hypothetical gene #210 
TGGCCACATCTGGGGTGGGCGCTAAAAAGCAAAATACGTTTTCGCCATAATGACTGGAAAAACTGGCC
CCCGGCTTTGCTGGATCCGTTATTAACTGGGTTGATTGCCGGGCTTTCGCTA
Sequence number 3474: Yersinia pestis Chromosomal hypothetical gene #211 
AACTGGCCCCCGGCTTTGCTGGATCCGTTATTAACTGGGTTGATTGCCGGGCTTTCGCTATTAGCCTT
AACGTTAAGCGCAAATCATTCGATCCTGGTCTGGAGCACGTTGGTATTCTCC
Sequence number 3475: Yersinia pestis Chromosomal hypothetical gene #212 
TTAGCCTTAACGTTAAGCGCAAATCATTCGATCCTGGTCTGGAGCACGTTGGTATTCTCCTGTATTAG
CCTGTTACTGATTGCCAGACGCTCACCTTCATTGGATTTATTTGTTCCACTG
Sequence number 3476: Yersinia pestis Chromosomal hypothetical gene #213 
TGTATTAGCCTGTTACTGATTGCCAGACGCTCACCTTCATTGGATTTATTTGTTCCACTGGCCGCGAT
ATTAGCCATTTCTCCCTTTATTAATGCAGGAACGCTGTTTGAGCCAGAAACC
Sequence number 3477: Yersinia pestis Chromosomal hypothetical gene #214 
GCCGCGATATTAGCCATTTCTCCCTTTATTAATGCAGGAACGCTGTTTGAGCCAGAAACCGTCTGGAC
GAGTTTGCTCGCGCTCAGCACATTAGCGCTATTCTGCGGGATATATGGTAGC
Sequence number 3478: Yersinia pestis Chromosomal hypothetical gene #215 
GTCTGGACGAGTTTGCTCGCGCTCAGCACATTAGCGCTATTCTGCGGGATATATGGTAGCTACCAGGT
CACCCGGCCGGTTTACCGCAAGCTGTGGTGGGCGTTATTGTCGGTGCTGGTG
Sequence number 3479: Yersinia pestis Chromosomal hypothetical gene #216 
TACCAGGTCACCCGGCCGGTTTACCGCAAGCTGTGGTGGGCGTTATTGTCGGTGCTGGTGCCAATGTA
TCTCGTTGCAAGTACCTATTATGCCTGTCGCGATAATTTTGAGCTGGCTCCA
Sequence number 3480: Yersinia pestis Chromosomal hypothetical gene #217 
CCAATGTATCTCGTTGCAAGTACCTATTATGCCTGTCGCGATAATTTTGAGCTGGCTCCAGTTAAATA
CACACTGCTGGCGGTTTGTCTGGCTATGTATATCGCGTGGAATGCATTCAGT
Sequence number 3481: Yersinia pestis Chromosomal hypothetical gene #218 
GTTAAATACACACTGCTGGCGGTTTGTCTGGCTATGTATATCGCGTGGAATGCATTCAGTGATGGGTA
TCGCCGTGGCTCAGCGCTGTTGCGGACGATCTATCAGGTCGCCGCCCAACTG
Sequence number 3482: Yersinia pestis Chromosomal hypothetical gene #219 
GATGGGTATCGCCGTGGCTCAGCGCTGTTGCGGACGATCTATCAGGTCGCCGCCCAACTGACGTTGAC
CTTTGCATTATTTATGGCTTTCAGCCAGGTTACGCTAACCCTGCTGCTCGCG
Sequence number 3483: Yersinia pestis Chromosomal hypothetical gene #220 
ACGTTGACCTTTGCATTATTTATGGCTTTCAGCCAGGTTACGCTAACCCTGCTGCTCGCGGGTCAGCT
TTTAGTTTTGGTACTCTGGCAACGTTGGCGACAGGTTTCGTTACTGTCTTGG
Sequence number 3484: Yersinia pestis Chromosomal hypothetical gene #221 
GGTCAGCTTTTAGTTTTGGTACTCTGGCAACGTTGGCGACAGGTTTCGTTACTGTCTTGGATTTTGAA
ACTGCATGCGATCATACTGCTGGTTCGTCTGTCAATGAACCCGTATATCCTT
Sequence number 3485: Yersinia pestis Chromosomal hypothetical gene #222 
ATTTTGAAACTGCATGCGATCATACTGCTGGTTCGTCTGTCAATGAACCCGTATATCCTTGGATATTC
CTCGCCGTTGCACATTGGTTCGGTCGTTATTCCTTGGACGCTATACGGTTTT
Sequence number 3486: Yersinia pestis Chromosomal hypothetical gene #223 
GGATATTCCTCGCCGTTGCACATTGGTTCGGTCGTTATTCCTTGGACGCTATACGGTTTTGGGATTCC
GATTCTCTGCCTGTGGCTAAGTTCACGCTGGTTACCTGCGCGTCGCGGTGAG
Sequence number 3487: Yersinia pestis Chromosomal hypothetical gene #224 
GGGATTCCGATTCTCTGCCTGTGGCTAAGTTCACGCTGGTTACCTGCGCGTCGCGGTGAGCAGACCGC
CAATTGGTTAGCGGCCAATGCTATTTATCTGTTGGCCTTGTGGATCAACGTT
Sequence number 3488: Yersinia pestis Chromosomal hypothetical gene #225 
CAGACCGCCAATTGGTTAGCGGCCAATGCTATTTATCTGTTGGCCTTGTGGATCAACGTTGAATTACG
TCACCTGCTGCATGGCAGCTATCATCTGTCGCTGGATTTCGCTTCTCTTACC
Sequence number 3489: Yersinia pestis Chromosomal hypothetical gene #226 
GAATTACGTCACCTGCTGCATGGCAGCTATCATCTGTCGCTGGATTTCGCTTCTCTTACCGATTGTGC
GTTGCATGGGGTGACCTTCGGGCTGATGGCGCTGGCCTACGGCTATCGTGAA
Sequence number 3490: Yersinia pestis Chromosomal hypothetical gene #227 
GATTGTGCGTTGCATGGGGTGACCTTCGGGCTGATGGCGCTGGCCTACGGCTATCGTGAACAGTTTGC
AGATTCGCTACAACGGATATACCGCCTGGCGGCTAAAACCAGTGCCATTCTG
Sequence number 3491: Yersinia pestis Chromosomal hypothetical gene #228 
CAGTTTGCAGATTCGCTACAACGGATATACCGCCTGGCGGCTAAAACCAGTGCCATTCTGATGCTCAC
CCTAACGTTGCTCTGCCTGGTTGTCTTTAACCCCATCTGGTCAGCACAACAG
Sequence number 3492: Yersinia pestis Chromosomal hypothetical gene #229 
ATGCTCACCCTAACGTTGCTCTGCCTGGTTGTCTTTAACCCCATCTGGTCAGCACAACAGGTTGGCGG
CCTACCGCTGCTGAATATGGTGACCGTTGCCTATGCGATCCCGATGGTGTTC
Sequence number 3493: Yersinia pestis Chromosomal hypothetical gene #230 
GTTGGCGGCCTACCGCTGCTGAATATGGTGACCGTTGCCTATGCGATCCCGATGGTGTTCATGGTGGT
AGCACTAAGATACTGGCCAGATCACTGGCTAGATATCCGTCTGAAGCCCTAC
Sequence number 3494: Yersinia pestis Chromosomal hypothetical gene #231 
ATGGTGGTAGCACTAAGATACTGGCCAGATCACTGGCTAGATATCCGTCTGAAGCCCTACGTTATCGG
TGCGATTGCGCTACTGGGAATGGTGTTCATTACCTTGACGGTCCGCCAGCTC
Sequence number 3495: Yersinia pestis Chromosomal hypothetical gene #232 
GTTATCGGTGCGATTGCGCTACTGGGAATGGTGTTCATTACCTTGACGGTCCGCCAGCTCTGGCATGG
TGACCGGTTGGATAATGATATCGTGTACAGCGGCGAACAATACTCTTACTCC
Sequence number 3496: Yersinia pestis Chromosomal hypothetical gene #233 
TGGCATGGTGACCGGTTGGATAATGATATCGTGTACAGCGGCGAACAATACTCTTACTCCTTAGCGTG
GATGTTAACTGCCATTGGCATGATGTACAGCGCAATACACTGGCCTAACTAT
Sequence number 3497: Yersinia pestis Chromosomal hypothetical gene #234 
TTAGCGTGGATGTTAACTGCCATTGGCATGATGTACAGCGCAATACACTGGCCTAACTATAAAGTGCG
CCGCGCCTCCCTGGGGTTGTTGGCGCTCACGATCGTCAAACTGTTCCTGTGG
Sequence number 3498: Yersinia pestis Chromosomal hypothetical gene #235 
AAAGTGCGCCGCGCCTCCCTGGGGTTGTTGGCGCTCACGATCGTCAAACTGTTCCTGTGGGATATGTC
TGGTCTTGAAGGGCTATATCGTTCGGTCTCATTCCTCGGTCTGGGGCTGTGT
Sequence number 3499: Yersinia pestis Chromosomal hypothetical gene #236 
GATATGTCTGGTCTTGAAGGGCTATATCGTTCGGTCTCATTCCTCGGTCTGGGGCTGTGTCTGGTAGC
GATTGGTTGGTTCTACCAGAAGTTTGTCGTTATGAAGGAAGTCATTGAGCAA
Sequence number 3500: Yersinia pestis Chromosomal hypothetical gene #237 
CTGGTAGCGATTGGTTGGTTCTACCAGAAGTTTGTCGTTATGAAGGAAGTCATTGAGCAAAACAAGGA
TCAGCCGACAGCGGACGTGGTACAAAGCGGGCAATAATCGCCATTTGTCTTA
Sequence number 3501: Yersinia pestis Chromosomal hypothetical gene #238 
AACAAGGATCAGCCGACAGCGGACGTGGTACAAAGCGGGCAATAATCGCCATTTGTCTTACCGGAGCG
TGCATCGACTGATGTGGGAAAATCAATATACAGCAAGGAGTCAGTGTGATTA
Sequence number 3502: Yersinia pestis Chromosomal hypothetical gene #239 
CCGGAGCGTGCATCGACTGATGTGGGAAAATCAATATACAGCAAGGAGTCAGTGTGATTAATTTTGTG
AAGTTAATGTGTAATGTGAAGTTAATGTGTAAATGGGGGGCGATATTGATGT
Sequence number 3503: Yersinia pestis Chromosomal hypothetical gene #240 
ATTTTGTGAAGTTAATGTGTAATGTGAAGTTAATGTGTAAATGGGGGGCGATATTGATGTCACCCTTC
TTTCTTTCTGCTTGCGTAACTAACCAGCTTTCAGATGATATCCGGGGCCATG
Sequence number 3504: Yersinia pestis Chromosomal hypothetical gene #241 
CACCCTTCTTTCTTTCTGCTTGCGTAACTAACCAGCTTTCAGATGATATCCGGGGCCATGAGAAAGGC
TACACGCATTATAATGATGATATTATTGTCGGCGTGTCTTTGGCTAAGCAGG
Sequence number 3505: Yersinia pestis Chromosomal hypothetical gene #242 
AGAAAGGCTACACGCATTATAATGATGATATTATTGTCGGCGTGTCTTTGGCTAAGCAGGGTGATAAT
AAAAACTGGGCATTTGTCGGTACGCATTTCGATTATGTGTTGTCATCGGGAG
Sequence number 3506: Yersinia pestis Chromosomal hypothetical gene #243 
GTGATAATAAAAACTGGGCATTTGTCGGTACGCATTTCGATTATGTGTTGTCATCGGGAGCCGACGAG
TTCTTAACGCTATTAGTTACCGGGCAGATTGATAAAAAGAAGATTGAGGTAG
Sequence number 3507: Yersinia pestis Chromosomal hypothetical gene #244 
CCGACGAGTTCTTAACGCTATTAGTTACCGGGCAGATTGATAAAAAGAAGATTGAGGTAGTCAGGGAC
GGTAGCTTTAATCTTAATAAGAAAAAGGATGGCTTCACAGGGAAAATCGCTC
Sequence number 3508: Yersinia pestis Chromosomal hypothetical gene #245 
TCAGGGACGGTAGCTTTAATCTTAATAAGAAAAAGGATGGCTTCACAGGGAAAATCGCTCTGAAATAT
CGTTATCAGACGGCGGAAGAGCGAGGCAAAATAGAGCCGCTGATAAAAGGGG
Sequence number 3509: Yersinia pestis Chromosomal hypothetical gene #246 
TGAAATATCGTTATCAGACGGCGGAAGAGCGAGGCAAAATAGAGCCGCTGATAAAAGGGGCTGATTGG
AATTGTCCAAGCCTCACGGAAACAACGGGTATTTGCAATATTAACCTGAATA
Sequence number 3510: Yersinia pestis Chromosomal hypothetical gene #247 
CTGATTGGAATTGTCCAAGCCTCACGGAAACAACGGGTATTTGCAATATTAACCTGAATAATCTGGTC
GGCACTATTCACAGAAAAGGTGCTACACCGGCCGATATCTTCCGTTTTGAAC
Sequence number 3511: Yersinia pestis Chromosomal hypothetical gene #248 
ATCTGGTCGGCACTATTCACAGAAAAGGTGCTACACCGGCCGATATCTTCCGTTTTGAACATCCTTTG
CAAGTGAAGTTTTATAGTAAAAATACGACCTCAGCTAAGAGGGCGCTTTACC
Sequence number 3512: Yersinia pestis Chromosomal hypothetical gene #249 
ATCCTTTGCAAGTGAAGTTTTATAGTAAAAATACGACCTCAGCTAAGAGGGCGCTTTACCCGGTGGCC
GTAGCGGCAGATGTCGTGATGTTGCCCGTGTATCTATTGAGTGCTGCTGCCG
Sequence number 3513: Yersinia pestis Chromosomal hypothetical gene #250 
CGGTGGCCGTAGCGGCAGATGTCGTGATGTTGCCCGTGTATCTATTGAGTGCTGCTGCCGTGGCCGCT
TTCTATGGTGTGGTCTCATTGAATTAGTCGTTCTTTTTAAACCCGGCTAACA
Sequence number 3514: Yersinia pestis Chromosomal hypothetical gene #251 
TGGCCGCTTTCTATGGTGTGGTCTCATTGAATTAGTCGTTCTTTTTAAACCCGGCTAACAAAATAGAG
AGGGGTAGCCTACCGGGCATTAAACACAACAGTGTCCGGTATCGCCCAATAA
Sequence number 3515: Yersinia pestis Chromosomal hypothetical gene #252 
AAATAGAGAGGGGTAGCCTACCGGGCATTAAACACAACAGTGTCCGGTATCGCCCAATAATAATGTTT
GGCCGGTAAATGTTAACACTTGGTTTCACCCCGTTAAAATAATCCTCCCCAC
Sequence number 3516: Yersinia pestis Chromosomal hypothetical gene #253 
TAATGTTTGGCCGGTAAATGTTAACACTTGGTTTCACCCCGTTAAAATAATCCTCCCCACGGTAAAGT
TCCCCTTTTTAGTGCCGATAACACACTTAATTAAACGTCTTTGTGTTTTGAC
Sequence number 3517: Yersinia pestis Chromosomal hypothetical gene #254 
GGTAAAGTTCCCCTTTTTAGTGCCGATAACACACTTAATTAAACGTCTTTGTGTTTTGACGTGTCTGT
GTTTTGTCGTACTGATGAAGGGTTAATATGTTCAAGCGTATGAAAGTGGTTA
Sequence number 3518: Yersinia pestis Chromosomal hypothetical gene #255 
GTGTCTGTGTTTTGTCGTACTGATGAAGGGTTAATATGTTCAAGCGTATGAAAGTGGTTACCAGCCTG
CTGCTGGTATTAGTATTATTTGGTGCCTTACAACTGGTATCCGGCGGGCTTT
Sequence number 3519: Yersinia pestis Chromosomal hypothetical gene #256 
CCAGCCTGCTGCTGGTATTAGTATTATTTGGTGCCTTACAACTGGTATCCGGCGGGCTTTTTTTCAAA
TCATTGCAGAATGACCAGGAAAACTTCAACGTTTTACAAGTTATTCGCCAGC
Sequence number 3520: Yersinia pestis Chromosomal hypothetical gene #257 
TTTTCAAATCATTGCAGAATGACCAGGAAAACTTCAACGTTTTACAAGTTATTCGCCAGCAACAATCG
GTATTGAATGAAAGCTGGGTCTATCTGTTACAAACACGCAATACGTTAAACC
Sequence number 3521: Yersinia pestis Chromosomal hypothetical gene #258 
AACAATCGGTATTGAATGAAAGCTGGGTCTATCTGTTACAAACACGCAATACGTTAAACCGTGCCGGT
GTTCGTTACATGATGGATATTAACCATACTGGCAGTGGCCCTACGGTGAAGG
Sequence number 3522: Yersinia pestis Chromosomal hypothetical gene #259 
GTGCCGGTGTTCGTTACATGATGGATATTAACCATACTGGCAGTGGCCCTACGGTGAAGGACCTTATC
TCTTCGGCTAAAGGGACGCTAAGCGTCGCGGAAGACCGCTTCAAACGTTACG
Sequence number 3523: Yersinia pestis Chromosomal hypothetical gene #260 
ACCTTATCTCTTCGGCTAAAGGGACGCTAAGCGTCGCGGAAGACCGCTTCAAACGTTACGAAAAAATC
CCACAAAATAGCAGTCAGGATCCAGAAGGCGCCAAAAAATTAAAACAGTCCT
Sequence number 3524: Yersinia pestis Chromosomal hypothetical gene #261 
AAAAAATCCCACAAAATAGCAGTCAGGATCCAGAAGGCGCCAAAAAATTAAAACAGTCCTACGAGGAA
TATTTTAGTGCCTTAACCGAACTGATTAATTTGATGGAAACGAACAAAATCA
Sequence number 3525: Yersinia pestis Chromosomal hypothetical gene #262 
ACGAGGAATATTTTAGTGCCTTAACCGAACTGATTAATTTGATGGAAACGAACAAAATCAATGAGTTC
TTCAATCACCCCACCACCAGTTTCCAAAATGAATTTGAGCAAAATTACAACA
Sequence number 3526: Yersinia pestis Chromosomal hypothetical gene #263 
ATGAGTTCTTCAATCACCCCACCACCAGTTTCCAAAATGAATTTGAGCAAAATTACAACAACTATCTG
ACGCAAAATGACGGACTGTATGATTCGGCGGTCGAGGACAGTAATCAATCCT
Sequence number 3527: Yersinia pestis Chromosomal hypothetical gene #264 
ACTATCTGACGCAAAATGACGGACTGTATGATTCGGCGGTCGAGGACAGTAATCAATCCTTTAGCTTT
GCTATGGGCGTGGTTATTACTGTACTGGTCGTCGTATTAGTGATGATGCTCC
Sequence number 3528: Yersinia pestis Chromosomal hypothetical gene #265 
TTAGCTTTGCTATGGGCGTGGTTATTACTGTACTGGTCGTCGTATTAGTGATGATGCTCCTTGTCTGG
CTGGGGATGCAACAGATCTTAATTCACCCCCTCAACCACCTGATTGCACACA
Sequence number 3529: Yersinia pestis Chromosomal hypothetical gene #266 
TTGTCTGGCTGGGGATGCAACAGATCTTAATTCACCCCCTCAACCACCTGATTGCACACATTAAACAT
ATTGCCAACGGTGATTTGACGCAAAATATTGAAGTTCATGGTCGCAACGAAG
Sequence number 3530: Yersinia pestis Chromosomal hypothetical gene #267 
TTAAACATATTGCCAACGGTGATTTGACGCAAAATATTGAAGTTCATGGTCGCAACGAAGTGGGCACG
TTGGCTGCTAGCCTGAAACACATGCAATCTGAATTGGTCACTATCGTCGGCG
Sequence number 3531: Yersinia pestis Chromosomal hypothetical gene #268 
TGGGCACGTTGGCTGCTAGCCTGAAACACATGCAATCTGAATTGGTCACTATCGTCGGCGAGGTGCGT
GTAGGCGCTGATGCTATCTACAGCGGTGCGTCAGAAATTGCGGTGGGTAACA
Sequence number 3532: Yersinia pestis Chromosomal hypothetical gene #269 
AGGTGCGTGTAGGCGCTGATGCTATCTACAGCGGTGCGTCAGAAATTGCGGTGGGTAACAATGATTTA
TCCGCCCGTACTGAACAGCAAGCGGCATCATTGGAAGAAACGGCTGCCAGCA
Sequence number 3533: Yersinia pestis Chromosomal hypothetical gene #270 
ATGATTTATCCGCCCGTACTGAACAGCAAGCGGCATCATTGGAAGAAACGGCTGCCAGCATGGAGCAA
TTAACCGCAACGGTGAAACAGAACGCTGAAAACGCCCGTCAGGCGAGTCAAT
Sequence number 3534: Yersinia pestis Chromosomal hypothetical gene #271 
TGGAGCAATTAACCGCAACGGTGAAACAGAACGCTGAAAACGCCCGTCAGGCGAGTCAATTAGCATTG
AGTGCCTCAGAAACCGCTCAGAAAGGGGGGAAAGTGGTGGCGGATGTTGTGC
Sequence number 3535: Yersinia pestis Chromosomal hypothetical gene #272 
TAGCATTGAGTGCCTCAGAAACCGCTCAGAAAGGGGGGAAAGTGGTGGCGGATGTTGTGCAAACCATG
CACGAGATCGCGGGGAGTTCGCAGAAGATTGCCGACATTACCAGCGTCATCG
Sequence number 3536: Yersinia pestis Chromosomal hypothetical gene #273 
AAACCATGCACGAGATCGCGGGGAGTTCGCAGAAGATTGCCGACATTACCAGCGTCATCGACGGTATC
GCCTTCCAGACCAATATCTTGGCATTGAATGCCGCCGTTGAAGCCGCCCGTG
Sequence number 3537: Yersinia pestis Chromosomal hypothetical gene #274 
ACGGTATCGCCTTCCAGACCAATATCTTGGCATTGAATGCCGCCGTTGAAGCCGCCCGTGCAGGTGAG
CAAGGCCGTGGTTTTGCCGTGGTTGCCGGTGAAGTGCGCAATTTAGCACAAC
Sequence number 3538: Yersinia pestis Chromosomal hypothetical gene #275 
CAGGTGAGCAAGGCCGTGGTTTTGCCGTGGTTGCCGGTGAAGTGCGCAATTTAGCACAACGCAGTGCA
CAGGCGGCGAAGGAAATTAAAGGTCTGATTGACGATTCGGTTAACCGGGTAG
Sequence number 3539: Yersinia pestis Chromosomal hypothetical gene #276 
GCAGTGCACAGGCGGCGAAGGAAATTAAAGGTCTGATTGACGATTCGGTTAACCGGGTAGATCTGGGC
TCAGTACTGGTTGAAAGTGCCGGTGAAACCATGGGCGATATCGTTAATGCCG
Sequence number 3540: Yersinia pestis Chromosomal hypothetical gene #277 
ATCTGGGCTCAGTACTGGTTGAAAGTGCCGGTGAAACCATGGGCGATATCGTTAATGCCGTGACCCGC
GTGACCGACATCATGGGCGAAATCGCTTCTGCCTCAGATGAACAAAGTCGTG
Sequence number 3541: Yersinia pestis Chromosomal hypothetical gene #278 
TGACCCGCGTGACCGACATCATGGGCGAAATCGCTTCTGCCTCAGATGAACAAAGTCGTGGTATTGAC
CAGATTGGTCAGGCAGTTGCCGAAATGGACCGCGTGACACAGCAAAATGCTT
Sequence number 3542: Yersinia pestis Chromosomal hypothetical gene #279 
GTATTGACCAGATTGGTCAGGCAGTTGCCGAAATGGACCGCGTGACACAGCAAAATGCTTCCTTGGTA
GAAGAATCGGCTTCCGCCGCCGCTTCACTGGAAGAGCAAGCCAGCATGTTAA
Sequence number 3543: Yersinia pestis Chromosomal hypothetical gene #280 
CCTTGGTAGAAGAATCGGCTTCCGCCGCCGCTTCACTGGAAGAGCAAGCCAGCATGTTAACTCAAGCG
ATGTCCGTATTTGTTCTGAGTGTGGATAACAGTAATTCCACTAGCGACGTCA
Sequence number 3544: Yersinia pestis Chromosomal hypothetical gene #281 
CTCAAGCGATGTCCGTATTTGTTCTGAGTGTGGATAACAGTAATTCCACTAGCGACGTCAGAAAAGTC
AAACAACCCACGCAGGAAATGAACAGCACGGCTAAAAAAGCGCTGGGAAATA
Sequence number 3545: Yersinia pestis Chromosomal hypothetical gene #282 
GAAAAGTCAAACAACCCACGCAGGAAATGAACAGCACGGCTAAAAAAGCGCTGGGAAATAATCTGCAA
GACAATTGGGAAACTTTCTAGACCTATAACCTTCGAGCTTGACGCTGTTGAC
Sequence number 3546: Yersinia pestis Chromosomal hypothetical gene #283 
ATCTGCAAGACAATTGGGAAACTTTCTAGACCTATAACCTTCGAGCTTGACGCTGTTGACGTGTTAGC
TGCGCGCACTCATCCGAATCACAAACTTGAGTCAGCACATCGGGATCCGCCT
Sequence number 3547: Yersinia pestis Chromosomal hypothetical gene #284 
GTGTTAGCTGCGCGCACTCATCCGAATCACAAACTTGAGTCAGCACATCGGGATCCGCCTGCTGGCCA
TTTACCTGCCACGCCAATTGATTTGGGTGTATGCAAAATAATATTAATAAAG
Sequence number 3548: Yersinia pestis Chromosomal hypothetical gene #285 
GCTGGCCATTTACCTGCCACGCCAATTGATTTGGGTGTATGCAAAATAATATTAATAAAGTCGGCCTA
TGAGCCGGCTGAGAAGAGGTTACGATGTTTGGTCAAATCCGGATTTCTACCA
Sequence number 3549: Yersinia pestis Chromosomal hypothetical gene #286 
TCGGCCTATGAGCCGGCTGAGAAGAGGTTACGATGTTTGGTCAAATCCGGATTTCTACCAGTTTTTTC
CTGCTGCTGATGTTTCTTTGCGCCATACAGTTAATTTCAAGTGGTCTATCAT
Sequence number 3550: Yersinia pestis Chromosomal hypothetical gene #287 
GTTTTTTCCTGCTGCTGATGTTTCTTTGCGCCATACAGTTAATTTCAAGTGGTCTATCATTTACGGCT
TTTCGCTCAGACTACCAAAATTTAAATCGAGTGGAGTTAAGTAGTCAGCAAC
Sequence number 3551: Yersinia pestis Chromosomal hypothetical gene #288 
TTACGGCTTTTCGCTCAGACTACCAAAATTTAAATCGAGTGGAGTTAAGTAGTCAGCAACGCGATGCA
TTAAGCCTCAGTTGGGTATCTTTGCTTCAGGCACGTAATACCTTAAACCGTG
Sequence number 3552: Yersinia pestis Chromosomal hypothetical gene #289 
GCGATGCATTAAGCCTCAGTTGGGTATCTTTGCTTCAGGCACGTAATACCTTAAACCGTGCGGCTACT
CGCTCGGCGCTCAATGTTCCTCAGGAGCAAGTTAACGCTTTAATGGGGAGTG
Sequence number 3553: Yersinia pestis Chromosomal hypothetical gene #290 
CGGCTACTCGCTCGGCGCTCAATGTTCCTCAGGAGCAAGTTAACGCTTTAATGGGGAGTGCGCGCAGT
GCATTGCAGAAAGCTGAACTTTACTTTAATCAGTTTCAGGCGGTACCGCGCC
Sequence number 3554: Yersinia pestis Chromosomal hypothetical gene #291 
CGCGCAGTGCATTGCAGAAAGCTGAACTTTACTTTAATCAGTTTCAGGCGGTACCGCGCCTTGATGAG
AGTGAGAGCAGTGGGCAATTACTGGATGCGACCAAAAACAGTTATCAAAACC
Sequence number 3555: Yersinia pestis Chromosomal hypothetical gene #292 
TTGATGAGAGTGAGAGCAGTGGGCAATTACTGGATGCGACCAAAAACAGTTATCAAAACCTTCGTAGC
GCATTACGTGAGTTAATTGATTTTCTGGAAGCGGGTGATTTACAAGCATTTA
Sequence number 3556: Yersinia pestis Chromosomal hypothetical gene #293 
TTCGTAGCGCATTACGTGAGTTAATTGATTTTCTGGAAGCGGGTGATTTACAAGCATTTATGGATCAA
CCCACTCAGAAAACGCAAGATTTGTTTGAAGCGGATTTCATCCAGTATTTGC
Sequence number 3557: Yersinia pestis Chromosomal hypothetical gene #294 
TGGATCAACCCACTCAGAAAACGCAAGATTTGTTTGAAGCGGATTTCATCCAGTATTTGCAATACGCC
AATGAGGTTATTGCCGATGCCGGTCAGCAGAATCAGCAGGCTTATCAGCTCT
Sequence number 3558: Yersinia pestis Chromosomal hypothetical gene #295 
AATACGCCAATGAGGTTATTGCCGATGCCGGTCAGCAGAATCAGCAGGCTTATCAGCTCTCAATCTGG
ATATTTAGTGGTGCGATTCTGATGGTTATCACGATGGCCATTTCGTCGTTGA
Sequence number 3559: Yersinia pestis Chromosomal hypothetical gene #296 
CAATCTGGATATTTAGTGGTGCGATTCTGATGGTTATCACGATGGCCATTTCGTCGTTGATCTGGTTG
CGCAACATGTTTGTTCGGCCATTAGCGACGATGCGTGAGCATTTTGGCCGCA
Sequence number 3560: Yersinia pestis Chromosomal hypothetical gene #297 
TCTGGTTGCGCAACATGTTTGTTCGGCCATTAGCGACGATGCGTGAGCATTTTGGCCGCATTGCTCAA
GGGGATTTGTCTGGGCAGATCTCGGTGACTGGCCGTAATGAAATCAGCCAGA
Sequence number 3561: Yersinia pestis Chromosomal hypothetical gene #298 
TTGCTCAAGGGGATTTGTCTGGGCAGATCTCGGTGACTGGCCGTAATGAAATCAGCCAGATGTTCGCC
AGCTTACGCACCATGCAGCAGTCGCTGATCTCAACGGTCAGCAATGTGCGTG
Sequence number 3562: Yersinia pestis Chromosomal hypothetical gene #299 
TGTTCGCCAGCTTACGCACCATGCAGCAGTCGCTGATCTCAACGGTCAGCAATGTGCGTGAGGGTACC
GAATCGATGTTGACCGGTATCCAGGAAATTTCCGCAGGTAACAACGATTTAT
Sequence number 3563: Yersinia pestis Chromosomal hypothetical gene #300 
AGGGTACCGAATCGATGTTGACCGGTATCCAGGAAATTTCCGCAGGTAACAACGATTTATCGGCAAGA
ACTGAACAGCAAGCGGCTTCCTTGGAACAAACTGCCGCGAGTATGGAACAAC
Sequence number 3564: Yersinia pestis Chromosomal hypothetical gene #301 
CGGCAAGAACTGAACAGCAAGCGGCTTCCTTGGAACAAACTGCCGCGAGTATGGAACAACTGACCGCC
ACGGTAAAACAGAATGCGGACAATGCGCATCAGGCCACGGTACTGGCGCAAG
Sequence number 3565: Yersinia pestis Chromosomal hypothetical gene #302 
TGACCGCCACGGTAAAACAGAATGCGGACAATGCGCATCAGGCCACGGTACTGGCGCAAGAGGCTTCC
GGTACGGCAGCTAAAGGCGGAGAACTGACGGCGAGTGTCGTCACCACCATGC
Sequence number 3566: Yersinia pestis Chromosomal hypothetical gene #303 
AGGCTTCCGGTACGGCAGCTAAAGGCGGAGAACTGACGGCGAGTGTCGTCACCACCATGCATGCAATC
GCTACCAGTTCGCAGAAGATTGGTGCGATCACCAGCGTTATCGACGGTATTG
Sequence number 3567: Yersinia pestis Chromosomal hypothetical gene #304 
ATGCAATCGCTACCAGTTCGCAGAAGATTGGTGCGATCACCAGCGTTATCGACGGTATTGCCTTCCAA
ACCAATATTTTGGCACTGAATGCGGCCGTGGAGGCGGCCCGTGCGGGTGAGC
Sequence number 3568: Yersinia pestis Chromosomal hypothetical gene #305 
CCTTCCAAACCAATATTTTGGCACTGAATGCGGCCGTGGAGGCGGCCCGTGCGGGTGAGCAAGGGCGT
GGTTTTGCGGTGGTTGCGGGTGAAGTCCGTAATTTAGCATAACGCAGTGCGC
Sequence number 3569: Yersinia pestis Chromosomal hypothetical gene #306 
AAGGGCGTGGTTTTGCGGTGGTTGCGGGTGAAGTCCGTAATTTAGCATAACGCAGTGCGCAAGCGGCC
AAAGAGATCAAAGGGCTGATTGATGAGTCCGTCAGCCGTGTCCGCCAGGGGT
Sequence number 3570: Yersinia pestis Chromosomal hypothetical gene #307 
AAGCGGCCAAAGAGATCAAAGGGCTGATTGATGAGTCCGTCAGCCGTGTCCGCCAGGGGTCTACGCTG
GTCGAAAGTGCAGGAACTACCATGGAAGAGATCGTCCGCTCGGTGGCGCGTG
Sequence number 3571: Yersinia pestis Chromosomal hypothetical gene #308 
CTACGCTGGTCGAAAGTGCAGGAACTACCATGGAAGAGATCGTCCGCTCGGTGGCGCGTGTGACTGAC
ATTATGGGGGAGATCGAATCTGCTTCGGATGAACAGAGCCGGGGTATTGAAC
Sequence number 3572: Yersinia pestis Chromosomal hypothetical gene #309 
TGACTGACATTATGGGGGAGATCGAATCTGCTTCGGATGAACAGAGCCGGGGTATTGAACAAATATCA
CTCGCCGTAACGCAAATGGATCAGGTTACTCAGCAAAATGCGTCATTAGTGG
Sequence number 3573: Yersinia pestis Chromosomal hypothetical gene #310 
AAATATCACTCGCCGTAACGCAAATGGATCAGGTTACTCAGCAAAATGCGTCATTAGTGGAAGAGGCC
GCGGCGGCAGCCAATGCACTGGAAGAGCAGGCCAGCATGCTCTCCGATGCCG
Sequence number 3574: Yersinia pestis Chromosomal hypothetical gene #311 
AAGAGGCCGCGGCGGCAGCCAATGCACTGGAAGAGCAGGCCAGCATGCTCTCCGATGCCGTCTCTGTT
TTCCGTTTGGGGCAGGGGCACGACGAACAGTCAGTTGCGGGCAATAGCCAGC
Sequence number 3575: Yersinia pestis Chromosomal hypothetical gene #312 
TCTCTGTTTTCCGTTTGGGGCAGGGGCACGACGAACAGTCAGTTGCGGGCAATAGCCAGCAATCATTG
GCCACGCATTATTAGTCGCTCATTATGGGTCGATCAGCATGAGTGGTTCCGA
Sequence number 3576: Yersinia pestis Chromosomal hypothetical gene #313 
AATCATTGGCCACGCATTATTAGTCGCTCATTATGGGTCGATCAGCATGAGTGGTTCCGACCAGGAGT
GACAATCGATCATCACTCGTAACGGGTCATCACGAGTAACGACAAGTGGCTG
Sequence number 3577: Yersinia pestis Chromosomal hypothetical gene #314 
CCAGGAGTGACAATCGATCATCACTCGTAACGGGTCATCACGAGTAACGACAAGTGGCTGTAAAGGAA
ATCGTAGAGTGTCAAACAGCGTAACGAAGACGGTATTGGGGGGTAAGGTTGG
Sequence number 3578: Yersinia pestis Chromosomal hypothetical gene #315 
TAAAGGAAATCGTAGAGTGTCAAACAGCGTAACGAAGACGGTATTGGGGGGTAAGGTTGGATGAAACG
ATCATCAATGAAGTCAGCGCCTCAATCATTACCTCAAGAGTCCGGGTCGATA
Sequence number 3579: Yersinia pestis Chromosomal hypothetical gene #316 
ATGAAACGATCATCAATGAAGTCAGCGCCTCAATCATTACCTCAAGAGTCCGGGTCGATACTGACCCA
AATGATTCAACGGTTGCCGCTGTCGGATATTCACTTCCGGCGTATCTGTCAA
Sequence number 3580: Yersinia pestis Chromosomal hypothetical gene #317 
CTGACCCAAATGATTCAACGGTTGCCGCTGTCGGATATTCACTTCCGGCGTATCTGTCAACTTATCTA
TCAACGGGCTGGAATTGTTCTGGCTGAACATAAGCGGGAAATGGTCTATAAC
Sequence number 3581: Yersinia pestis Chromosomal hypothetical gene #318 
CTTATCTATCAACGGGCTGGAATTGTTCTGGCTGAACATAAGCGGGAAATGGTCTATAACCGCCTGGT
CAGACGGTTGAGATTACTGAATATCTCTGATTTTGGTCAGTATCTGGCCTTG
Sequence number 3582: Yersinia pestis Chromosomal hypothetical gene #319 
CGCCTGGTCAGACGGTTGAGATTACTGAATATCTCTGATTTTGGTCAGTATCTGGCCTTGTTGGAGAG
TGATCCGAACAGTGCTGAGTGGCAGGCATTTGTGAATGCGTTGACCACTAAC
Sequence number 3583: Yersinia pestis Chromosomal hypothetical gene #320 
TTGGAGAGTGATCCGAACAGTGCTGAGTGGCAGGCATTTGTGAATGCGTTGACCACTAACCTGACGGC
ATTTTTTCGTGAGGCTCATCATTTTCCGCTCTTGGCAGAACATGCTCGTCAG
Sequence number 3584: Yersinia pestis Chromosomal hypothetical gene #321 
CTGACGGCATTTTTTCGTGAGGCTCATCATTTTCCGCTCTTGGCAGAACATGCTCGTCAGCGGGCGGG
CGGTTATTCAGTCTGGAGTACGGCGGCATCAACCGGTGAAGAACCTTACTCC
Sequence number 3585: Yersinia pestis Chromosomal hypothetical gene #322 
CGGGCGGGCGGTTATTCAGTCTGGAGTACGGCGGCATCAACCGGTGAAGAACCTTACTCCATCGCTAT
GACGCTGTGTGATGTGTTAGGAAATCGCGCGGGAGCCTGCCAGATATTGGCC
Sequence number 3586: Yersinia pestis Chromosomal hypothetical gene #323 
ATCGCTATGACGCTGTGTGATGTGTTAGGAAATCGCGCGGGAGCCTGCCAGATATTGGCCAGCGATAT
CGATACACAGGTACTGGAGAAAGCTACCAGCGGTGTATATCGGCAAGATGAA
Sequence number 3587: Yersinia pestis Chromosomal hypothetical gene #324 
AGCGATATCGATACACAGGTACTGGAGAAAGCTACCAGCGGTGTATATCGGCAAGATGAATTGCGCTC
ATTGTCAACCCAGCAGATGCAACGCTACTTTTTGCGAGGTACGGGCCCGCAT
Sequence number 3588: Yersinia pestis Chromosomal hypothetical gene #325 
TTGCGCTCATTGTCAACCCAGCAGATGCAACGCTACTTTTTGCGAGGTACGGGCCCGCATCAGGGCAT
GGTGCGAGTTCGTCCTGAACTGACCAATTTAGTCCAGTTTCAGCAGTTGAAT
Sequence number 3589: Yersinia pestis Chromosomal hypothetical gene #326 
CAGGGCATGGTGCGAGTTCGTCCTGAACTGACCAATTTAGTCCAGTTTCAGCAGTTGAATTTGCTGGC
ACCTGAGTGGGCGCTACCGGGGCAATTTGATGCCATTTTTTGCCGTAATGTC
Sequence number 3590: Yersinia pestis Chromosomal hypothetical gene #327 
TTGCTGGCACCTGAGTGGGCGCTACCGGGGCAATTTGATGCCATTTTTTGCCGTAATGTCATGATCTA
TTTTGATAAAGAGACGCAGGAACGCATTCTGCGTCGTTTTGTACCTTTGCTA
Sequence number 3591: Yersinia pestis Chromosomal hypothetical gene #328 
ATGATCTATTTTGATAAAGAGACGCAGGAACGCATTCTGCGTCGTTTTGTACCTTTGCTAAAACCAGG
CGGCCTGATGTTCGCTGGCCACTCGGAGAATTTCAGCCAGATCAGTCGGGAA
Sequence number 3592: Yersinia pestis Chromosomal hypothetical gene #329 
GTACCTTTGCTAAAACCAGGCGGCCTGATGTTCGCTGGCCACTCGGAGAATTTCAGCCAGATCAGTCG
GGAATTCTATTTGCGCGGGCAGACCGTTTATGGGCTGACCAAGGAGAGGTGA
Sequence number 3593: Yersinia pestis F1 operon #1 
TAACTCTTTGGTAATCCTAATGTTACTGACATTTCCGGCTTCACAATATTATTGTGATATACCTGCAT
TTCATTGTAAAACTCCATGTCATCACAATAAATCGGGAGAAAATAATGACAA
Sequence number 3594: Yersinia pestis F1 operon #2 
ACCTGCATTTCATTGTAAAACTCCATGTCATCACAATAAATCGGGAGAAAATAATGACAATCAATAGT
ATTGTCATTTCGTCTTTTTATGACCTCCACATCGTAACCATCCCGCTTATTT
Sequence number 3595: Yersinia pestis F1 operon #3 
TCAATAGTATTGTCATTTCGTCTTTTTATGACCTCCACATCGTAACCATCCCGCTTATTTAAATTATA
AATAGGCAAAGAGTTATAATATATGTTGTACATGTGATGAACATACTGATCA
Sequence number 3596: Yersinia pestis F1 operon #4 
AAATTATAAATAGGCAAAGAGTTATAATATATGTTGTACATGTGATGAACATACTGATCATATGTATC
ATTGAAGAAAAAATAAGCATACAGGCCTTTATCTACCTTTATTTCACTATCG
Sequence number 3597: Yersinia pestis F1 operon #5 
TATGTATCATTGAAGAAAAAATAAGCATACAGGCCTTTATCTACCTTTATTTCACTATCGCTGAAATG
CTTATTCCTATCCCAAACAACCGTTCTTGCAATAATGTCATTCGTTTTATCA
Sequence number 3598: Yersinia pestis F1 operon #6 
CTGAAATGCTTATTCCTATCCCAAACAACCGTTCTTGCAATAATGTCATTCGTTTTATCATGAAAGGG
GATTCTGTTTGATACTGTTATAGCTGTATTTTTCTTCAACGAATCATATAAC
Sequence number 3599: Yersinia pestis F1 operon #7 
TGAAAGGGGATTCTGTTTGATACTGTTATAGCTGTATTTTTCTTCAACGAATCATATAACTTACCCAA
TCTACATTTTGAATCTATCCCAGAGTAGAACACTAAATCCCTAAAATTAAAG
Sequence number 3600: Yersinia pestis F1 operon #8 
TTACCCAATCTACATTTTGAATCTATCCCAGAGTAGAACACTAAATCCCTAAAATTAAAGCATTGACC
AATTATATTTCTCTCTTTAAGGTAACAGATTCGTGGTTGAAGGTATTCACAG
Sequence number 3601: Yersinia pestis F1 operon #9 
CATTGACCAATTATATTTCTCTCTTTAAGGTAACAGATTCGTGGTTGAAGGTATTCACAGTTAATTTC
CCTTCTACCCAACAAACCTTTAAAGGACCAAAAAGGGATCATCCTATACTGC
Sequence number 3602: Yersinia pestis F1 operon #10 
TTAATTTCCCTTCTACCCAACAAACCTTTAAAGGACCAAAAAGGGATCATCCTATACTGCCGTGGGGT
ATAACCAAATATTTTCTTAAATTCTCTGGTGAATGTCTGTTGCGAATCATAA
Sequence number 3603: Yersinia pestis F1 operon #11 
CGTGGGGTATAACCAAATATTTTCTTAAATTCTCTGGTGAATGTCTGTTGCGAATCATAAAAAAGCTT
TGCTGATATCTCTATTATTGTCAGCCTGGTAAGCCGTAATAGTGCAGCAGCT
Sequence number 3604: Yersinia pestis F1 operon #12 
AAAAGCTTTGCTGATATCTCTATTATTGTCAGCCTGGTAAGCCGTAATAGTGCAGCAGCTCTACTAGC
CCTTCTAACTCTAATATATGTTCCAATAGGCATTCCGACATATTCCTTAAAG
Sequence number 3605: Yersinia pestis F1 operon #13 
CTACTAGCCCTTCTAACTCTAATATATGTTCCAATAGGCATTCCGACATATTCCTTAAAGGAAATTTG
CAAATACCTTCTGCTGAATCCTGAATACAAAACCAAACAGTCAATGTTAATG
Sequence number 3606: Yersinia pestis F1 operon #14 
GAAATTTGCAAATACCTTCTGCTGAATCCTGAATACAAAACCAAACAGTCAATGTTAATGAATTTCGA
CTCGAGATTCTCTTCTATATATTGAATAATTGAATTTACAGTCATCTGTTTT
Sequence number 3607: Yersinia pestis F1 operon #15 
AATTTCGACTCGAGATTCTCTTCTATATATTGAATAATTGAATTTACAGTCATCTGTTTTAGCATGAA
TATAACCCAAATCAAAATAATAGCATTCTAGATAGTGGGCCGGCGCCGGGAC
Sequence number 3608: Yersinia pestis F1 operon #16 
AGCATGAATATAACCCAAATCAAAATAATAGCATTCTAGATAGTGGGCCGGCGCCGGGACTTAGCTAT
TTGCGCATACCCAGCAACACCAATCTTAGCTATTTGTGCACGCGCATCAATA
Sequence number 3609: Yersinia pestis F1 operon #17 
TTAGCTATTTGCGCATACCCAGCAACACCAATCTTAGCTATTTGTGCACGCGCATCAATATCAAAATT
AGCTATTTGCGCAACAAGCAAGTGGAGTGCGCGAAAAGCTAAACTTTGTGTG
Sequence number 3610: Yersinia pestis F1 operon #18 
TCAAAATTAGCTATTTGCGCAACAAGCAAGTGGAGTGCGCGAAAAGCTAAACTTTGTGTGCATTTTTA
AATAAAATTGTTCTCAGTGAGGCTGTGCTACGGATATAAAAATCCCCTTCAT
Sequence number 3611: Yersinia pestis F1 operon #19 
CATTTTTAAATAAAATTGTTCTCAGTGAGGCTGTGCTACGGATATAAAAATCCCCTTCATTTGTTACC
CACCTTTTTACGCATATCGTCGATATGAAATGATGGGGAGGGGGTGGGAAGG
Sequence number 3612: Yersinia pestis F1 operon #20 
TTGTTACCCACCTTTTTACGCATATCGTCGATATGAAATGATGGGGAGGGGGTGGGAAGGTGTTGTCA
CCATTCCGTAAGGAGGTTAAGCTCATGATTTTAAATAGATTAAGTACGTTAG
Sequence number 3613: Yersinia pestis F1 operon #21 
TGTTGTCACCATTCCGTAAGGAGGTTAAGCTCATGATTTTAAATAGATTAAGTACGTTAGGAATTATT
ACTTTCGGCATGCTTAGTTTTGCTGCGAACTCTGCTCAACCAGATATCAAAT
Sequence number 3614: Yersinia pestis F1 operon #22 
GAATTATTACTTTCGGCATGCTTAGTTTTGCTGCGAACTCTGCTCAACCAGATATCAAATTCGCAAGC
AAAGAGTATGGCGTGACTATAGGTGAGAGTAGGATCATATACCCGTTAGATG
Sequence number 3615: Yersinia pestis F1 operon #23 
TCGCAAGCAAAGAGTATGGCGTGACTATAGGTGAGAGTAGGATCATATACCCGTTAGATGCTGCTGGC
GTTATGGTCTCGGTGAAAAACACCCAAGATTATCCGGTTCTCATTCAGTCTA
Sequence number 3616: Yersinia pestis F1 operon #24 
CTGCTGGCGTTATGGTCTCGGTGAAAAACACCCAAGATTATCCGGTTCTCATTCAGTCTAGGATCTAC
GACGAGAATAAAGAAAAAGAATCAGAGGATCCTTTCGTGGTCACTCCGCCAT
Sequence number 3617: Yersinia pestis F1 operon #25 GGATCTACGACGAGAATAAAGAAAAAGAATCAGAGGATCCTTTCGTGGTCACTCCGCCATTGTTTCGA TTGGATGCTAAGCAACAAAATTCTTTGCGTATAGCTCAGGCTGGAGGTGTTT
Sequence number 3618: Yersinia pestis F1 operon #26 
TGTTTCGATTGGATGCTAAGCAACAAAATTCTTTGCGTATAGCTCAGGCTGGAGGTGTTTTCCCGCGA
GATAAAGAGAGCCTAAAGTGGTTATGCGTAAAAGGGATTCCACCAAAGGATG
Sequence number 3619: Yersinia pestis F1 operon #27 
TCCCGCGAGATAAAGAGAGCCTAAAGTGGTTATGCGTAAAAGGGATTCCACCAAAGGATGAAGATATA
TGGGTTGATGATGCGACAAATAAGCAAAAATTCAATCCAGACAAAGATGTGG
Sequence number 3620: Yersinia pestis F1 operon #28 
AAGATATATGGGTTGATGATGCGACAAATAAGCAAAAATTCAATCCAGACAAAGATGTGGGAGTGTTC
GTGCAATTCGCAATTAATAATTGCATTAAGCTTTTGGTTCGACCGAATGAAT
Sequence number 3621: Yersinia pestis F1 operon #29 
GAGTGTTCGTGCAATTCGCAATTAATAATTGCATTAAGCTTTTGGTTCGACCGAATGAATTAAAAGGA
ACCCCTATACAGTTTGCTGAAAACTTAAGCTGGAAAGTTGATGGGGGGAAGC
Sequence number 3622: Yersinia pestis F1 operon #30 
TAAAAGGAACCCCTATACAGTTTGCTGAAAACTTAAGCTGGAAAGTTGATGGGGGGAAGCTAATTGCT
GAAAACCCCTCACCTTTCTACATGAACATAGGTGAATTAACATTTGGAGGGA
Sequence number 3623: Yersinia pestis F1 operon #31 
TAATTGCTGAAAACCCCTCACCTTTCTACATGAACATAGGTGAATTAACATTTGGAGGGAAAAGTATT
CCTTCTCACTATATTCCACCTAAATCGACGTGGGCTTTTGATTTGCCAAAAG
Sequence number 3624: Yersinia pestis F1 operon #32 
AAAGTATTCCTTCTCACTATATTCCACCTAAATCGACGTGGGCTTTTGATTTGCCAAAAGGACTAGCG
GGAGCACGTAATGTTTCGTGGAGAATAATTAATGATCAGGGAGGGTTGGATC
Sequence number 3625: Yersinia pestis F1 operon #33 
AAATCGACGTGGGCTTTTGATTTGCCAAAAGGACTAGCGGGAGCACGTAATGTTTCGTGGAGAATAAT
TAATGATCAGGGAGGGTTGGATCGTTTGTATTCCAAAAATGTGACTTTATGA
Sequence number 3626: Yersinia pestis pla #1 
TGAAGAAAAGTTCTATTGTGGCAACCATTATAACTATTCTGTCCGGGAGTGCTAATGCAGCATCATCT
CAGTTAATACCAAATATATCCCCTGACAGCTTTACAGTTGCAGCCTCCACCG
Sequence number 3627: Yersinia pestis pla #2 
CATCATCTCAGTTAATACCAAATATATCCCCTGACAGCTTTACAGTTGCAGCCTCCACCGGGATGCTG
AGTGGAAAGTCTCATGAAATGCTTTATGACGCAGAAACAGGAAGAAAGATCA
Sequence number 3628: Yersinia pestis pla #3 
GGATGCTGAGTGGAAAGTCTCATGAAATGCTTTATGACGCAGAAACAGGAAGAAAGATCAGCCAGTTA
GACTGGAAGATCAAAAATGTCGCTATCCTGAAAGGTGATATATCCTGGGATC
Sequence number 3629: Yersinia pestis pla #4 
GCCAGTTAGACTGGAAGATCAAAAATGTCGCTATCCTGAAAGGTGATATATCCTGGGATCCATACTCA
TTTCTGACCCTGAATGCCAGGGGGTGGACGTCTCTGGCTTCCGGGTCAGGTA
Sequence number 3630: Yersinia pestis pla #5 
CATACTCATTTCTGACCCTGAATGCCAGGGGGTGGACGTCTCTGGCTTCCGGGTCAGGTAATATGGAT
GACTACGACTGGATGAATGAAAATCAATCTGAGTGGACAGATCACTCATCTC
Sequence number 3631: Yersinia pestis pla #6 
ATATGGATGACTACGACTGGATGAATGAAAATCAATCTGAGTGGACAGATCACTCATCTCATCCTGCT
ACAAATGTTAATCATGCCAATGAATATGACCTCAATGTGAAAGGCTGGTTAC
Sequence number 3632: Yersinia pestis pla #7 
ATCCTGCTACAAATGTTAATCATGCCAATGAATATGACCTCAATGTGAAAGGCTGGTTACTCCAGGAT
GAGAATTATAAAGCAGGTATAACAGCAGGATATCAGGAAACACGTTTCAGTT
Sequence number 3633: Yersinia pestis pla #8 
TCCAGGATGAGAATTATAAAGCAGGTATAACAGCAGGATATCAGGAAACACGTTTCAGTTGGACAGCT
ACAGGTGGTTCATATAGTTATAATAATGGAGCTTATACCGGAAACTTCCCGA
Sequence number 3634: Yersinia pestis pla #9 
GGACAGCTACAGGTGGTTCATATAGTTATAATAATGGAGCTTATACCGGAAACTTCCCGAAAGGAGTG
CGGGTAATAGGTTATAACCAGCGCTTTTCTATGCCATATATTGGACTTGCAG
Sequence number 3635: Yersinia pestis pla #10 
AAGGAGTGCGGGTAATAGGTTATAACCAGCGCTTTTCTATGCCATATATTGGACTTGCAGGCCAGTAT
CGCATTAATGATTTTGAGTTAAATGCATTATTTAAATTCAGCGACTGGGTTC
Sequence number 3636: Yersinia pestis pla #11 
GCCAGTATCGCATTAATGATTTTGAGTTAAATGCATTATTTAAATTCAGCGACTGGGTTCGGGCACAT
GATAATGATGAGCACTATATGAGAGATCTTACTTTCCGTGAGAAGACATCCG
Sequence number 3637: Yersinia pestis pla #12 
GGGCACATGATAATGATGAGCACTATATGAGAGATCTTACTTTCCGTGAGAAGACATCCGGCTCACGT
TATTATGGTACCGTAATTAACGCTGGATATTATGTCACACCTAATGCCAAAG
Sequence number 3638: Yersinia pestis pla #13 
GCTCACGTTATTATGGTACCGTAATTAACGCTGGATATTATGTCACACCTAATGCCAAAGTCTTTGCG
GAATTTACATACAGTAAATATGATGAGGGCAAAGGAGGTACTCAGACCATTG
Sequence number 3639: Yersinia pestis pla #14 
TCTTTGCGGAATTTACATACAGTAAATATGATGAGGGCAAAGGAGGTACTCAGACCATTGATAAGAAT
AGTGGAGATTCTGTCTCTATTGGCGGAGATGCTGCCGGTATTTCCAATAAAA
Sequence number 3640: Yersinia pestis pla #15 
ATAAGAATAGTGGAGATTCTGTCTCTATTGGCGGAGATGCTGCCGGTATTTCCAATAAAAATTATACT
GTGACGGCGGGTCTGCAATATCGCTTCTGAAAAATACAGATCATATCTCTCT
Sequence number 3641: Yersinia pestis pla #16 
ATTATACTGTGACGGCGGGTCTGCAATATCGCTTCTGAAAAATACAGATCATATCTCTCTTTTCATCC
TCCCCTAGCGGGGAGGATGTCTGTGGAAAGGAGGTTGGTGTTTGACCAACCT
Sequence number 3642: Yersinia pestis pla #17 
TTTCATCCTCCCCTAGCGGGGAGGATGTCTGTGGAAAGGAGGTTGGTGTTTGACCAACCTTCAGATGT
GTGAAAAATCACCTTTTTCACCATAATGACGGGGCGCTCATTCTGTTGTTTT
Sequence number 3643: Yersinia pestis pla #18 
TCAGATGTGTGAAAAATCACCTTTTTCACCATAATGACGGGGCGCTCATTCTGTTGTTTTGCCTTGAC
ATTCTCCACGTCTTTCAGGGCATGGAGAAGGTCAAATTAGACATGGAACGCT
Sequence number 3644: Yersinia pestis pla #19 
GCCTTGACATTCTCCACGTCTTTCAGGGCATGGAGAAGGTCAAATTAGACATGGAACGCTACTCTCCT
TCCTGTAGGAAGCTCAACATCCAAGCTTAATTTGCCTCCCATTGCTTCAACG
Sequence number 3645: Yersinia pestis pla #20 ACTCTCCTTCCTGTAGGAAGCTCAACATCCAAGCTTAATTTGCCTCCCATTGCTTCAACGTAACGCTT TAACGTCGCCAGCTTTAAATCATTTCCGCGCTGCTCCAGCTTTGTTACTGCT
Sequence number 3646: Yersinia pestis pla #21 
TAACGCTTTAACGTCGCCAGCTTTAAATCATTTCCGCGCTGCTCCAGCTTTGTTACTGCTGGCTGGCT
TATACCCATCGCCTCAGCAACTTGTTTTTGTGATAACTGGAGTTCTTCACGC
Sequence number 3647: Yersinia pestis pla #22 
GGCTGGCTTATACCCATCGCCTCAGCAACTTGTTTTTGTGATAACTGGAGTTCTTCACGCATCATCTG
CAAGCCGACCTCAAGAATCATCTCATCTGCCATTTCTTTAATTCGTGTCTGG
Sequence number 3648: Yersinia pestis pla #23 
CTGGAGTTCTTCACGCATCATCTGCAAGCCGACCTCAAGAATCATCTCATCTGCCATTTCTTTAATTC
GTGTCTGGCTTTCAGGTGAACGACTGGCAATCACCTCATCTAATGTTCTCAT
Sequence number 3649: Zika virus Whole genome #1 
AGTTGTTGATCTGTGTGAATCAGACTGCGACAGTTCGAGTTTGAAGCGAGAGCTAACAACAGTATCAA
CAGGTTTAATTTGGATTTGGAAACGAGAGTTTCTGGTCATGAAAAACCCAAA
Sequence number 3650: Zika virus Whole genome #2 
AGTATCAACAGGTTTAATTTGGATTTGGAAACGAGAGTTTCTGGTCATGAAAAACCCAAAGAAGAAAT
CCGGAGGATTCCGGATTGTCAATATGCTAAAACGCGGAGTAGCCCGTGTAAA
Sequence number 3651: Zika virus Whole genome #3 
GAAGAAATCCGGAGGATTCCGGATTGTCAATATGCTAAAACGCGGAGTAGCCCGTGTAAACCCCTTGG
GGGGTTTGAAGAGGCTGCCAGCCGGACTTCTGCTGGGCCATGGACCCATCAG
Sequence number 3652: Zika virus Whole genome #4 
CCCCTTGGGGGGTTTGAAGAGGCTGCCAGCCGGACTTCTGCTGGGCCATGGACCCATCAGAATGGTTT
TGGCGATACTAGCCTTCTTGAGATTCACAGCAATCAAACCATCACTGGGCCT
Sequence number 3653: Zika virus Whole genome #5 
AATGGTTTTGGCGATACTAGCCTTCTTGAGATTCACAGCAATCAAACCATCACTGGGCCTCATCAATA
GATGGGGTTCCGTGGGGAAGAAGGAGGCTATGGAAATAATAAAAAAGTTCAA
Sequence number 3654: Zika virus Whole genome #6 
CATCAATAGATGGGGTTCCGTGGGGAAGAAGGAGGCTATGGAAATAATAAAAAAGTTCAAGAAAGACC
TTGCTGCCATGTTGAGAATTATCAATGCTAGGAAGGAGAGGAAGAGACGTGG
Sequence number 3655: Zika virus Whole genome #7 
GAAAGACCTTGCTGCCATGTTGAGAATTATCAATGCTAGGAAGGAGAGGAAGAGACGTGGCGCTGACA
CCAGCATCGGAATCGTCGGCCTCCTGCTGACCACAGCCATGGCAGCCGAGAT
Sequence number 3656: Zika virus Whole genome #8 
CGCTGACACCAGCATCGGAATCGTCGGCCTCCTGCTGACCACAGCCATGGCAGCCGAGATCACTAGAC
GTGGGAGTGCATACTACATGTACTTGGACAGGAGCGATGCTGGTAAGGCCAT
Sequence number 3657: Zika virus Whole genome #9 
CACTAGACGTGGGAGTGCATACTACATGTACTTGGACAGGAGCGATGCTGGTAAGGCCATTTCTTTTG
CCACCACATTGGGGGTGAACAAATGCCATGTACAGATCATGGACCTCGGGCA
Sequence number 3658: Zika virus Whole genome #10 
TTCTTTTGCCACCACATTGGGGGTGAACAAATGCCATGTACAGATCATGGACCTCGGGCACATGTGTG
ACGCCACCATGAGTTATGAGTGCCCCATGCTAGACGAGGGAGTGGAGCCAGA
Sequence number 3659: Zika virus Whole genome #11 
CATGTGTGACGCCACCATGAGTTATGAGTGCCCCATGCTAGACGAGGGAGTGGAGCCAGATGACGTCG
ATTGCTGGTGCAACACGACATCGACTTGGGTTGTGTACGGAACCTGTCATCA
Sequence number 3660: Zika virus Whole genome #12 
TGACGTCGATTGCTGGTGCAACACGACATCGACTTGGGTTGTGTACGGAACCTGTCATCATAAAAAAG
GTGAAGCACGACGATCCAGAAGAGCCGTGACGCTTCCTTCTCACTCCACGAG
Sequence number 3661: Zika virus Whole genome #13 
TAAAAAAGGTGAAGCACGACGATCCAGAAGAGCCGTGACGCTTCCTTCTCACTCCACGAGGAAGCTGC
AAACGCGATCGCAGACTTGGCTAGAGTCAAGAGAATACACAAAGCACCTGAT
Sequence number 3662: Zika virus Whole genome #14 
GAAGCTGCAAACGCGATCGCAGACTTGGCTAGAGTCAAGAGAATACACAAAGCACCTGATCAAGGTTG
AAAATTGGATATTTAGGAACCCCGGGTTTGCGCTAGTGGCTGTAGCTATAGC
Sequence number 3663: Zika virus Whole genome #15 
CAAGGTTGAAAATTGGATATTTAGGAACCCCGGGTTTGCGCTAGTGGCTGTAGCTATAGCCTGGCTCC
TGGGAAGCTCGACGAGCCAAAAAGTTATATACTTGGTCATGATATTGTTGAT
Sequence number 3664: Zika virus Whole genome #16 
CTGGCTCCTGGGAAGCTCGACGAGCCAAAAAGTTATATACTTGGTCATGATATTGTTGATTGCCCCGG
CATACAGCATTAGGTGCATAGGAGTTAGCAATAGAGATTTCGTGGAGGGCAT
Sequence number 3665: Zika virus Whole genome #17 
TGCCCCGGCATACAGCATTAGGTGCATAGGAGTTAGCAATAGAGATTTCGTGGAGGGCATGTCAGGTG
GGACCTGGGTTGATGTTGTCTTGGAACATGGGGGTTGCGTCACCGTGATGGC
Sequence number 3666: Zika virus Whole genome #18 
GTCAGGTGGGACCTGGGTTGATGTTGTCTTGGAACATGGGGGTTGCGTCACCGTGATGGCACAGGACA
AGCCAACAGTTGATATCGAGTTGGTCACGACAACGGTTAGCAACATGGCCGA
Sequence number 3667: Zika virus Whole genome #19 
ACAGGACAAGCCAACAGTTGATATCGAGTTGGTCACGACAACGGTTAGCAACATGGCCGAGGTAAGAT
CCTACTGCTATGAGGCATCAATATCGGACATGGCTTCGGACAGCCGTTGTCC
Sequence number 3668: Zika virus Whole genome #20 
GGTAAGATCCTACTGCTATGAGGCATCAATATCGGACATGGCTTCGGACAGCCGTTGTCCAACACAAG
GTGAAGCCTACCTTGACAAGCAGTCAGACACTCAATATGTCTGCAAGAGAAC
Sequence number 3669: Zika virus Whole genome #21 
AACACAAGGTGAAGCCTACCTTGACAAGCAGTCAGACACTCAATATGTCTGCAAGAGAACATTGGTGG
ATAGAGGTTGGGGAAATGGGTGTGGACTTTTTGGCAAAGGGAGCTTGGTGAC
Sequence number 3670: Zika virus Whole genome #22 
ATTGGTGGATAGAGGTTGGGGAAATGGGTGTGGACTTTTTGGCAAAGGGAGCTTGGTGACATGTGCCA
AGTTTACGTGCTCCAAGAAAATGACAGGCAAGAGCATCCAGCCGGAGAACTT
Sequence number 3671: Zika virus Whole genome #23 
ATGTGCCAAGTTTACGTGCTCCAAGAAAATGACAGGCAAGAGCATCCAGCCGGAGAACTTGGAGTACC
GGATAATGCTATCAGTGCATGGATCCCAGCACAGTGGGATGATTGTGAATGA
Sequence number 3672: Zika virus Whole genome #24 
GGAGTACCGGATAATGCTATCAGTGCATGGATCCCAGCACAGTGGGATGATTGTGAATGACACAGGAC
ATGAAACTGACGAAAACAGAGCAAAAGTCGAGGTCACACCCAATTCACCAAG
Sequence number 3673: Zika virus Whole genome #25 CACAGGACATGAAACTGACGAAAACAGAGCAAAAGTCGAGGTCACACCCAATTCACCAAGAGCAGAAG CAACCTTGGGAGGTTTTGGAAGCTTGGGACTTGACTGTGAACCAAGGACAGG
Sequence number 3674: Zika virus Whole genome #26 
AGCAGAAGCAACCTTGGGAGGTTTTGGAAGCTTGGGACTTGACTGTGAACCAAGGACAGGCCTTGACT
TCTCAGATCTGTATTACCTGACCATGAACAATAAGCATTGGTTGGTGCACAA
Sequence number 3675: Zika virus Whole genome #27 
CCTTGACTTCTCAGATCTGTATTACCTGACCATGAACAATAAGCATTGGTTGGTGCACAAGGAGTGGT
TTCATGACATCCCATTACCTTGGCATGCTGGTGCAGACACTGGAACTCCACA
Sequence number 3676: Zika virus Whole genome #28 
GGAGTGGTTTCATGACATCCCATTACCTTGGCATGCTGGTGCAGACACTGGAACTCCACACTGGAACA
ACAAAGAGGCATTGGTGGAGTTCAAGGACGCCCACGCCAAGAGGCAAACTGT
Sequence number 3677: Zika virus Whole genome #29 
CTGGAACAACAAAGAGGCATTGGTGGAGTTCAAGGACGCCCACGCCAAGAGGCAAACTGTTGTGGTTC
TGGGGAGCCAAGAGGGAGCTGTTCATACGGCCCTCGCTGGAGCTTTGGAGGC
Sequence number 3678: Zika virus Whole genome #30 
TGTGGTTCTGGGGAGCCAAGAGGGAGCTGTTCATACGGCCCTCGCTGGAGCTTTGGAGGCTGAGATGG
ATGGTGCAAAGGGAAGGCTATTCTCTGGCCATTTGAAATGCCGCCTAAAAAT
Sequence number 3679: Zika virus Whole genome #31 
TGAGATGGATGGTGCAAAGGGAAGGCTATTCTCTGGCCATTTGAAATGCCGCCTAAAAATGGACAAGC
TTAGGTTGAAGGGTGTGTCATATTCCCTGTGTACCGCAGCGTTCACATTTAC
Sequence number 3680: Zika virus Whole genome #32 
GGACAAGCTTAGGTTGAAGGGTGTGTCATATTCCCTGTGTACCGCAGCGTTCACATTTACCAAGGTCC
CAGCTGAAACATTGCATGGAACAGTCACAGTGGAGGTGCAGTATGCAGGGAC
Sequence number 3681: Zika virus Whole genome #33 
CAAGGTCCCAGCTGAAACATTGCATGGAACAGTCACAGTGGAGGTGCAGTATGCAGGGACAGACGGAC
CCTGCAAAGTCCCAGCCCAGATGGCGGTGGACATGCAGACCCTGACCCCAGT
Sequence number 3682: Zika virus Whole genome #34 
AGACGGACCCTGCAAAGTCCCAGCCCAGATGGCGGTGGACATGCAGACCCTGACCCCAGTTGGAAGGC
TGATAACCGCCAATCCTGTGATCACTGAAAGTACTGAGAATTCAAAGATGAT
Sequence number 3683: Zika virus Whole genome #35 
TGGAAGGCTGATAACCGCCAATCCTGTGATCACTGAAAGTACTGAGAATTCAAAGATGATGTTGGAGC
TCGACCCACCATTTGGGGATTCTTACATTGTCATAGGAGTCGGGGACAAGAA
Sequence number 3684: Zika virus Whole genome #36 
GTTGGAGCTCGACCCACCATTTGGGGATTCTTACATTGTCATAGGAGTCGGGGACAAGAAAATCACCC
ATCACTGGCATCGGAGTGGTAGCACCATCGGAAAGGCATTTGAAGCCACTGT
Sequence number 3685: Zika virus Whole genome #37 
AATCACCCATCACTGGCATCGGAGTGGTAGCACCATCGGAAAGGCATTTGAAGCCACTGTGAGAGGTG
CCAAGAGAATGGCAGTCTTGGGGGACACAGCCTGGGACTTTGGATCAGTTGG
Sequence number 3686: Zika virus Whole genome #38 
GAGAGGTGCCAAGAGAATGGCAGTCTTGGGGGACACAGCCTGGGACTTTGGATCAGTTGGGGGTGTGT
TTAATTCATTGGGTAAGGGTATTCACCAGATCTTTGGAGCAGCTTTCAAATC
Sequence number 3687: Zika virus Whole genome #39 
GGGTGTGTTTAATTCATTGGGTAAGGGTATTCACCAGATCTTTGGAGCAGCTTTCAAATCACTGTTTG
GAGGAATGTCCTGGTTCTCACAGATCCTCATAGGCACACTGTTGGTGTGGTT
Sequence number 3688: Zika virus Whole genome #40 
ACTGTTTGGAGGAATGTCCTGGTTCTCACAGATCCTCATAGGCACACTGTTGGTGTGGTTAGGTTTGA
ACACAAAGAATGGATCTATCTCCCTCACATGCTTAGCCCTGGGGGGAGTGAT
Sequence number 3689: Zika virus Whole genome #41 
AGGTTTGAACACAAAGAATGGATCTATCTCCCTCACATGCTTAGCCCTGGGGGGAGTGATGATCTTCC
TTTCCACGGCTGTTTCTGCTGATGTTGGGTGCTCGGTGGACTTCTCAAAAAA
Sequence number 3690: Zika virus Whole genome #42 
GATCTTCCTTTCCACGGCTGTTTCTGCTGATGTTGGGTGCTCGGTGGACTTCTCAAAAAAGGAAACGA
GATGTGGCACGGGGGTGTTCGTCTACAATGACGTTGAAGCCTGGAGGGACCG
Sequence number 3691: Zika virus Whole genome #43 
GGAAACGAGATGTGGCACGGGGGTGTTCGTCTACAATGACGTTGAAGCCTGGAGGGACCGGTACAAGT
ACCATCCTGACTCCCCCCGCAGATTGGCAGCAGCTGTTAAGCAGGCTTGGGA
Sequence number 3692: Zika virus Whole genome #44 
GTACAAGTACCATCCTGACTCCCCCCGCAGATTGGCAGCAGCTGTTAAGCAGGCTTGGGAAGAGGGGA
TTTGTGGGATCTCCTCCGTTTCGAGAATGGAAAACATCATGTGGAAATCAGT
Sequence number 3693: Zika virus Whole genome #45 
AGAGGGATTTGTGGGATCTCCTCCGTTTCGAGAATGGAAAACATCATGTGGAAATCAGTGGAAGGGG
AGCTTAATGCAATCCTAGAGGAGAATGGAGTTCAACTGACAGTTGTAGTGGG
Sequence number 3694: Zika virus Whole genome #46 
GGAAGGGGAGCTTAATGCAATCCTAGAGGAGAATGGAGTTCAACTGACAGTTGTAGTGGGGTCTGTAA
AAAACCCTATGTGGAGAGGTCCACAGAGATTGCCAGTGCCTGTGAATGAGCT
Sequence number 3695: Zika virus Whole genome #47 
GTCTGTAAAAAACCCTATGTGGAGAGGTCCACAGAGATTGCCAGTGCCTGTGAATGAGCTGCCCCATG
GCTGGAAAGCCTGGGGGAAATCGTACTTTGTCAGAGCAGCAAAGACCAACAA
Sequence number 3696: Zika virus Whole genome #48 
GCCCCATGGCTGGAAAGCCTGGGGGAAATCGTACTTTGTCAGAGCAGCAAAGACCAACAACAGTTTTG
TTGTCGACGGTGACACACTGAAGGAGTGTCCGCTCAAACATAGAGCATGGAA
Sequence number 3697: Zika virus Whole genome #49 
CAGTTTTGTTGTCGACGGTGACACACTGAAGGAGTGTCCGCTCAAACATAGAGCATGGAATAGCTTCC
TTGTGGAGGATCACGGGTTTGGGATCTTCCACACCAGTGTTTGGCTGAAGGT
Sequence number 3698: Zika virus Whole genome #50 
TAGCTTCCTTGTGGAGGATCACGGGTTTGGGATCTTCCACACCAGTGTTTGGCTGAAGGTCAGAGAGG
ACTACTCACTAGAGTGTGACCCAGCCGTCATAGGAACAGCTGTCAAGGGAAA
Sequence number 3699: Zika virus Whole genome #51 
CAGAGAGGACTACTCACTAGAGTGTGACCCAGCCGTCATAGGAACAGCTGTCAAGGGAAAGGAAGCTG
CACACAGTGATCTAGGCTATTGGATTGAGAGTGAAAAGAATGACACATGGAG
Sequence number 3700: Zika virus Whole genome #52 
GGAAGCTGCACACAGTGATCTAGGCTATTGGATTGAGAGTGAAAAGAATGACACATGGAGGCTGAGGA
GGGCTCATCTGATTGAGATGAAAACATGTGAGTGGCCAAAGTCTCACACACT
Sequence number 3701: Zika virus Whole genome #53 GCTGAGGAGGGCTCATCTGATTGAGATGAAAACATGTGAGTGGCCAAAGTCTCACACACTGTGGACAG ATGGAGTGGAAGAAAGTGATCTGATCATACCCAAGTCCTTAGCTGGTCCACT
Sequence number 3702: Zika virus Whole genome #54 
GTGGACAGATGGAGTGGAAGAAAGTGATCTGATCATACCCAAGTCCTTAGCTGGTCCACTCAGCCACC
ACAACACCAGAGAGGGTTACAGAACTCAAGTGAAAGGGCCATGGCATAGTGA
Sequence number 3703: Zika virus Whole genome #55 
CAGCCACCACAACACCAGAGAGGGTTACAGAACTCAAGTGAAAGGGCCATGGCATAGTGAAGAGCTCG
AAATCCGGTTTGAGGAGTGCCCAGGAACCAAGGTTCACGTGGAGGAGACATG
Sequence number 3704: Zika virus Whole genome #56 
AGAGCTCGAAATCCGGTTTGAGGAGTGCCCAGGAACCAAGGTTCACGTGGAGGAGACATGCGGAACTA
GAGGACCATCTCTGAGATCAACTACTGCTAGTGGAAGGGTCATAGAGGAATG
Sequence number 3705: Zika virus Whole genome #57 
CGGAACTAGAGGACCATCTCTGAGATCAACTACTGCTAGTGGAAGGGTCATAGAGGAATGGTGCTGTA
GGGAATGCACAATGCCTCCACTATCGTTCCGGGCGAAAGACGGCTGCTGGTA
Sequence number 3706: Zika virus Whole genome #58 
GTGCTGTAGGGAATGCACAATGCCTCCACTATCGTTCCGGGCGAAAGACGGCTGCTGGTATGGAATGG
AGATAAGGCCCAGAAAGGAACCAGAGAGCAACTTAGTGAGGTCCATGGTGAC
Sequence number 3707: Zika virus Whole genome #59 
TGGAATGGAGATAAGGCCCAGAAAGGAACCAGAGAGCAACTTAGTGAGGTCCATGGTGACAGCAGGAT
CAACCGATCATATGGATCACTTCTCTCTTGGAGTGCTTGTGATTCTACTCAT
Sequence number 3708: Zika virus Whole genome #60 
AGCAGGATCAACCGATCATATGGATCACTTCTCTCTTGGAGTGCTTGTGATTCTACTCATGGTGCAGG
AAGGTTTGAAGAAGAGAATGACCACAAAGATCATAATGAGCACATCAATGGC
Sequence number 3709: Zika virus Whole genome #61 
GGTGCAGGAAGGTTTGAAGAAGAGAATGACCACAAAGATCATAATGAGCACATCAATGGCAGTGCTGG
TAGCCATGGTCTTGGGAGGATTCTCAATGAGTGACCTGGCTAAGCTTGTGAT
Sequence number 3710: Zika virus Whole genome #62 
AGTGCTGGTAGCCATGGTCTTGGGAGGATTCTCAATGAGTGACCTGGCTAAGCTTGTGATCCTGATGG
GTGCTACTTTCGCAGAAATGAACACTGGAGGAGATGTAGCTCACTTGGCATT
Sequence number 3711: Zika virus Whole genome #63 
CCTGATGGGTGCTACTTTCGCAGAAATGAACACTGGAGGAGATGTAGCTCACTTGGCATTGGTTGCGG
CATTTAAAGTCAGACCAGCCTTGTTGGTTTCCTTCATCTTCAGAGCCAACTG
Sequence number 3712: Zika virus Whole genome #64 
GGTTGCGGCATTTAAAGTCAGACCAGCCTTGTTGGTTTCCTTCATCTTCAGAGCCAACTGGACACCCC
GTGAGAGCATGCTGCTAGCCCTGGCTTCGTGCCTCCTGCAGACTGCGATCTC
Sequence number 3713: Zika virus Whole genome #65 
GACACCCCGTGAGAGCATGCTGCTAGCCCTGGCTTCGTGCCTCCTGCAGACTGCGATCTCTGCTCTTG
AAGGCGAGCTGATGGTCCTCGTTAATGGATTTGCTTTGGCCTGGTTGGCAAT
Sequence number 3714: Zika virus Whole genome #66 
TGCTCTTGAAGGCGAGCTGATGGTCCTCGTTAATGGATTTGCTTTGGCCTGGTTGGCAATACGAGCAA
TGGCCGTGCCACGCACTGACAATATCGCTCTGGCAATTCTGGCTGCTCTAAC
Sequence number 3715: Zika virus Whole genome #67 
ACGAGCAATGGCCGTGCCACGCACTGACAATATCGCTCTGGCAATTCTGGCTGCTCTAACACCATTAG
CCAGAGGCACACTGCTCGTGGCATGGAGAGCGGGCCTCGCCACTTGTGGAGG
Sequence number 3716: Zika virus Whole genome #68 
ACCATTAGCCAGAGGCACACTGCTCGTGGCATGGAGAGCGGGCCTCGCCACTTGTGGAGGGTTCATGC
TCCTTTCCCTGAAAGGGAAAGGTAGTGTGAAGAAGAACCTGCCTTTTGTCAT
Sequence number 3717: Zika virus Whole genome #69 
GTTCATGCTCCTTTCCCTGAAAGGGAAAGGTAGTGTGAAGAAGAACCTGCCTTTTGTCATGGCCTTGG
GGTTGACCGCTGTGAGGATAGTGGACCCCATCAATGTGGTAGGACTACTGTT
Sequence number 3718: Zika virus Whole genome #70 
GGCCTTGGGGTTGACCGCTGTGAGGATAGTGGACCCCATCAATGTGGTAGGACTACTGTTACTCACAA
GGAGTGGAAAACGGAGCTGGCCTCCTAGTGAAGTGCTTACAGCTGTTGGCCT
Sequence number 3719: Zika virus Whole genome #71 
ACTCACAAGGAGTGGAAAACGGAGCTGGCCTCCTAGTGAAGTGCTTACAGCTGTTGGCCTGATATGTG
CACTGGCCGGAGGGTTTGCCAAGGCAGACATAGAGATGGCTGGGCCCATGGC
Sequence number 3720: Zika virus Whole genome #72 
GATATGTGCACTGGCCGGAGGGTTTGCCAAGGCAGACATAGAGATGGCTGGGCCCATGGCTGCAGTGG
GCCTGCTAATTGTCAGTTATGTGGTCTCGGGAAAGAGTGTGGACATGTACAT
Sequence number 3721: Zika virus Whole genome #73 
TGCAGTGGGCCTGCTAATTGTCAGTTATGTGGTCTCGGGAAAGAGTGTGGACATGTACATCGAAAGAG
CAGGTGATATCACATGGGAAAAAGACGCGGAAGTCACTGGAAACAGTCCTCG
Sequence number 3722: Zika virus Whole genome #74 
CGAAAGAGCAGGTGATATCACATGGGAAAAAGACGCGGAAGTCACTGGAAACAGTCCTCGGCTTGACG
TGGCACTAGATGAGAGTGGTGATTTCTCCTTGGTAGAGGAGGATGGCCCACC
Sequence number 3723: Zika virus Whole genome #75 
GCTTGACGTGGCACTAGATGAGAGTGGTGATTTCTCCTTGGTAGAGGAGGATGGCCCACCCATGAGAG
AGATCATACTCAAGGTGGTTCTGATGGCCATCTGTGGCATGAACCCAATAGC
Sequence number 3724: Zika virus Whole genome #76 
CATGAGAGAGATCATACTCAAGGTGGTTCTGATGGCCATCTGTGGCATGAACCCAATAGCCATACCCT
TCGCTGCAGGAGCGTGGTATGTGTATGTAAAGACTGGAAAAAGGAGTGGTGC
Sequence number 3725: Zika virus Whole genome #77 
CATACCCTTCGCTGCAGGAGCGTGGTATGTGTATGTAAAGACTGGAAAAAGGAGTGGTGCCCTCTGGG
ACGTGCCTGCTCCCAAAGAAGTGAAAAAAGGAGAGACTACAGATGGAGTGTA
Sequence number 3726: Zika virus Whole genome #78 
CCTCTGGGACGTGCCTGCTCCCAAAGAAGTGAAAAAAGGAGAGACTACAGATGGAGTGTACAGAGTGA
TGACTCGCAGACTGCTGGGTTCAACACAGGTTGGAGTGGGAGTCATGCAAGA
Sequence number 3727: Zika virus Whole genome #79 
CAGAGTGATGACTCGCAGACTGCTGGGTTCAACACAGGTTGGAGTGGGAGTCATGCAAGAGGGAGTCT
TCCACACCATGTGGCACGTCACAAAAGGAGCCGCATTGAGGAGCGGTGAAGG
Sequence number 3728: Zika virus Whole genome #80 
GGGAGTCTTCCACACCATGTGGCACGTCACAAAAGGAGCCGCATTGAGGAGCGGTGAAGGAAGACTTG
ATCCATATTGGGGGGACGTCAAGCAGGACTTGGTGTCATATTGTGGGCCTTG
Sequence number 3729: Zika virus Whole genome #81 AAGACTTGATCCATATTGGGGGGACGTCAAGCAGGACTTGGTGTCATATTGTGGGCCTTGGAAGTTGG ATGCAGCCTGGGATGGACTAAGTGAAGTGCAGCTTTTGGCCGTACCCCCCGG
Sequence number 3730: Zika virus Whole genome #82 
GAAGTTGGATGCAGCCTGGGATGGACTAAGTGAAGTGCAGCTTTTGGCCGTACCCCCCGGAGAGAGGG
CTAGAAACATTCAGACTCTGCCTGGAATATTCAAGACAAAGGATGGGGACAT
Sequence number 3731: Zika virus Whole genome #83 
AGAGAGGGCTAGAAACATTCAGACTCTGCCTGGAATATTCAAGACAAAGGATGGGGACATCGGAGCAG
TCGCTCTAGACTACCCCGCAGGAACCTCAGGATCTCCAATCCTAGACAAATG
Sequence number 3732: Zika virus Whole genome #84 
CGGAGCAGTCGCTCTAGACTACCCCGCAGGAACCTCAGGATCTCCAATCCTAGACAAATGCGGAAGAG
TGATAGGACTTTATGGCAATGGGGTTGTGATCAAGAATGGAAGCTATGTTAG
Sequence number 3733: Zika virus Whole genome #85 
CGGAAGAGTGATAGGACTTTATGGCAATGGGGTTGTGATCAAGAATGGAAGCTATGTTAGTGCCATAA
CCCAGGGAAAAAGGGAGGAGGAGGCTCCAGTTGAGTGCTTTGAACCCTCGAT
Sequence number 3734: Zika virus Whole genome #86 
TGCCATAACCCAGGGAAAAAGGGAGGAGGAGGCTCCAGTTGAGTGCTTTGAACCCTCGATGCTGAGGA
AGAAGCAGCTAACAGTCTTGGATCTGCATCCAGGAGCCGGGAAAACCAGGAG
Sequence number 3735: Zika virus Whole genome #87 
GCTGAGGAAGAAGCAGCTAACAGTCTTGGATCTGCATCCAGGAGCCGGGAAAACCAGGAGGGTTCTTC
CTGAAATAGTCCGTGAAGCCATAAAGAAGAGACTTCGCACAGTGATCCTAGC
Sequence number 3736: Zika virus Whole genome #88 
GGTTCTTCCTGAAATAGTCCGTGAAGCCATAAAGAAGAGACTTCGCACAGTGATCCTAGCACCAACCA
GGGTCGTTGCCGCTGAGATGGAGGAAGCCCTAAGAGGACTGCCGGTGCGTTA
Sequence number 3737: Zika virus Whole genome #89 
ACCAACCAGGGTCGTTGCCGCTGAGATGGAGGAAGCCCTAAGAGGACTGCCGGTGCGTTACATGACAA
CAGCAGTCAACGTCACCCACTCTGGGACAGAAATCGTCGATTTGATGTGCCA
Sequence number 3738: Zika virus Whole genome #90 
CATGACAACAGCAGTCAACGTCACCCACTCTGGGACAGAAATCGTCGATTTGATGTGCCATGCCACCT
TCACTTCACGCCTACTACAACCCATCAGAGTCCCCAACTACAACCTTTACAT
Sequence number 3739: Zika virus Whole genome #91 
TGCCACCTTCACTTCACGCCTACTACAACCCATCAGAGTCCCCAACTACAACCTTTACATCATGGATG
AAGCTCATTTCACAGATCCCTCAAGCATAGCTGCACGAGGATATATATCAAC
Sequence number 3740: Zika virus Whole genome #92 
CATGGATGAAGCTCATTTCACAGATCCCTCAAGCATAGCTGCACGAGGATATATATCAACAAGGGTTG
AAATGGGCGAGGCGGCTGCTATCTTCATGACTGCTACACCACCAGGAACCCG
Sequence number 3741: Zika virus Whole genome #93 
AAGGGTTGAAATGGGCGAGGCGGCTGCTATCTTCATGACTGCTACACCACCAGGAACCCGCGATGCGT
TTCCGGATTCCAACTCACCAATCATGGACACAGAAGTGGAAGTTCCAGAGAG
Sequence number 3742: Zika virus Whole genome #94 
CGATGCGTTTCCGGATTCCAACTCACCAATCATGGACACAGAAGTGGAAGTTCCAGAGAGAGCCTGGA
GCTCAGGCTTTGACTGGGTGACGGATCATTCTGGGAAAACAATTTGGTTTGT
Sequence number 3743: Zika virus Whole genome #95 
AGCCTGGAGCTCAGGCTTTGACTGGGTGACGGATCATTCTGGGAAAACAATTTGGTTTGTTCCAAGTG
TGAGAAACGGAAATGAAATCGCAGCCTGTCTGACAAAGGCTGGAAAGCGGGT
Sequence number 3744: Zika virus Whole genome #96 
TCCAAGTGTGAGAAACGGAAATGAAATCGCAGCCTGTCTGACAAAGGCTGGAAAGCGGGTCATACAGC
TCAGCAGGAAGACTTTTGAGACAGAGTTTCAGAAGACAAGAAATCAAGAGTG
Sequence number 3745: Zika virus Whole genome #97 
CATACAGCTCAGCAGGAAGACTTTTGAGACAGAGTTTCAGAAGACAAGAAATCAAGAGTGGGACTTTG
TCATAACAACTGACATTTCAGAGATGGGTGCCAATTTCAAGGCTGACCGGGT
Sequence number 3746: Zika virus Whole genome #98 
GGACTTTGTCATAACAACTGACATTTCAGAGATGGGTGCCAATTTCAAGGCTGACCGGGTCATAGATT
CCAGGAGATGCCTAAAGCCAGTCATACTCGATGGCGAGAGAGTCATCCTGGC
Sequence number 3747: Zika virus Whole genome #99 
CATAGATTCCAGGAGATGCCTAAAGCCAGTCATACTCGATGGCGAGAGAGTCATCCTGGCTGGGCCCA
TGCCTGTCACGCATGCCAGTGCTGCTCAGAGGAGAGGACGTGTAGGCAGGAA
Sequence number 3748: Zika virus Whole genome #100 
TGGGCCCATGCCTGTCACGCATGCCAGTGCTGCTCAGAGGAGAGGACGTGTAGGCAGGAACCCCAACA
AACCTGGAGATGAGTACATGTATGGAGGTGGGTGTGCAGAGACTGATGAAGA
Sequence number 3749: Zika virus Whole genome #101 
CCCCAACAAACCTGGAGATGAGTACATGTATGGAGGTGGGTGTGCAGAGACTGATGAAGACCATGCAC
ACTGGCTTGAAGCAAGAATGCTTCTTGACAACATTTACCTCCAGGATGGCCT
Sequence number 3750: Zika virus Whole genome #102 
CCATGCACACTGGCTTGAAGCAAGAATGCTTCTTGACAACATTTACCTCCAGGATGGCCTCATAGCCT
CGCTCTATCGGCCTGAGGCCGACAAGGTAGCCGCCATTGAGGGAGAGTTCAA
Sequence number 3751: Zika virus Whole genome #103 
CATAGCCTCGCTCTATCGGCCTGAGGCCGACAAGGTAGCCGCCATTGAGGGAGAGTTCAAGCTGAGGA
CAGAGCAAAGGAAGACCTTTGTGGAACTCATGAAGAGAGGAGACCTTCCCGT
Sequence number 3752: Zika virus Whole genome #104 
GCTGAGGACAGAGCAAAGGAAGACCTTTGTGGAACTCATGAAGAGAGGAGACCTTCCCGTTTGGCTGG
CCTATCAAGTAGCATCTGCCGGAATAACTTACACAGATAGAAGATGGTGCTT
Sequence number 3753: Zika virus Whole genome #105 
TTGGCTGGCCTATCAAGTAGCATCTGCCGGAATAACTTACACAGATAGAAGATGGTGCTTTGATGGCA
CTACCAACAACACCATAATGGAAGACAGTGTACCAGCAGAGGTGTGGACCAA
Sequence number 3754: Zika virus Whole genome #106 
TGATGGCACTACCAACAACACCATAATGGAAGACAGTGTACCAGCAGAGGTGTGGACCAAGTATGGAG
AGAAGAGAGTGCTTAAACCGAGGTGGATGGATGCTAGGGTCTGTTCAGATCA
Sequence number 3755: Zika virus Whole genome #107 
GTATGGAGAGAAGAGAGTGCTTAAACCGAGGTGGATGGATGCTAGGGTCTGTTCAGATCATGCGGCTC
TGAAGTCGTTCAAAGAATTTGCCGCTGGGAAGAGAGGAGCGGCTTTGGGAGT
Sequence number 3756: Zika virus Whole genome #108 
TGCGGCTCTGAAGTCGTTCAAAGAATTTGCCGCTGGGAAGAGAGGAGCGGCTTTGGGAGTAATGGATG
CCCTGGGAACATTGCCAGGACACATGACAGAGAGGTTTCAGGAAGCCATTGA
Sequence number 3757: Zika virus Whole genome #109 AATGGATGCCCTGGGAACATTGCCAGGACACATGACAGAGAGGTTTCAGGAAGCCATTGACAATCTCG CTGTGCTCATGCGAGCAGAGACTGGAAGTAGGCCCTACAAAGCAGCGGCAGC
Sequence number 3758: Zika virus Whole genome #110 
CAATCTCGCTGTGCTCATGCGAGCAGAGACTGGAAGTAGGCCCTACAAAGCAGCGGCAGCTCAACTGC
CGGAGACCTTAGAGACTATCATGCTTCTGGGCTTATTGGGAACAGTTTCGCT
Sequence number 3759: Zika virus Whole genome #111 
TCAACTGCCGGAGACCTTAGAGACTATCATGCTTCTGGGCTTATTGGGAACAGTTTCGCTAGGAATCT
TCTTTGTCTTGATGCGGAACAAGGGCATCGGGAAGATGGGCTTCGGAATGGT
Sequence number 3760: Zika virus Whole genome #112 
AGGAATCTTCTTTGTCTTGATGCGGAACAAGGGCATCGGGAAGATGGGCTTCGGAATGGTAACCCTTG
GGGCCAGCGCATGGCTCATGTGGCTTTCGGAAATTGAACCAGCCAGAATCGC
Sequence number 3761: Zika virus Whole genome #113 
AACCCTTGGGGCCAGCGCATGGCTCATGTGGCTTTCGGAAATTGAACCAGCCAGAATCGCATGTGTCC
TCATTGTCGTGTTTTTGTTGCTGGTGGTGCTCATACCCGAGCCAGAGAAGCA
Sequence number 3762: Zika virus Whole genome #114 
ATGTGTCCTCATTGTCGTGTTTTTGTTGCTGGTGGTGCTCATACCCGAGCCAGAGAAGCAAAGATCTC
CCCAGGATAATCAAATGGCAATCATCATCATGGTGGCAGTTGGCCTTCTGGG
Sequence number 3763: Zika virus Whole genome #115 
AAGATCTCCCCAGGATAATCAAATGGCAATCATCATCATGGTGGCAGTTGGCCTTCTGGGTTTGATAA
CTGCAAATGAACTCGGATGGCTGGAAAGGACAAAAAGTGATATAGCTCATCT
Sequence number 3764: Zika virus Whole genome #116 
TTTGATAACTGCAAATGAACTCGGATGGCTGGAAAGGACAAAAAGTGATATAGCTCATCTAATGAGAA
GGAAAGAAGAGGGGACAACCATGGGATTCTCAATGGATATTGATCTGCGGCC
Sequence number 3765: Zika virus Whole genome #117 
AATGAGAAGGAAAGAAGAGGGGACAACCATGGGATTCTCAATGGATATTGATCTGCGGCCAGCCTCCG
CCTGGGCTATTTATGCCGCATTGACAACTCTCATCACCCCAGCCGTCCAACA
Sequence number 3766: Zika virus Whole genome #118 
AGCCTCCGCCTGGGCTATTTATGCCGCATTGACAACTCTCATCACCCCAGCCGTCCAACATGCGGTAA
CCACCTCATACAACAACTACTCCCTGATGGCGATGGCCACACAAGCTGGAGT
Sequence number 3767: Zika virus Whole genome #119 
TGCGGTAACCACCTCATACAACAACTACTCCCTGATGGCGATGGCCACACAAGCTGGAGTGCTGTTTG
GCATGGGCAAAGGGATGCCATTTTATGCATGGGACTTTGGAGTCCCGCTGCT
Sequence number 3768: Zika virus Whole genome #120 
GCTGTTTGGCATGGGCAAAGGGATGCCATTTTATGCATGGGACTTTGGAGTCCCGCTGCTAATGATGG
GTTGCTACTCACAATTAACACCCCTGACCCTGATAGTGGCCATCATTCTGCT
Sequence number 3769: Zika virus Whole genome #121 
AATGATGGGTTGCTACTCACAATTAACACCCCTGACCCTGATAGTGGCCATCATTCTGCTTGTGGCAC
ACTACATGTACTTGATCCCAGGTTTGCAGGCAGCAGCAGCACGTGCTGCCCA
Sequence number 3770: Zika virus Whole genome #122 
TGTGGCACACTACATGTACTTGATCCCAGGTTTGCAGGCAGCAGCAGCACGTGCTGCCCAGAAGAGGA
CAGCAGCTGGCATCATGAAGAATCCCGTTGTGGATGGAATAGTGGTGACTGA
Sequence number 3771: Zika virus Whole genome #123 
GAAGAGGACAGCAGCTGGCATCATGAAGAATCCCGTTGTGGATGGAATAGTGGTGACTGACATTGACA
CAATGACAATTGACCCCCAAGTGGAGAAGAAGATGGGACAAGTGTTACTCAT
Sequence number 3772: Zika virus Whole genome #124 
CATTGACACAATGACAATTGACCCCCAAGTGGAGAAGAAGATGGGACAAGTGTTACTCATAGCAGTAG
CTGTCTCCAGTGCTGTGCTACTGCGGACCGCTTGGGGATGGGGGGAGGCTGG
Sequence number 3773: Zika virus Whole genome #125 
AGCAGTAGCTGTCTCCAGTGCTGTGCTACTGCGGACCGCTTGGGGATGGGGGGAGGCTGGGGCTCTGA
TCACAGCAGCAACCTCCACCTTATGGGAAGGCTCTCCAAACAAATACTGGAA
Sequence number 3774: Zika virus Whole genome #126 
GGCTCTGATCACAGCAGCAACCTCCACCTTATGGGAAGGCTCTCCAAACAAATACTGGAACTCCTCCA
CAGCCACCTCACTGTGCAACATCTTTAGAGGAAGTTATTTGGCAGGGGCTTC
Sequence number 3775: Zika virus Whole genome #127 
CTCCTCCACAGCCACCTCACTGTGCAACATCTTTAGAGGAAGTTATTTGGCAGGGGCTTCCCTTATTT
ACACAGTGACAAGAAATGCCGGTCTGGTTAAGAGACGTGGAGGTGGAACGGG
Sequence number 3776: Zika virus Whole genome #128 
CCTTATTTACACAGTGACAAGAAATGCCGGTCTGGTTAAGAGACGTGGAGGTGGAACGGGAGAGACCC
TGGGAGAGAAGTGGAAAGCCCGCCTGAACCAGATGTCGGCCTTGGAGTTCTA
Sequence number 3777: Zika virus Whole genome #129 
AGAGACCCTGGGAGAGAAGTGGAAAGCCCGCCTGAACCAGATGTCGGCCTTGGAGTTCTACTCTTACA
AAAAGTCAGGCATCACCGAAGTGTGTAGGGAGGAGGCGCGCCGCGCCCTCAA
Sequence number 3778: Zika virus Whole genome #130 
CTCTTACAAAAAGTCAGGCATCACCGAAGTGTGTAGGGAGGAGGCGCGCCGCGCCCTCAAGGATGGAG
TGGCCACAGGAGGACATGCTGTATCCCGGGGAAGCGCAAAGCTTAGATGGTT
Sequence number 3779: Zika virus Whole genome #131 
GGATGGAGTGGCCACAGGAGGACATGCTGTATCCCGGGGAAGCGCAAAGCTTAGATGGTTGGTAGAGA
GAGGATACCTGCAGCCCCATGGAAAGGTTGTTGACCTTGGATGTGGCAGAGG
Sequence number 3780: Zika virus Whole genome #132 
GGTAGAGAGAGGATACCTGCAGCCCCATGGAAAGGTTGTTGACCTTGGATGTGGCAGAGGAGGCTGGA
GTTATTACGCTGCCACCATCCGTAAAGTGCAGGAGGTCAGAGGATACACAAA
Sequence number 3781: Zika virus Whole genome #133 
AGGCTGGAGTTATTACGCTGCCACCATCCGTAAAGTGCAGGAGGTCAGAGGATACACAAAGGGAGGTC
CTGGCCATGAAGAGCCCATGCTGGTGCAAAGCTATGGGTGGAACATAGTTCG
Sequence number 3782: Zika virus Whole genome #134 
GGGAGGTCCTGGCCATGAAGAGCCCATGCTGGTGCAAAGCTATGGGTGGAACATAGTTCGCCTCAAGA
GTGGAGTGGACGTCTTCCACATGGCGGCTGAACCGTGTGACACTCTGCTGTG
Sequence number 3783: Zika virus Whole genome #135 
CCTCAAGAGTGGAGTGGACGTCTTCCACATGGCGGCTGAACCGTGTGACACTCTGCTGTGTGACATAG
GCGAGTCATCATCCAGTCCTGAAGTGGAAGAGACGCGAACACTCAGAGTGCT
Sequence number 3784: Zika virus Whole genome #136 
TGACATAGGCGAGTCATCATCCAGTCCTGAAGTGGAAGAGACGCGAACACTCAGAGTGCTCTCCATGG
TGGGAGACTGGCTTGAAAAAAGACCAGGGGCCTTCTGCATAAAGGTGCTGTG
Sequence number 3785: Zika virus Whole genome #137 CTCCATGGTGGAGACTGGCTTGAAAAAGACCAGGGGCCTTCTGCATAAAGGTGCTGTGCCCATACA CCAGCACAATGATGGAGACCATGGAGCGACTGCAACGTAGGCATGGGGGAGG
Sequence number 3786: Zika virus Whole genome #138 
CCCATACACCAGCACAATGATGGAGACCATGGAGCGACTGCAACGTAGGCATGGGGGAGGATTAGTCA
GAGTGCCATTGTCCCGCAACTCTACACATGAGATGTATTGGGTCTCTGGAGC
Sequence number 3787: Zika virus Whole genome #139 
ATTAGTCAGAGTGCCATTGTCCCGCAACTCTACACATGAGATGTATTGGGTCTCTGGAGCCAAAAGTA
ACATCATAAAGAGTGTGTCCACCACAAGTCAGCTCCTCTTGGGACGCATGGA
Sequence number 3788: Zika virus Whole genome #140 
CAAAAGTAACATCATAAAGAGTGTGTCCACCACAAGTCAGCTCCTCTTGGGACGCATGGAAGGGCCTA
GGAGGCCAGTGAAATATGAGGAGGATGTGAACCTCGGCTCAGGCACACGAGC
Sequence number 3789: Zika virus Whole genome #141 
AGGGCCTAGGAGGCCAGTGAAATATGAGGAGGATGTGAACCTCGGCTCAGGCACACGAGCTGTGGCAA
GCTGCGCTGAGGCTCCCAACATGAAGATCATTGGTAGGCGCATTGAGAGAAT
Sequence number 3790: Zika virus Whole genome #142 
TGTGGCAAGCTGCGCTGAGGCTCCCAACATGAAGATCATTGGTAGGCGCATTGAGAGAATCCGCAATG
AACATGCAGAGACATGGTTCTTTGATGAAAACCACCCATACAGGACATGGGC
Sequence number 3791: Zika virus Whole genome #143 
CCGCAATGAACATGCAGAGACATGGTTCTTTGATGAAAACCACCCATACAGGACATGGGCCTACCATG
GGAGCTACGAAGCCCCCACGCAGGGGTCAGCGTCATCCCTCGTGAACGGGGT
Sequence number 3792: Zika virus Whole genome #144 
CTACCATGGGAGCTACGAAGCCCCCACGCAGGGGTCAGCGTCATCCCTCGTGAACGGGGTTGTTAGAC
TCTTGTCAAAGCCCTGGGATGTGGTGACTGGAGTTACAGGAATAGCTATGAC
Sequence number 3793: Zika virus Whole genome #145 
TGTTAGACTCTTGTCAAAGCCCTGGGATGTGGTGACTGGAGTTACAGGAATAGCTATGACTGACACCA
CGCCATACGGCCAACAAAGAGTCTTCAAGGAAAAAGTGGACACCAGGGTGCC
Sequence number 3794: Zika virus Whole genome #146 
TGACACCACGCCATACGGCCAACAAAGAGTCTTCAAGGAAAAAGTGGACACCAGGGTGCCAGATCCCC
AAGAAGGCACTCGCCAAGTGATGAACATGGTATCGTCTTGGCTATGGAAGGA
Sequence number 3795: Zika virus Whole genome #147 
AGATCCCCAAGAAGGCACTCGCCAAGTGATGAACATGGTATCGTCTTGGCTATGGAAGGAGCTGGGAA
AACGCAAGCGACCACGTGTCTGCACCAAAGAAGAGTTCATTAATAAGGTGCG
Sequence number 3796: Zika virus Whole genome #148 
GCTGGGAAAACGCAAGCGACCACGTGTCTGCACCAAAGAAGAGTTCATTAATAAGGTGCGCAGCAATG
CAGCACTGGGAGCAATATTTGAAGAGGAAAAAGAATGGAAGACAGCTGTAGA
Sequence number 3797: Zika virus Whole genome #149 
CAGCAATGCAGCACTGGGAGCAATATTTGAAGAGGAAAAAGAATGGAAGACAGCTGTAGAAGCTGTGA
ATGATCCAAGATTTTGGGCTCTAGTGGACAAGGAAAGAGAACACCACCTGAG
Sequence number 3798: Zika virus Whole genome #150 
AGCTGTGAATGATCCAAGATTTTGGGCTCTAGTGGACAAGGAAAGAGAACACCACCTGAGAGGAGAGT
GTCATAGCTGTGTGTACAACATGATGGGCAAAAGAGAAAAGAAGCAAGGAGA
Sequence number 3799: Zika virus Whole genome #151 
AGGAGAGTGTCATAGCTGTGTGTACAACATGATGGGCAAAAGAGAAAAGAAGCAAGGAGAATTCGGGA
AAGCAAAAGGCAGCCGCGCAATCTGGTACATGTGGTTGGGAGCCAGATTTTT
Sequence number 3800: Zika virus Whole genome #152 
ATTCGGGAAAGCAAAAGGCAGCCGCGCAATCTGGTACATGTGGTTGGGAGCCAGATTTTTGGAGTTTG
AAGCTCTTGGGTTCTTGAACGAGGACCACTGGATGGGGAGAGAAAACTCAGG
Sequence number 3801: Zika virus Whole genome #153 
GGAGTTTGAAGCTCTTGGGTTCTTGAACGAGGACCACTGGATGGGGAGAGAAAACTCAGGAGGTGGCG
TTGAAGGGCTAGGACTGCAAAGGCTTGGATATATCCTAGAAGAAATGAACCG
Sequence number 3802: Zika virus Whole genome #154 
AGGTGGCGTTGAAGGGCTAGGACTGCAAAGGCTTGGATATATCCTAGAAGAAATGAACCGGGCACCAG
GAGGAAAGATGTATGCAGATGACACTGCTGGCTGGGACACCCGCATTAGCAA
Sequence number 3803: Zika virus Whole genome #155 
GGCACCAGGAGGAAAGATGTATGCAGATGACACTGCTGGCTGGGACACCCGCATTAGCAAGTTTGATC
TAGAGAATGAAGCCCTGATCACTAACCAGATGGAAGAAGGGCACAGAGCTCT
Sequence number 3804: Zika virus Whole genome #156 
GTTTGATCTAGAGAATGAAGCCCTGATCACTAACCAGATGGAAGAAGGGCACAGAGCTCTGGCGTTGG
CCGTGATTAAATACACATACCAAAACAAAGTGGTGAAGGTTCTCAGACCAGC
Sequence number 3805: Zika virus Whole genome #157 
GGCGTTGGCCGTGATTAAATACACATACCAAAACAAAGTGGTGAAGGTTCTCAGACCAGCTGAAGGAG
GGAAAACAGTCATGGACATCATCTCAAGACAAGACCAGAGAGGGAGCGGACA
Sequence number 3806: Zika virus Whole genome #158 
TGAAGGAGGGAAAACAGTCATGGACATCATCTCAAGACAAGACCAGAGAGGGAGCGGACAAGTTGTTA
CTTATGCTCTCAACACATTCACCAACCTGGTGGTGCAGCTTATCCGGAACAT
Sequence number 3807: Zika virus Whole genome #159 
AGTTGTTACTTATGCTCTCAACACATTCACCAACCTGGTGGTGCAGCTTATCCGGAACATGGAGGCTG
AGGAAGTGCTAGAGATGCATGATCTGTGGTTGTTGAGGAAGCCAGAGAAAGT
Sequence number 3808: Zika virus Whole genome #160 
GGAGGCTGAGGAAGTGCTAGAGATGCATGATCTGTGGTTGTTGAGGAAGCCAGAGAAAGTGACCAGAT
GGTTGCAGAGCAATGGATGGGACAGACTCAAACGGATGGCAGTTAGTGGAGA
Sequence number 3809: Zika virus Whole genome #161 
GACCAGATGGTTGCAGAGCAATGGATGGGACAGACTCAAACGGATGGCAGTTAGTGGAGATGACTGCG
TTGTAAAGCCAATTGATGATAGGTTTGCACATGCCCTCAGGTTCTTGAATGA
Sequence number 3810: Zika virus Whole genome #162 
TGACTGCGTTGTAAAGCCAATTGATGATAGGTTTGCACATGCCCTCAGGTTCTTGAATGACATGGGAA
AGGTTAGGAAAGACACACAGGAATGGAAACCCTCGACTGGATGGAGCAATTG
Sequence number 3811: Zika virus Whole genome #163 
CATGGGAAAGGTTAGGAAAGACACACAGGAATGGAAACCCTCGACTGGATGGAGCAATTGGGAAGAGG
TCCCGTTCTGTTCTCACCACTTCAACAAGCTGCACCTCAAGGACGGGAGATC
Sequence number 3812: Zika virus Whole genome #164 
GGAAGAGGTCCCGTTCTGTTCTCACCACTTCAACAAGCTGCACCTCAAGGACGGGAGATCCATTGTGG
TCCCCTGCCGCCACCAAGATGAACTGATTGGCCGAGCCCGTGTCTCACCAGG
Sequence number 3813: Zika virus Whole genome #165 CATTGTGGTCCCCTGCCGCCACCAAGATGAACTGATTGGCCGAGCCCGTGTCTCACCAGGGGCAGGAT GGAGCATCCGGGAGACTGCTTGTCTTGCAAAATCATATGCACAGATGTGGCA
Sequence number 3814: Zika virus Whole genome #166 
GGCAGGATGGAGCATCCGGGAGACTGCTTGTCTTGCAAAATCATATGCACAGATGTGGCAGCTTCTTT
ATTTCCACAGAAGAGACCTCCGACTGATGGCCAATGCCATTTGTTCGGCTGT
Sequence number 3815: Zika virus Whole genome #167 
GCTTCTTTATTTCCACAGAAGAGACCTCCGACTGATGGCCAATGCCATTTGTTCGGCTGTGCCAATTG
ACTGGGTACCAACTGGGAGAACCACCTGGTCAATCCATGGAAAGGGAGAATG
Sequence number 3816: Zika virus Whole genome #168 
GCCAATTGACTGGGTACCAACTGGGAGAACCACCTGGTCAATCCATGGAAAGGGAGAATGGATGACTA
CTGAGGACATGCTCATGGTGTGGAATAGAGTGTGGATTGAGGAGAACGACCA
Sequence number 3817: Zika virus Whole genome #169 
GATGACTACTGAGGACATGCTCATGGTGTGGAATAGAGTGTGGATTGAGGAGAACGACCACATGGAGG
ACAAGACCCCTGTAACAAAATGGACAGACATTCCCTATTTGGGAAAAAGGGA
Sequence number 3818: Zika virus Whole genome #170 
CATGGAGGACAAGACCCCTGTAACAAAATGGACAGACATTCCCTATTTGGGAAAAAGGGAGGACTTAT
GGTGTGGATCCCTTATAGGGCATAGACCTCGCACCACTTGGGCTGAGAACAT
Sequence number 3819: Zika virus Whole genome #171 
GGACTTATGGTGTGGATCCCTTATAGGGCATAGACCTCGCACCACTTGGGCTGAGAACATCAAAGACA
CAGTCAACATGGTGCGTAGGATCATAGGTGATGAAGAAAAGTTCATGGACTA
Sequence number 3820: Zika virus Whole genome #172 
CAAAGACACAGTCAACATGGTGCGTAGGATCATAGGTGATGAAGAAAAGTTCATGGACTACCTATCCA
CCCAAGTACGCTACTTGGGTGAGGAAGGGTCCACACCTGGAGTGCTGTAAGC
Sequence number 3821: Zika virus Whole genome #173 
CCTATCCACCCAAGTACGCTACTTGGGTGAGGAAGGGTCCACACCTGGAGTGCTGTAAGCATCAATTT
CAATGTTGTCAGGCCTGCTAGTCAGCCACAGTTTGGGGAAAGCTGTGCAGCC
Sequence number 3822: Zika virus Whole genome #174 
ATCAATTTCAATGTTGTCAGGCCTGCTAGTCAGCCACAGTTTGGGGAAAGCTGTGCAGCCTGTAACCC
CCCCAGGAGAAGCTGGGAAACCAAGCTCATAGTCAGGCCGATAACGCCATGG
Sequence number 3823: Zika virus Whole genome #175 
TGTAACCCCCCCAGGAGAAGCTGGGAAACCAAGCTCATAGTCAGGCCGATAACGCCATGGCACGGAAA
AAGCCATGCTGCCTGTGAGCCCCTCAGAGGACACTGAGTCAAAAAACCCCAC
Sequence number 3824: Zika virus Whole genome #176 
CACGGAAAAAGCCATGCTGCCTGTGAGCCCCTCAGAGGACACTGAGTCAAAAAACCCCACGCGCTTGG
AAGCGCAGGATGGGAAAAGAAGGTGGCGACCTTCCCCACCCTTTAATCTGGG
Sequence number 3825: Zika virus Whole genome #177 
GCGCTTGGAAGCGCAGGATGGGAAAAGAAGGTGGCGACCTTCCCCACCCTTTAATCTGGGGCCTGAAC
TGGAGATTAGCTGTGAATCTCCGGCAGAAGGACTAGTGGTTAGAGGAGACCC
Sequence number 3826: Zika virus Whole genome #178 
GCCTGAACTGGAGATTAGCTGTGAATCTCCGGCAGAAGGACTAGTGGTTAGAGGAGACCCCCCGGAAA
ACGCAAAACAGCATATTGACGCTGGGAAAGACCAGAGACTCCATGAGTTTCC
Sequence number 3827: Zika virus Whole genome #179 
CCCGGAAAACGCAAAACAGCATATTGACGCTGGGAAAGACCAGAGACTCCATGAGTTTCCACCACGCT
GGCCGCCAGGCACAGATCGCCGAACAGCGGCGGCCGGTGTGGGGAAATCCAT
Sequence number 3828: Zika virus Whole genome #180

AAACGCAAAACAGCATATTGACGCTGGGAAAGACCAGAGACTCCATGAGTTTCCACCACGCTGGCCGC
CAGGCACAGATCGCCGAACAGCGGCGGCCGGTGTGGGGAAATCCATGGGTCT

A prototype kit containing all reagent components required for the entire experimental process from nucleic acid samples isolated from specimens to NGS analysis equipment was produced.
- Basic production components: NGS Library production module, Target Capture module, rash syndrome pathogen capture panel.
- Additional included components: cDNA synthesis module, Polymerase, Magnetic Beads, Streptavidin Beads.

### <Example 2> Kit component design - NGS analysis pipeline development

A dedicated pipeline for analyzing NGS data generated using rash syndrome Multi-Pathogen Capture Panel was developed.

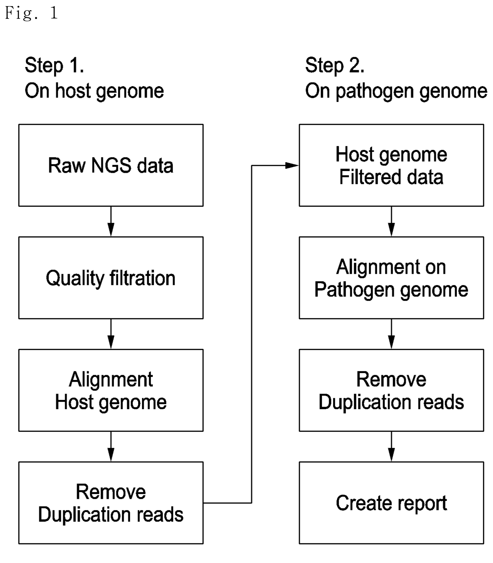

Since the actual sample contains not only the pathogen genome but also nucleic acids derived from the host human genome, a two-step analysis method was applied to first confirm human genome-derived data from the NGS raw data and then confirm pathogen information from the remaining data after excluding it (see Figure 1).

An analysis pipeline was developed that can complete the process from NGS raw data to pathogen analysis report production by executing simple commands without having to run each analysis process separately.

An in silico analysis program was developed that generates simulation data based on reference genome sequences for pathogens included in the panel and can confirm whether each pathogen can be detected.

### <Example 3> Performance Verification - In Silico Analysis Using Simulation Data

In silico analysis using simulation data can estimate the performance of a kit for pathogens for which actual samples are difficult to obtain, and can predict how the kit will respond when new mutations occur in each pathogen.

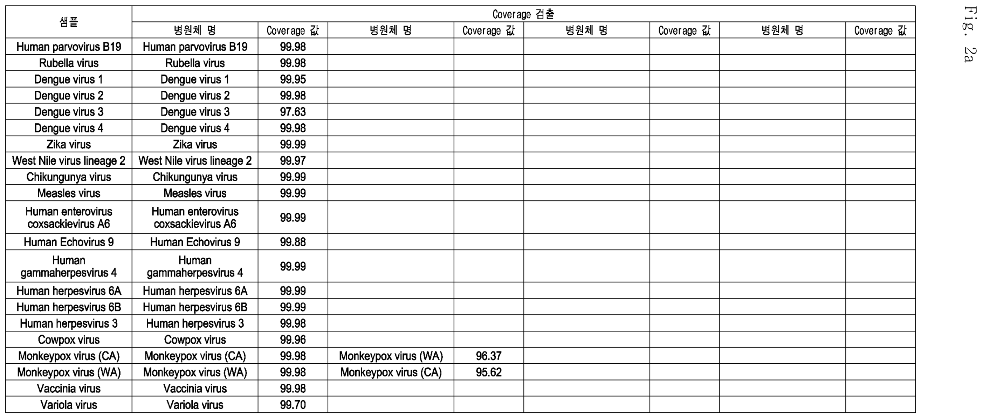

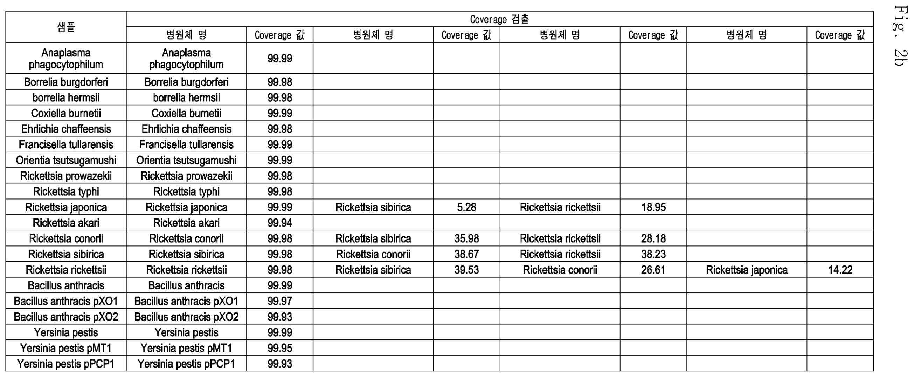

Using the developed in silico analysis program, simulation NGS data was produced and analyzed assuming that mutations occurred at a frequency of 10% in the standard reference genome sequence for each pathogen. The results are shown in Figure 2.

The analysis results show that the CA and WA types of monkeypox virus have very high genome sequence similarity, making it difficult to distinguish them from each other.

In the case of the Rickettsia bacteria group of the erythematous fever group, the similarity in the target marker area was relatively high, but it is believed that it will be possible to distinguish them by setting analysis criteria.

Based on the results of the in silico analysis, the criteria for determining whether each pathogen was detected in the analysis pipeline were determined. The detection of each pathogen is determined by referring to the general human germline variant analysis standards for subsequent analysis of accurate mutation information of the pathogen, and is based on 20x coverage, which is the ratio of regions where NGS reads are detected at least 20 times among the pathogen marker regions.

### <Example 4> Performance Verification - Panel LOD Evaluation

Using a standard sample with a known pathogen copy number, serially diluted samples were prepared and the number of detectable pathogen copies was confirmed using a panel.

The samples were prepared under conditions similar to actual specimen environments by adding 5 ng of human-derived nucleic acid standard material to the pathogen isolate samples.

The diluted samples were prepared using a Rubella virus sample with a known exact copy number, and the analysis results for the rash Syndrome panel were as shown in Table 3.

**[Table 3]**

| Type of pathogen | Number of copies | Detected pathogens | 20X Coverage(%) |
|---|---|---|---|
| Rubella virus | 100,000 copy | Rubella virus | 70.71 |
| Rubella virus | 50,000 copy | Rubella virus | 64.56 |
| Rubella virus | 10,000 copy | N/A | N/A |
| Rubella virus | 1,000 copy | N/A | N/A |

As the number of virus copies decreased, it was confirmed that the 20x coverage detection value actually decreased, and at 50,000 copies or more, the coverage value was above the analysis standard, but at 10,000 copies, the value was below the standard, and the pathogen could not be detected.

### <Example 5> Performance Verification - Panel Validity Evaluation

A validity test was conducted to determine whether the rash syndrome Panel could detect the pathogen in samples for which the actual exact copy number could not be confirmed among the pathogen samples included in the panel.

A 5 ng human-derived nucleic acid standard was added to pathogen isolate samples to create a test kit that mimics real-world conditions.

The results of a validation test using a total of 32 samples show that pathogens detected with a 20x coverage value above the standard are shown in Table 4.

**[Table 4]**

| Sample name | Detected pathogens | 20x Coverage(%) |
|---|---|---|
| Anaplasma phagocytophilum str.JM | Anaplasma phagocytophilum | 98.72 |
| Bacillus anthracis | Bacillus anthracis | 99.99 |
| Borrelia burgdorferi ZS7 | Borrelia burgdorferi | 99.58 |
| Borrelia hermsii YOR | Borrelia hermsii | 97.00 |
| C Burnerii | Coxiella burnetii | 98.36 |
| Chikungunya virus strain Ross NH177 | Chikungunya virus | 99.97 |
| Coxiella burnetii | Coxiella burnetii | 98.51 |
| Dengue virus 1 | Dengue virus 1 | 63.20 |
| Dengue virus 2 | Dengue virus 2 | 83.82 |
| Dengue virus 3 | Dengue virus 3 | 96.24 |
| Dengue virus 4 | Dengue virus 4 | 66.59 |
| Ehrlichia chaffeensis strArkansas | Ehrlichia chaffeensis | 99.97 |
| Measles virus 40202 | Measles virus | 72.34 |
| Measles virus 40204 | Measles virus | 91.69 |
| Measles virus 43161 | Measles virus | 98.96 |
| Measles virus genotype B3 strain | Measles virus | 92.10 |
| MPXV | Monkeypox virus WA | 99.98 |
| Orientia tsutsugamushi Boryong | Orientia tsutsugamushi | 99.91 |
| R akari | Rickettsia akari | 99.5 |
| R japonica | Rickettsia japonica 999 | 99.9 |
| R prowazekii | Rickettsia prowazekii | 97.67 |
| Rickettsia conorii strMalish7 | Rickettsia conorii | 99.93 |
| Rickettsia typhi strWilmington | Rickettsia typhi | 71.64 |
| RuV | Rubella virus | 70.71 |
| VACV | Vaccinia virus | 99.96 |
| VZV | Human herpesvirus 3 9998 | 99.98 |
| Yersinia pestis | Yersinia pestis | 99.99 |
| Zika virus isolate 41525 | Zika virus isolate | 81.71 |
| West Nile virus lineage 2 | N/A | N/A |
| Rickettsia sibirica str246 | N/A | N/A |
| Human Echovirus 9 | N/A | N/A |
| Human enterovirus coxsackievirus A6 | N/A | N/A |

Among the 32 specimens, the pathogen used in the experiment was successfully detected in 28 specimens.

To determine the cause, direct deep sequencing was performed on samples without panel application for the four pathogens that failed detection. The results showed a low proportion of data corresponding to each pathogen among the total data produced, suggesting that the actual copy numbers of each pathogen within the samples were very low.

**[Table 5]**

| Sample name | Deep sequencing read number | Data multiplication compared to genome | Pathogen Data Ratio | 20x Coverage |
|---|---|---|---|---|
| Coxsackievirus A6 | 1,979,826 | 40214.4 | 0.002% | 0.000% |
| Echovirus 9 | 1,983,614 | 40199.4 | 0.16% | 31.66% |
| Rickettsia sibirica str246 | 1,975,756 | 238.7 | 0.14% | 0.19% |
| West Nile virus lineage 2 | 1,945,824 | 26803.5 | 3.61% | 29.77% |

### <Example 6> Performance Verification - Verification of Simultaneous Pathogen Detection Performance

Samples prepared by mixing two or three pathogen samples included in the panel to confirm whether all pathogens included in the samples could be detected simultaneously.

5 ng of human-derived nucleic acid standard material was added to the pathogen isolate sample to produce conditions similar to the actual sample environment. The results of the simultaneous detection experiment for a sample containing two types of pathogens are shown in Table 6.

**[Table 6]**

| Type of pathogen | Detected pathogens | 20x Coverage (%) | Detected pathogens | 20x Coverage (%) |
|---|---|---|---|---|
| Coxiella burnetii + Human gammaherpesvirus 4 | Coxiella burnetii | 98.46 | Human gammaherpesvirus 4 | 97.21 |
| Monkeypox virus WA + Human gammaherpesvirus 4 | Monkeypox virus WA | 99.98 | Human gammaherpesvirus 4 | 96.52 |
| Rickettsia akari + Human gammaherpesvirus 4 | Rickettsia akari | 99.5 | Human gammaherpesvirus 4 | 97.06 |
| Rickettsia japonica + Human gammaherpesvirus 4 | Rickettsia japonica | 99.9 | Human gammaherpesvirus 4 | 97.13 |
| Rickettsia prowazekii + Human gammaherpesvirus 4 | Rickettsia prowazekii | 97.67 | Human gammaherpesvirus 4 | 96.52 |
| Vaccinia virus + Human gammaherpesvirus 4 | Vaccinia virus | 99.96 | Human gammaherpesvirus 4 | 89.45 |
| Human herpesvirus 3 + Human gammaherpesvirus 4 | Human herpesvirus 3 | 99.98 | Human gammaherpesvirus 4 | 97.25 |

In all seven samples produced, two pathogens included in the panel were successfully detected simultaneously.

The results of simultaneous experiments on samples containing three types of pathogens are shown in Table 7.

**[Table 7]**

| Type of pathogen | Detected pathogens | 20x Coverage (%) | Detected pathogens | 20x Coverage (%) | Detected pathogens | 20x Coverage (%) |
|---|---|---|---|---|---|---|
| Rickettsia akari + Rickettsia japonica + Rickettsia prowazekii | Rickettsia akari | 99.5 | Rickettsia japonica | 99.9 | Rickettsia prowazekii | 97.67 |

In a mixed sample of three pathogens, Rickettsia akari, Rickettsia japonica, and Rickettsia prowazekii, all three pathogens were successfully detected, and it was confirmed that each pathogen could be detected normally without confusion in a mixed sample of similar species.

## Claims

1. A capture probe set for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, including SEQ ID NO: 1 to SEQ ID NO: 3828.

2. A composition for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, comprising the capture probe set for simultaneous detection of claim 1.

3. The composition for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus of claim 2, wherein the sample for detection is isolated from feces, blood, serum, urine or biological tissue.

4. A composition for diagnosing rash syndrome comprising the capture probe set for simultaneous detection of the claim 1.

5. The composition for diagnosing rash syndrome of claim 4, wherein the rash syndrome is at least one selected from the group consisting of anaplasmosis, anthrax, Lyme disease, relapsing fever, chikungunya fever, Q fever, dengue fever, ehrlichiosis, chickenpox, measles, monkeypox, tsutsugamushi disease, rickettsial infection, typhus, rash, rubella, vaccinia, plague, and Zika virus infection.

6. The composition for diagnosing rash syndrome of claim 4, wherein the samples for diagnosis are isolated from feces, blood, serum, urine or biological tissue.

7. A kit for diagnosing rash syndrome, comprising a composition for diagnosing rash syndrome and instructions for use in claim 4.

8. The kit for diagnosing rash syndrome of claim 7, wherein the rash syndrome may be at least one selected from the group consisting of anaplasmosis, anthrax, Lyme disease, relapsing fever, chikungunya fever, Q fever, dengue fever, ehrlichiosis, chickenpox, measles, monkeypox, tsutsugamushi disease, rickettsial infection, typhus, rash, rubella, vaccinia, plague, and Zika virus infection.

9. The kit for diagnosing rash syndrome of claim 7, wherein the sample for diagnosis may be isolated from feces, blood, serum, urine or biological tissue.

10. A method for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus, comprising the steps of preparing an isolated DNA or RNA sample; reacting the isolated DNA or RNA sample using the capture probe set of claim 1; and obtaining a detection result.

11. The method for simultaneous detection of Anaplasma phagocytophilum, Bacillus anthracis, Borrelia burgdorferi, Borrelia hermsii, Chikungunya virus, Coxiella burnetii, Dengue virus, Ehrlichia chaffeensis, Human herpesvirus, Measles virus, Monkeypox virus, Orientia tsutsugamushi, Rickettsia akari, Rickettsia conorii, Rickettsia japonica, Rickettsia prowazekii, Rickettsia typhi, Rubella virus, Vaccinia virus, Yersinia pestis, and Zika virus of claim 10, wherein the sample for detection is isolated from feces, blood, serum, urine or living tissue.
